# EUROPEAN PATENT APPLICATION

(11) **EP 2 108 690 A1**
(43) Date of publication of application: **14.10.2009**
(21) Application number: 09250836.5
(22) Date of filing: 24.03.2009
(51) Int. Cl.: C09K 11/06

(54) **Novel organic electroluminescent compounds and organic electroluminescent device using the same**

(30) Priority: 02.04.2008 KR 20080030977
(71) Applicant: Gracel Display Inc., Seoul 133-833 (KR)
(72) Inventor: Lee, Mi Ae, Seoul, 121-190 (KR); Cho, Young Jun, Seoul 136-060 (KR); Kwon, Hyuck Joo, Seoul 130-100 (KR); Kim, Bong Ok, Seoul 135-090 (KR); Kim, Sung Min, Seoul-city 157-886 (KR); Yoon, Seung Soo, Seoul 135-884 (KR)
(74) Representative: Kent, Venetia Katherine

(57) **Abstract**

The present invention relates to novel organic electroluminescent compounds, and organic electroluminescent devices employing the same in an electroluminescent layer. Specifically, the organic electroluminescent compounds according to the invention are **characterized in that** they are represented by Chemical Formula (1) or Chemical Formula (2): 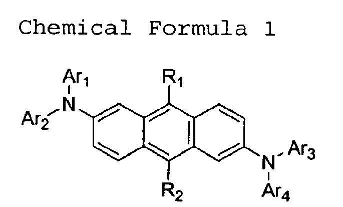 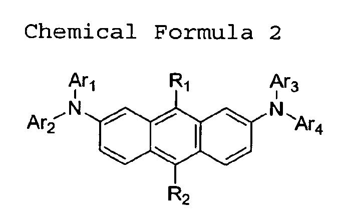 provided that the total number of carbons in R₁ or R₂ is from 21 to 60.

Since the organic electroluminescent compounds according to the invention have good luminous efficiency and excellent life property of material, organic electroluminescent devices having high color purity and luminance with very good operation life can be manufactured therefrom.

## Description

### FIELD OF THE INVENTION

The present invention relates to novel organic electroluminescent compounds, and organic electroluminescent devices employing the same in an electroluminescent layer. Specifically, the organic electroluminescent compounds according to the present invention are represented by Chemical Formula (1) or Chemical Formula (2): 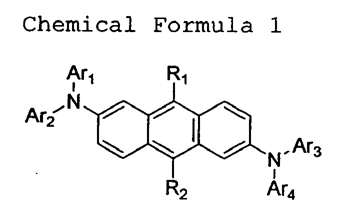 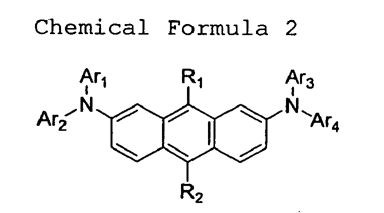 wherein, R₁ and R₂ independently represent (C6-C60)aryl or (C5-C60)heteroaryl, and the aryl and heteroaryl may be further substituted by one or more substituent(s) selected from halogen, (C1-C60)alkyl, (C6-C60)aryl, (C4-C60)heteroaryl, halo(C1-C30)alkyl, halo(C1-C30)alkoxy, (C1-C30)alkyl(C6-C30)aryl, (C6-C30)ar(C1-C30)alkyl, (C6-C30)ar(C1-C30)alkoxy, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, provided that the total number of carbons in R₁ or R₂ is from 21 to 60;

Ar₁ through Ar₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and the alkyl, cycloalkyl, heterocycloalkyl, aryl, heteroaryl, arylsilyl, alkylsilyl, alkylamino and arylamino of Ar₁ through Ar₄ may be further substituted by one or more substituent(s) selected from halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

### BACKGROUND OF INVENTION

The most important factor in developing organic electroluminescent devices of high efficiency and long life is development of electroluminescent material of high performance. In view of current development of electroluminescent material, green electroluminescent materials show superior electroluminescent property to red or blue electroluminescent materials. However, conventional green electroluminescent materials still have many problems to achieve manufacturing panels of large size with low power consumption. In view of practical efficiency and life, various kinds of electroluminescent materials for green have been reported up to now. Though they exhibit from 2 to 5 times of electroluminescent property as compared to red or blue electroluminescent materials, development of green electroluminescent material is getting challenged by the improvement of properties of red or blue electroluminescent material. In the meanwhile, enhancement of lifetime of green material is still insufficient, so that a green electroluminescent material providing long life is seriously required.

As green fluorescent material, a coumarin derivative (Compound D), quinacrydone derivatives (Compound E), DPT (Compound F) and the like have been known. Compound D is the structure of C545T that is the most widely used coumarin derivative up to the present. In general, those materials are doped, by using Alq as the host, at a concentration of several % to about several ten %, to form an electroluminescent device.

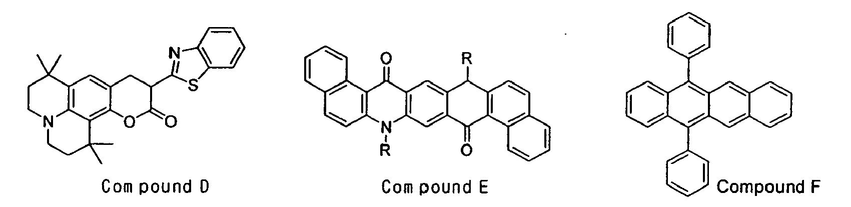

Japanese Patent Laid-Open No. 2001-131541 discloses bis(2,6-diarylamino)-9,10-diphenylanthracene derivatives represented by Compound G shown below, wherein diarylamino groups are directly substituted at 2- and 6-position of anthracene, respectively.

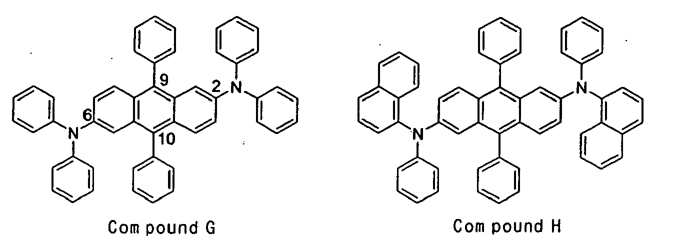

Japanese Patent Laid-Open No. 2003-146951 (which discloses compounds for a hole transport layer) does not mention the compounds wherein diarylamino groups are directly substituted at 2- and 6-position of anthracene, respectively, but simply describing the compounds having phenyl substituents at 9- and 10-position of anthracene. As considering that Japanese Patent Laid-Open No. 2003-146951 indicated the problem of Compound (H) (wherein diarylamino groups are directly substituted at 2- and 6-position of the anthracene ring, respectively) having poor luminous efficiency, it is found that the invention of Japanese Patent Laid-Open No. 2003-146951 did not recognize the compounds other than those having phenyl substituents at 9- and 10-position of anthracene.

In the meanwhile, Japanese Patent Laid-Open No. 2004-91334 suggested the organic electroluminescent compounds represented by Compound (J), which overcomes poor luminous efficiency of conventional compounds but exhibits low ionization potential and excellent hole transportation, by further substituting the aryl group of the diarylamino group with diarylamino groups, even though diarylamino groups are directly substituted on the anthracene group.

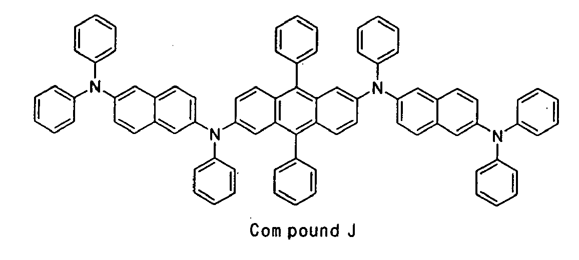

The compounds suggested by Japanese Patent Laid-Open No. 2004-91334 (applied as a hole transport layer), however, show the problem of shortened operation life as a hole transport layer because of too many amine functional groups, even though they showed lowered ionization potential due to many amine functional groups and overcame the problem of increase in hole transporting property.

### SUMMARY OF THE INVENTION

The present inventors found that anthracene compounds, wherein (C21-C60) bulky aryl or heteroaryl is incorporated at the 9- and 10-position, and amino groups having two alkyl, aryl, heteroaryl, cycloalkyl or heterocycloalkyl groups being substituted, respectively, are directly substituted at the 2- and 6-, or 2- and 7-position, show excellent improvement in luminescent properties, and completed the present invention.

Thus, the inventors have intensively endeavored to overcome the problems described above and to develop novel electroluminescent compounds which can realize an organic electroluminescent device having excellent color purity and luminous efficiency with long life.

The object of the invention is to provide novel organic electroluminescent compounds which are anthracene compounds wherein (C21-C60) bulky aryl or heteroaryl is incorporated at the 9- and 10-position, and amino groups having two alkyl, aryl, heteroaryl, cycloalkyl or heterocycloalkyl groups being substituted, respectively, are directly substituted at the 2- and 6- or 2- and 7-position.

Another object of the present invention is to provide an organic electroluminescent device having an electroluminescent region, by using one or more organic electroluminescent compound(s) described above, together with one or more compound(s) selected from anthracene derivatives and benz[a]anthracene derivatives as electroluminescent host.

Thus the object of the present invention is to provide organic electroluminescent compounds having excellent color purity and luminous efficiency with very good device life, and to provide organic electroluminescent devices comprising said novel organic electroluminescent compounds.

The present invention relates to novel organic electroluminescent compounds and organic electroluminescent devices employing the same in an electroluminescent layer. Specifically, the organic electroluminescent compounds according to the present invention are represented by Chemical Formula (1) or Chemical Formula (2): 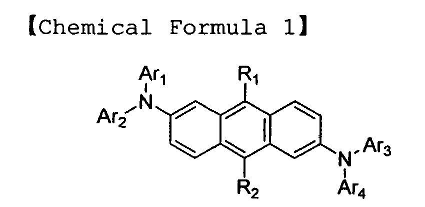 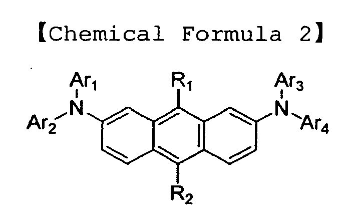 wherein, R₁ and R₂ independently represent (C6-C60)aryl or (C5-C60)heteroaryl, and the aryl and heteroaryl may be further substituted by one or more substituent(s) selected from halogen, (C1-C60)alkyl, (C6-C60)aryl, (C4-C60)heteroaryl, halo(C1-C30)alkyl, halo(C1-C30)alkoxy, (C1-C30)alkyl(C6-C30)aryl, (C6-C30)ar(C1-C30)alkyl, (C6-C30)ar(C1-C30)alkoxy, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, provided that the total number of carbons in R₁ or R₂ is from 21 to 60;

Ar₁ through Ar₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; and the alkyl, cycloalkyl, heterocycloalkyl, aryl, heteroaryl, arylsilyl, alkylsilyl, alkylamino and arylamino of Ar₁ through Ar₄ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

### BRIEF DESCRIPTION OF THE DRAWINGS

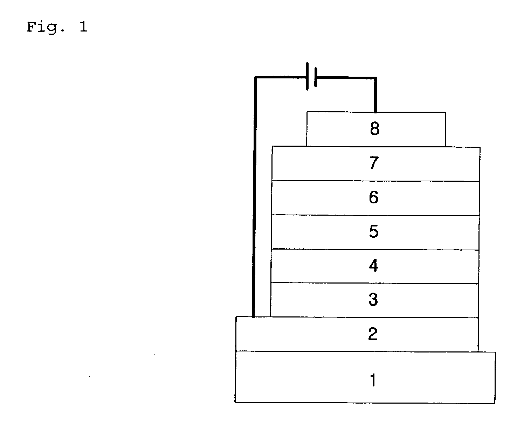

Fig. 1 is a cross-sectional view of an organic light emitting diode (OLED).

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The term "alkyl", "alkoxy" and other subsituents containing "alkyl" moiety include both linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, tetrahydronaphthyl, indanyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be 5- or 6-membered monocyclic heteroaryl or a polycyclic heteroaryl which is fused with one or more benzene ring(s), and may be partially saturated. Specific examples include monocyclic heteroaryl groups such as furyl, thiophenyl, pyrrolyl, pyranyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; and polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl; but they are not restricted thereto.

The organic electroluminescent compounds of the invention which are represented by Chemical Formula (1) or (2) are characterized by their structure of novel concept which maximizes luminous efficiency of green electroluminescent devices resulted from those compounds and their device life, being unexpected by conventional inventions.

The organic electroluminescent compounds of Chemical Formula (1) or (2) according to the invention adopted a structure showing an efficient energy transfer mechanism between the host and the dopant, which can realize electroluminescent property with a reliably high efficiency on the basis of improvement in electron density distribution. The structure of the novel compounds according to the present invention can provide a skeletal which can also tune an electroluminescent property with high efficiency in the range from blue to red, not only for green electroluminescence. Beyond the concept of using a host material with high electron conductivity such as Alq, the invention applies a host having appropriate balance of hole conductivity and electron conductivity, thereby overcoming the problems of conventional materials including low initial efficiency and short lifetime, and ensures electroluminescent properties with high performance having high efficiency and long life for each color.

The organic electroluminescent compounds according to the present invention include those represented by Chemical Formula (3) or (4): 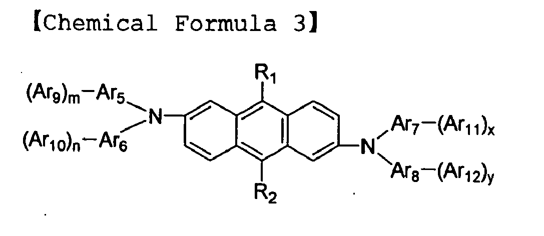 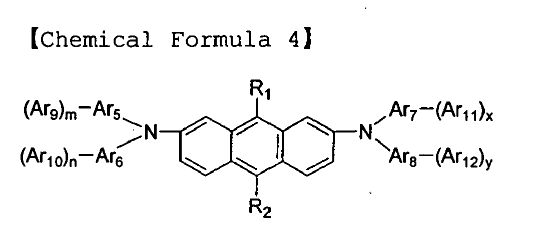
wherein, R₁ and R₂ are defined as in Chemical Formula (1);
Ar₅ through Ar₈ independently represent (C6-C60)aryl, (C4-C60)heteroaryl, (C3-C60)cycloalkyl, 5- or 6-membered heterocycloalkyl containing one or more heteroatom(s) selected from N, O and S, (C6-C60)arylene or (C4-C60)heteroarylene; the aryl, heteroaryl, arylene or heteroarylene of Ar₅ through Ar₈ may be further substituted by one or more substituent(s) selected from a group consisting of (C1-C60)alkyl, halo(C1-C30)alkyl, (C1-C30)alkoxy, halo(C1-C30)alkoxy, (C3-C60)cycloalkyl, halogen, cyano, (C1-C30)alkyl(C6-C30)aryl, (C6-C30)ar(C1-C30)alkyl, (C6-C30)ar(C1-C30)alkoxy, tri(C1-C30)alkylsilyl, di(C1-C30)alkyl(C6-C30)arylsilyl and tri(C6-C30)arylsilyl;
Ar₉ through Ar₁₂ independently represent (C6-C60)aryl or (C4-C60)heteroaryl; the aryl or heteroaryl of Ar₉ through Ar₁₂ may be further substituted by one or more substituent(s) selected from a group consisting of (C1-C60)alkyl, halo(C1-C30)alkyl, (C1-C30)alkoxy, halo(C1-C30)alkoxy, (C3-C60)cycloalkyl, halogen, cyano, (C1-C30)alkyl(C6-C30)aryl, (C6-C30)ar(C1-C30)alkyl, (C6-C30)ar(C1-C30)alkoxy, tri(C1-C30)alkylsilyl, di(C1-C30)alkyl(C6-C30)arylsilyl and tri(C6-C30)arylsilyl;
provided that m is 0 when Ar₅ represents (C6-C60)aryl, (C4-C60)heteroaryl, (C3-C60)cycloalkyl or 5- or 6-membered heterocycloalkyl containing one or more heteroatom(s) selected from N, O and S, while m is an integer from 1 to 4 when Ar₅ represents (C6-C60)arylene or (C4-C60)heteroarylene;
n is 0 when Ar₆ represents (C6-C60)aryl, (C4-C60)heteroaryl, (C3-C60)cycloalkyl or 5- or 6-membered heterocycloalkyl containing one or more heteroatom(s) selected from N, O and S, while n is an integer from 1 to 4 when Ar₆ represents (C6-C60)arylene or (C4-C60)heteroarylene;
x is 0 when Ar₁₁ represents (C6-C60)aryl, (C4-C60)heteroaryl, (C3-C60)cycloalkyl or 5- or 6-membered heterocycloalkyl containing one or more heteroatom(s) selected from N, O and S, while x is an integer from 1 to 4 when Ar₁₁ represents (C6-C60)arylene or (C4-C60)heteroarylene; and
y is 0 when Ar₁₂ represents (C6-C60) aryl, (C4-C60)heteroaryl, (C3-C60)cycloalkyl or 5- or 6-membered heterocycloalkyl containing one or more heteroatom(s) selected from N, O and S, while y is an integer from 1 to 4 when Ar₁₂ represents (C6-C60)arylene or (C4-C60)heteroarylene.

The term "arylene" described herein means an organic radical derived from aromatic hydrocarbon via elimination of two or more hydrogen atoms. Each ring suitably comprises a monocyclic or fused ring system containing from 4 to 7, preferably from 5 to 6 cyclic atoms. The term "heteroarylene" means organic radicals derived from aromatic heterocyclic compounds via elimination of two or more hydrogen atoms, which may be 5- or 6-membered monocyclic heteroarylene or polycyclic heteroarylene fused with one or more benzene ring(s). The heteroarylene may be partially saturated.

In Chemical Formula (1) or (2), R₁ and R₂ may be independently selected from the following structures: 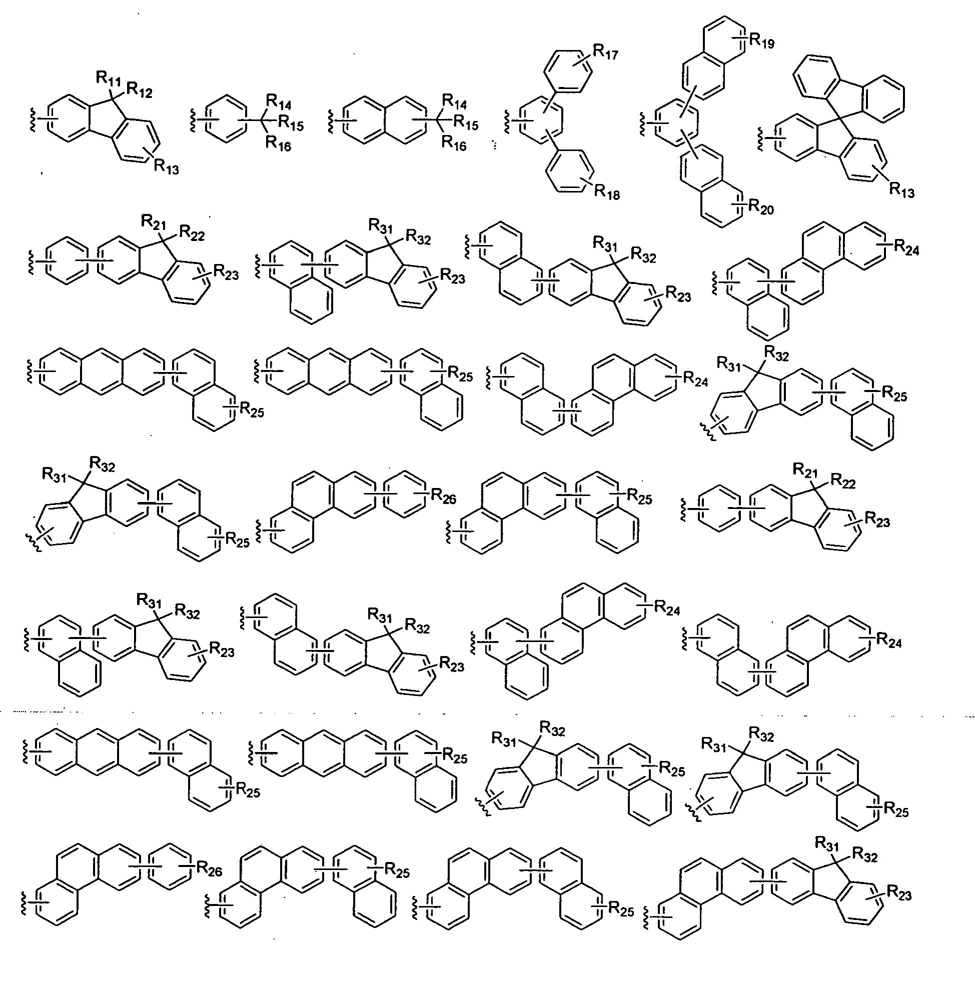 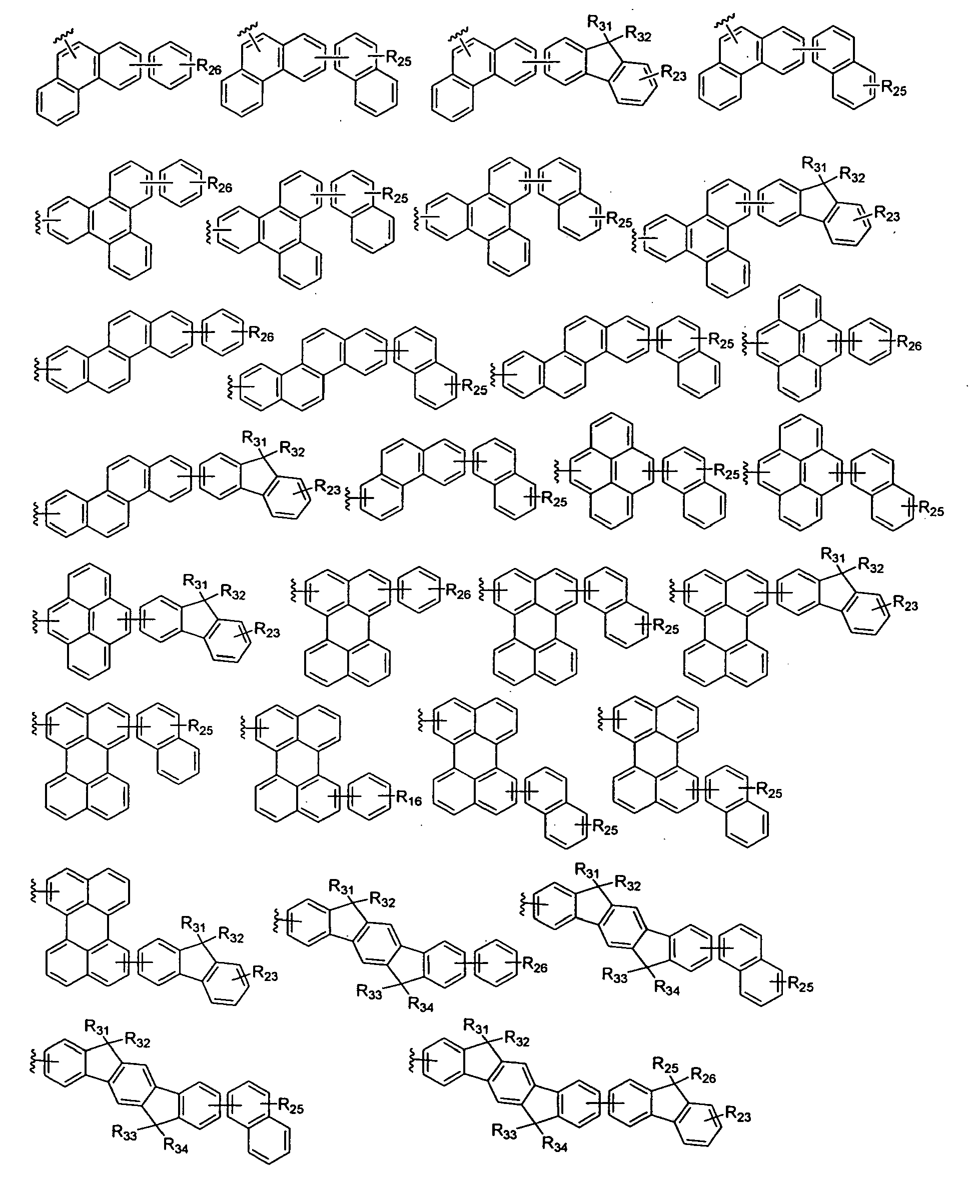 wherein, R₁₁ and R₁₂ independently represent (C6-C60)aryl, (C1-C30)alkyl(C6-C30)aryl or (C6-C30)ar(C1-C30)alkyl; R₁₃ represents hydrogen, (C1-C60)alkyl, (C6-C60)aryl, (C1-C30)alkyl(C6-C30)aryl or (C6-C30)ar(C1-C30)alkyl; R₁₄ through R₁₆ independently represent (C6-C60)aryl, (C1-C30)alkyl(C6-C30)aryl or (C6-C30)ar(C1-C30)alkyl; R₁₇ and R₁₈ independently represent (C1-C60)alkyl; R₁₉ and R₂₀ independently represent hydrogen or (C1-C60)alkyl; R₂₁ through R₂₆ independently represent (C1-C60)alkyl; and R₃₁ through R₃₄ independently represent hydrogen or (C1-C60)alkyl.

Preferably, R₁ and R₂ are selected from the following structures:

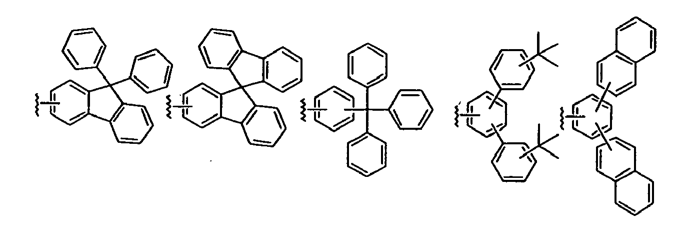

In Chemical Formulas (3) and (4), Ar₅ through Ar₈ are independently selected from phenyl, biphenyl, naphthyl, anthryl, fluorenyl, phenanthryl, pyrenyl, perylenyl, fluoranthenyl, pyridyl, quinolyl, furanyl, thiophenyl, thiazolyl, imidazolyl, oxazolyl, benzofuranyl, benzothiazolyl, benzimidazolyl, benzoxazolyl, morpholino, thiomorpholino, 1,2-phenylene, 1,3-phenylene, 1,4-phenylene, 1,3,4-phenylene, 1,3,5-phenylene, and the following structures: 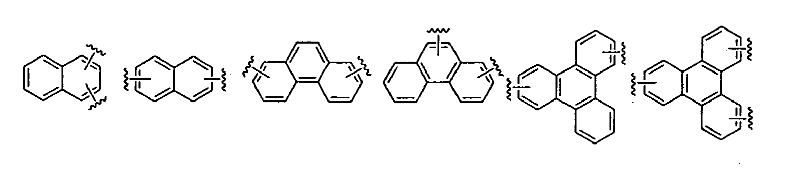 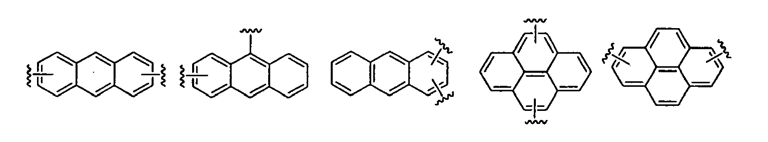 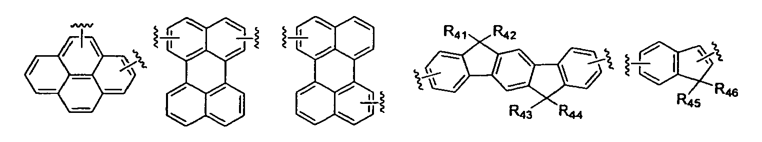 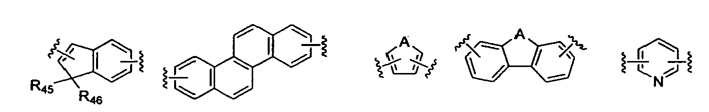
the phenyl, biphenyl, naphthyl, fluorenyl or benzimidazolyl of Ar₅ through Ar₈ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluorethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl;
R₄₁ through R₄₆ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; or R₄₁ and R₄₂, R₄₃ and R₄₄ or R₄₅ and R₄₆ may be linked via (C3-C60)alkylene or (C3-C60)alkenylene with or without a fused ring to form an alicyclic ring, or a monocyclic or polycyclic aromatic ring;
A represents CR₅₁R₅₂, NR₅₃, O or S;
wherein R₅₁ through R₅₃ independently represent hydrogen, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; or R₅₁ and R₅₂ may be linked via (C3-C60)alkylene or (C3-C60)alkenylene with or without a fused ring to form an alicyclic ring, or a monocyclic or polycyclic aromatic ring;
Ar₉ through Ar₁₂ independently represent phenyl, biphenyl, naphthyl, anthryl, fluorenyl, phenanthryl, pyrenyl, perylenyl, fluoranthenyl, pyridyl or quinolyl; and the phenyl, naphthyl, anthryl, fluorenyl, phenanthryl, pyrenyl, perylenyl, fluoranthenyl, pyridyl or quinolyl of Ar₉ through Ar₁₂ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluorethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl.

The organic electroluminescent compounds according to the present invention can be specifically exemplified by the following compounds, but are not restricted thereto: 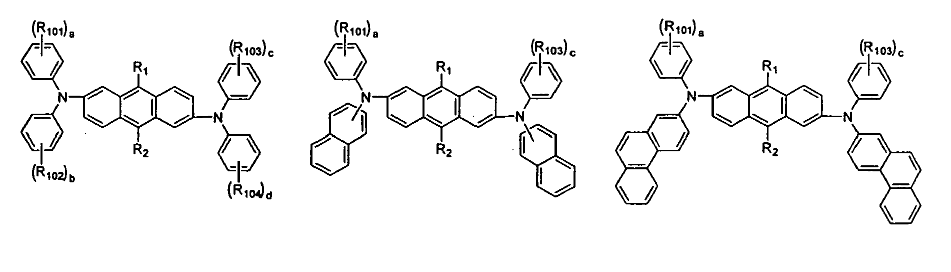 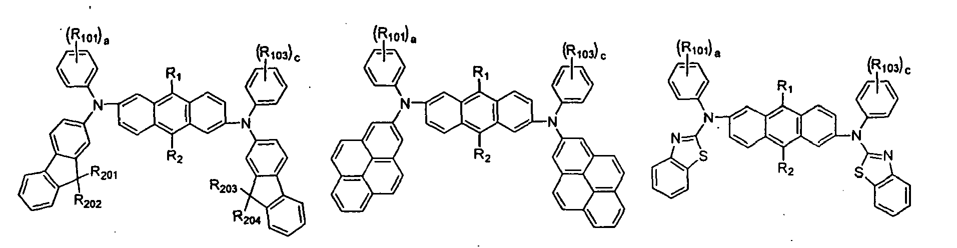 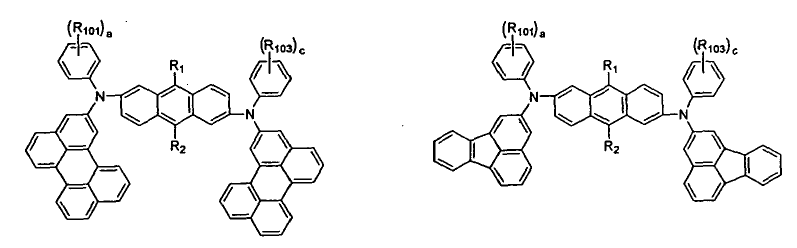 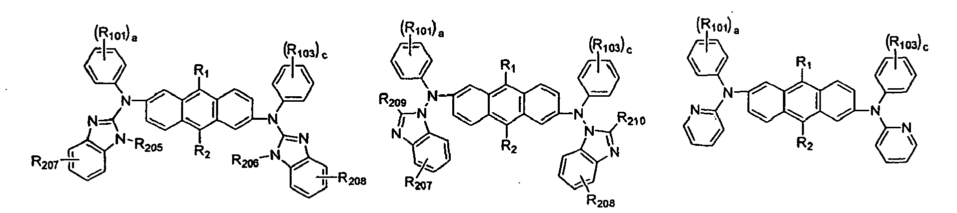 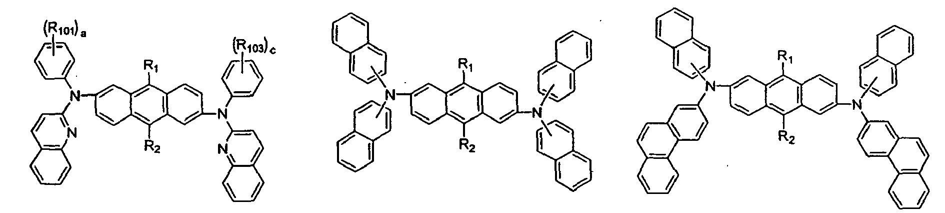 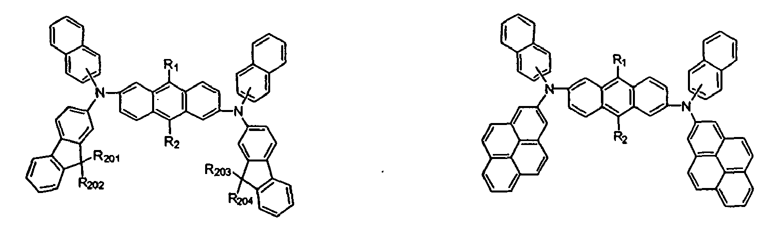 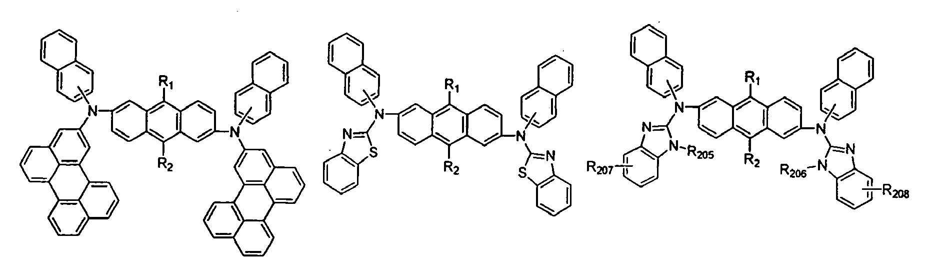 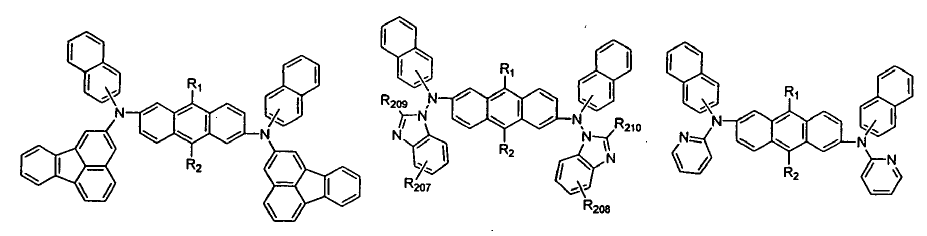 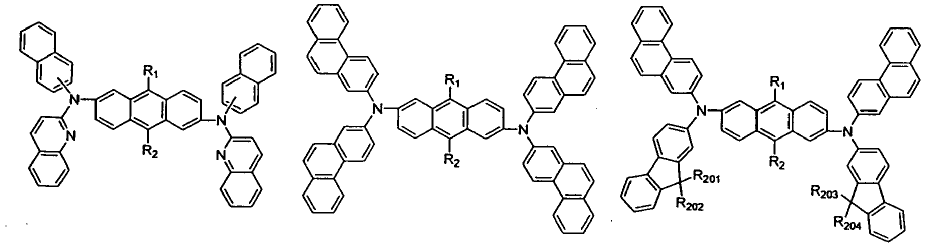 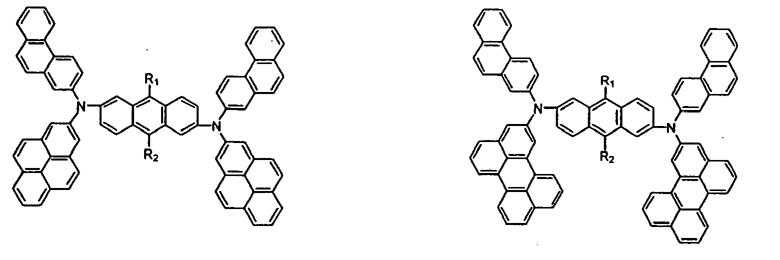 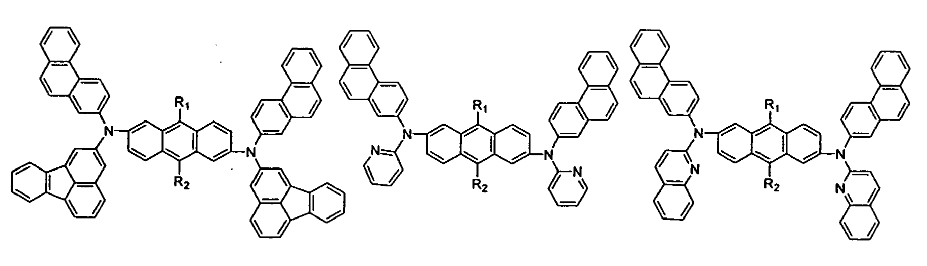 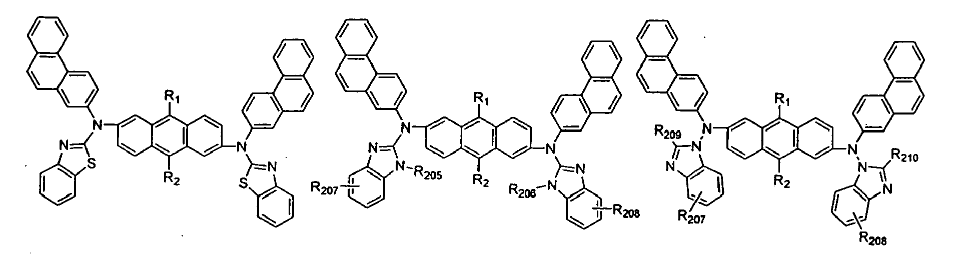 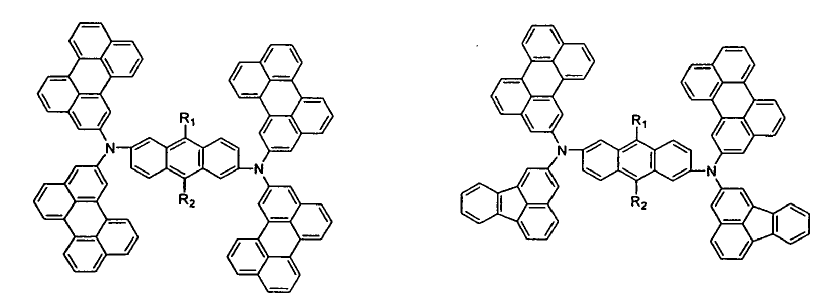 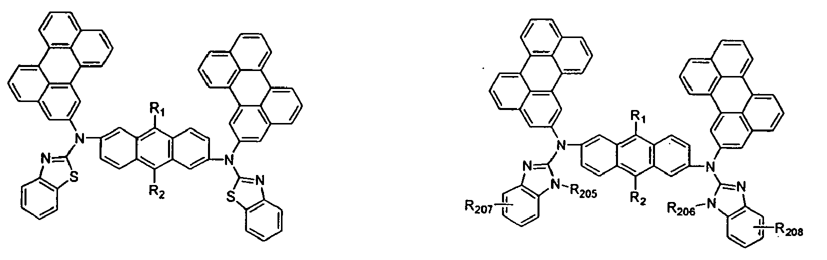 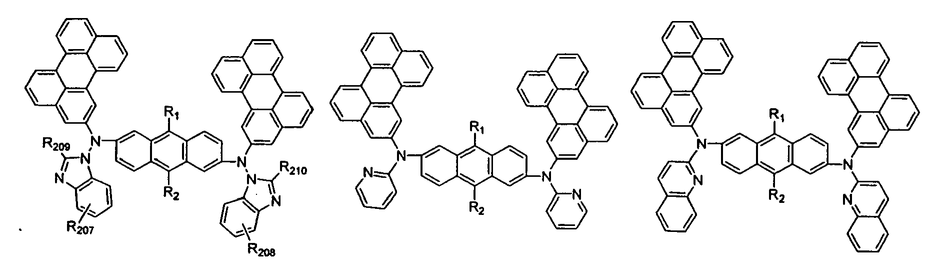 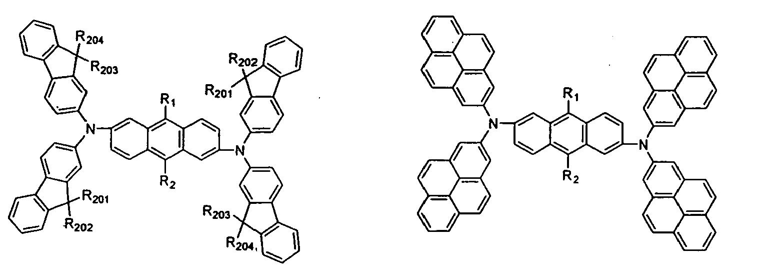 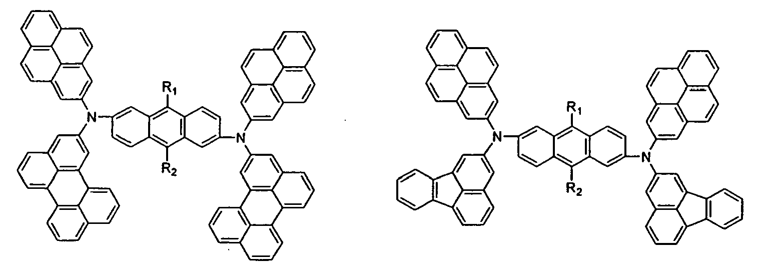 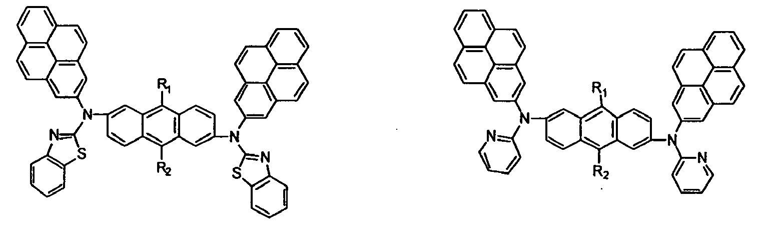 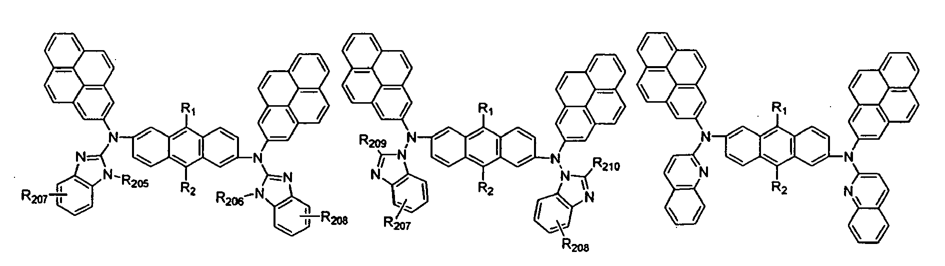 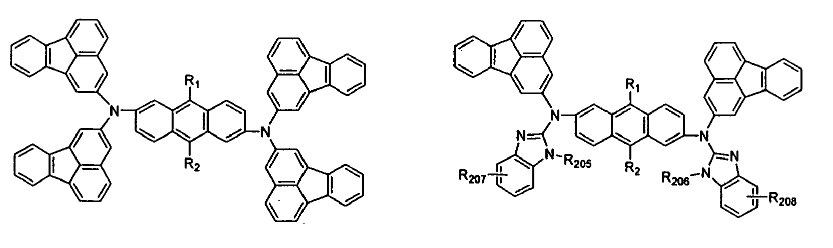 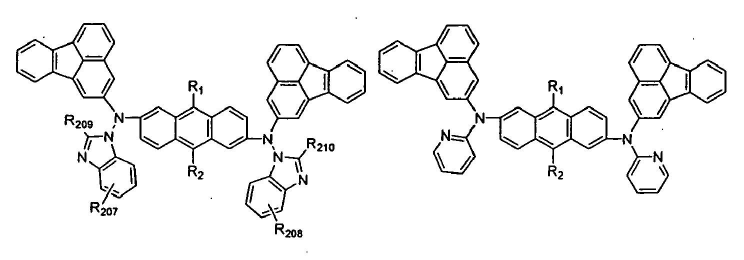 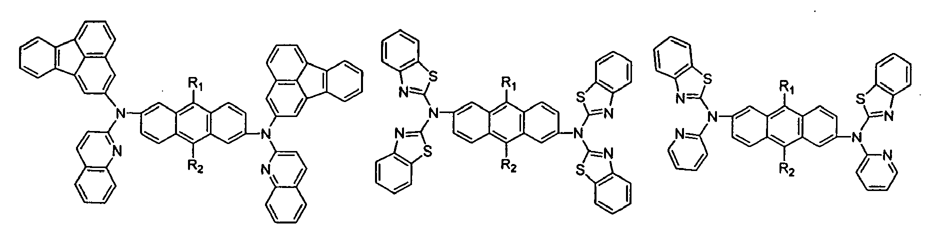 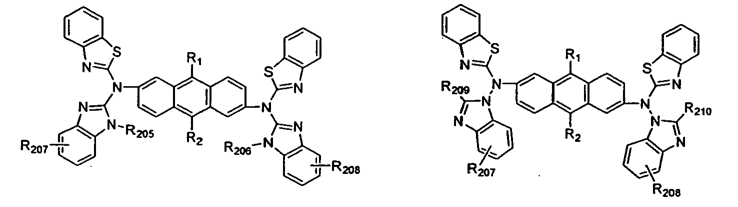 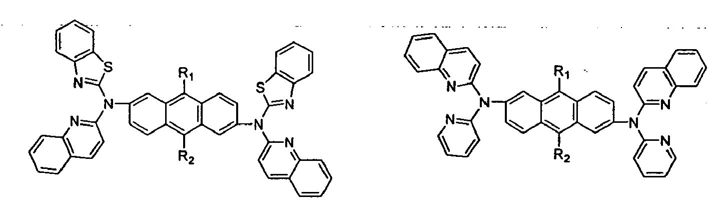 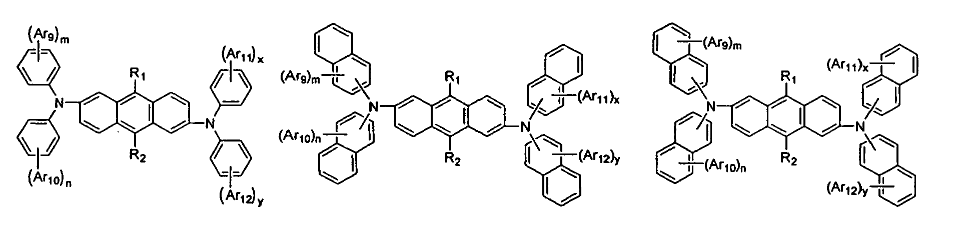 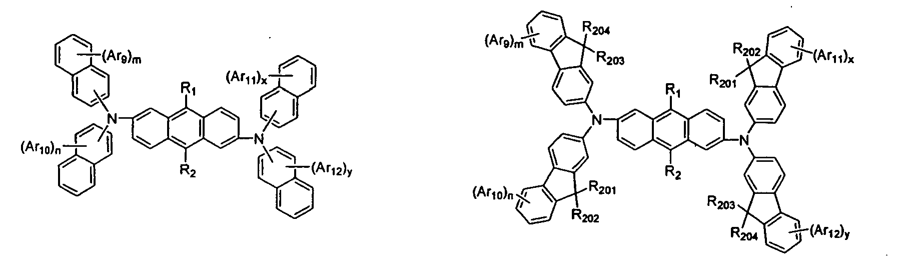 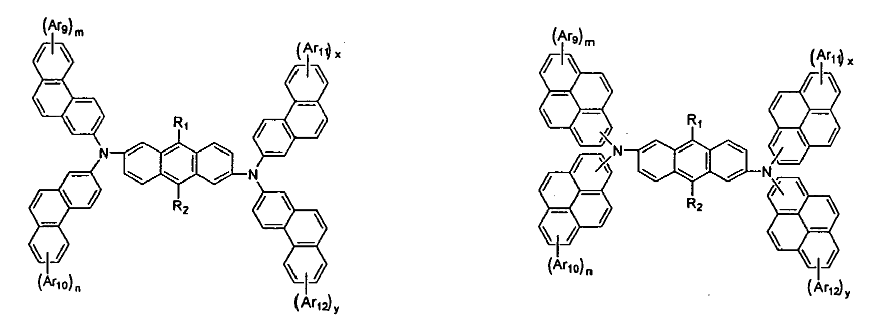 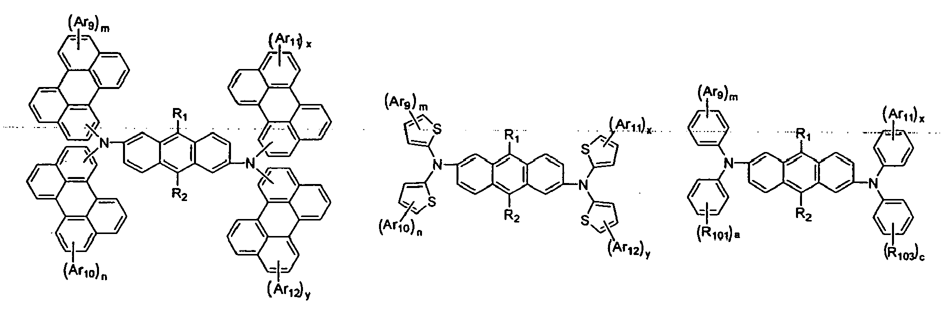 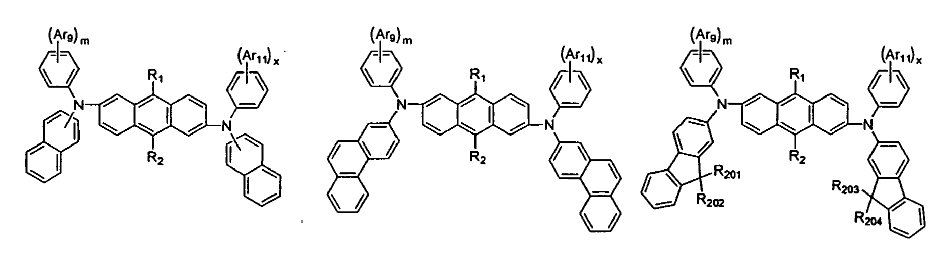 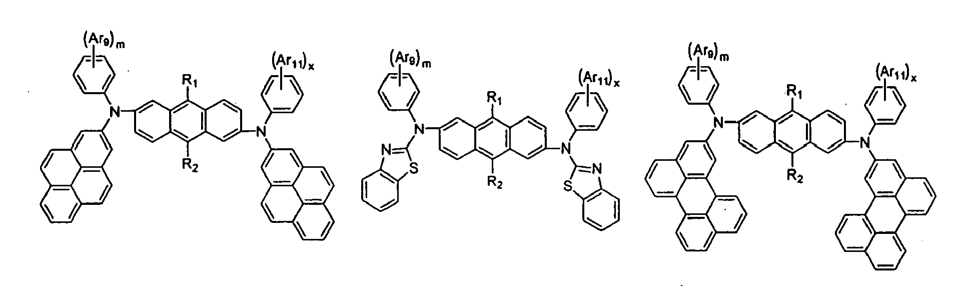 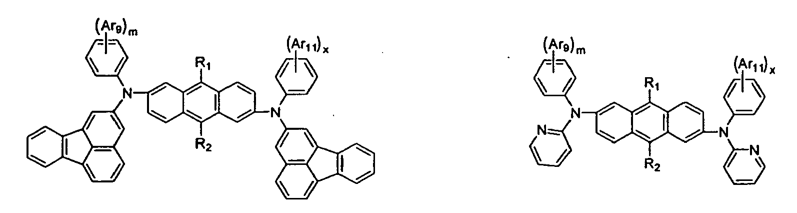 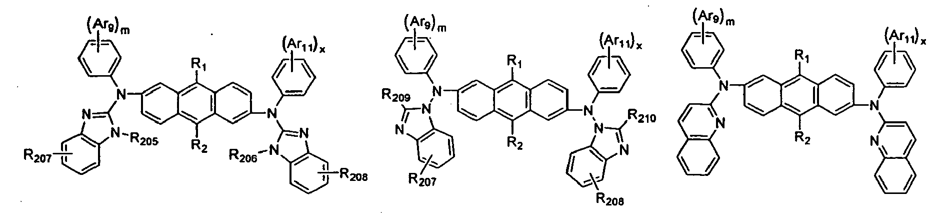 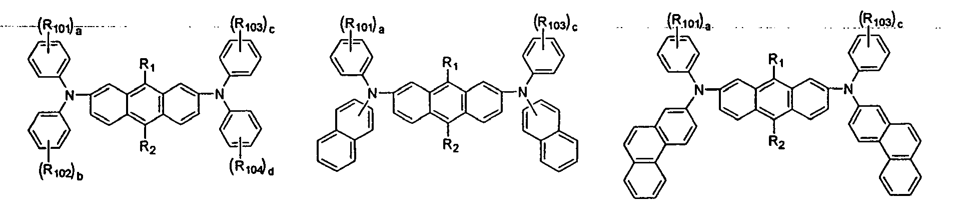 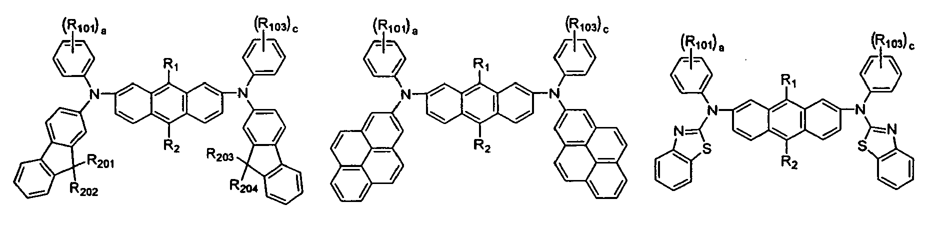 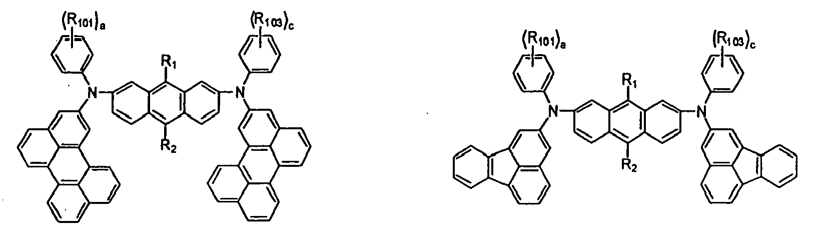 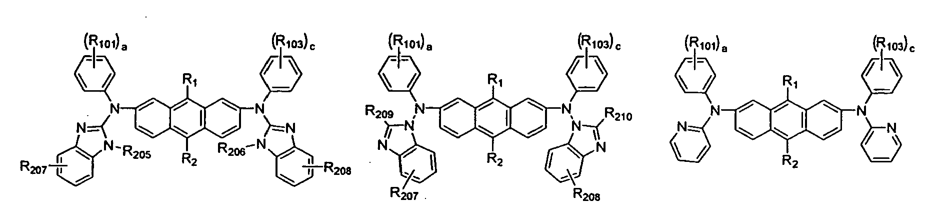 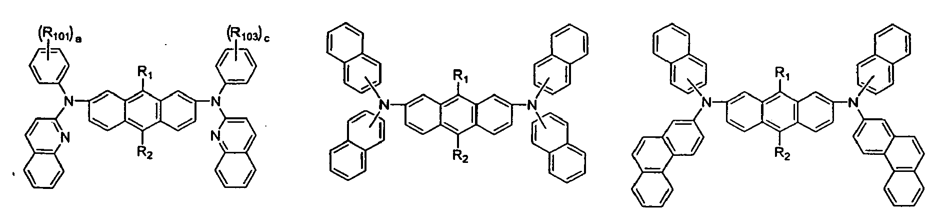 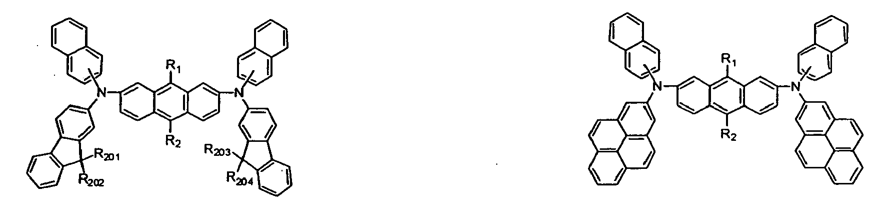 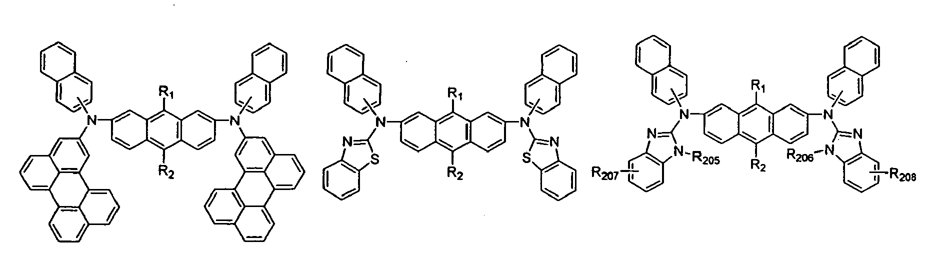 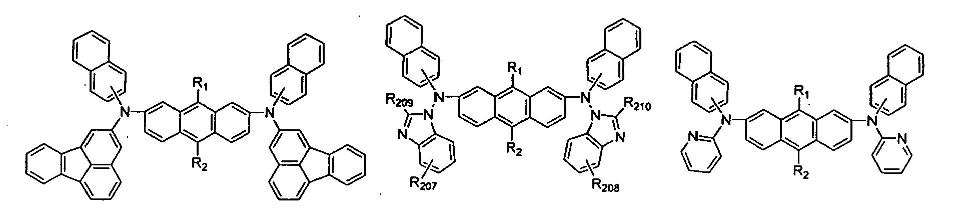 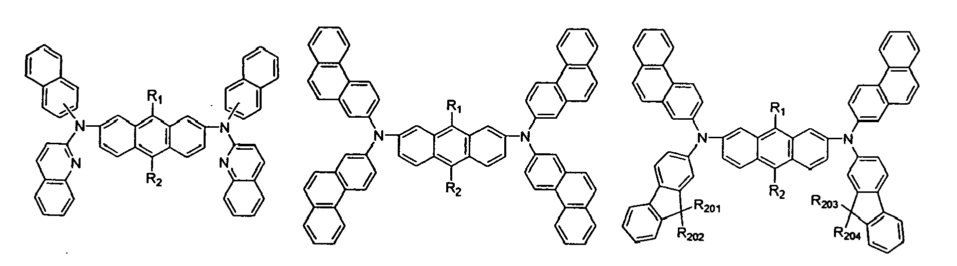 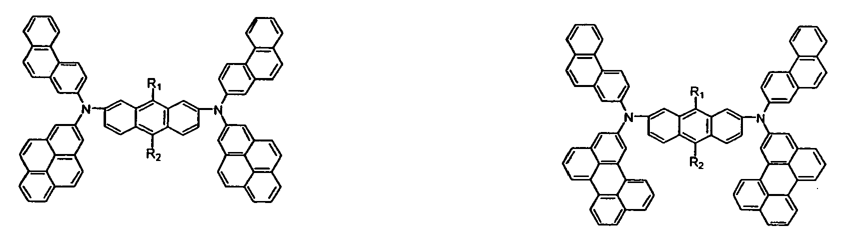 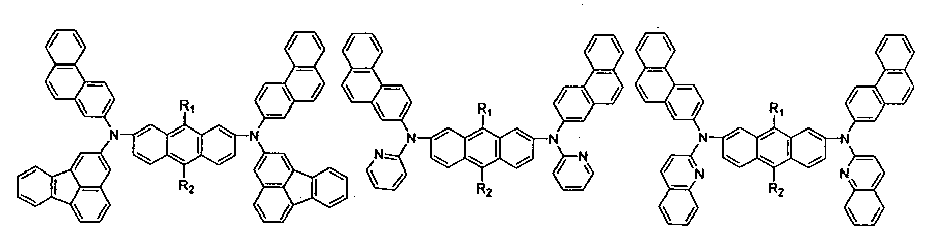 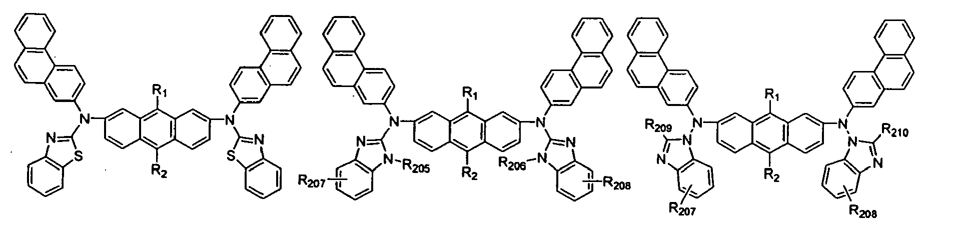 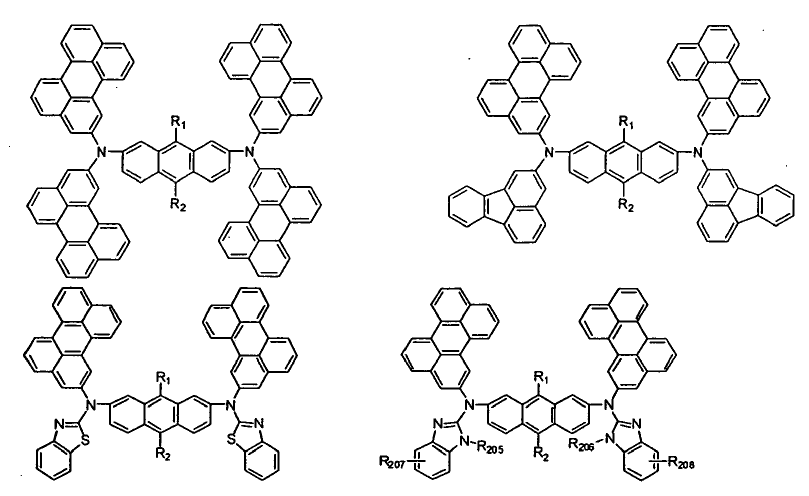 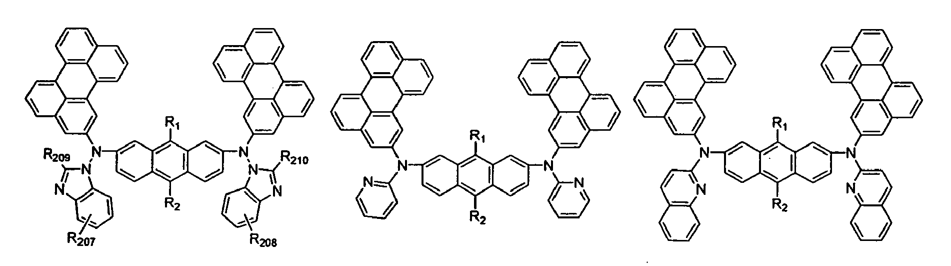 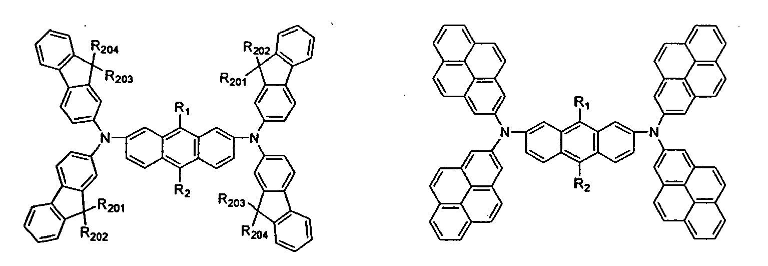 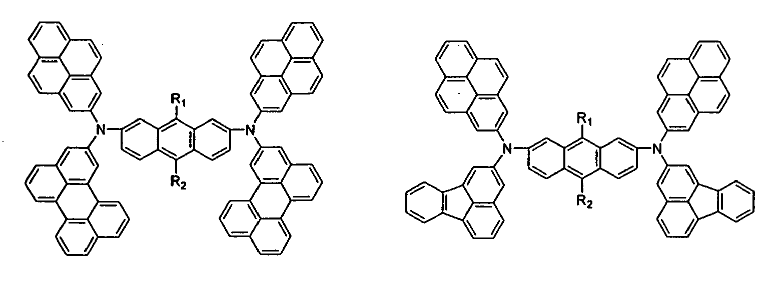 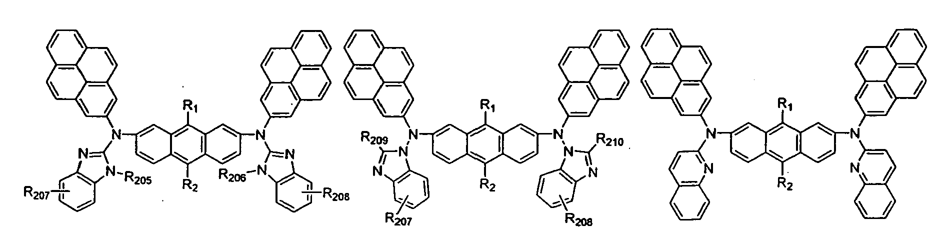 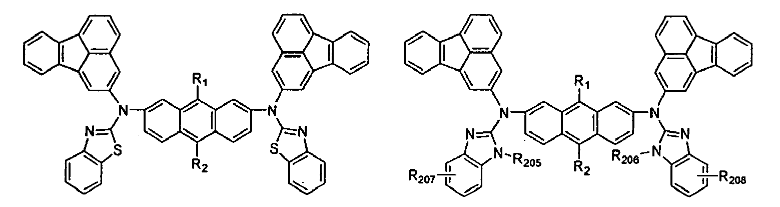 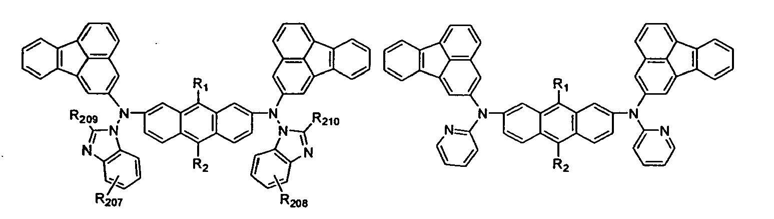 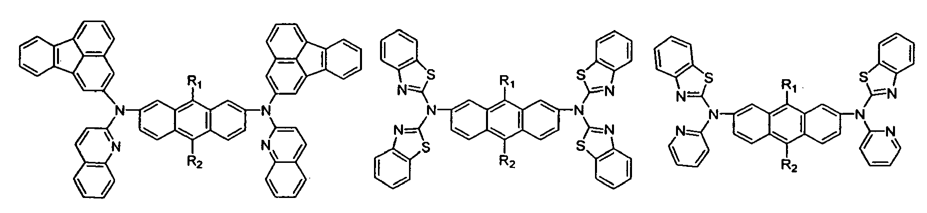 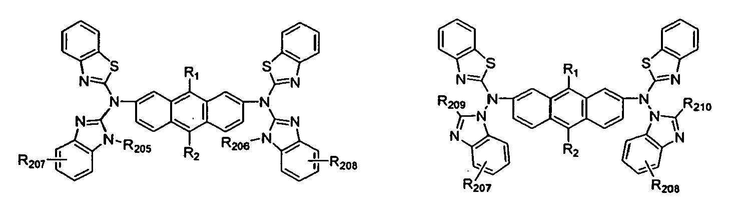 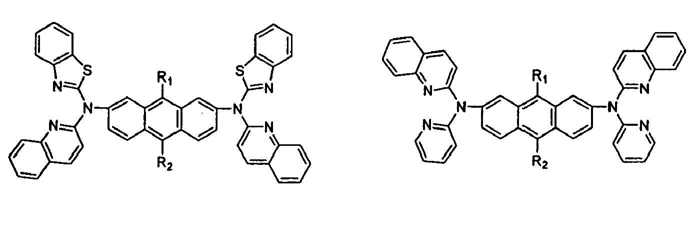 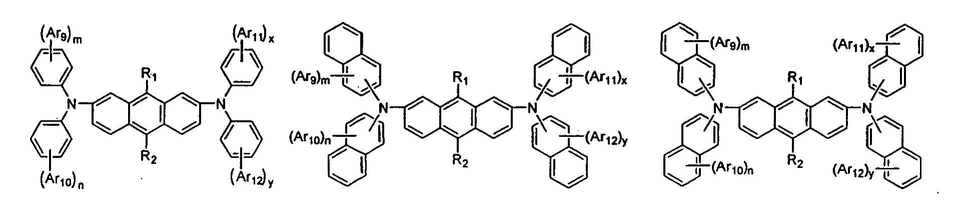 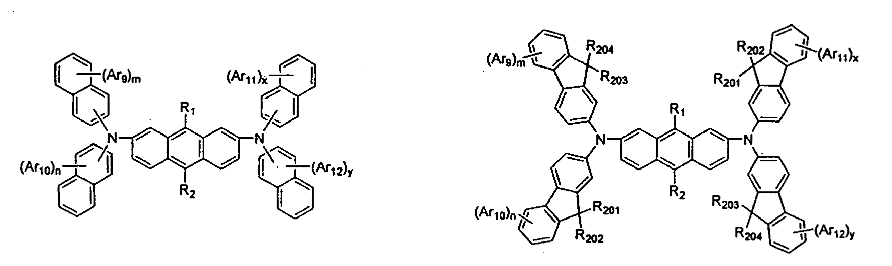 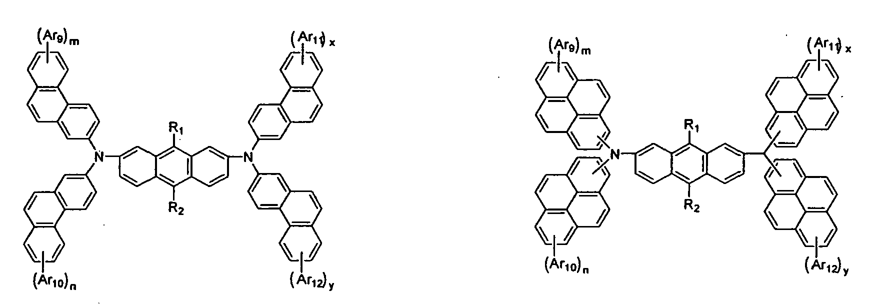 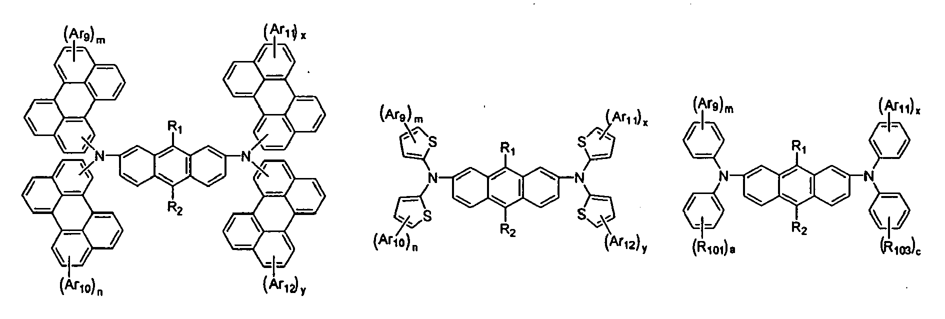 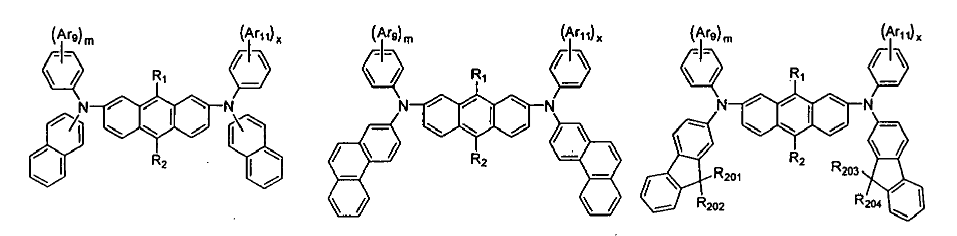 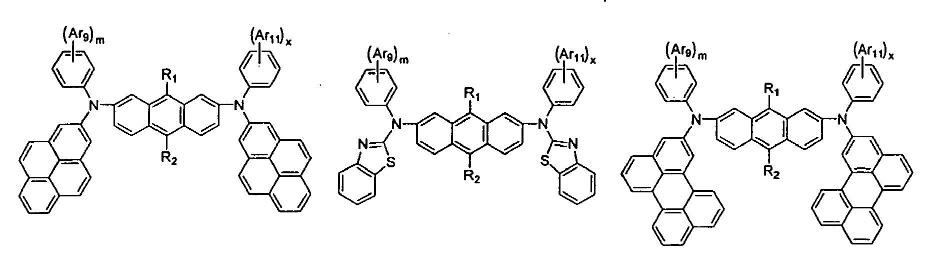 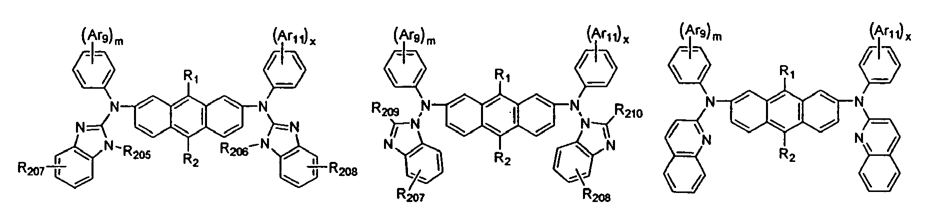
wherein, R₁ and R₂ are defined as in Chemical Formula (1) and Chemical Formula (2);
R₁₀₁, R₁₀₂, R₁₀₃ and R₁₀₄ independently represent methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluorethyl, perfluoropropyl, perfluorobutyl, methoxy, ethoxy, butoxy, hexyloxy, cyclopropyl, cyclopentyl, cyclohexyl, fluoro, cyano, trimethylsilyl, triethylsilyl, tripropylsilyl, tri(t-butyl)silyl, t-butyldimethylsilyl, dimethylphenylsilyl or triphenylsilyl;
R₂₀₁, R₂₀₂, R₂₀₃ and R₂₀₄ independently represent methyl, ethyl, n-propyl, i-propyl, n-butyl, i-butyl, t-butyl, n-pentyl, i-pentyl, n-hexyl, n-heptyl, n-octyl, 2-ethylhexyl, phenyl or naphthyl;
R₂₀₅ and R₂₀₆ independently represent methyl, ethyl, n-propyl, i-propyl, n-butyl, i-butyl, t-butyl, n-pentyl, i-pentyl, n-hexyl, n-heptyl, n-octyl, 2-ethylhexyl, phenyl or naphthyl;
R₂₀₇ and R₂₀₈ independently represent hydrogen, methyl, ethyl, n-propyl, i-propyl, n-butyl, i-butyl, t-butyl, n-pentyl, i-pentyl, n-hexyl, n-heptyl, n-octyl, fluoro or cyano;
R₂₀₉ and R₂₁₀ independently represent methyl, ethyl, n-propyl, i-propyl, n-butyl, i-butyl, t-butyl, n-pentyl, i-pentyl, n-hexyl, n-heptyl, n-octyl, 2-ethylhexyl, phenyl or naphthyl;
Ar₉, Ar₁₀, Ar₁₁ and Ar₁₂ independently represent phenyl, biphenyl, naphthyl, anthryl, fluorenyl, phenanthryl, pyrenyl, perylenyl, fluoranthenyl, pyridyl or quinolyl; the phenyl, naphthyl, anthryl, fluorenyl, phenanthryl, pyrenyl, perylenyl, fluoranthenyl, pyridyl or quinolyl of Ar₉ through Ar₁₂ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluorethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl;
a, b, c and d independently represent an integer from 0 to 4; and
m, n, x and y independently represent an integer from 1 to 3.

The organic electroluminescent compounds according to the present invention can be prepared according to the procedure illustrated by Reaction Scheme (1): 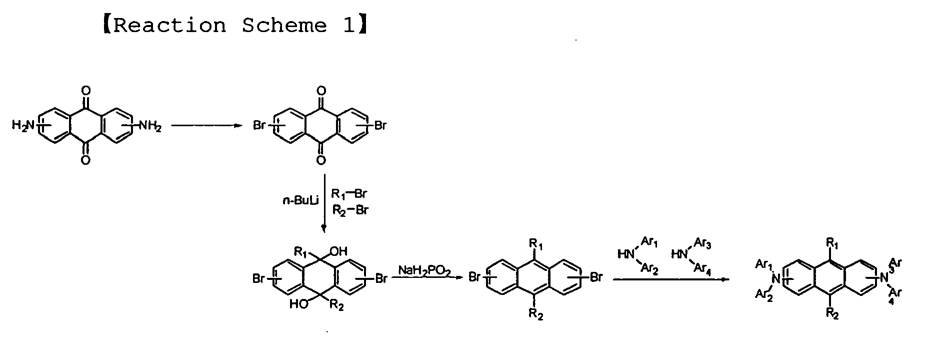 wherein, R₁, R₂, Ar₁, Ar₂, Ar₃ and Ar₄ are defined as in Chemical Formulas (1) and (2).

In addition, the present invention provides organic solar cells, which comprise one or more organic electroluminescent compound(s) represented by Chemical Formula (1) or Chemical Formula (2).

The present invention also provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1) or Chemical Formula (2).

The organic electroluminescent device according to the present invention is **characterized in that** the organic layer comprises an electroluminescent region containing one or more organic electroluminescent compound(s) represented by Chemical Formula (1) or Chemical Formula (2) as electroluminescent dopant, and one or more host(s).

The host applied to the organic electroluminescent device according to the invention is not particularly restricted, but preferably selected from the compounds represented by one of Chemical Formulas (5) to (7): 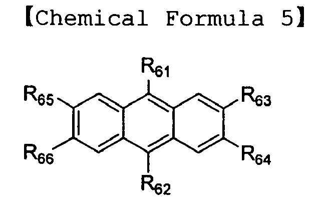 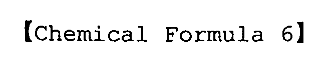 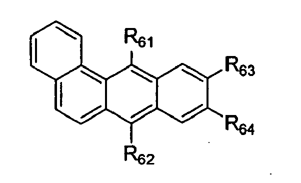 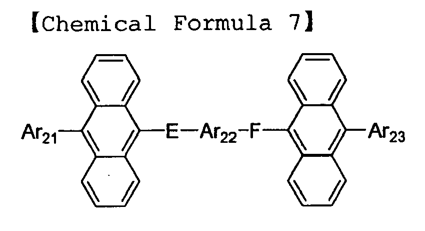
wherein, R₆₁ and R₆₂ independently represent (C6-C60)aryl, (C4-C60)heteroaryl, 5- or 6-membered heterocycloalkyl containing one or more heteroatom(s) selected from N, O and S, or (C3-C60)cycloalkyl; and the aryl or heteroaryl of R₆₁ and R₆₂ may be further substituted by one or more substituent(s) selected from a group consisting of (C1-C60)alkyl, halo(C1-C60)alkyl, (Cl-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₆₃ through R₆₆ represent hydrogen, (C1-C60)alkyl, (C1-C60)alkoxy, halogen, (C4-C60)heteroaryl, (C5-C60)cycloalkyl or (C6-C60)aryl; and the heteroaryl, cycloalkyl or aryl of R₆₃ through R₆₆ may be further substituted by one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
E and F independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₂₁ and Ar₂₃ represent aryl selected from the following structures, or (C4-C60)heteroaryl: 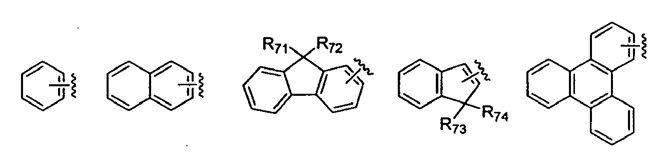 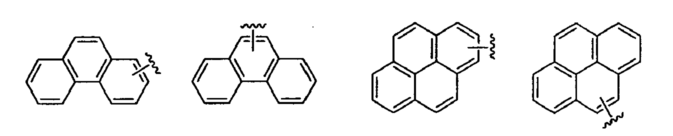 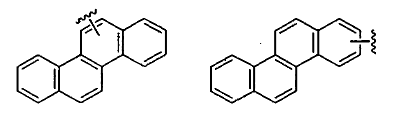
the aryl or heteroaryl of Ar₂₁ and Ar₂₃ may be substituted by one or more substituent(s) selected from (C1-C60)alkyl, (C1-C60)alkoxy, (C6-C60)aryl and (C4-C60)heteroaryl;
Ar₂₂ represents (C6-C60)arylene, (C4-C60)heteroarylene, or a compound represented by the following structural formula: 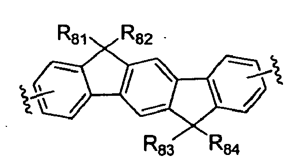
the arylene or heteroarylene of Ar₂₂ may be substituted by one or more substituent(s) selected from (C1-C60)alkyl, (C1-C60)alkoxy, (C6-C60)aryl, (C4-C60)heteroaryl and halogen;
R₇₁ through R₇₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₈₁ through R₈₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

The electroluminescent layer means the layer where electroluminescence occurs, and it may be a single layer or a multi-layer consisting of two or more layers laminated. When a mixture of host-dopant is used according to the constitution of the present invention, noticeable improvement in luminous efficiency due to the inventive electroluminescent host could be confirmed. This can be achieved by the doping concentration of 0.5 to 10% by weight. The host according to the present invention exhibits higher hole and electron conductivity, and excellent stability of material as compared to other conventional host materials, and provides improved device life as well as luminous efficiency.

Thus, it can be described that use of the compound represented by one of Chemical Formulas (5) to (7) as an electroluminescent host significantly supplements electronic drawback of the organic electroluminescent compounds of Chemical Formula (1) according to the present invention.

The host compounds represented by one of Chemical Formulas (5) to (7) can be exemplified by the following compounds, but are not restricted thereto.

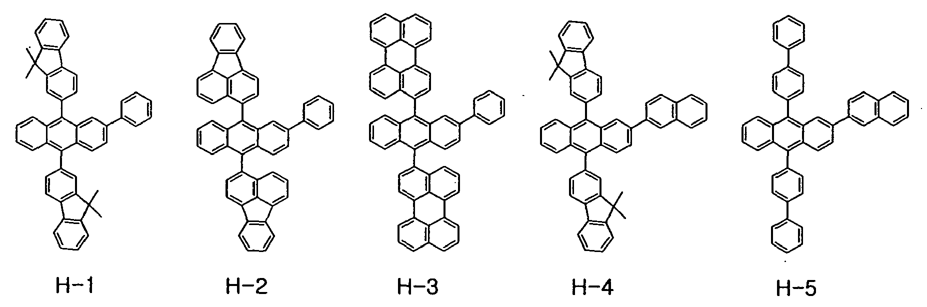

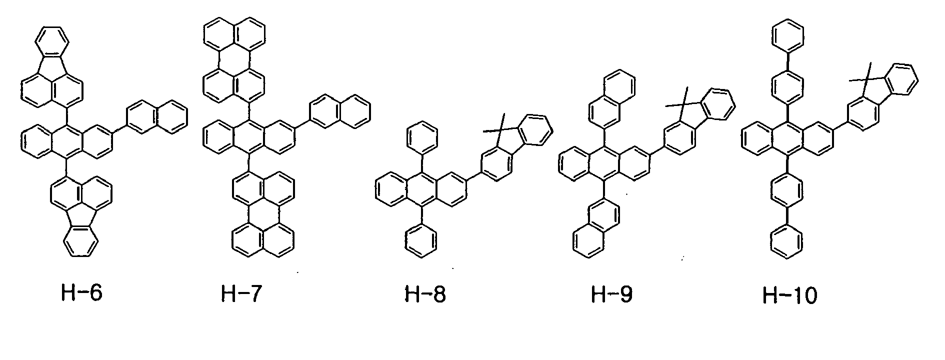

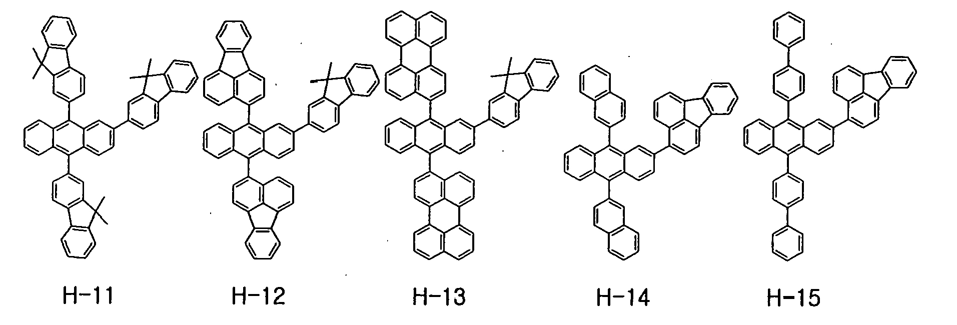

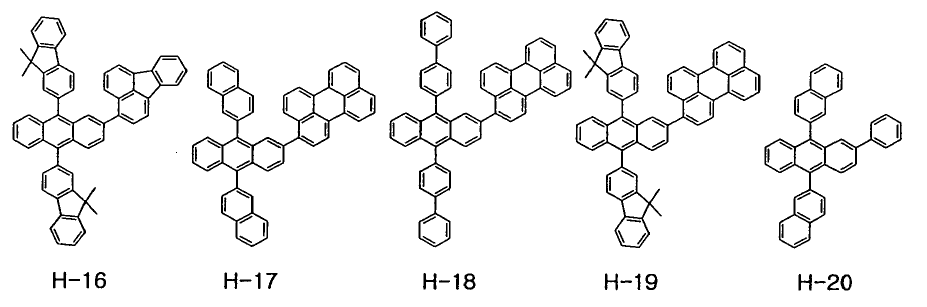

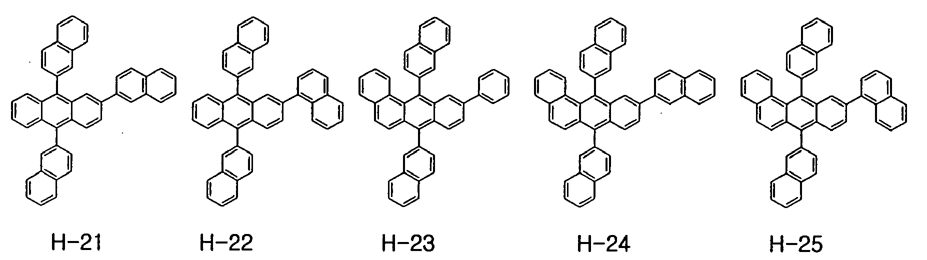

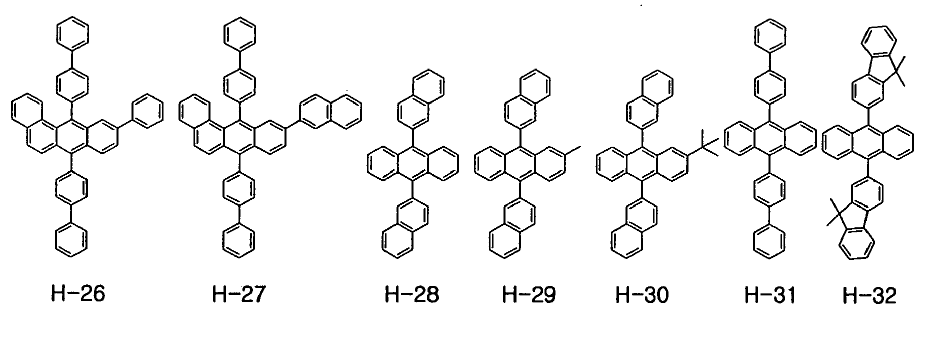

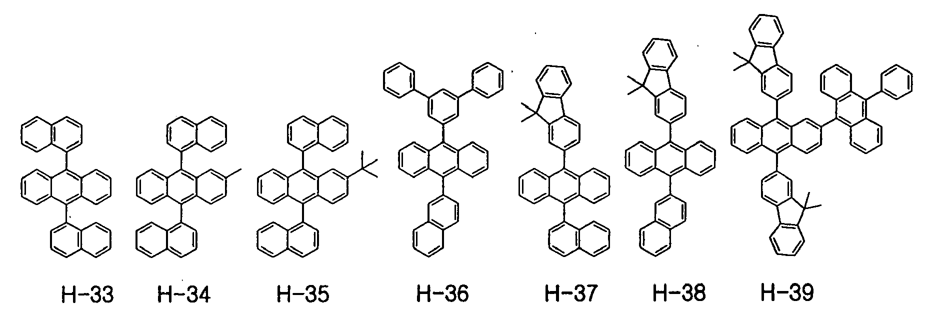

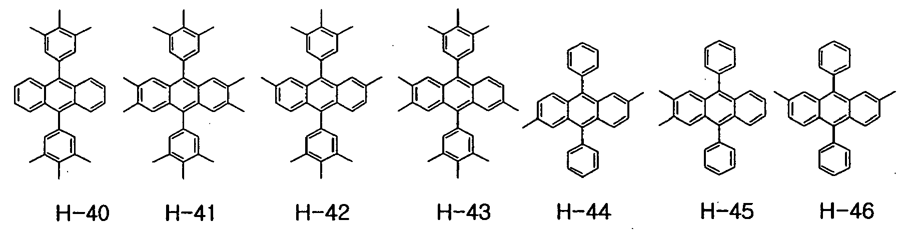

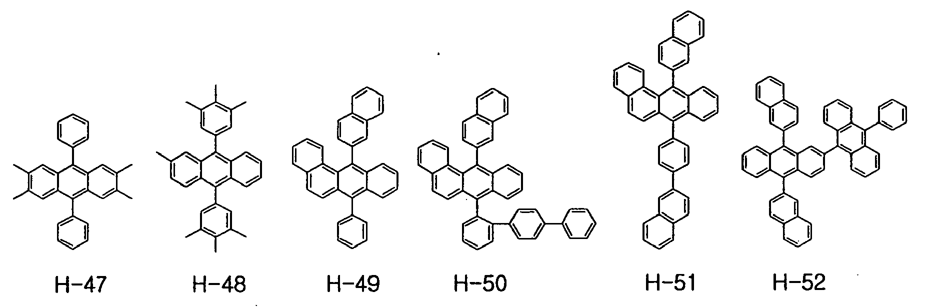

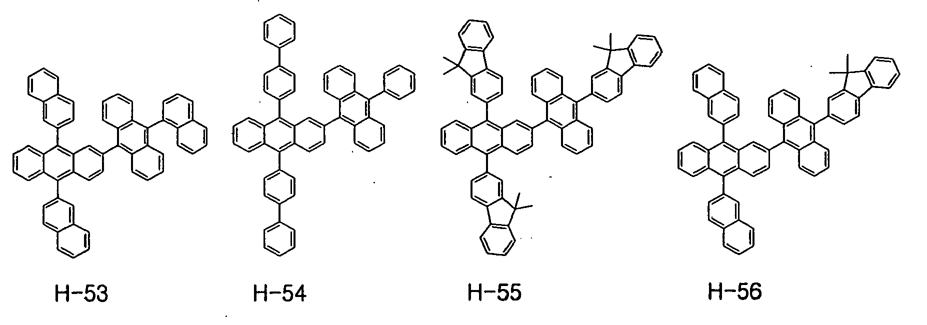

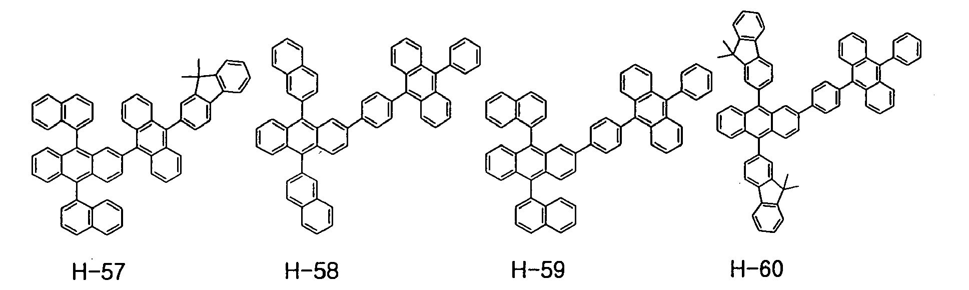

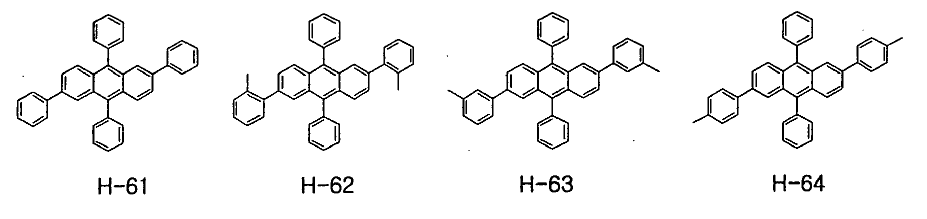

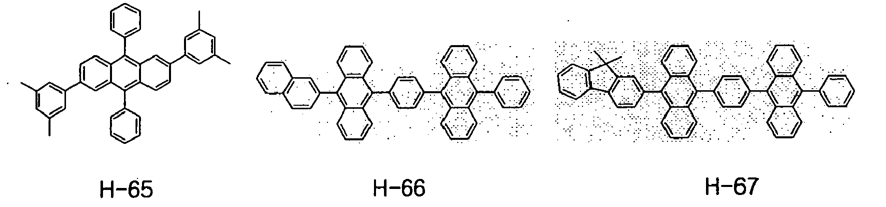

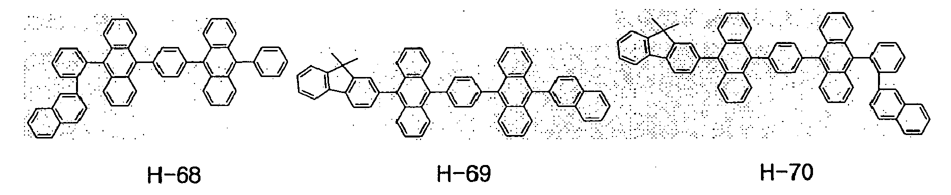

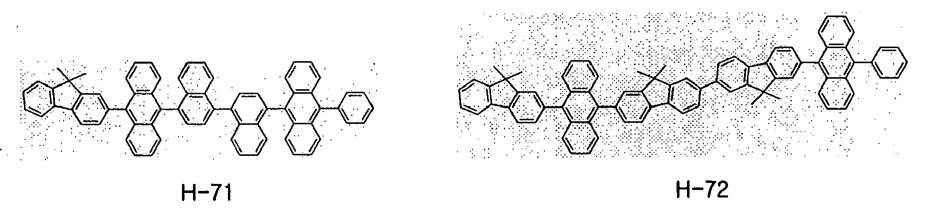

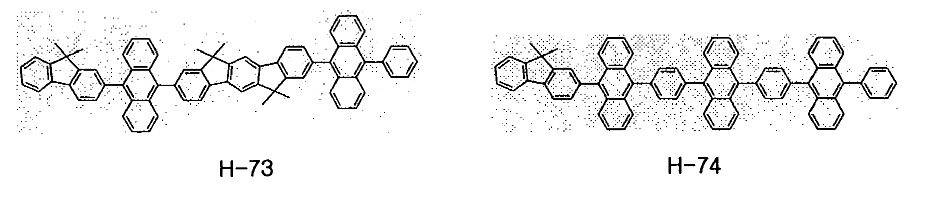

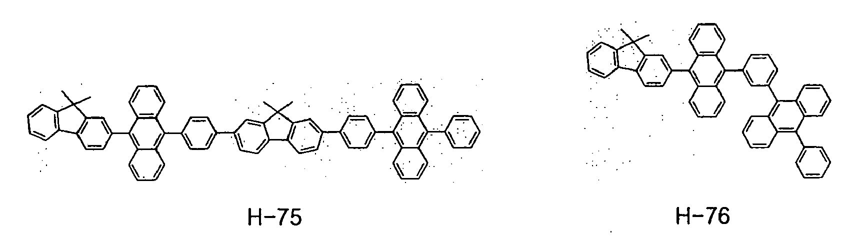

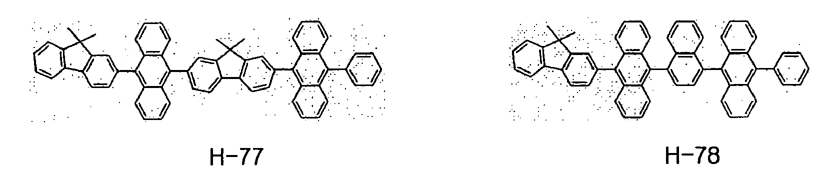

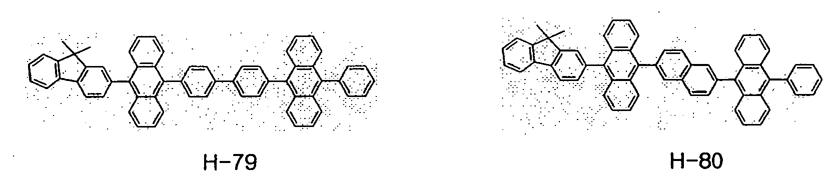

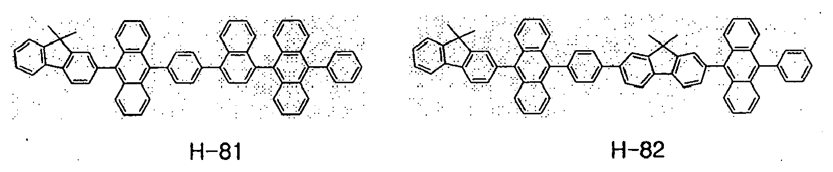

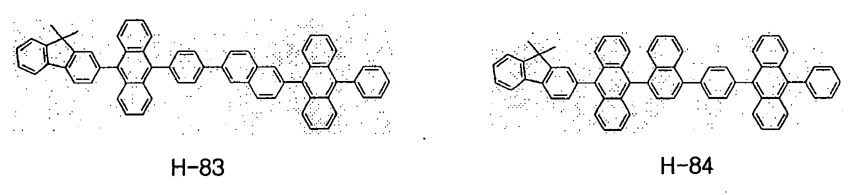

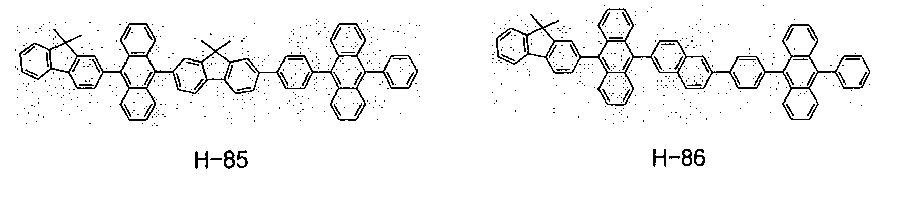

The organic electroluminescent device according to the invention may further comprise one or more compound(s) selected from arylamine compounds and styrylarylamine compounds, as well as the organic electroluminescent compound represented by Chemical Formula (1) or Chemical Formula (2). Examples of the arylamine or styrylarylamine compounds include the compounds represented by Chemical Formula (8), but they are not restricted thereto: 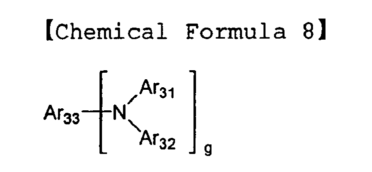
wherein, Ar₃₁ and Ar₃₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₃₁ and Ar₃₂ may be linked via (C3-C60)alkylene or (C3-C60)alkenylene with or without a fused ring to form an alicyclic ring, or a monocyclic or polycyclic aromatic ring; the aryl, heteroaryl, arylamino or heterocycloalkyl of Ar₃₁ and Ar₃₂ may be further substituted by one or more substituent(s) selected from halogen, (C1-C60)alkyl, (C2-C60)alkenyl, (C2-C60)alkynyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
Ar₃₃ represents (C6-C60)aryl, (C5-C60)heteroaryl or (C6-C60)arylamino; the aryl, heteroaryl or arylamino of Ar₃₃ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
g is an integer from 1 to 4.

The arylamine compounds or styrylarylamine compounds may be more specifically exemplified by the following compounds, but they are not restricted thereto.

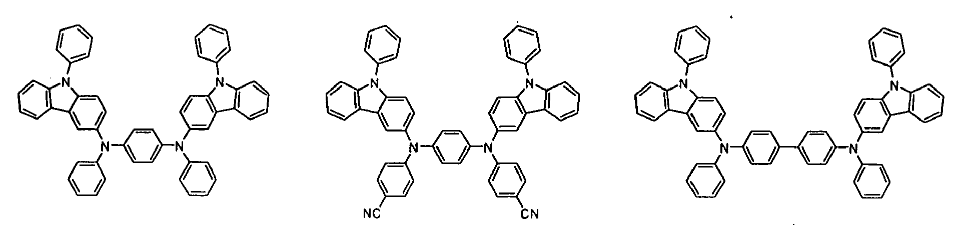

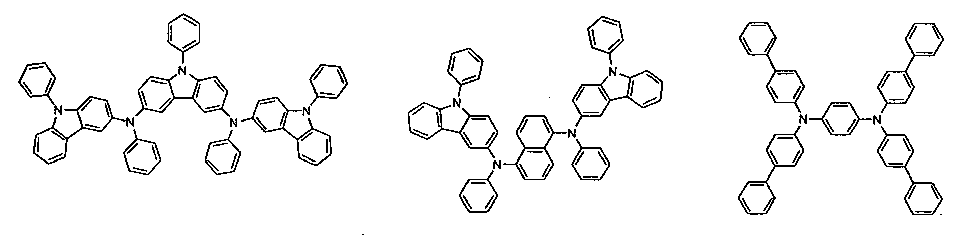

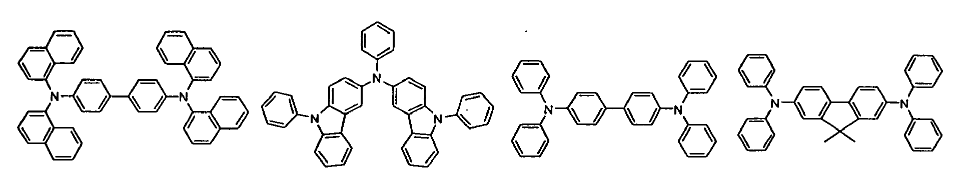

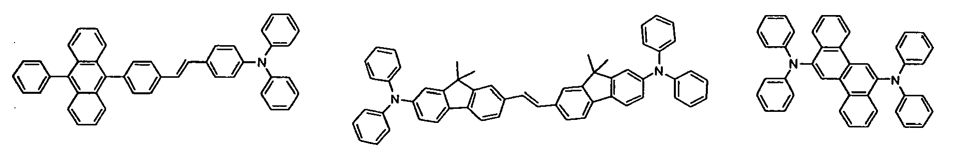

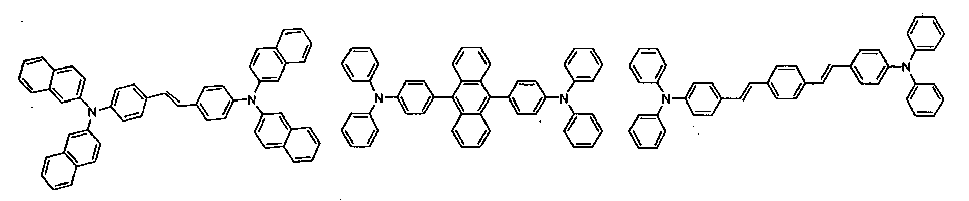

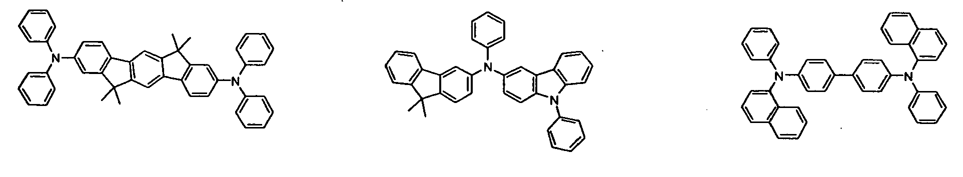

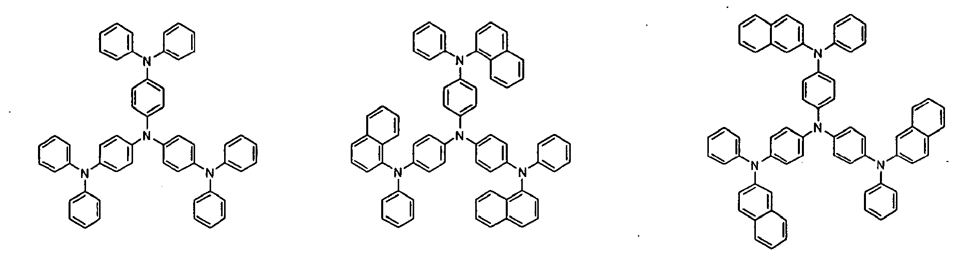

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements from the Periodic Table of Elements, as well as the organic electroluminescent compound represented by Chemical Formula (1) or Chemical Formula (2). The organic layer may comprise a charge generating layer, in addition to an electroluminescent layer.

The present invention can realize an organic electroluminescent device having a pixel structure of independent light-emitting mode, which comprises an organic electroluminescent device containing one of the organic electroluminescent compounds of Chemical Formula (1) or Chemical Formula (2) as a sub-pixel, and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

Further, the organic electroluminescent device is a white electroluminescent device wherein the organic layer comprises, in addition to the organic electroluminescent compound according to the invention, one or more compound(s) selected from compounds having the electroluminescent peak of wavelength of not more than 500 nm, or those having the electroluminescent peak of wavelength of not less than 560 nm, at the same time. Those compounds may be exemplified by the compounds represented by one of Chemical Formulas (9) to (15), but they are not restricted thereto.

[Chemical Formula 9] M¹L¹L²L³

In Chemical Formula (9), M¹ is selected from metals from Group 7, 8, 9, 10, 11, 13, 14, 15 and 16 in the Periodic Table of Elements, and ligands L¹, L² and L³ are independently selected from the following structures: 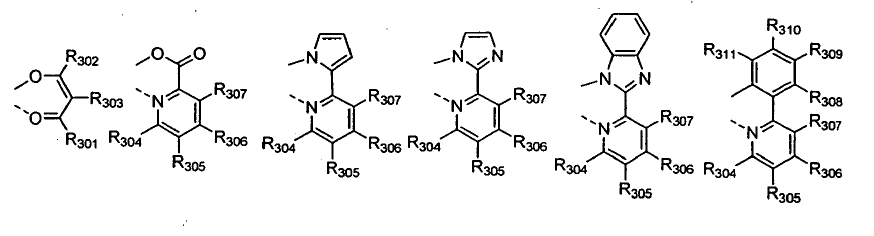 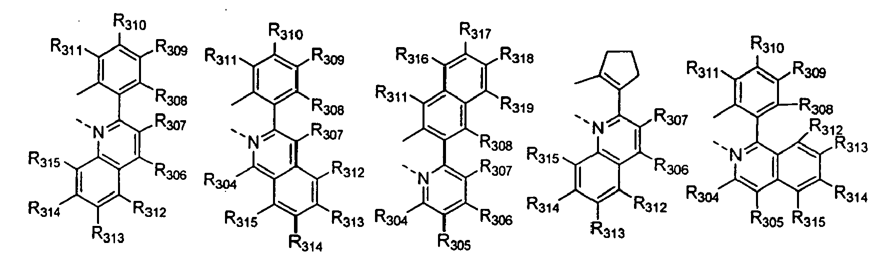 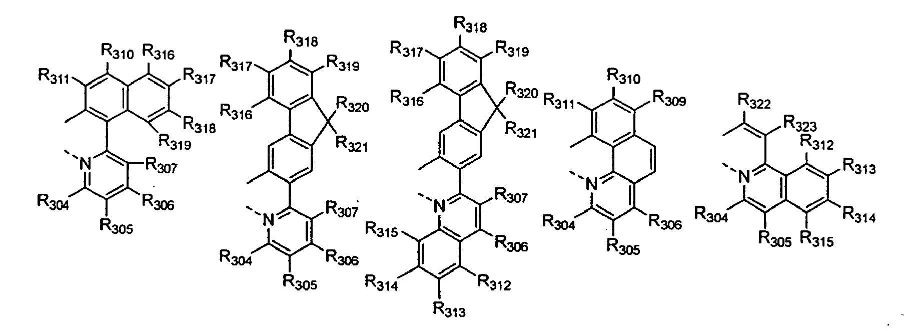 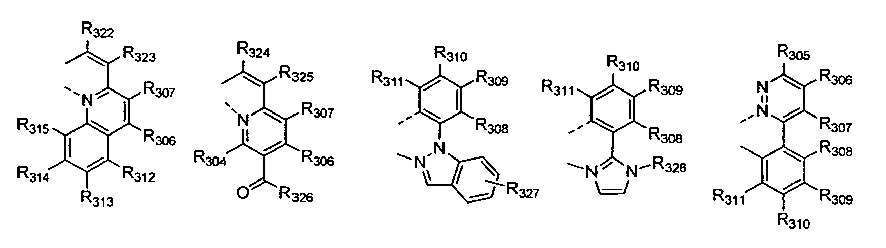 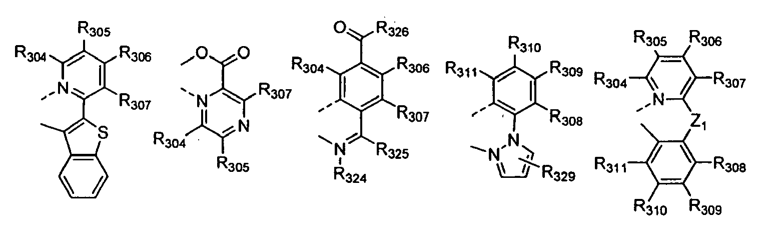
wherein, R₃₀₁ through R₃₀₃ independently represent hydrogen, (C1-C60)alkyl with or without halogen substituent(s), (C6-C60)aryl with or without (C1-C60)alkyl substituent(s), or halogen;
R₃₀₄ through R₃₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen; and the alkyl, cycloalkyl, alkenyl or aryl of R₃₀₄ through R₃₁₉ may be further substituted by one or more substituent(s) selected from (C1-C60)alkyl, (C6-C60)aryl and halogen;
R₃₂₀ through R₃₂₃ independently represent hydrogen, (C1-C60)alkyl with or without halogen substituent(s), or (C6-C60)aryl with or without (C1-C60)alkyl substituent(s);
R₃₂₄ and R₃₂₅ independently represent hydrogen, linear or branched (C1-C60)alkyl, (C6-C60)aryl or halogen, or R₃₂₄ and R₃₂₅ may be linked via (C3-C12)alkylene or (C3-C12)alkenylene with or without a fused ring to form an alicyclic ring, or a monocyclic or polycyclic aromatic ring; and the alkyl or aryl of R₃₂₄ and R₃₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from linear or branched (C1-C60)alkyl with or without halogen substituent(s), (C1-C30)alkoxy, halogen, tri(C1-C30)alkylsilyl, tri(C6-C30)arylsilyl and (C6-C60)aryl;
R₃₂₆ represents (C1-C60)alkyl, (C6-C60) aryl, (C5-C60)heteroaryl or halogen;
R₃₂₇ through R₃₂₉ independently represent hydrogen, (C1-C60)alkyl, (C6-C60)aryl or halogen, and the alkyl or aryl of R₃₂₆ through R₃₂₉ may be further substituted by halogen or (C1-C60)alkyl;
Z₁ represents 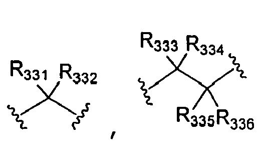 or 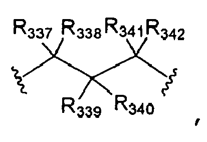 and R₃₃₁ through R₃₄₂ independently represent hydrogen, (C1-C60)alkyl with or without halogen substituent(s), (C1-C30)alkoxy, halogen, (C6-C60)aryl, cyano or (C5-C60)cycloalkyl, or each of R₃₃₁ through R₃₄₂ may be linked to an adjacent substituent via alkylene or alkenylene to form a (C5-C7) spiro-ring or (C5-C9) fused ring, or each of them may be linked to R₃₀₇ or R₃₀₈ via alkylene or alkenylene to form a (C5-C7) fused ring.

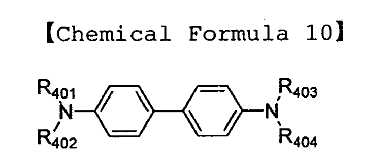

In Chemical Formula (10), R₄₀₁ through R₄₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₄₀₁ through R₄₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

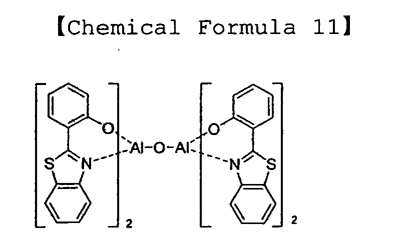

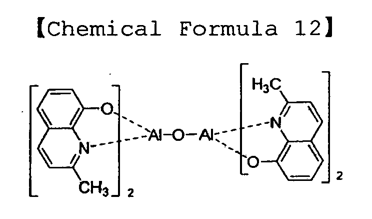

[Chemical Formula 13] L⁴L⁵M²(Q)ₕ

In Chemical Formula (13), the ligands, L⁴ and L⁵ are independently selected from the following structures: 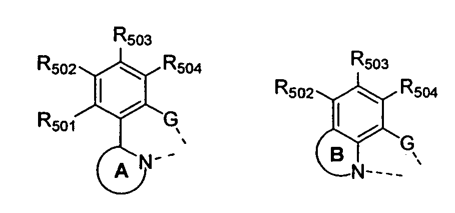
wherein, M² is a bivalent or trivalent metal;
h is 0 when M² is a bivalent metal, while h is 1 when M² is a trivalent metal;
Q represents (C6-C60)aryloxy or tri(C6-C60)arylsilyl, and the aryloxy and triarylsilyl of Q may be further substituted by (C1-C60)alkyl or (C6-C60)aryl;
G represents O, S or Se;
ring A represents oxazole, thiazole, imidazole, oxadiazole, thiadiazole, benzoxazole, benzothiazole, benzimidazole, pyridine or quinoline;
ring B represents pyridine or quinoline, and ring B may be further substituted by (C1-C60)alkyl, or phenyl or naphthyl with or without (C1-C60)alkyl substituent(s);
R₅₀₁ through R₅₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₅₀₁ to form a fused ring;
ring A or the aryl group of R₅₀₁ through R₅₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

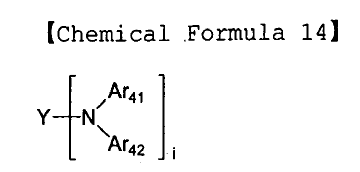

In Chemical Formula (14), Ar₄₁ and Ar₄₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, 0 and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring; the alkyl, aryl, heteroaryl, arylamino, alkylamino, cycloalkyl or heterocycloalkyl of Ar₄₁ and Ar₄₂ may be further substituted by one or more substituent(s) selected from a group consisting of halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
Y represents (C6-C60)arylene, (C4-C60)heteroarylene or arylene represented by one of the following structural formulas: 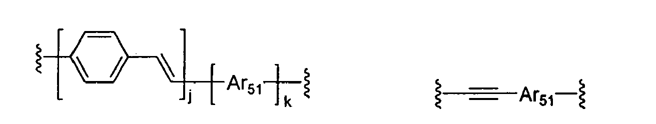
wherein, Ar₅₁ represents (C6-C60)arylene or (C4-C60)heteroarylene,
the arylene or heteroarylene of Y and Ar₅₁ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
i is an integer from 1 to 4;
j is an integer from 1 to 4; and
k is an integer of 0 or 1.

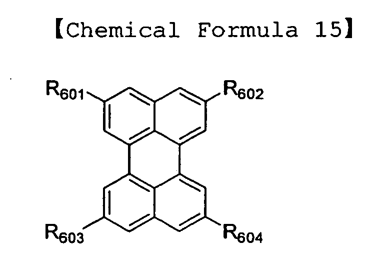

In Chemical Formula (15), R₆₀₁ through R₆₀₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₆₀₁ through R₆₀₄ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino or arylamino of R₆₀₁ through R₆₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compounds having electroluminescent peak of wavelength of not more than 500 nm, or those having electroluminescent peak of wavelength of not less than 560 nm, can be exemplified by the following compounds, but they are not restricted thereto.

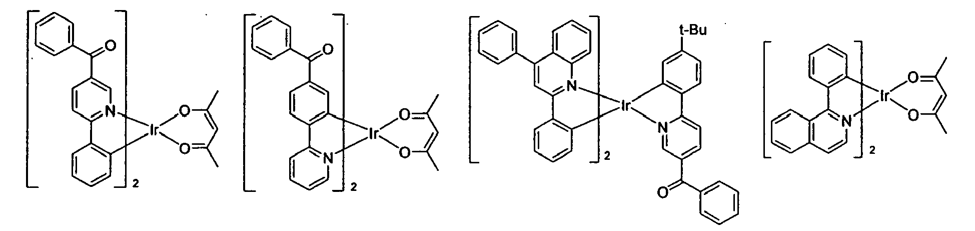

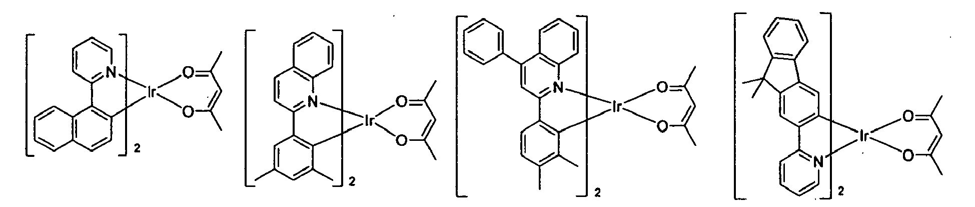

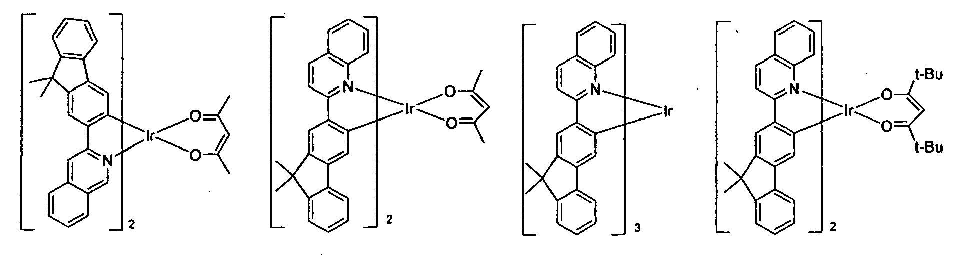

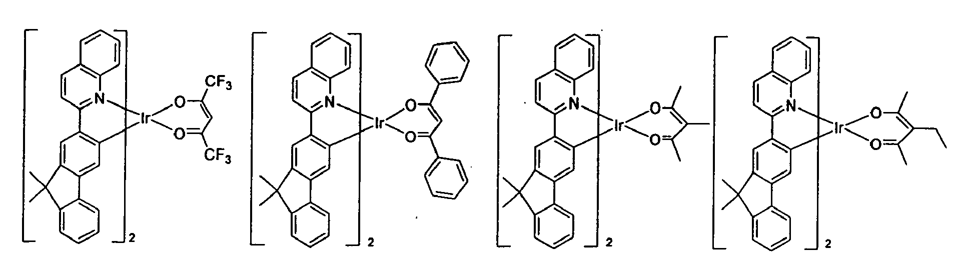

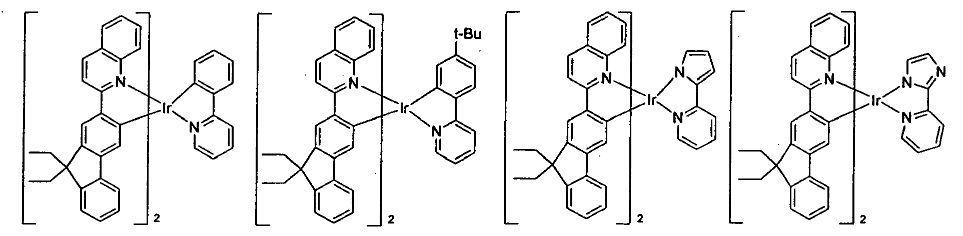

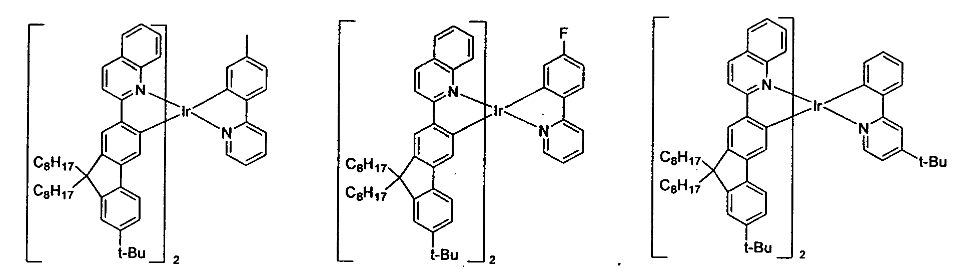

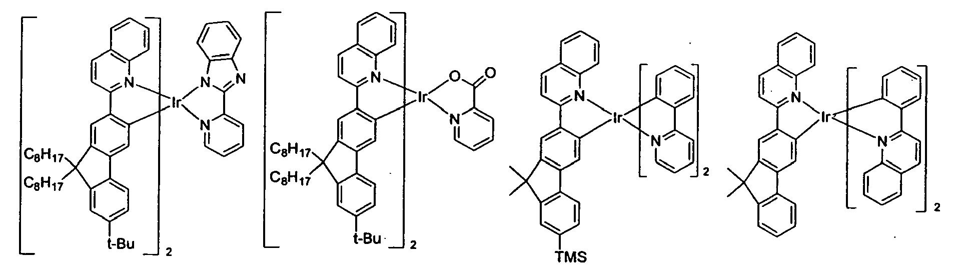

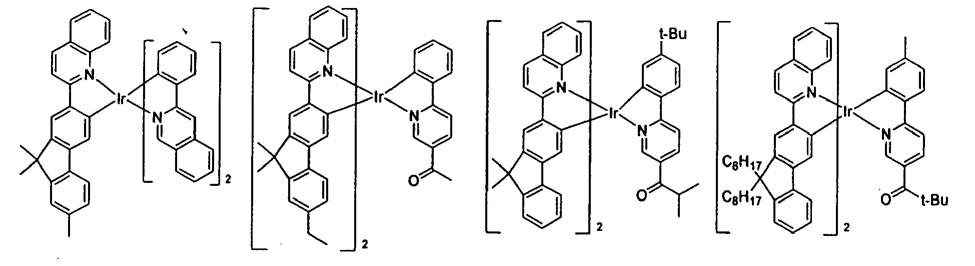

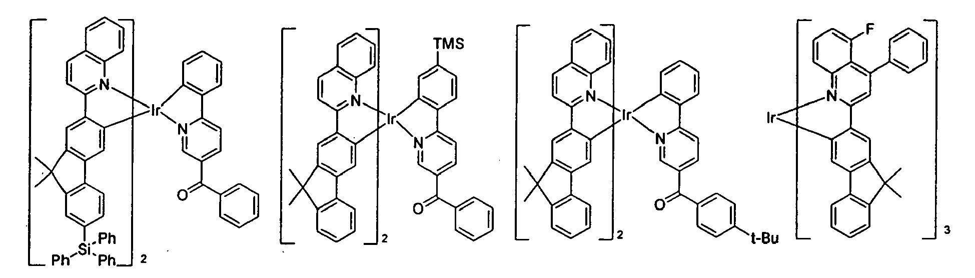

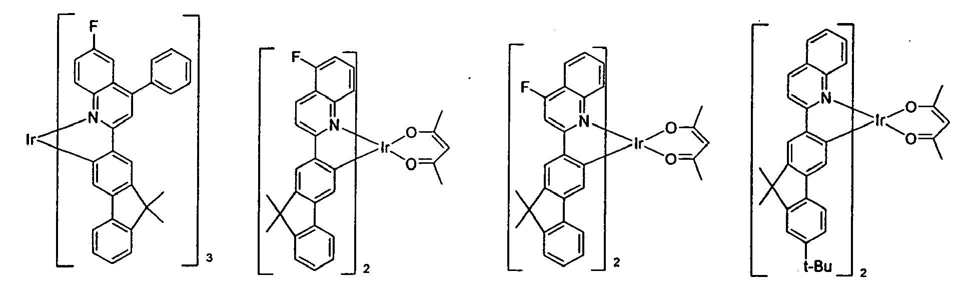

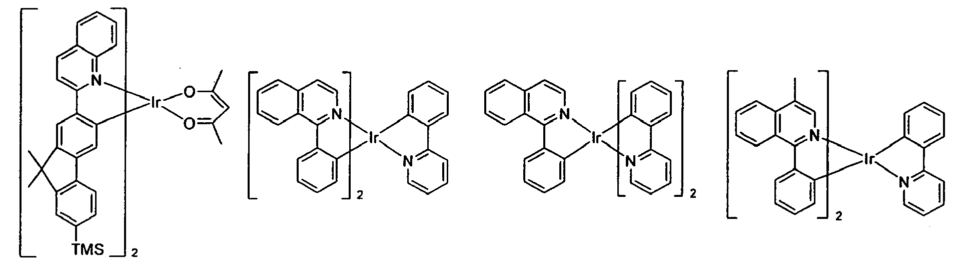

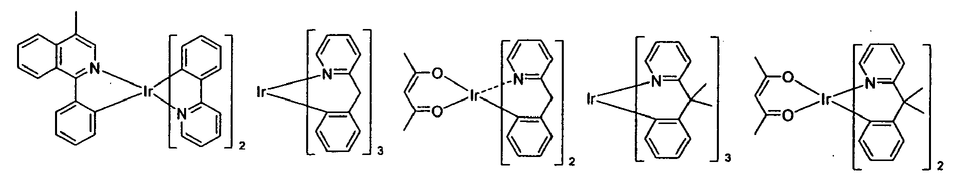

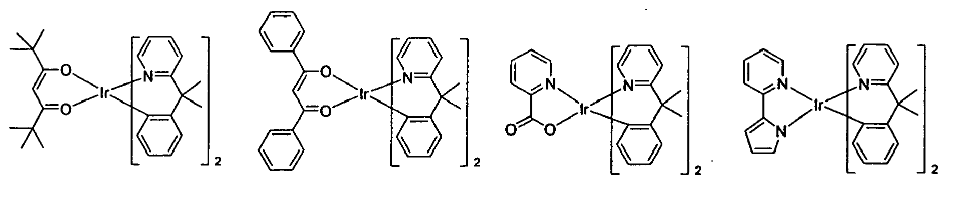

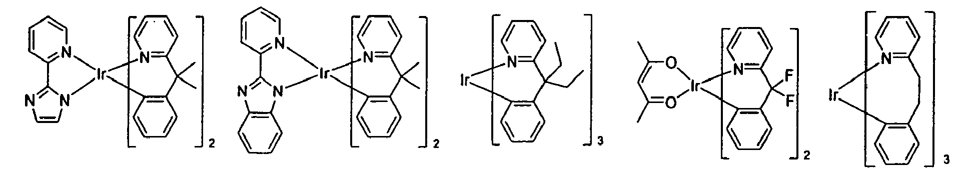

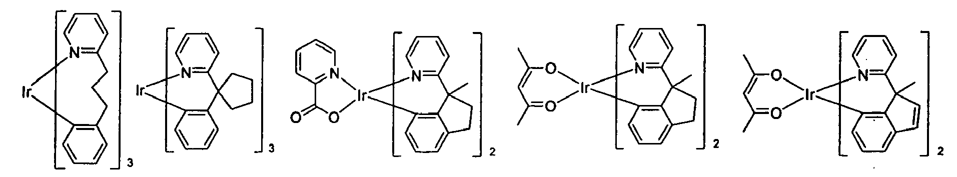

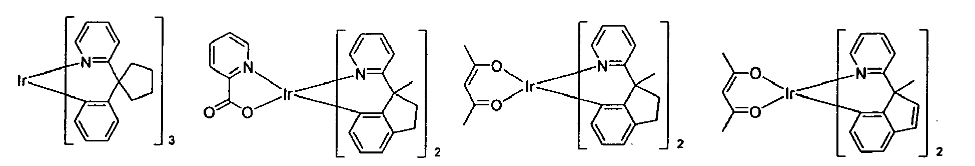

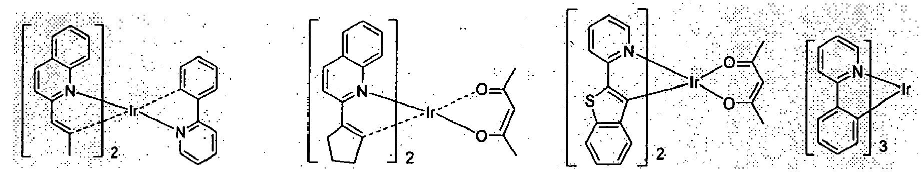

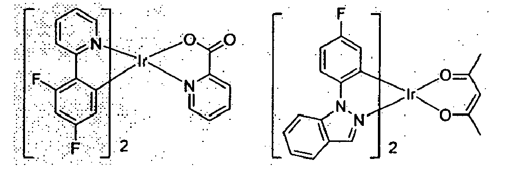

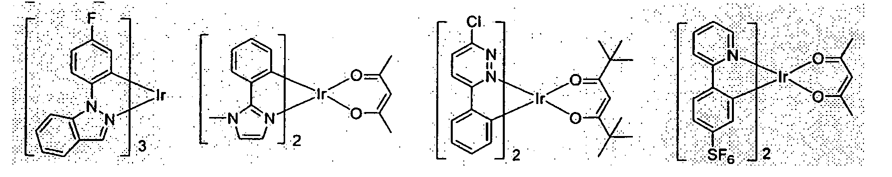

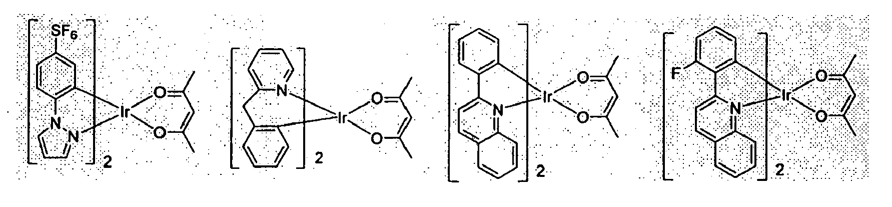

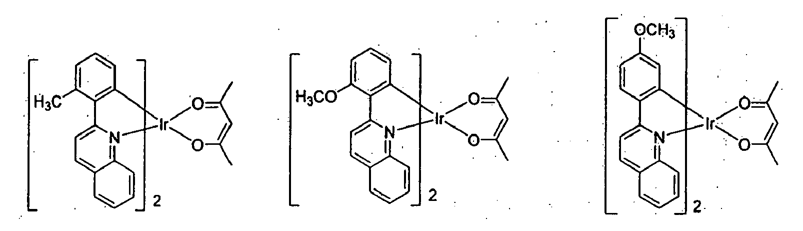

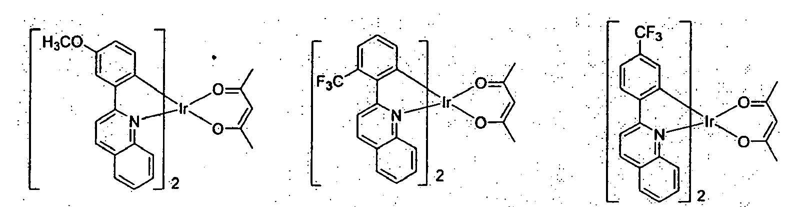

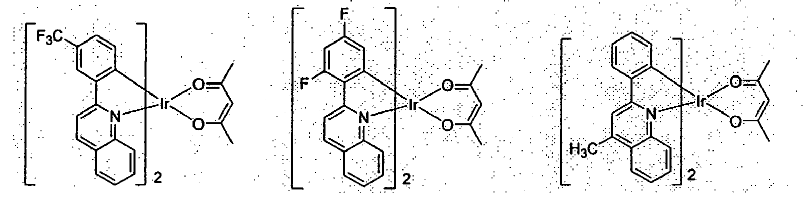

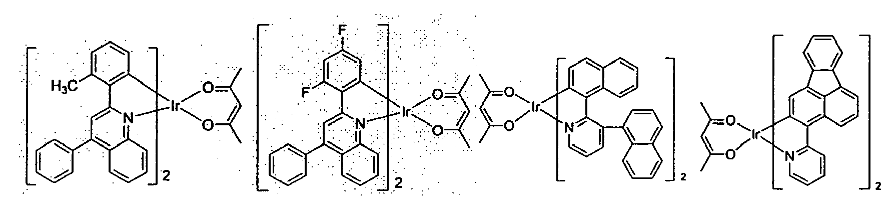

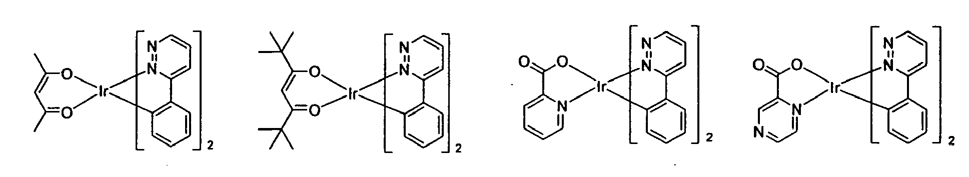

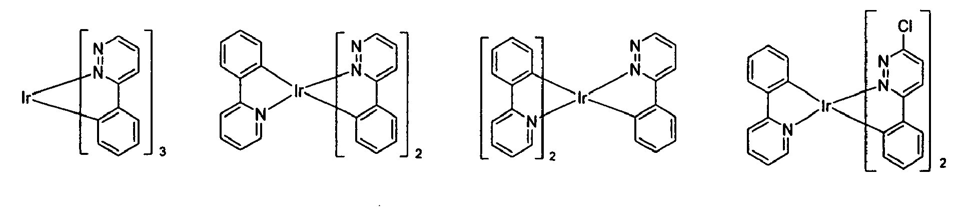

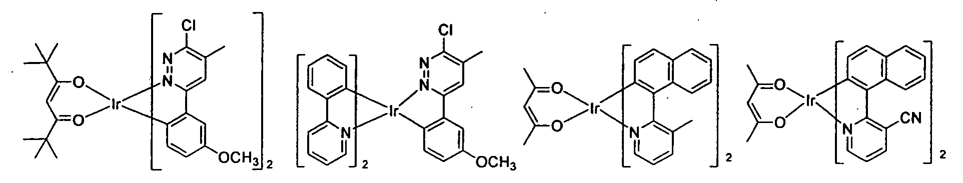

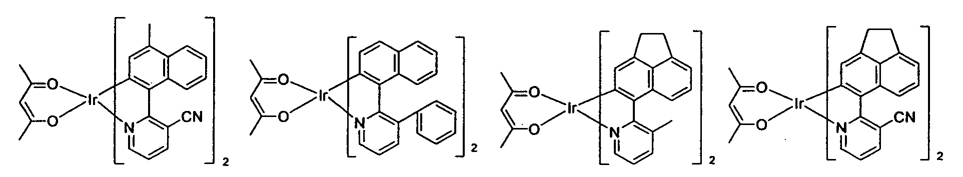

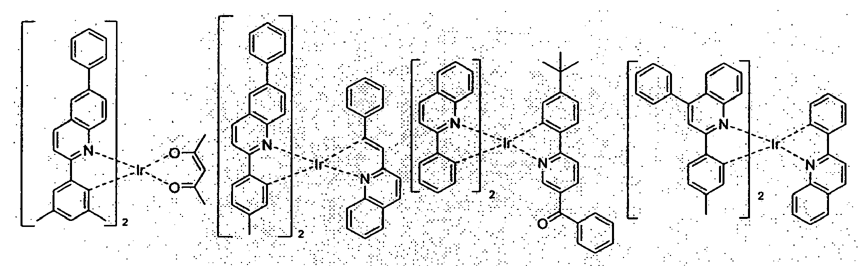

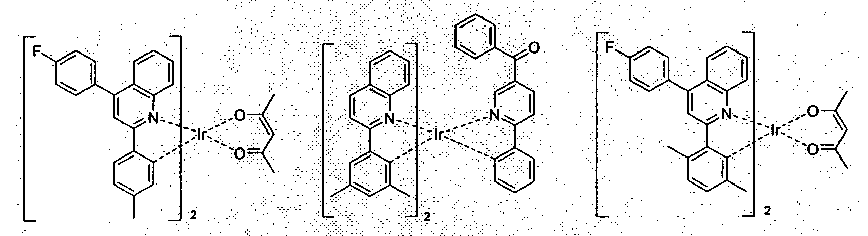

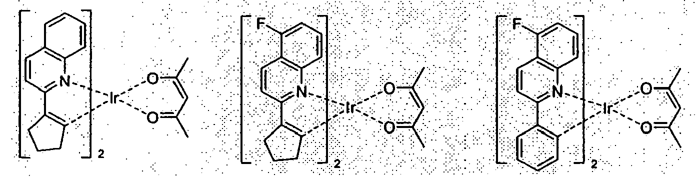

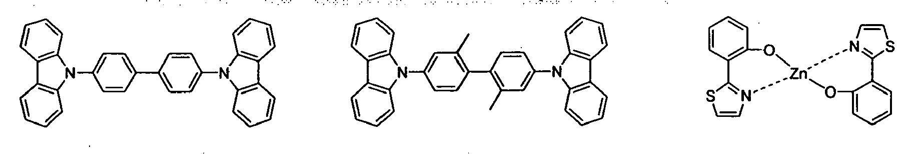

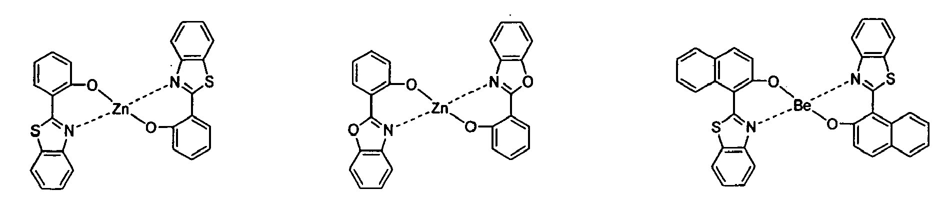

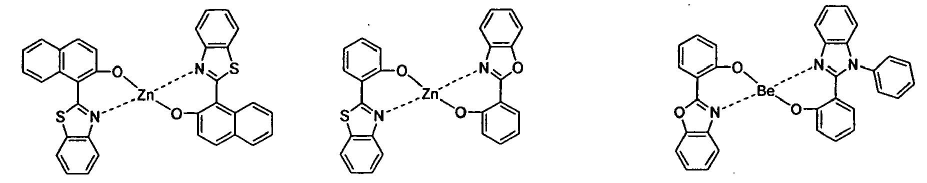

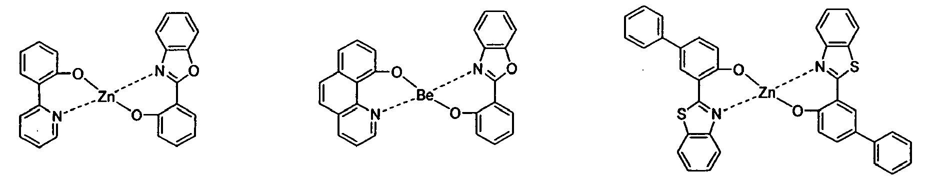

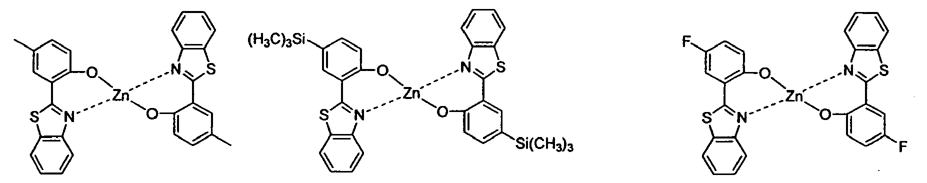

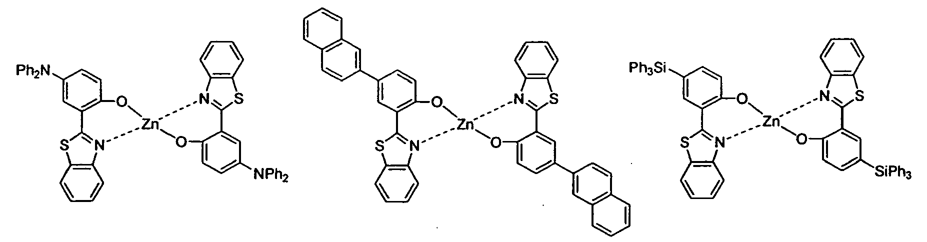

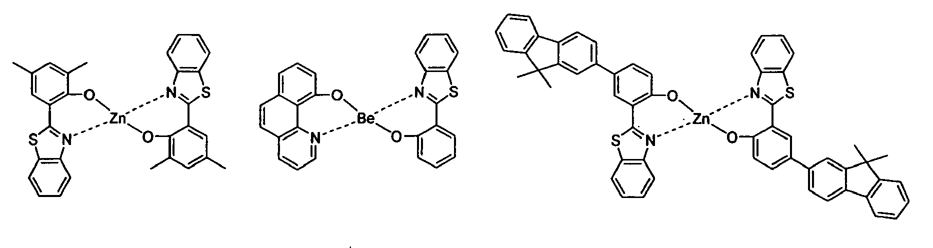

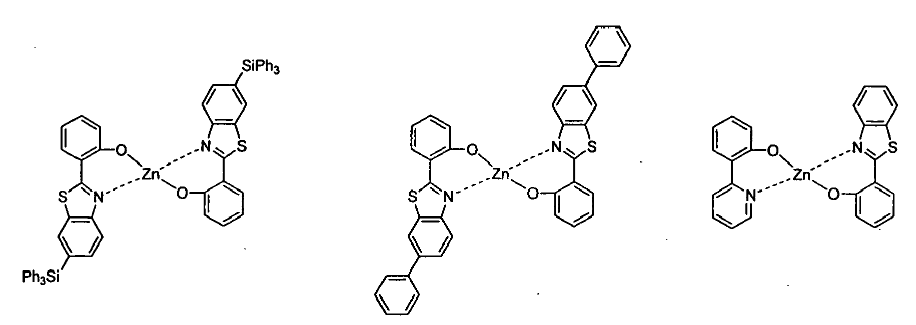

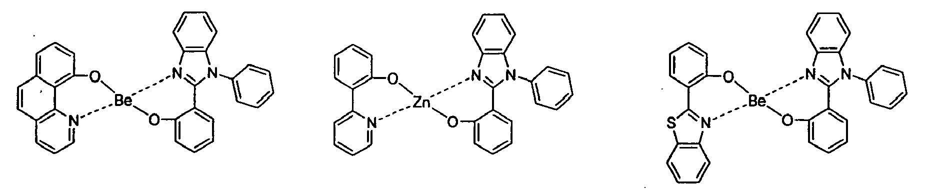

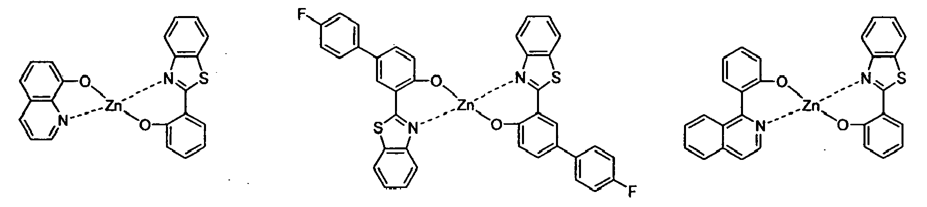

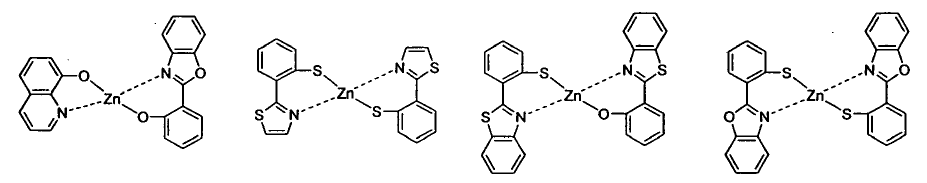

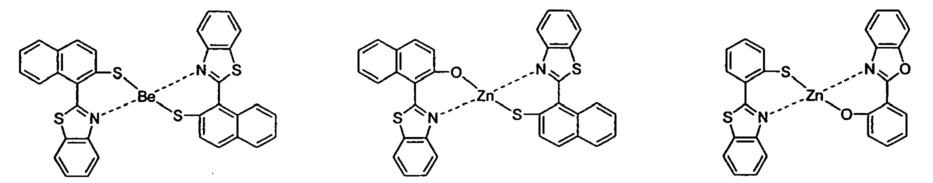

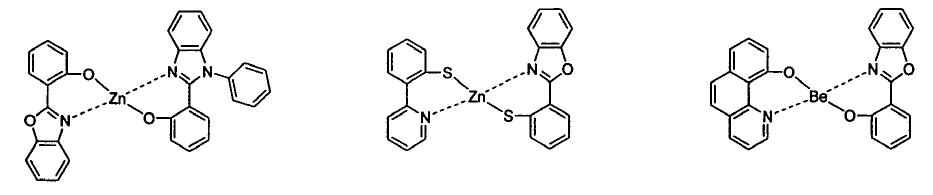

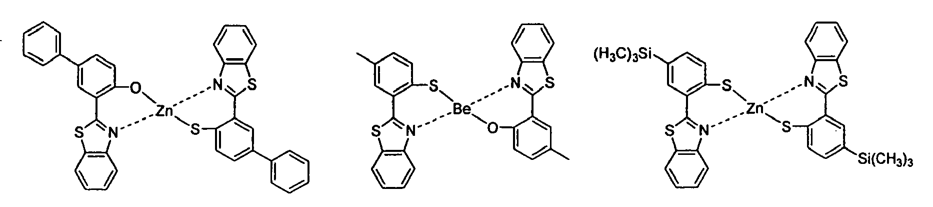

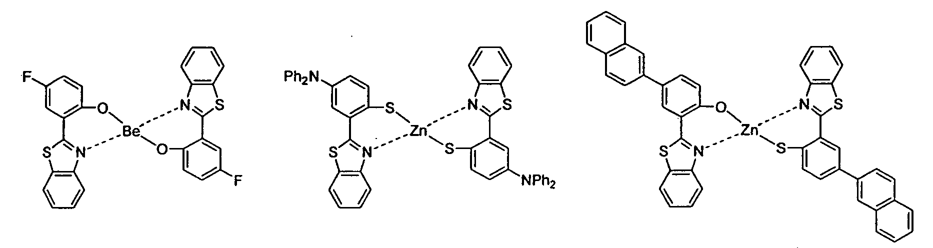

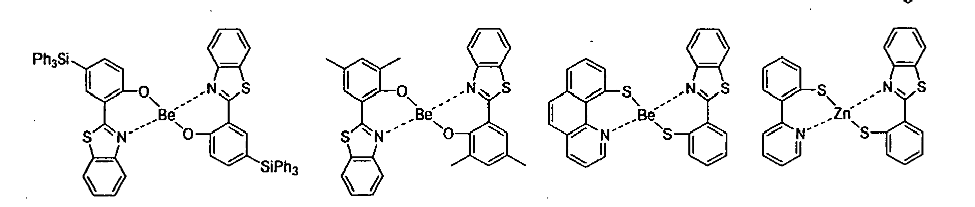

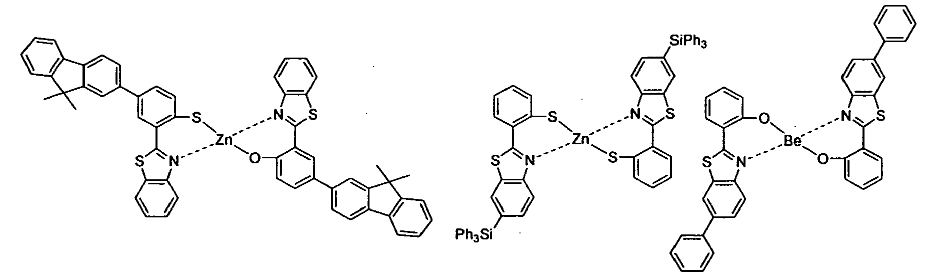

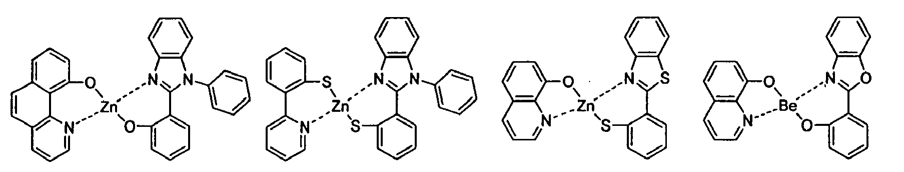

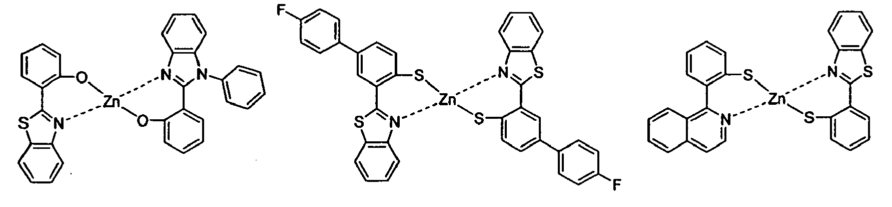

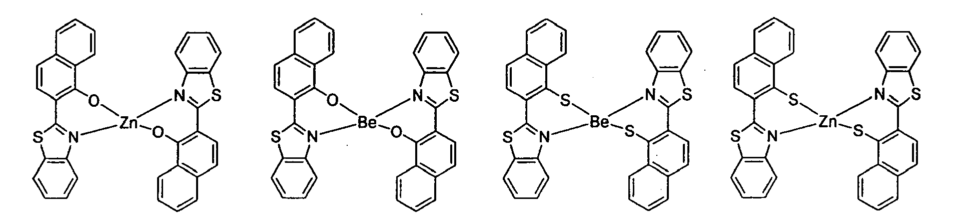

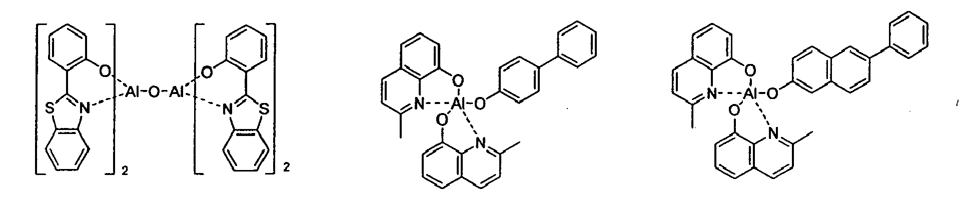

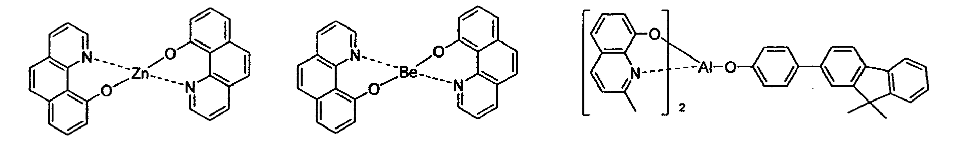

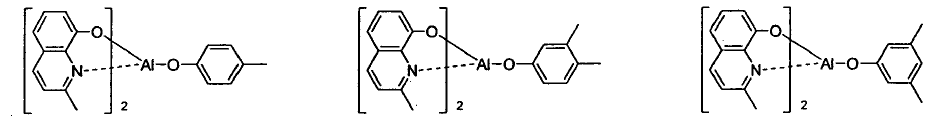

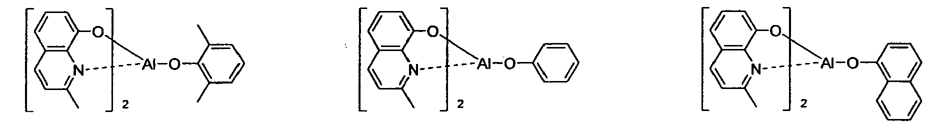

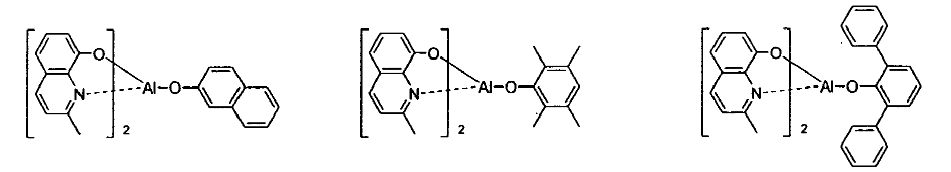

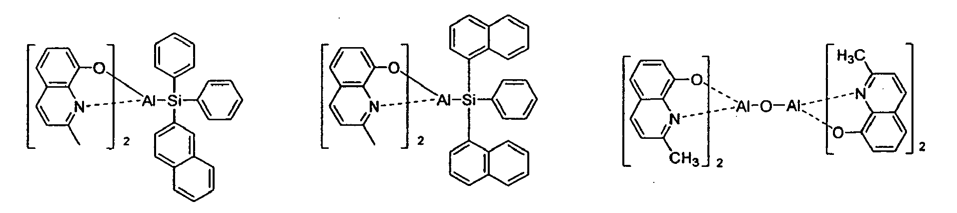

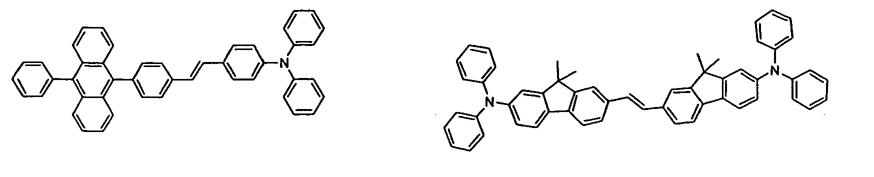

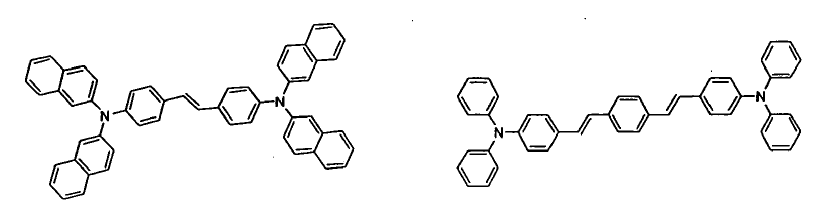

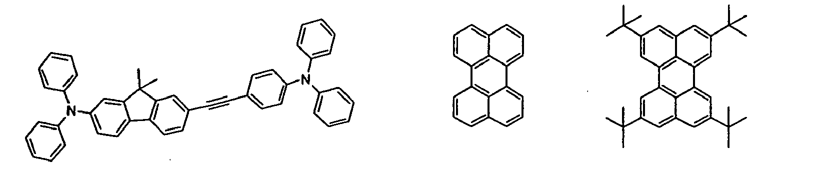

In an organic electroluminescent device according to the present invention, it is preferable to arrange one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the present invention, having high luminous efficiency and excellent life property of material, are advantageous in that they can be employed to manufacture organic light emitting diodes (OLED's) having very good operation life.

### [Best Mode]

The present invention is further described by referring to representative compounds with regard to the organic electroluminescent compounds according to the invention, preparation thereof and luminescent properties of the devices manufactured therefrom, but those examples are provided for illustration of the embodiments only, not being intended to limit the scope of the invention by any means.

### [Preparation Examples]

### [Preparation Example 1] Preparation of Compound (30)

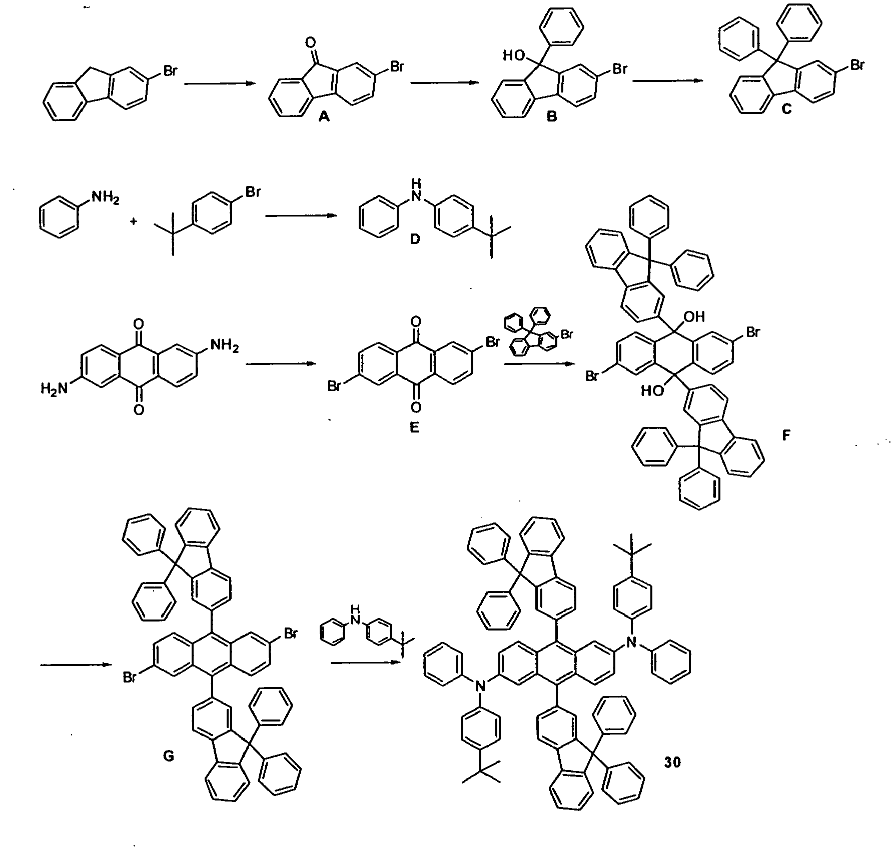

### Preparation of Compound (A)

A reaction vessel was charged with 2-bromofluorene (19.6 g, 79.96 mmol) and tetrabutylammonium hydroxide (6.4 mL, 6.4 mmol), and pyridine (80 mL) was added thereto with stirring. Air was blown to the mixture with vigorous stirring. After 3 days, acetic acid (100 mL) was added thereto to neutralize the reaction mixture. Yellow solid thus produced was filtered. The solid was stirred with ethanol (100 mL) and recrystallized therefrom to obtain Compound (A) (10.7 g, 41.3 mmol).

### Preparation of Compound (B)

Under nitrogen atmosphere, Compound (A) (7.0 g, 27.02 mmol) was charged to a reaction vessel and dissolved in dry tetrahydrofuran solvent (500 mL). Phenyl magnesium bromide (18.01 mL, 54.03 mmol) was slowly added dropwise thereto. Then, the temperature was raised to 80°C, and the mixture was stirred under reflux. After 18 hours, saturated ammonium chloride solution was slowly added thereto to quench the reaction. After one hour, the mixture was extracted with ethyl acetate (300 mL), and the extract was washed with water (500 mL). The organic layer was dried over magnesium sulfate, and distilled under reduced pressure. The solid obtained was recrystallized from dichloromethane (300 mL) and n-hexane (300 mL) to provide Compound (B) (8.0 g, 23.72 mmol) as white powder.

### Preparation of Compound (C)

Compound (B) (10.0 g, 29.65 mmol) was dissolved in benzene solvent (200 mL) in a reaction vessel, and the solution was stirred under reflux at 50°C. Trifluoromethane sulfonate (5.45 mL, 59.31 mmol) was slowly added dropwise thereto. After 20 hours, saturated ammonium chloride (NH₄Cl) solution was slowly added to the reaction mixture to quench the reaction. The organic layer obtained from extraction with dichloromethane (300 mL) was dried over magnesium sulfate, and distilled under reduced pressure. Recrystallization of the solid thus obtained from dichloromethane (100 mL) and methanol (300 mL) gave Compound (C) (7.8 g, 19.63 mmol).

### Preparation of Compound (D)

In a reaction vessel, bis(dibenzylidenacetone)palladium (0) (4.05 g, 4.69 mmol) and cesium carbonate (305.77 g, 938.48 mmol) were added to 1-bromo-4-tert-butylbenzene (100.0 g, 469.24 mmol) and aminobenzene (48.06 g, 516.16 mmol), and toluene solvent (4 L) was added thereto under nitrogen atmosphere. While stirring the mixture, tri-tert-butylphosphine was added thereto. Upon raising the temperature to 120°C, the mixture was stirred under reflux for 3 hours. The reaction was quenched by adding water (2 L), and the resultant mixture was extracted with ethyl acetate (2 L). The organic layer was dried over magnesium sulfate, and distilled under reduced pressure. The solid obtained was recrystallized from dichloromethane (300 mL) and n-hexane (300 mL) to provide Compound (D) (80.0 g, 75.6%).

### Preparation of Compound (E)

In a reaction vessel, copper bromide (101.0 g, 0.45 mmol), tert-butyl nitrate (58.34 mL, 0.49 mmol) and acetonitrile (800 mL) were stirred at 70°C. After 1 hour, 2,6-diaminoanthraquinone (45.0 g, 0.19 mmol) was added thereto, and the mixture was stirred at 85°C for 48 hours. Then, 20% hydrochloric acid (1 L) was added thereto, and the resultant mixture was stirred for 1 hour. The precipitate produced was filtered and washed several times with water and methanol. Additional washing twice with acetone and dichloromethane, respectively, gave Compound (E) (50.0 g, 72%).

### Preparation of Compound (F)

Compound (C) (20.0 g, 50.34 mmol) was dissolved in dry tetrahydrofuran solvent (200 mL) under nitrogen atmosphere, and 2.5 M n-butyllithium (in n-hexane) (26.85 mL, 67.12 mmol) was slowly added dropwise thereto at -78°C. After stirring for 1 hour, Compound (E) obtained as described above (6.14 g, 16.78 mmol) was added thereto. While slowly raising the temperature to room temperature, the mixture was stirred. After 17 hours, water was added, and the resultant mixture was stirred for 30 minutes. The mixture was extracted with ethyl acetate (500 mL) and the extract was washed with water (500 mL) to obtain organic layer. The organic layer was then dried over magnesium sulfate, and distilled under reduced pressure to obtain solid. Recrystallization of the solid from dichloromethane (300 mL) and n-hexane (300 mL) gave Compound (F) (7.3 g, 45%).

### Preparation of Compound (G)

Compound (F) (7.0 g, 6.98 mmol), potassium iodide (4.64 g, 27.92 mmol), sodium hydrophosphite (4.44 g, 41.88 mmol) and acetic acid (100 mL) were stirred under reflux in a reaction vessel. After 15 hours, water (500 mL) was added thereto, and the mixture was stirred for 1 hour. The precipitate obtained from filtering under reduced pressure was washed three times with water (300 mL) and once with acetone (300 mL). Recrystallization from dichloromethane (100 mL) and methanol (500 mL) gave Compound (G) (5.0 g, 76%) as yellow solid.

### Preparation of Compound (30)

A reaction vessel was charged with Compound (G) (4.0 g, 4.13 mmol), Compound (D) (2.79 g, 12.39 mmol), palladium acetate (II) (0.31 g, 0.34 mmol), tri-tert-butylphosphine (0.2 mL, 0.83 mmol), cesium carbonate (6.05 g, 12.39 mmol), and toluene solvent (50 mL), and the mixture was stirred under reflux in the presence of nitrogen atmosphere. After 8 hours, the mixture was cooled to room temperature, and water (100 mL) was added thereto to quench the reaction. The mixture was extracted with dichloromethane (300 mL) to obtain organic layer, which was dried over magnesium sulfate and distilled under reduced pressure. The organic product thus obtained was purified via column chromatography (dichloromethane: n-hexane = 5:1) to obtain the target compound (Compound 30) (2.0 g, 38%).

### [Preparation Example 2] Preparation of Compound (1579)

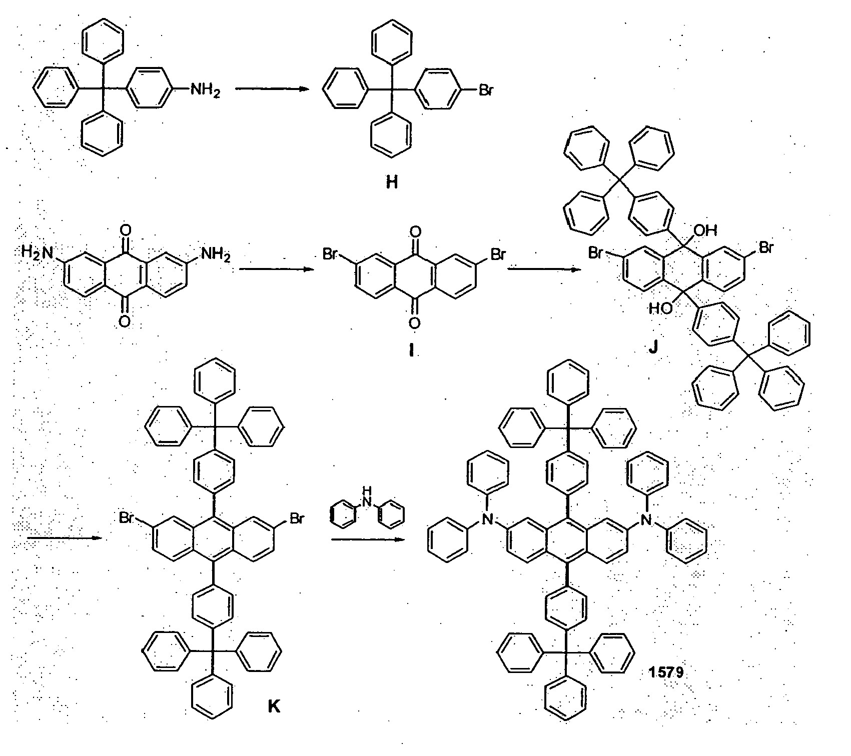

### Preparation of Compound (H)

Copper bromide (20.0 g, 89.43 mmol) was dissolved in acetonitrile solvent (250 mL), and tert-butyl nitrate (10.62 mL, 89.43 mmol) was added thereto. After stirring at 70°C for 1 hour, 4-tritylaniline (20.0 g, 59.62 mmol) was added thereto, and the resultant mixture was stirred at 85°C for 20 hours. Then the reaction mixture was cooled to room temperature, and poured into water. The mixture was extracted with chloroform (500 mL), and the extract washed with saline (500 mL). The organic layer obtained was dried over magnesium sulfate, and distilled under reduced pressure. The organic product was recrystallized from dichloromethane (20 mL) and methanol (100 mL) to obtain the target compound (Compound H) (22.0 g, 92%).

### Preparation of Compound (I)

In a reaction vessel, copper bromide (101.0 g, 0.45 mmol), tert-butyl nitrate (58.34 mL, 0.49 mmol) and acetonitrile (800 mL) were stirred at 70°C for 1 hour. Then, 2,7-diaminoanthraquinone (45.0 g, 0.19 mmol) was added thereto, and the mixture was stirred at 85°C for 48 hours. To the reaction mixture, 20% hydrochloric acid (1 L) was added, and the resultant mixture was stirred for one hour. The precipitate produced was filtered and washed several times with water and methanol. Additional washing with acetone and dichloromethane (twice, respectively) gave Compound (I) (50.0 g, 72%).

### Preparation of Compound (J)

Compound (H) (20.0 g, 45.58 mmol) was dissolved in dry tetrahydrofuran solvent (250 mL) under nitrogen atmosphere, and 2.5 M n-butyllithium (in n-hexane) (29.28 mL, 45.58 mmol) was slowly added dropwise thereto at -78°C. After stirring for one hour, Compound (I) obtained as described above (6.69 g, 18.30 mmol) was added thereto, and the resultant mixture was stirred while slowly raising the temperature to room temperature. After 17 hours, water (300 mL) was added, and the mixture was stirred for 30 minutes and extracted with ethyl acetate (500 mL). The extract was washed with water (500 mL), and the organic layer was dried over magnesium sulfate and distilled under reduced pressure. The solid obtained was recrystallized from dichloromethane (300 mL) and n-hexane (300 mL) to obtain Compound (J) (11.6 g, 65%).

### Preparation of Compound (K)

In a reaction vessel, Compound (J) (11.5 g, 11.42 mmol) and potassium iodide (7.58 g, 45.68 mmol), sodium hydrophosphite (7.26 g, 68.53 mmol) and acetic acid (100 mL) were stirred under reflux for 15 hours. Water (500 mL) was added thereto, and the mixture stirred for 1 hour. The precipitate obtained by filtering the mixture under reduced pressure was washed three times with water (300 mL) and once with acetone (300 mL), and recrystallized from dichloromethane (100 mL) and methanol (500 mL) to obtain Compound (K) (4.5 g, 40%) as pale yellow product.

### Preparation of Compound (1579)

A reaction vessel was charged with Compound (K) (4.5 g, 4.63 mmol), diphenylamine (2.4 g, 13.88 mmol), palladium acetate (II) (0.1 g, 0.46 mmol), tri-tert-butyl phosphine (0.3 mL, 0.93 mmol), cesium carbonate (6.78 g, 13.88 mmol) and toluene solvent (50 mL), and the mixture was stirred under reflux in the presence of nitrogen atmosphere for 8 hours. Then, the mixture was cooled to room temperature, and the reaction quenched by adding water (100 mL). The organic layer obtained from extraction of the mixture with dichloromethane (300 mL) was dried over magnesium sulfate, and distilled under reduced pressure. Purification of the organic product via column chromatography (dichloromethane: n-hexane = 5:1) gave the target compound (Compound 1579) (2.5 g, 47%).

The organic electroluminescent compounds shown in Table 1 (Compounds 1 to 1315) were prepared according to the same procedure as in Preparation Example 1, and those in Table 2 (Compounds 1316 to 2630) were prepared according to the procedure in Preparation Example 2. The ¹H NMR and MS/FAB data of organic electroluminescent compounds prepared are listed in Table 3.

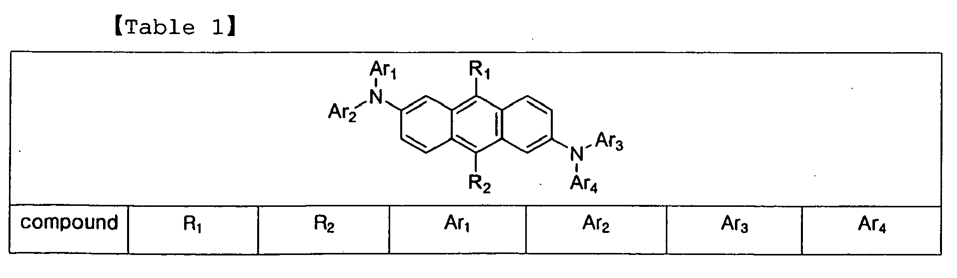

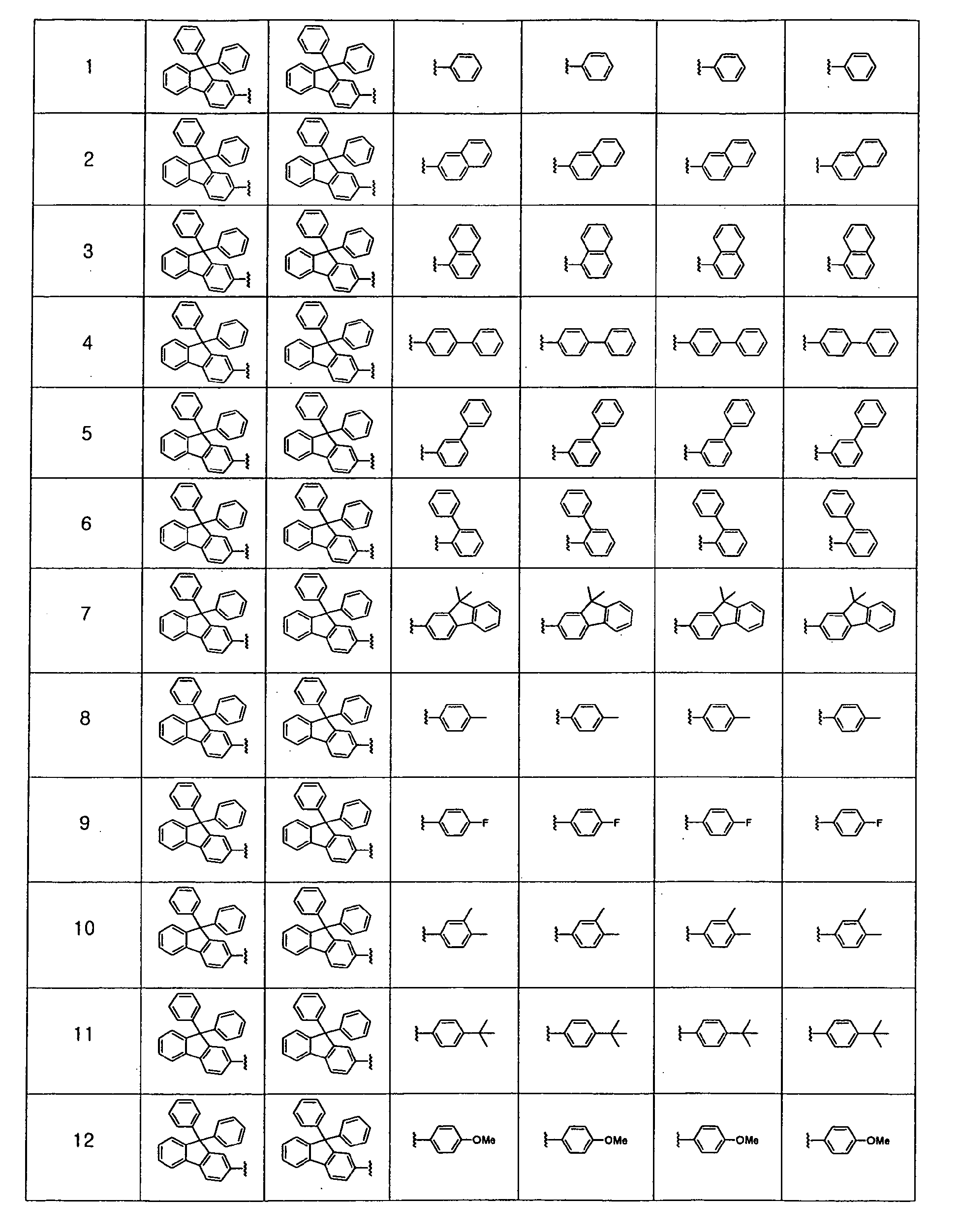

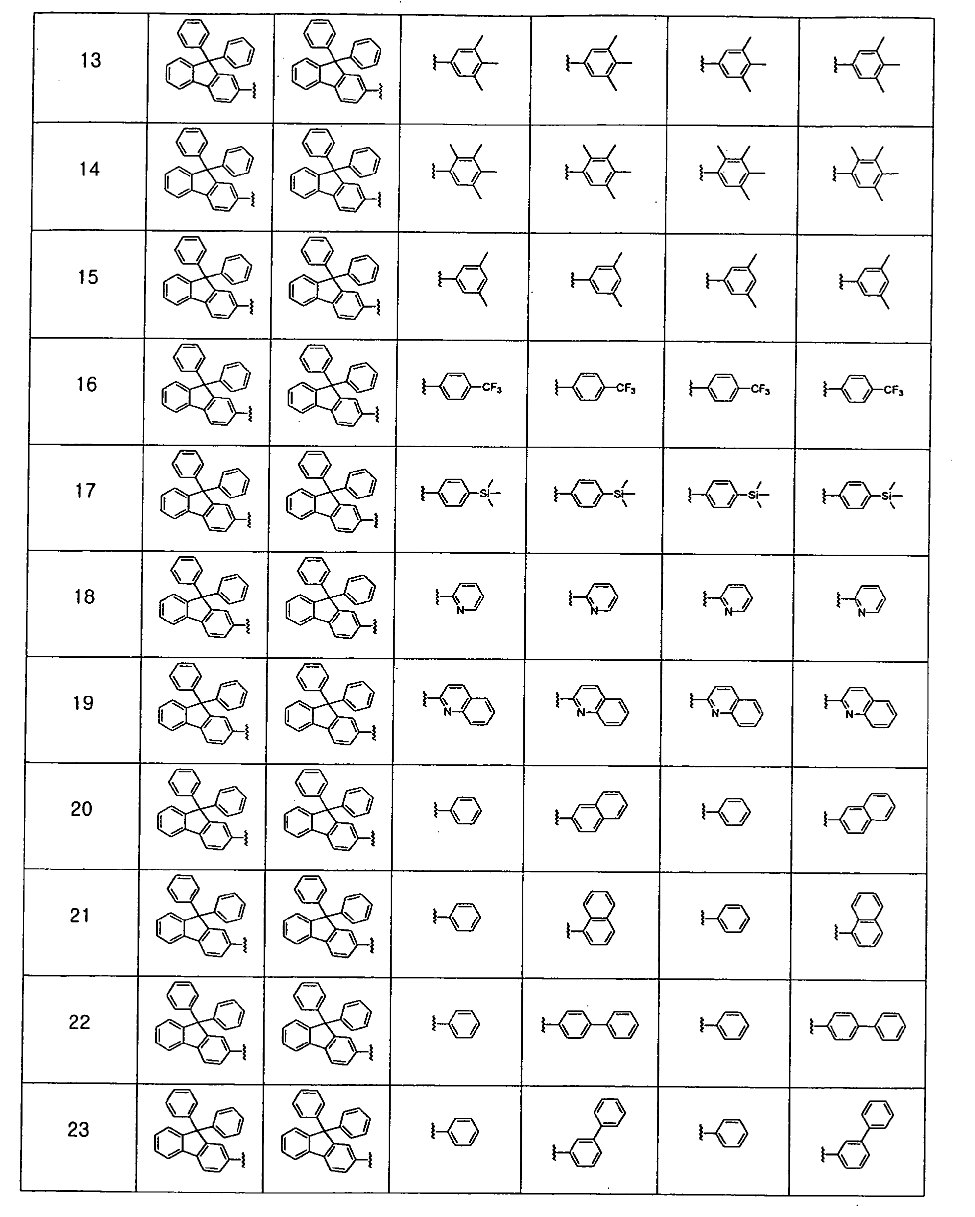

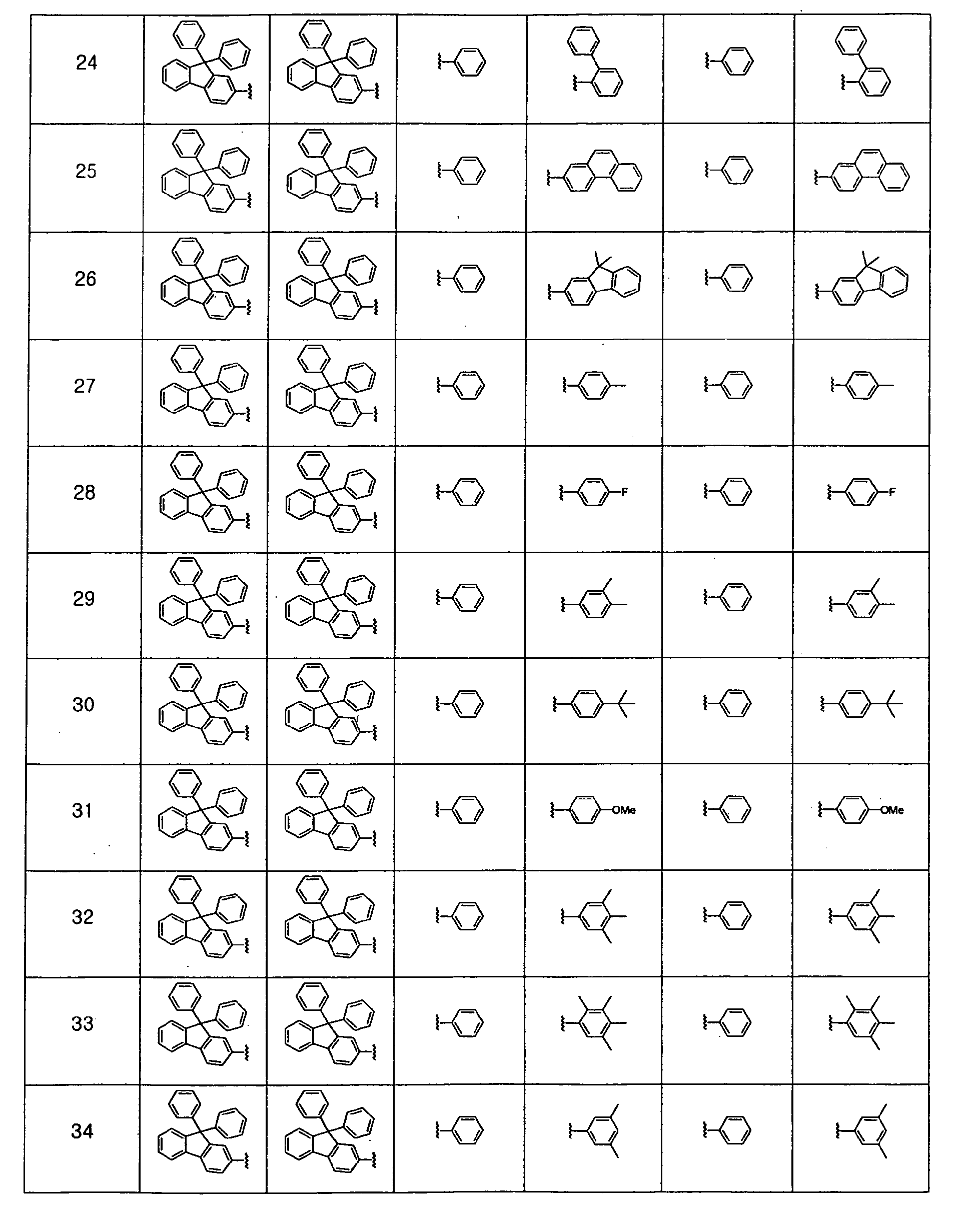

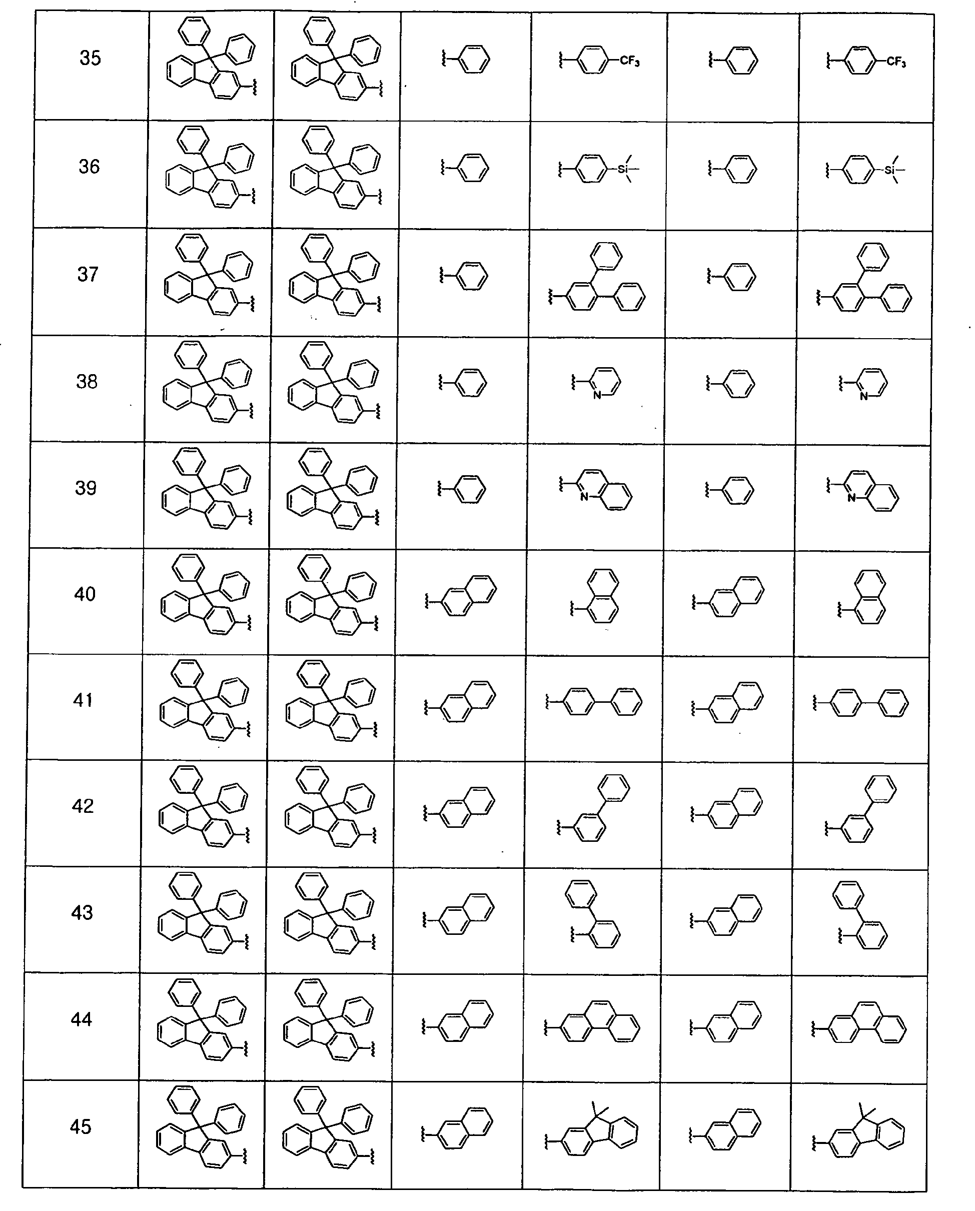

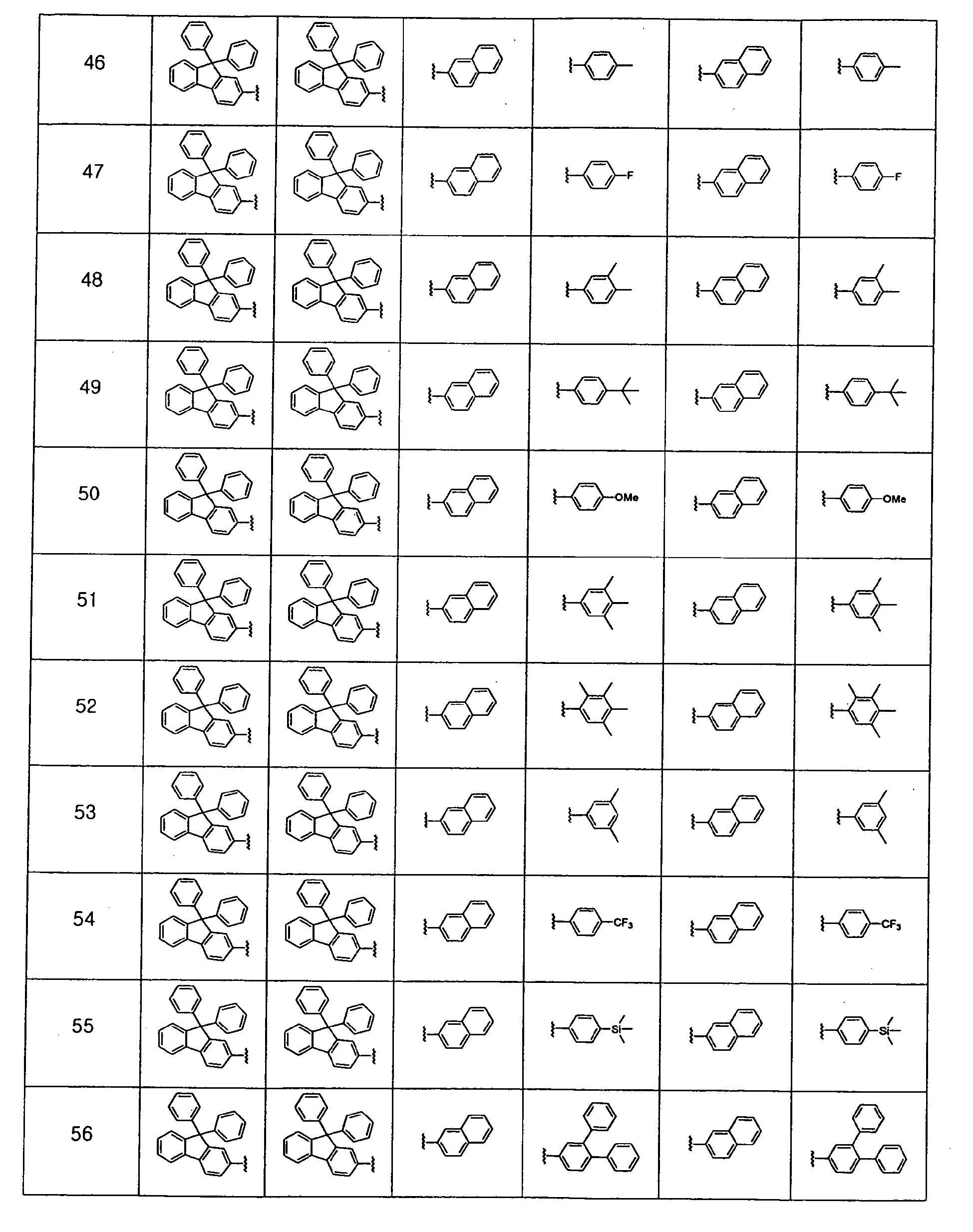

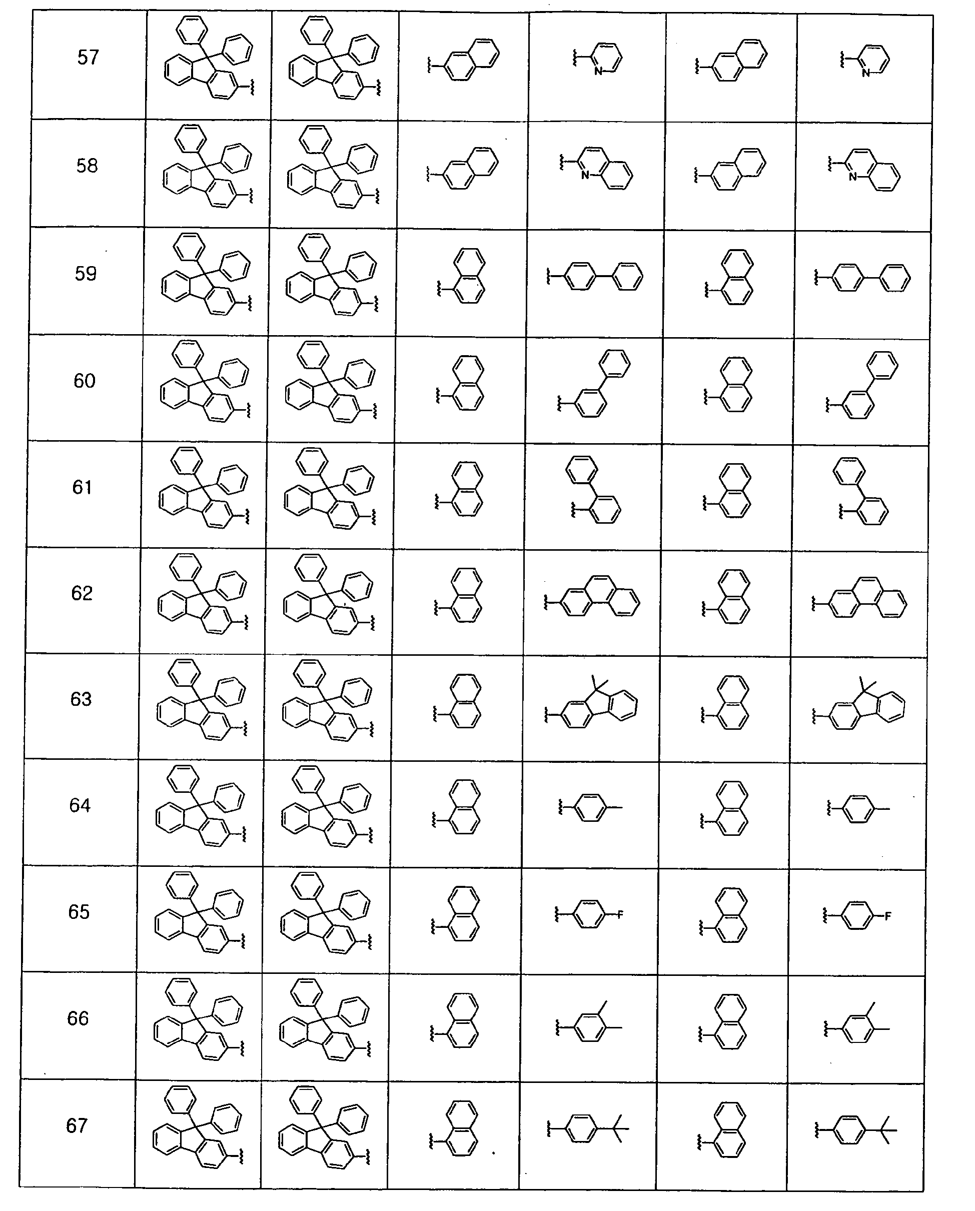

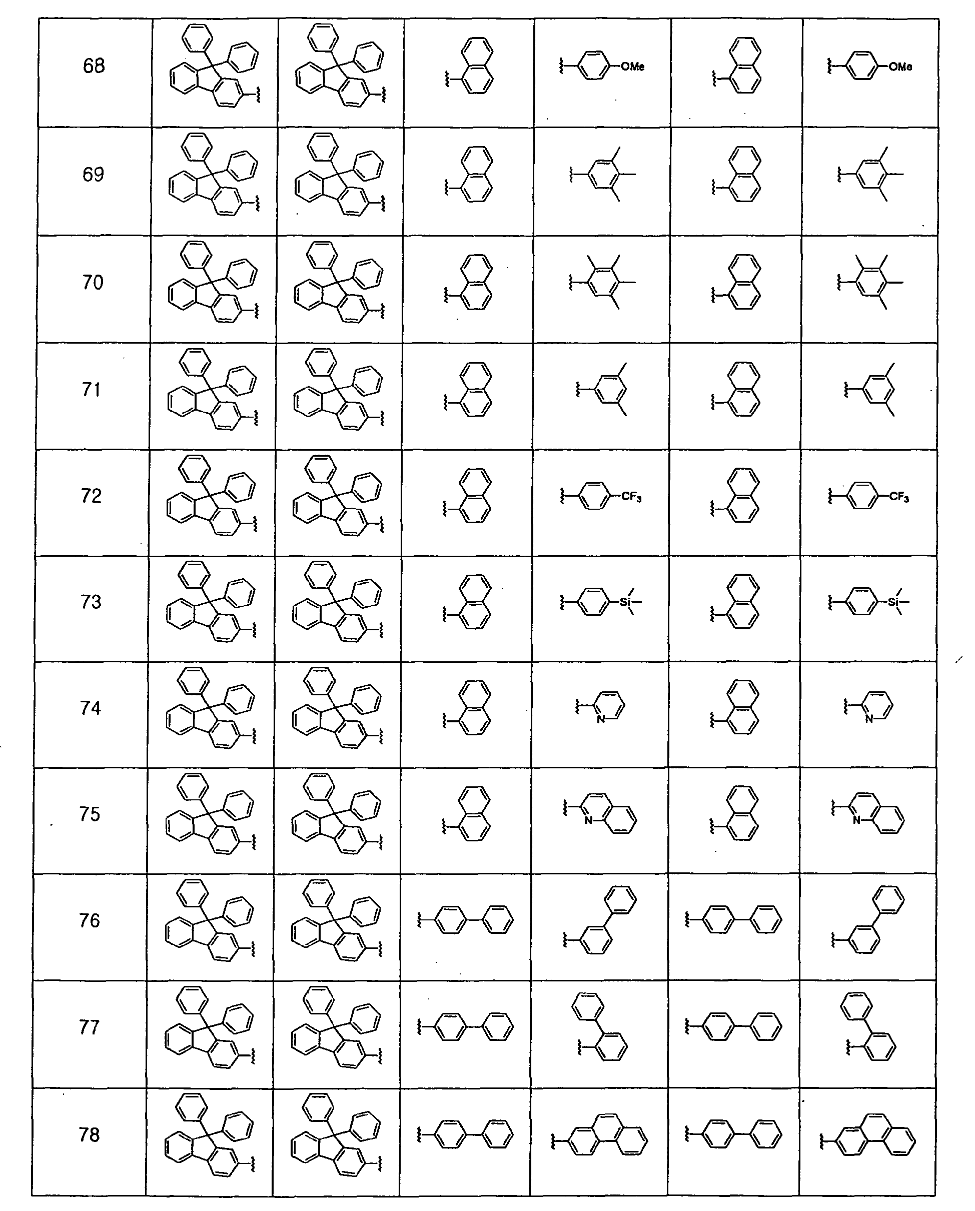

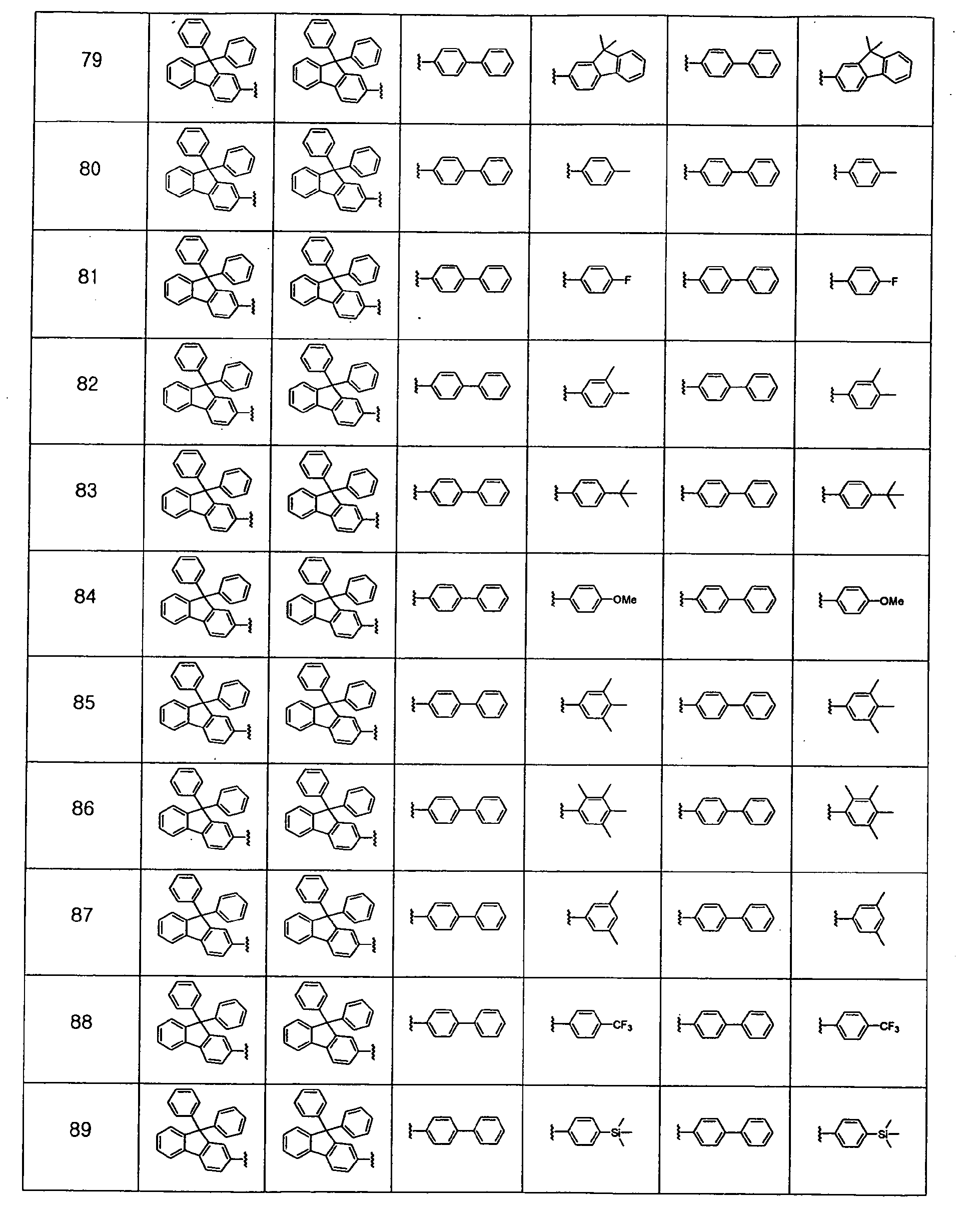

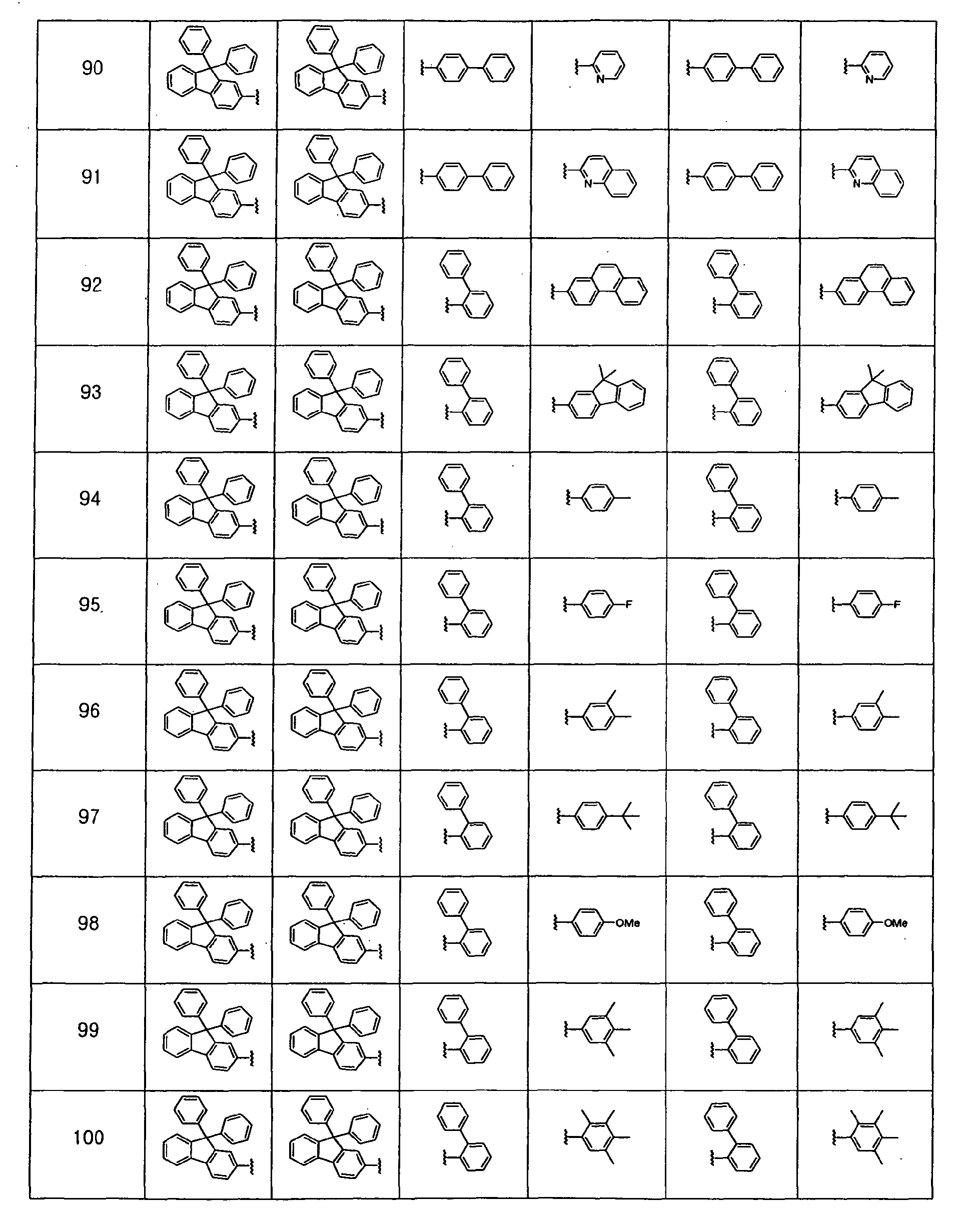

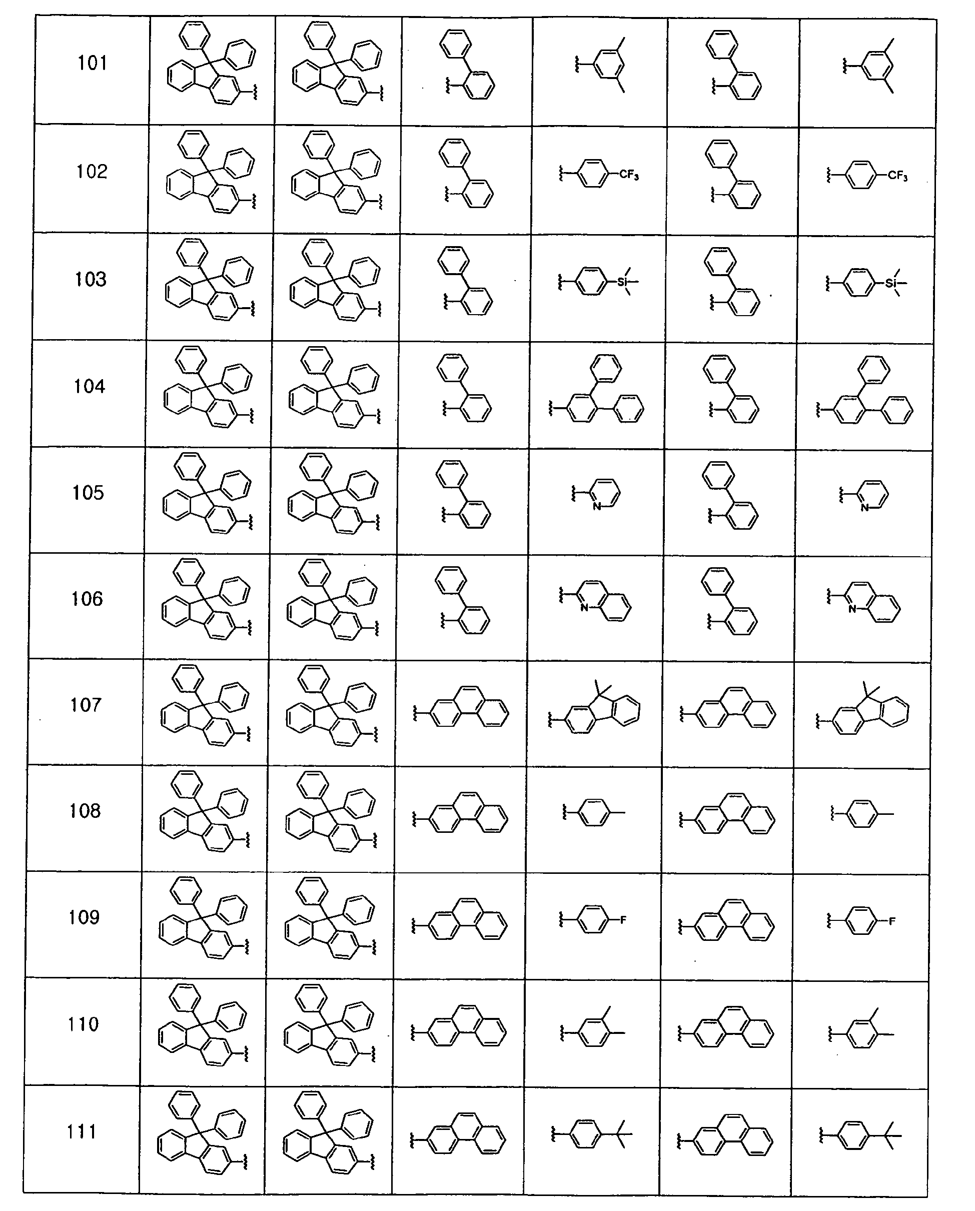

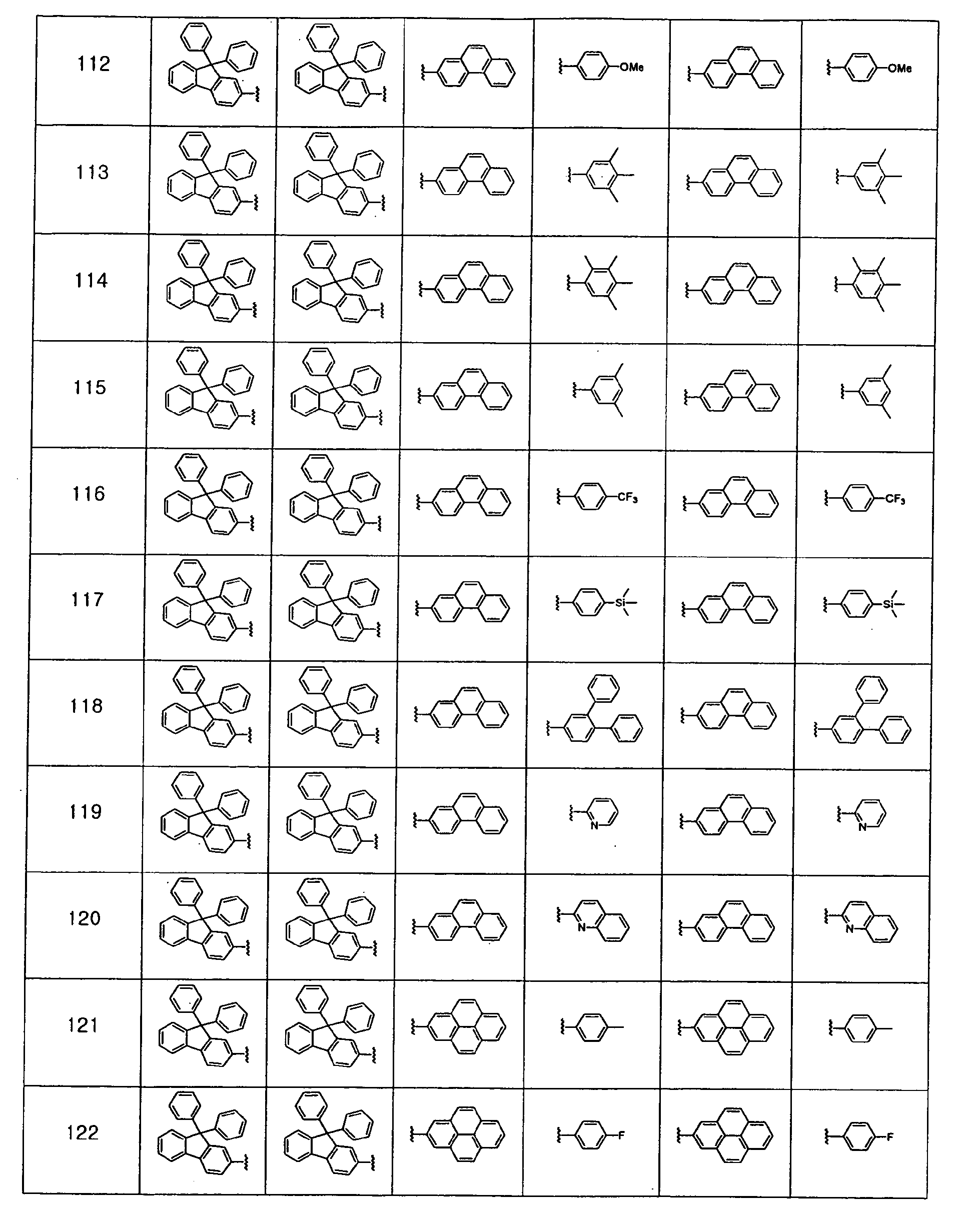

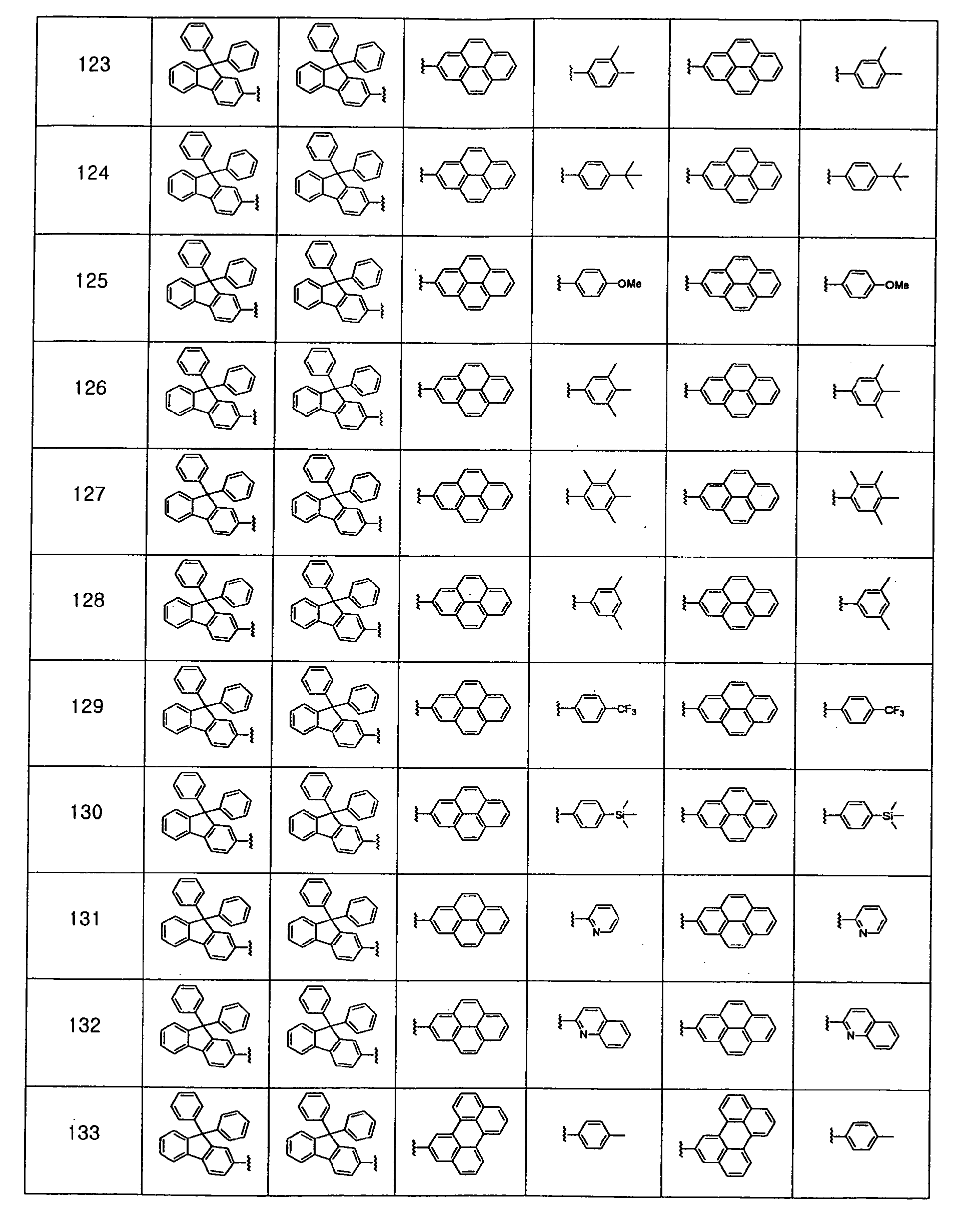

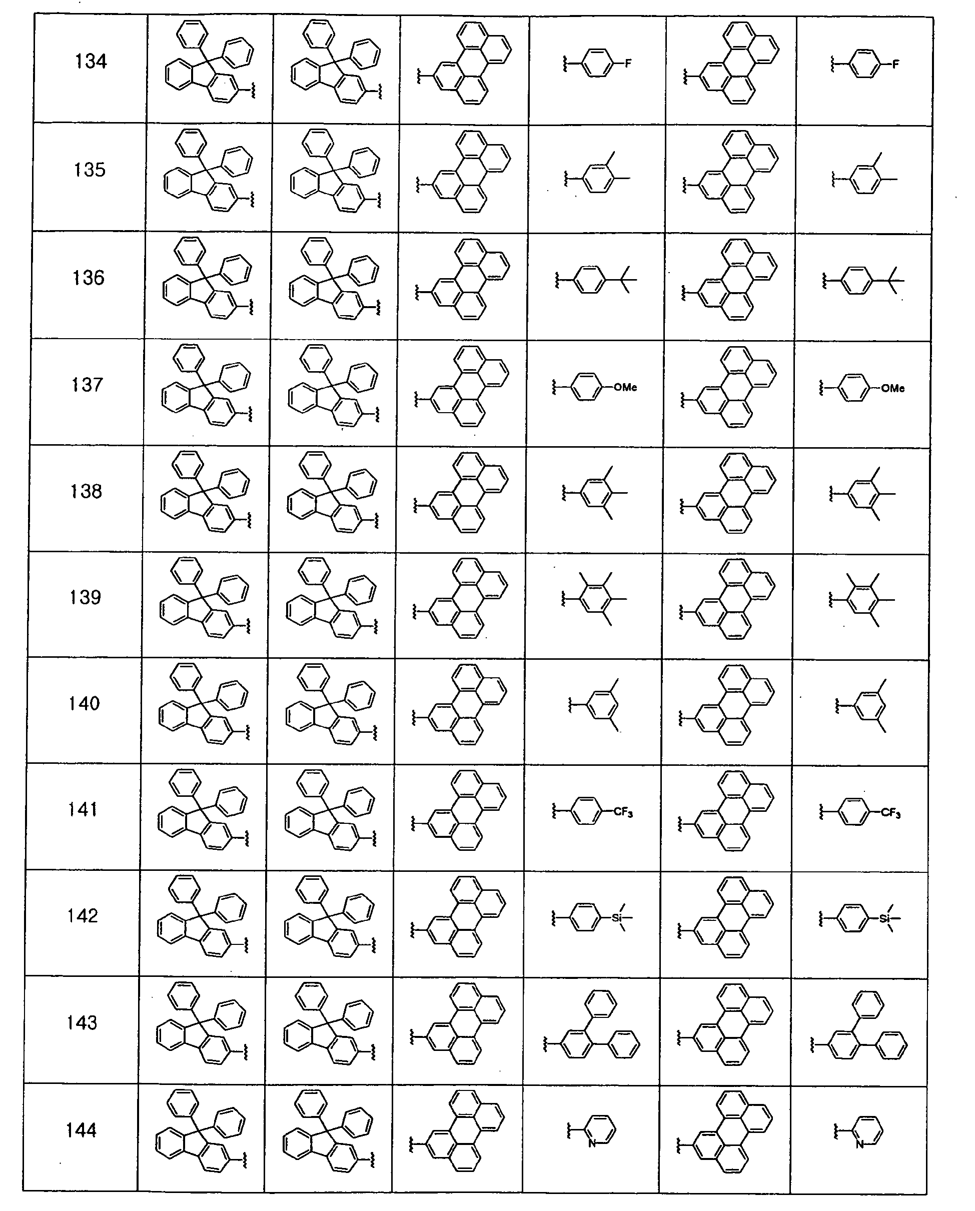

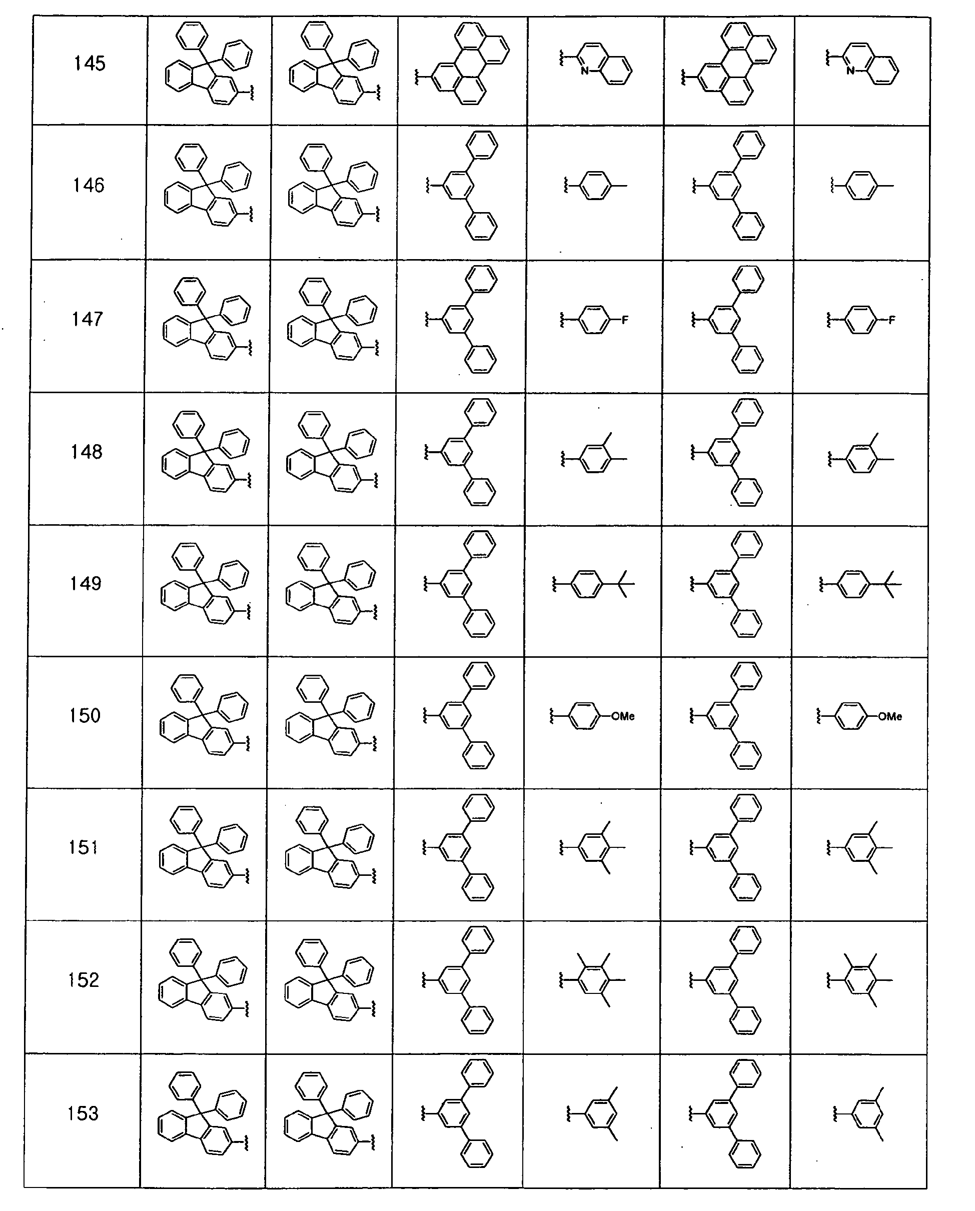

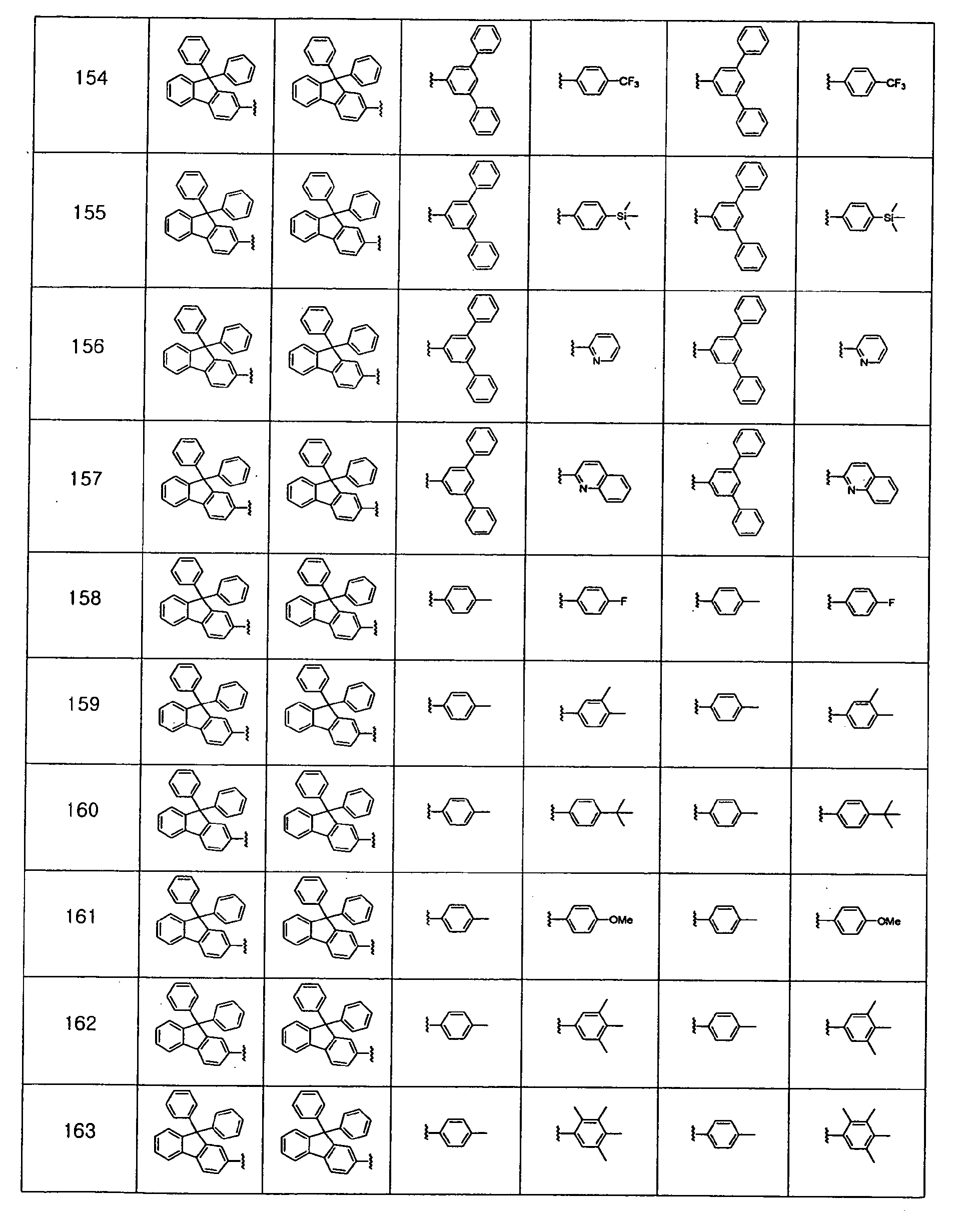

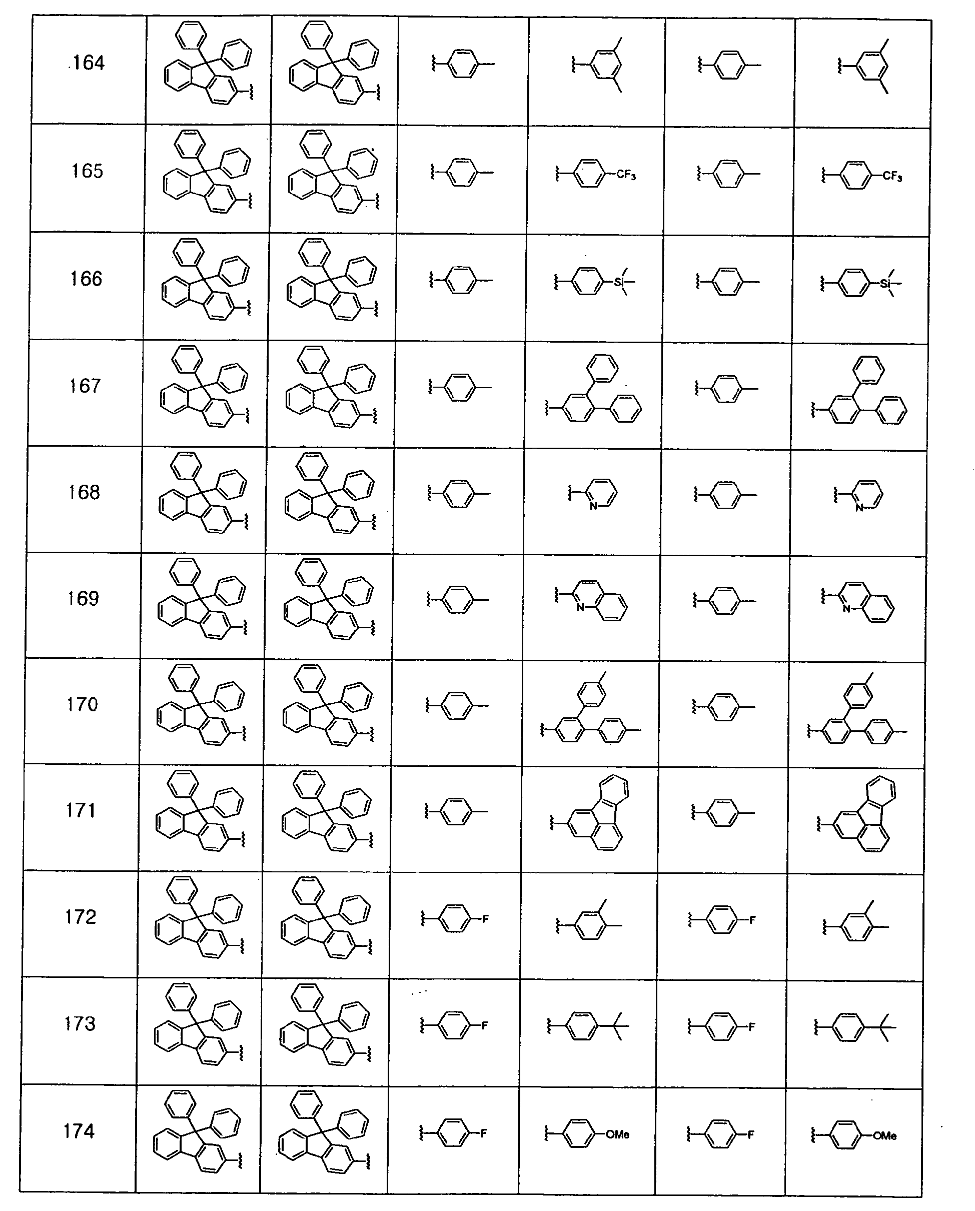

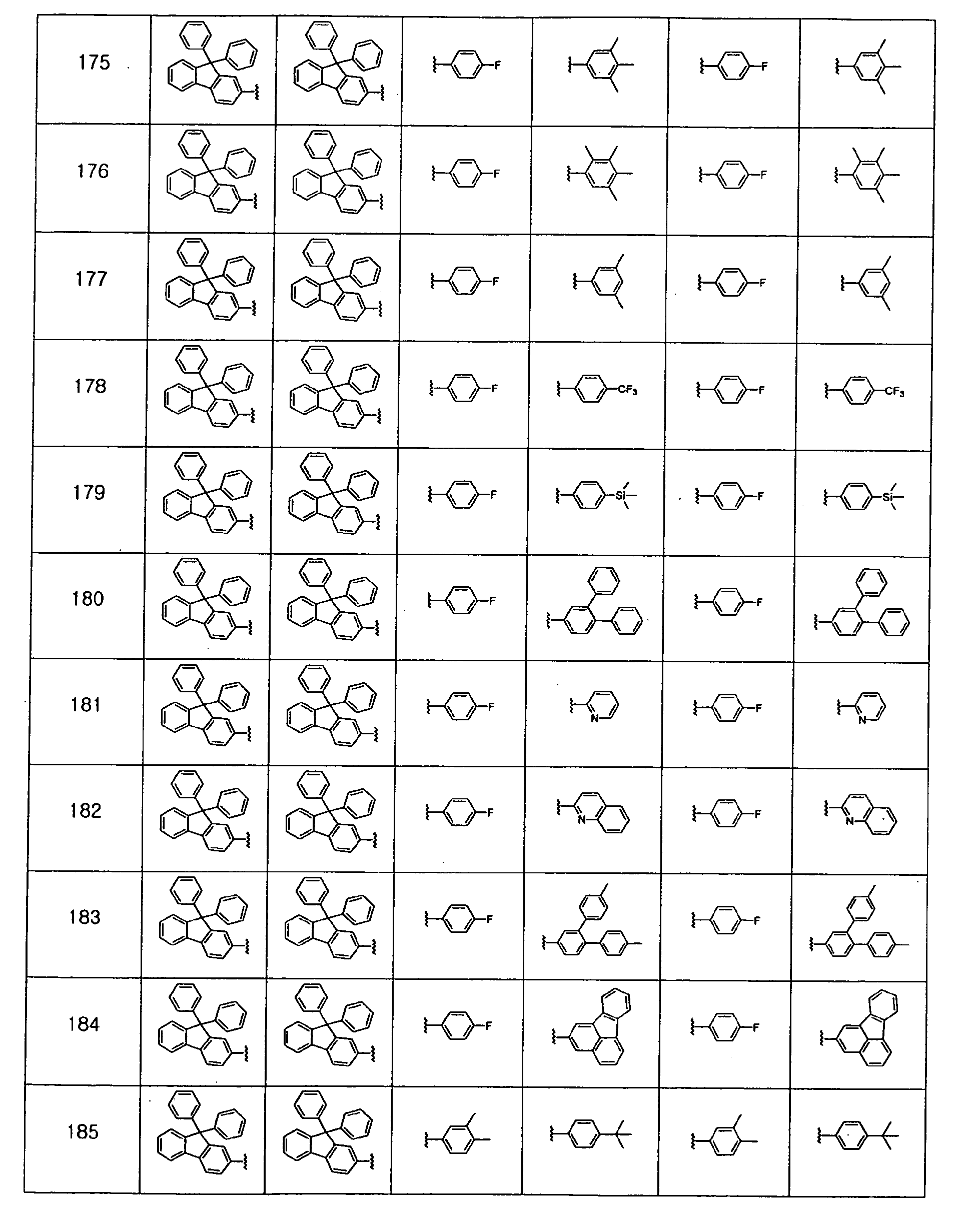

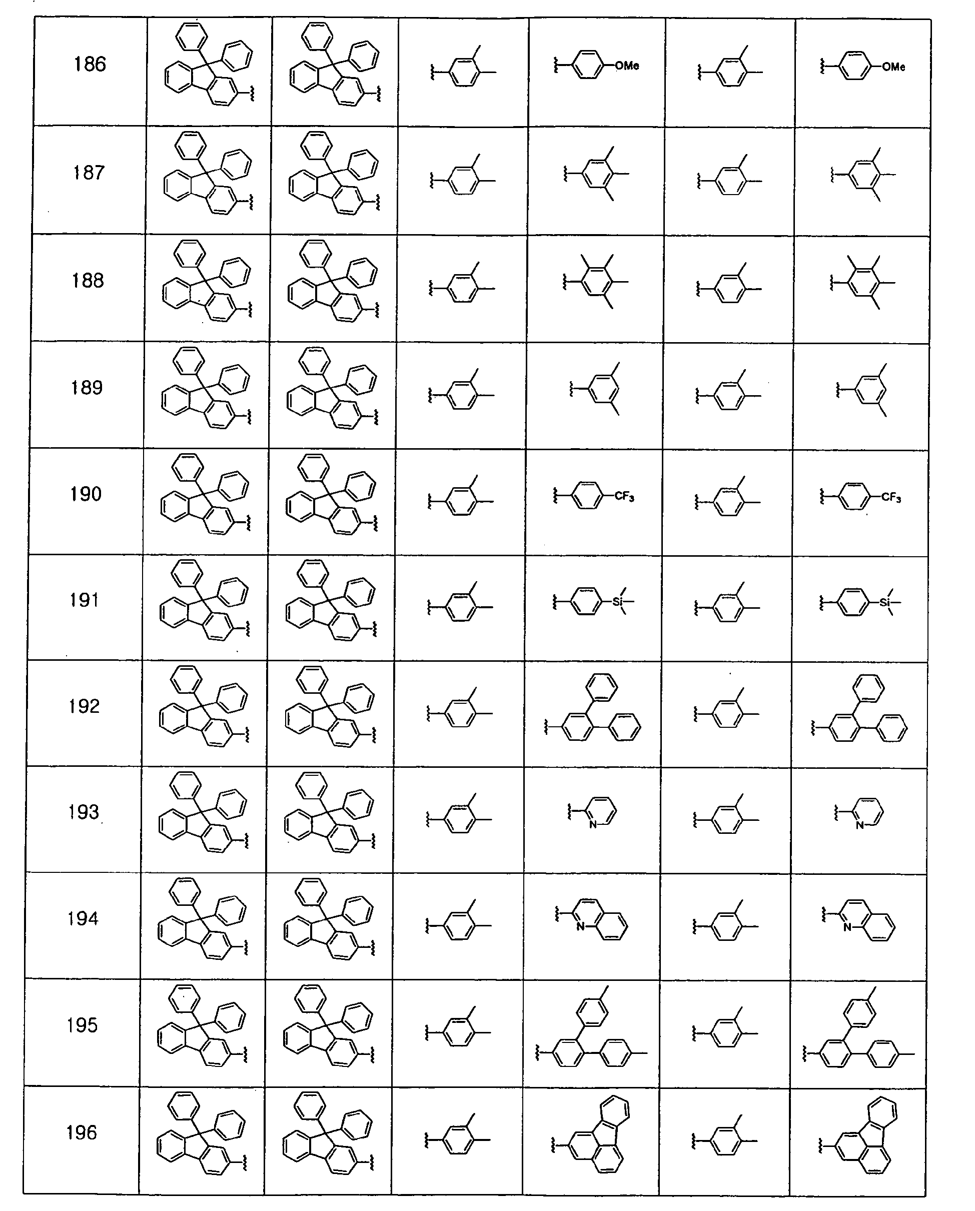

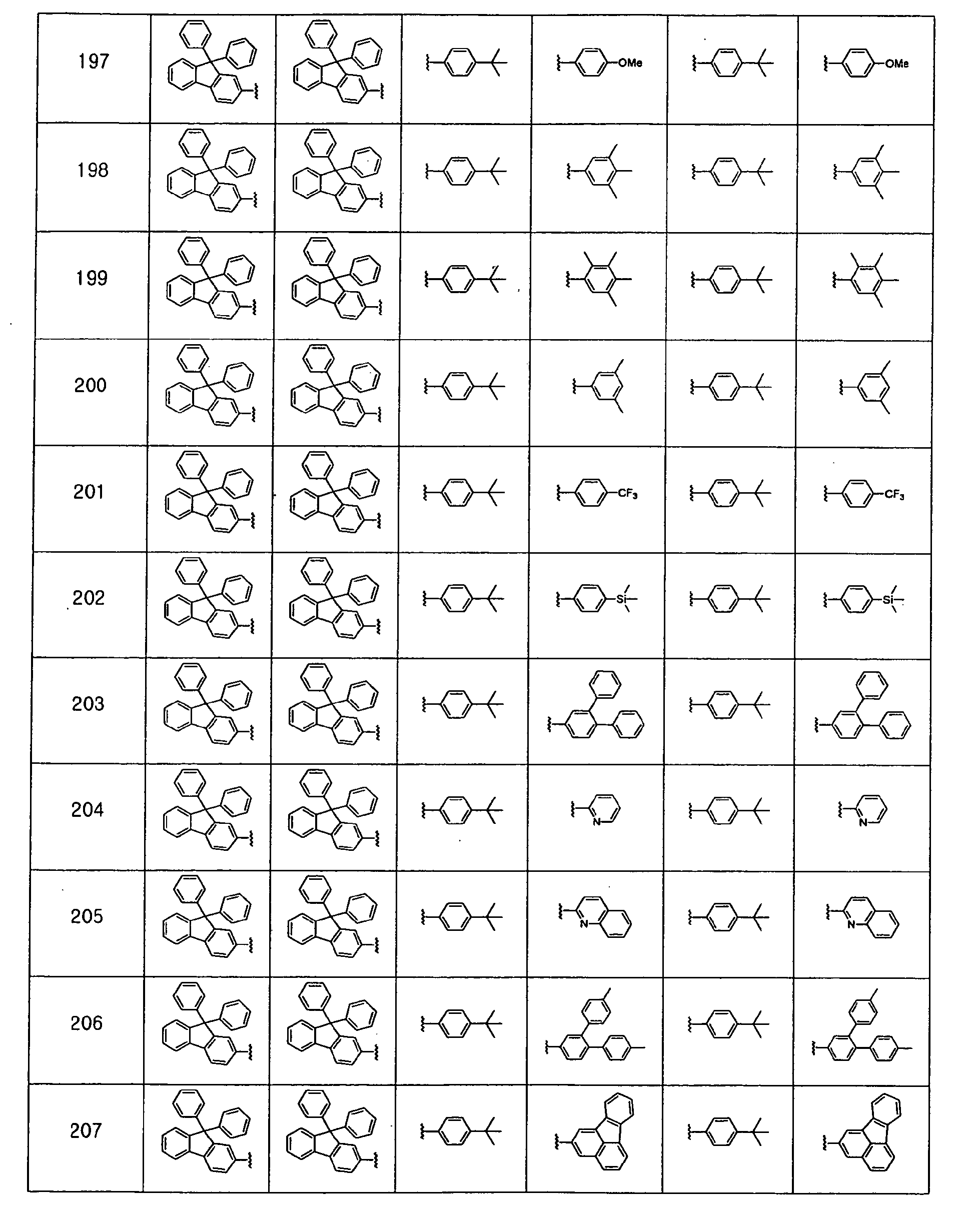

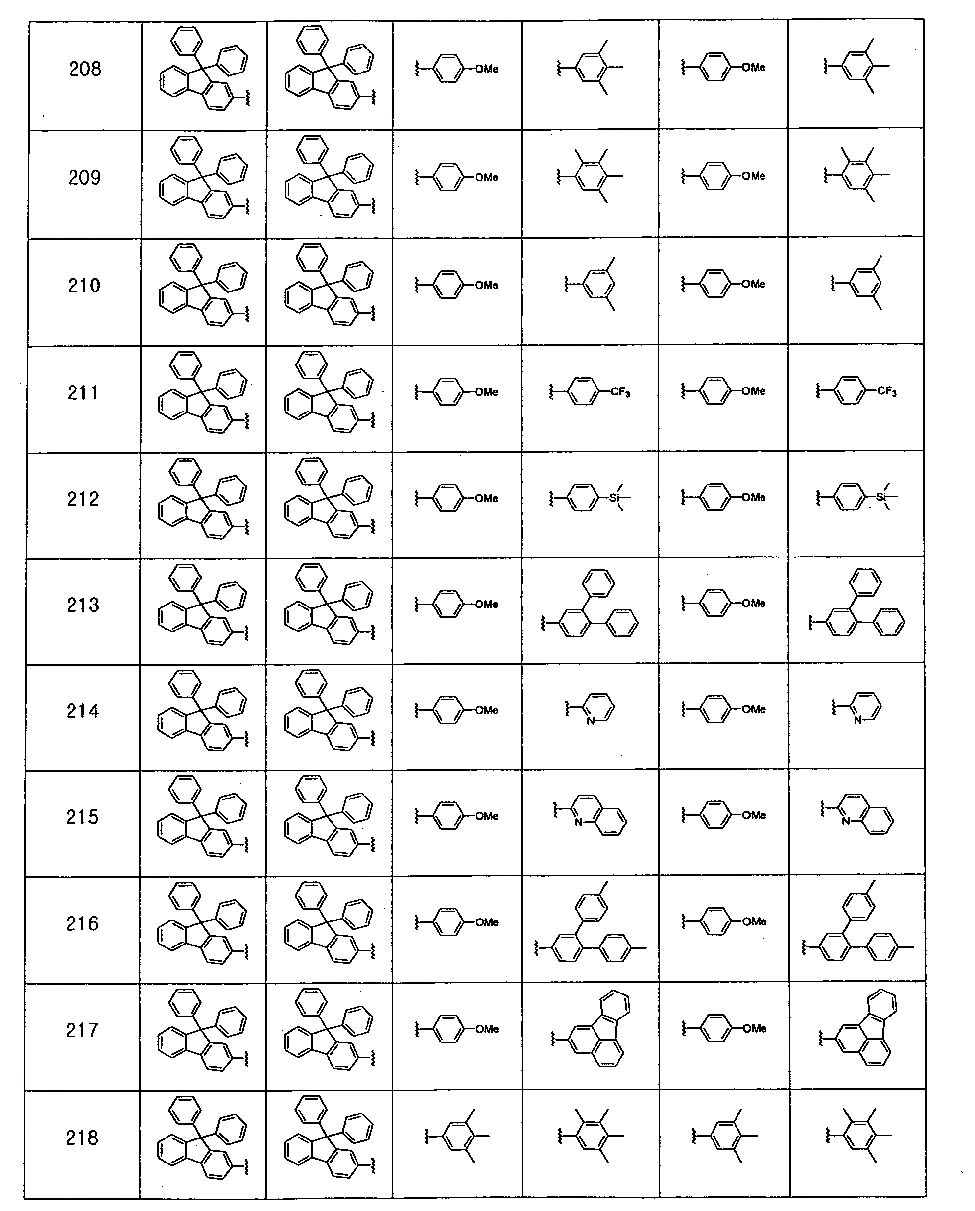

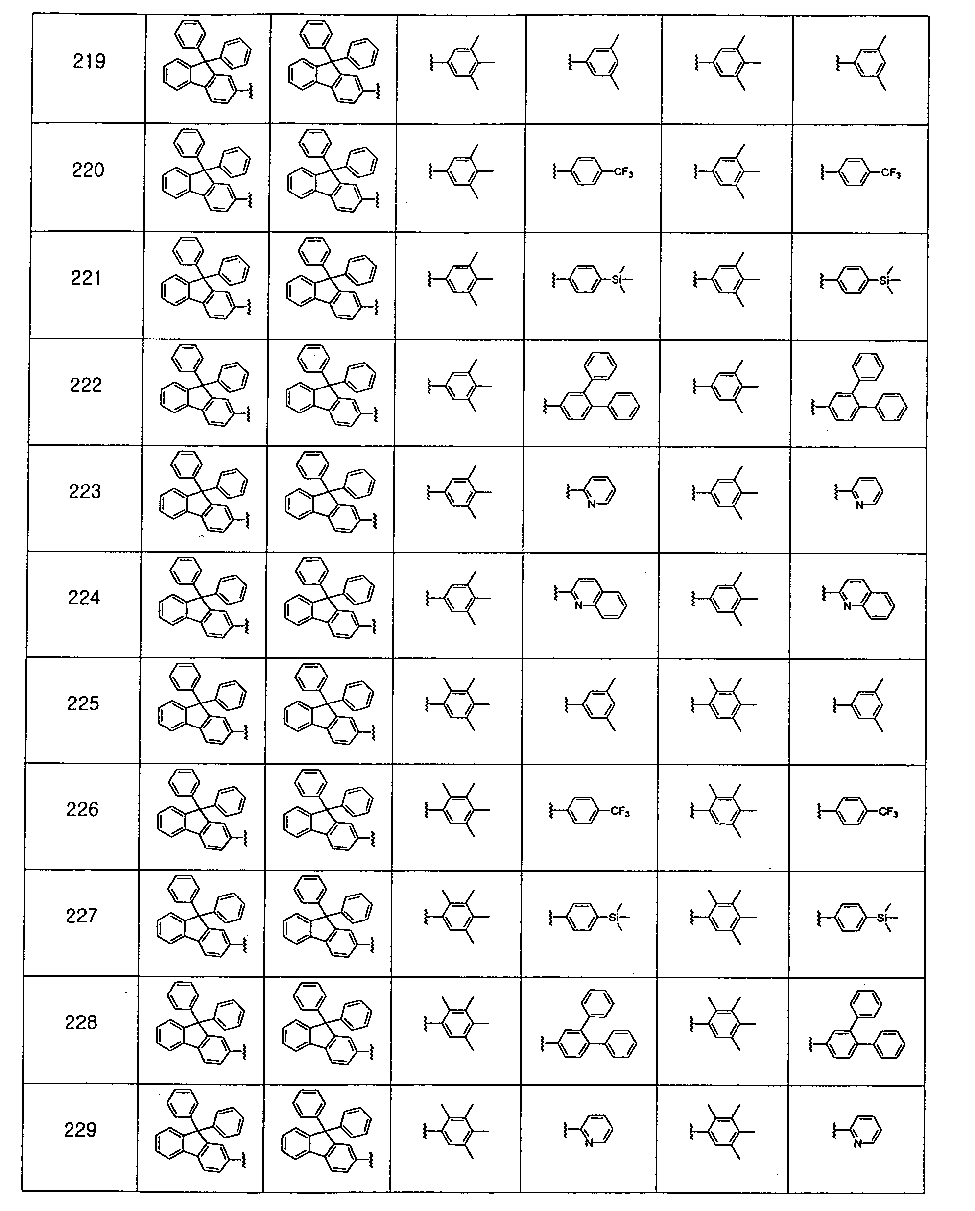

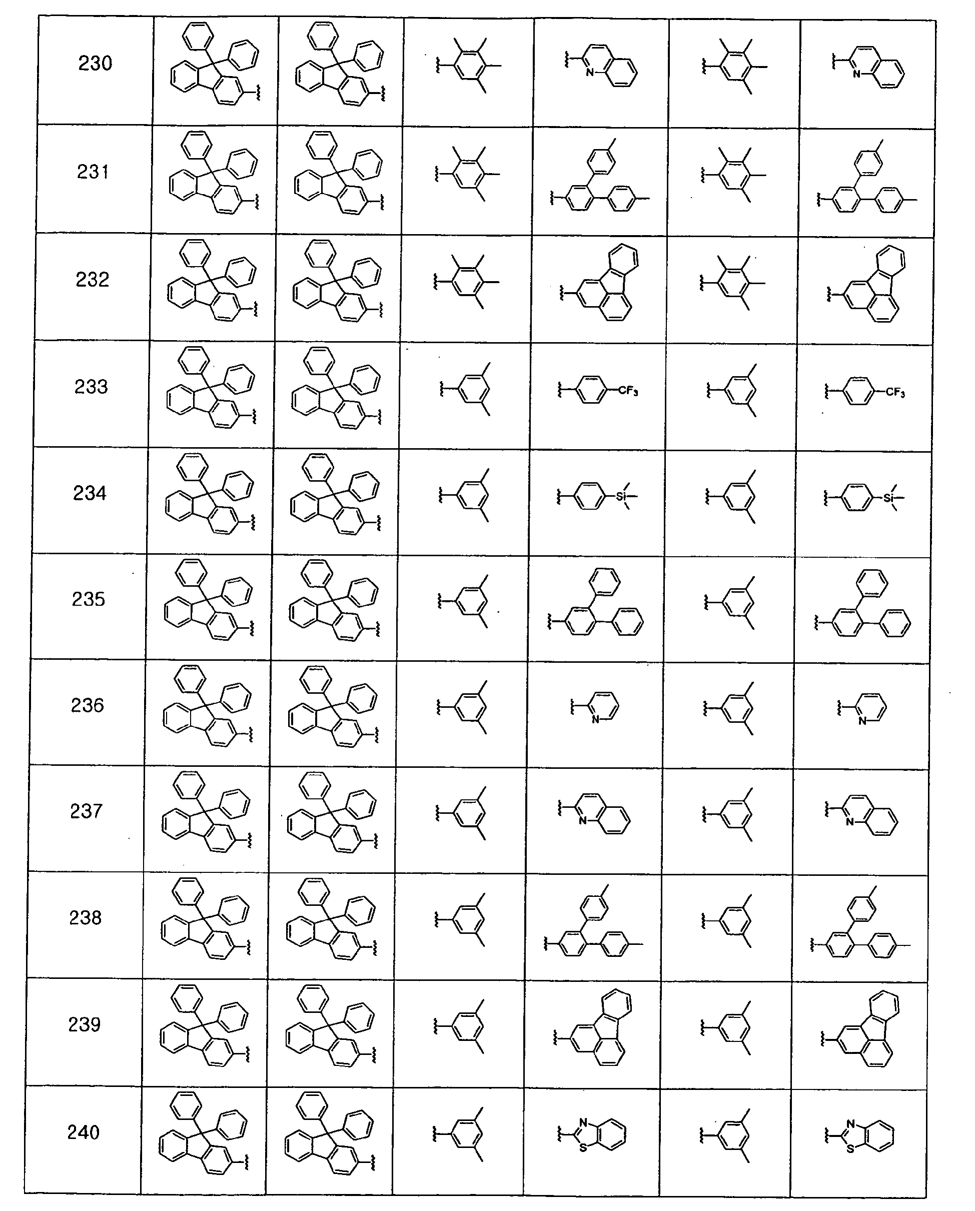

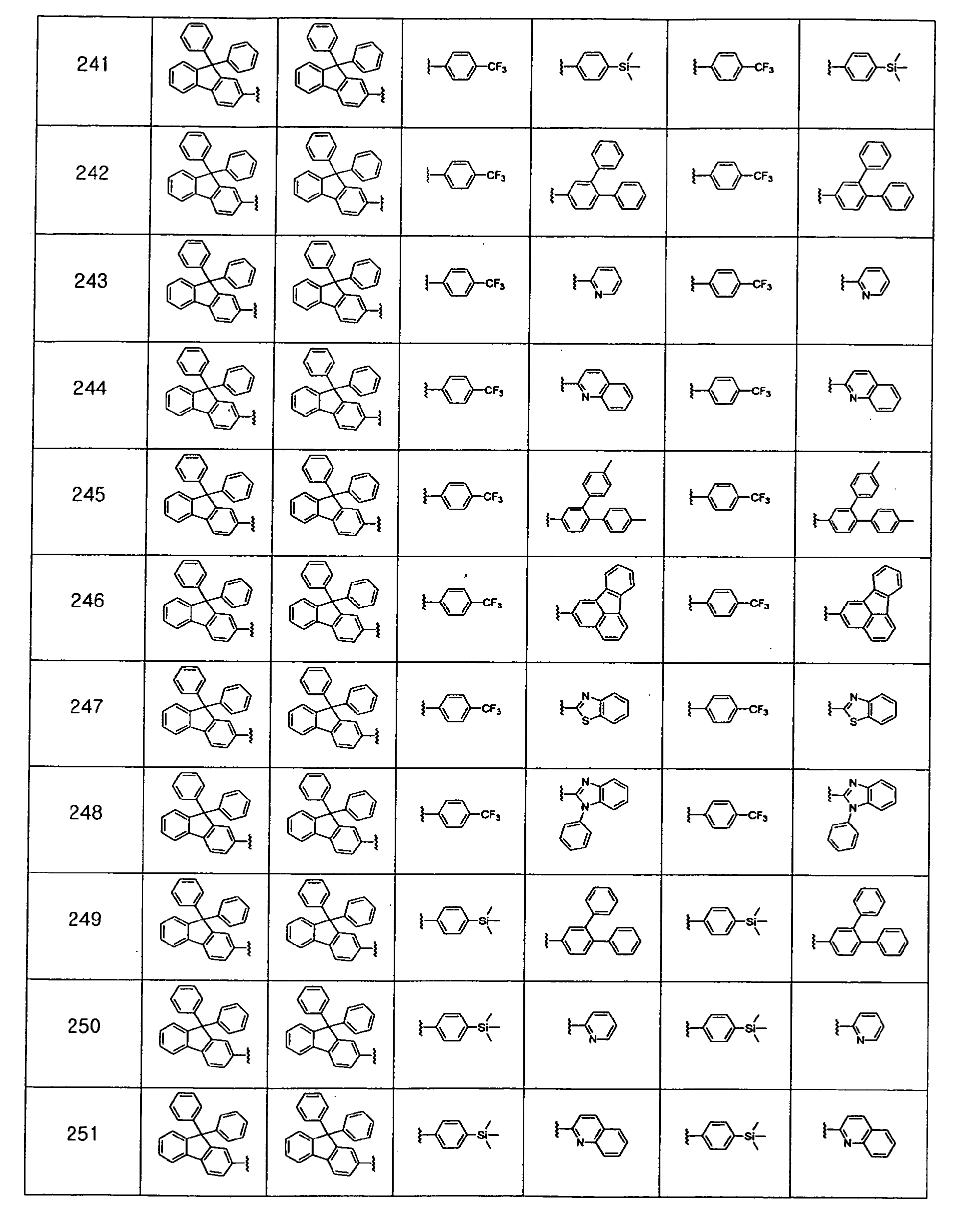

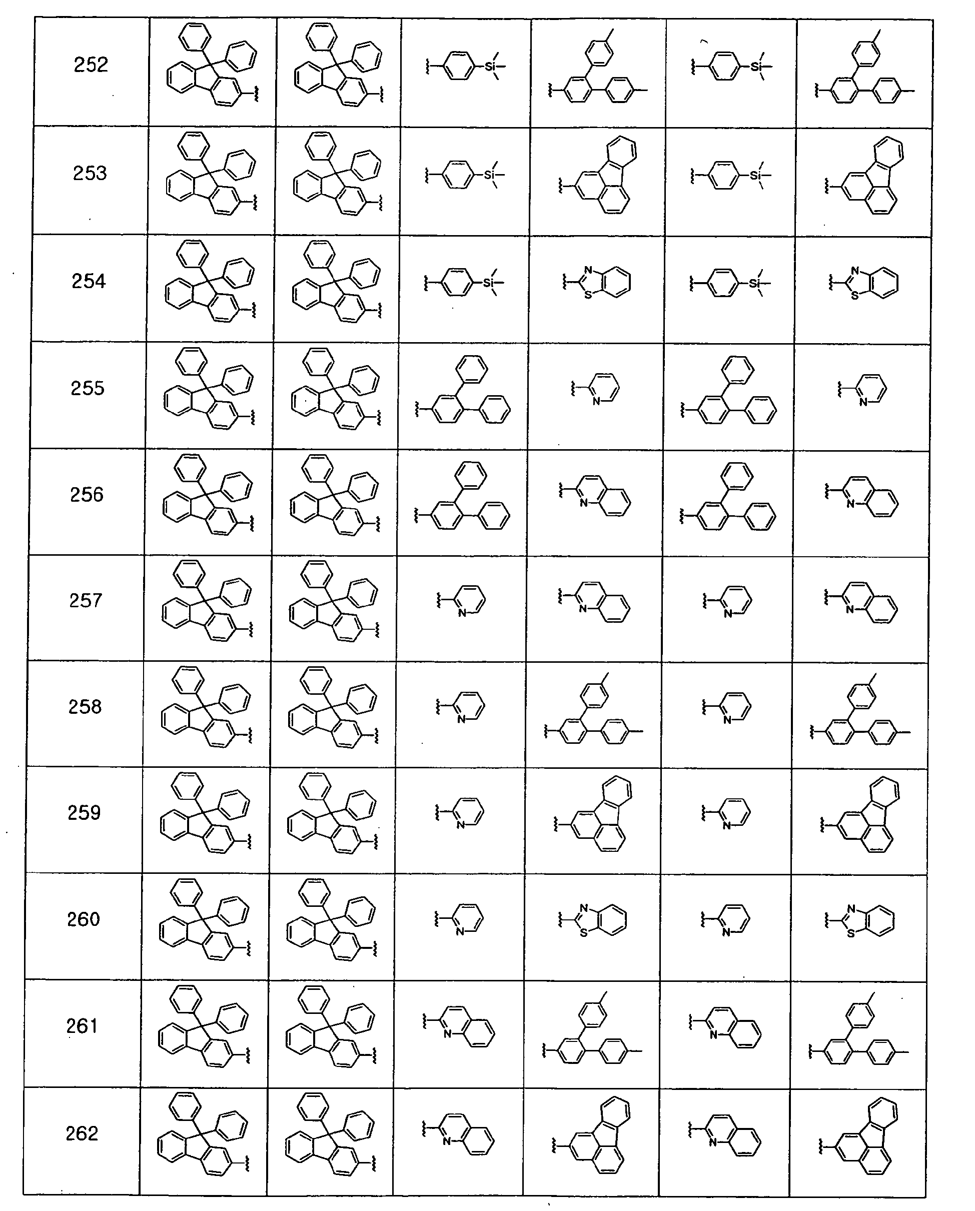

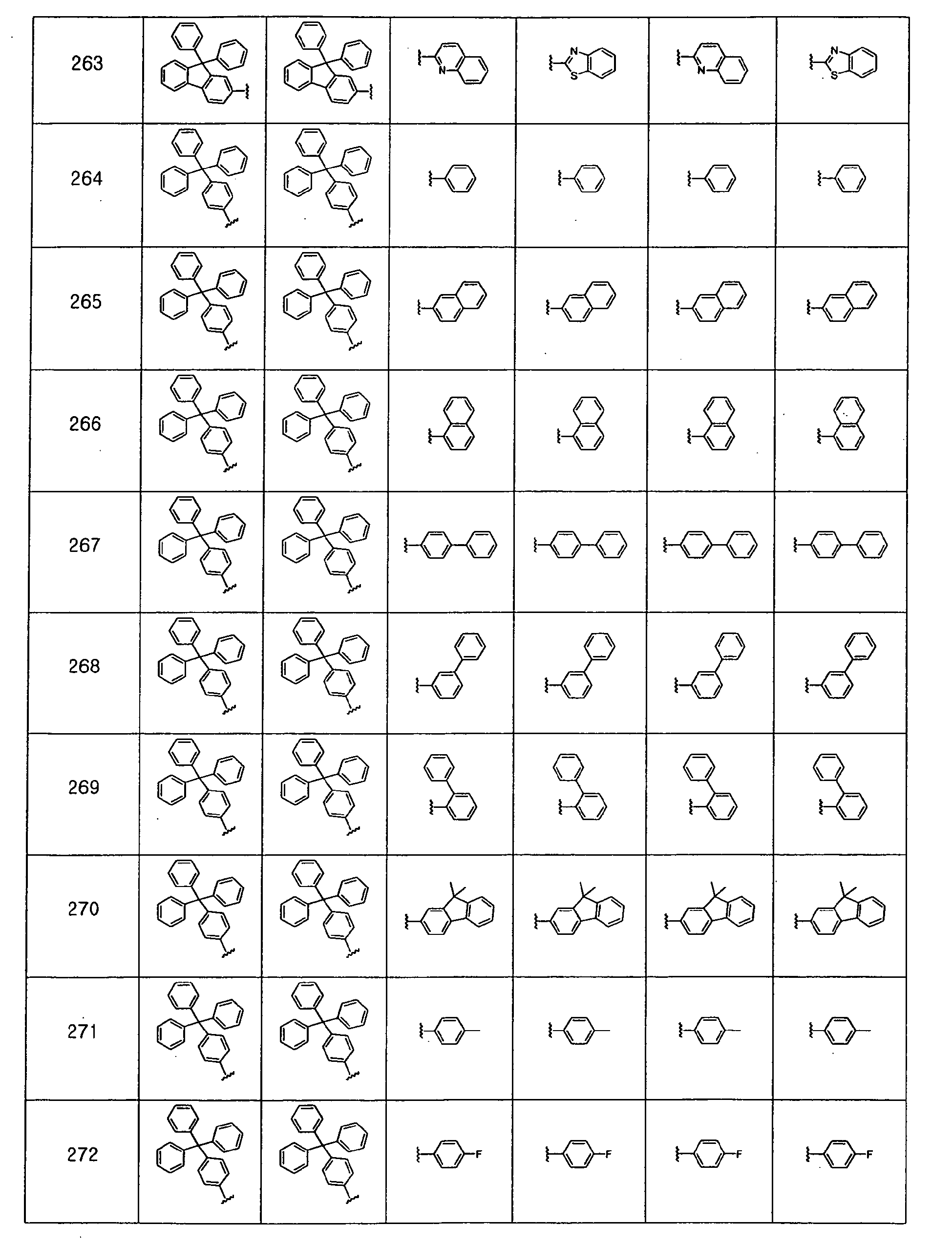

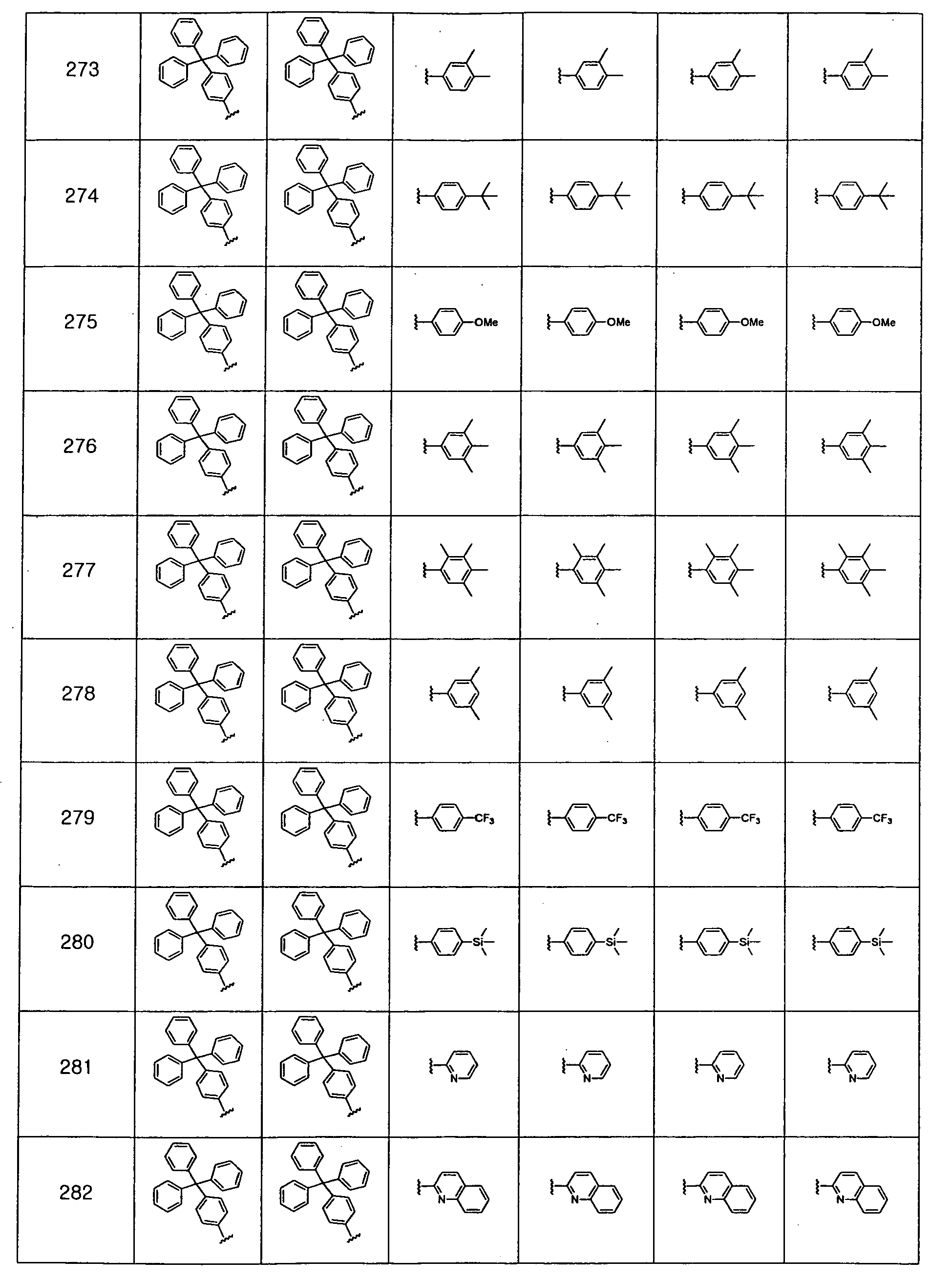

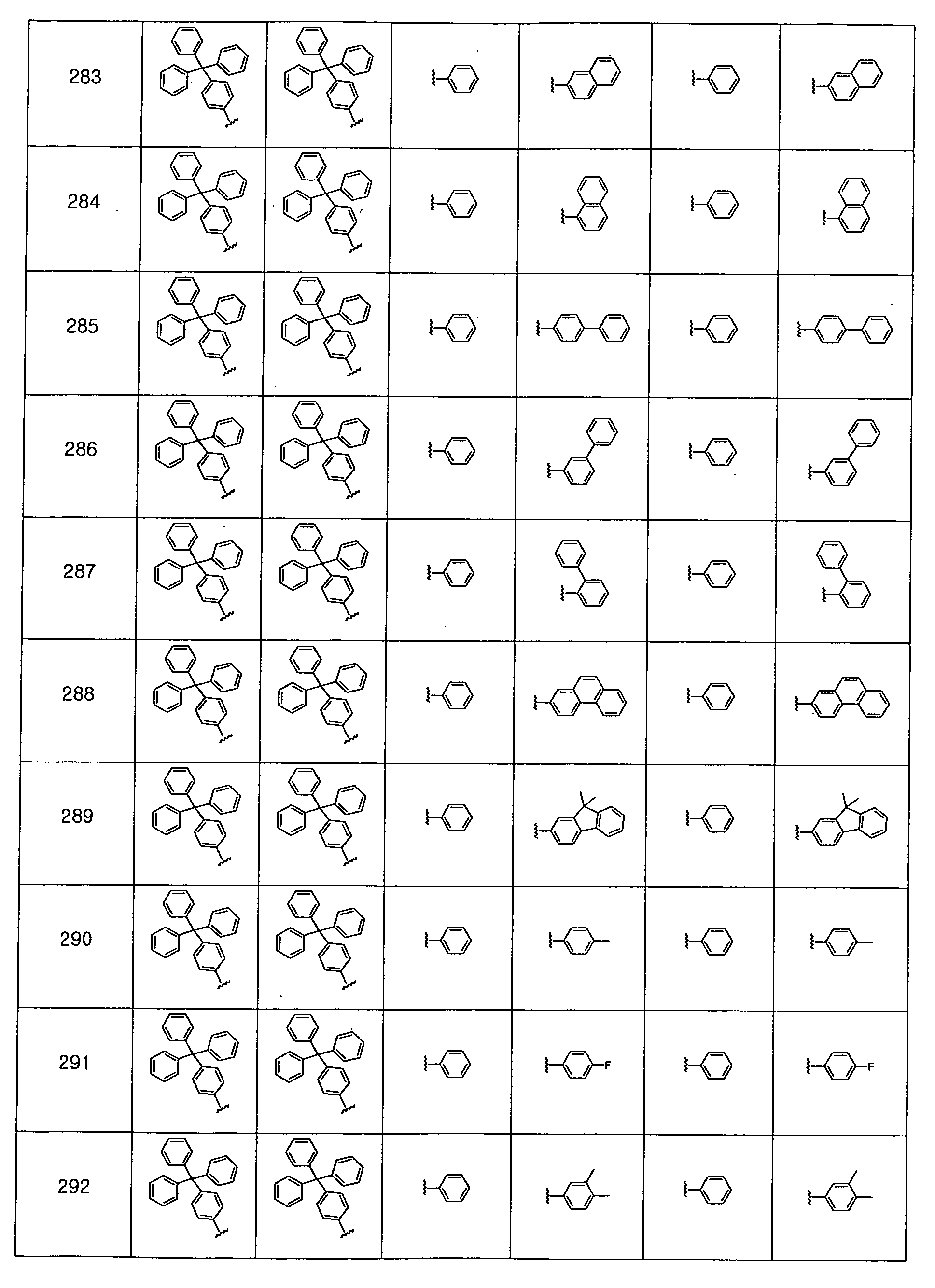

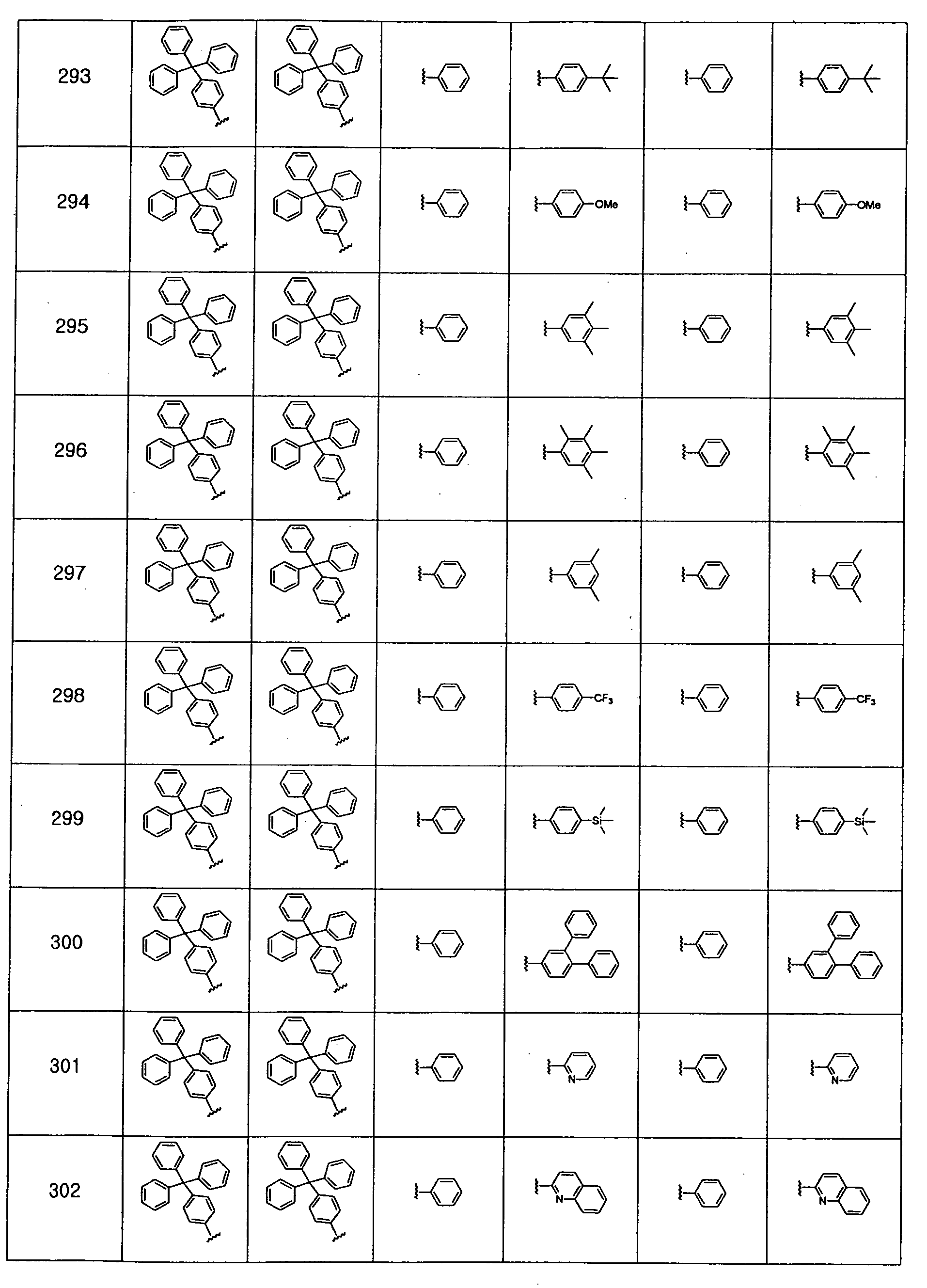

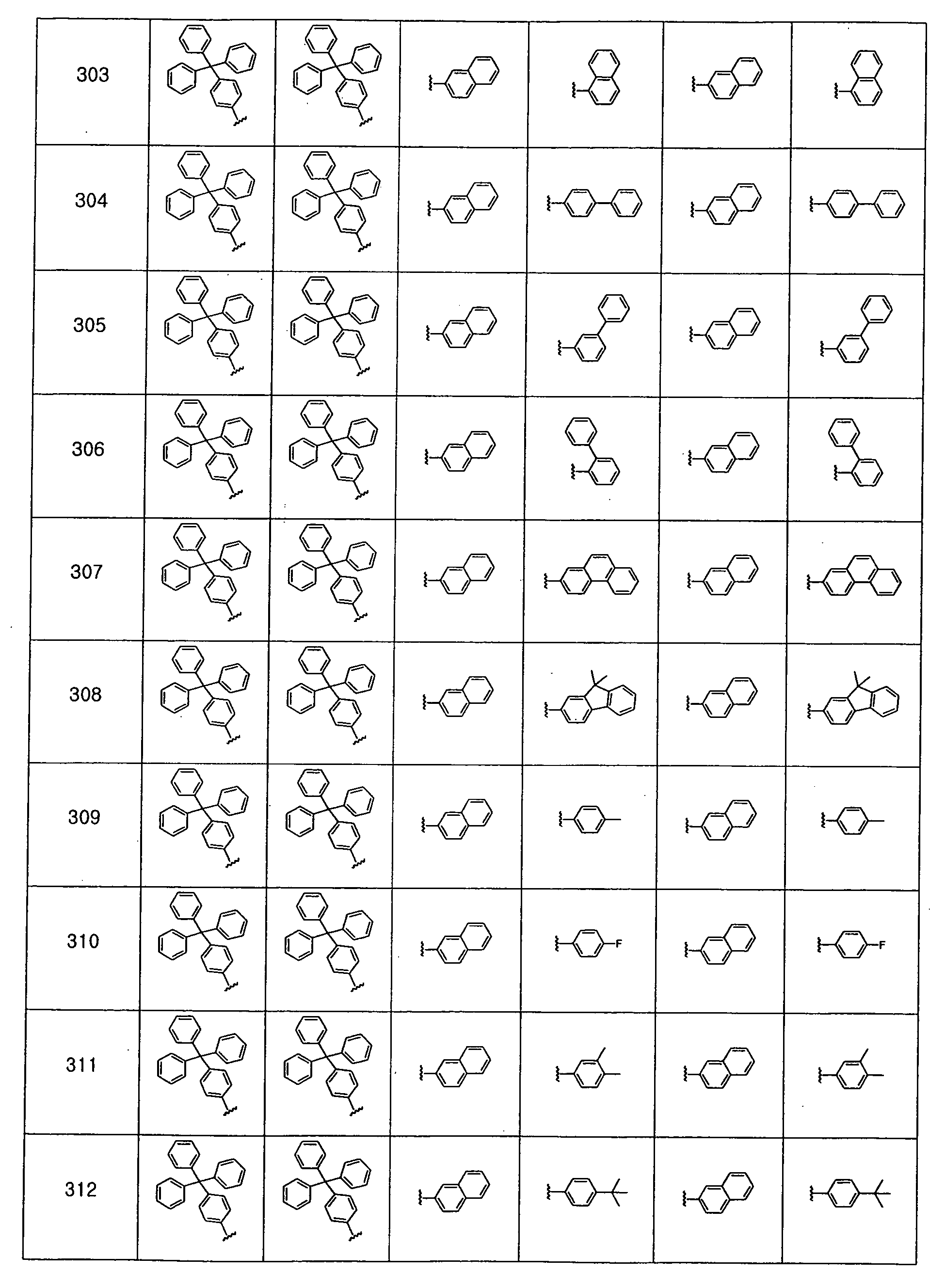

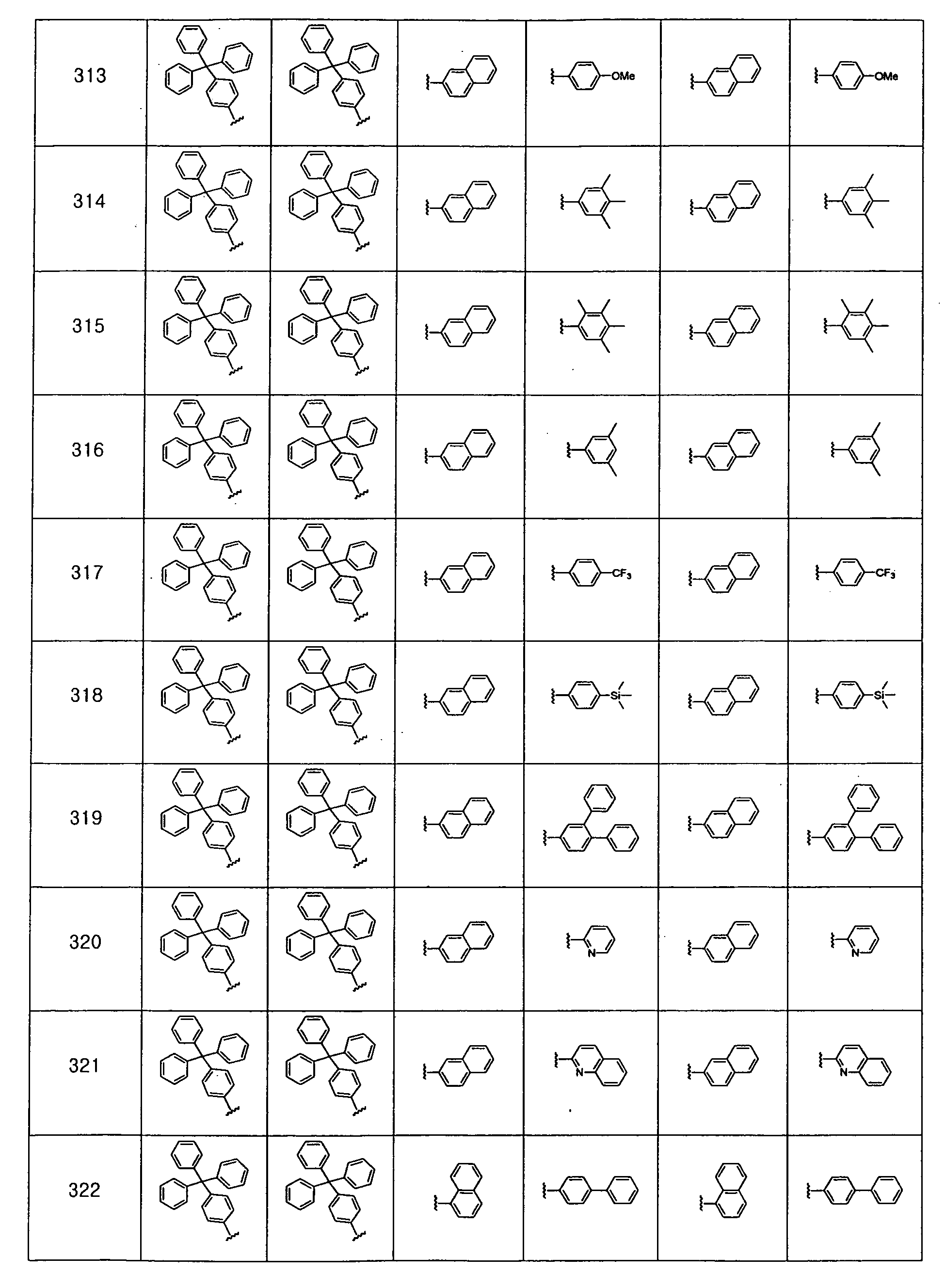

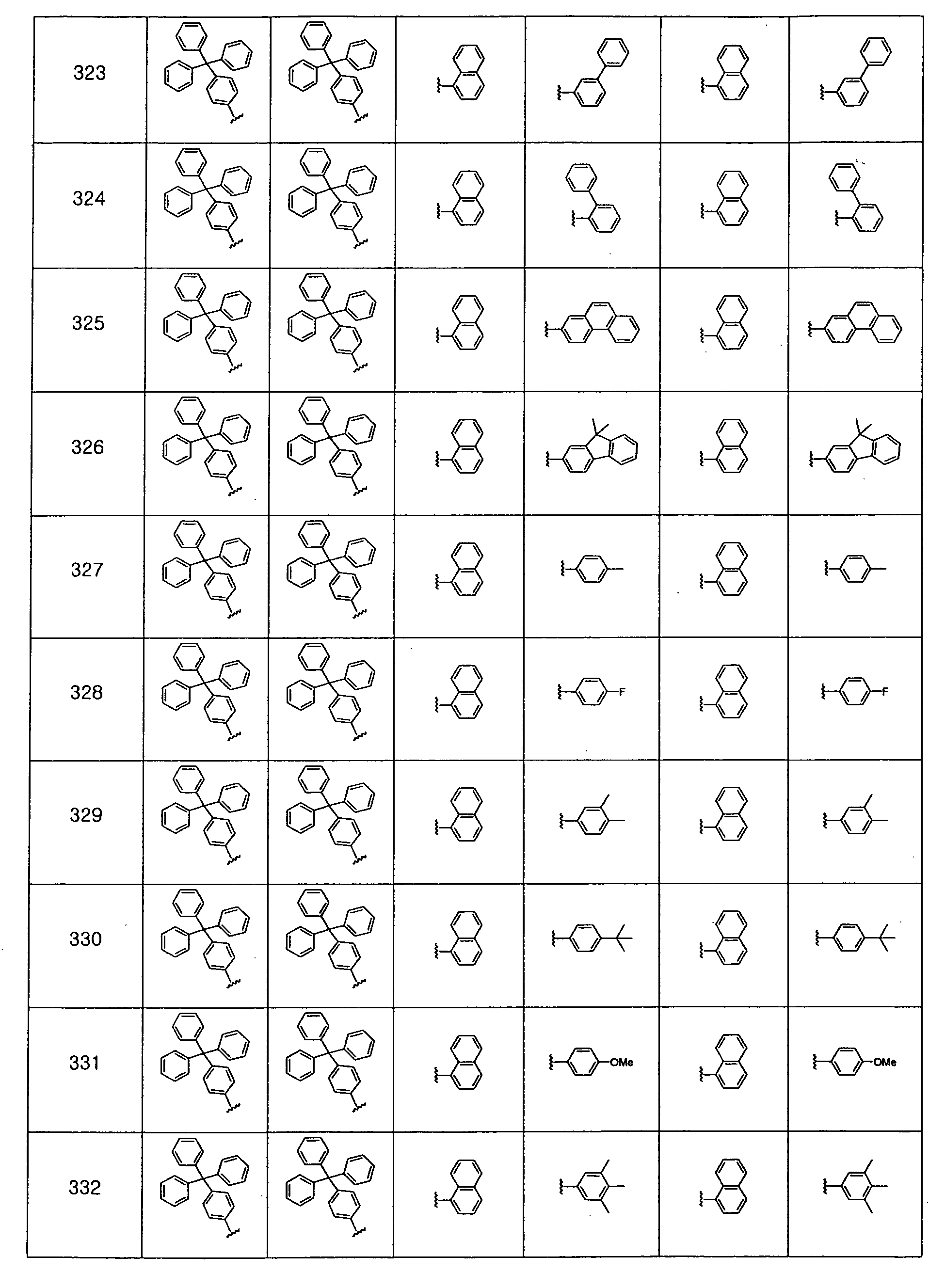

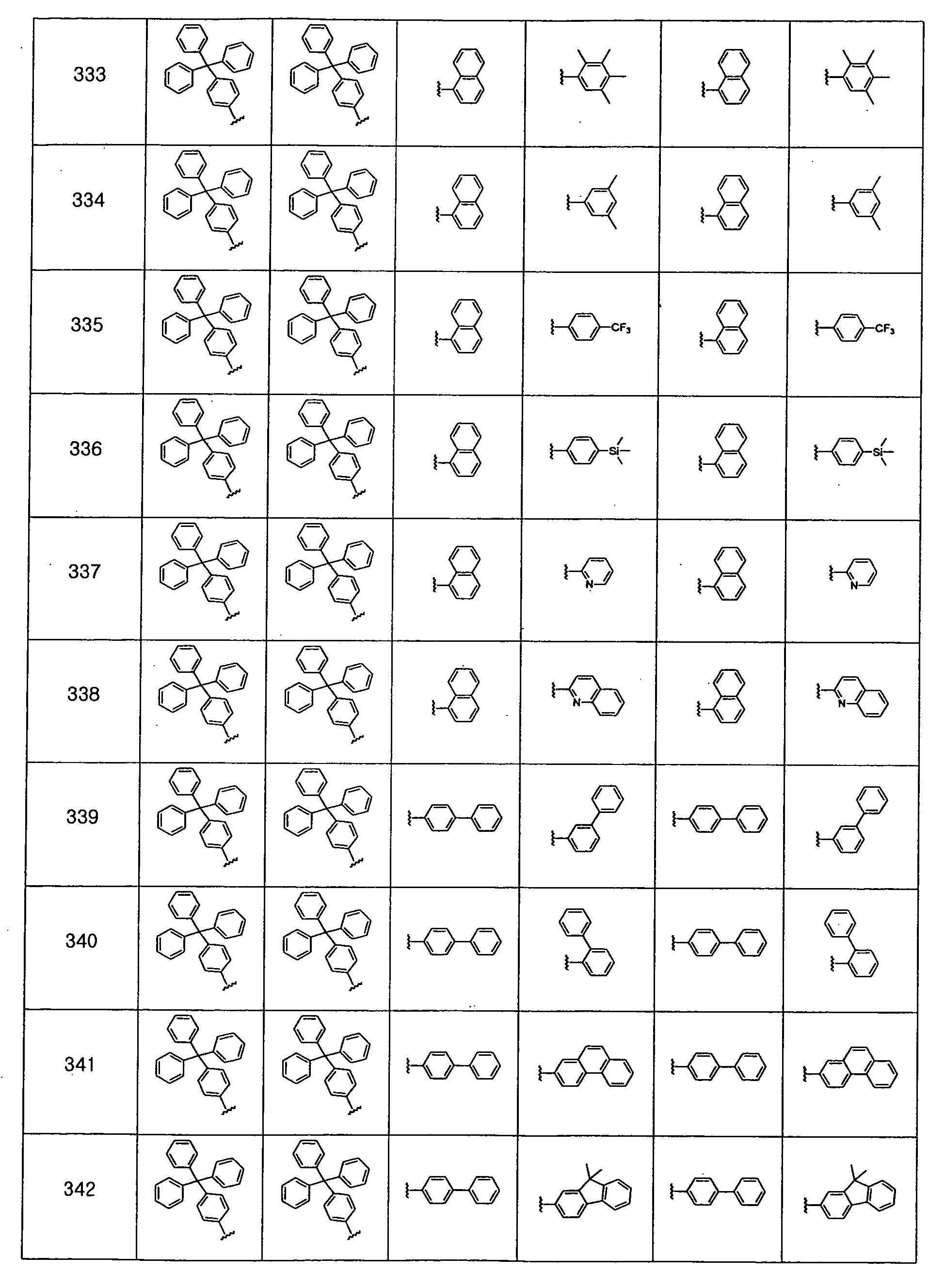

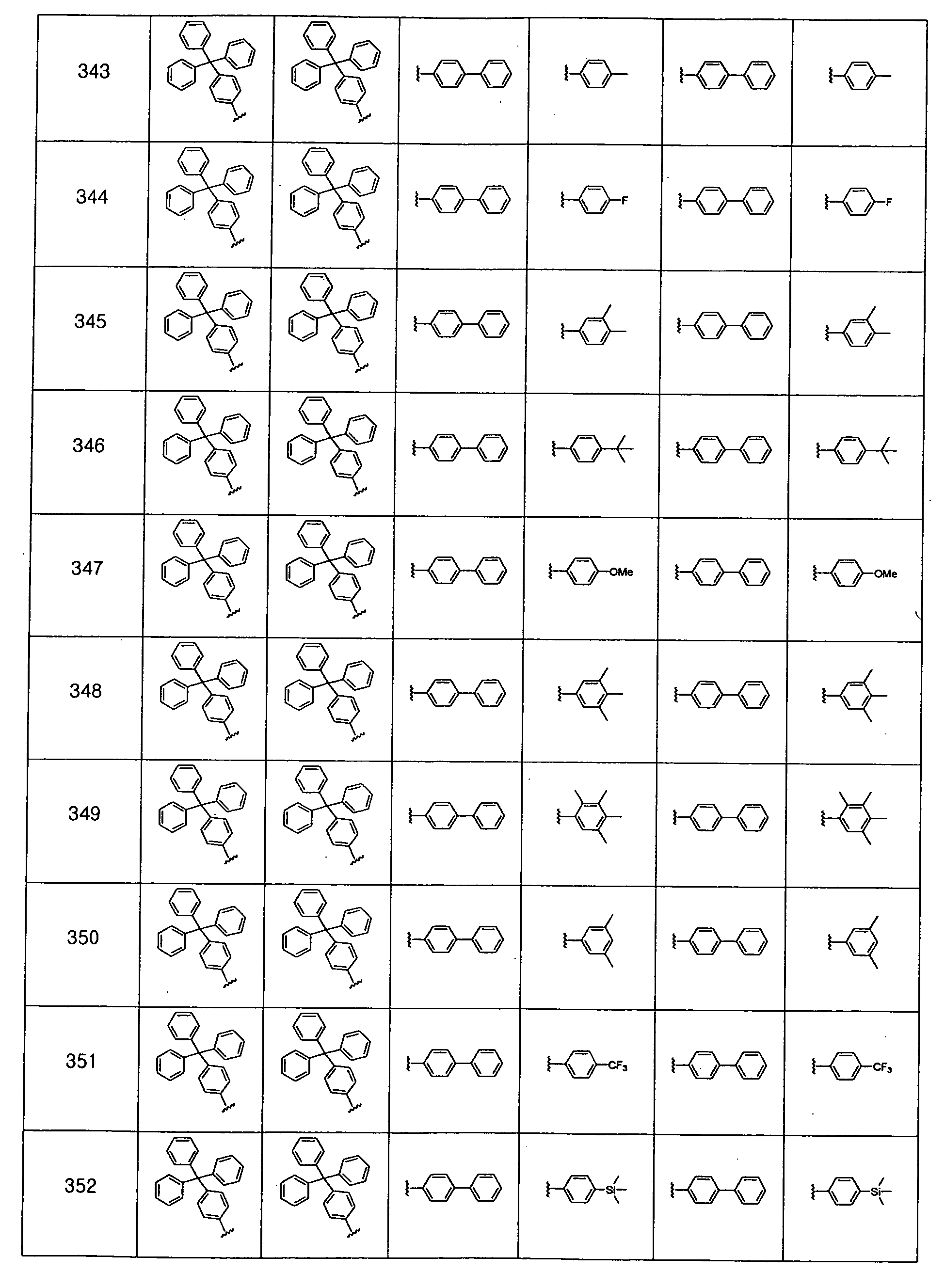

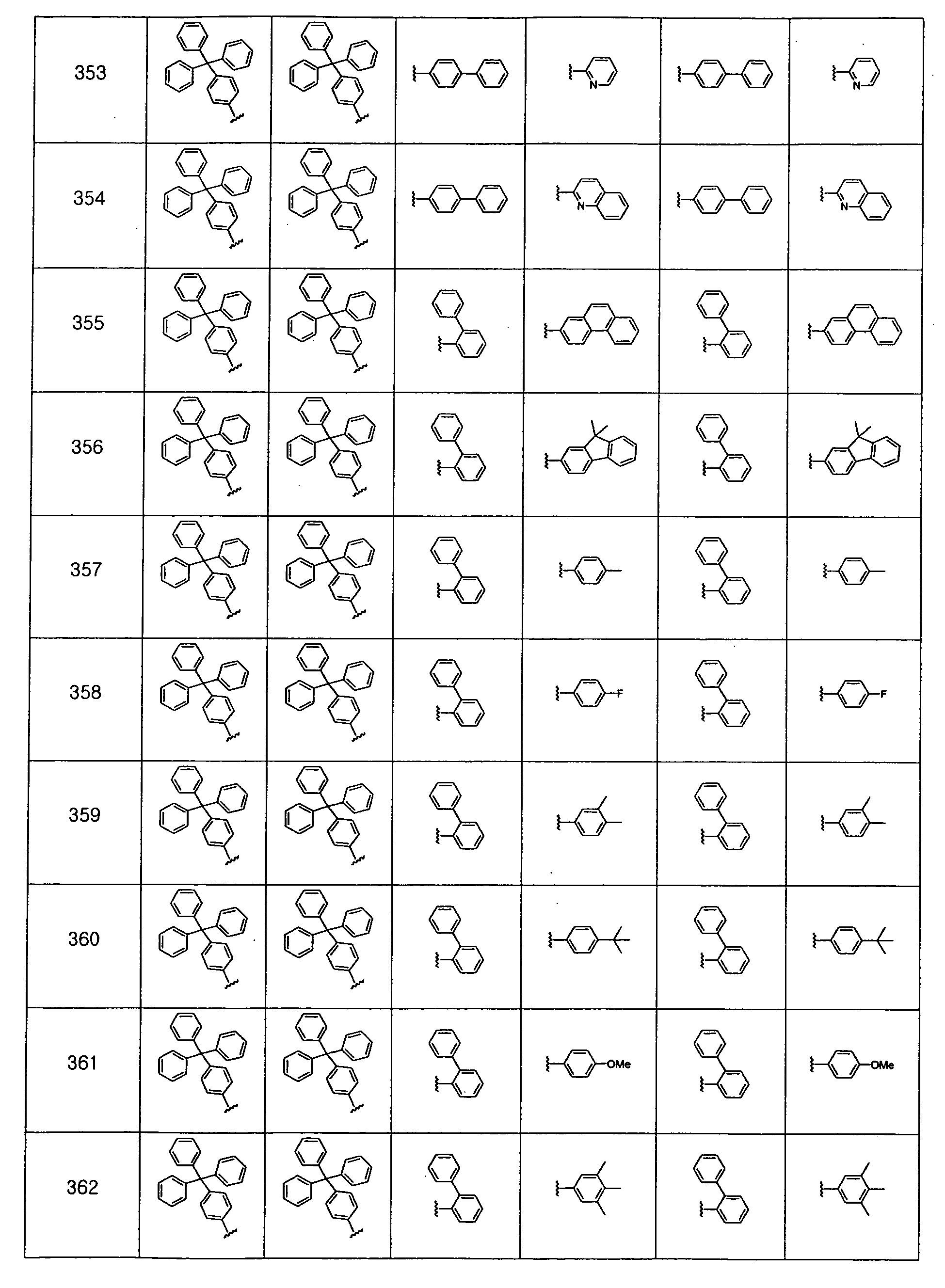

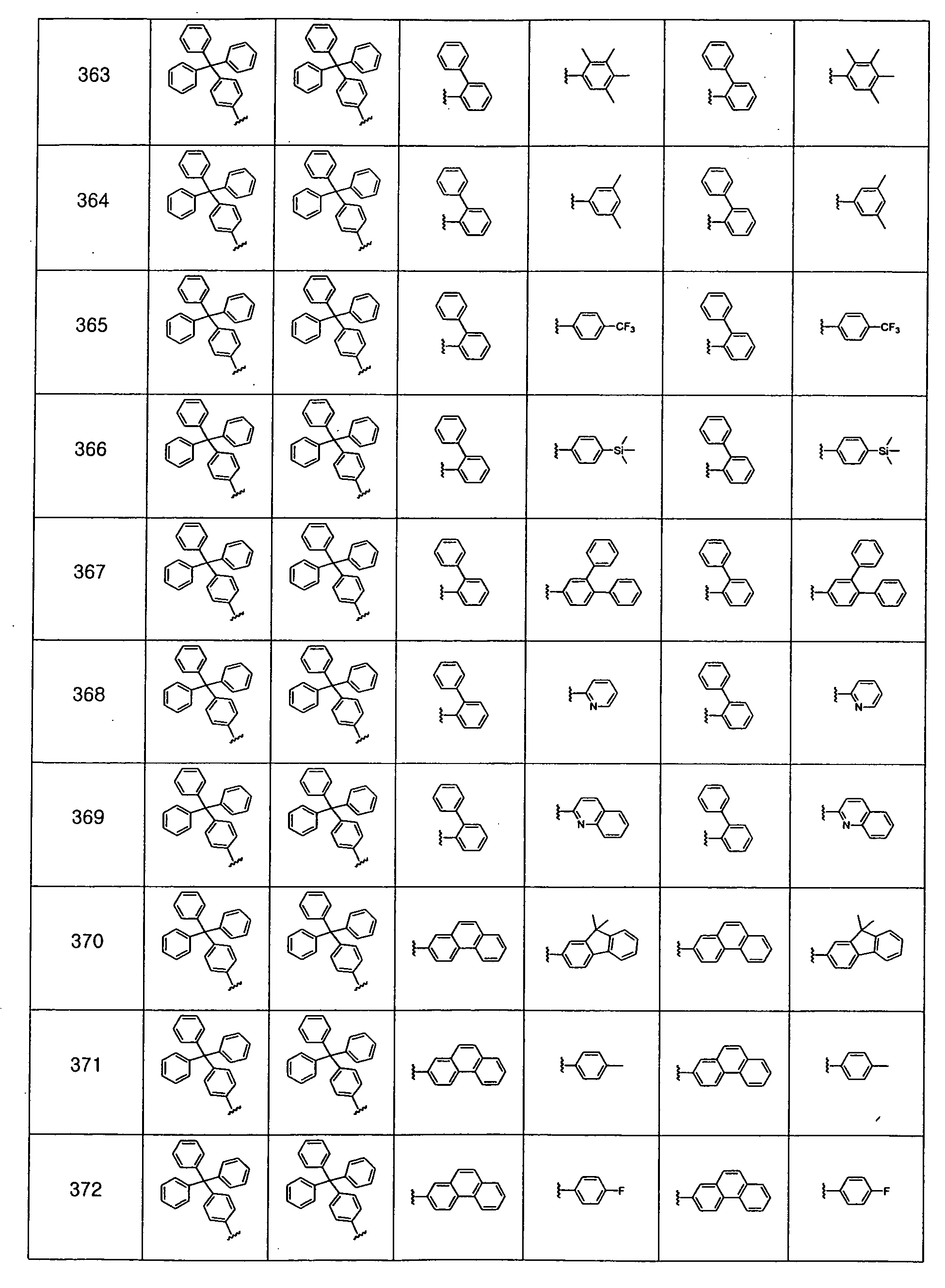

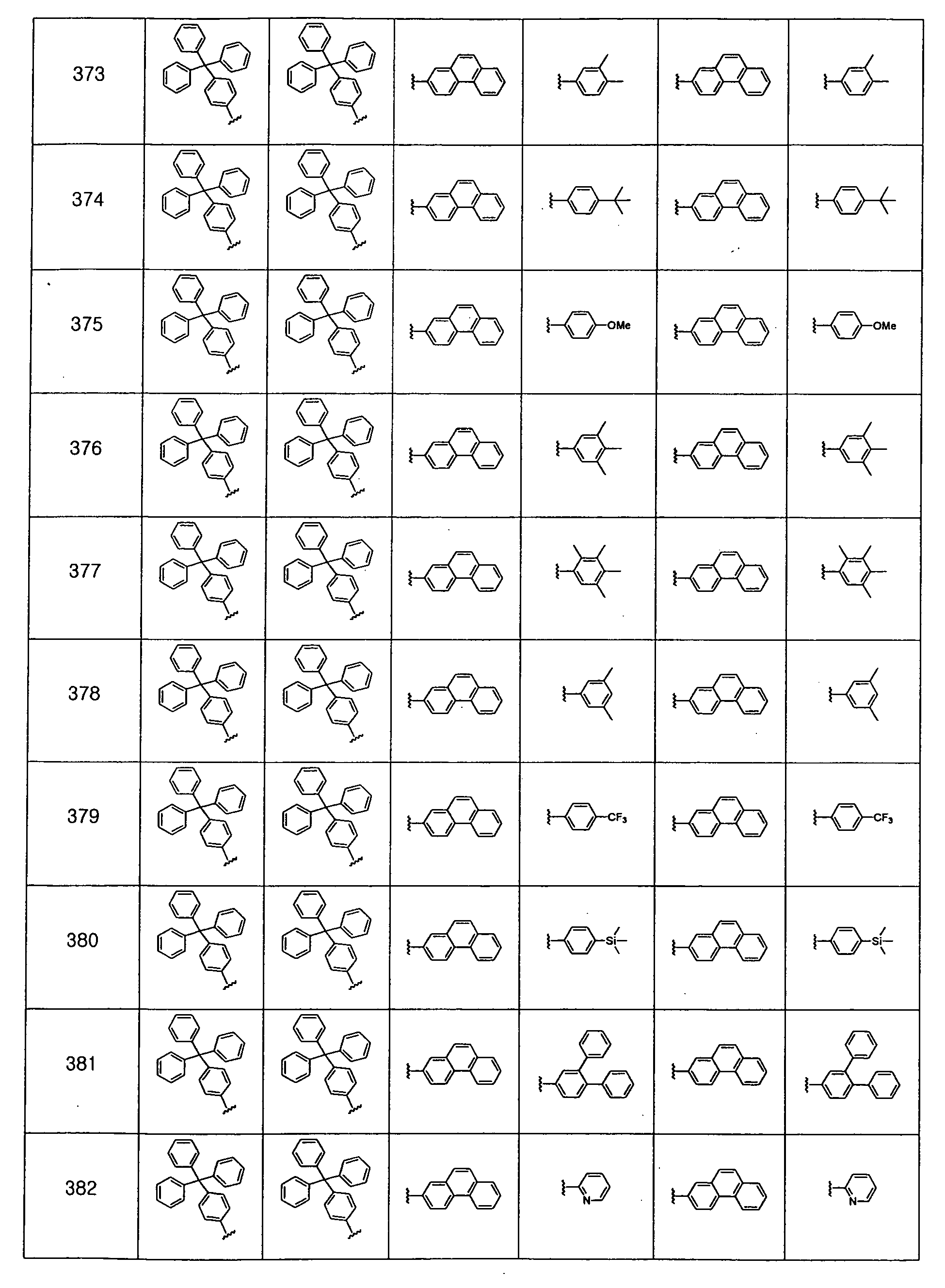

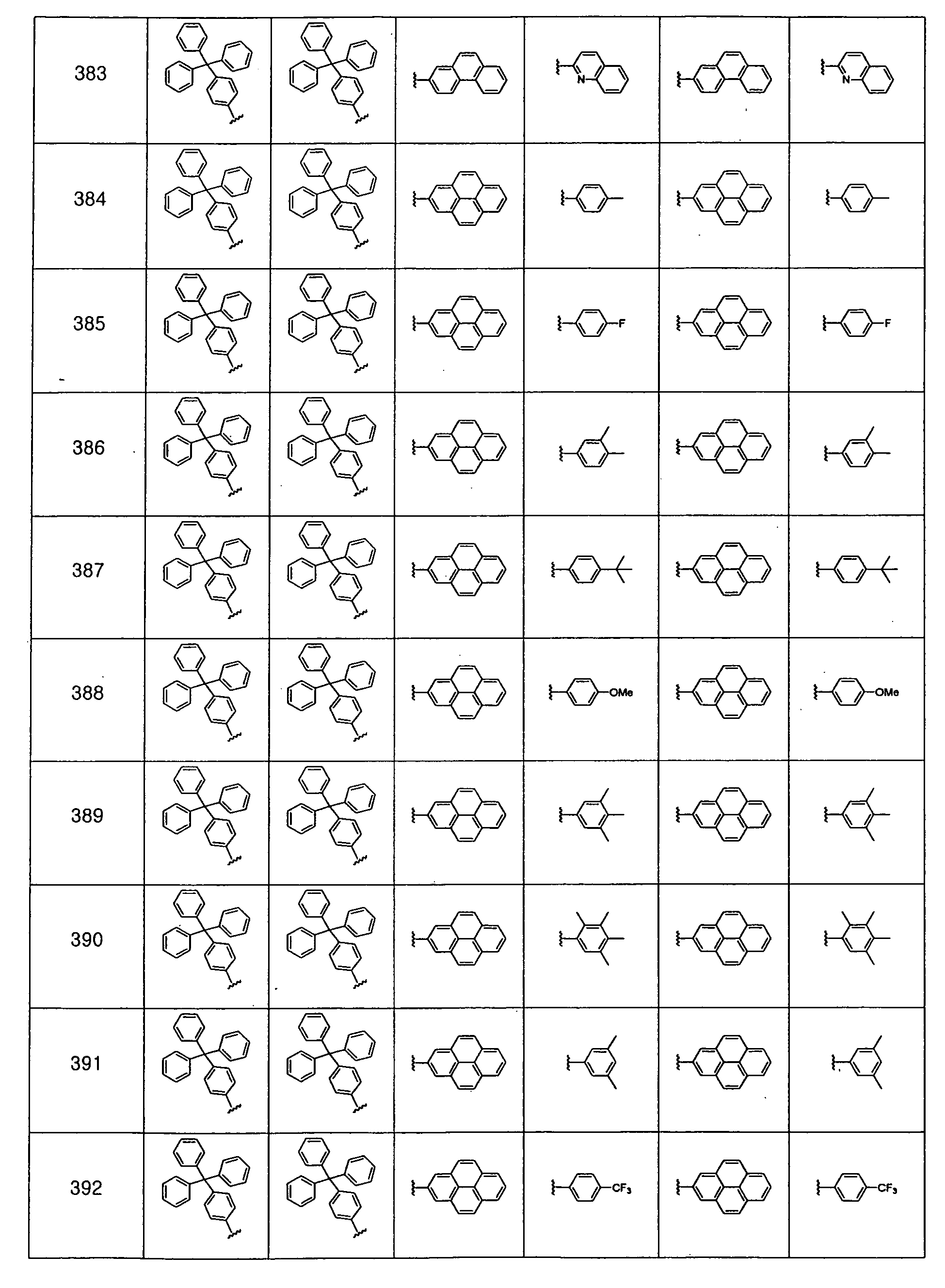

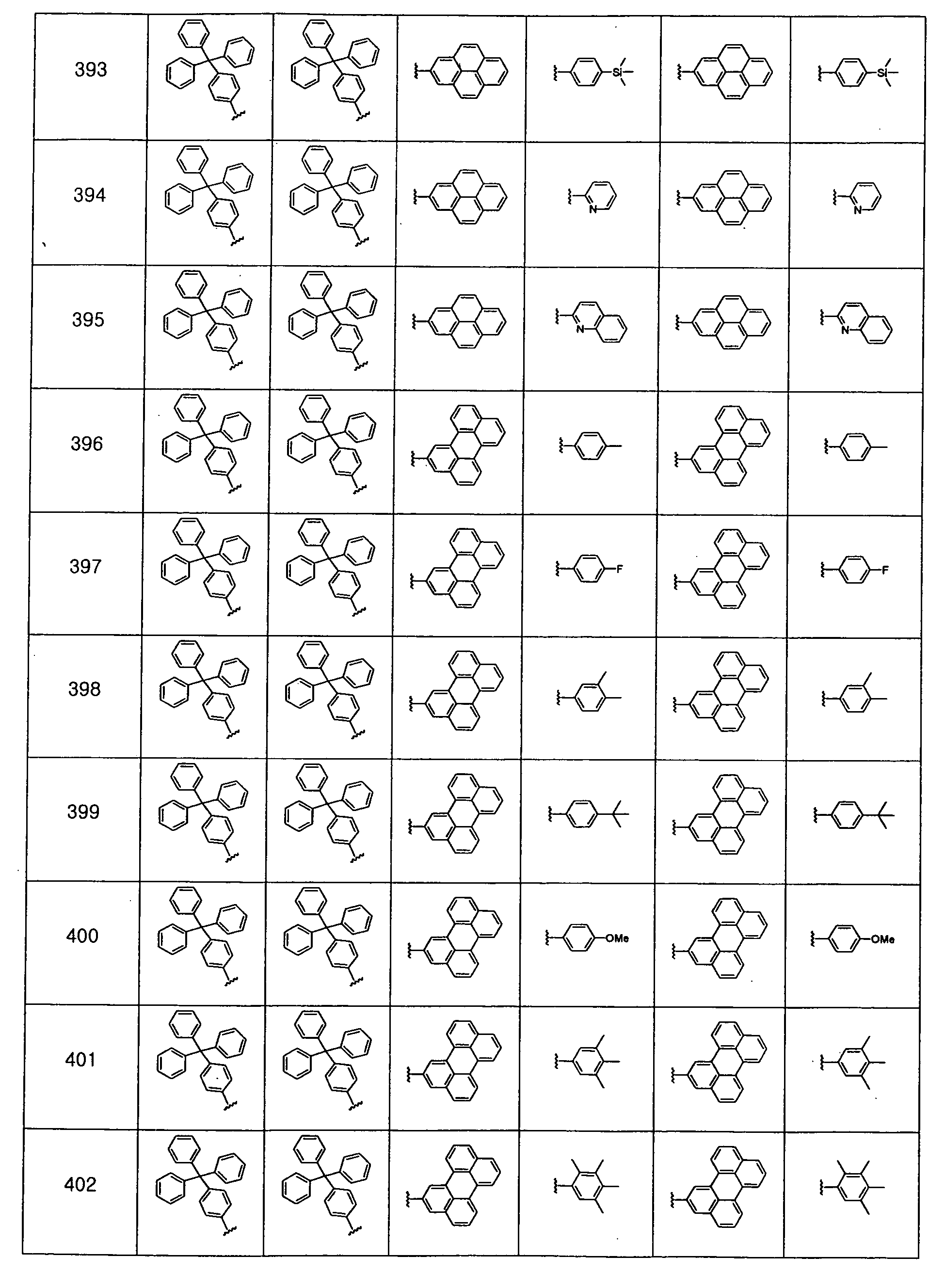

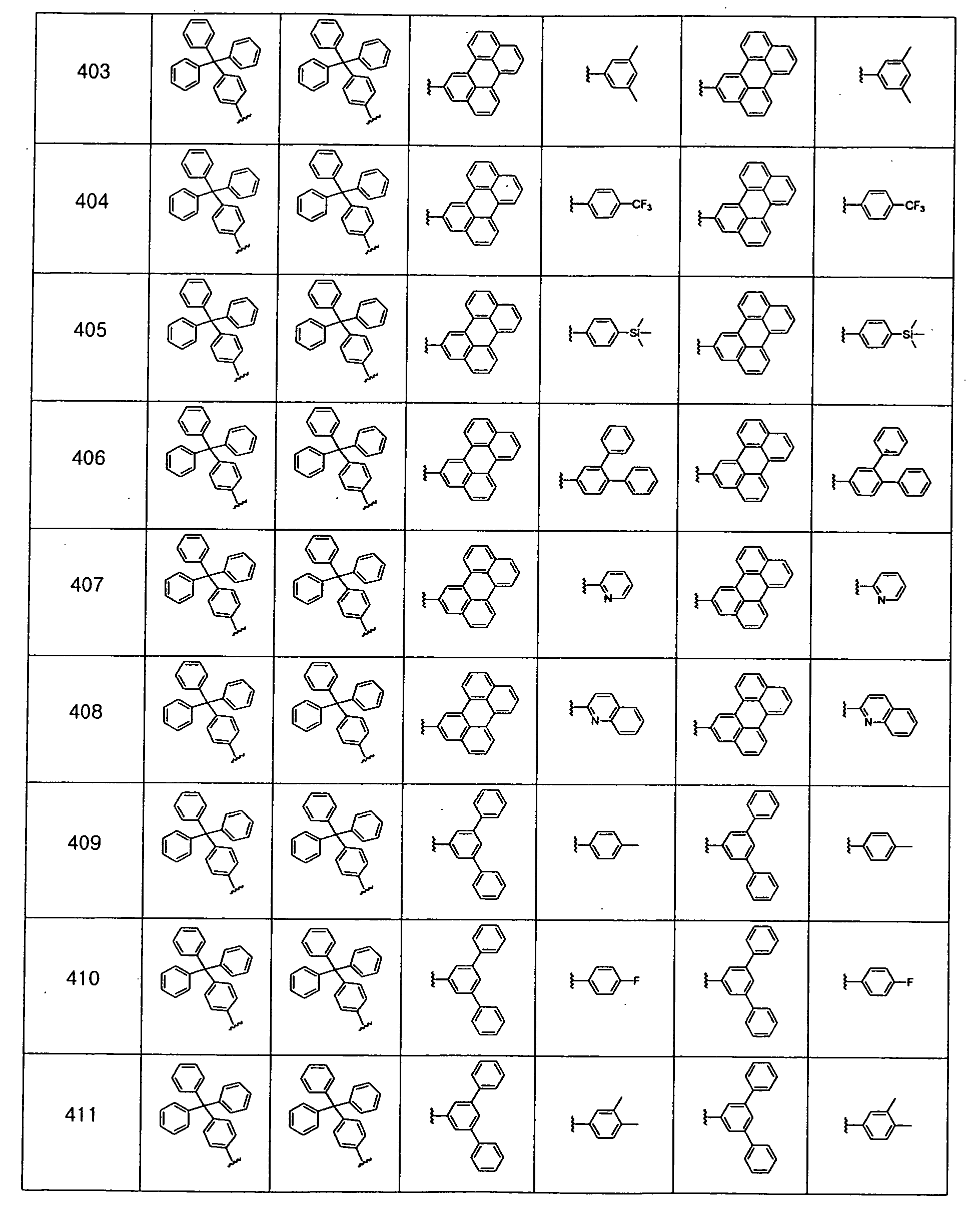

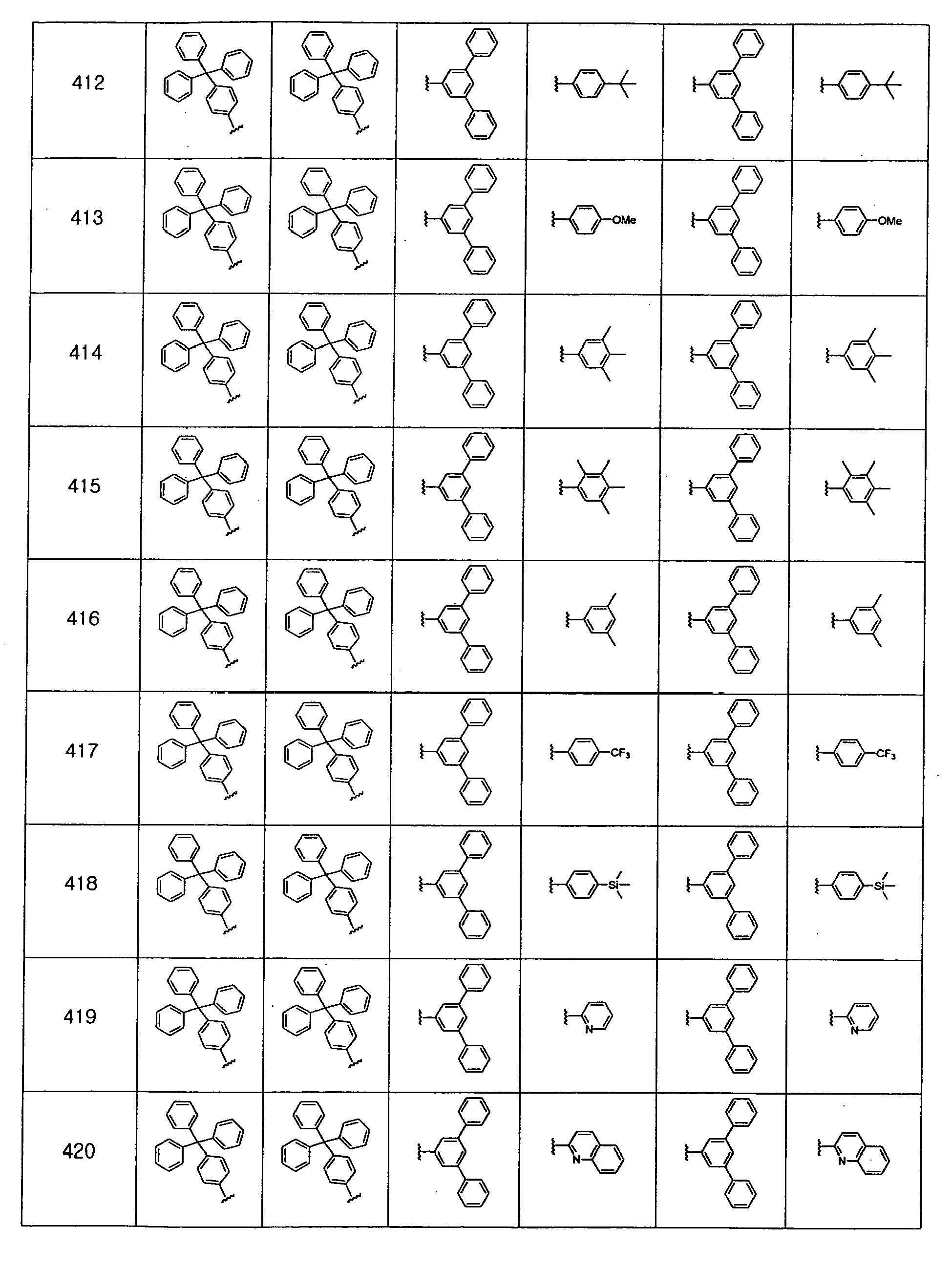

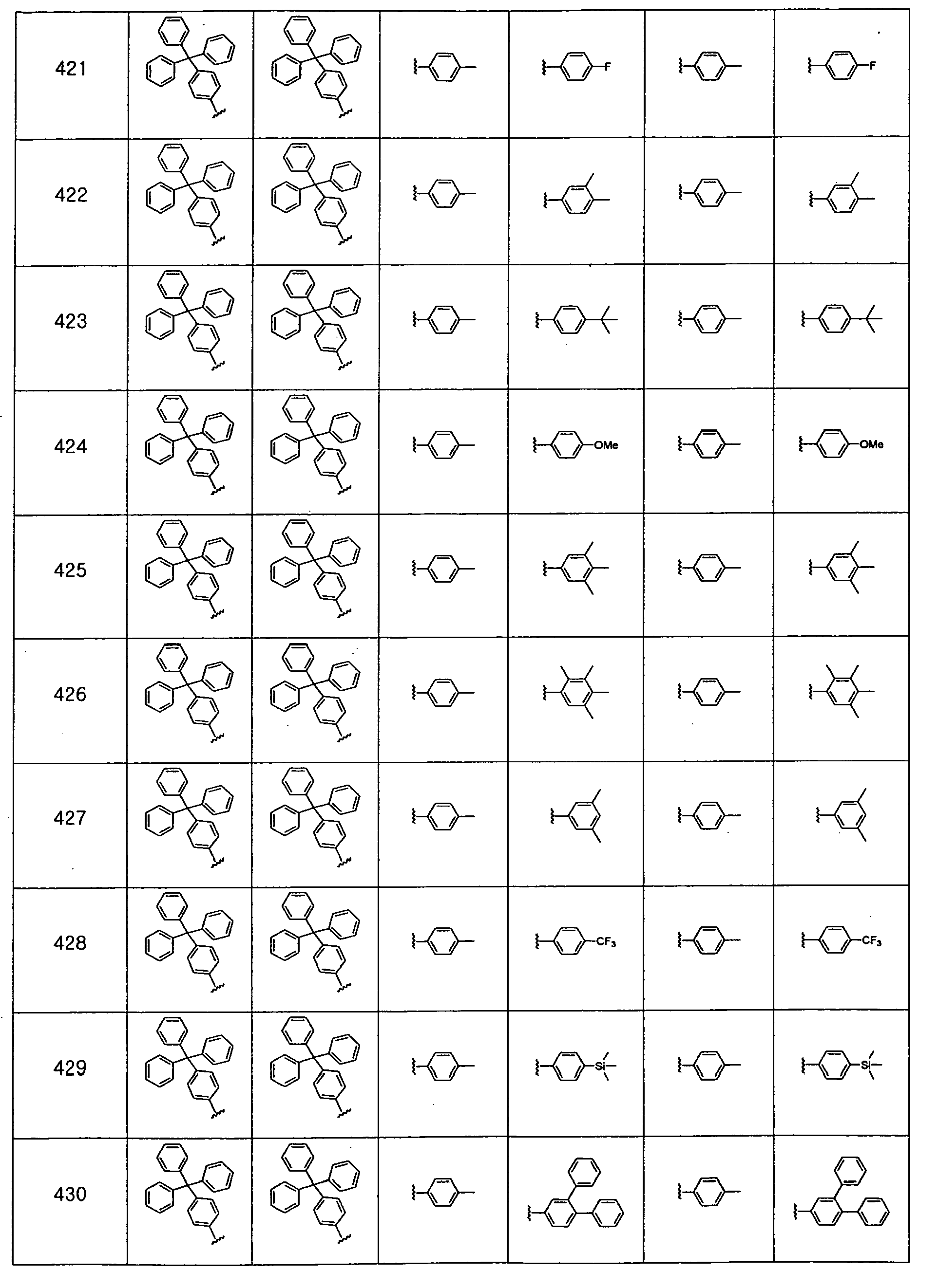

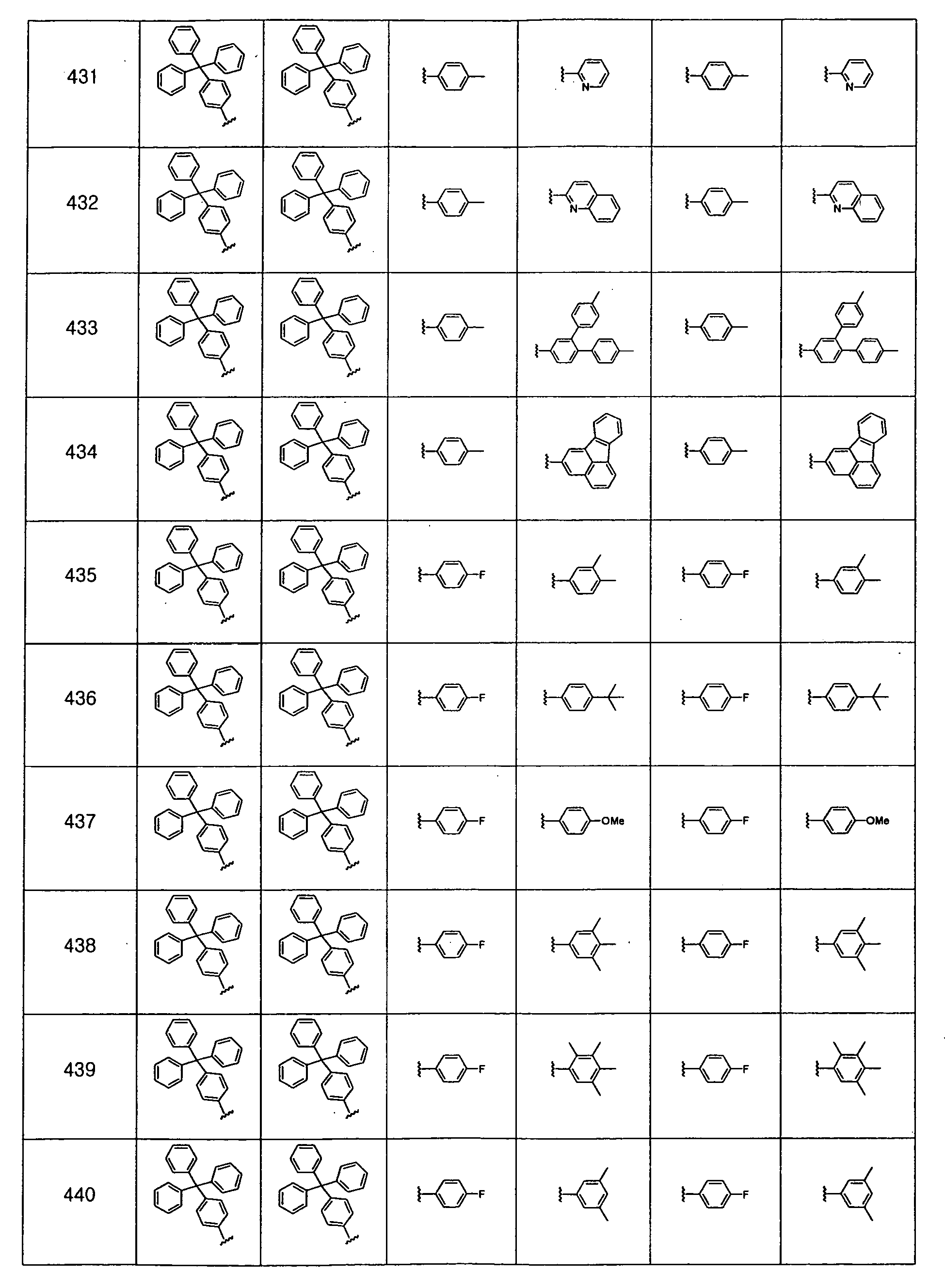

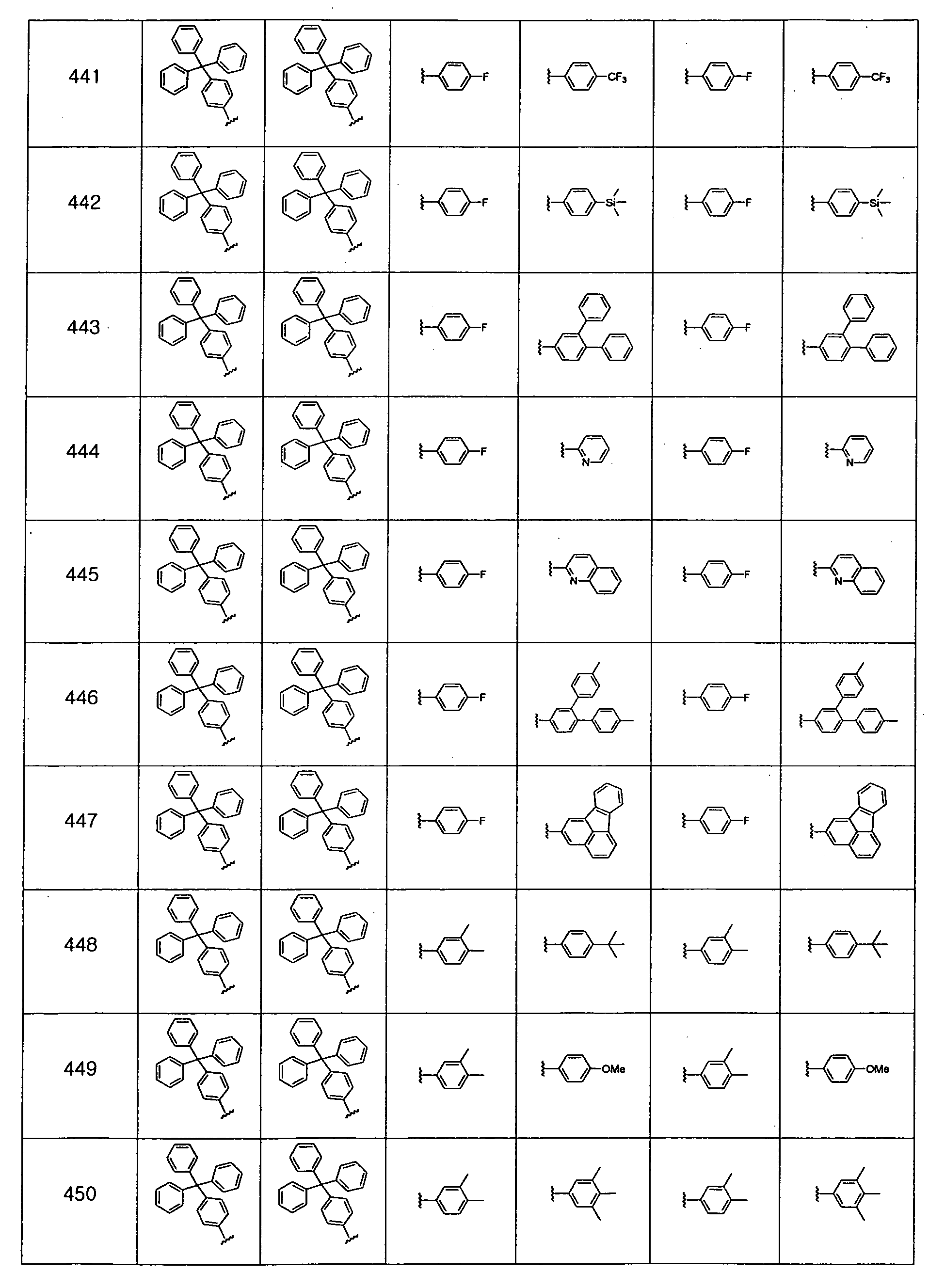

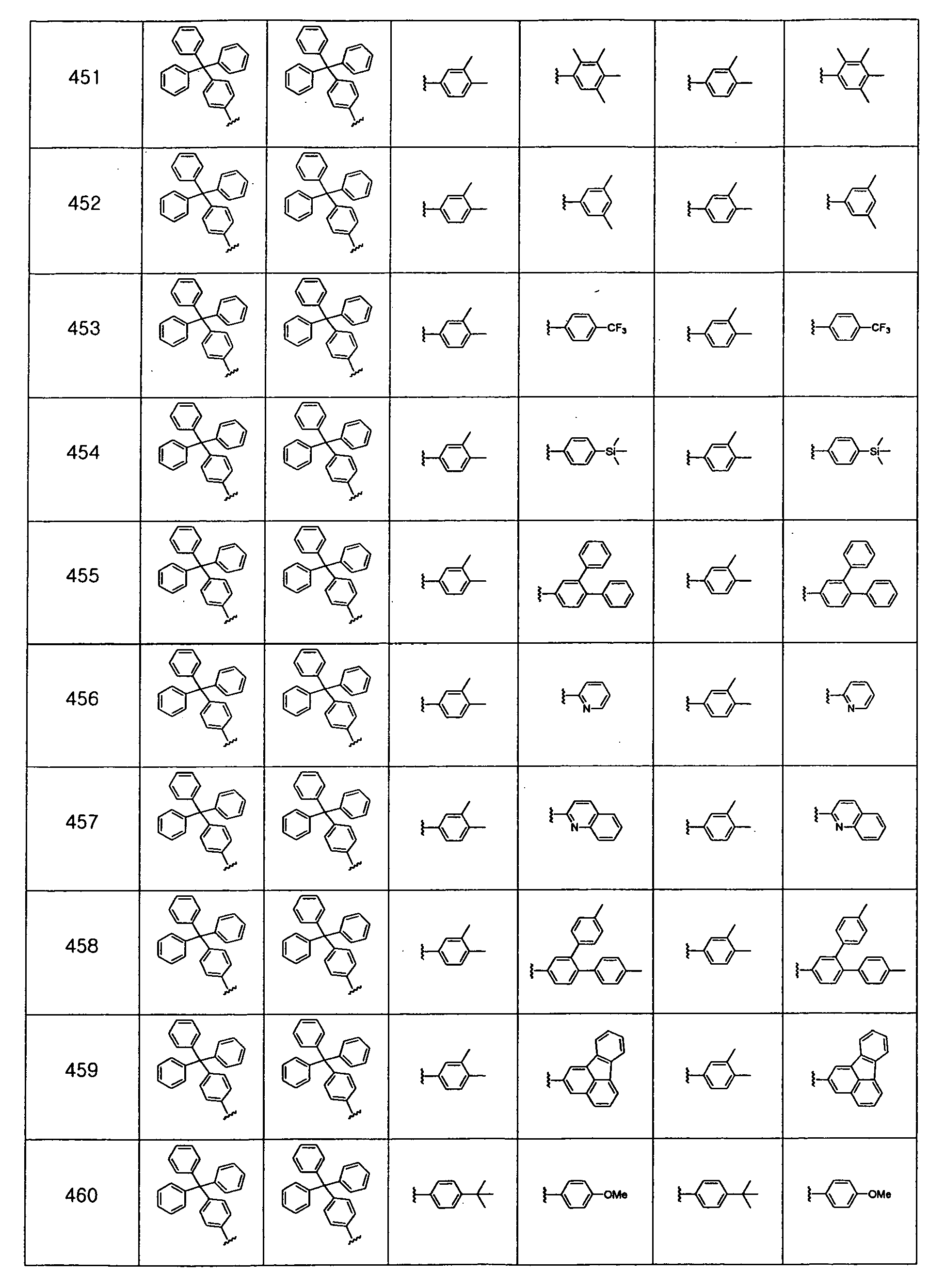

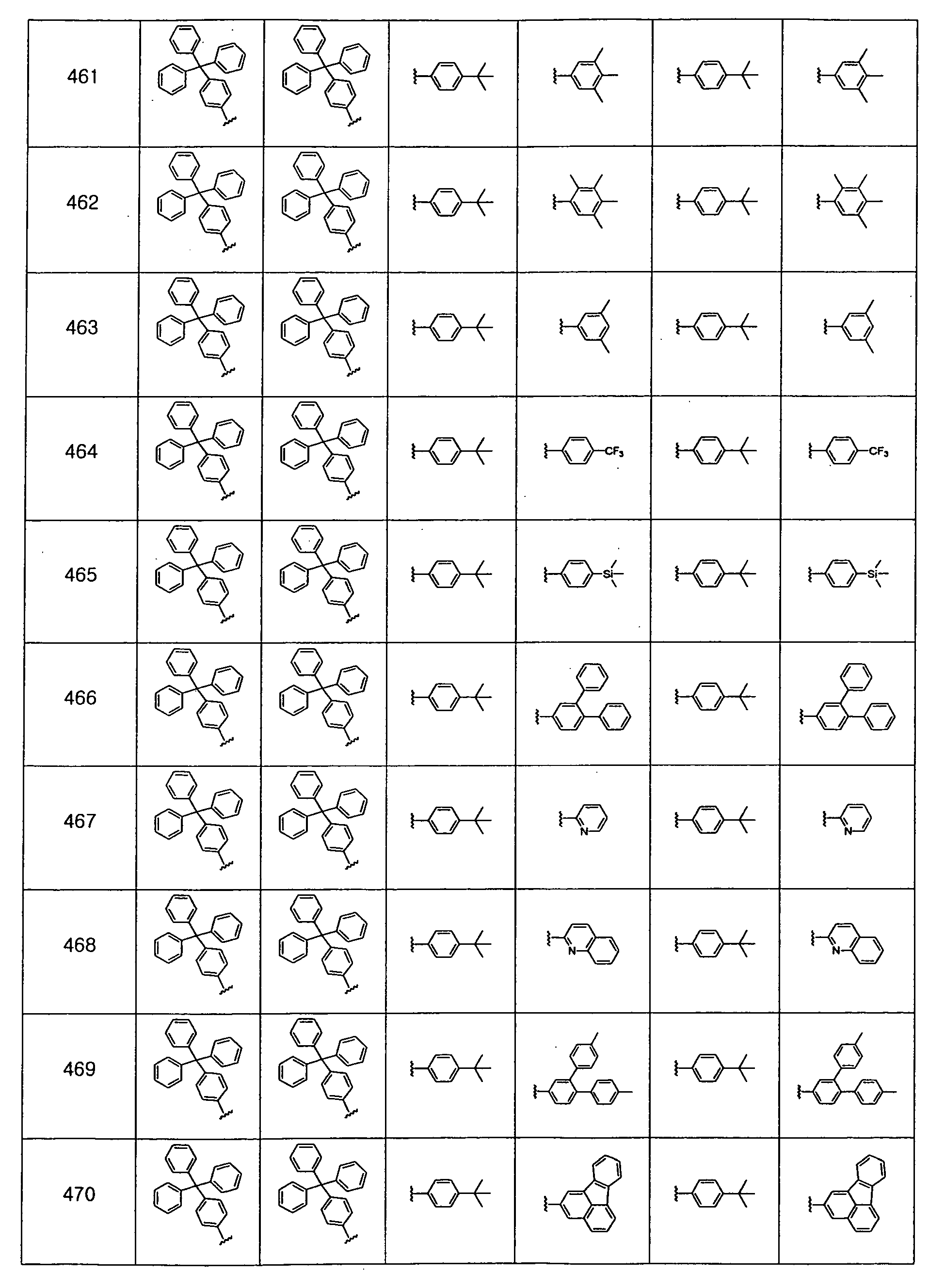

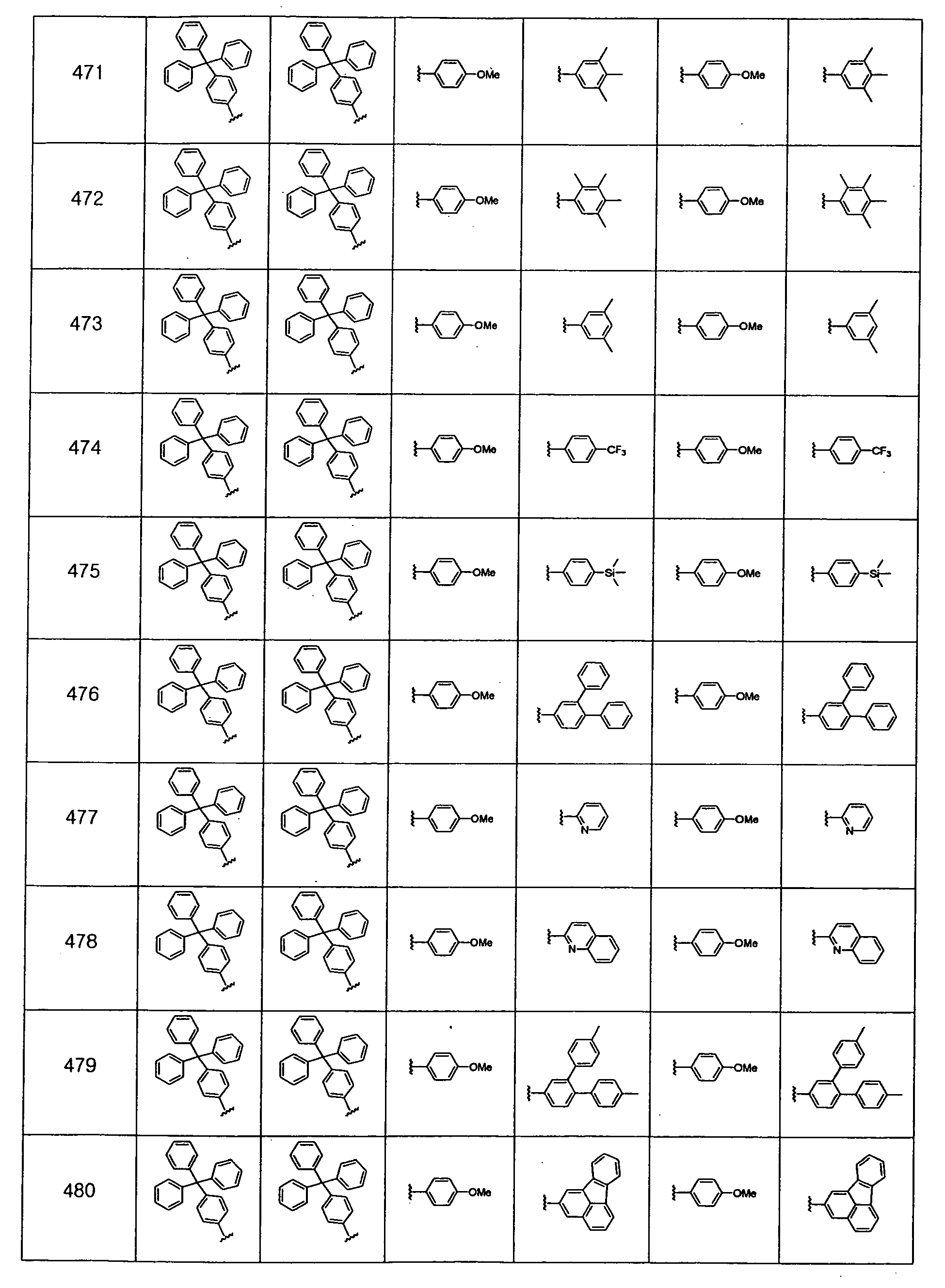

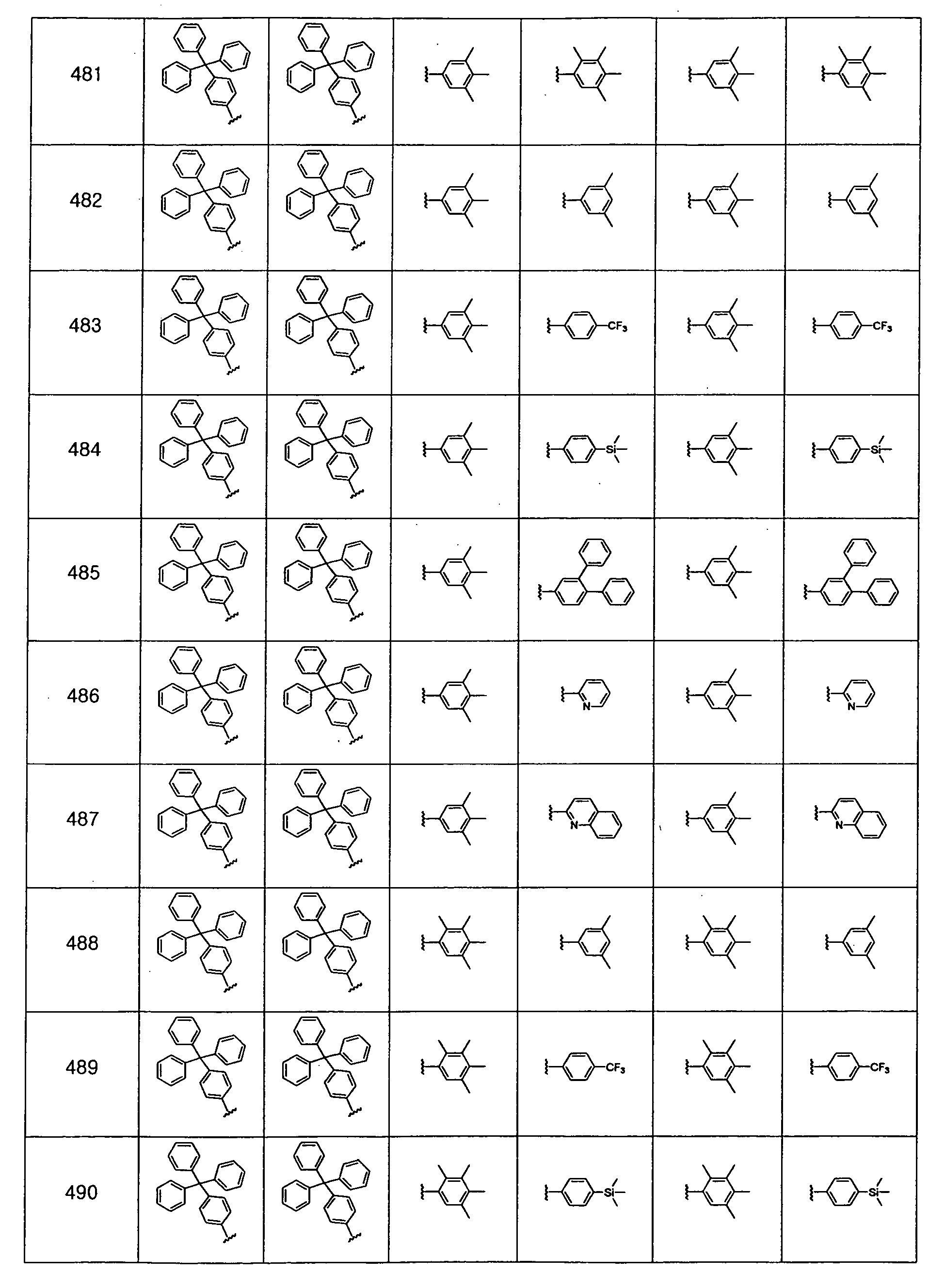

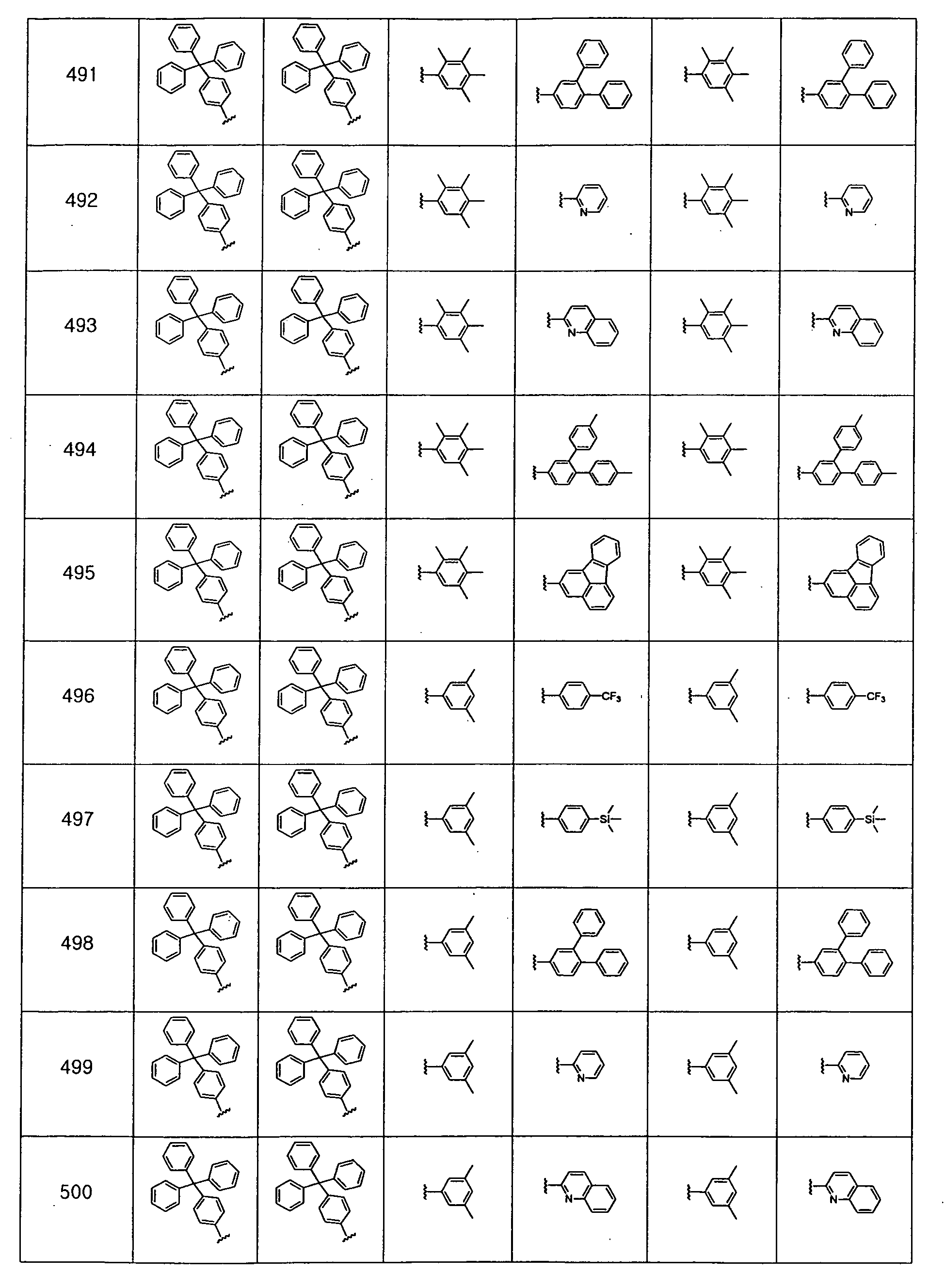

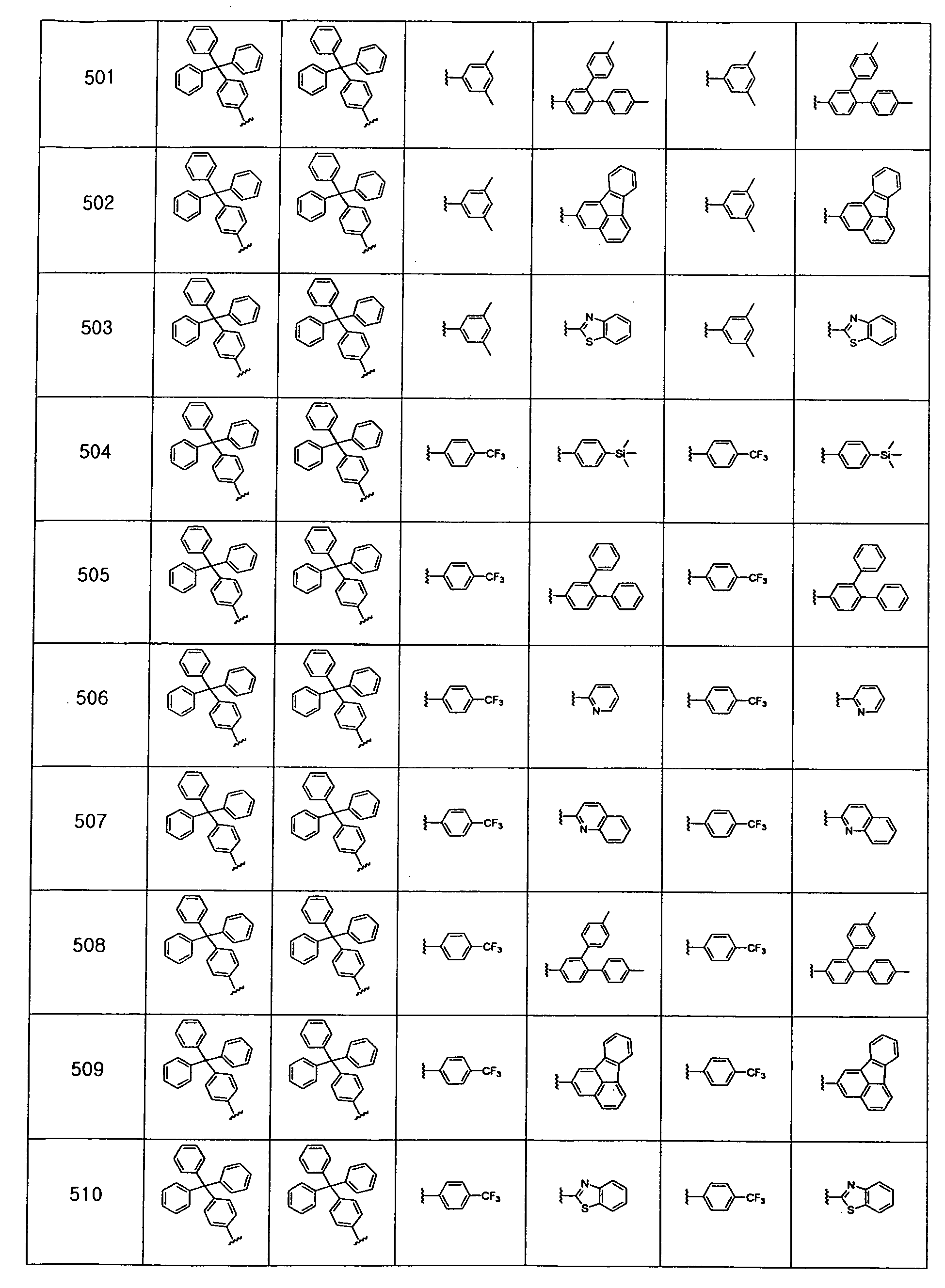

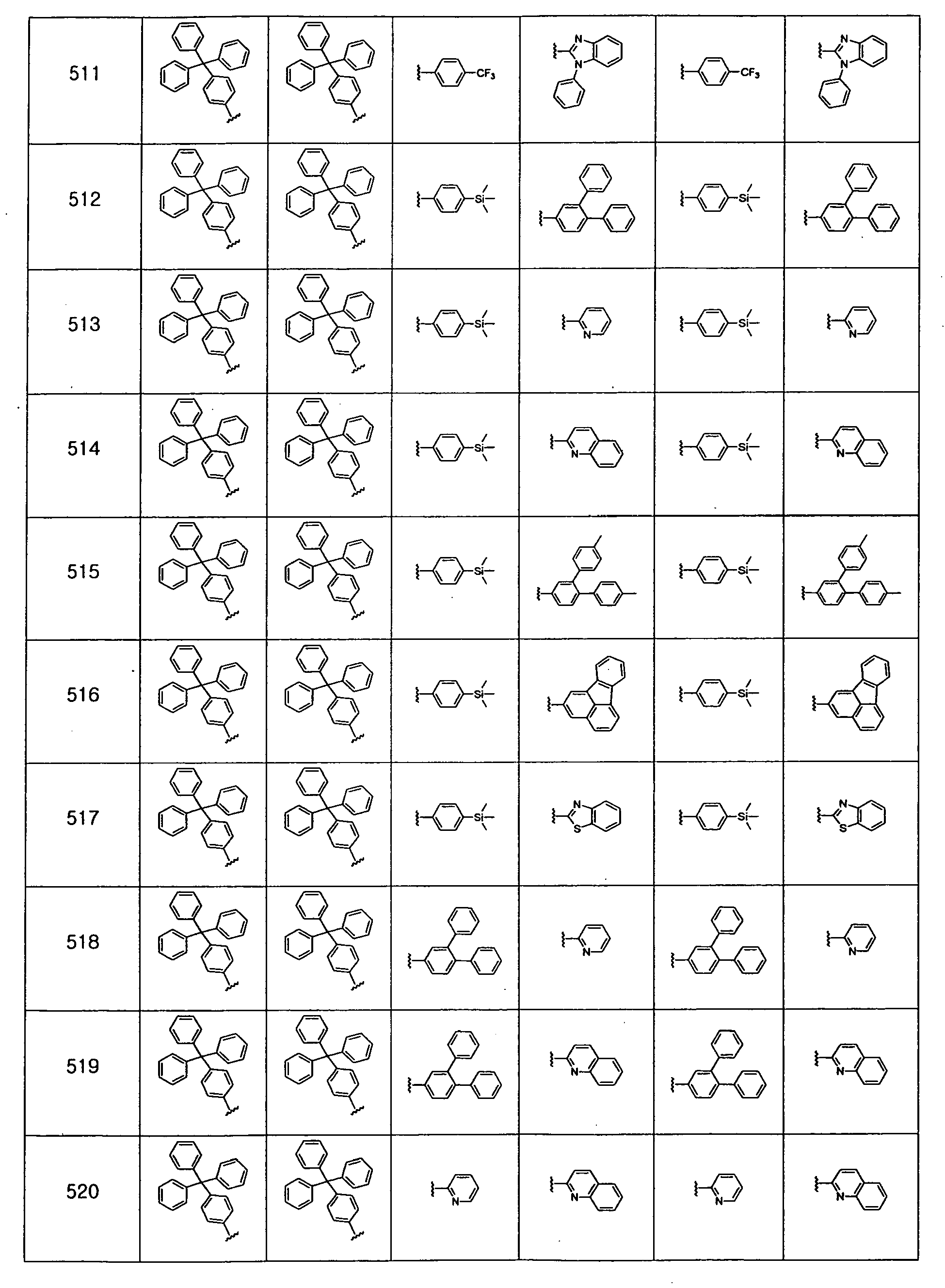

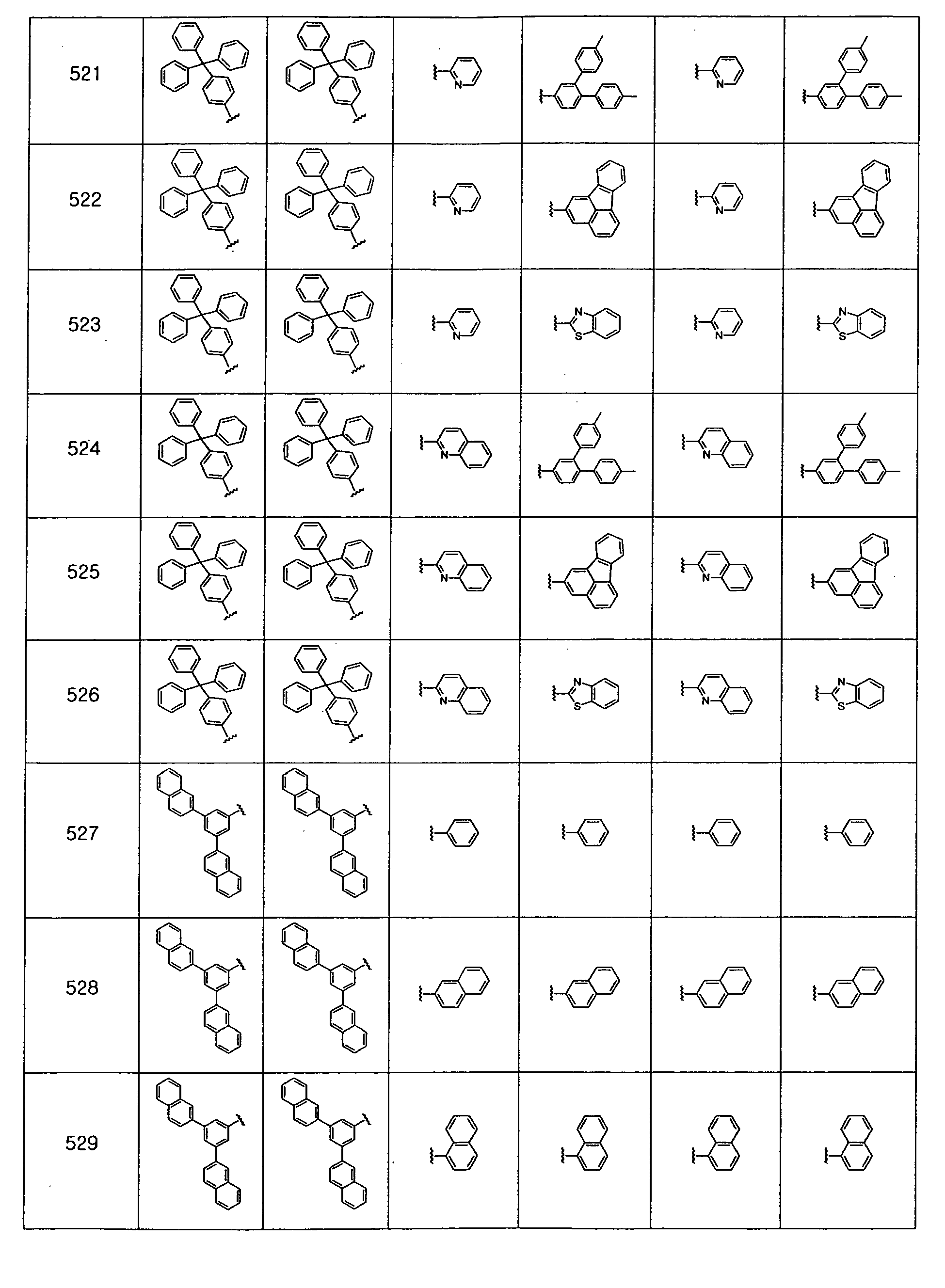

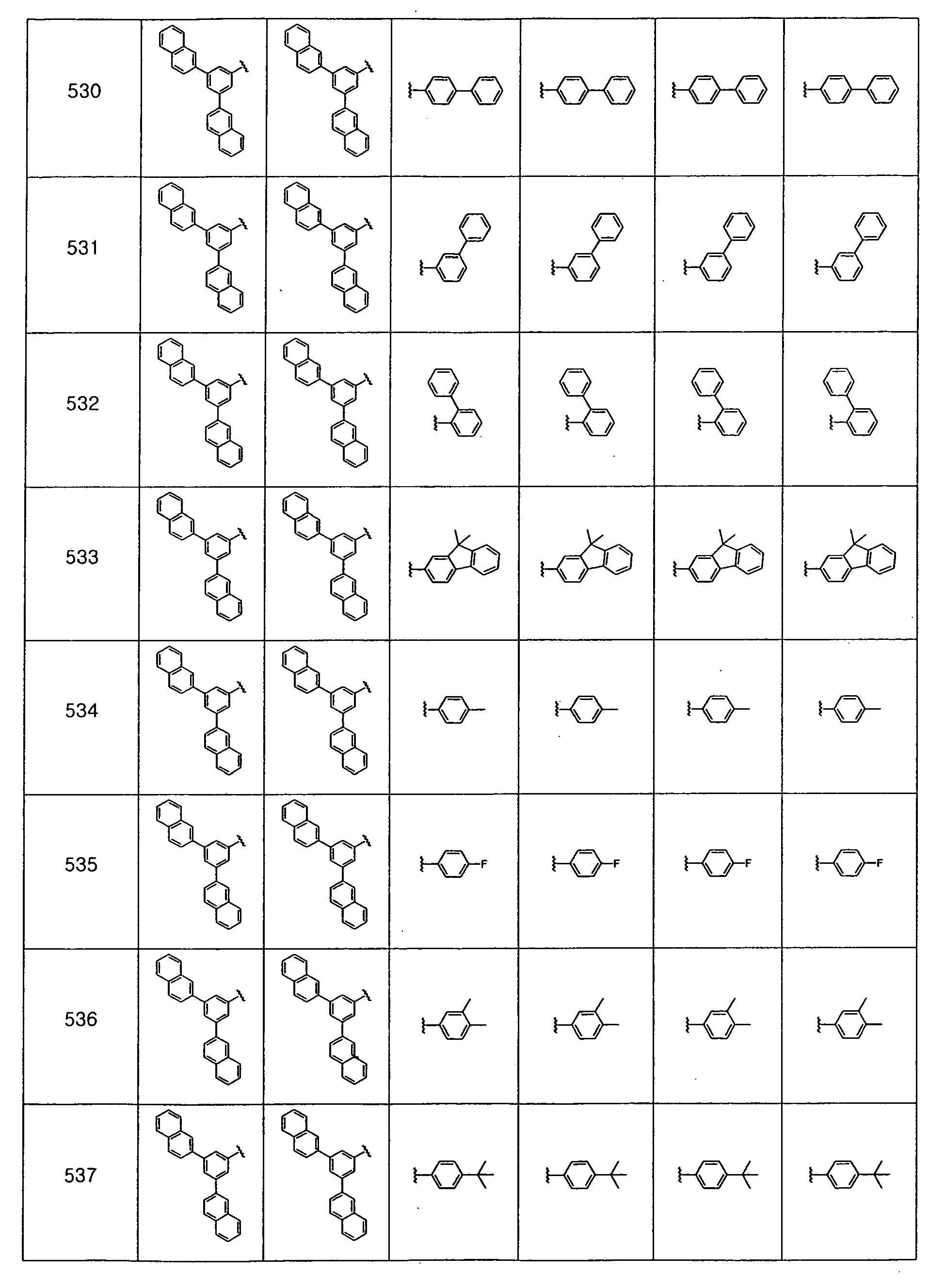

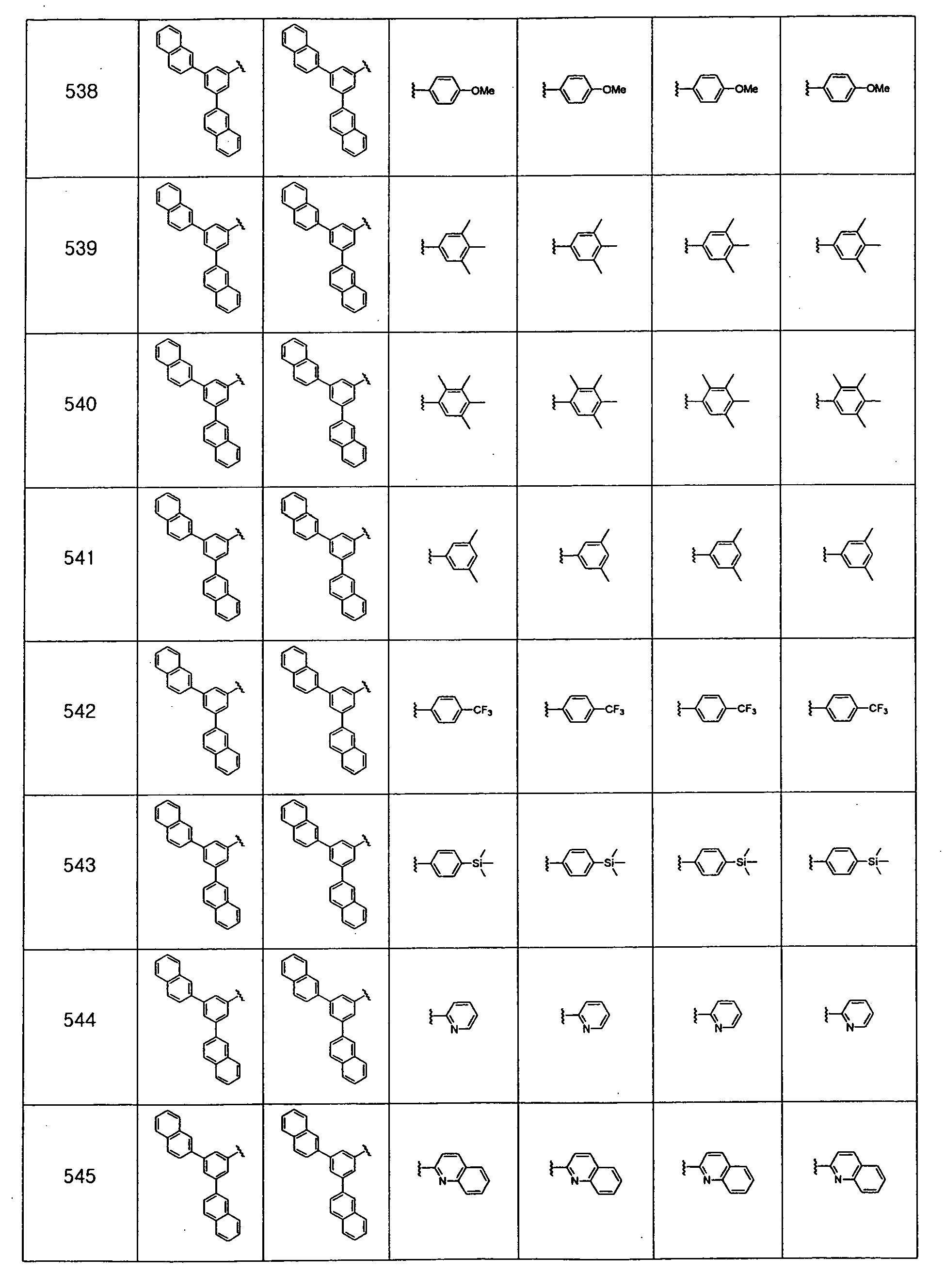

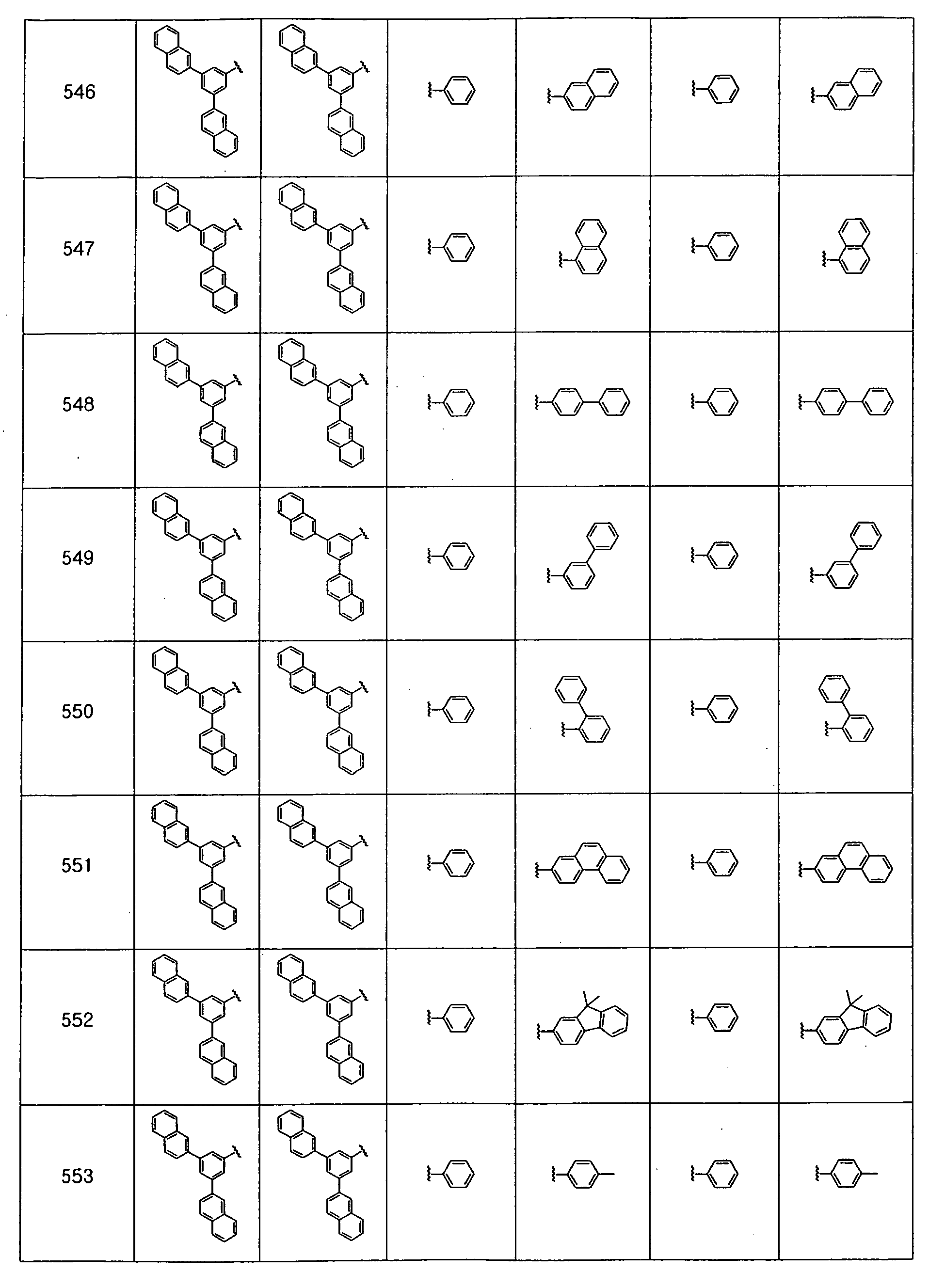

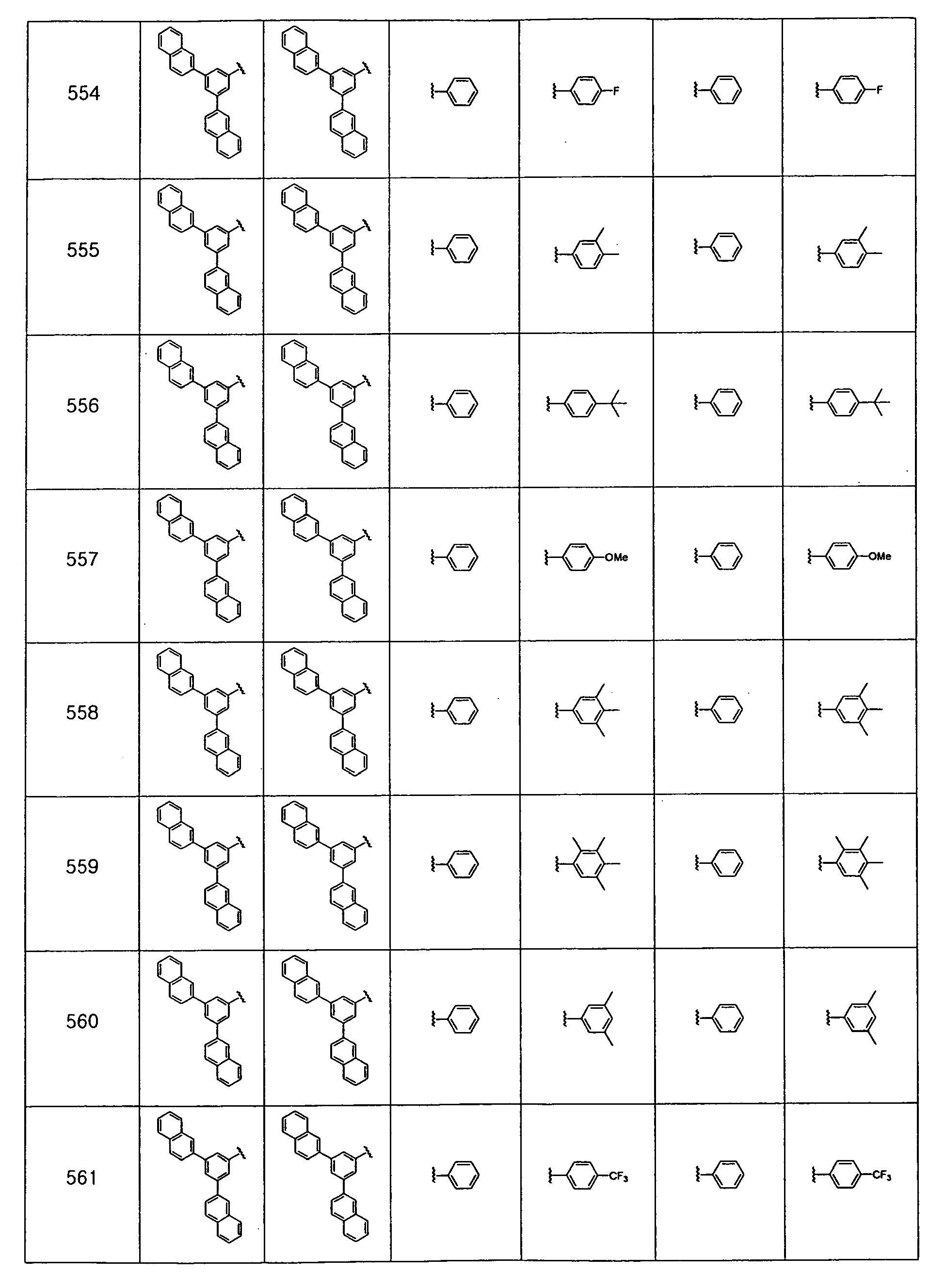

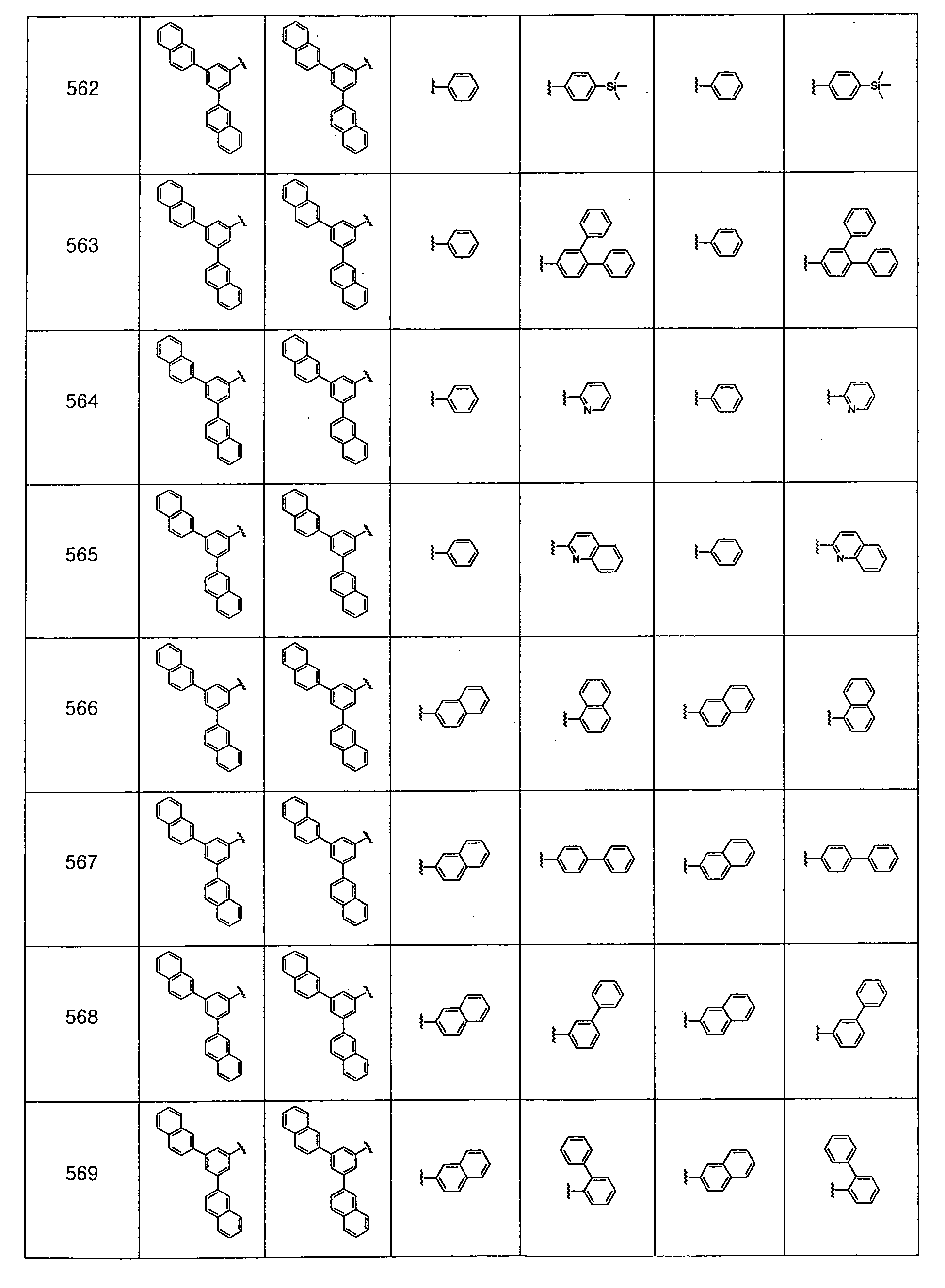

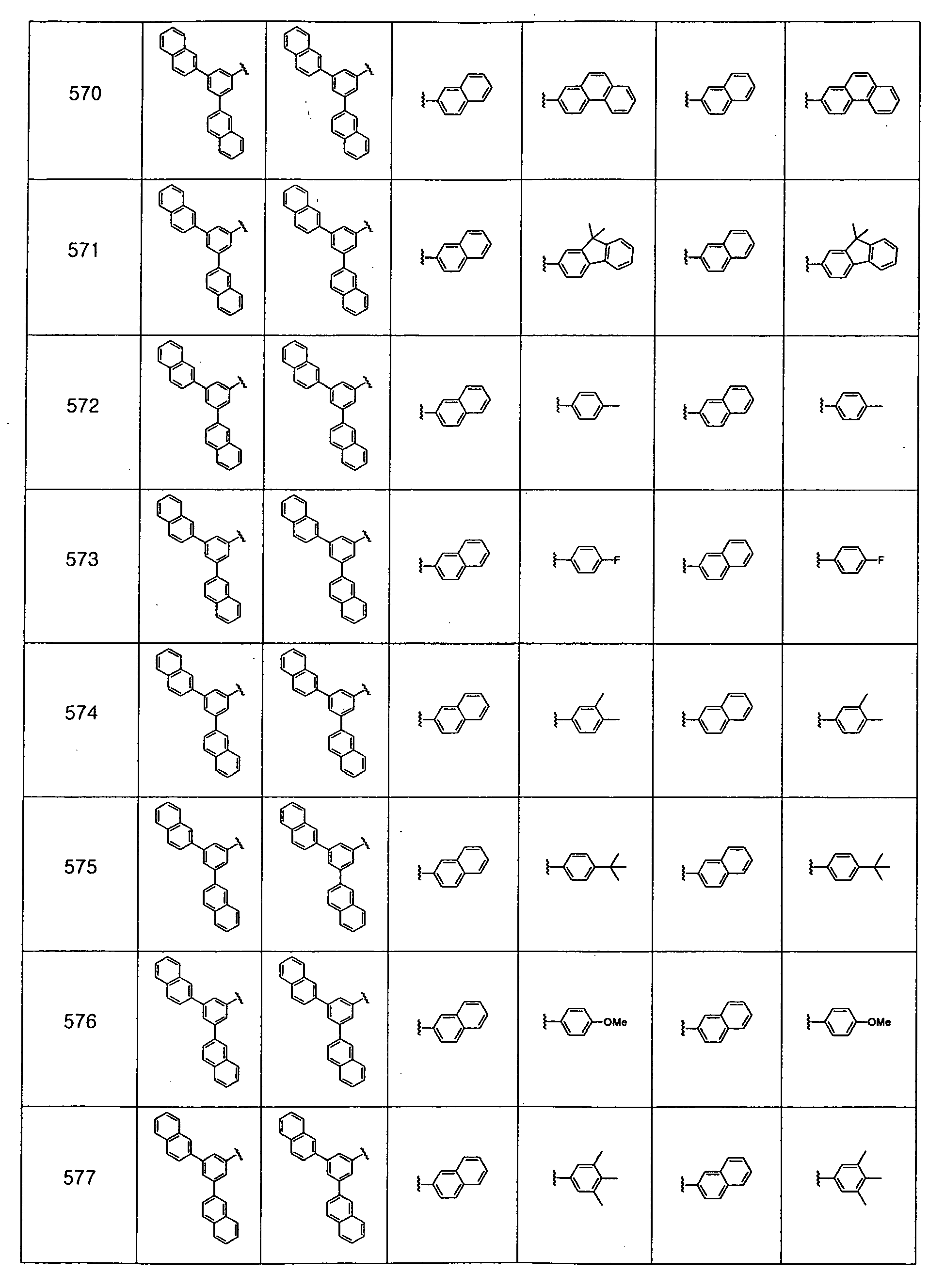

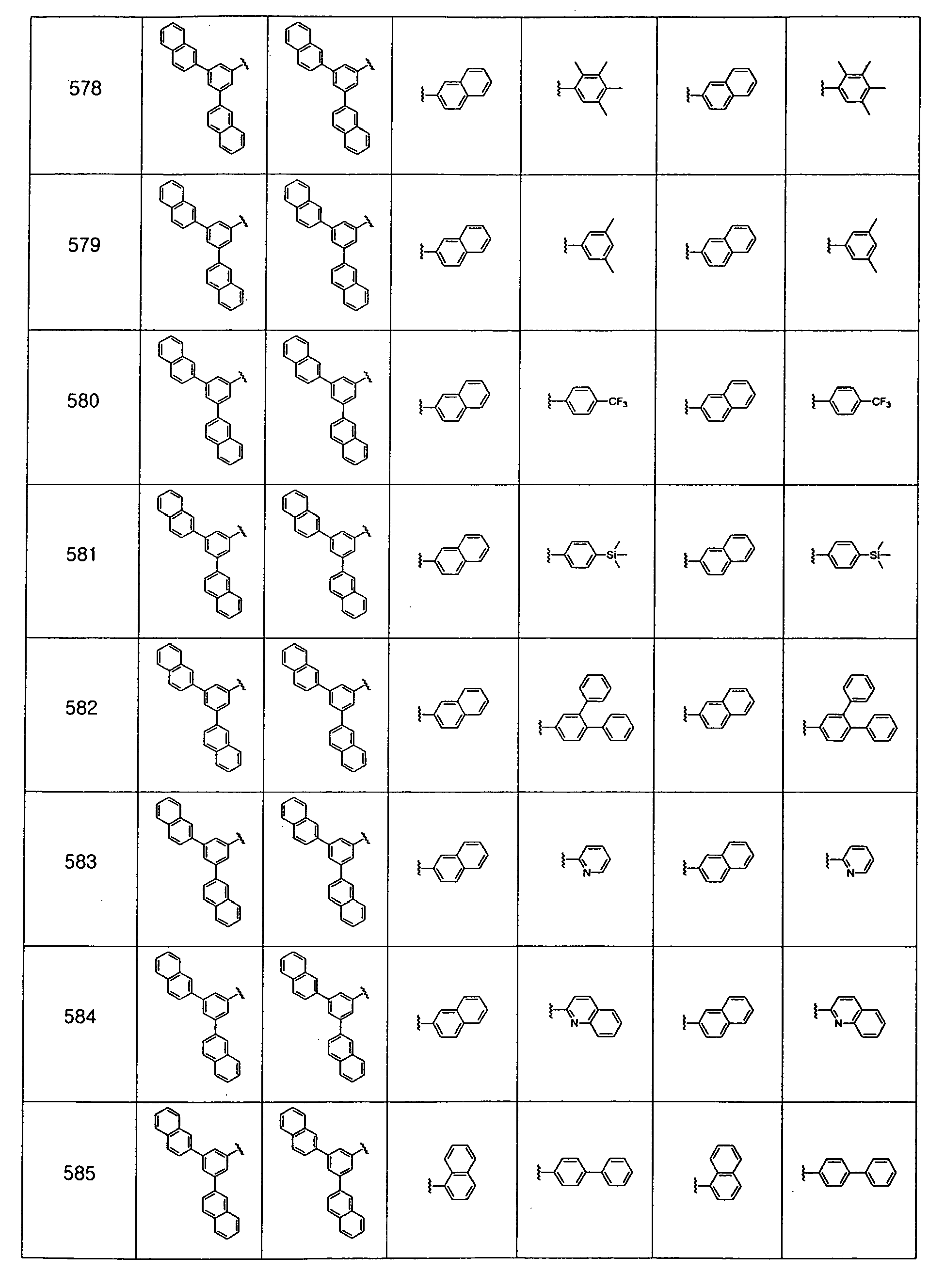

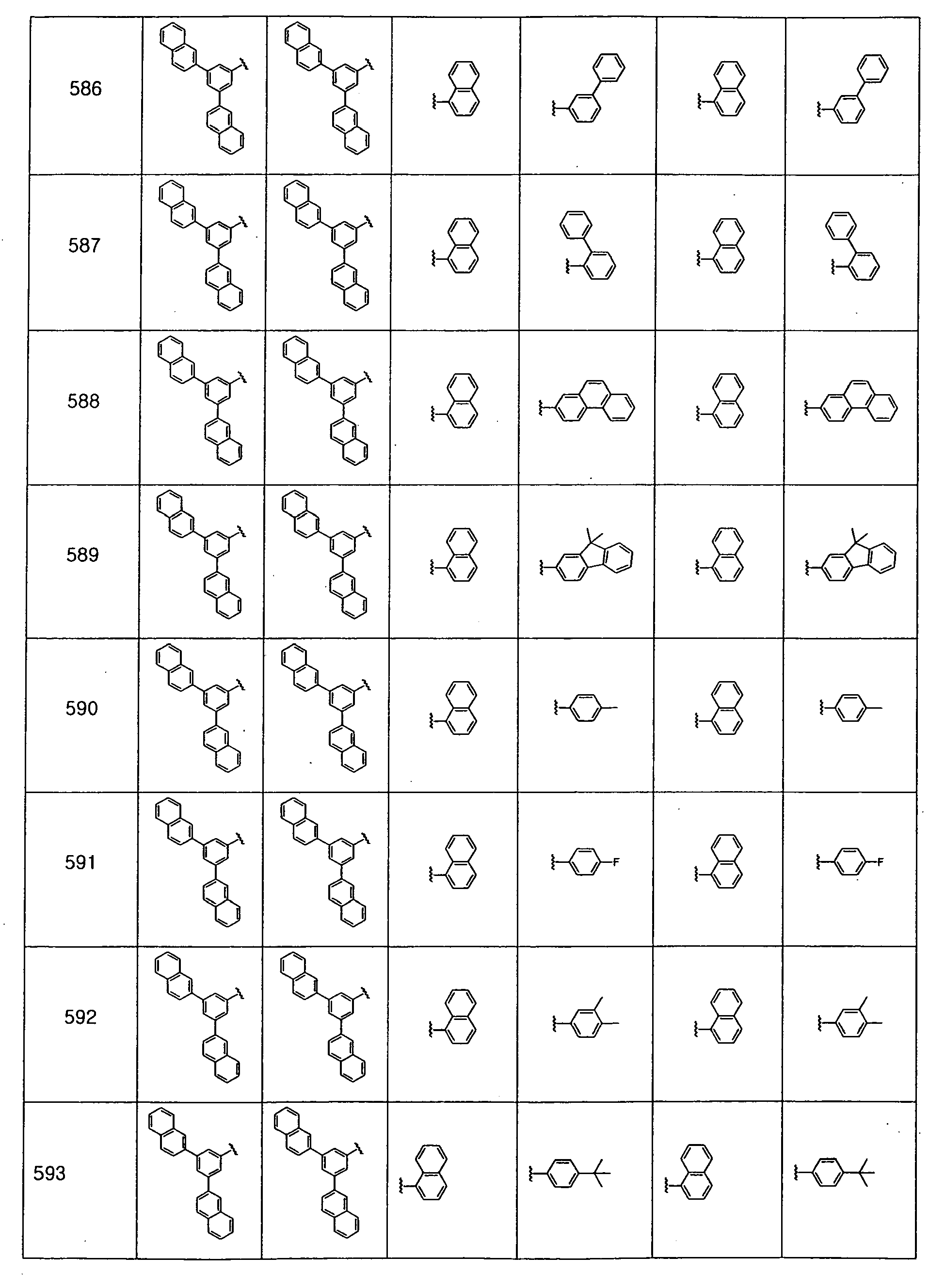

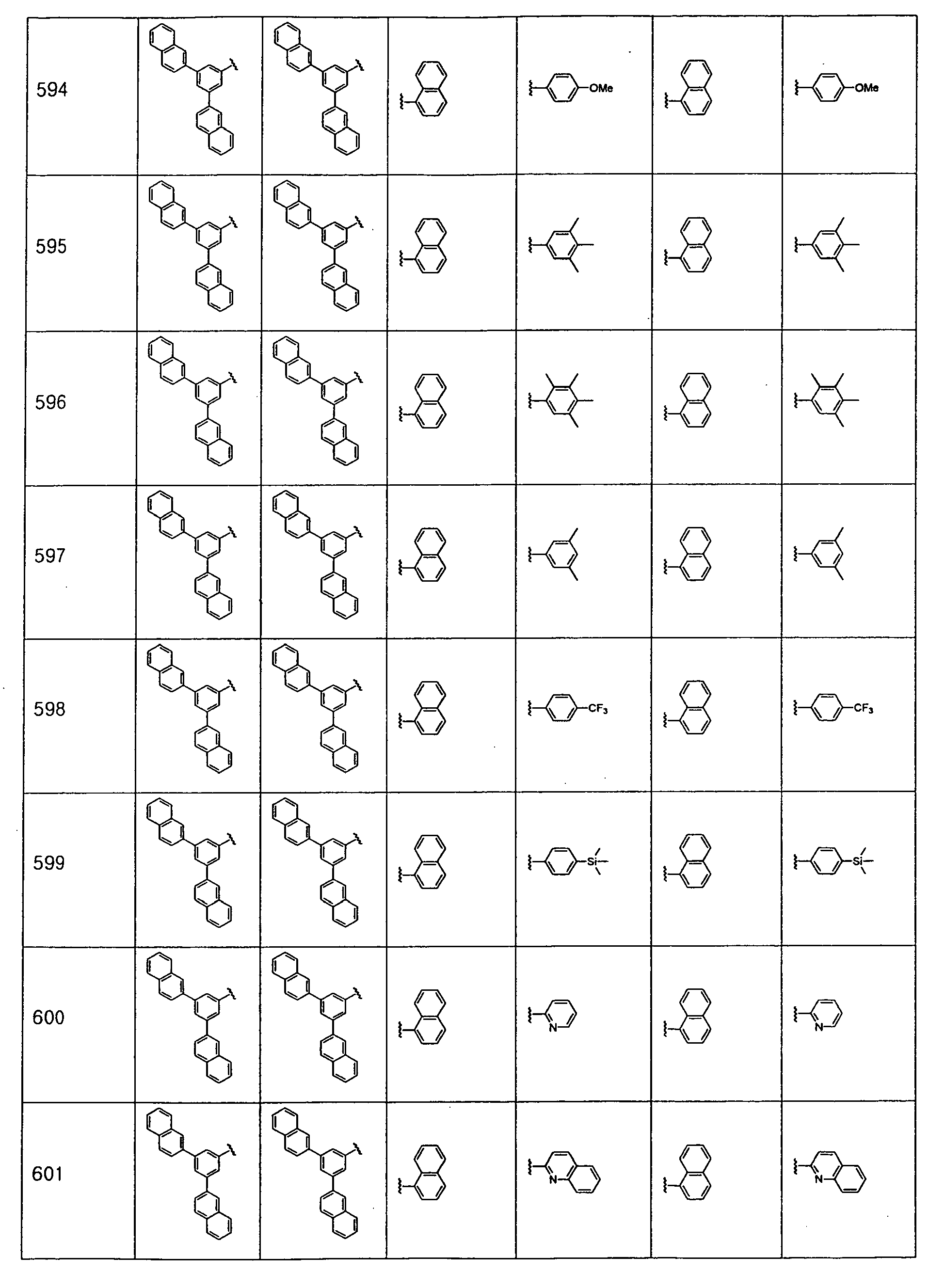

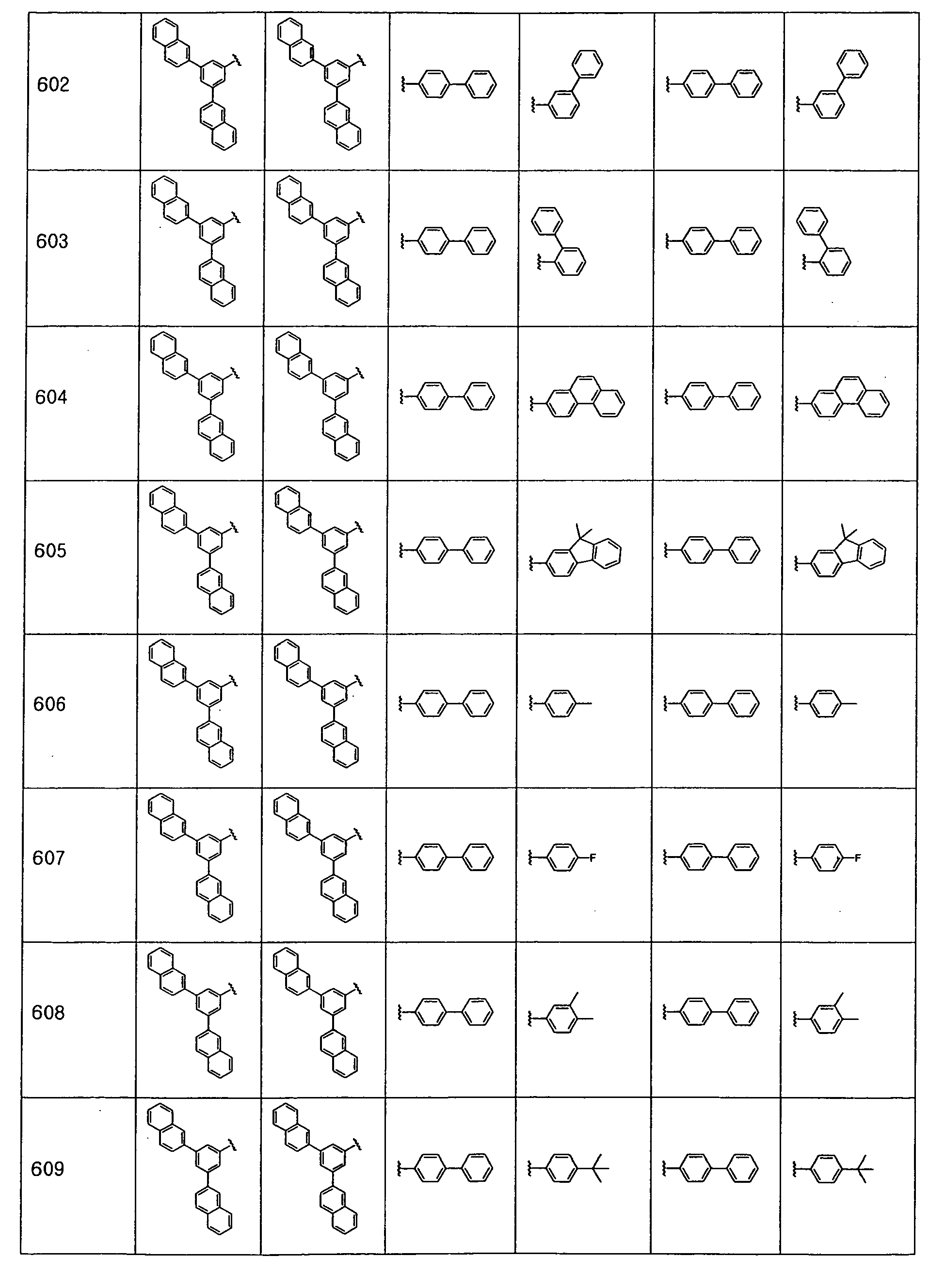

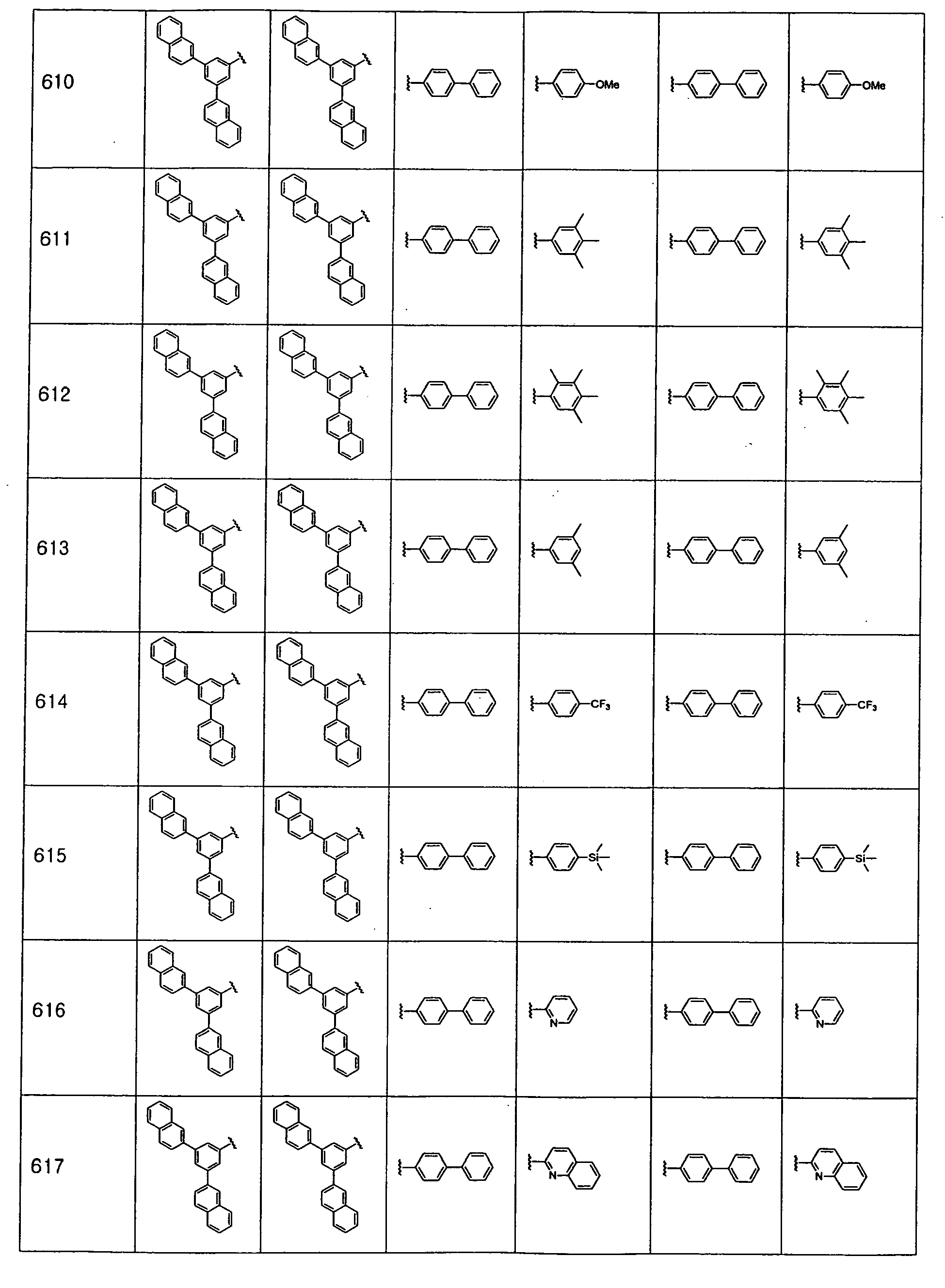

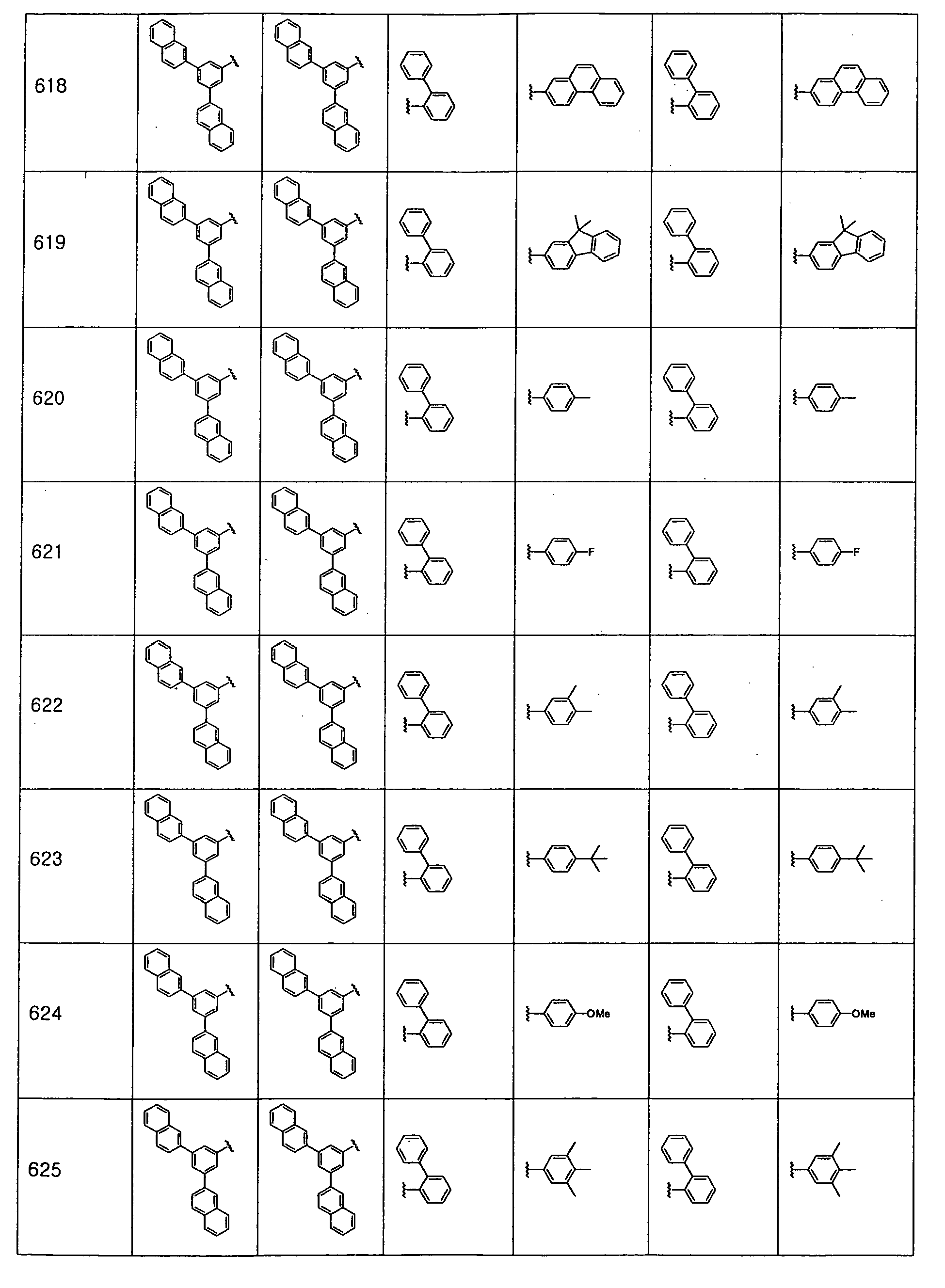

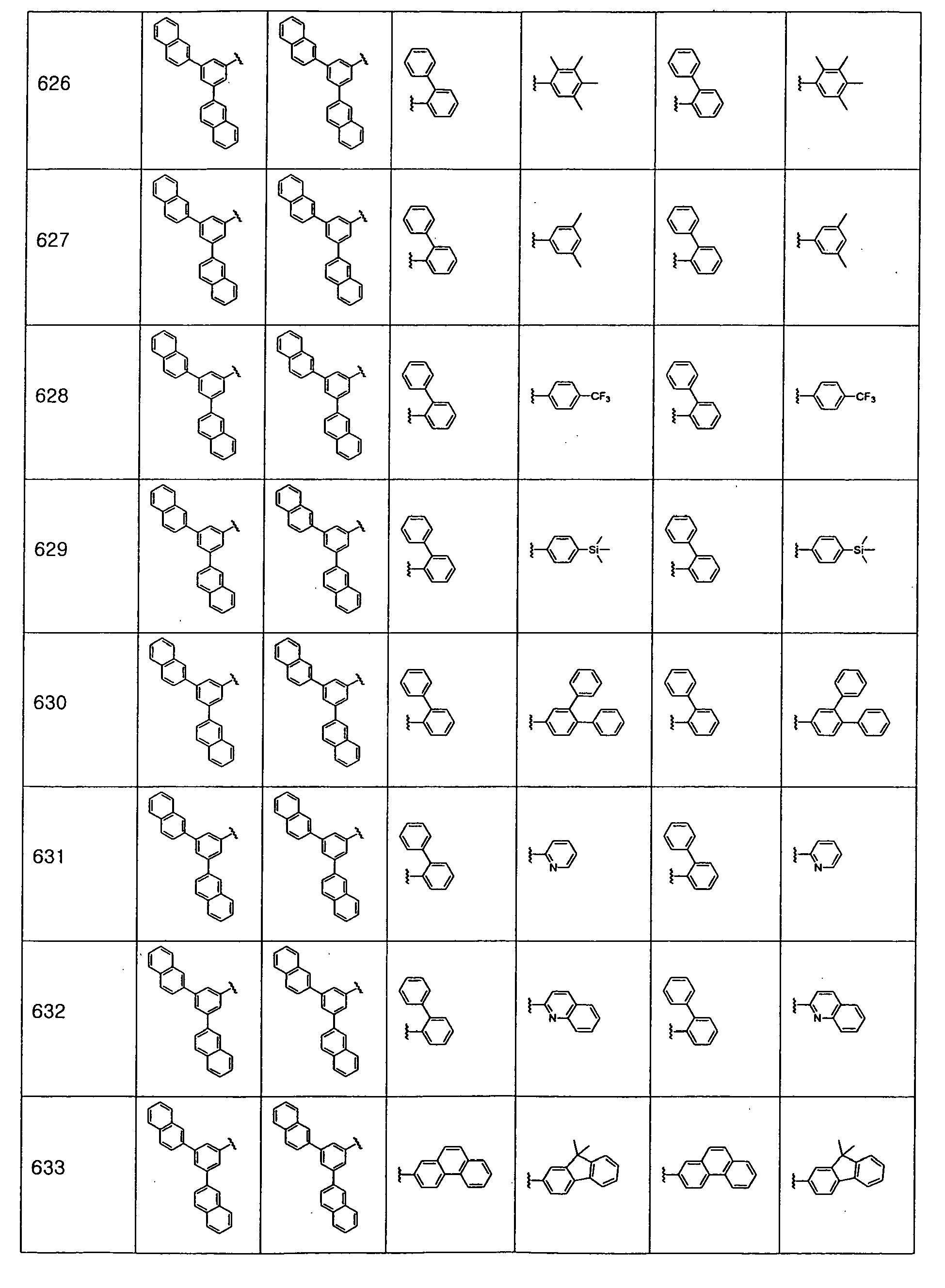

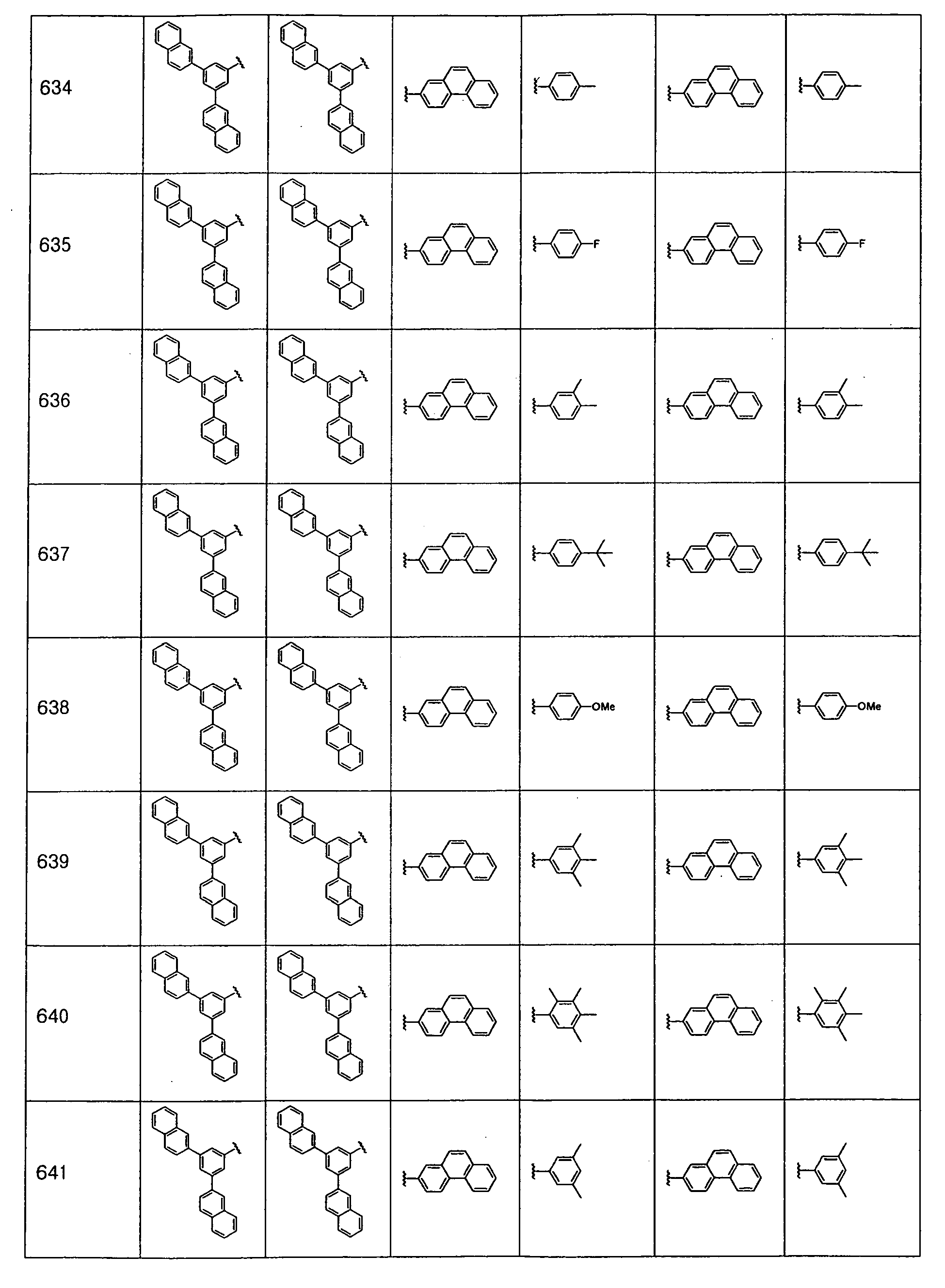

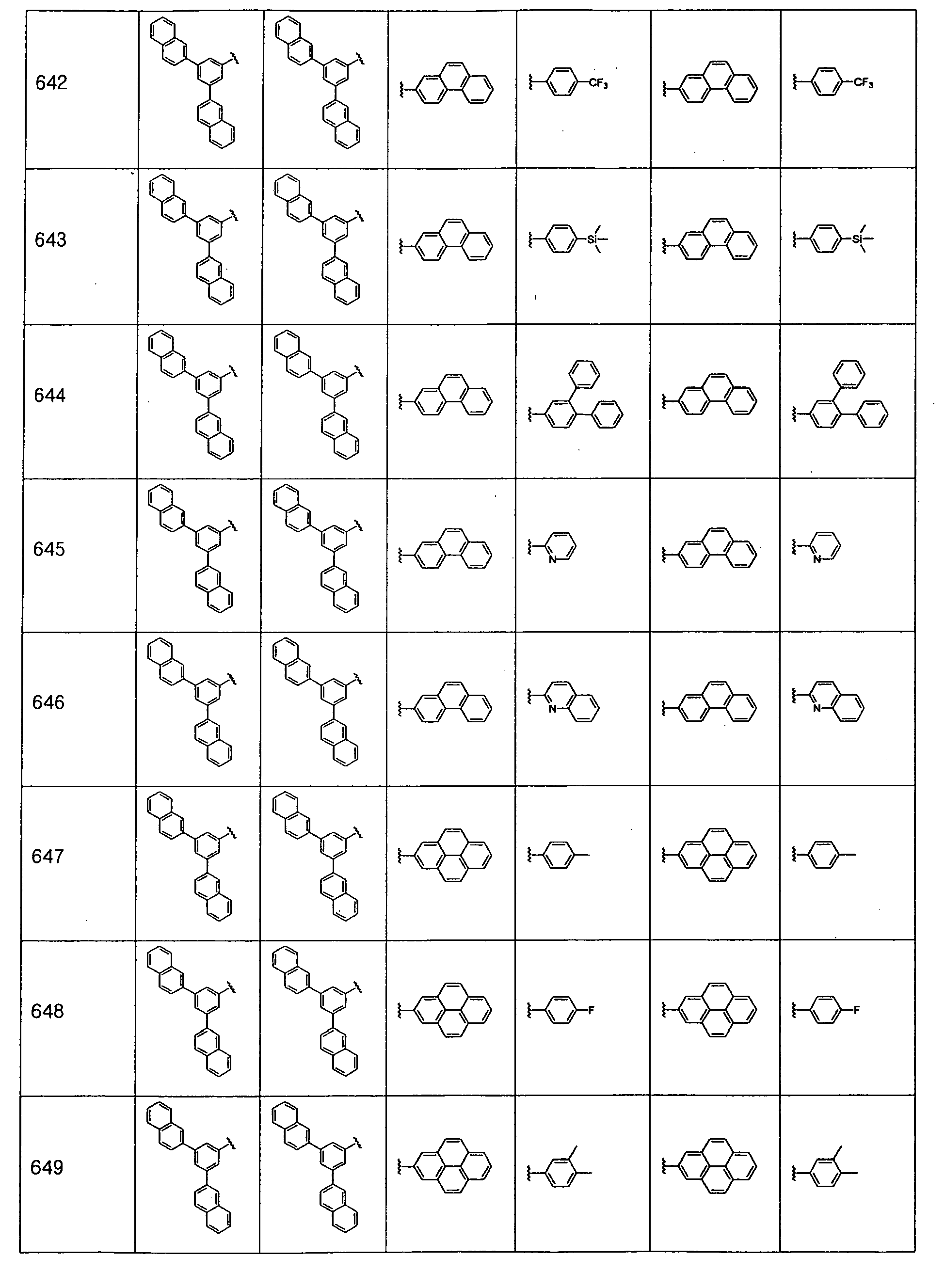

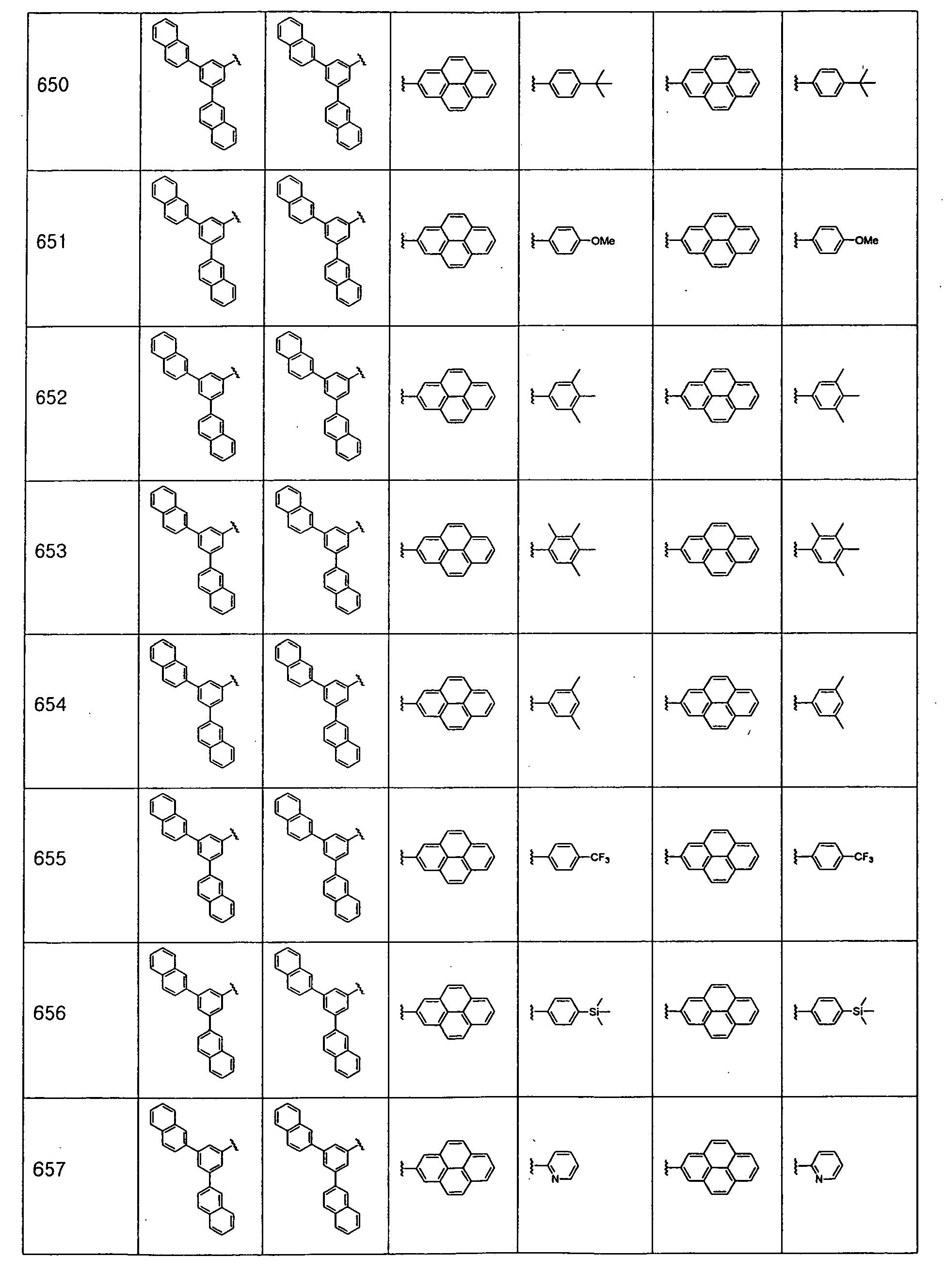

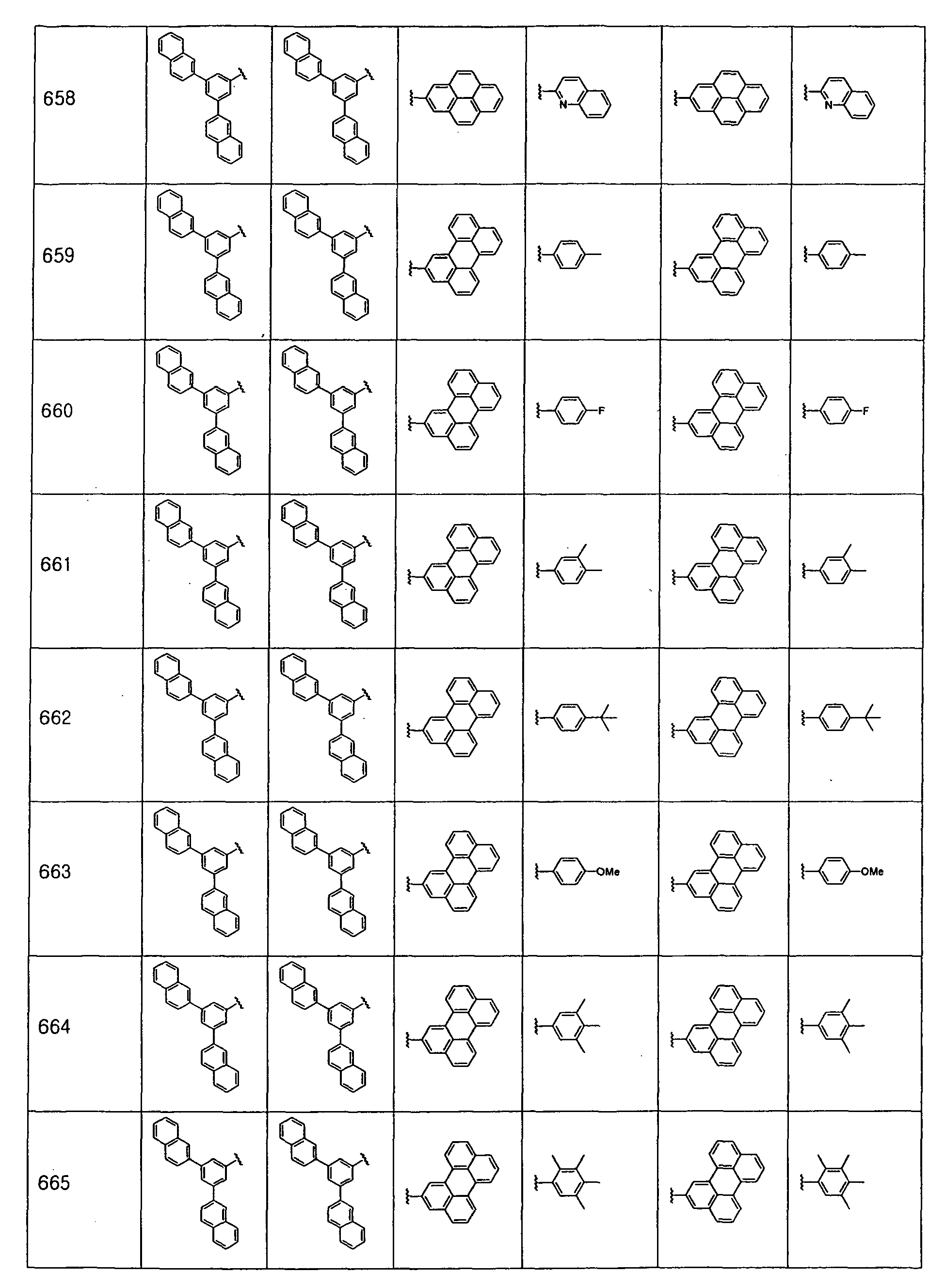

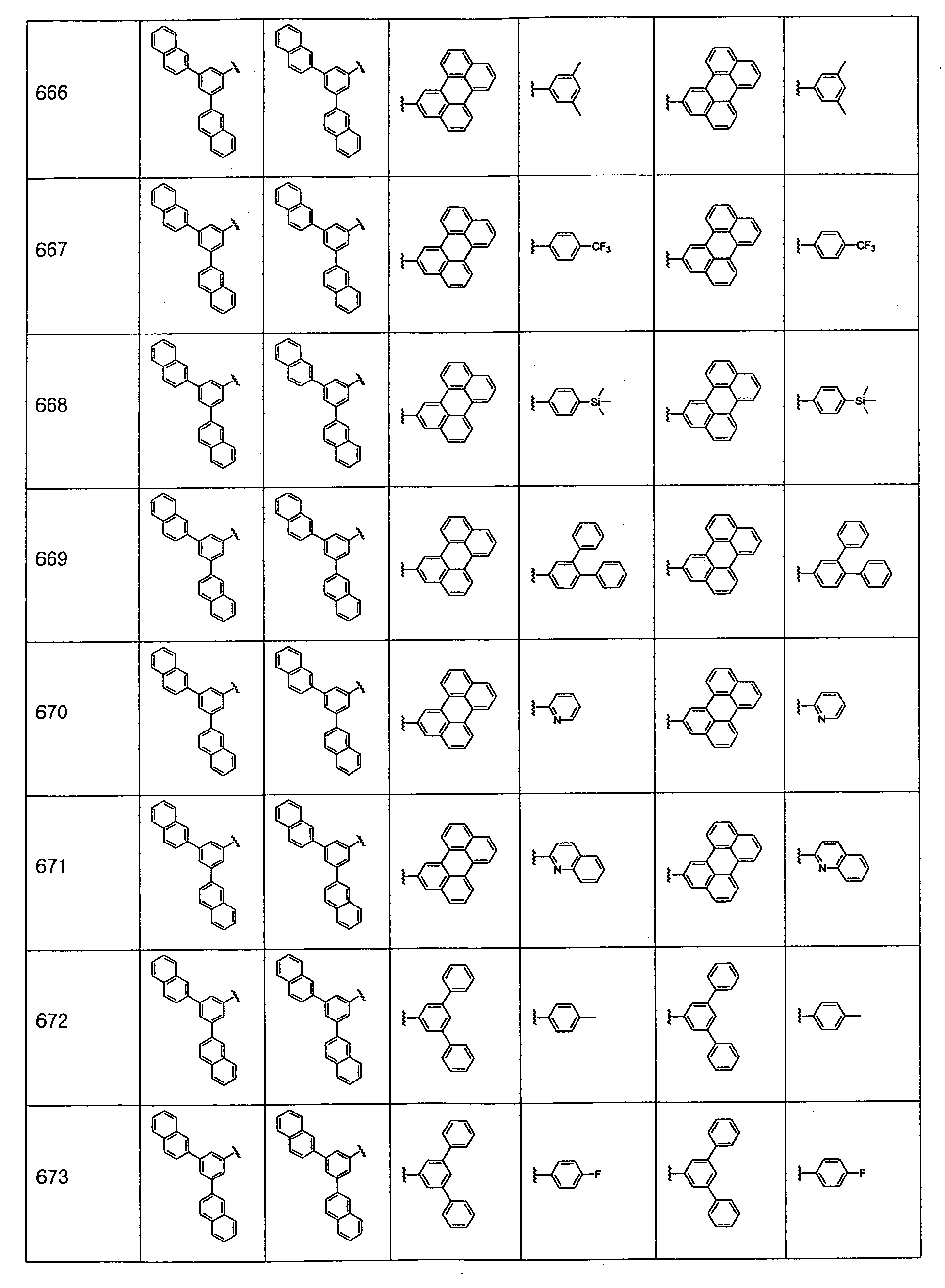

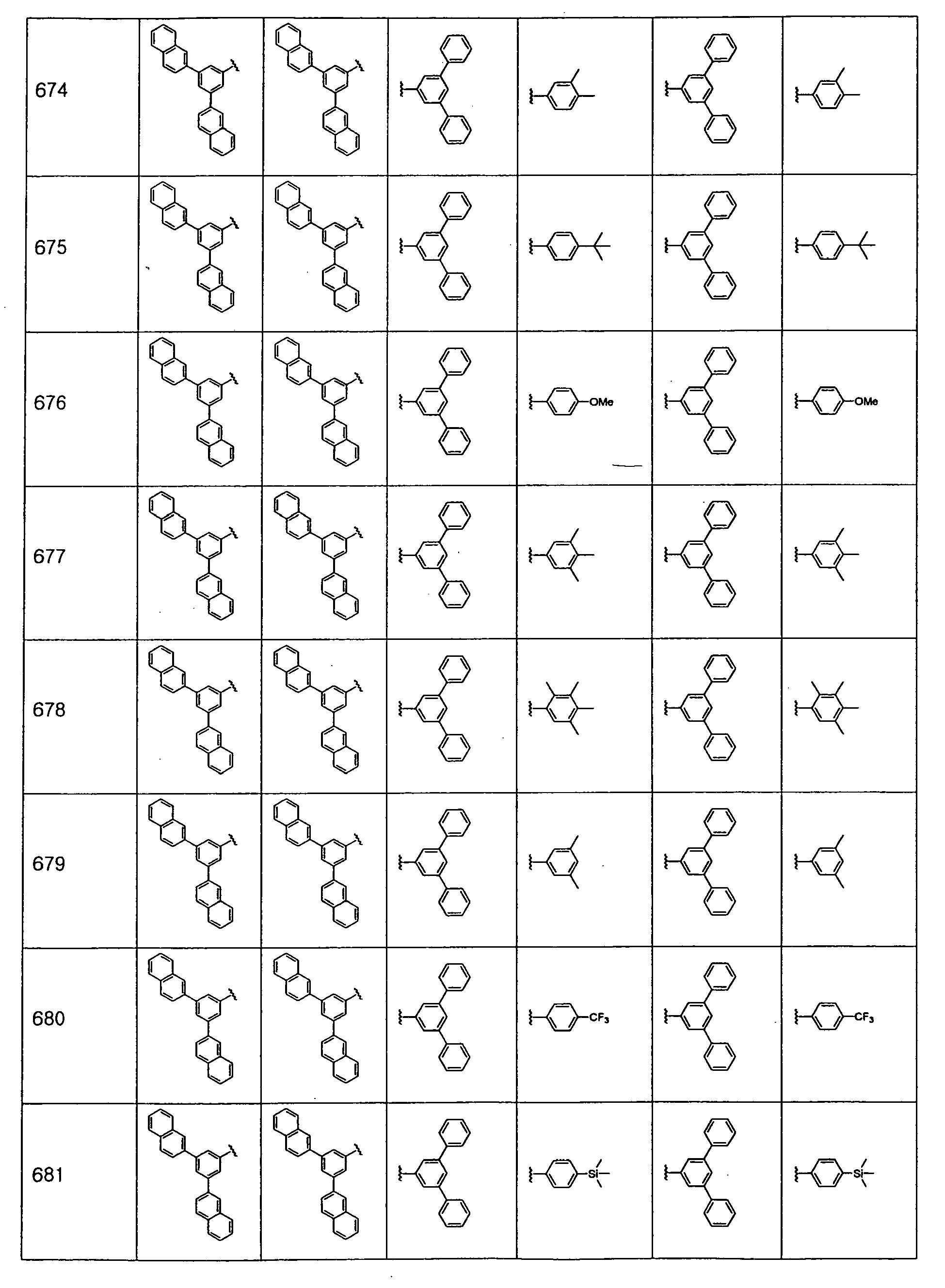

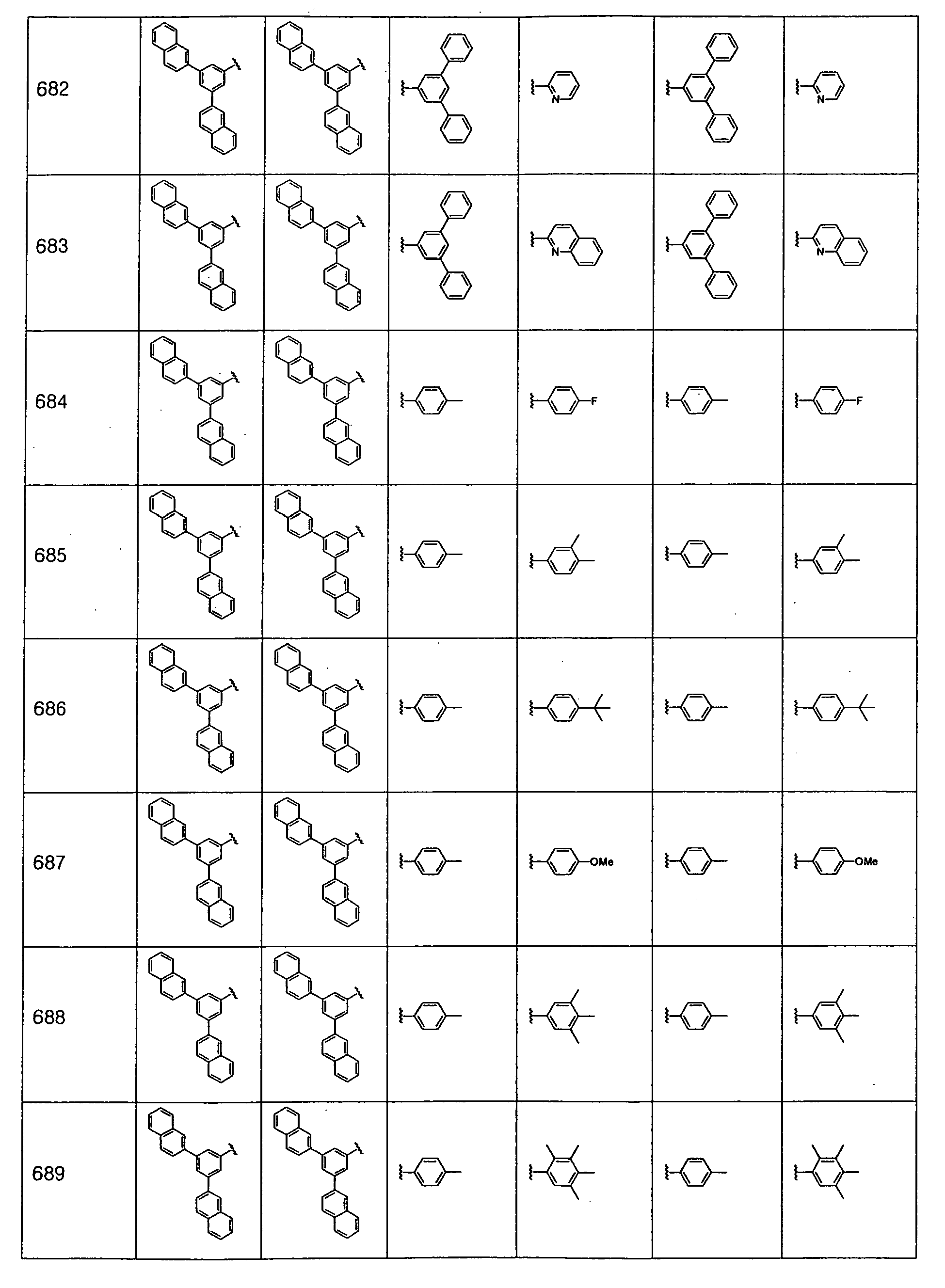

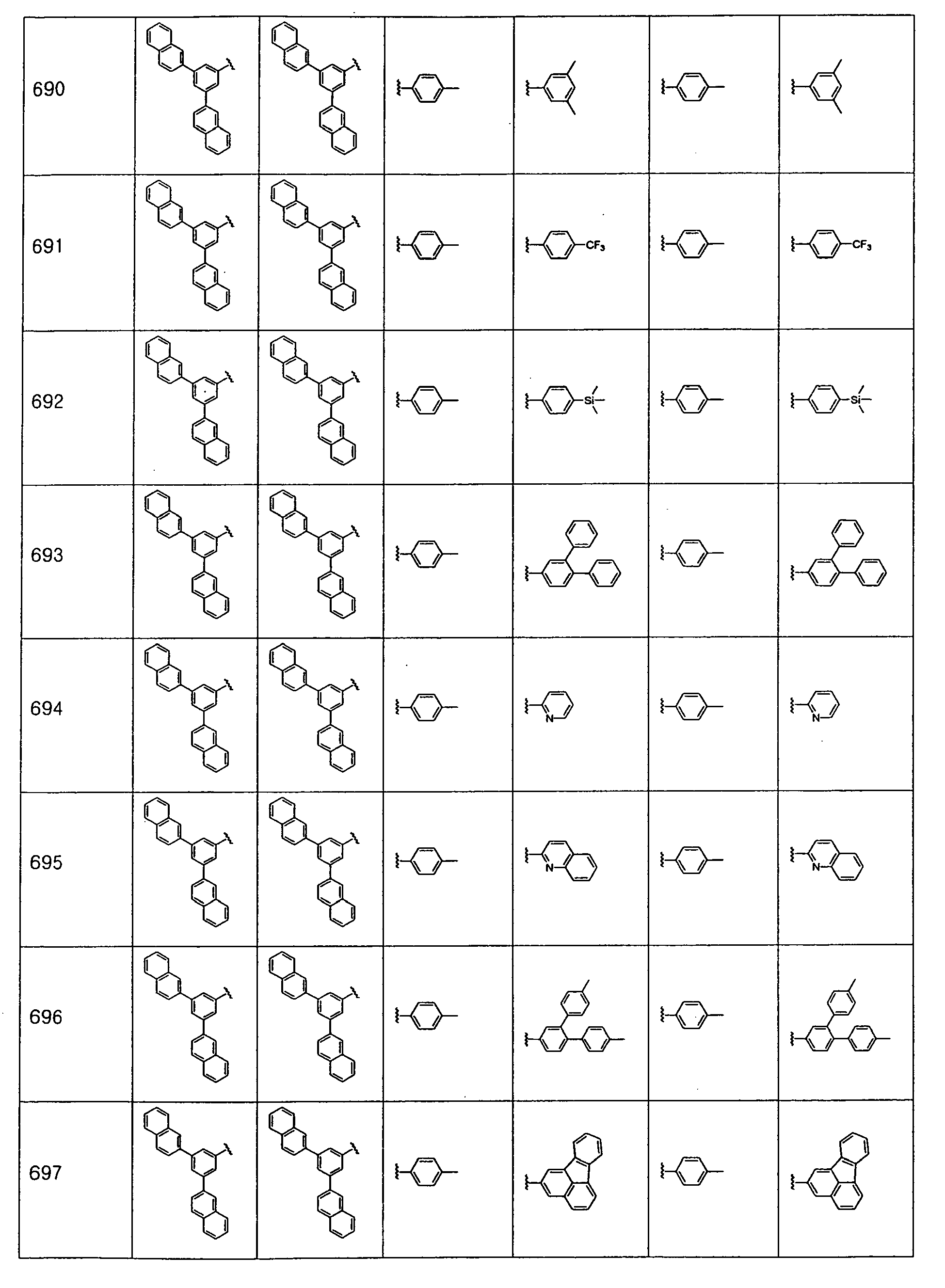

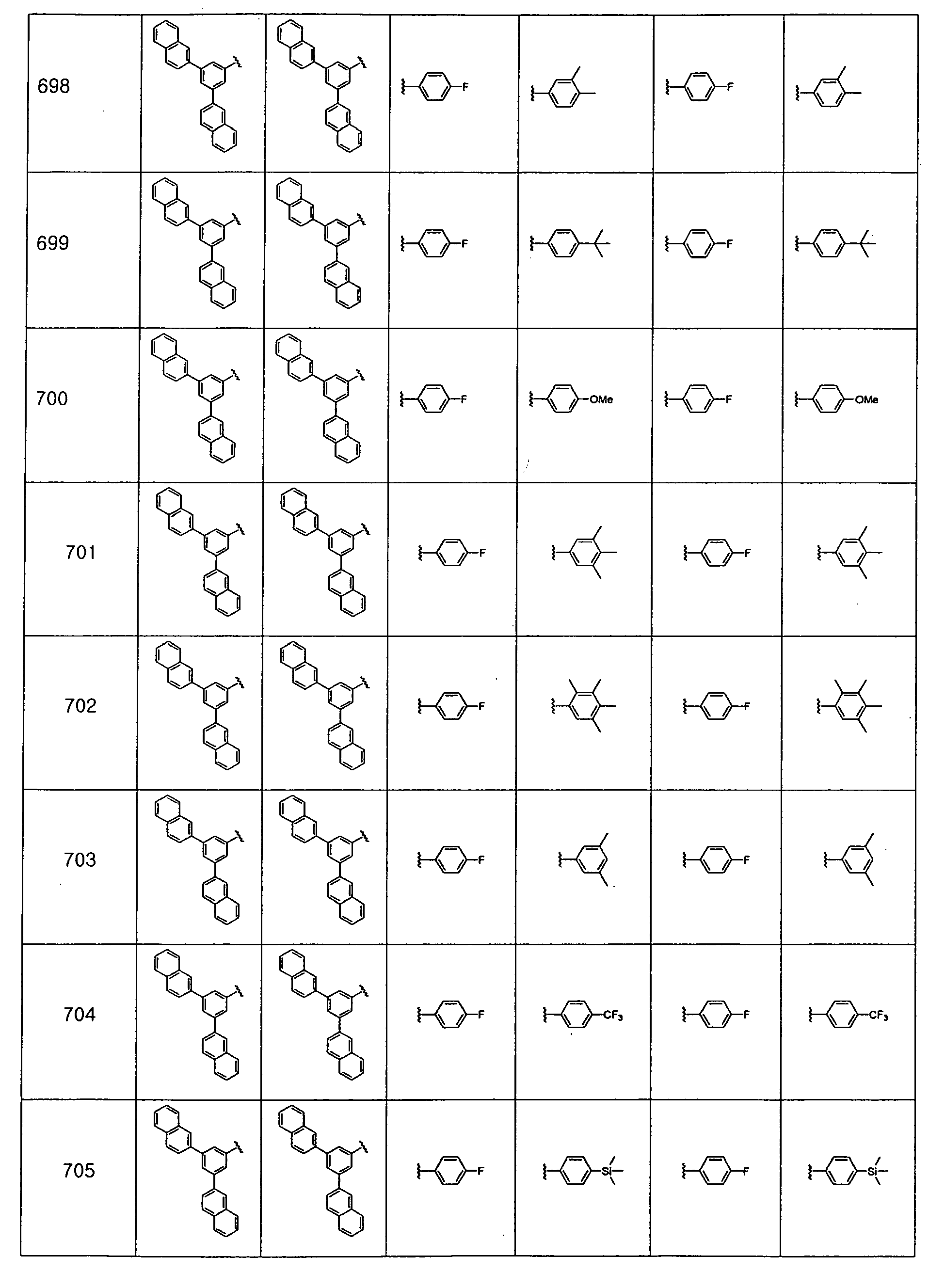

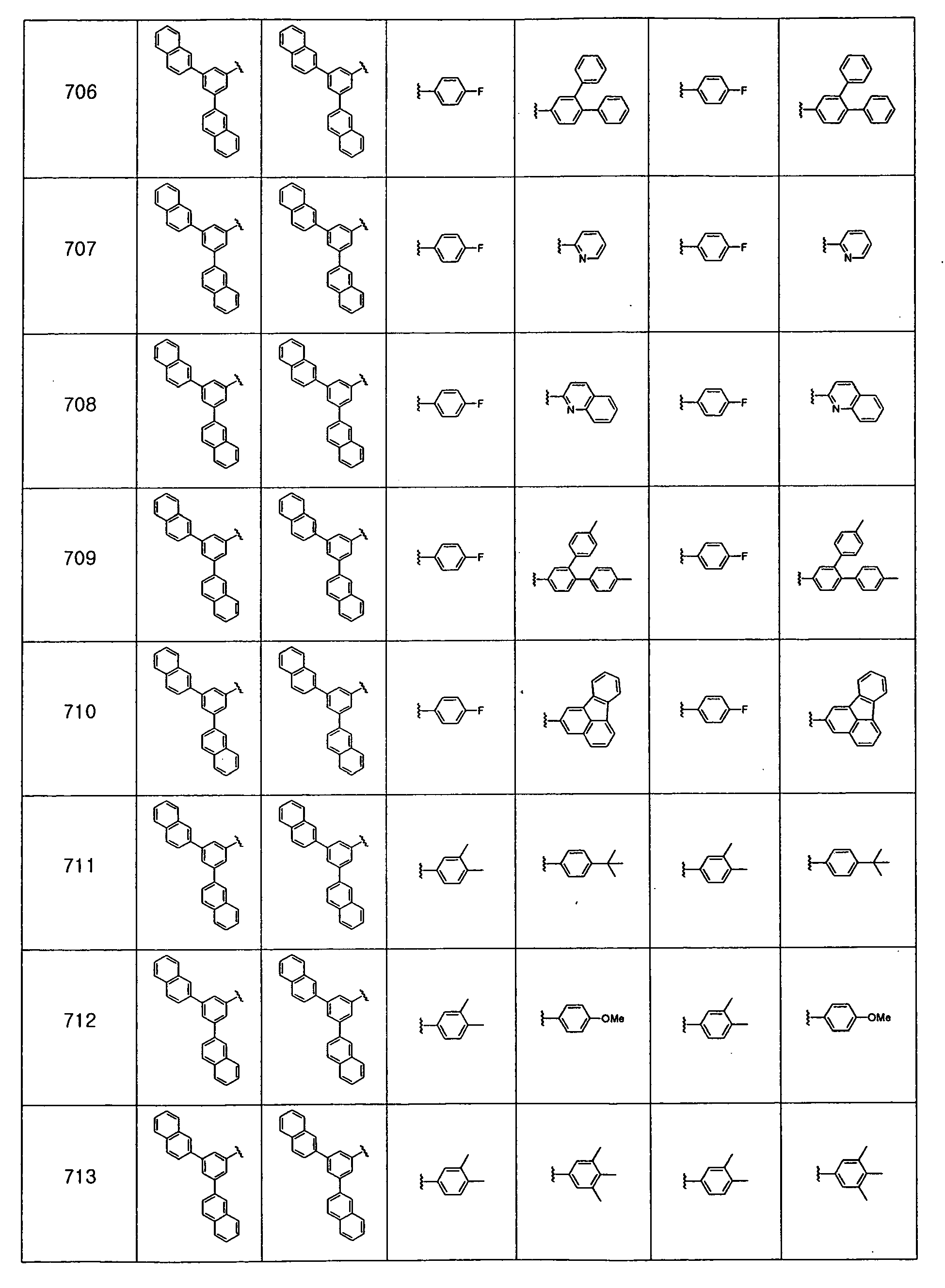

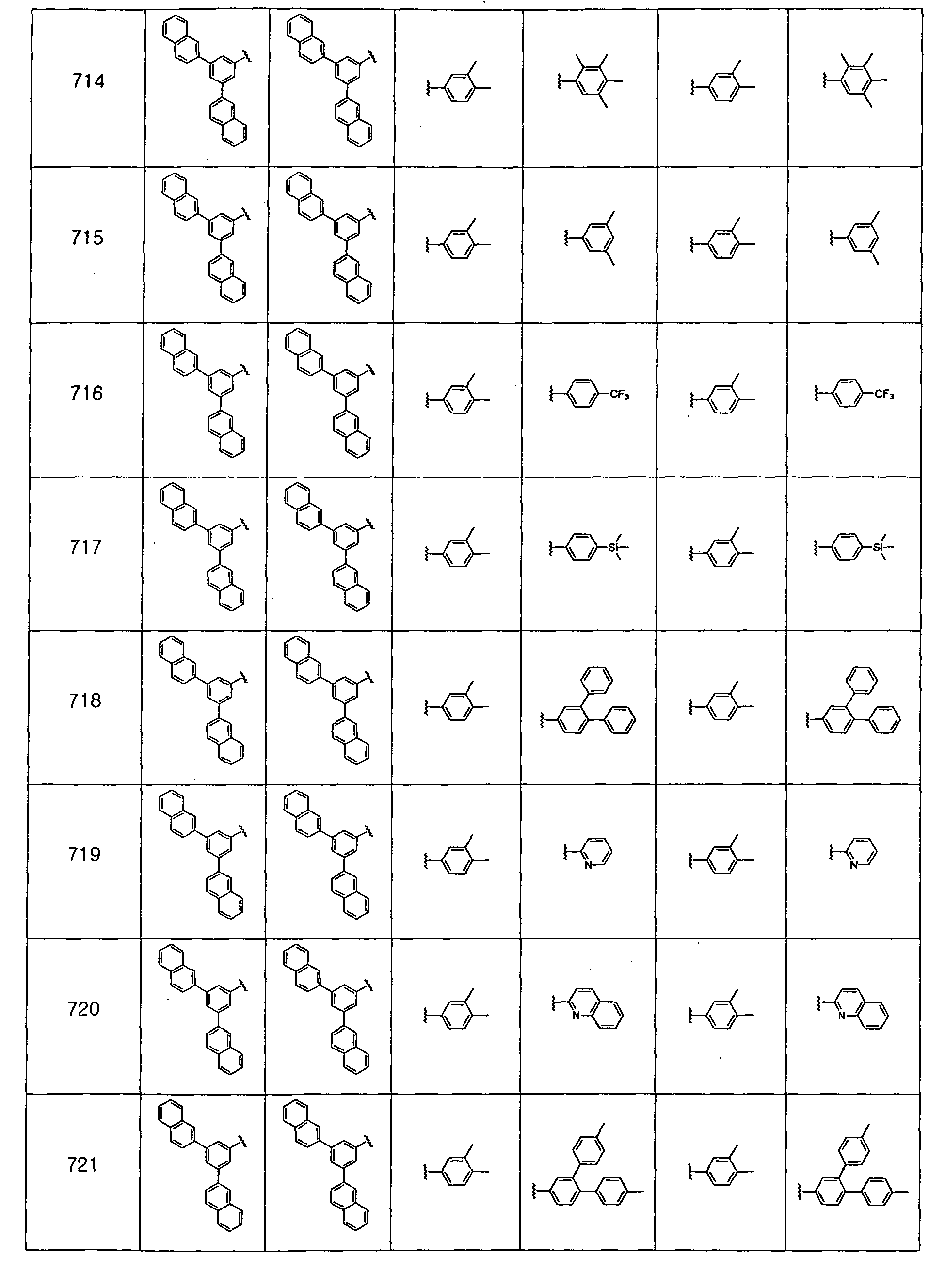

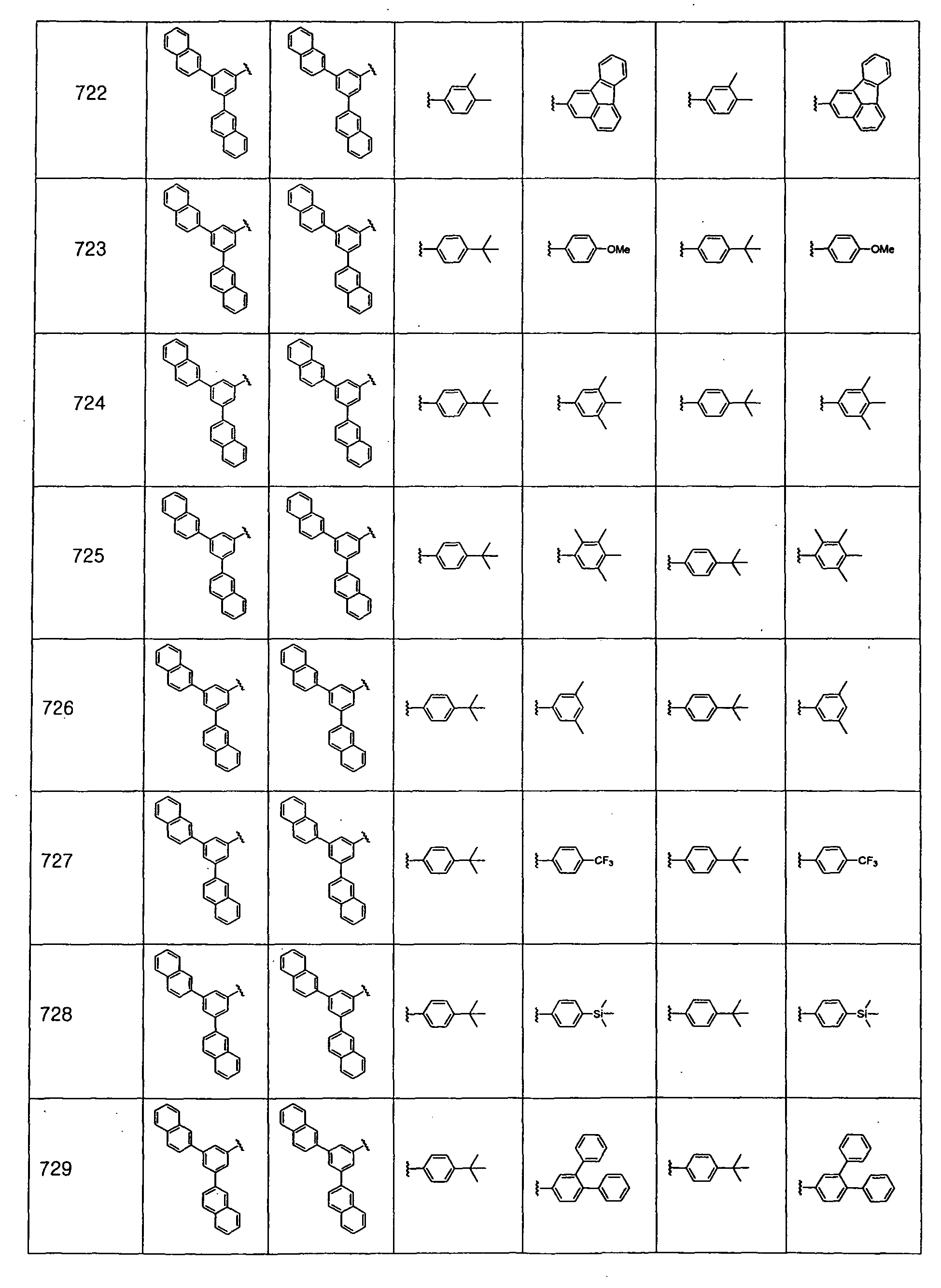

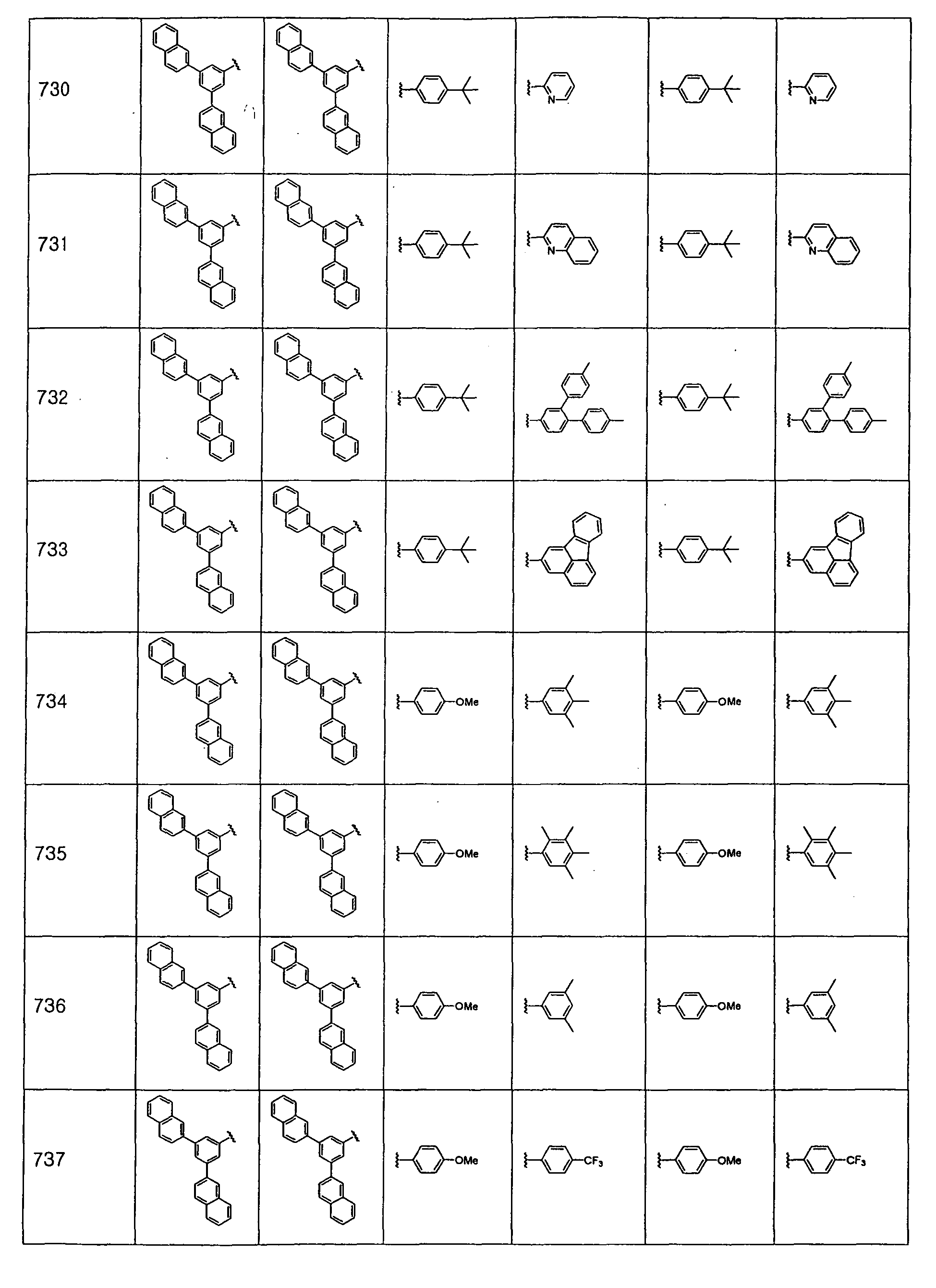

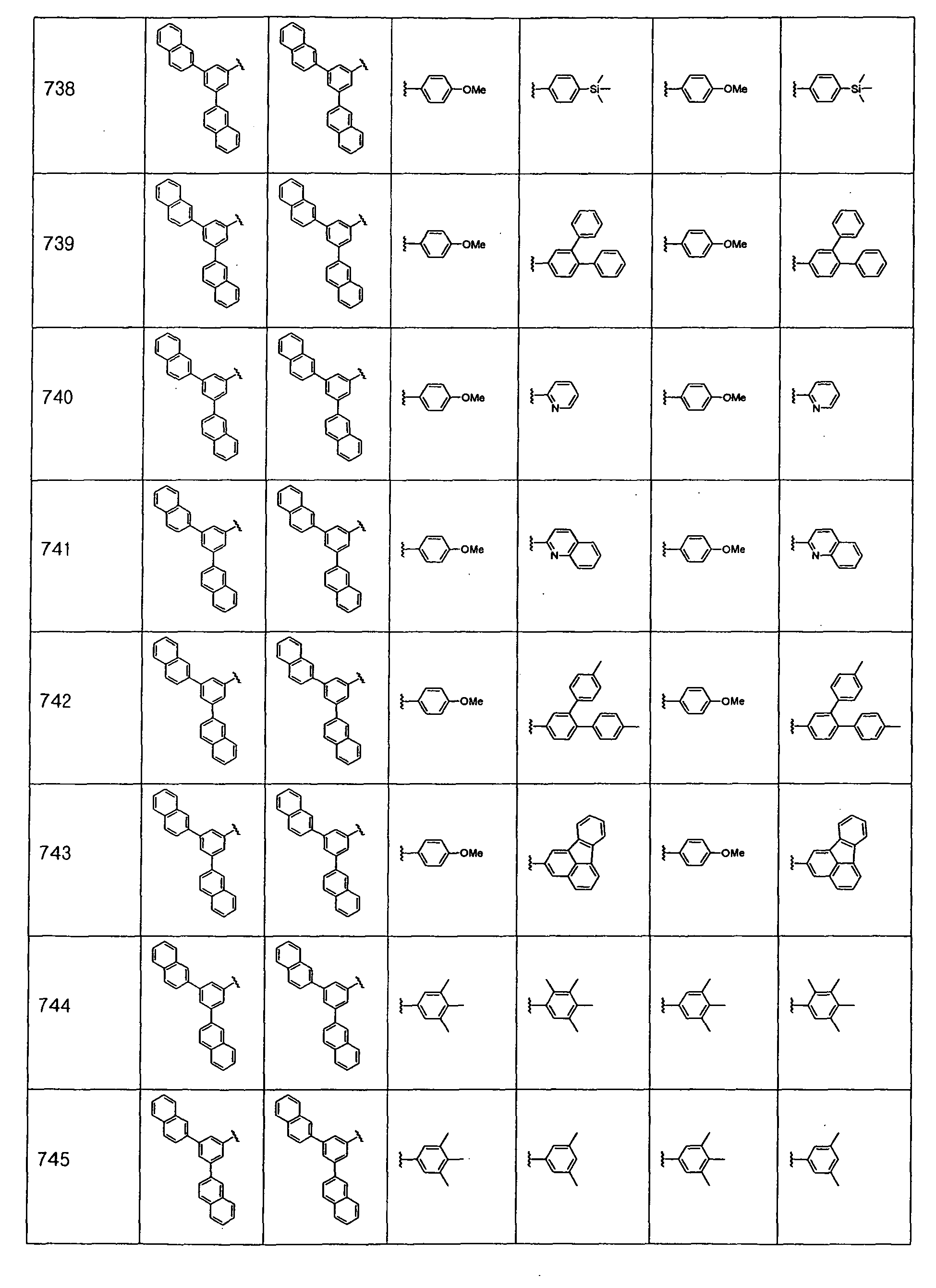

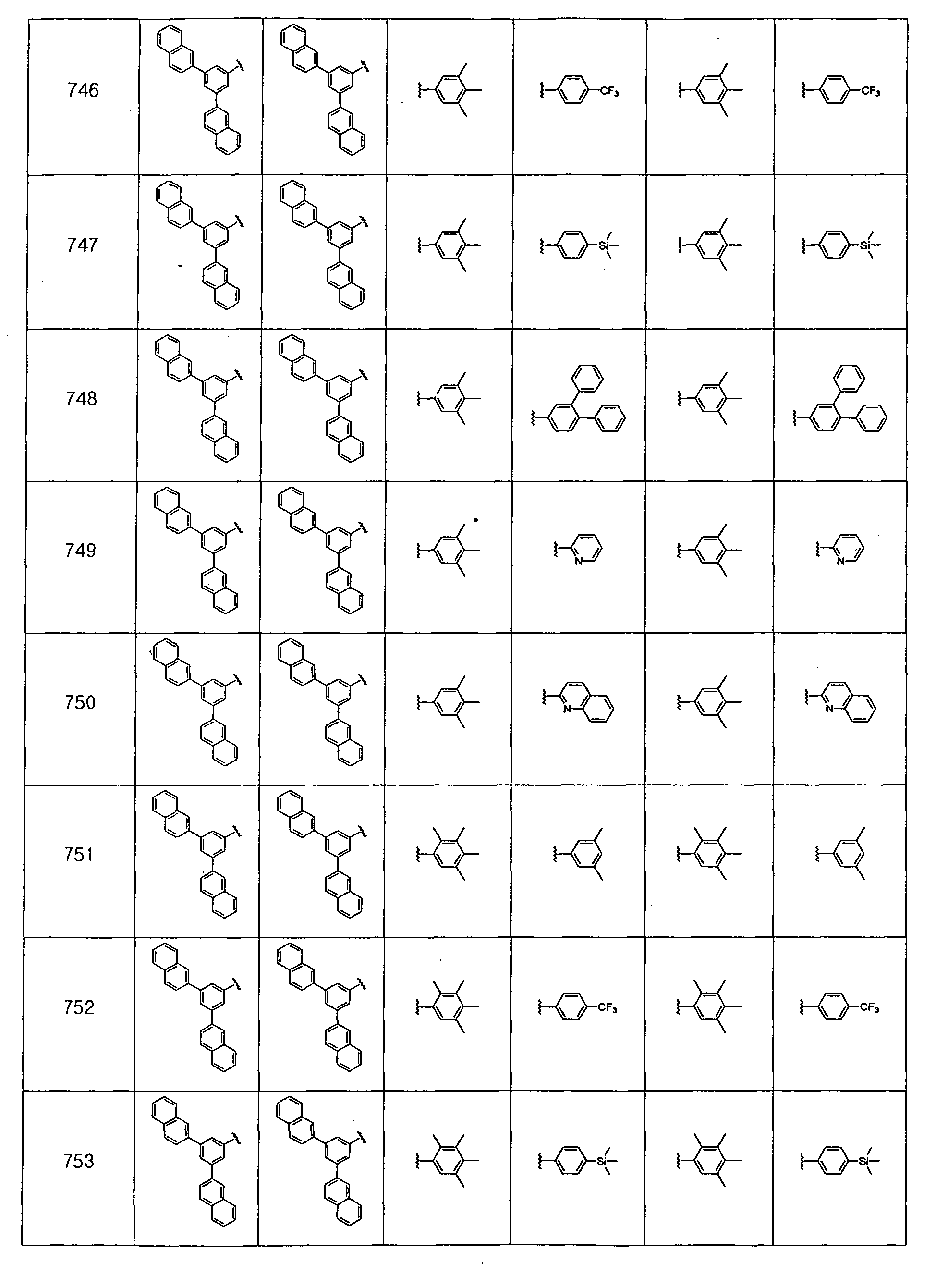

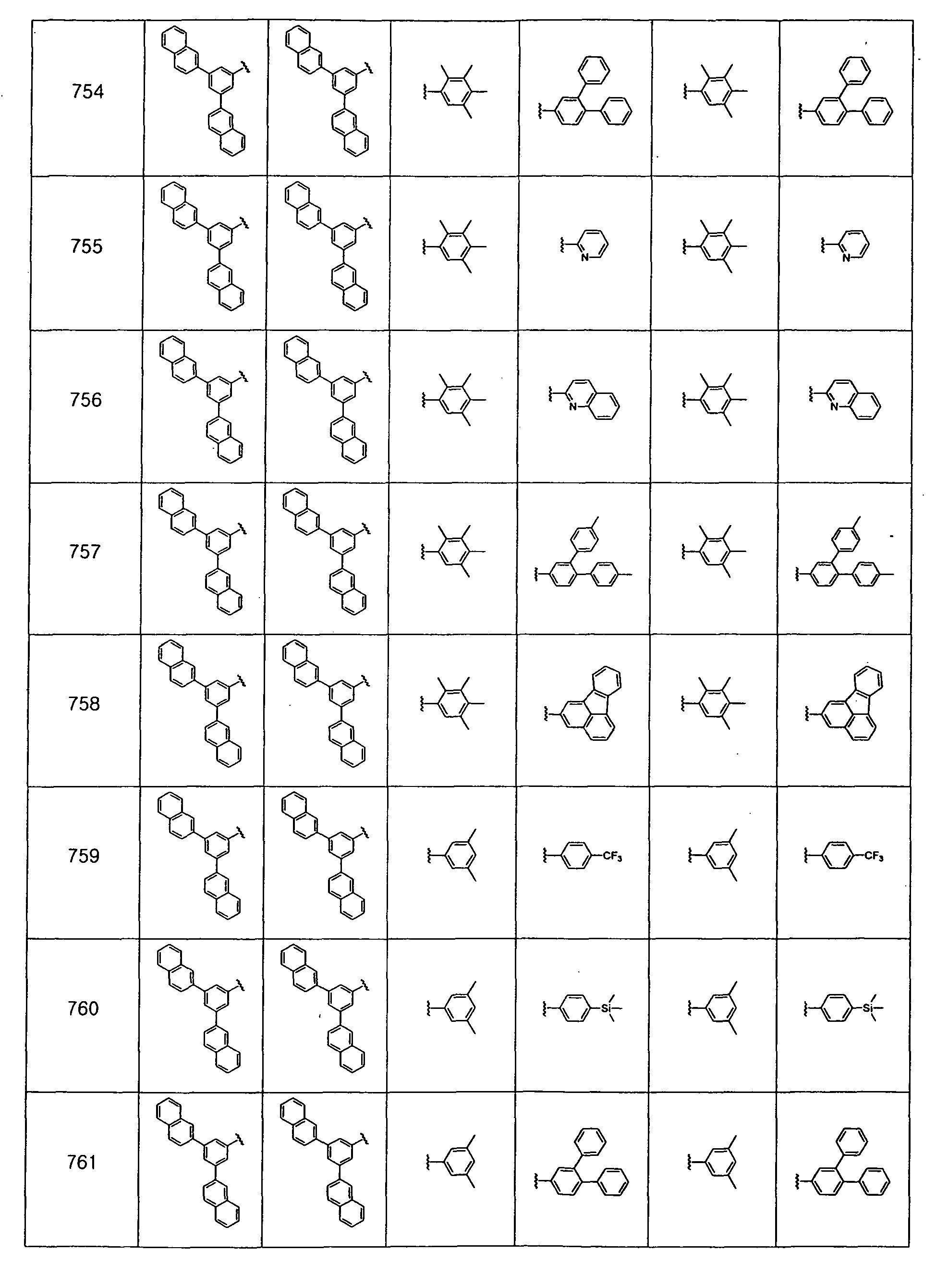

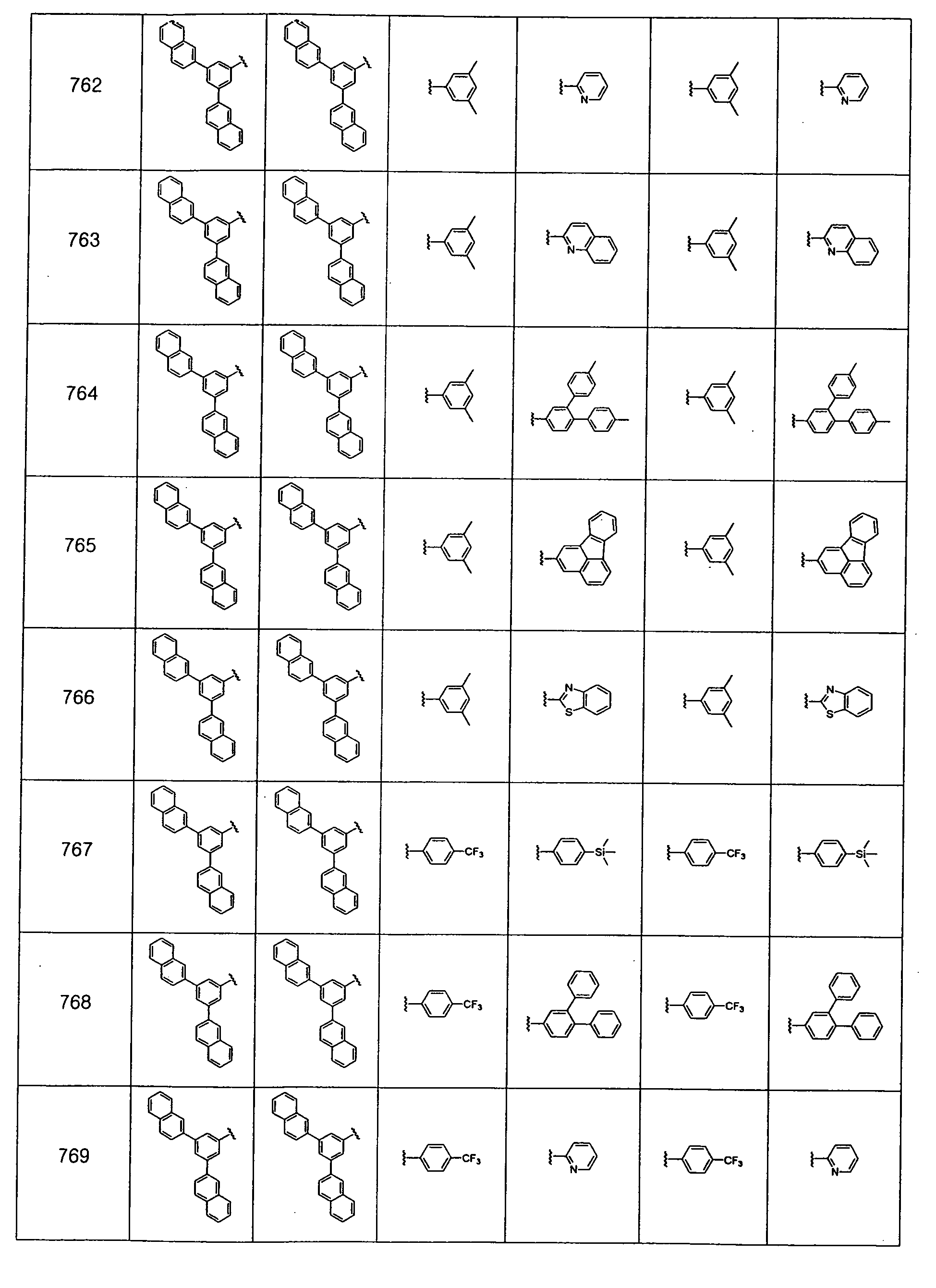

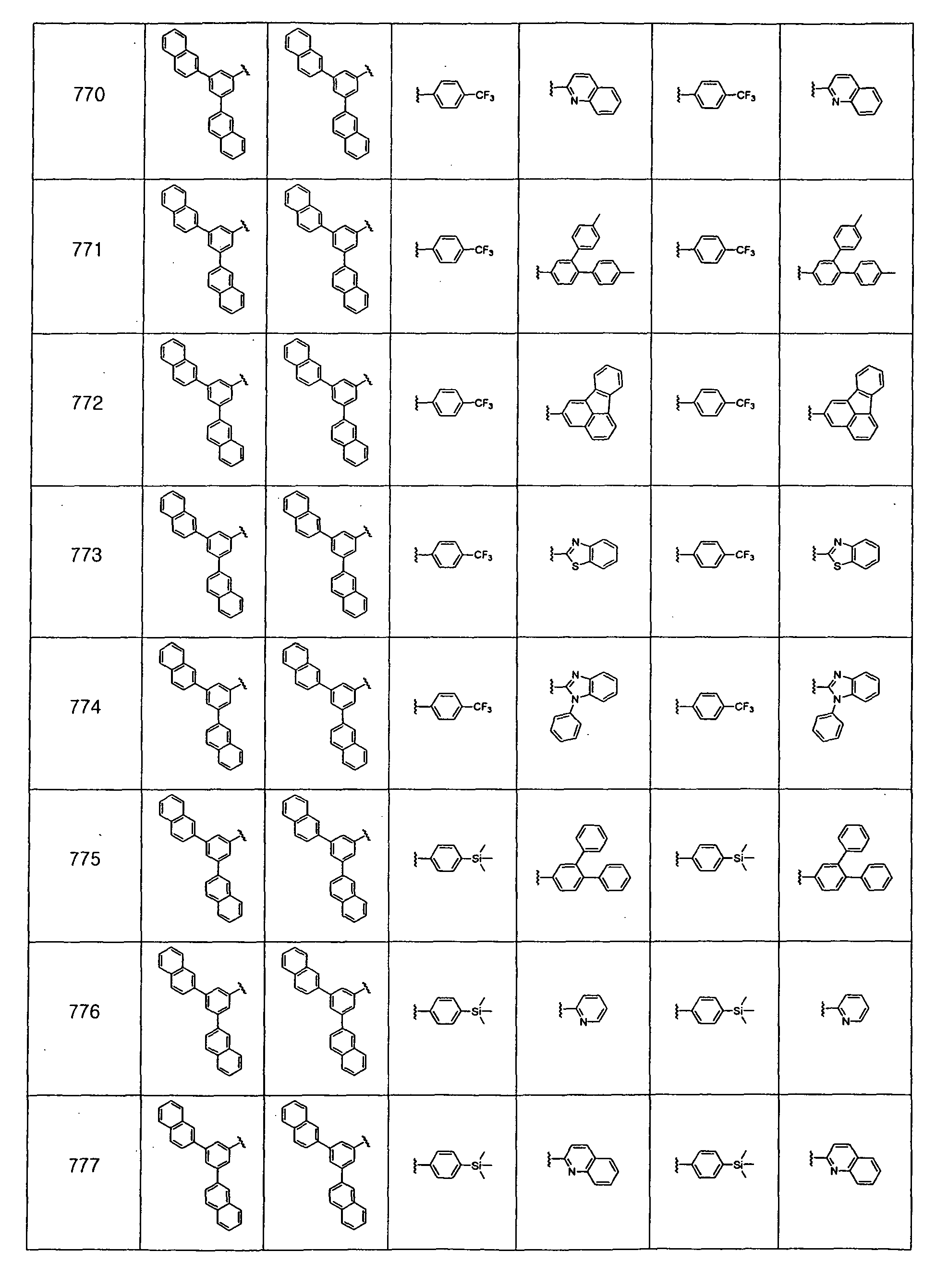

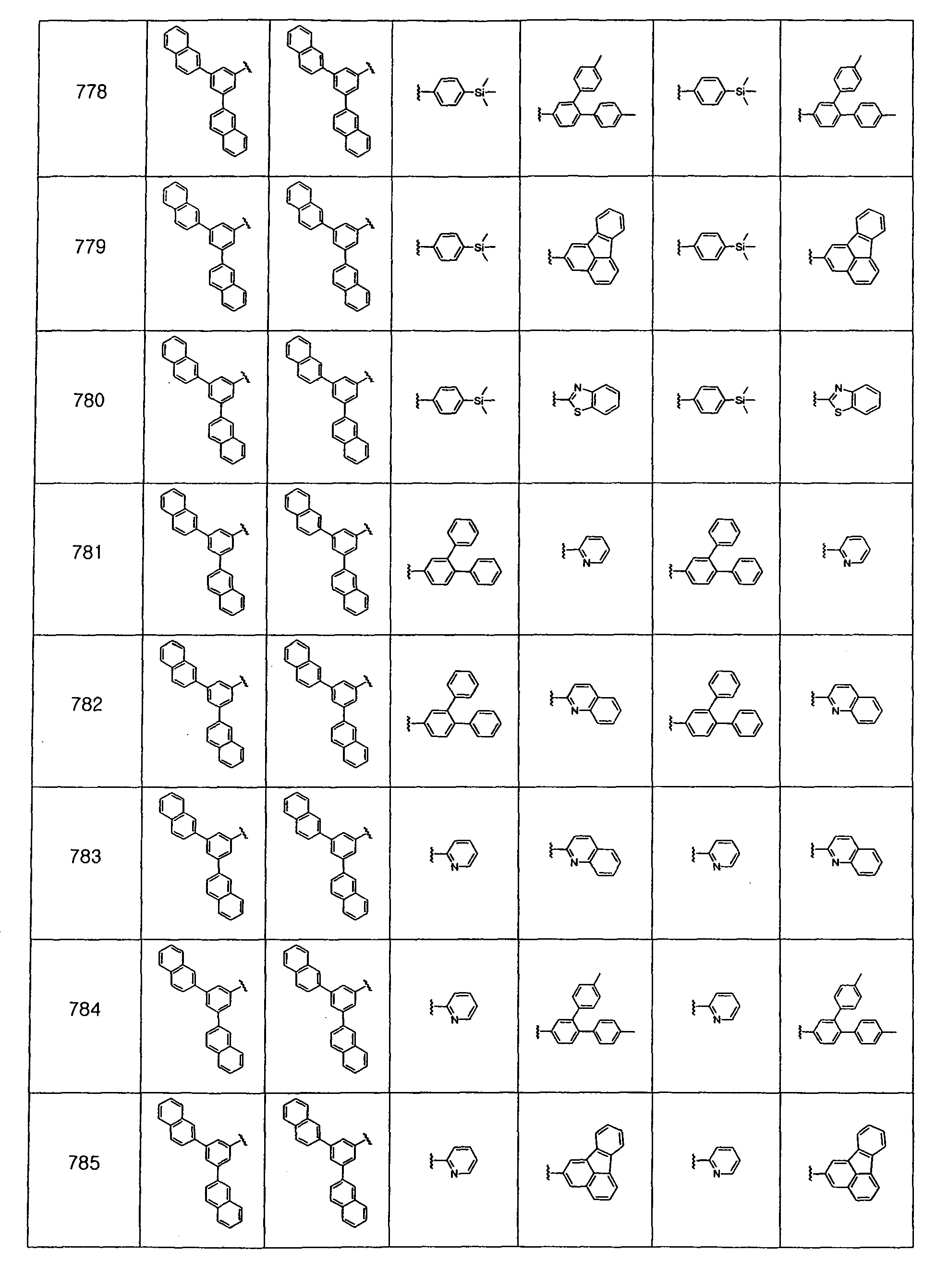

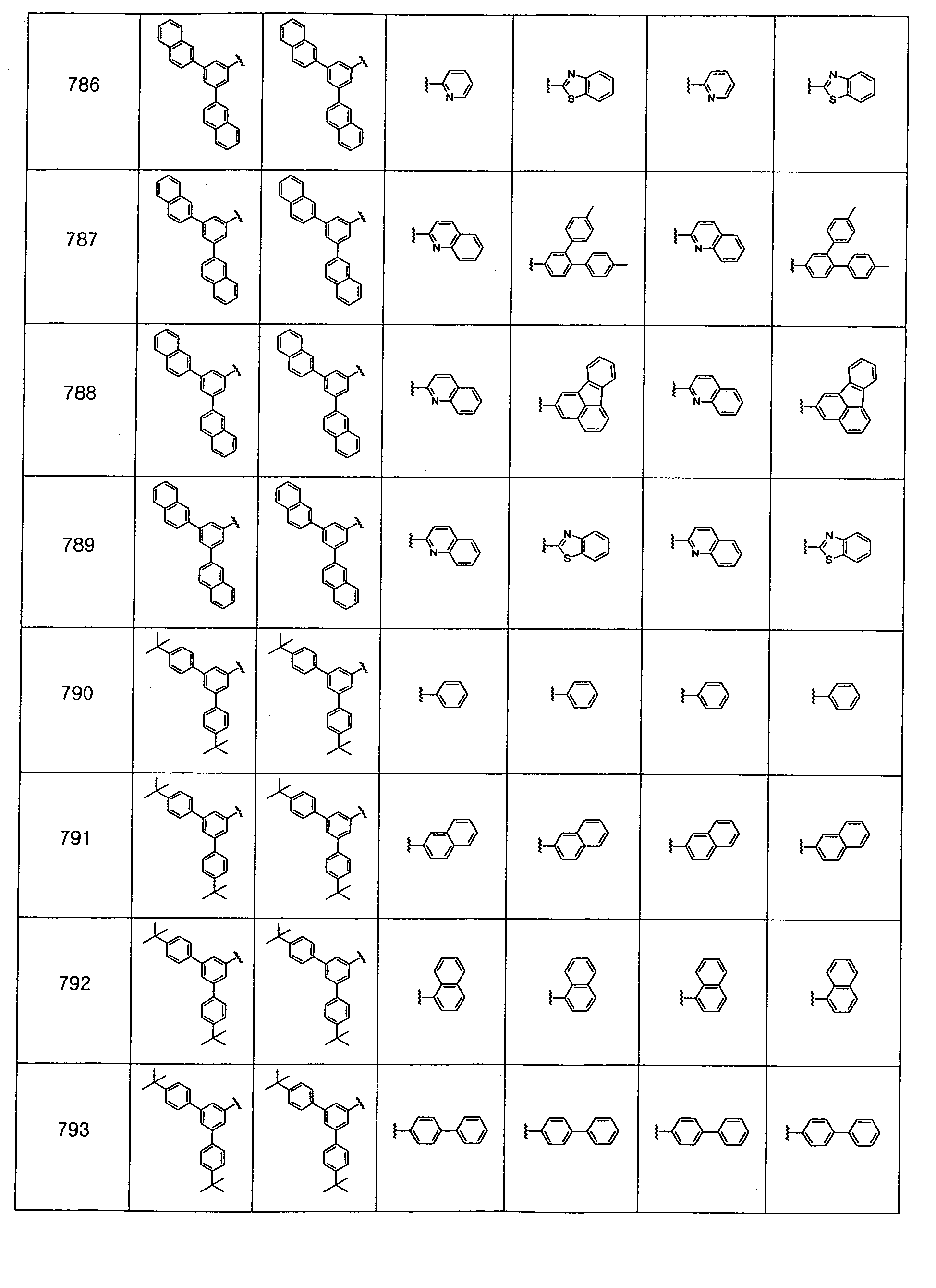

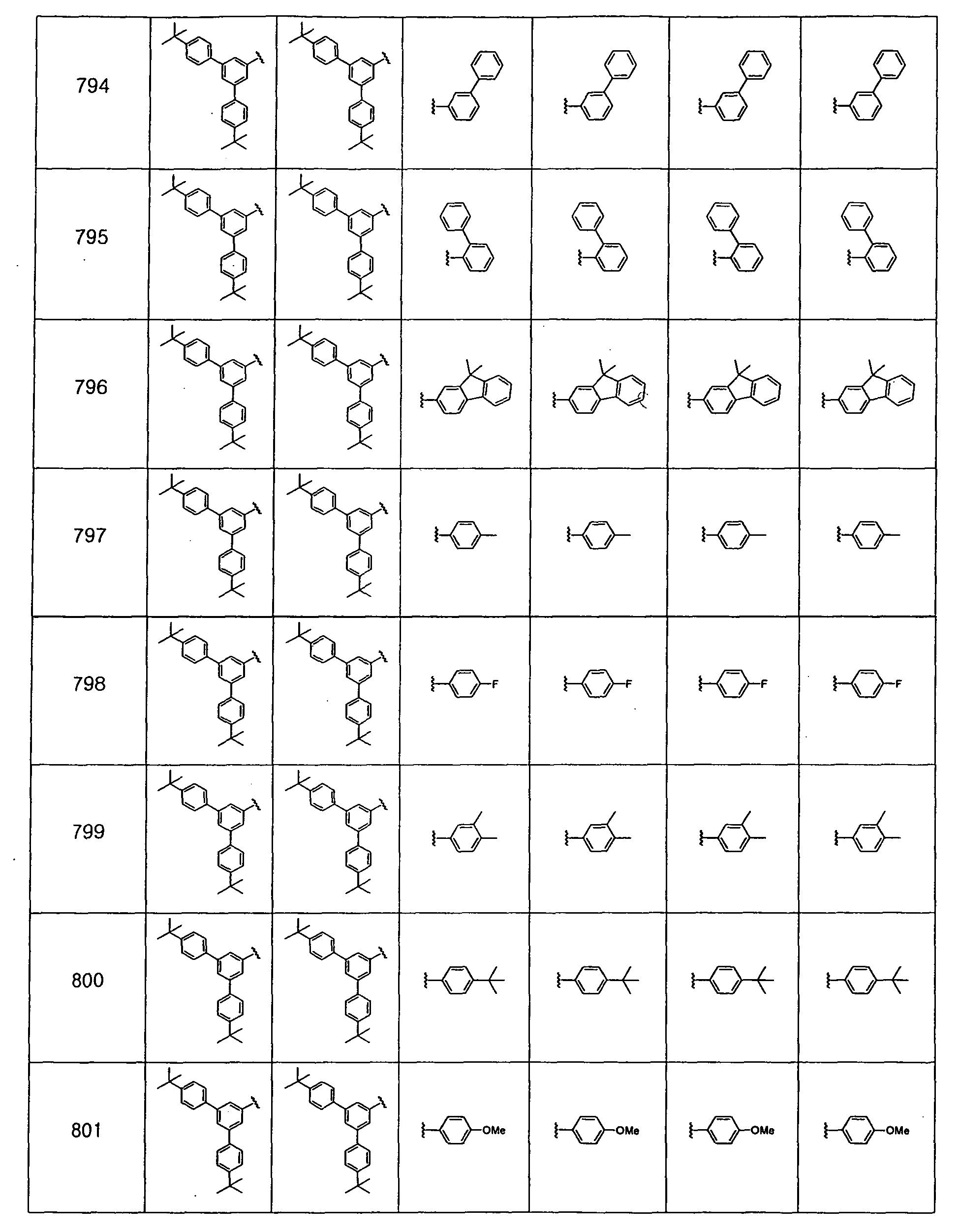

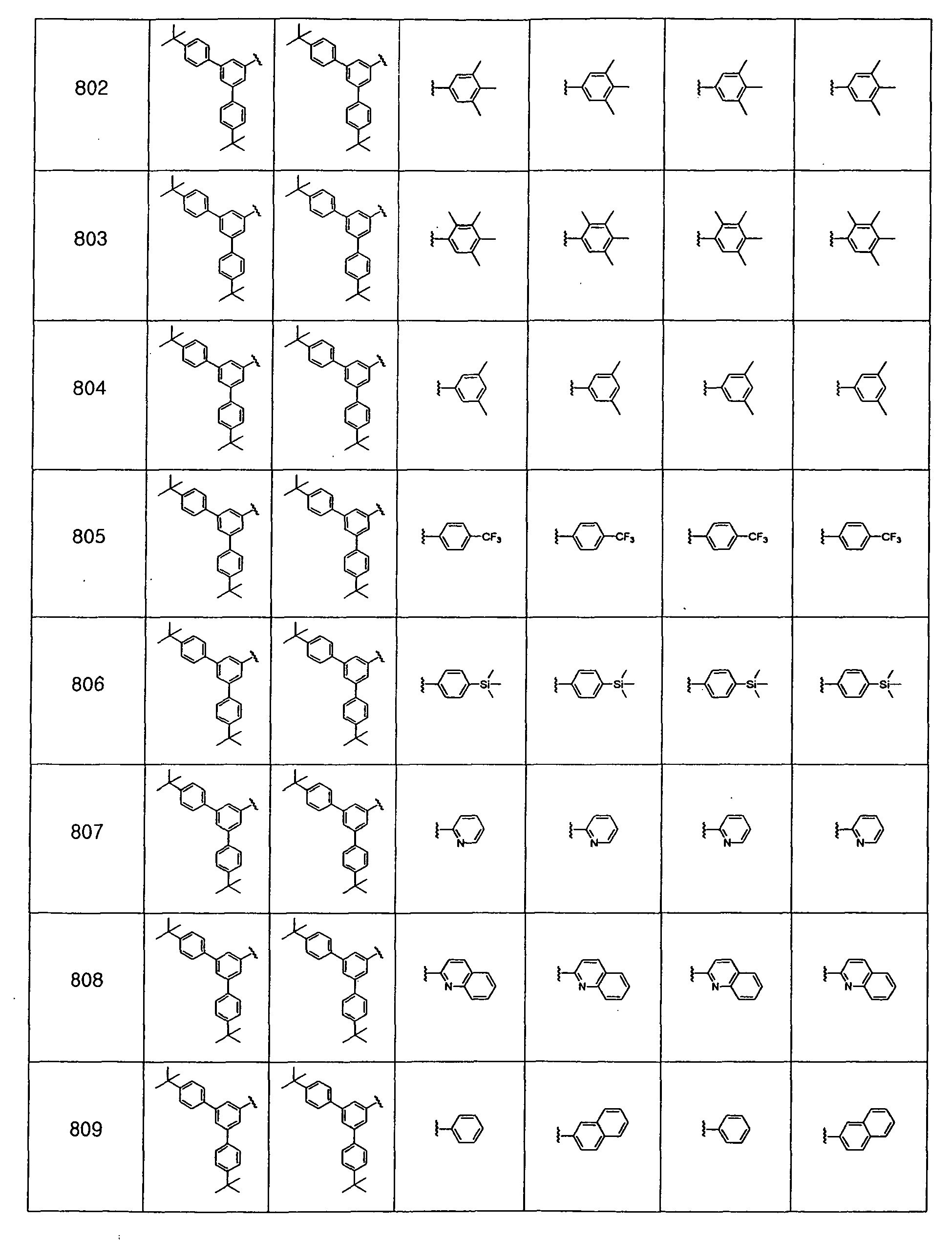

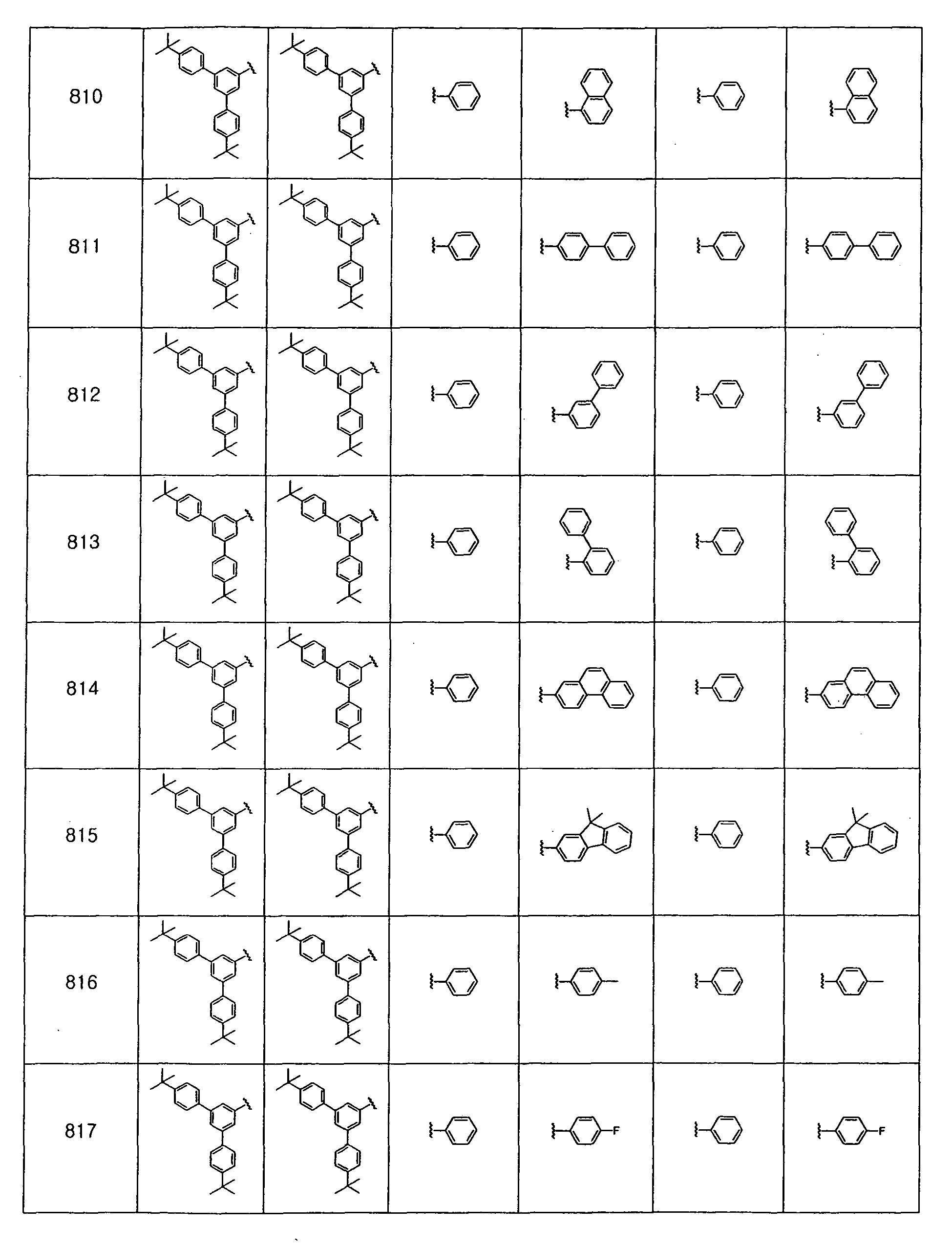

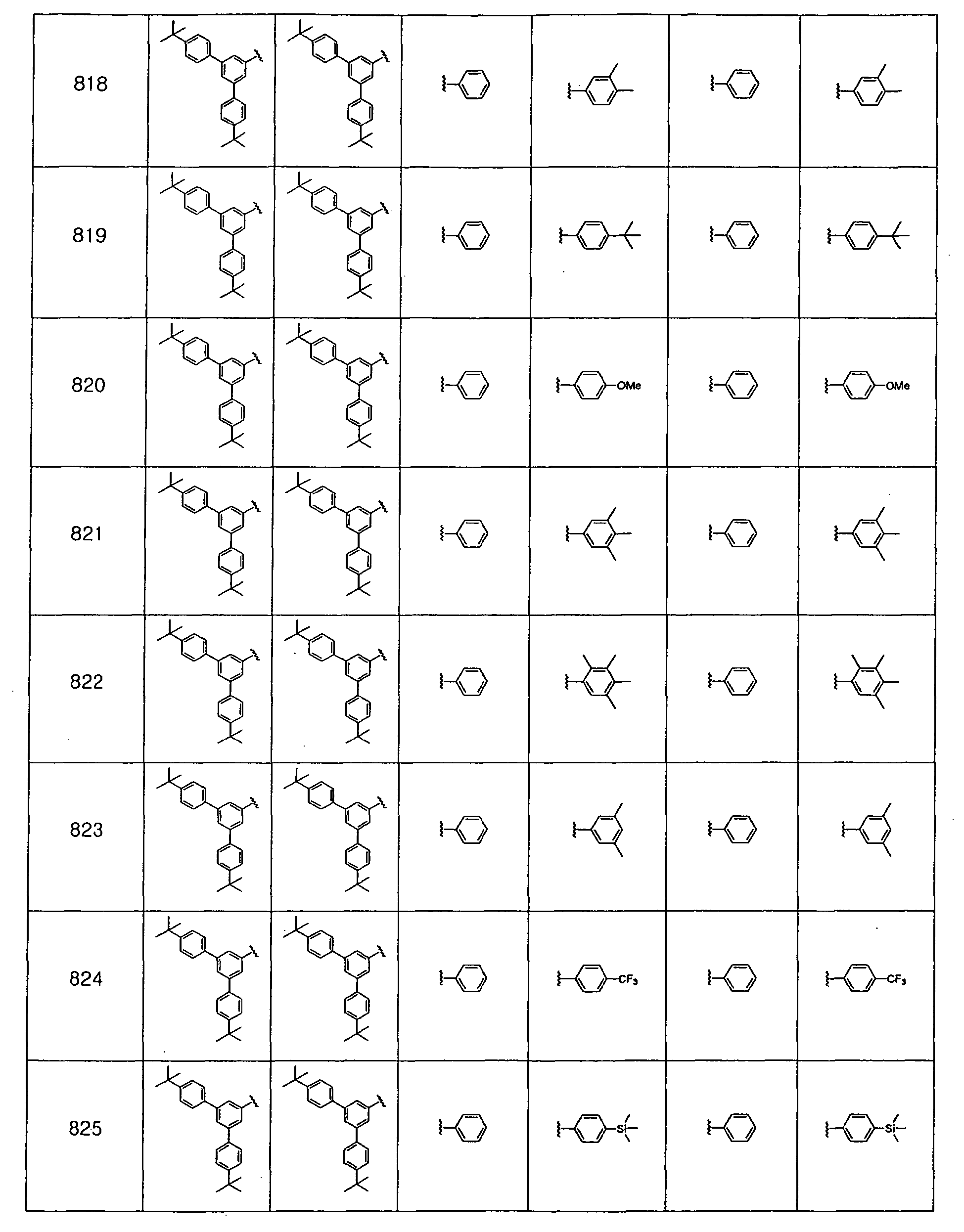

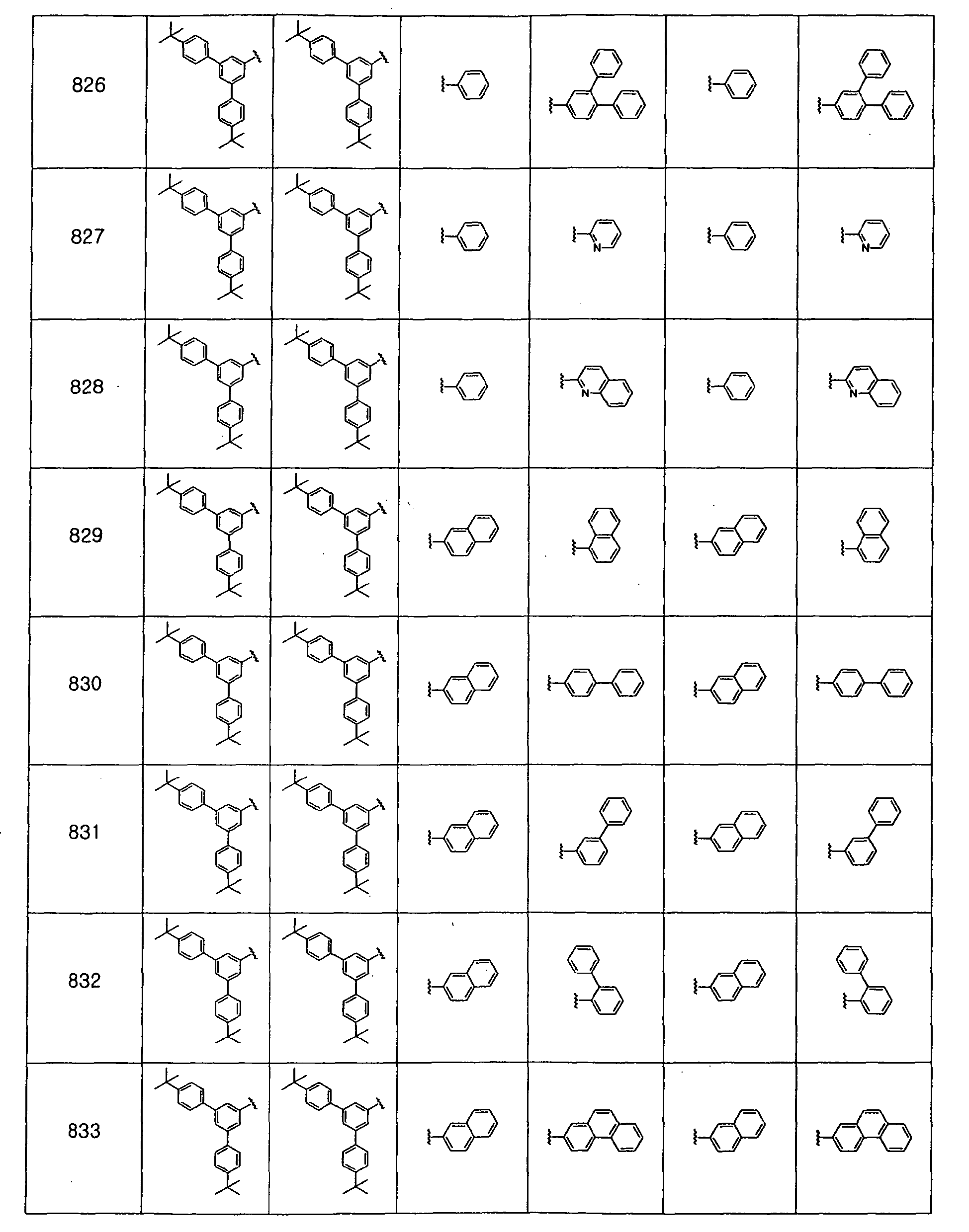

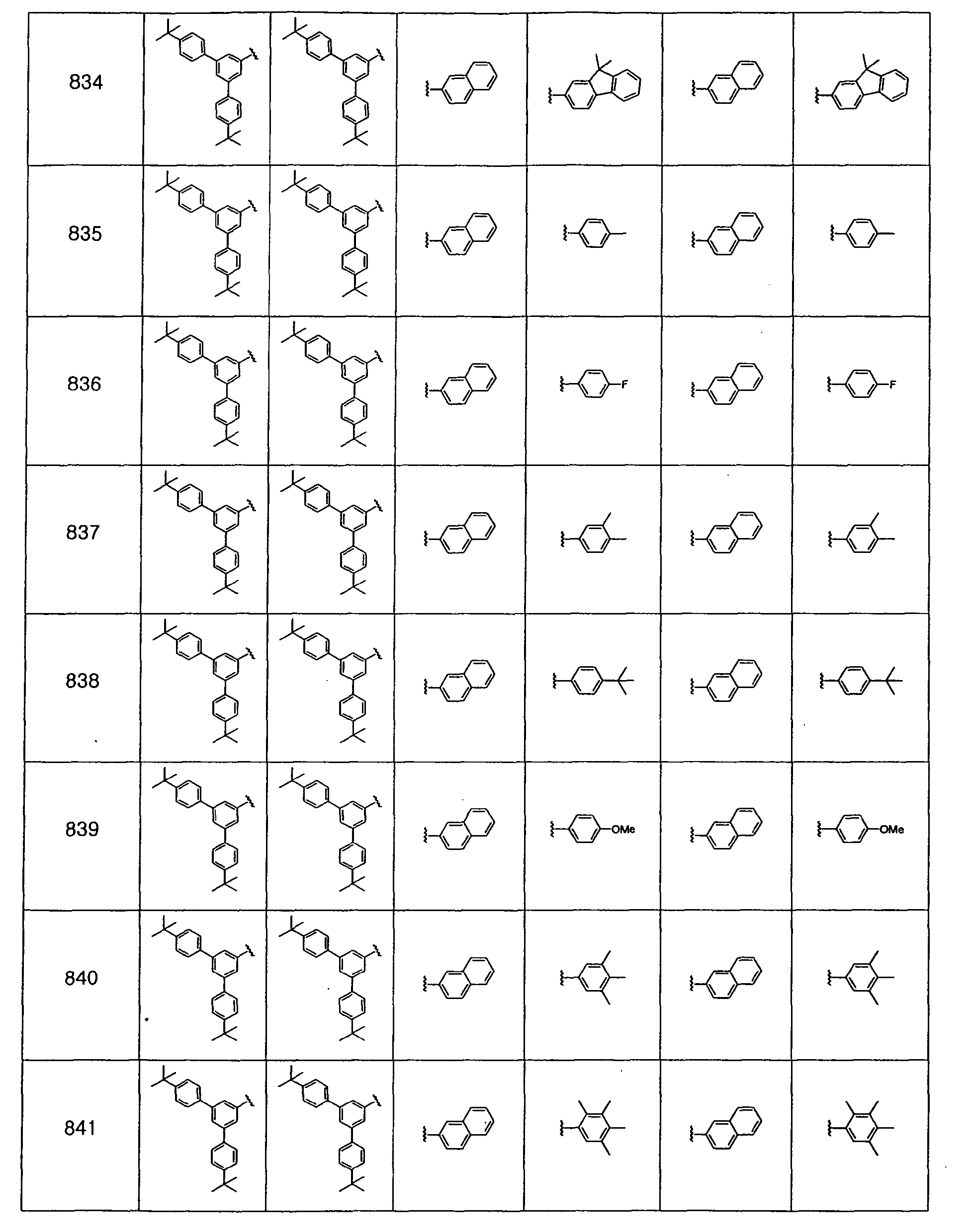

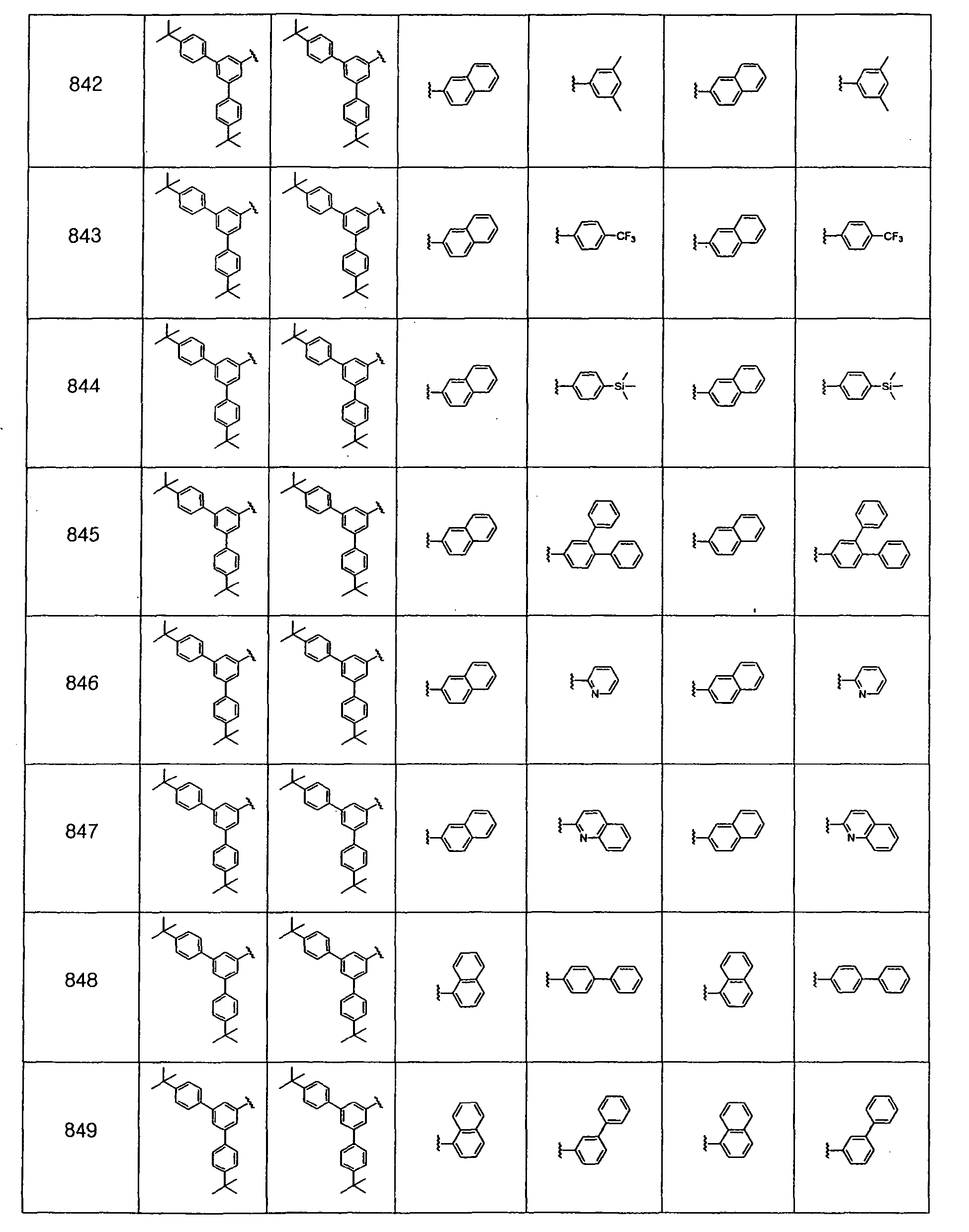

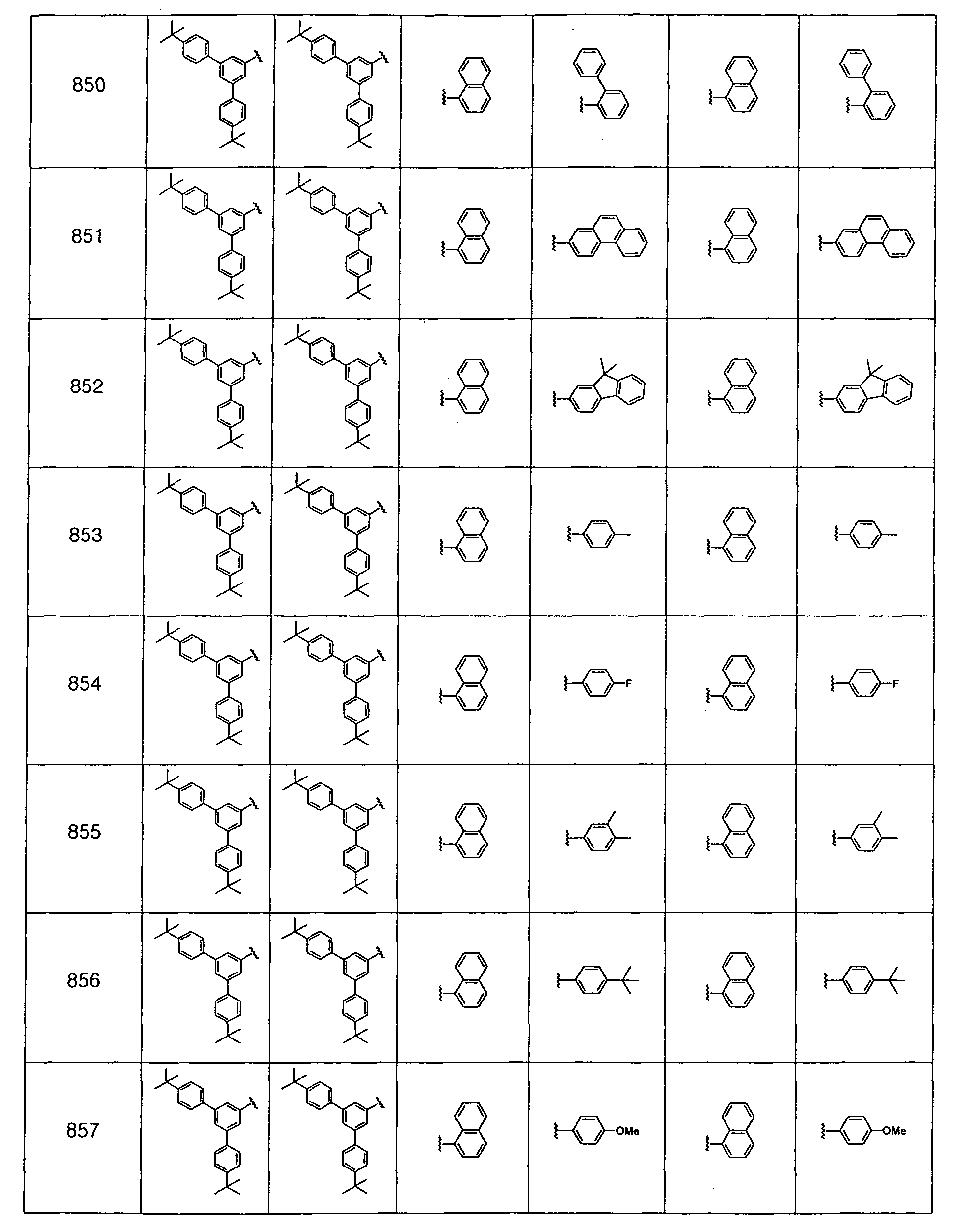

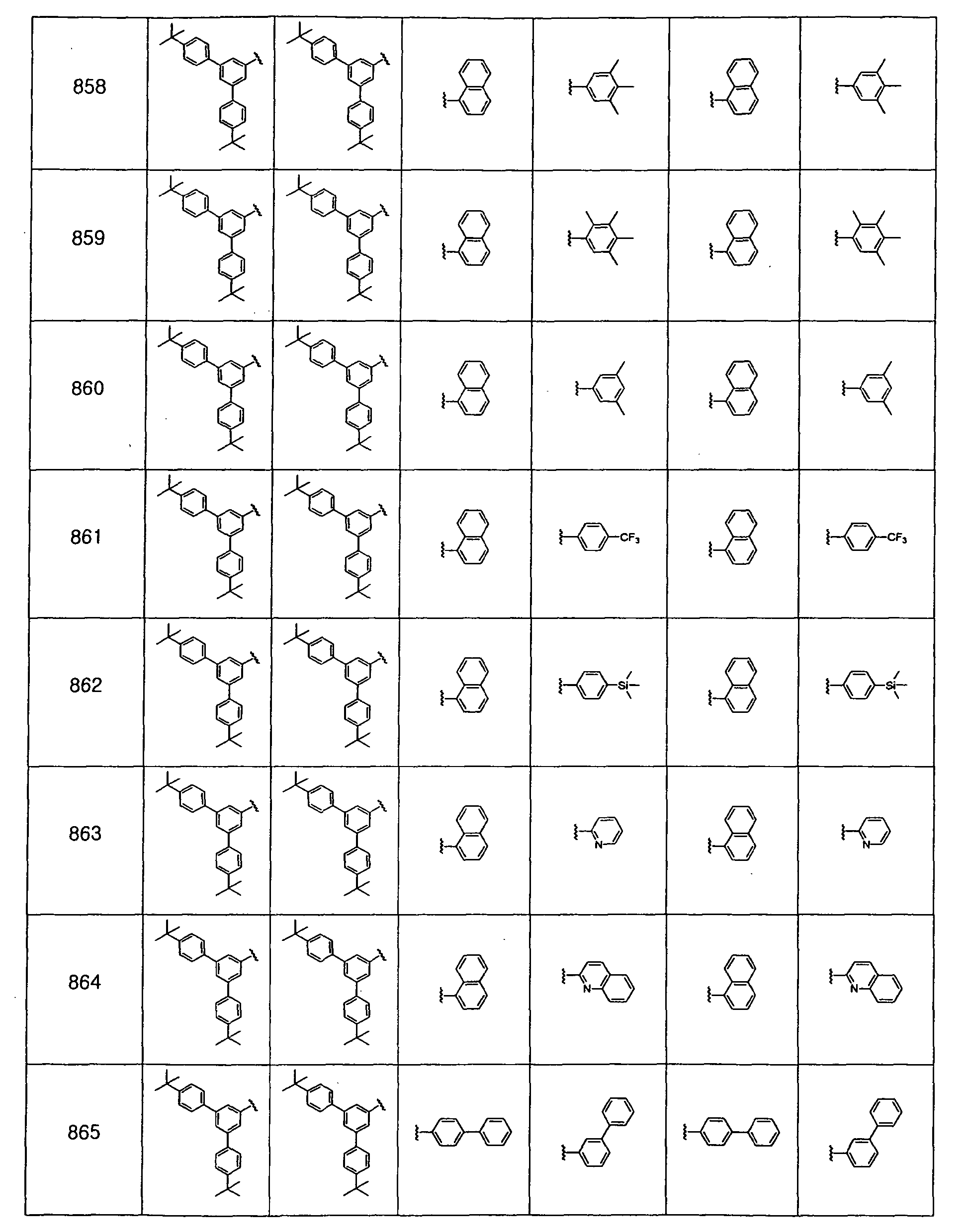

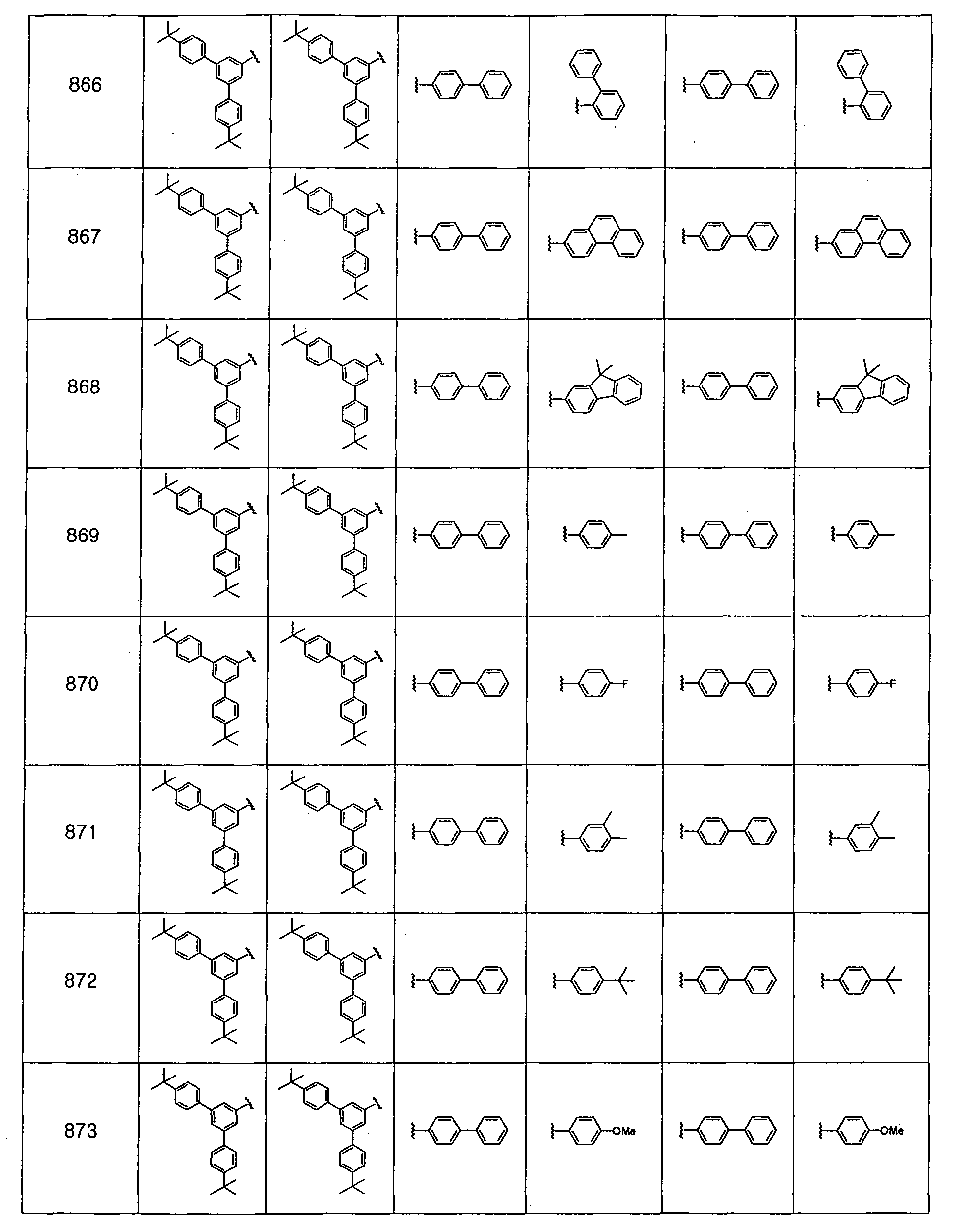

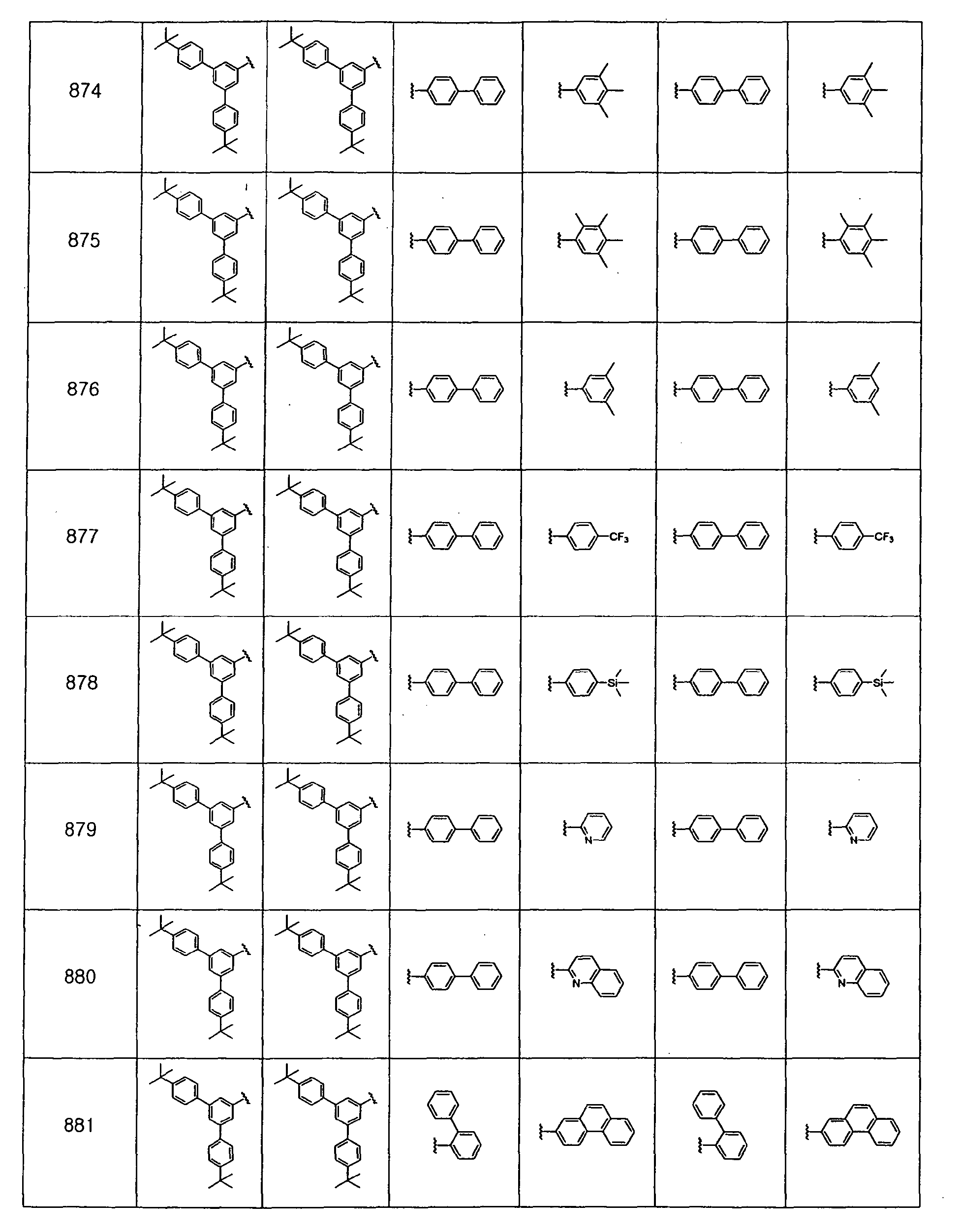

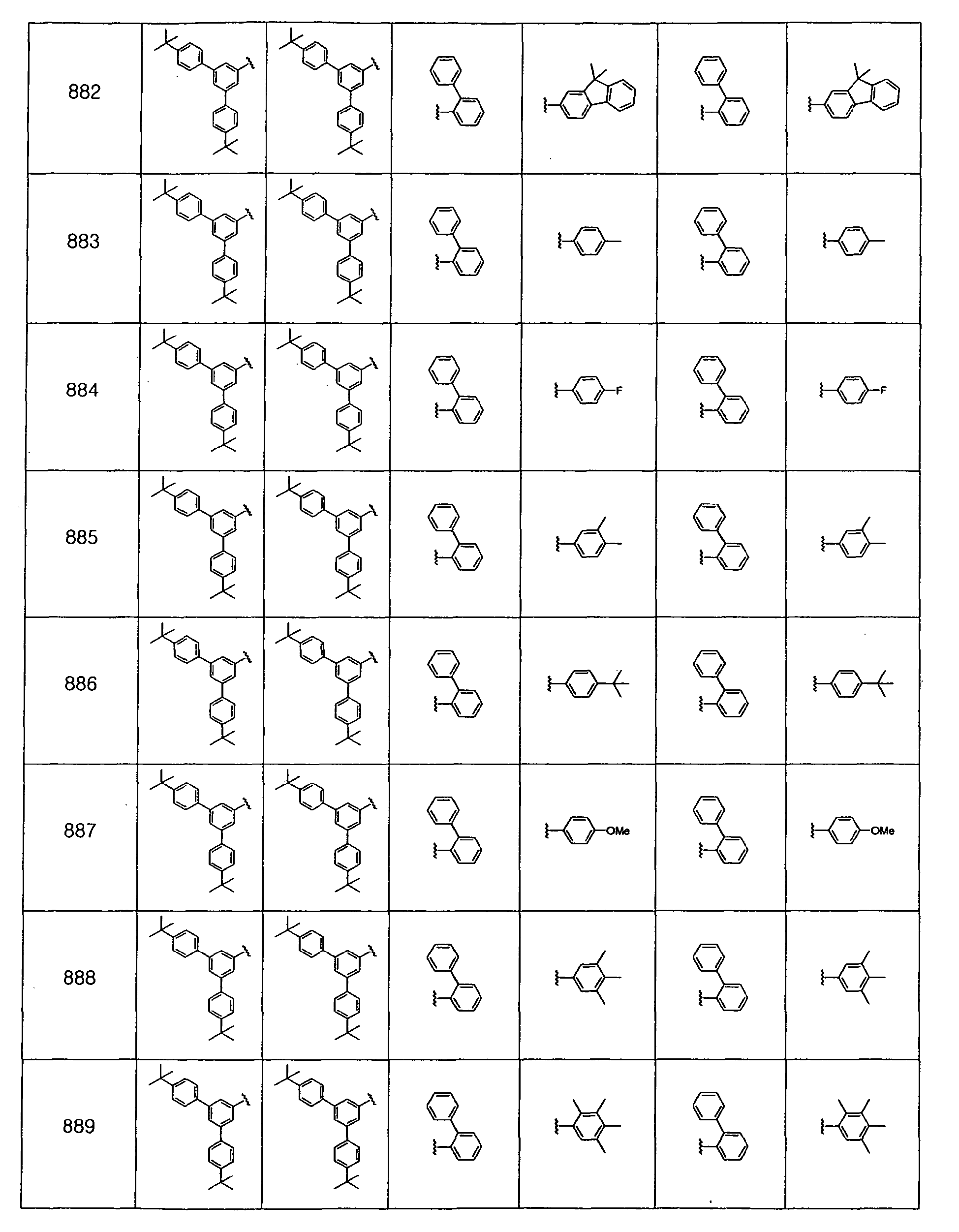

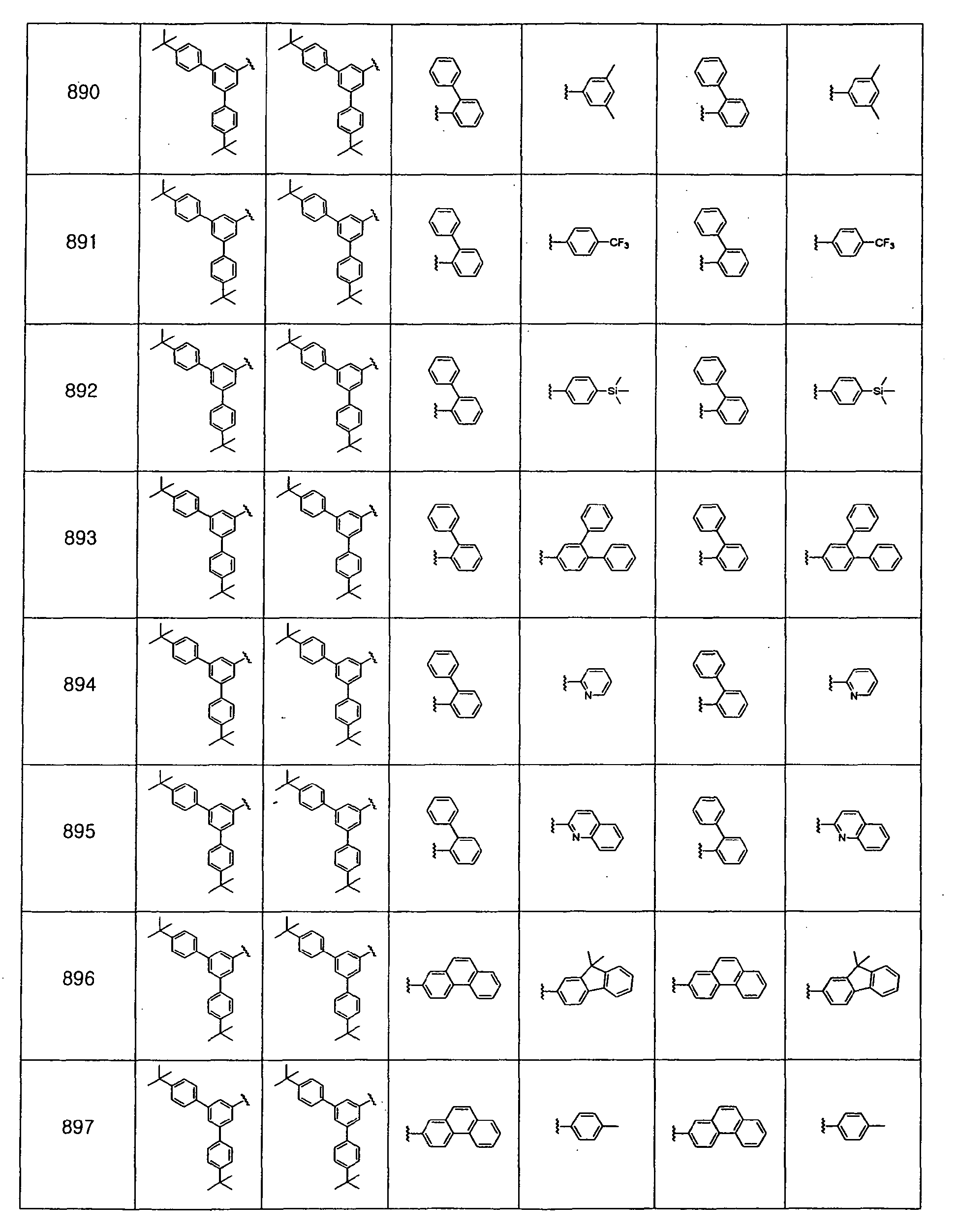

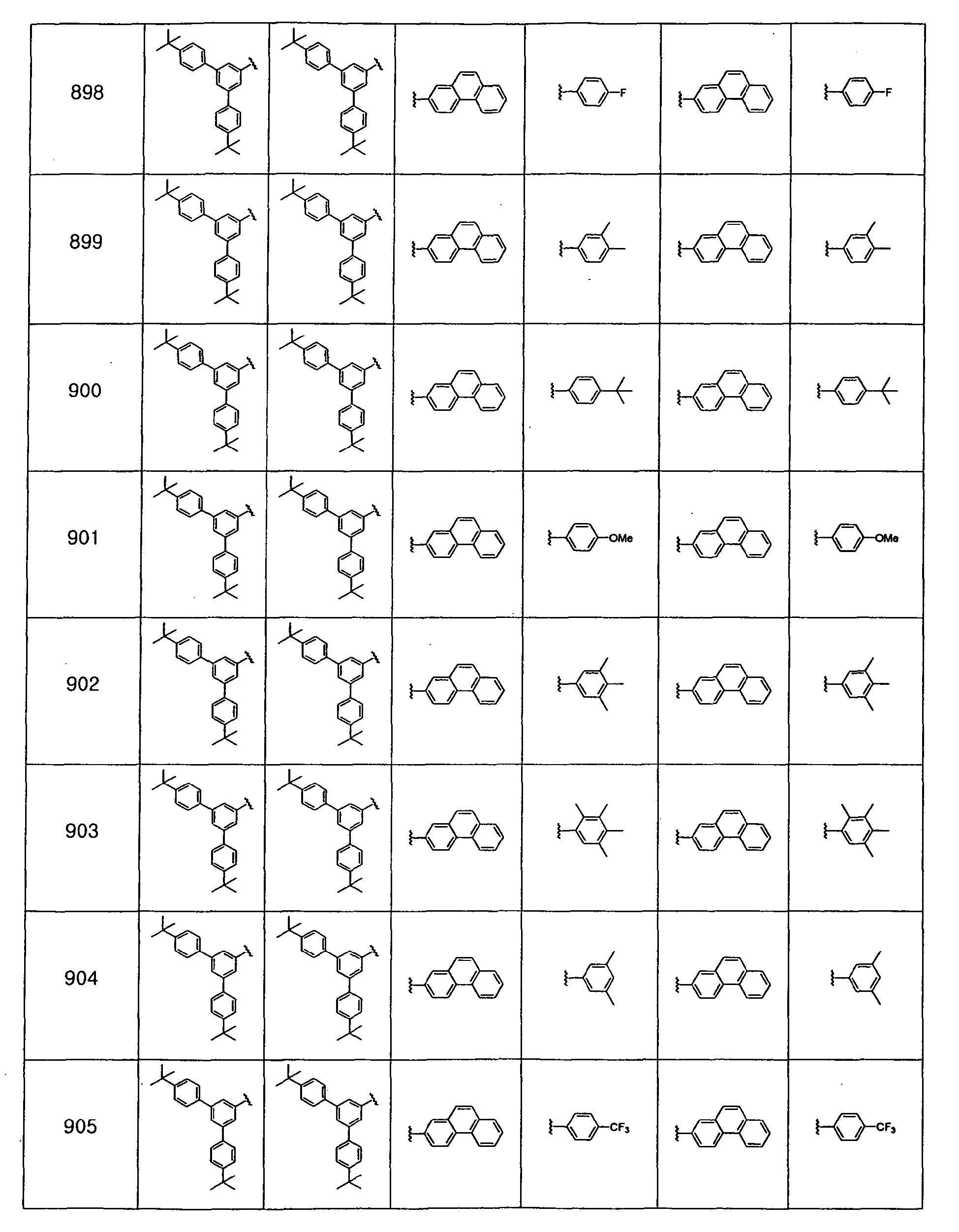

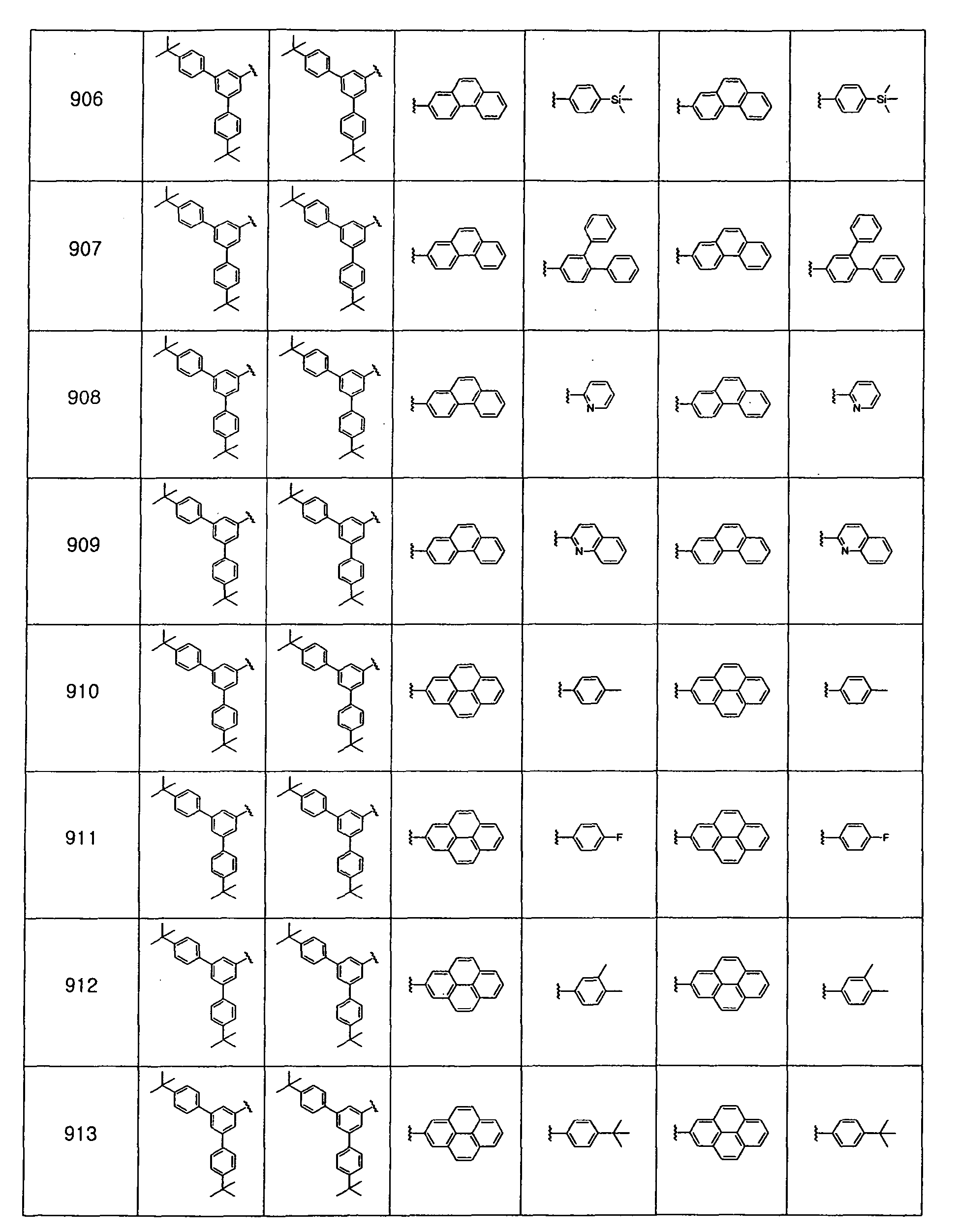

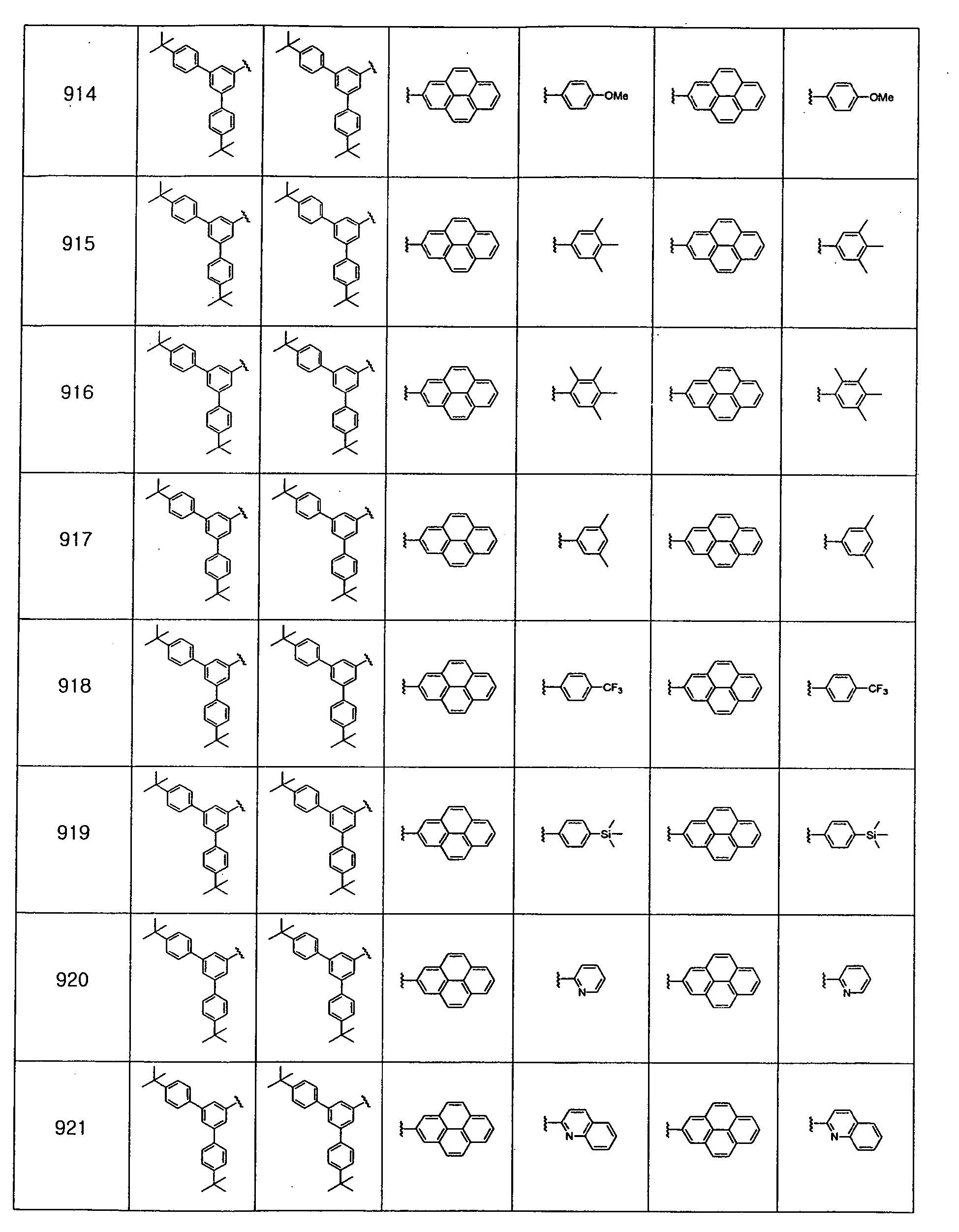

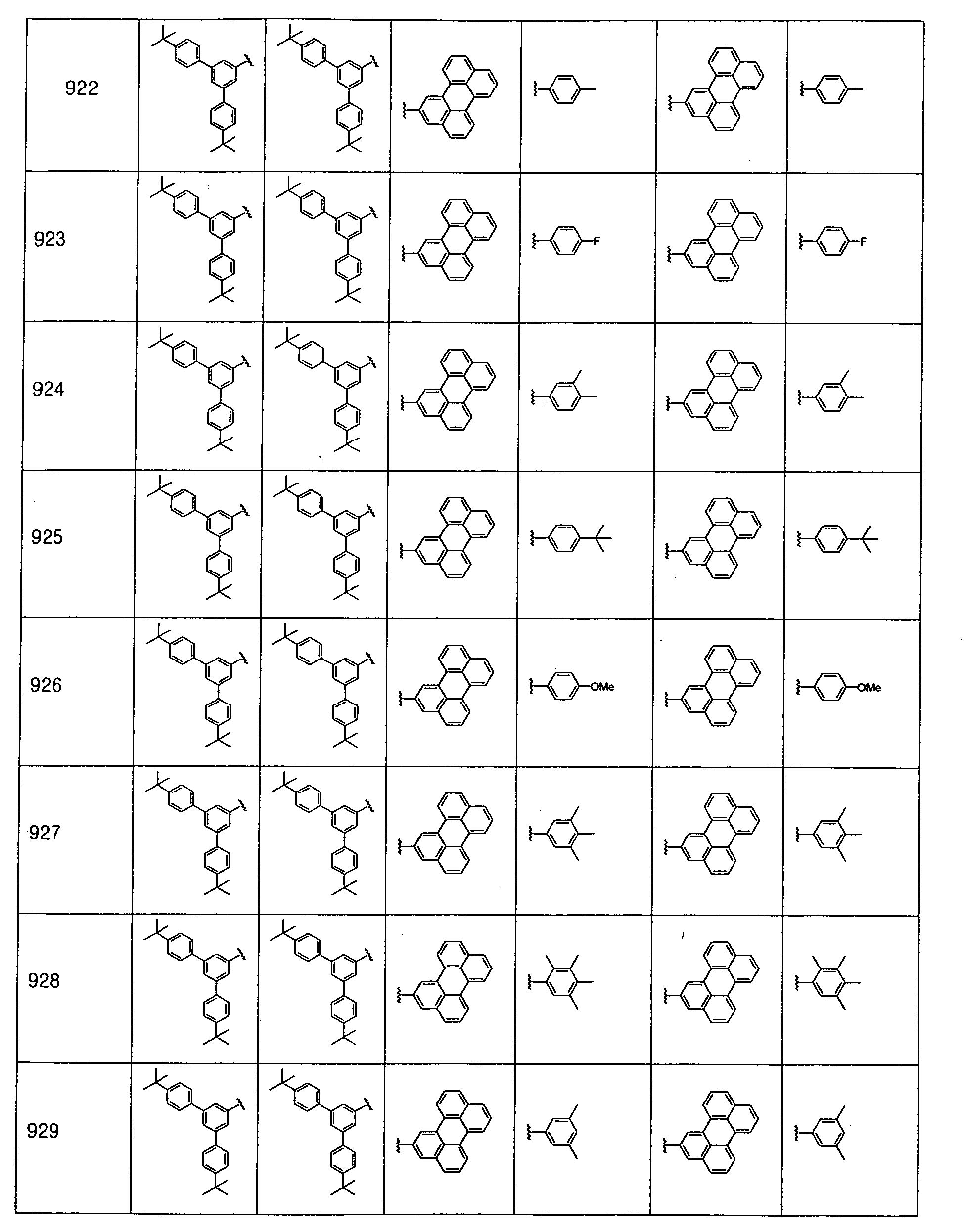

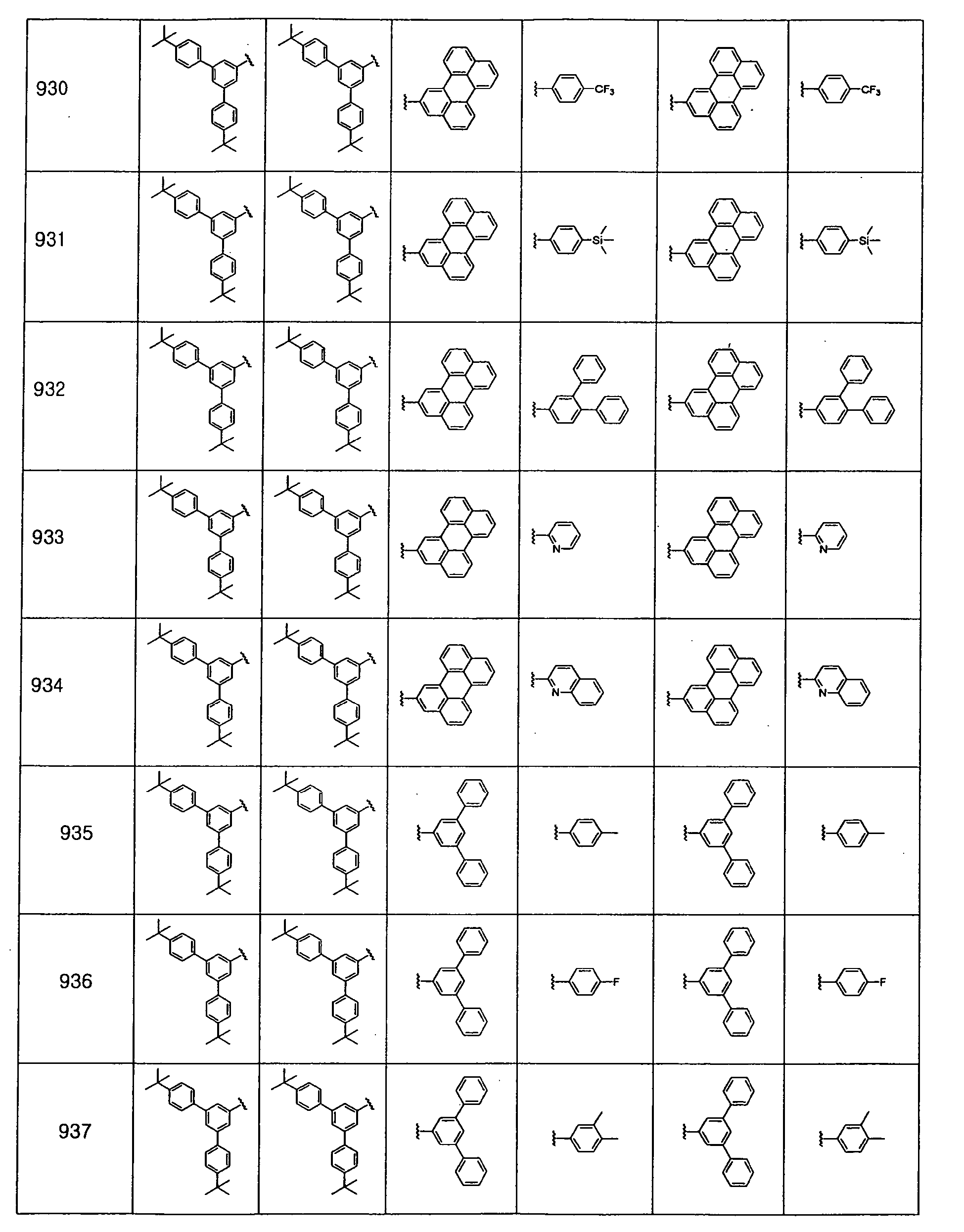

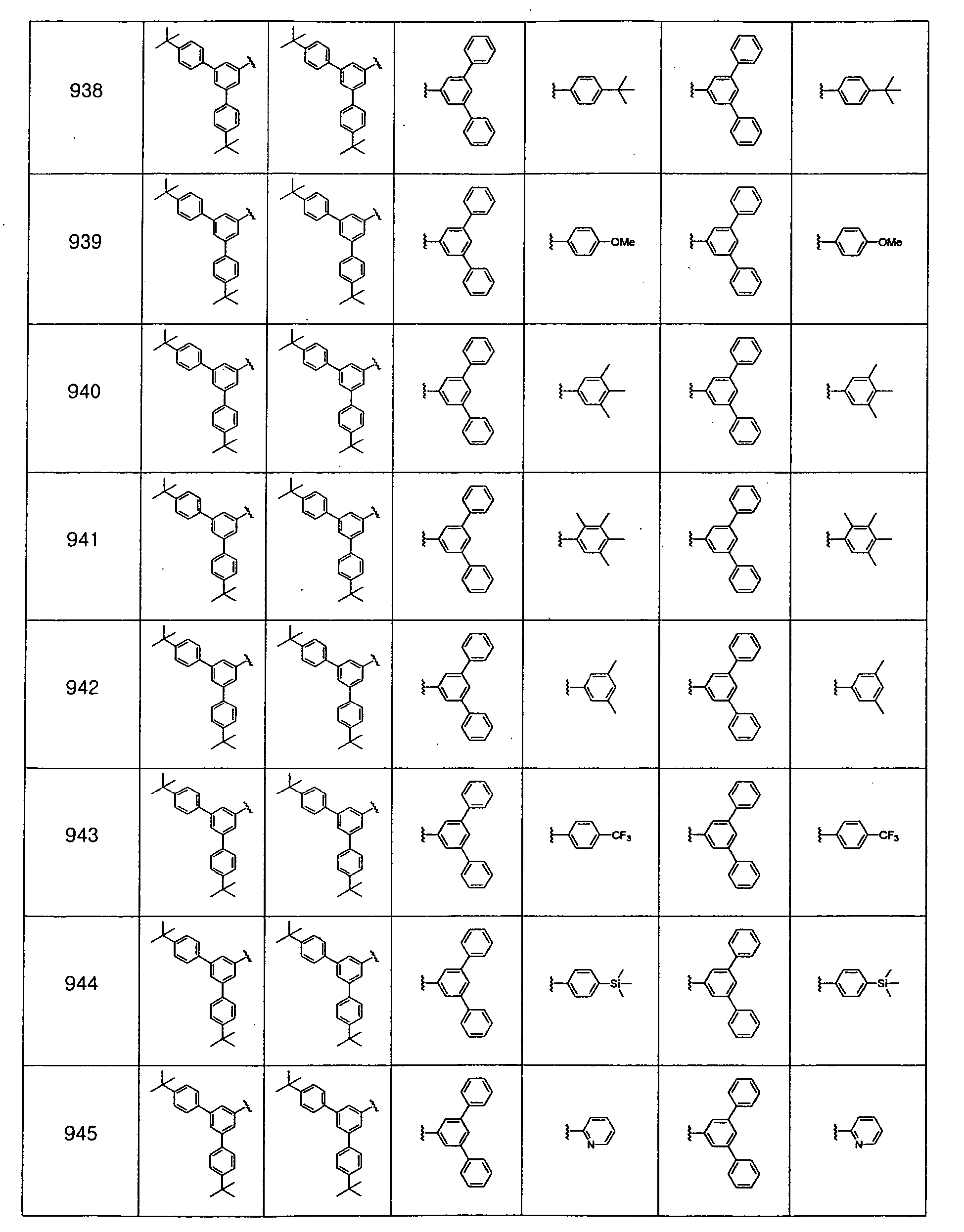

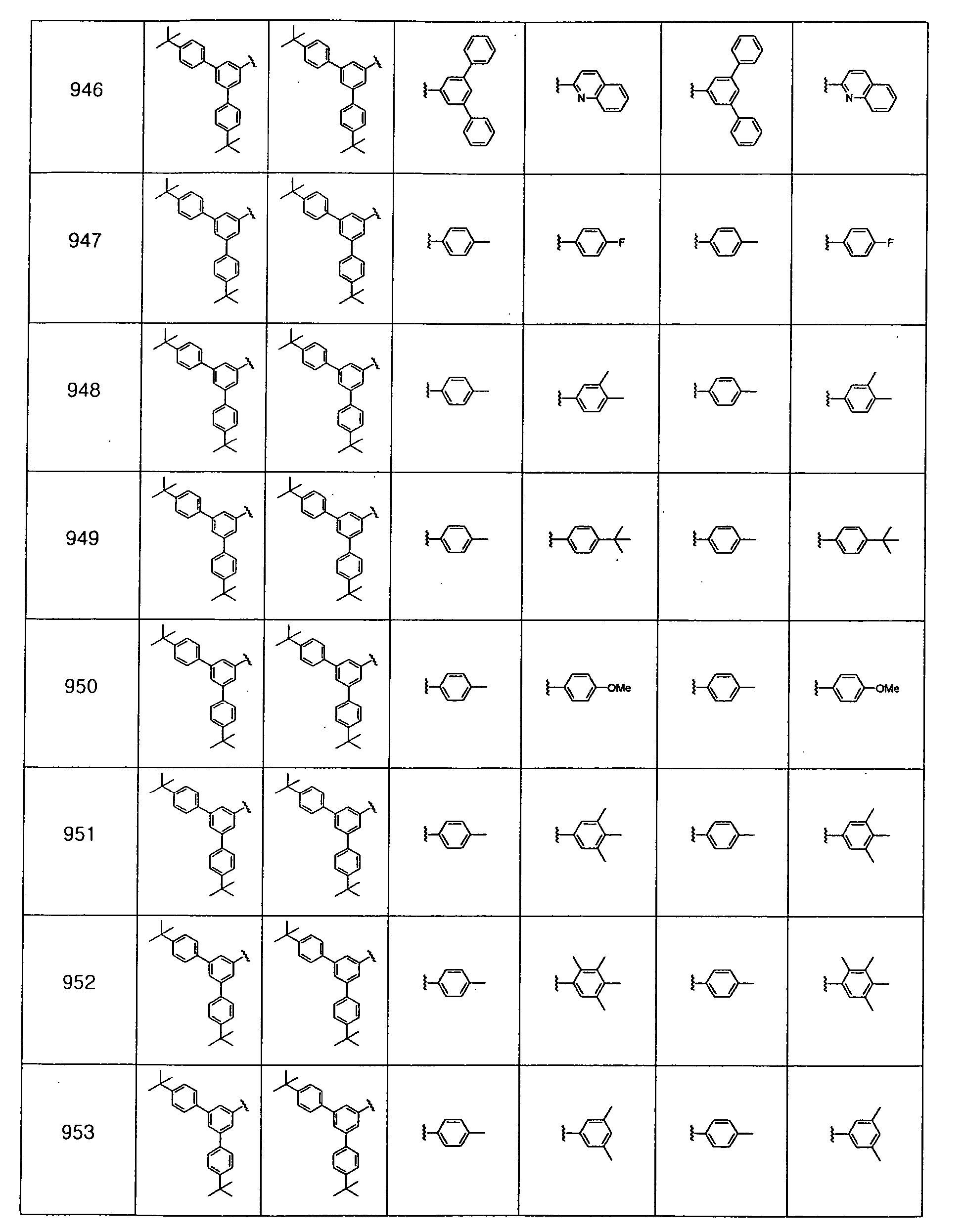

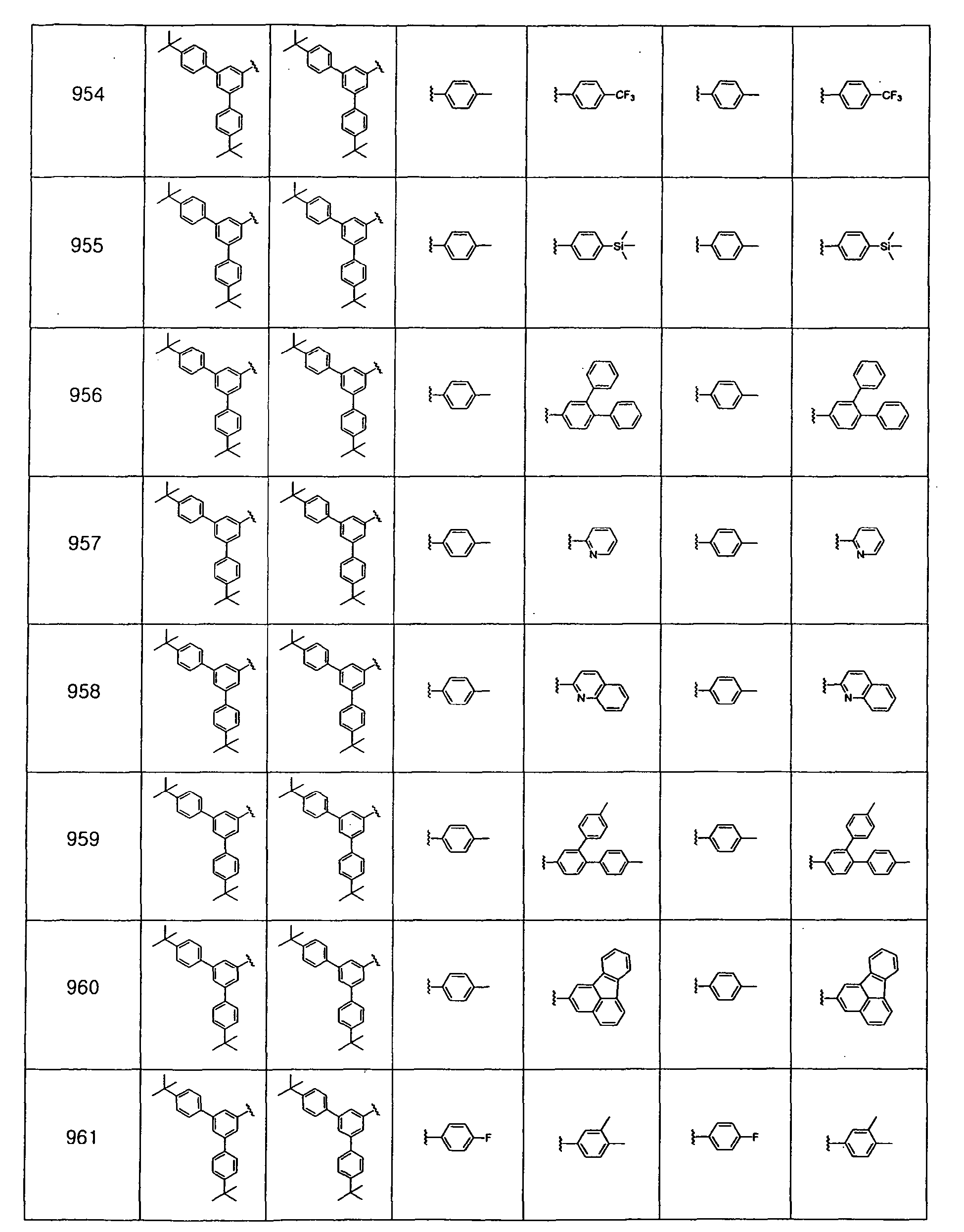

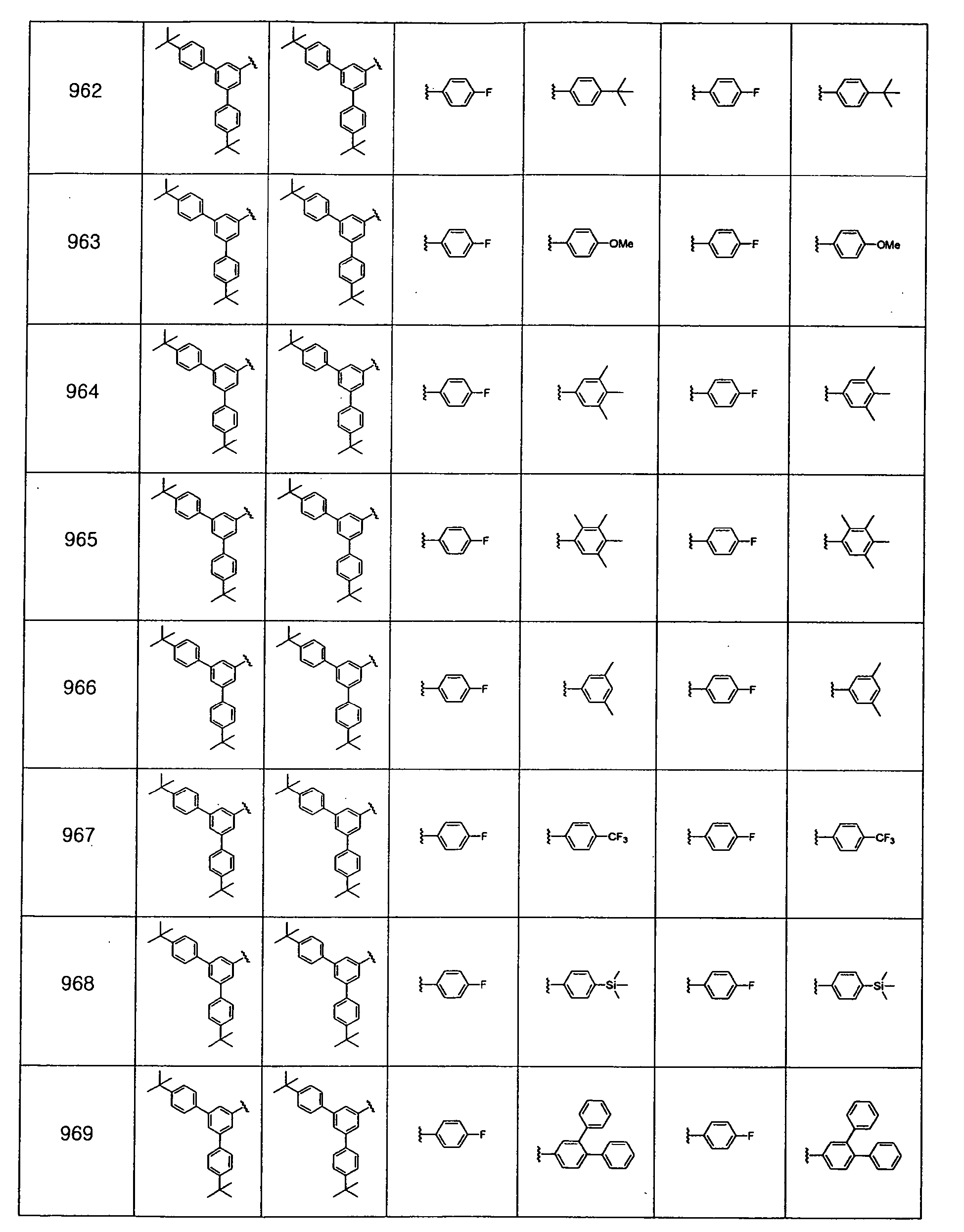

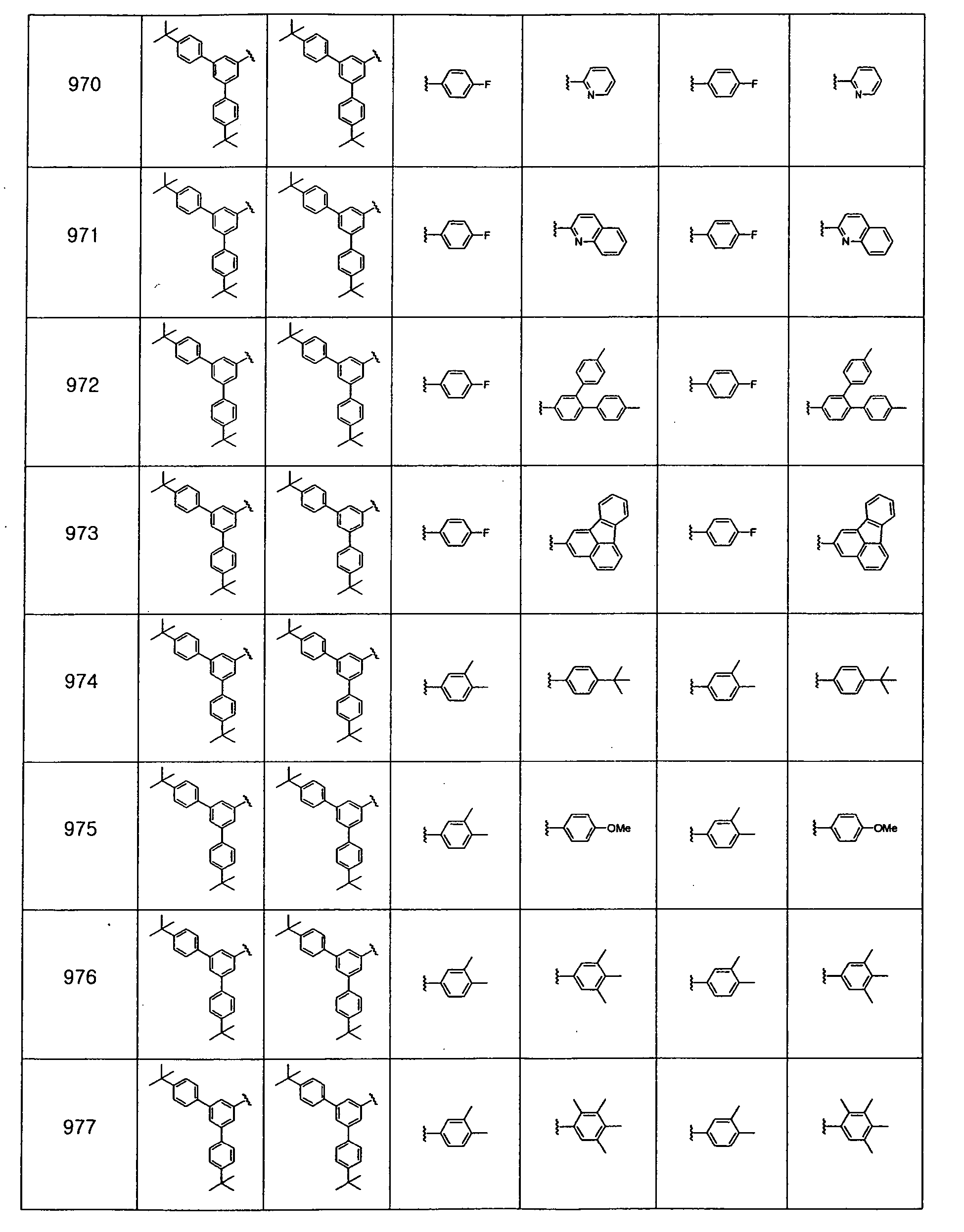

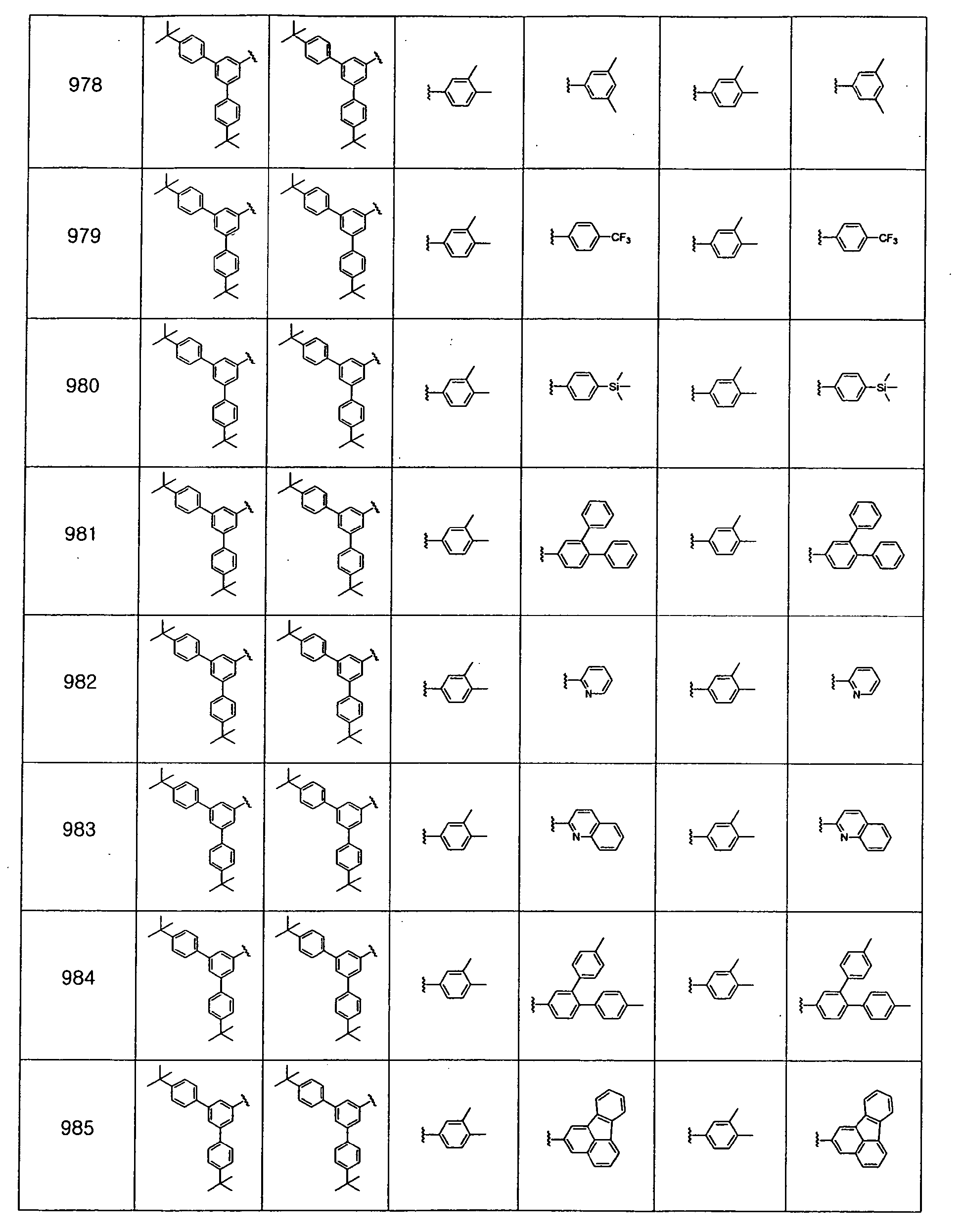

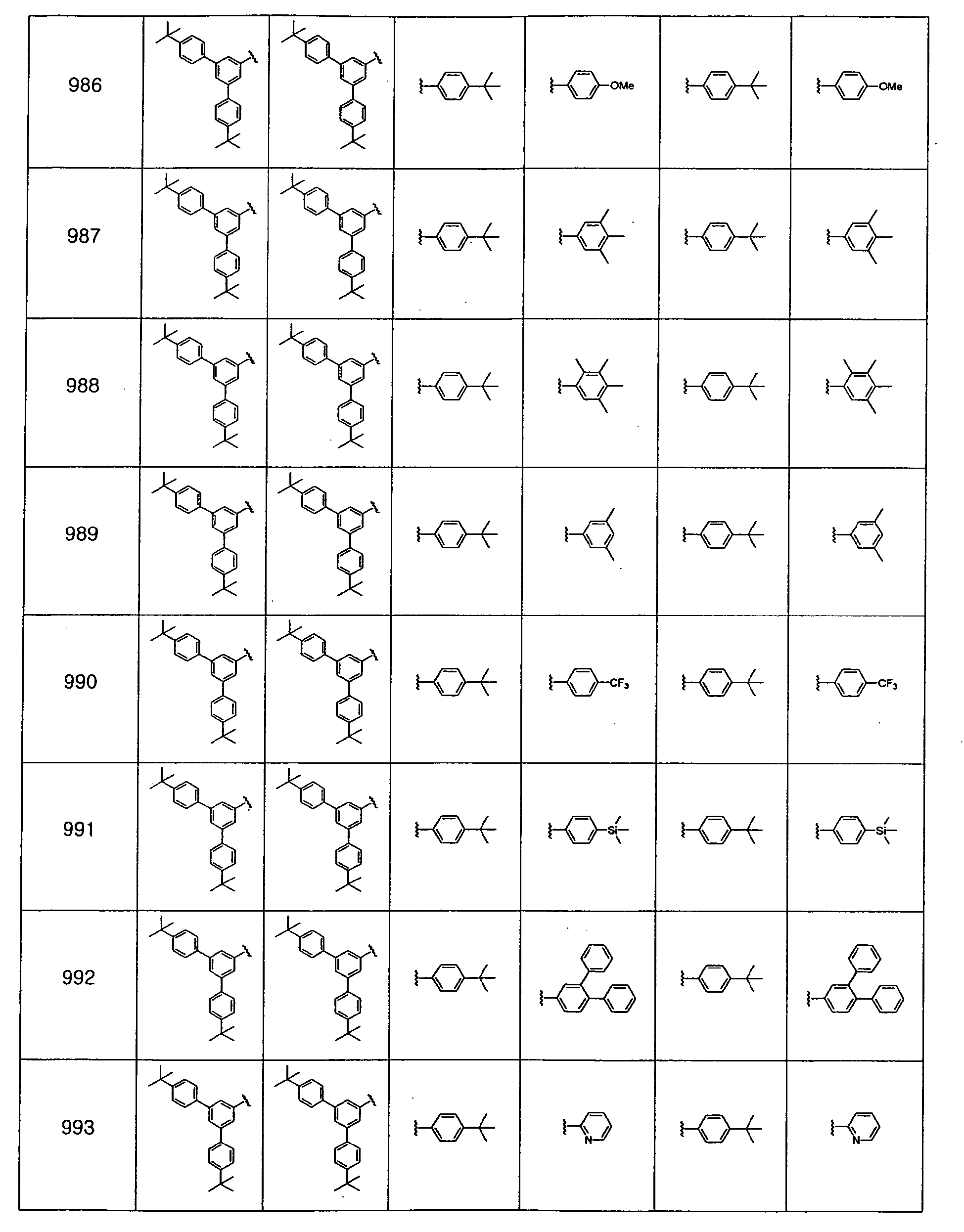

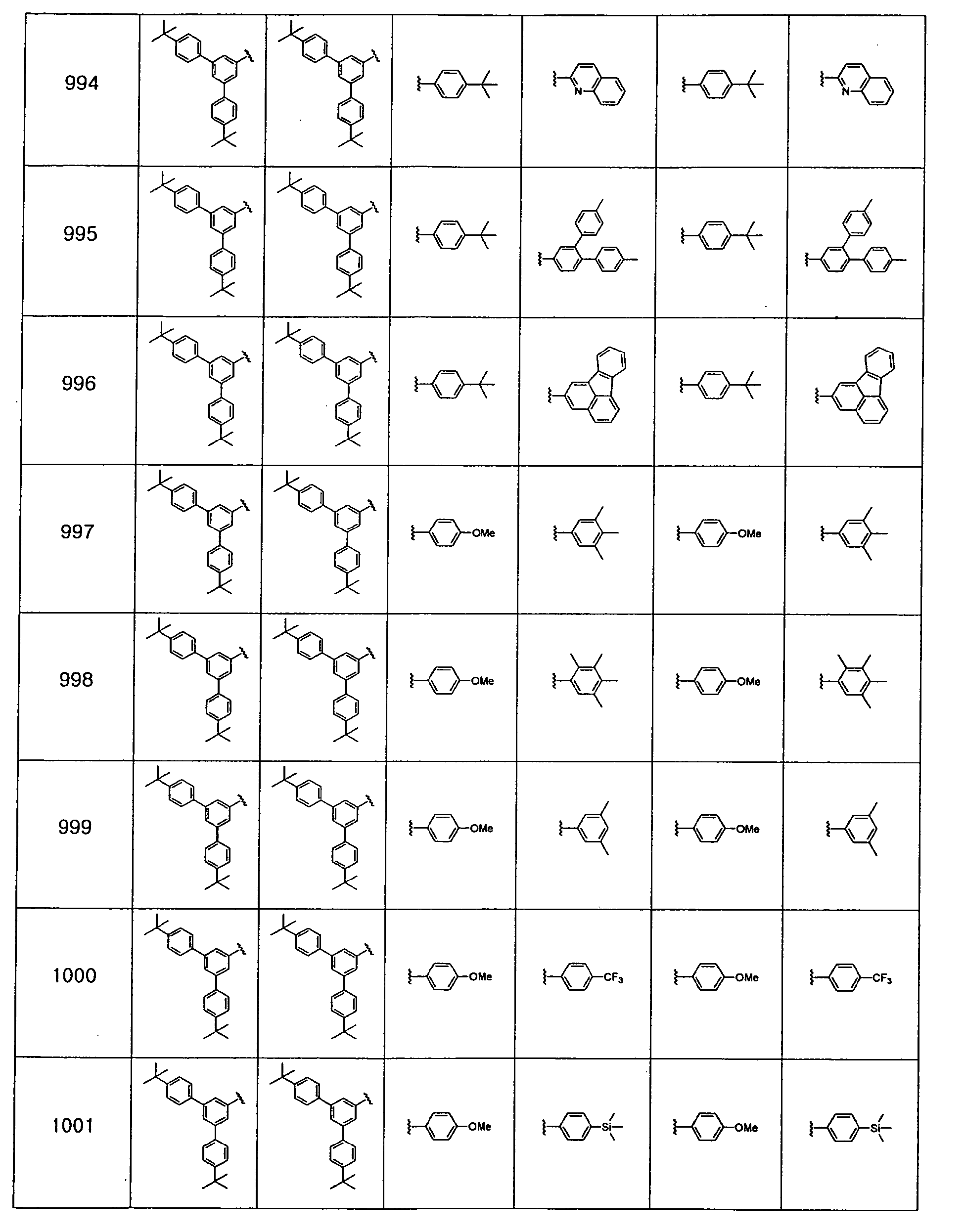

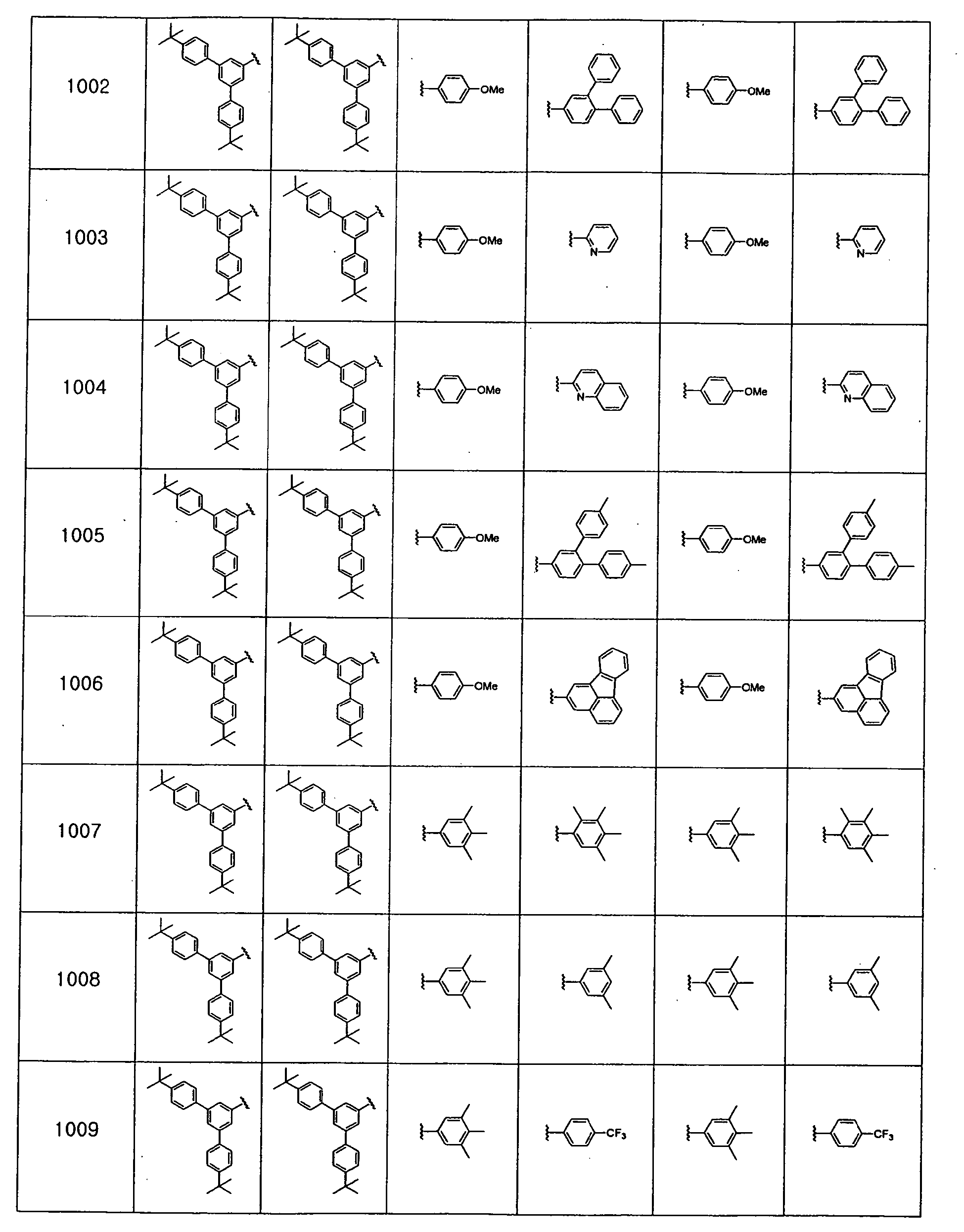

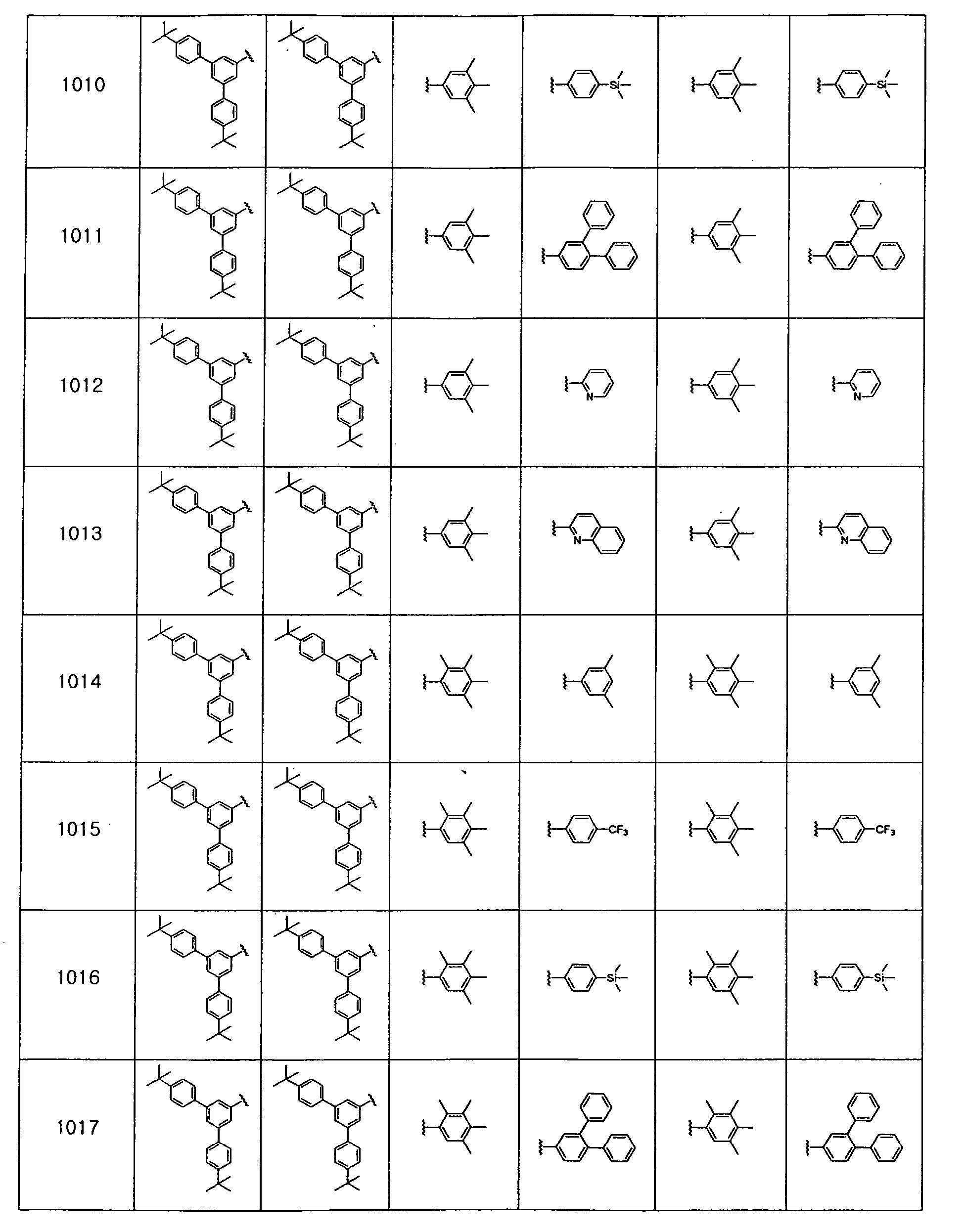

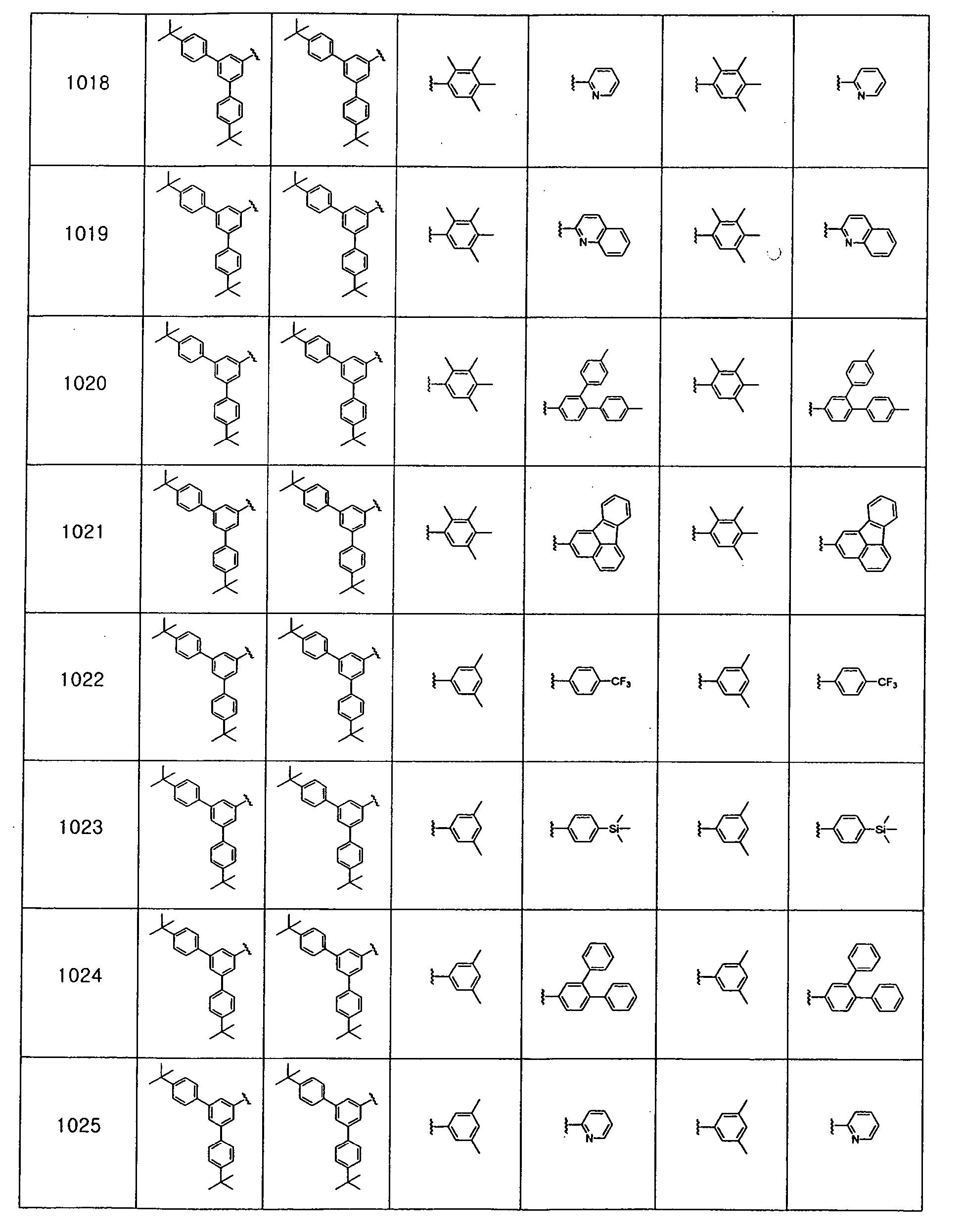

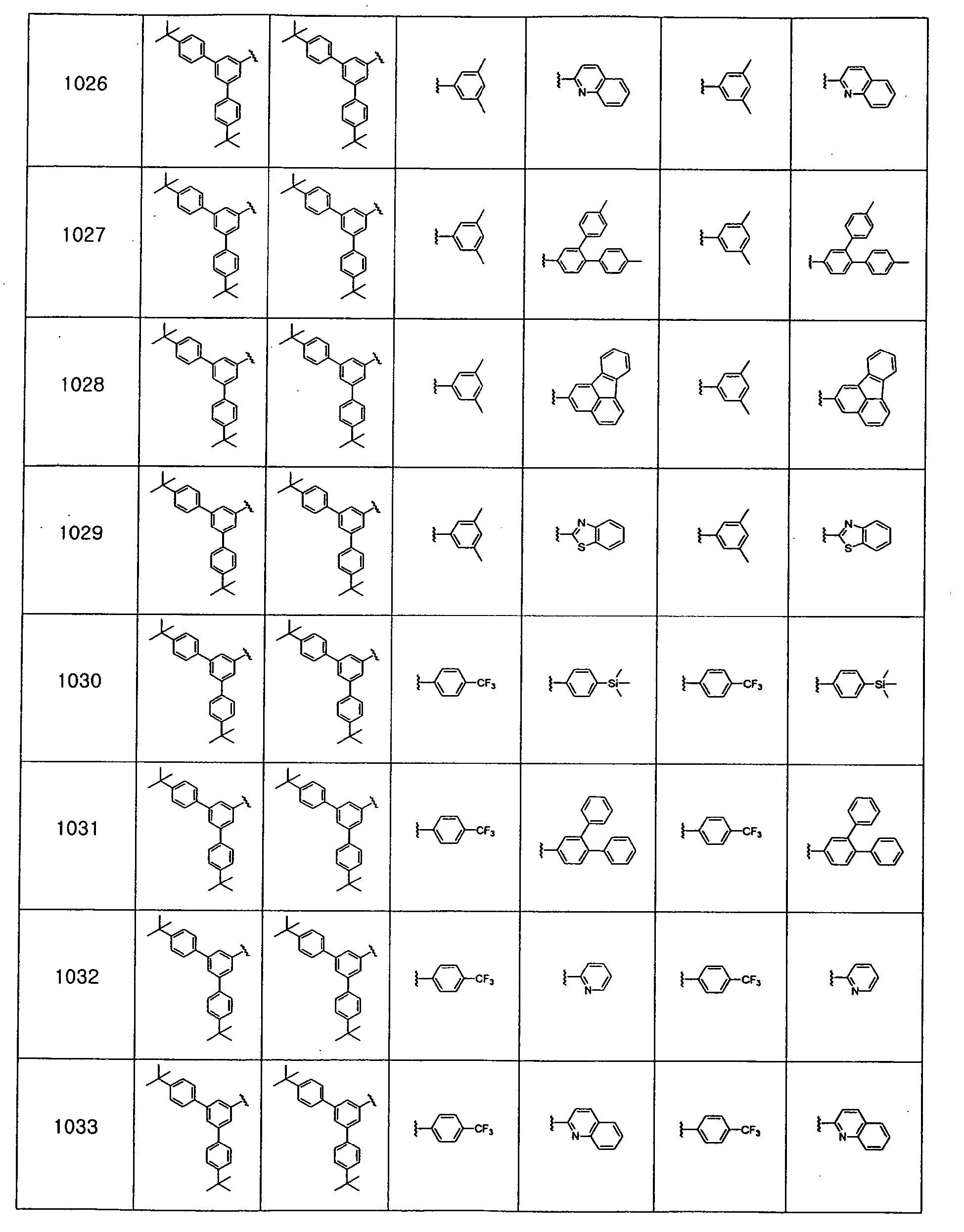

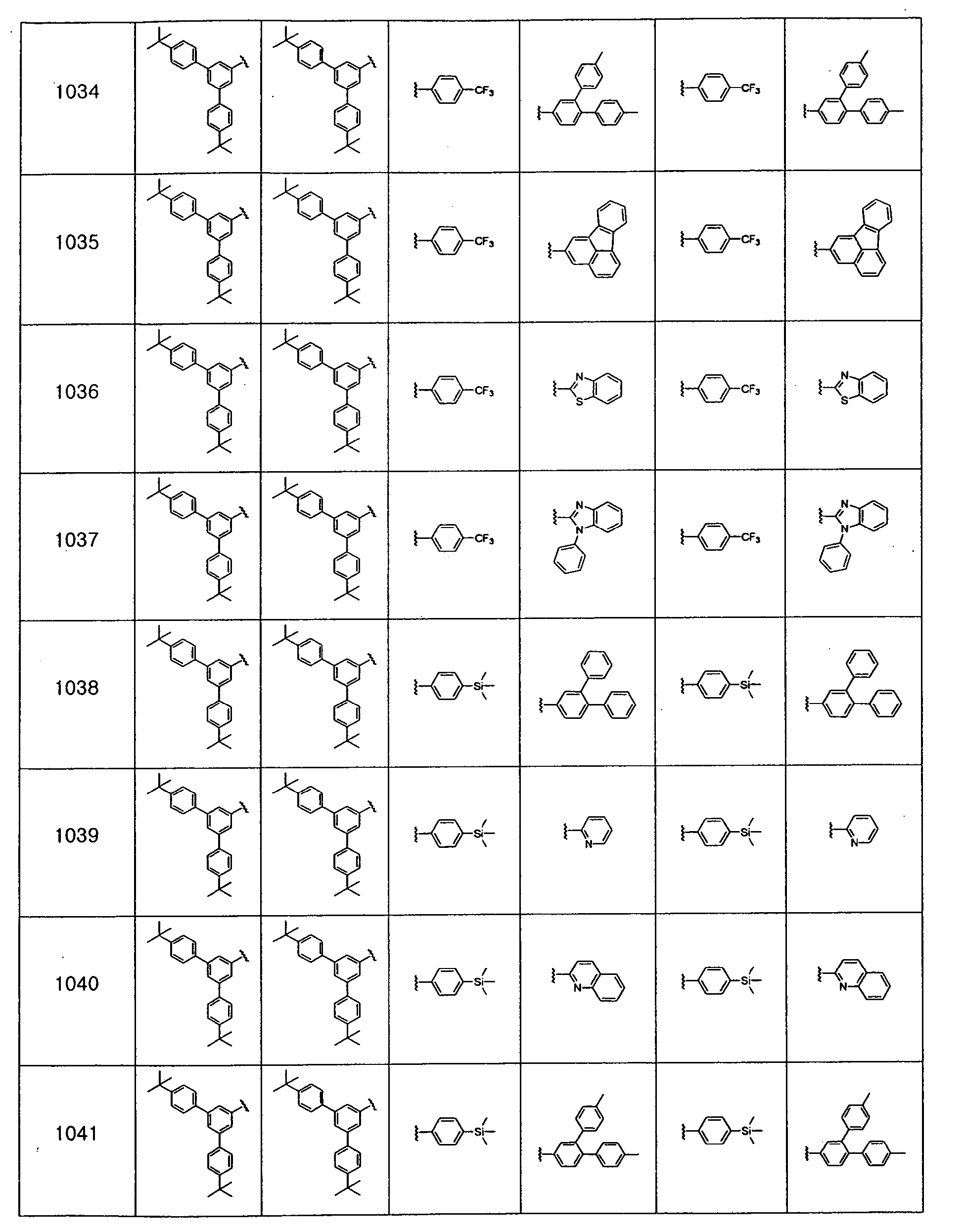

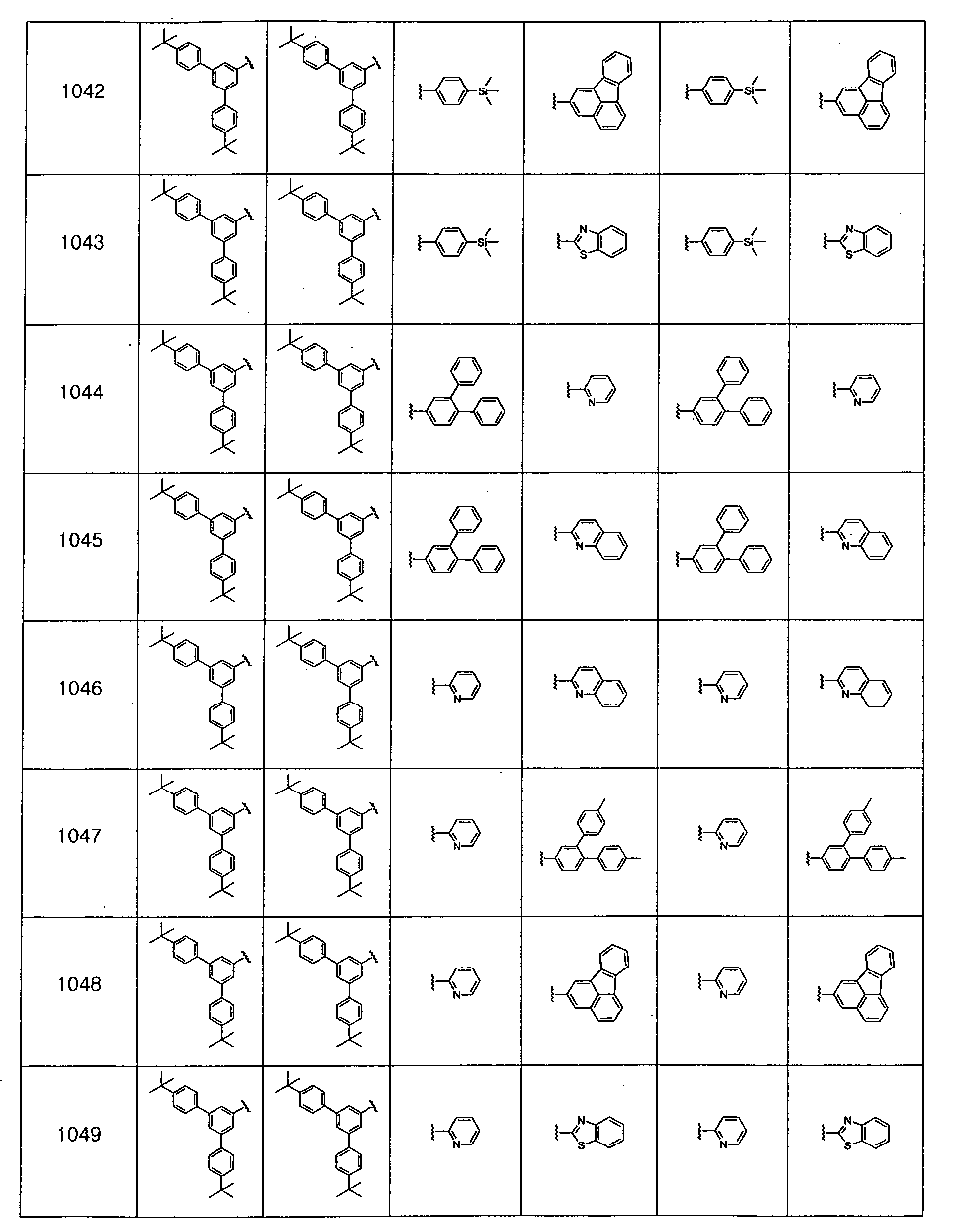

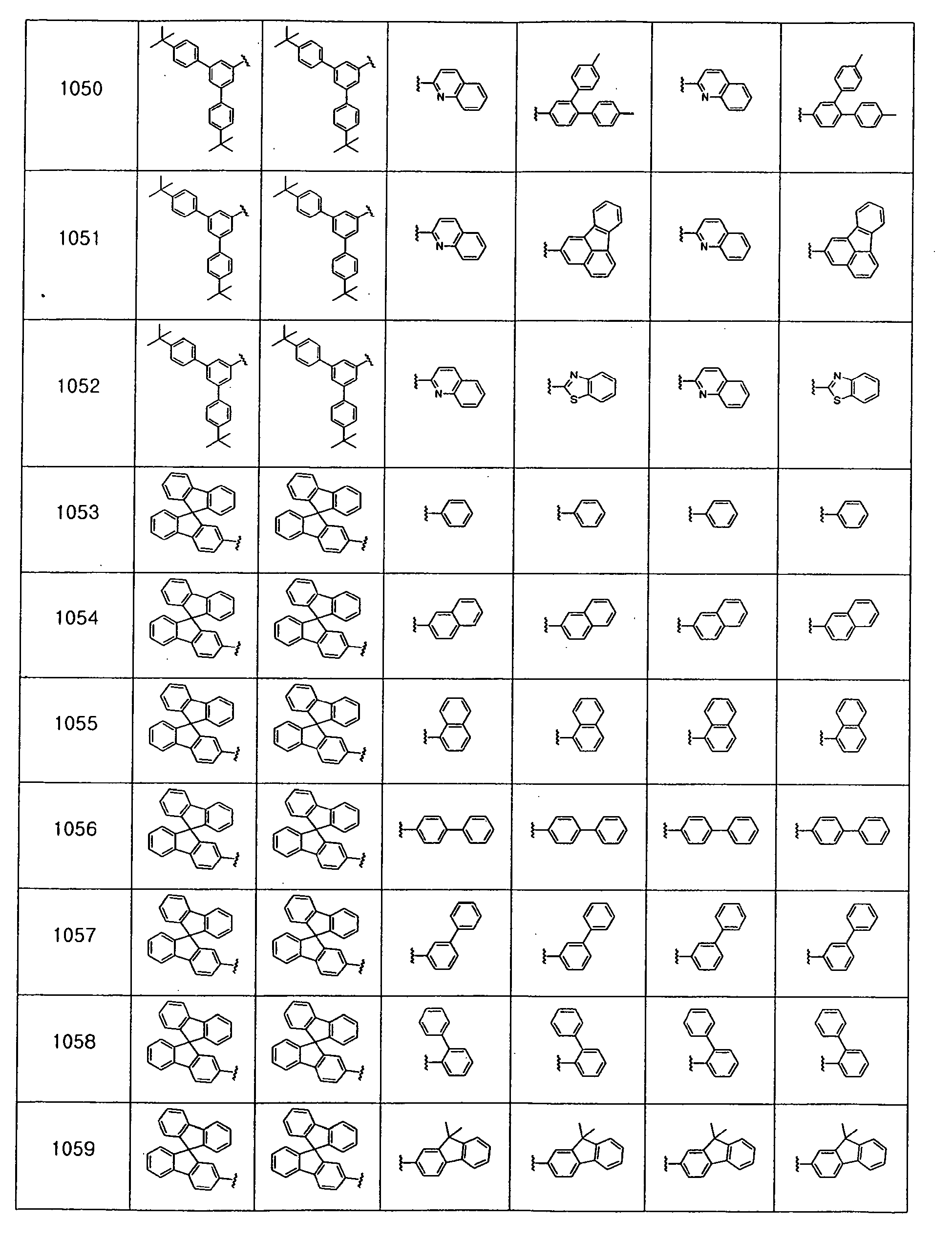

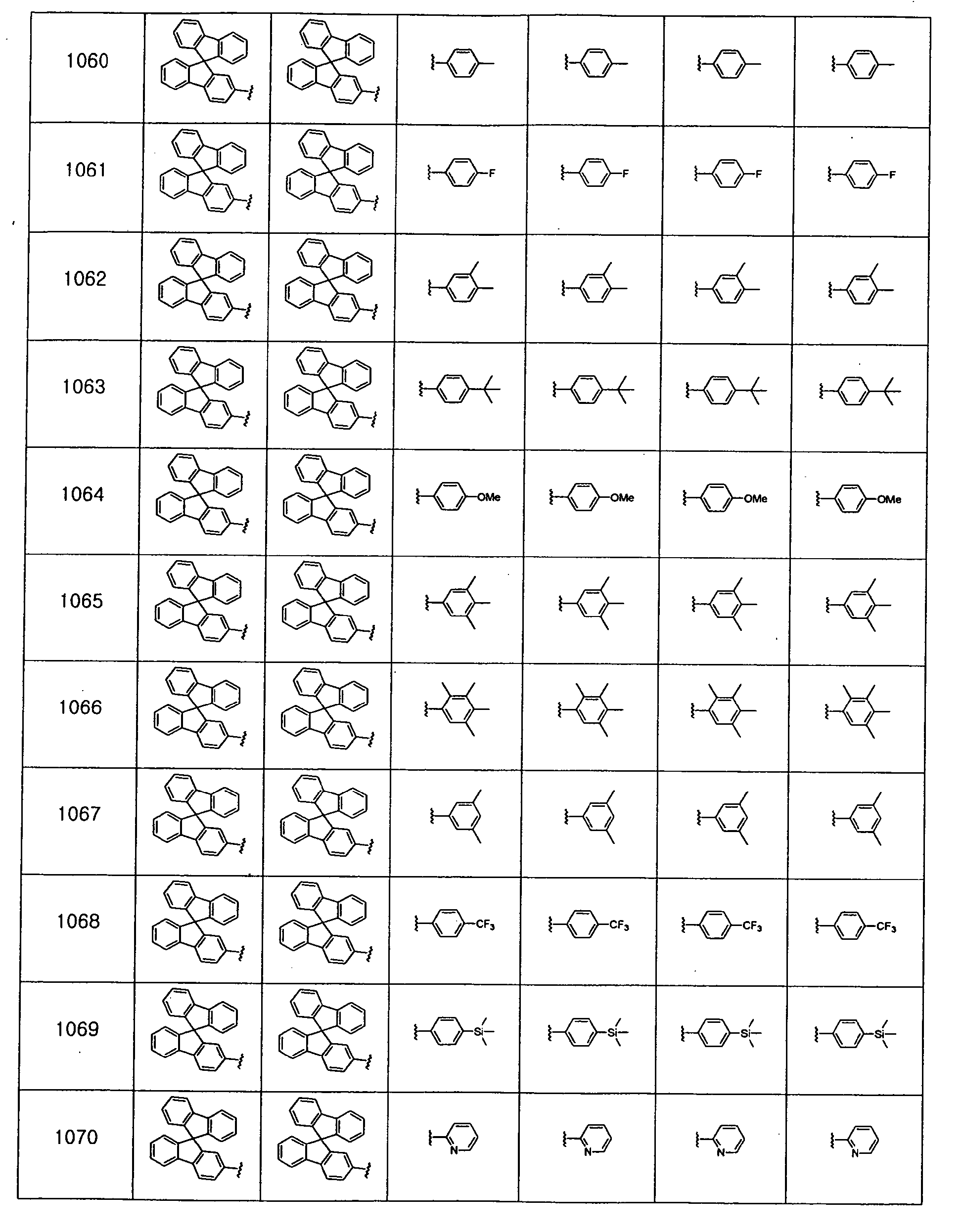

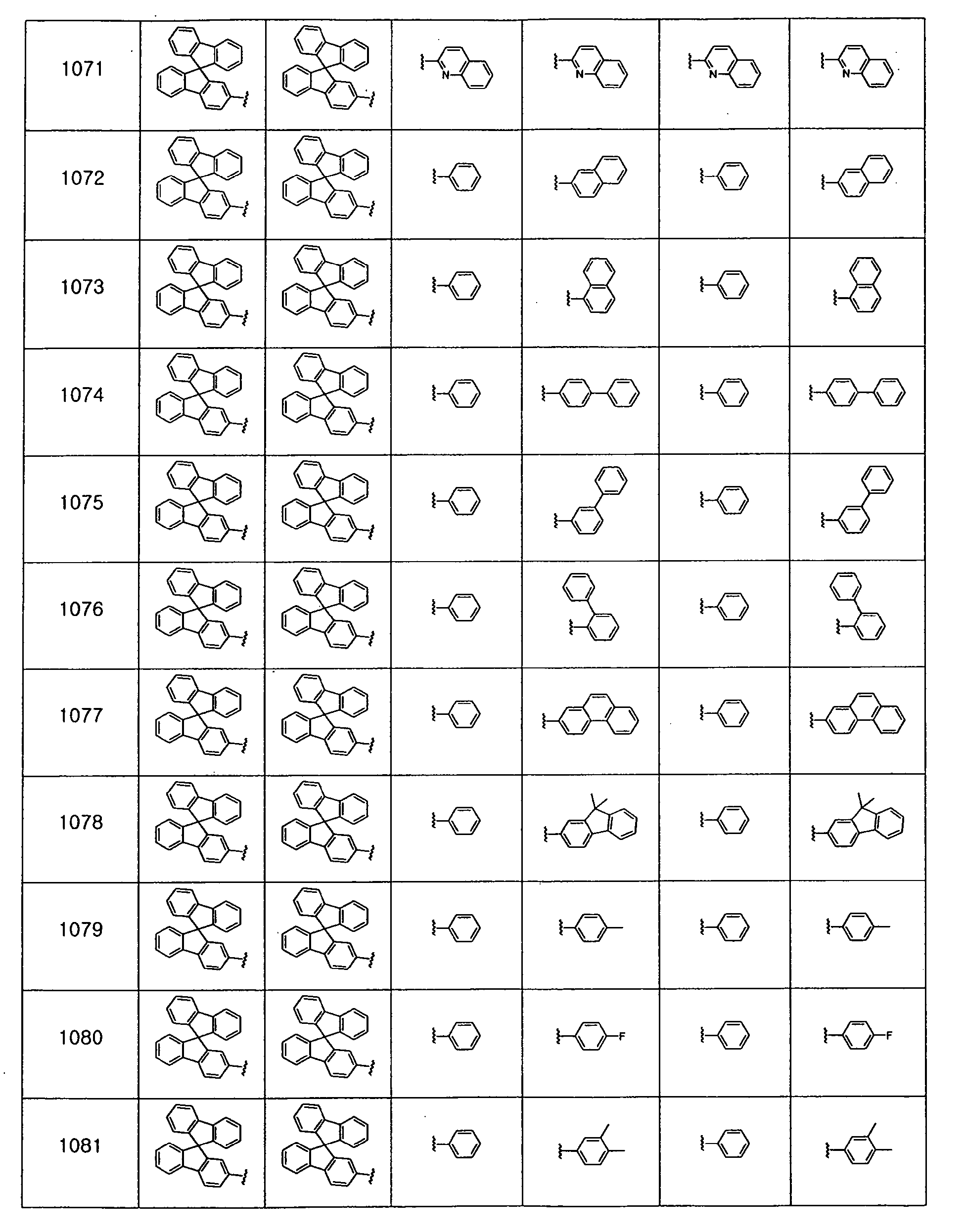

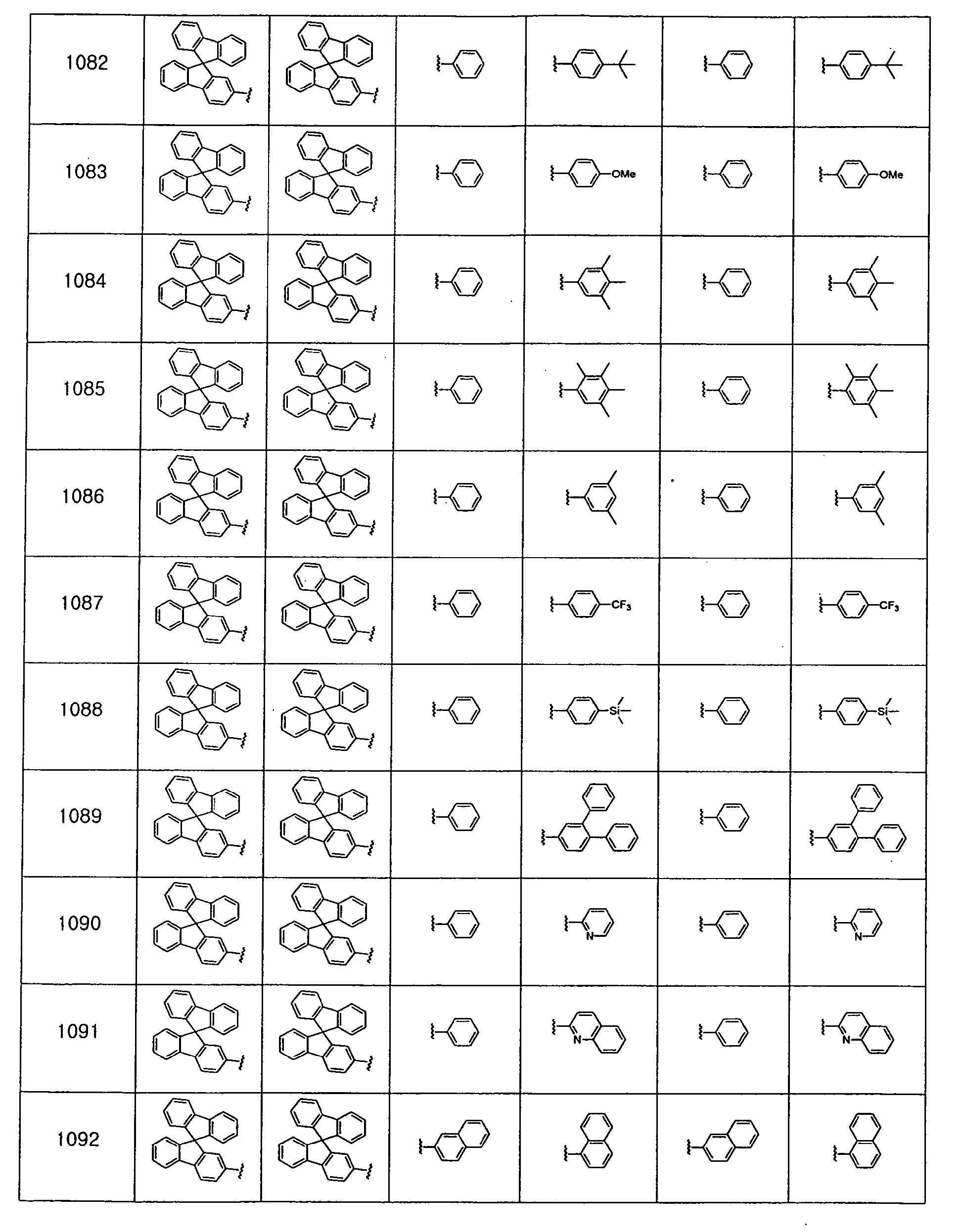

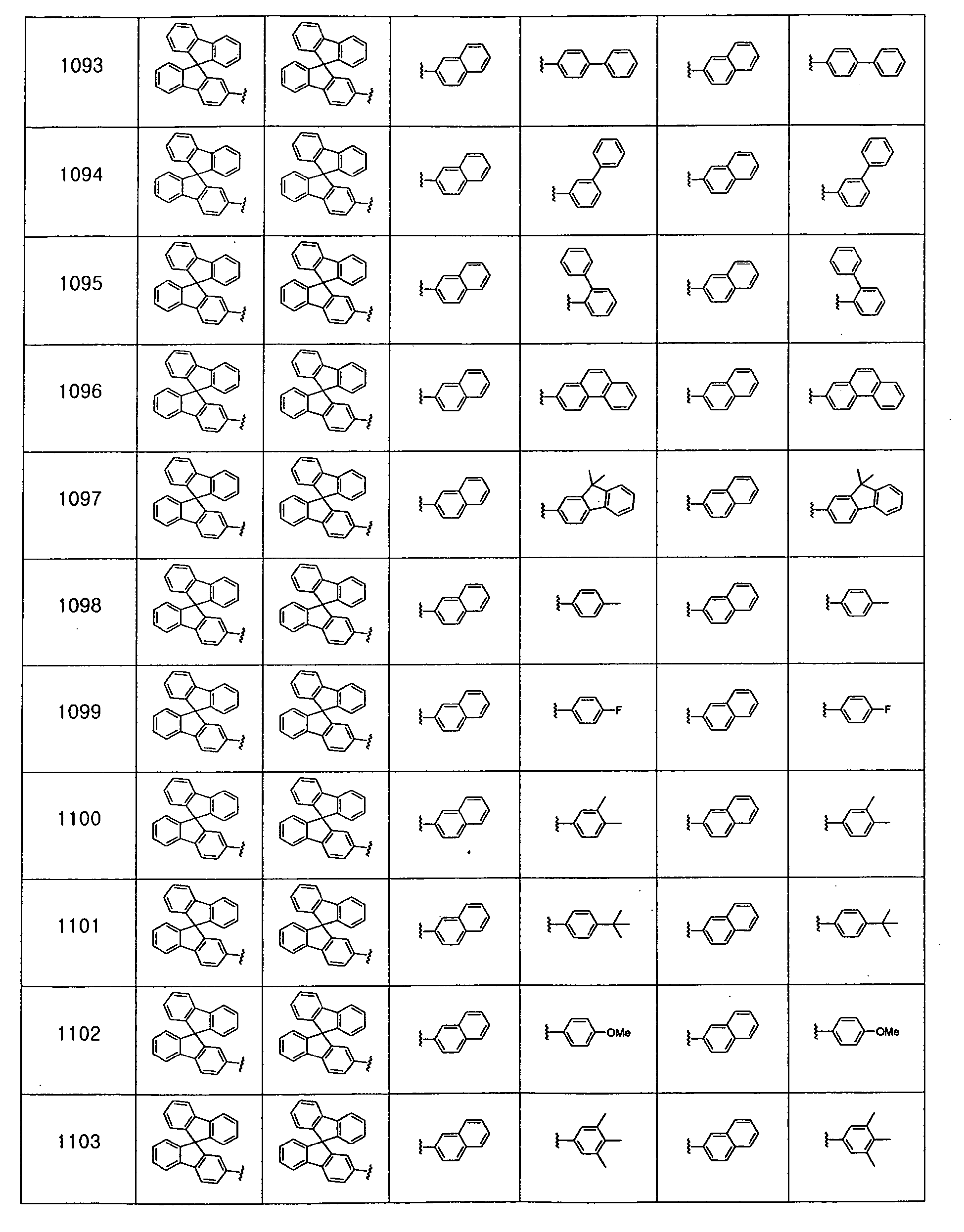

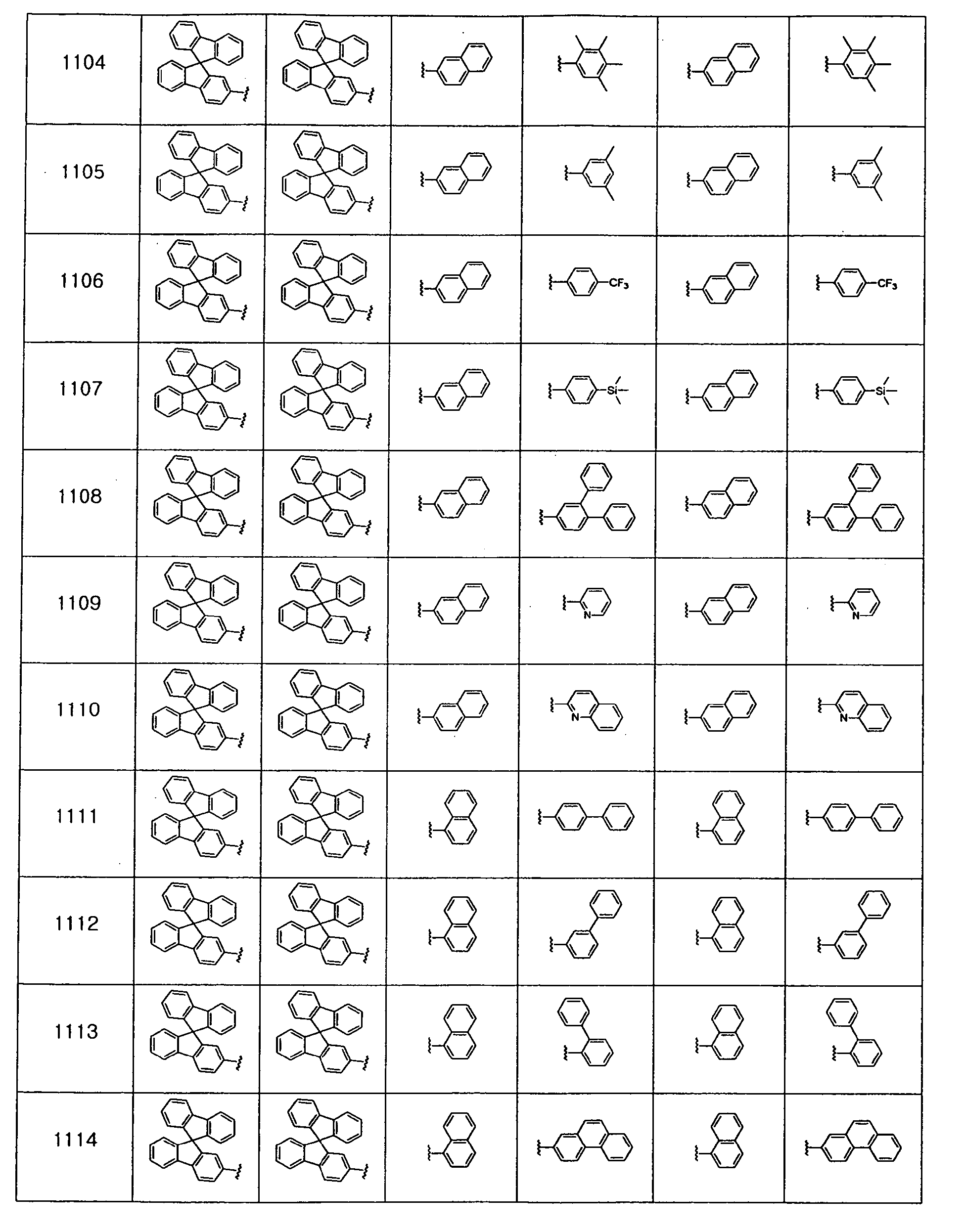

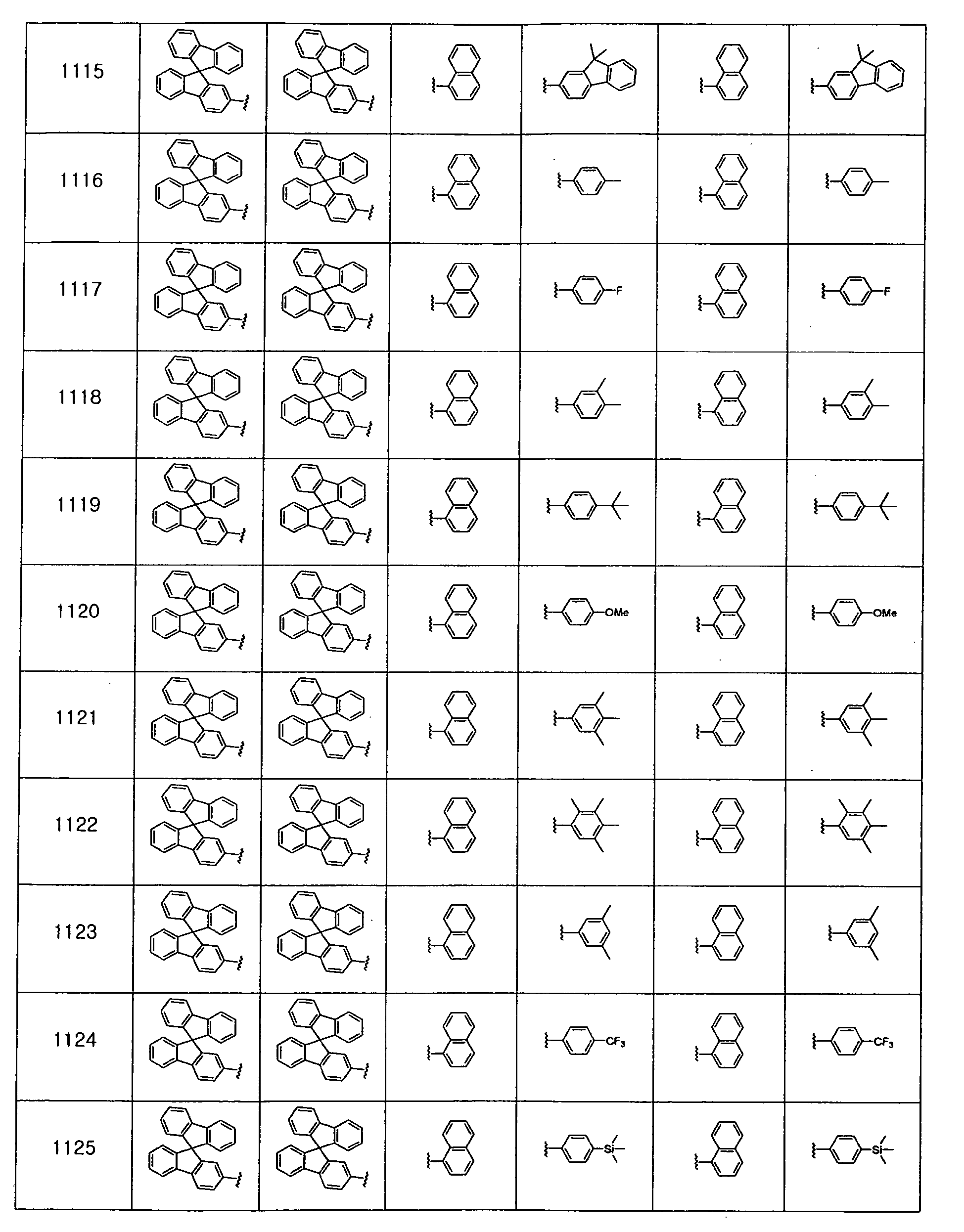

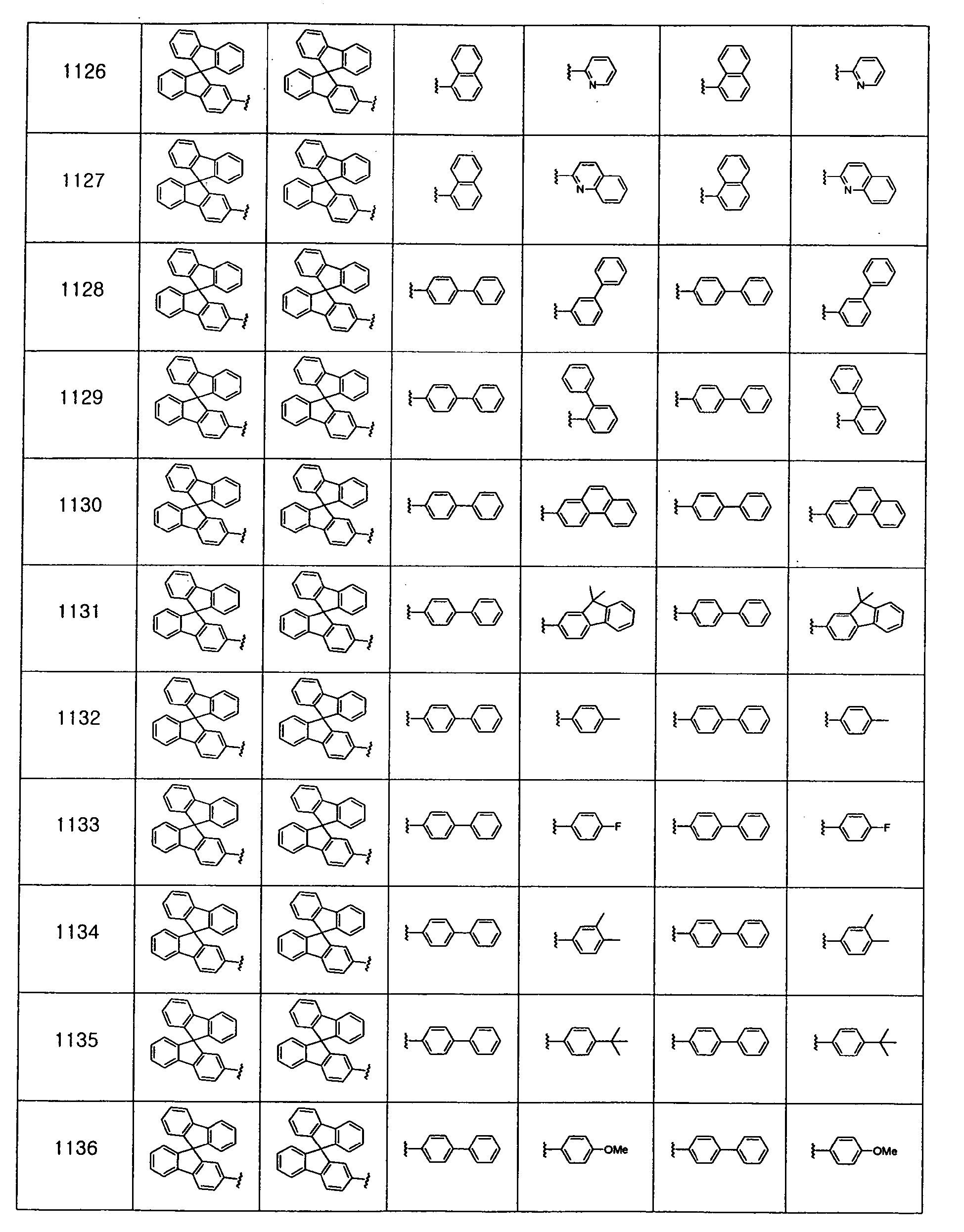

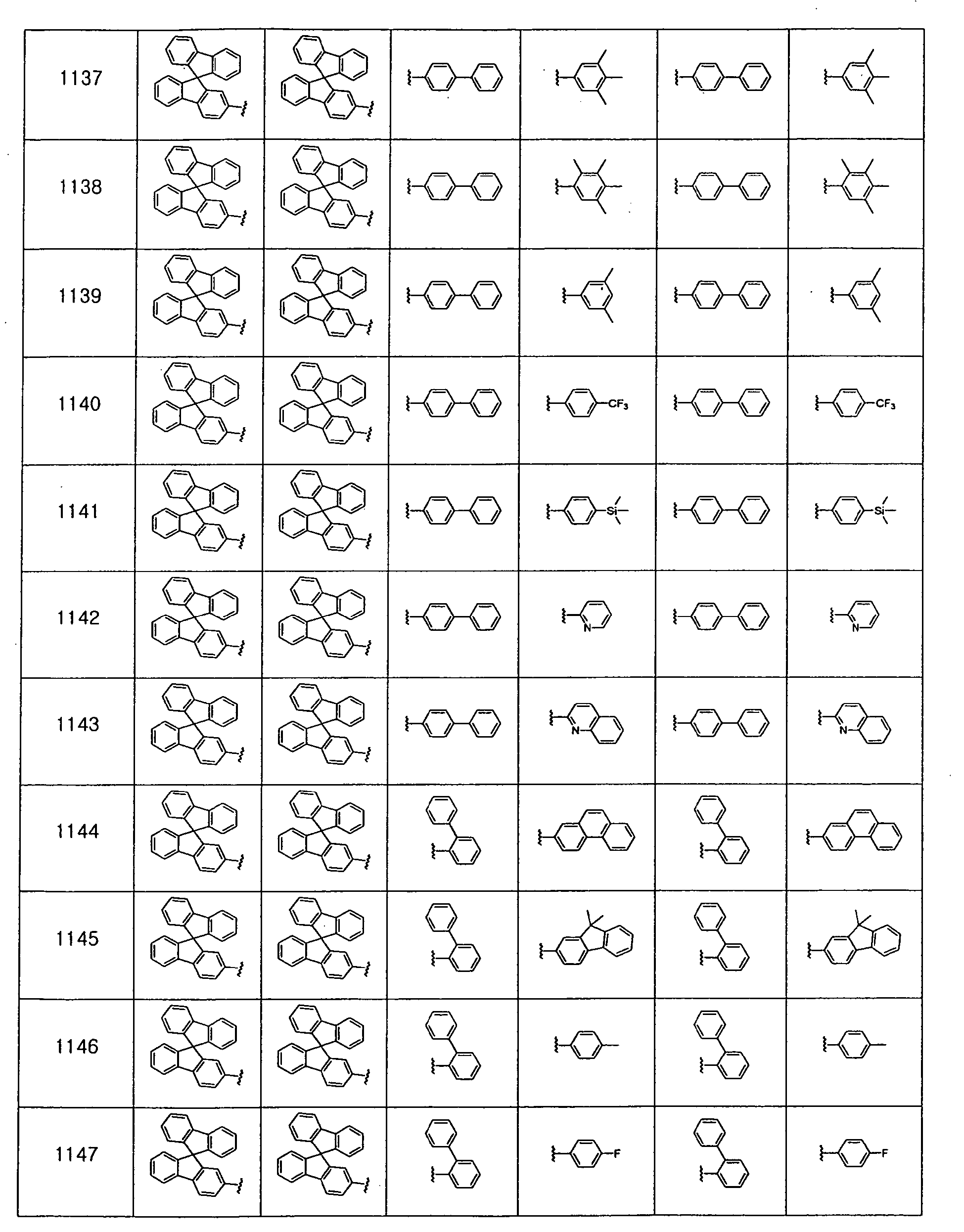

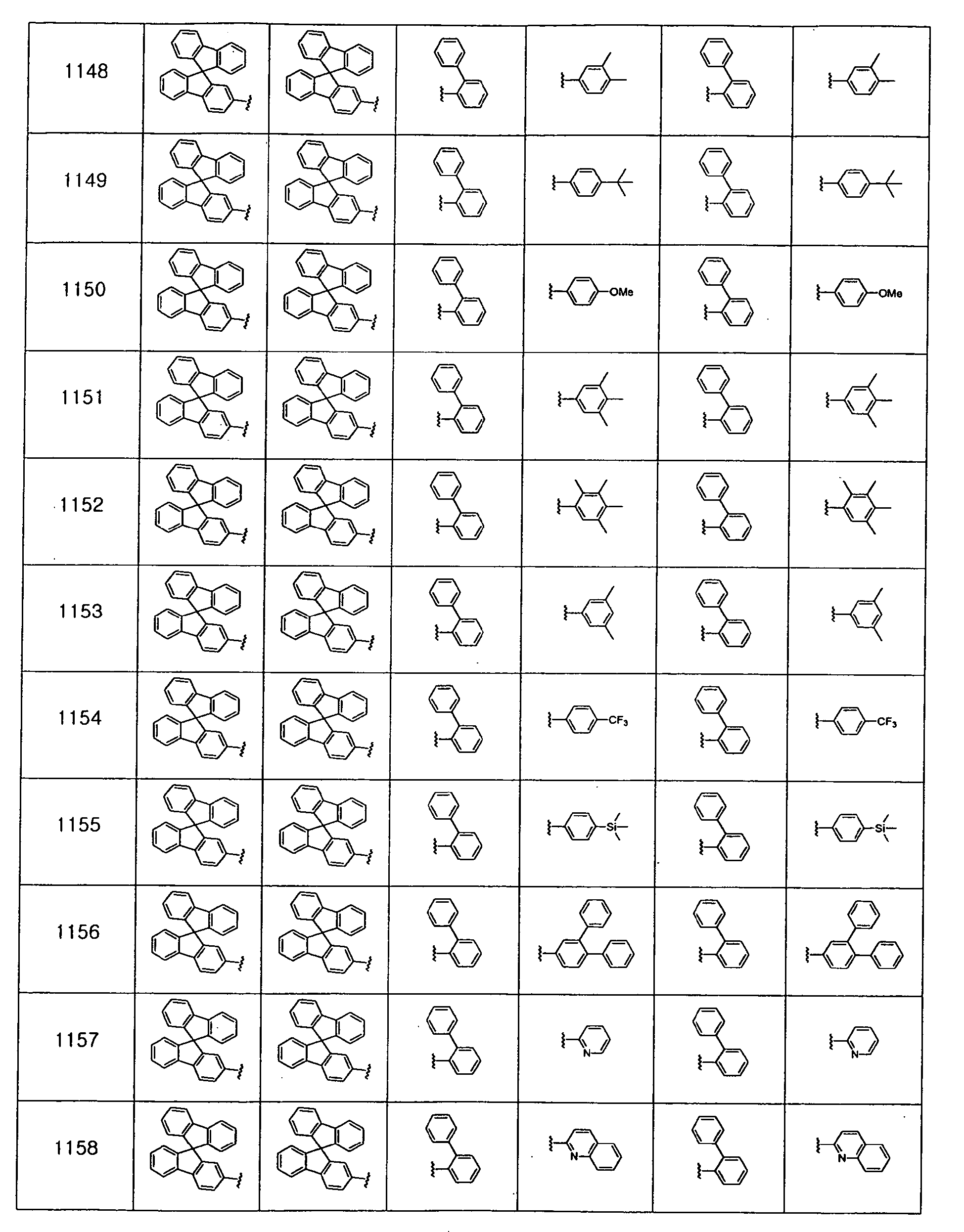

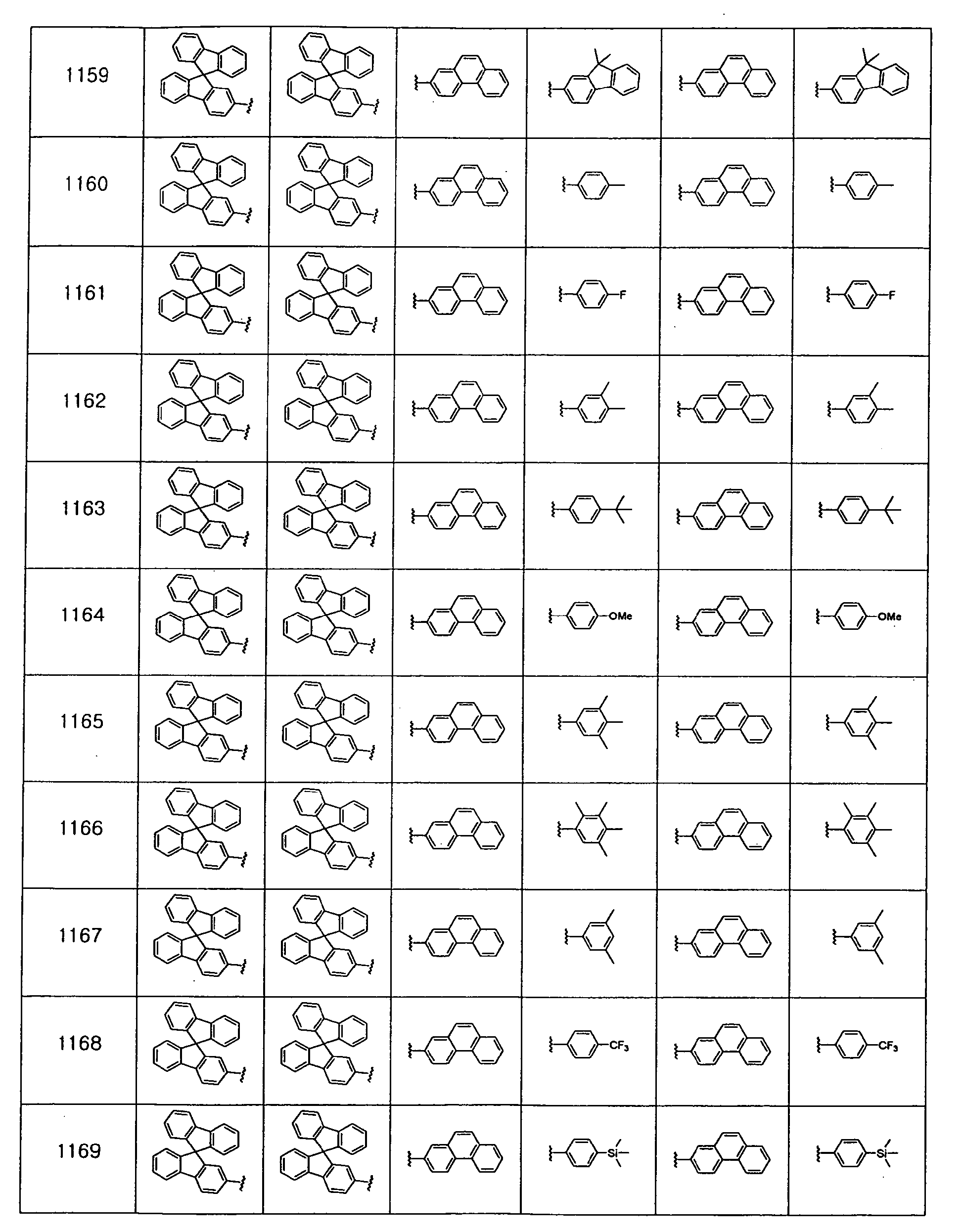

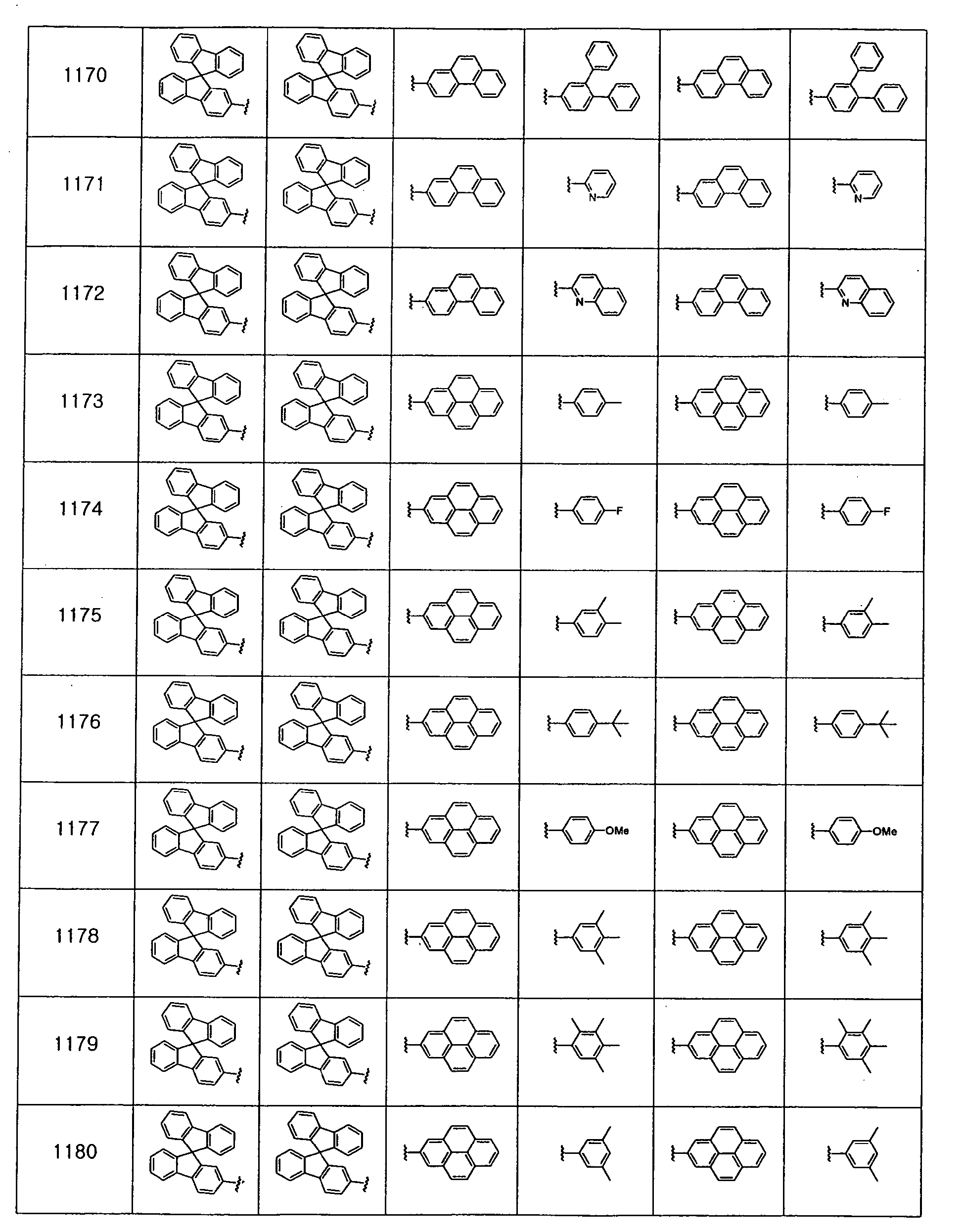

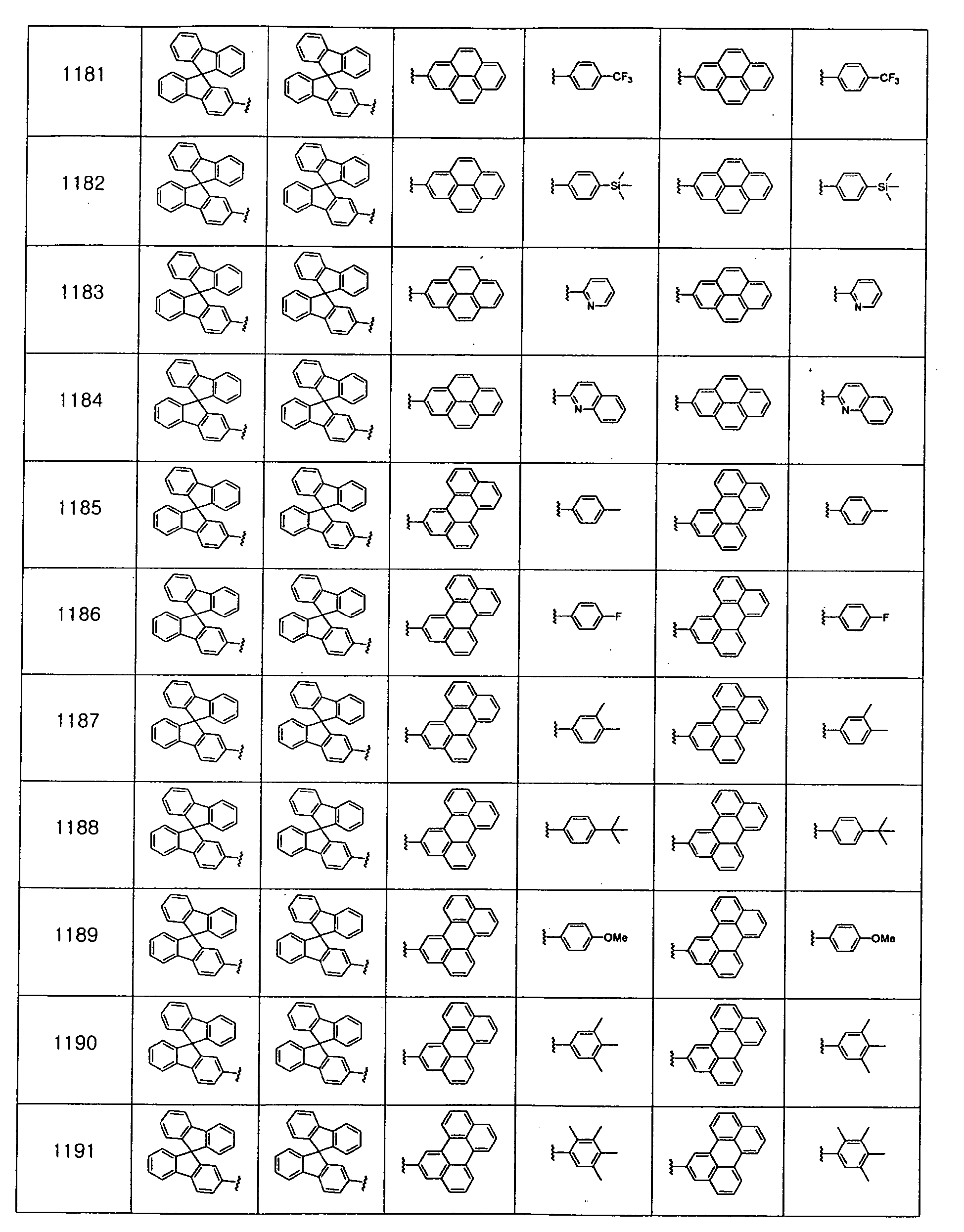

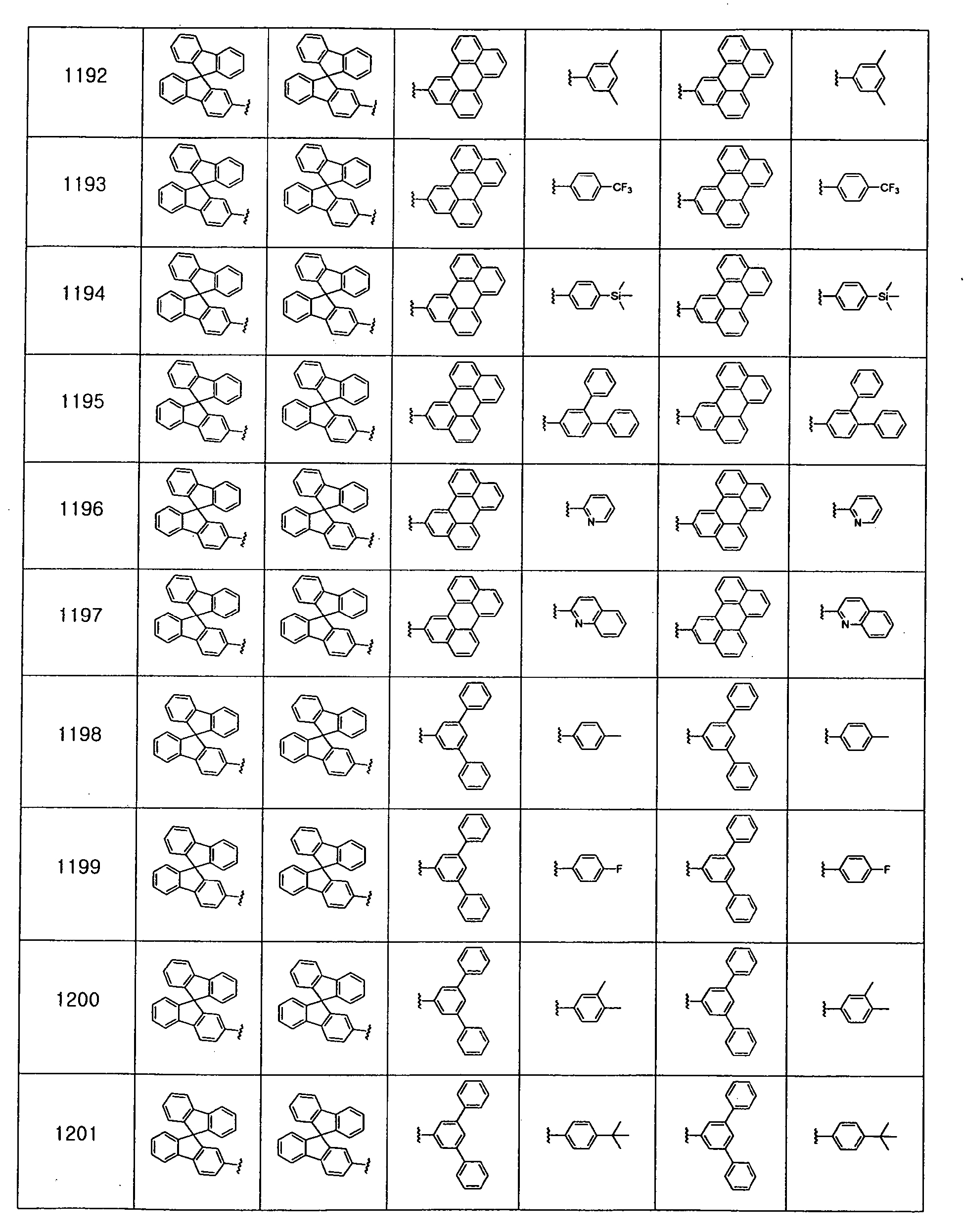

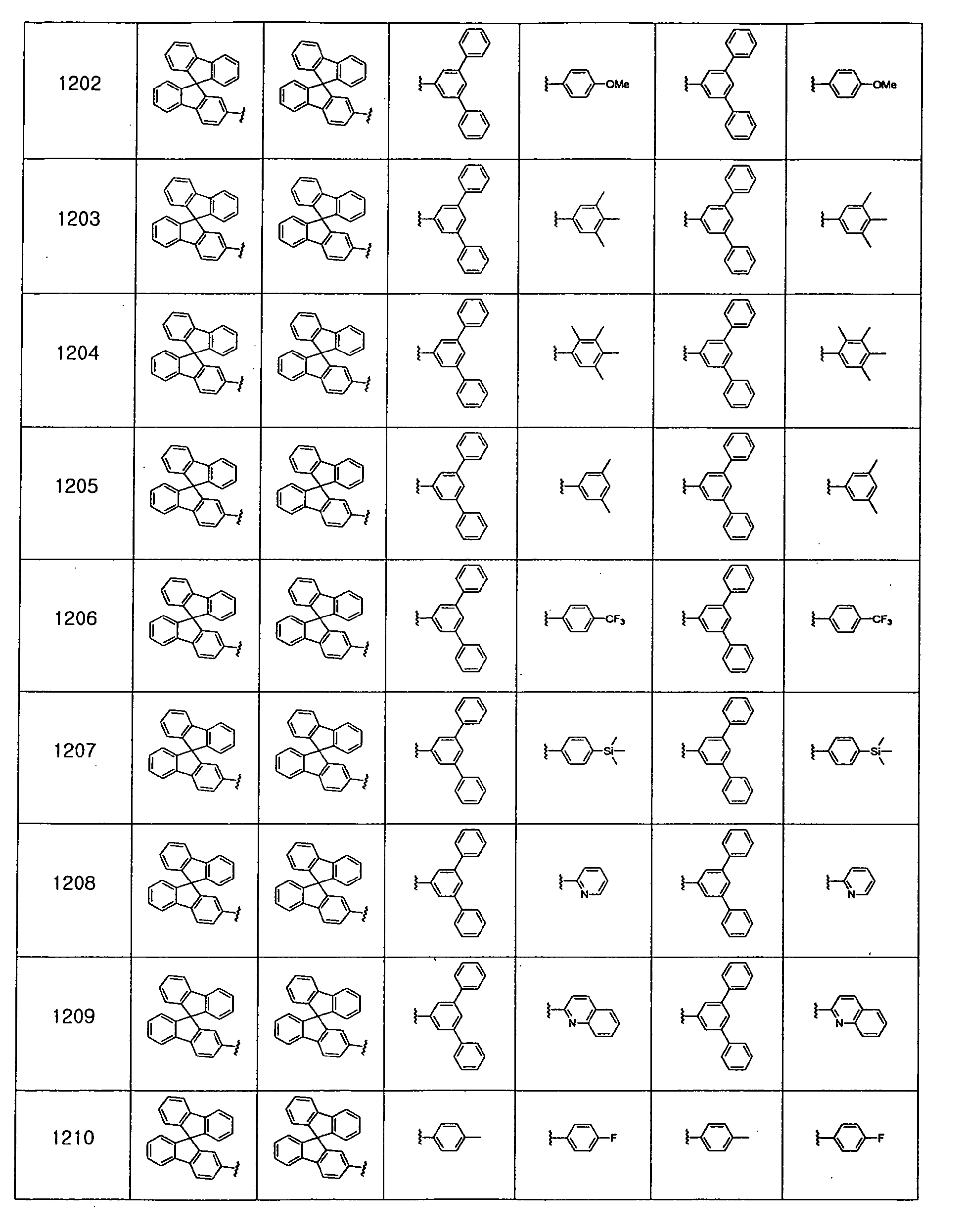

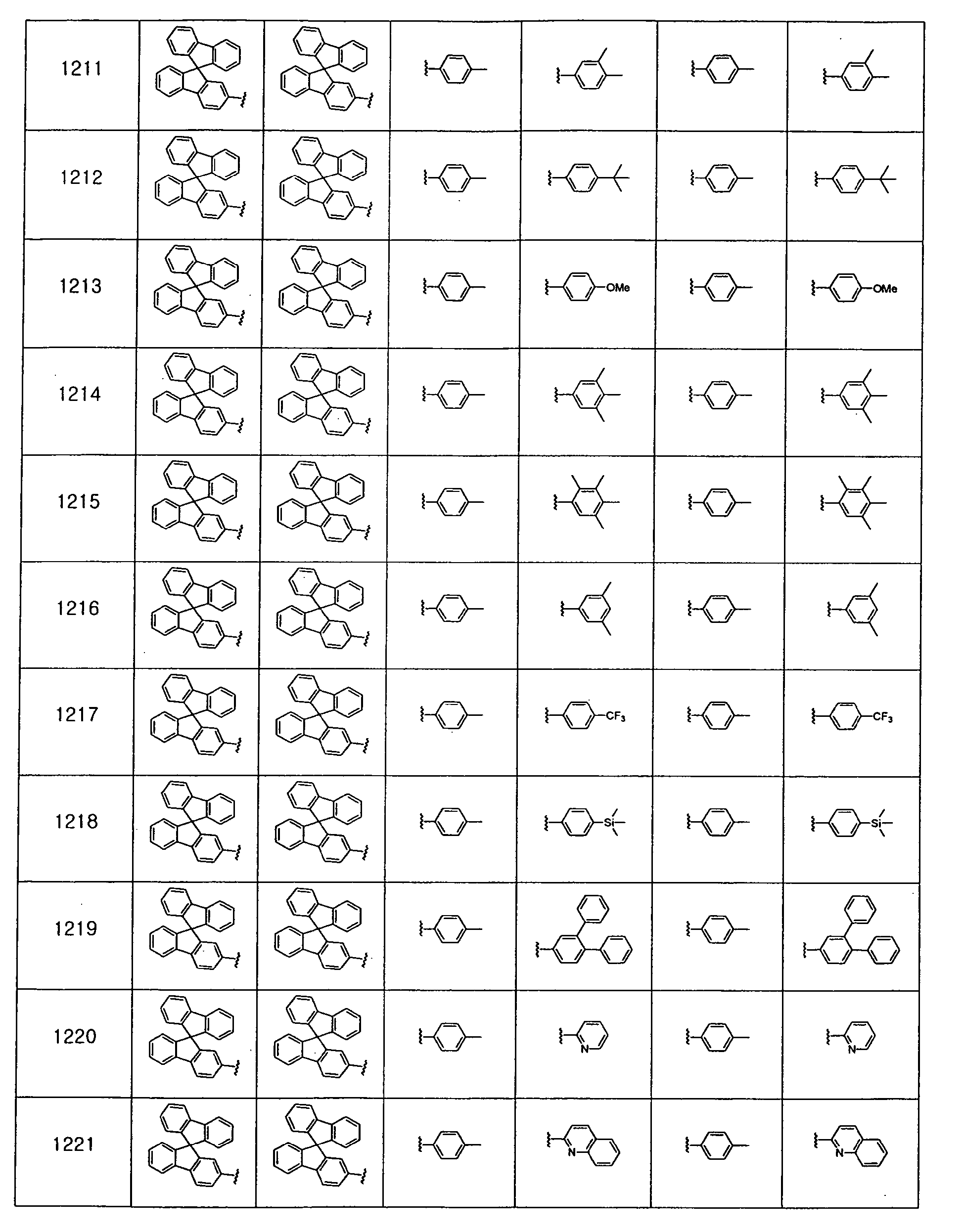

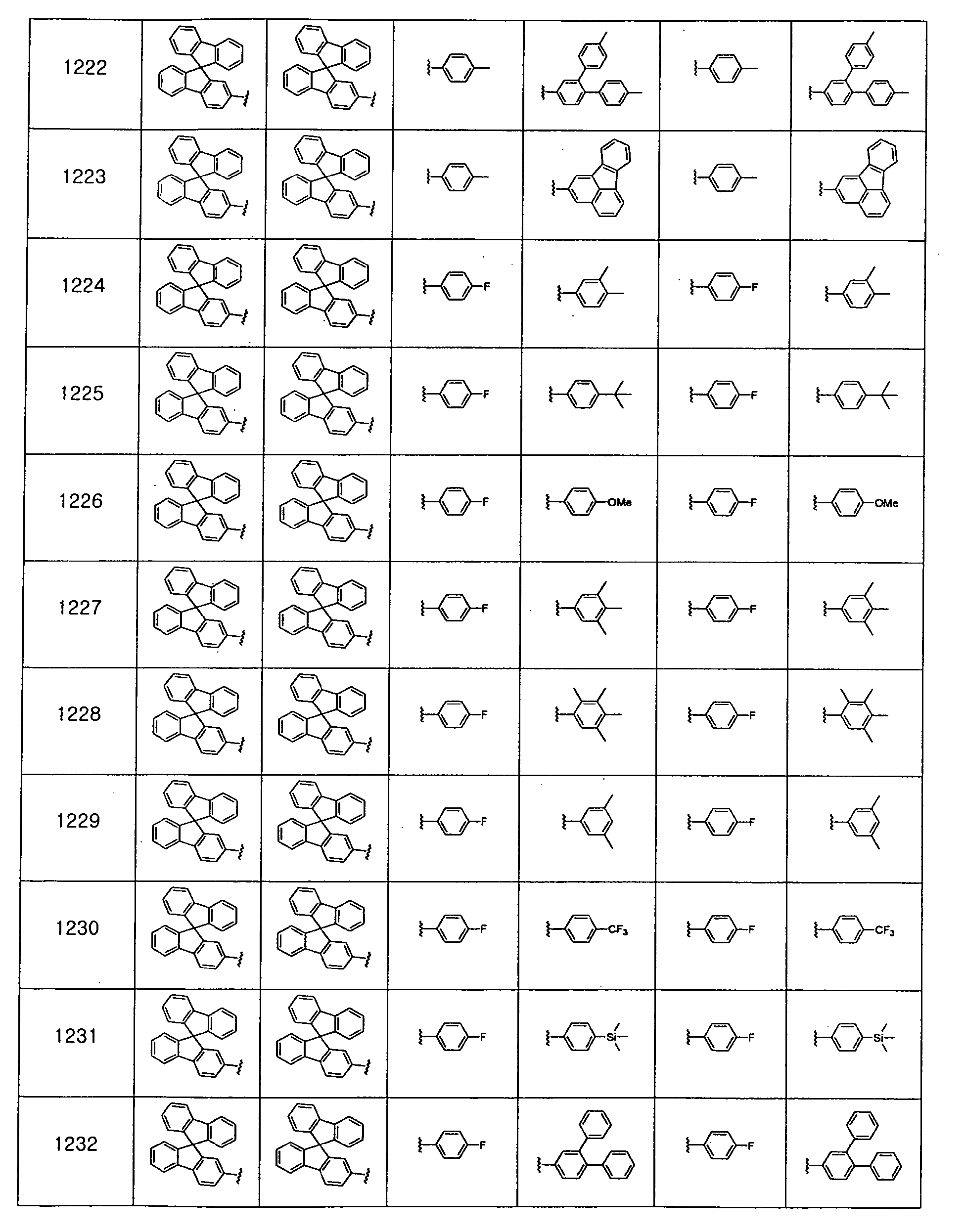

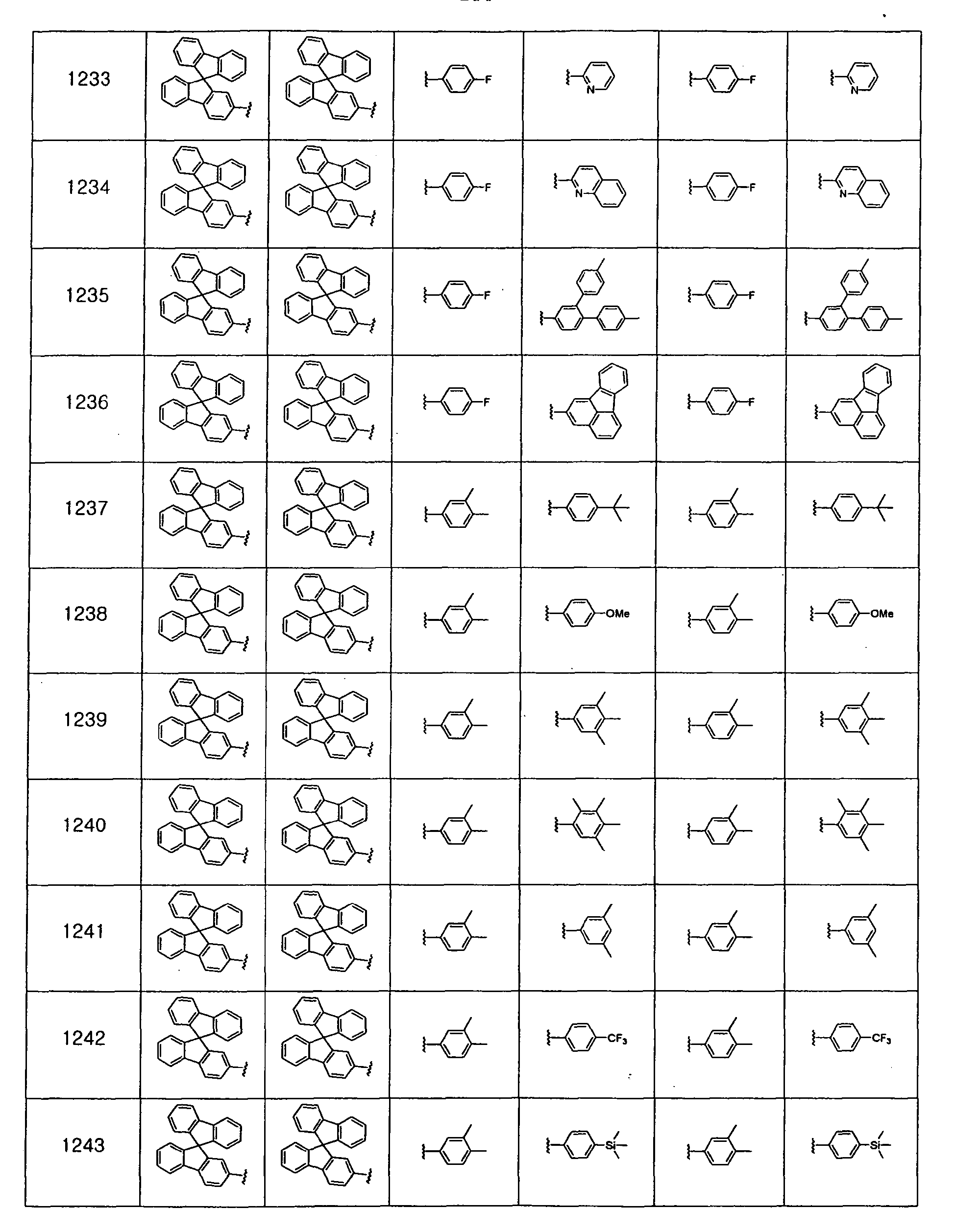

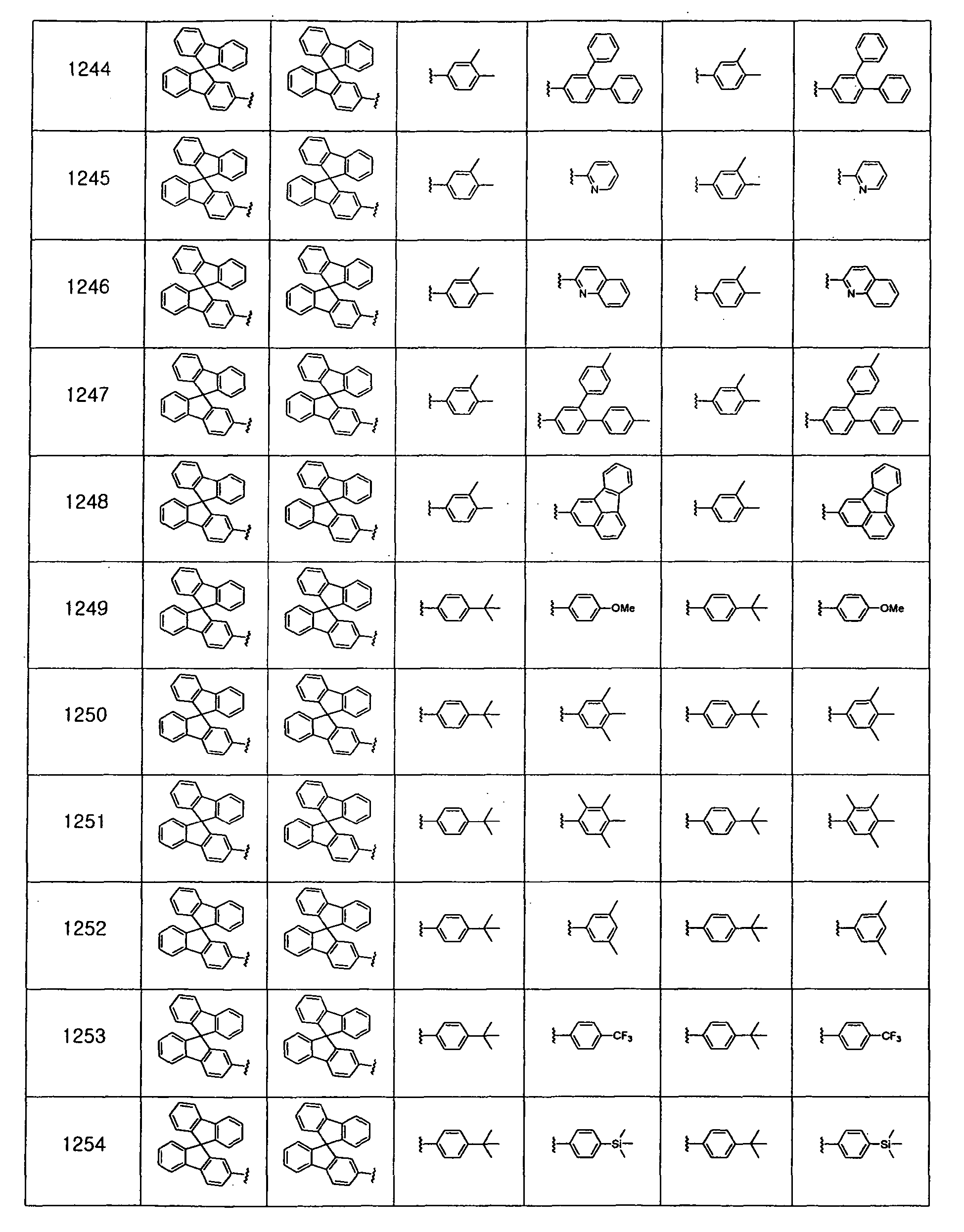

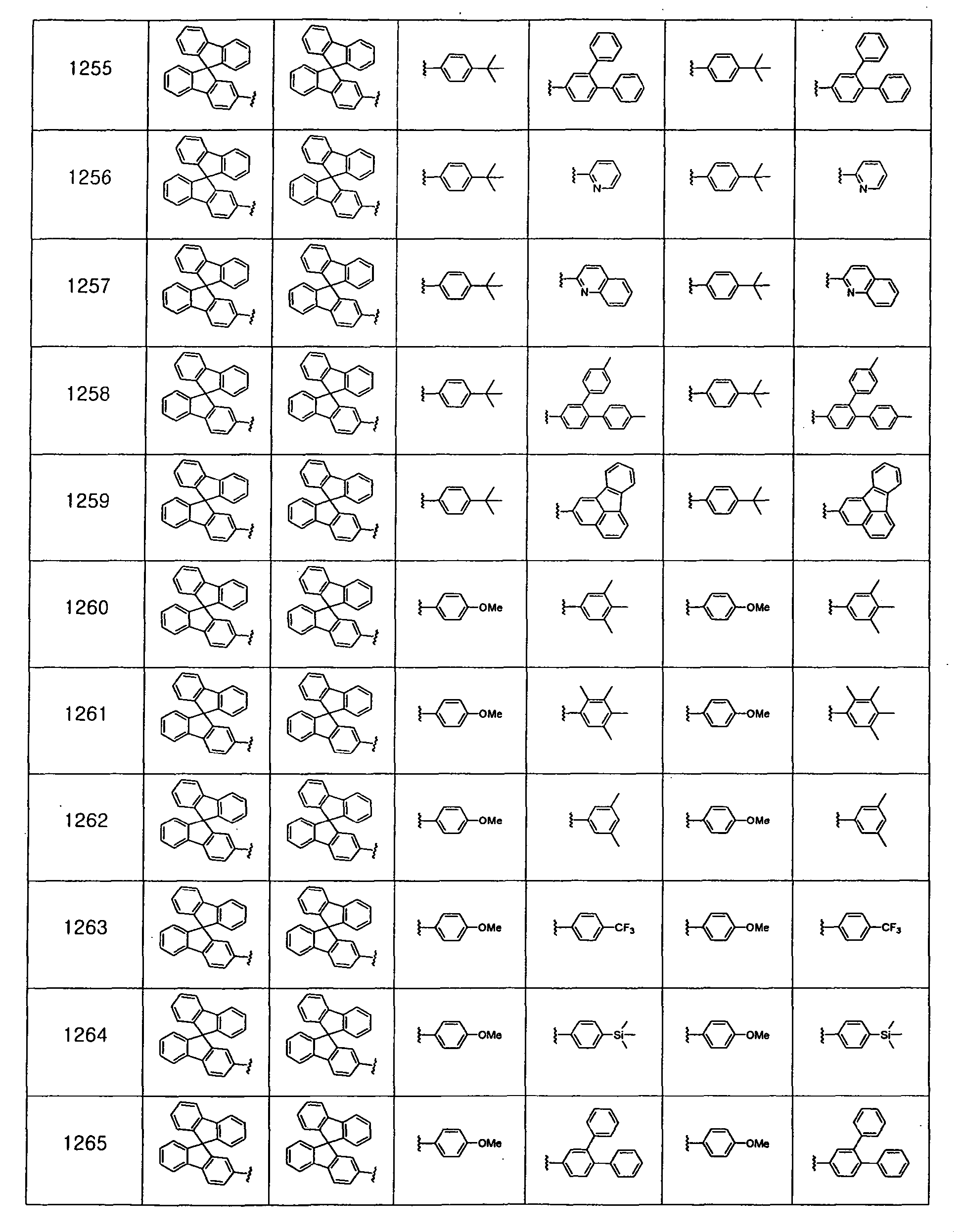

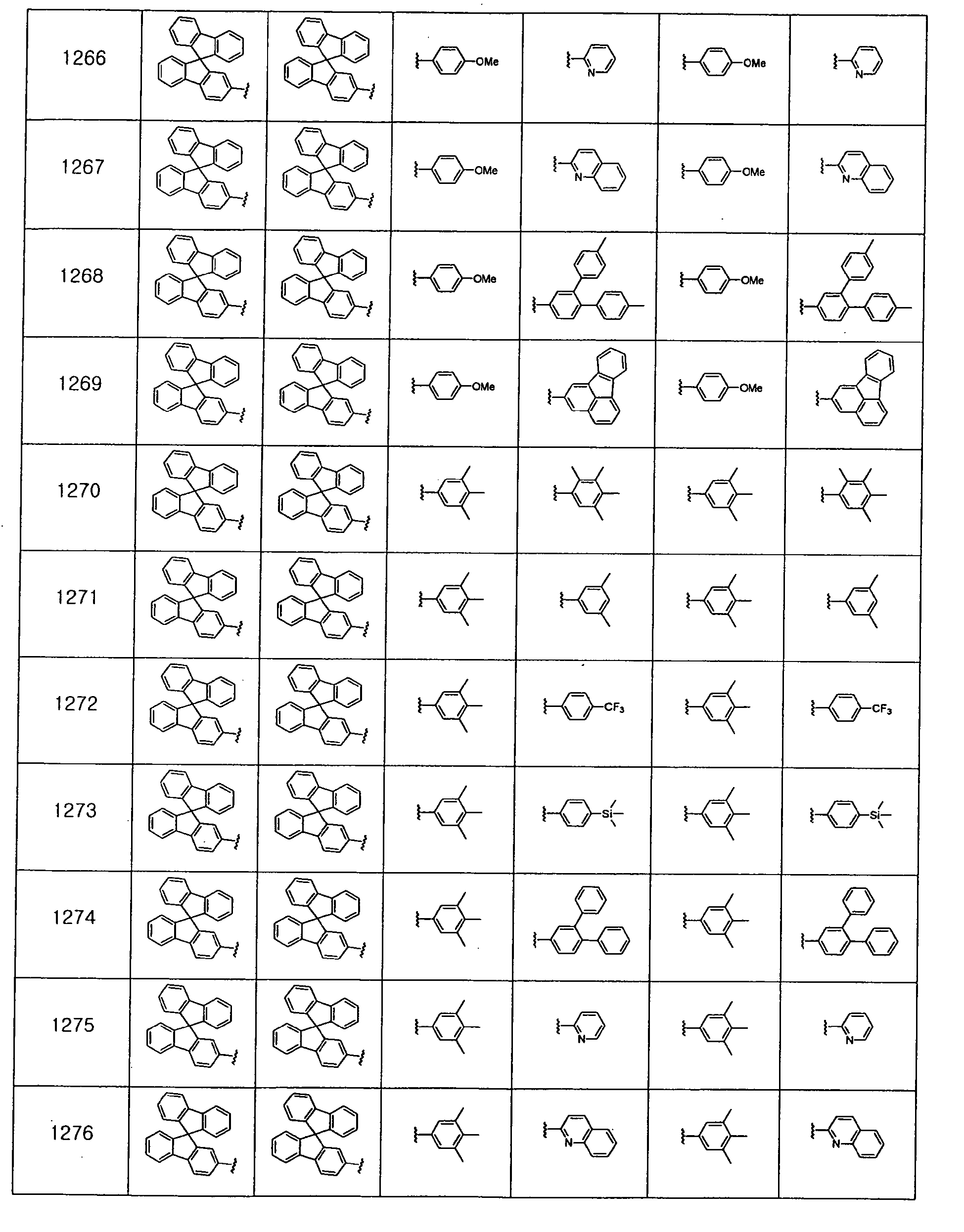

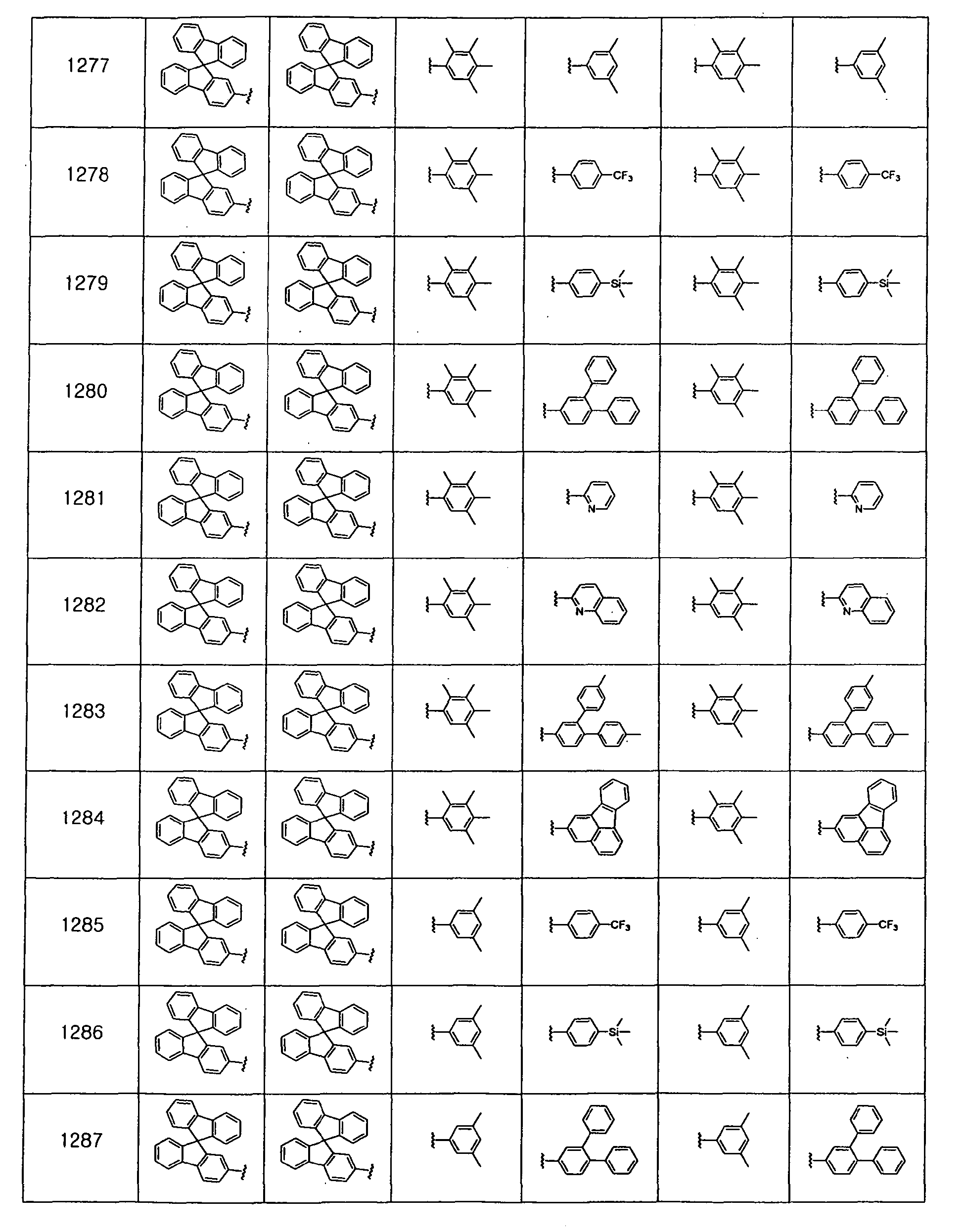

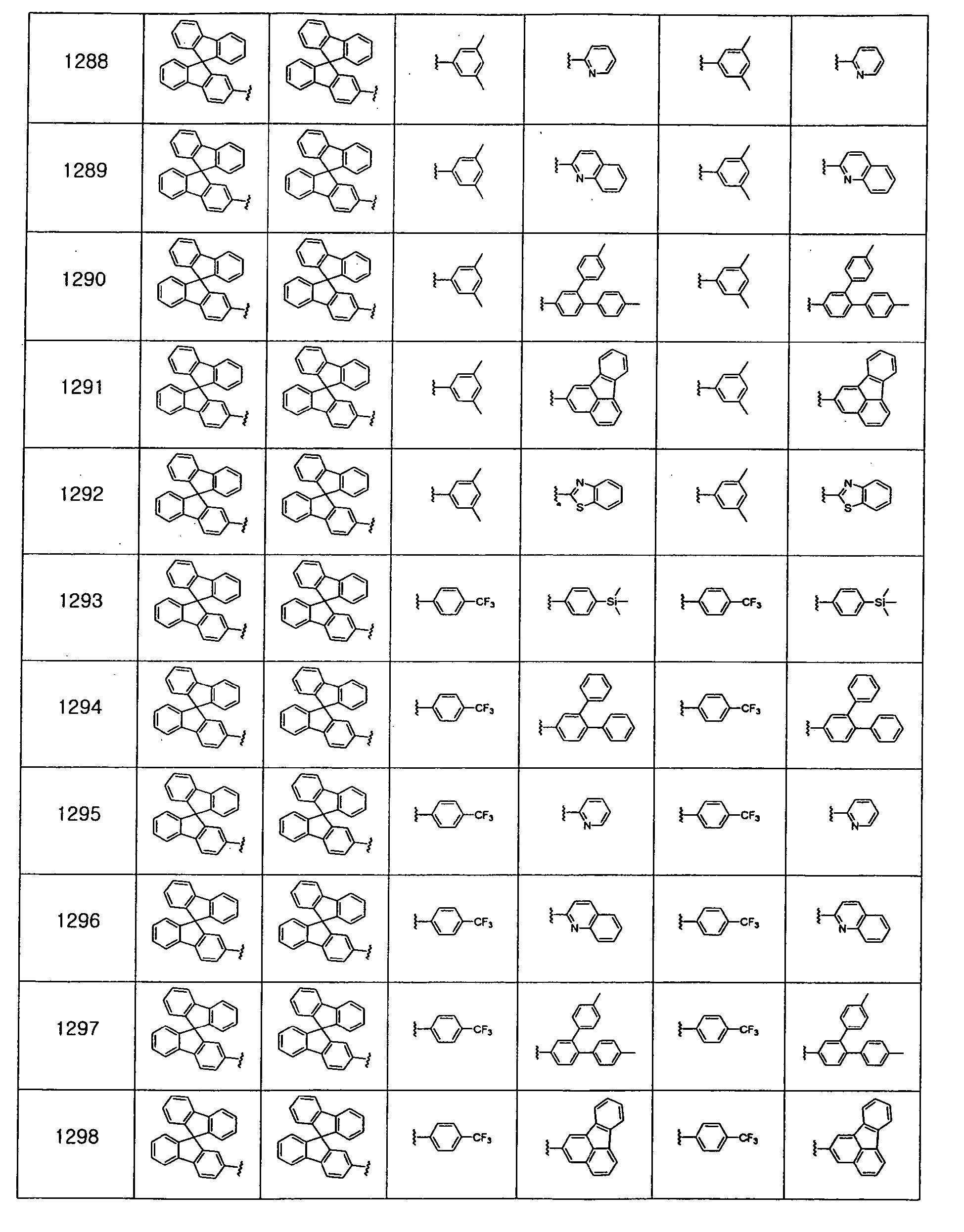

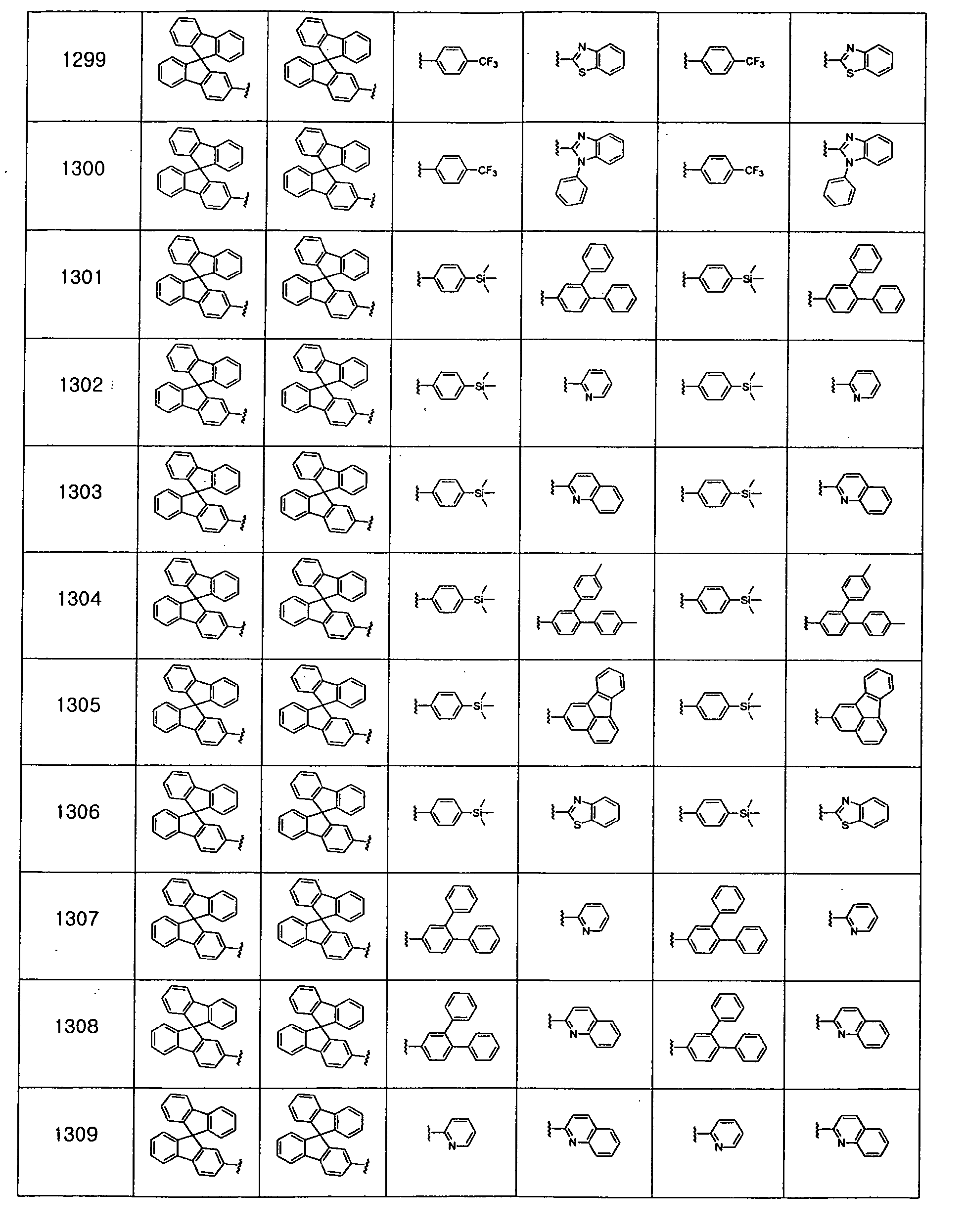

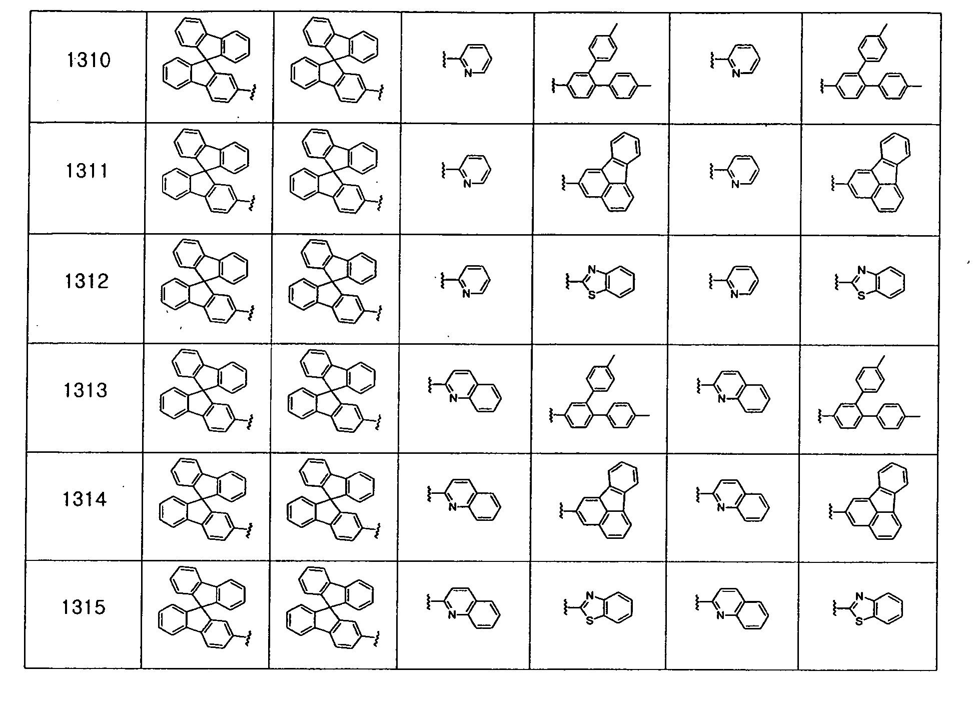

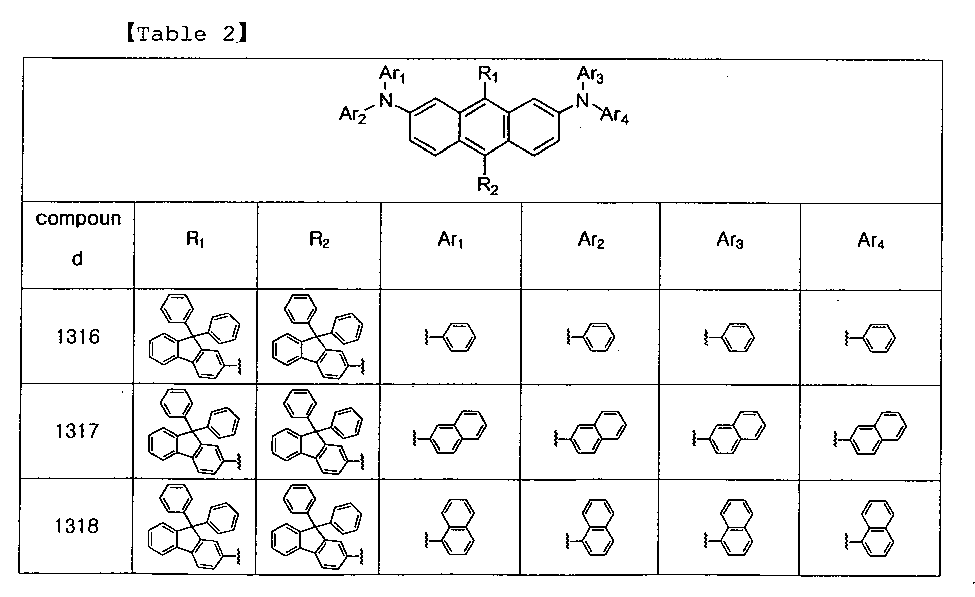

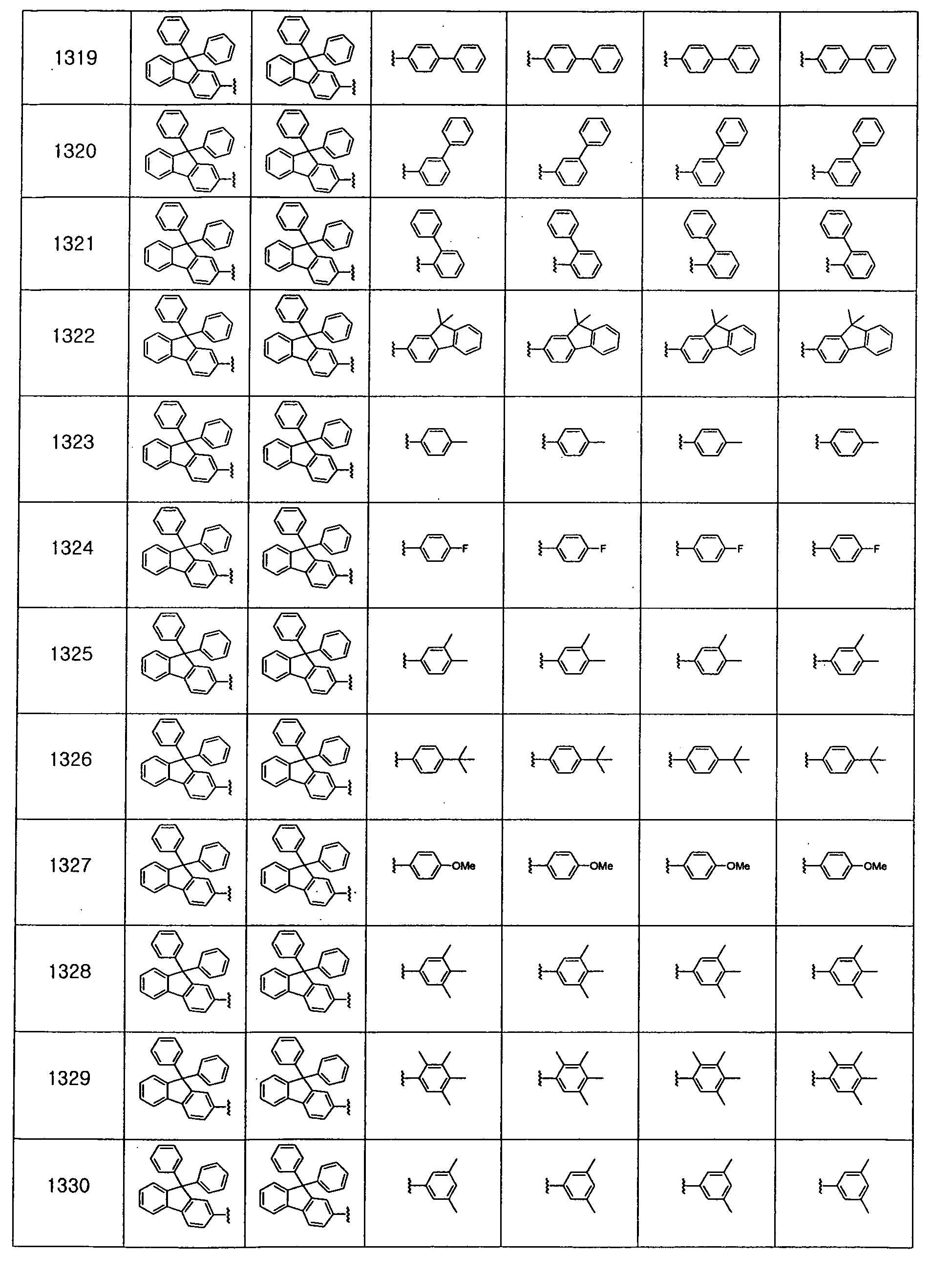

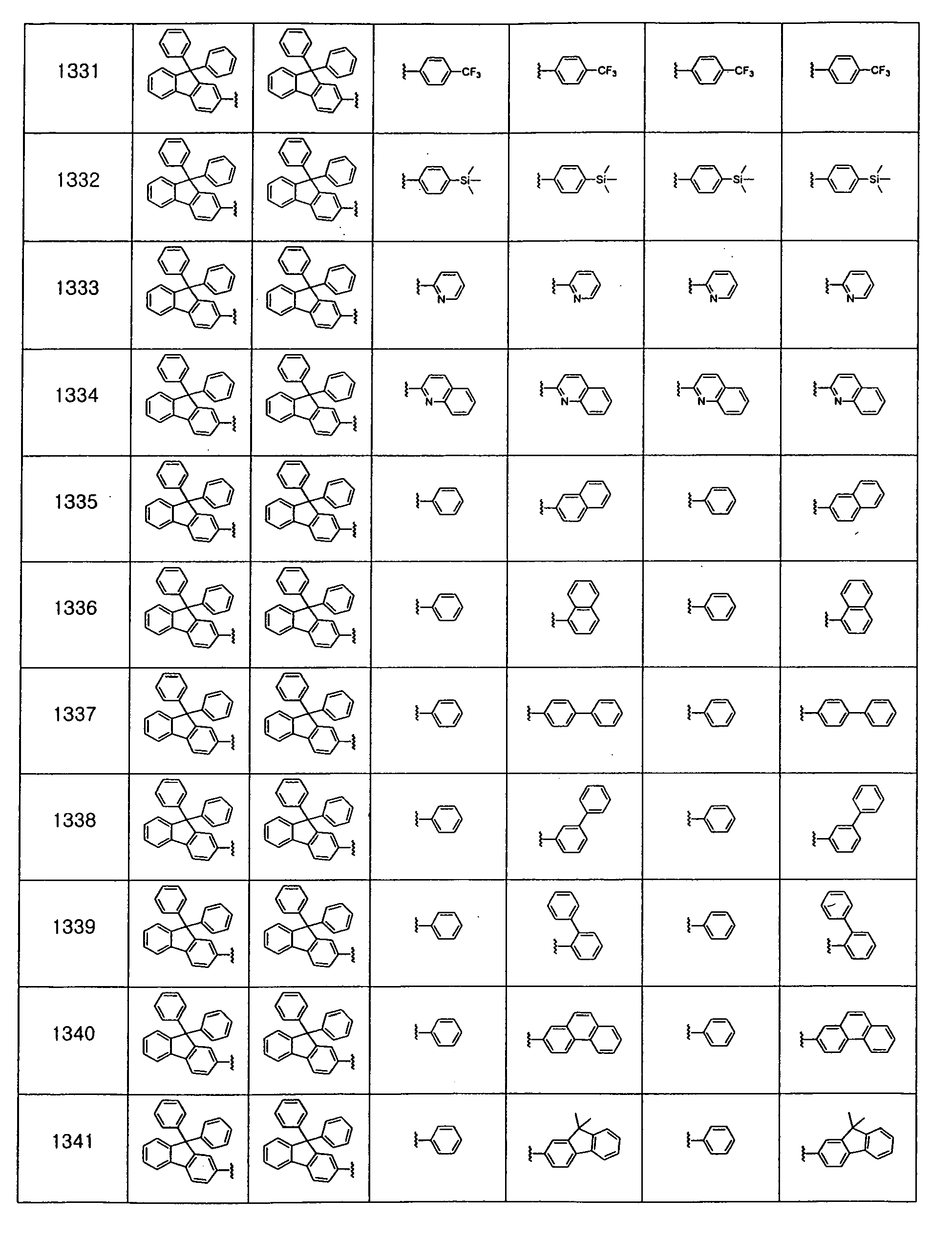

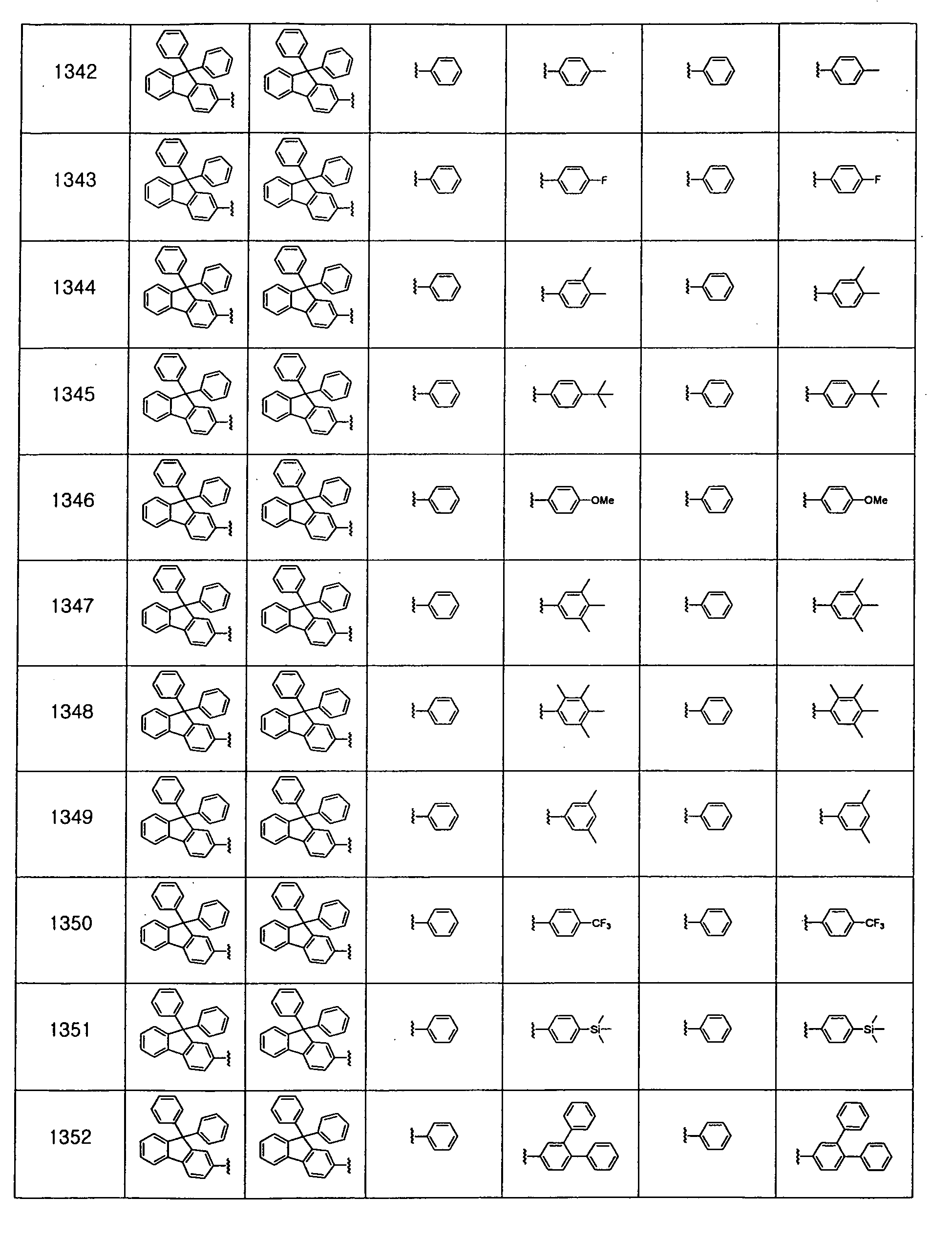

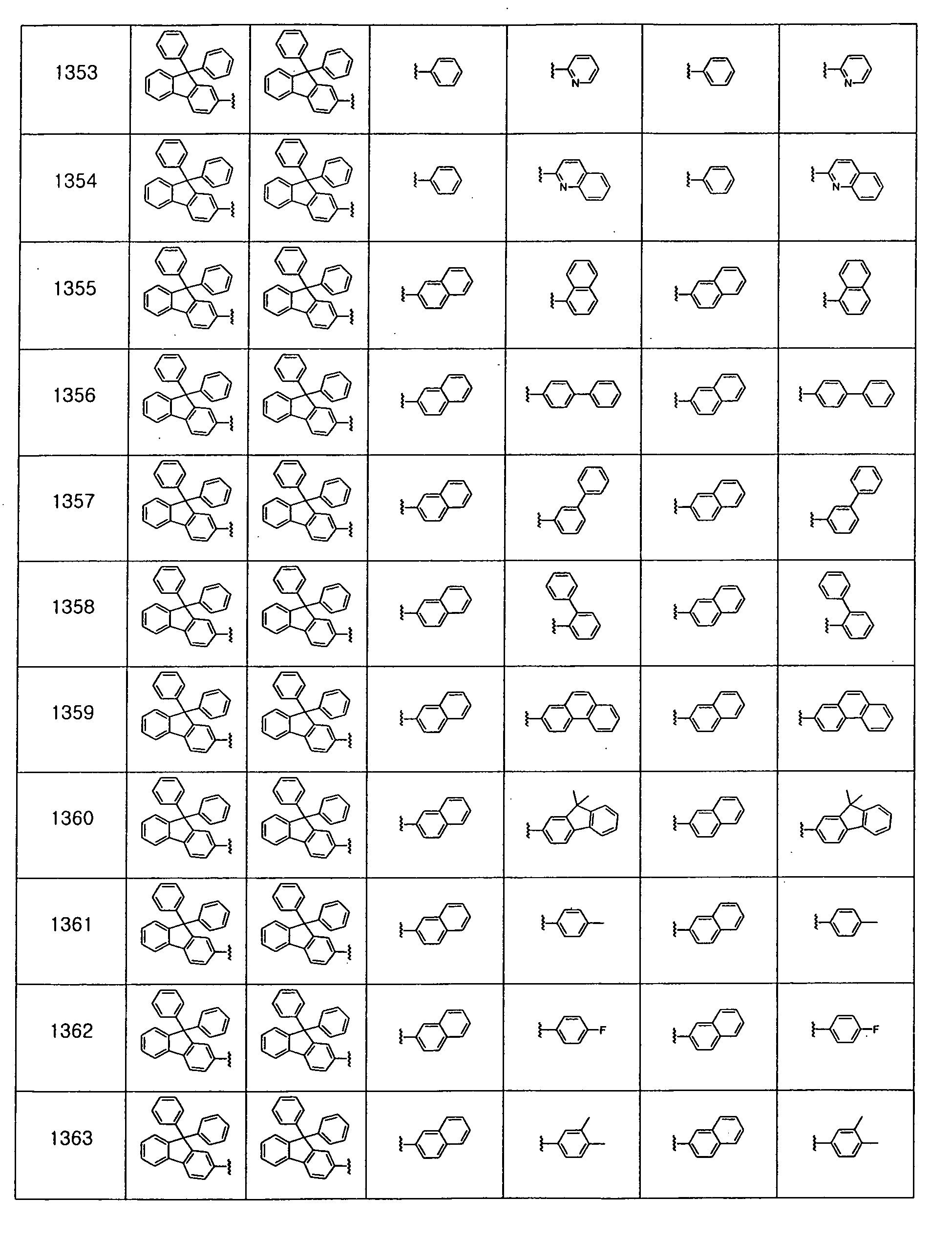

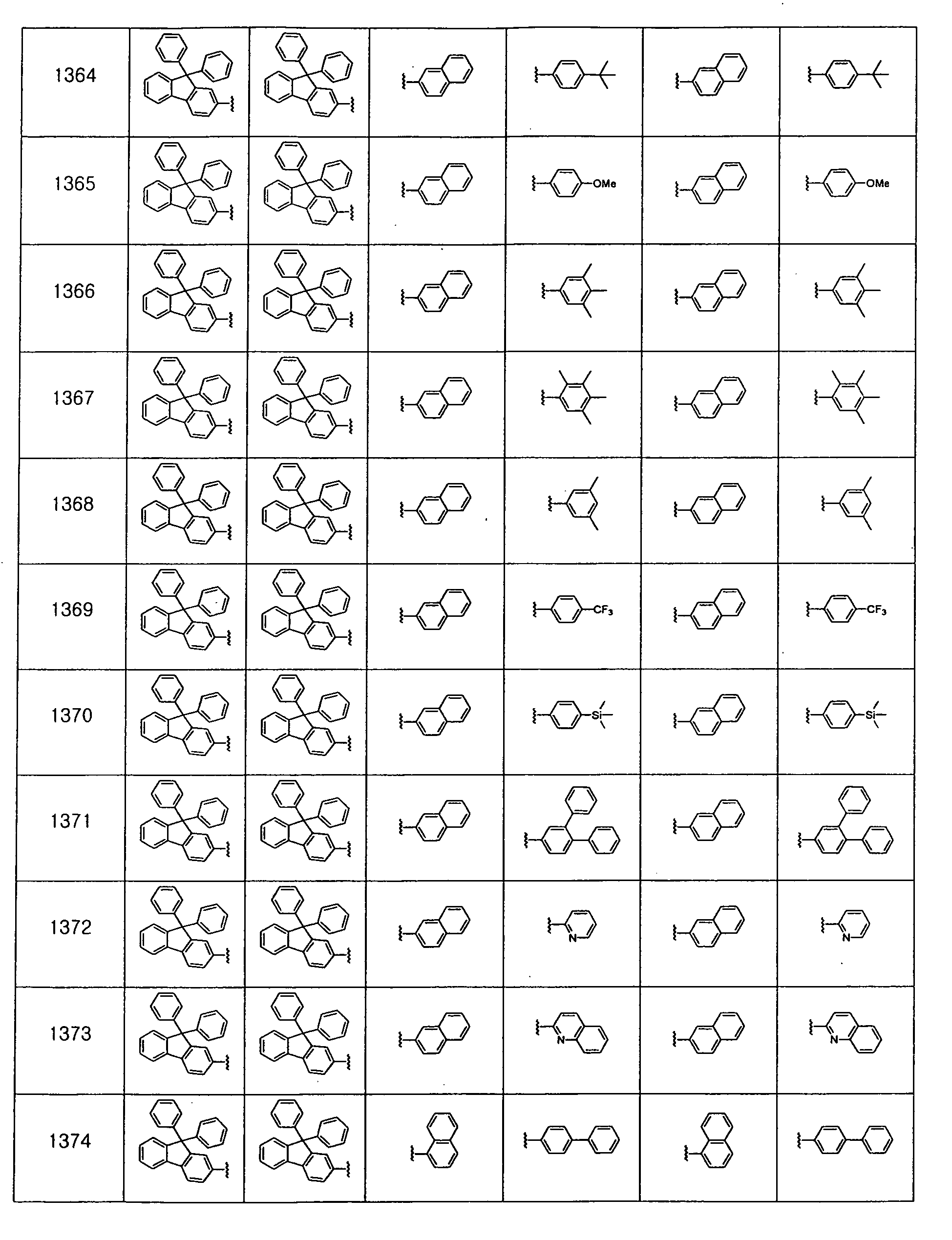

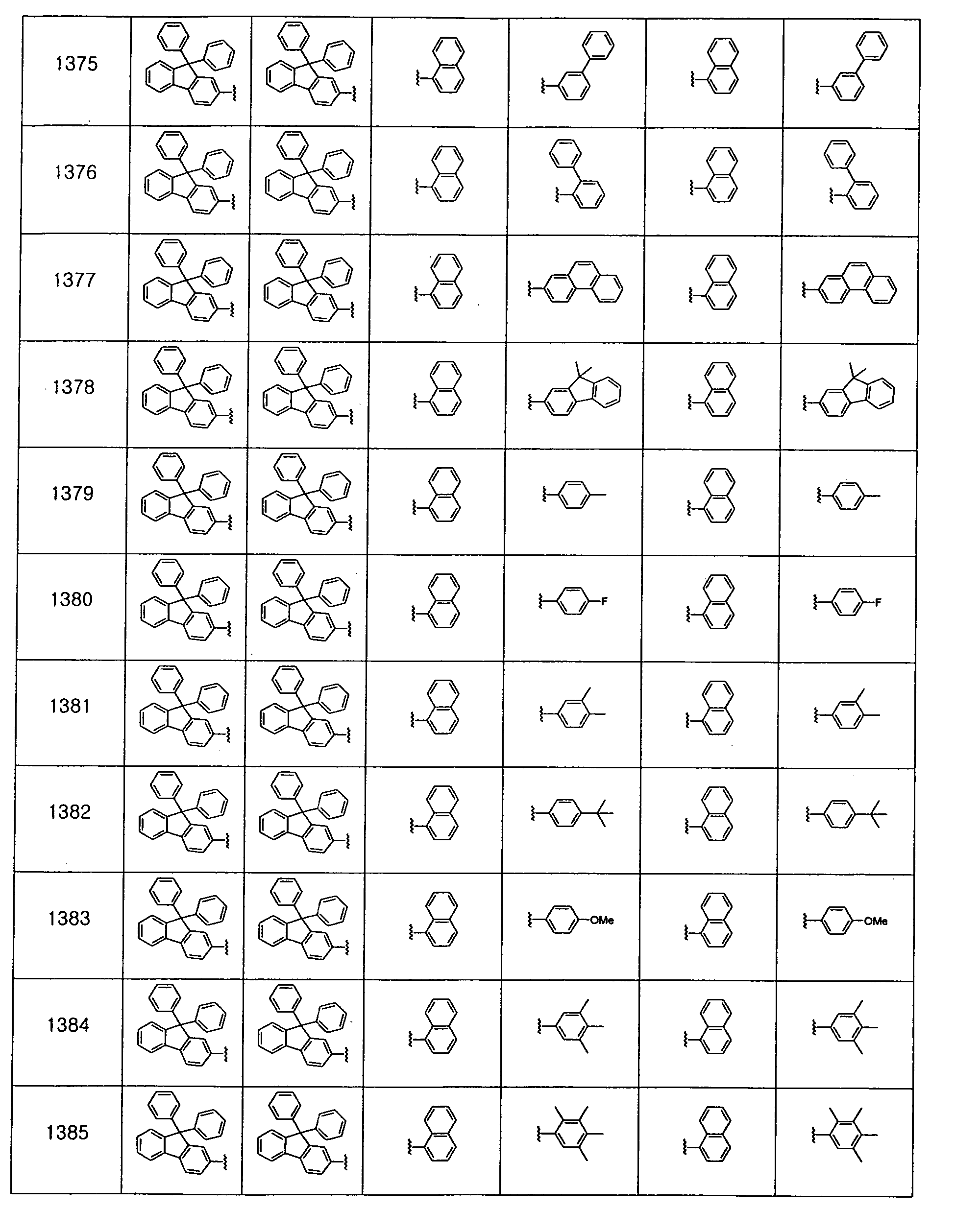

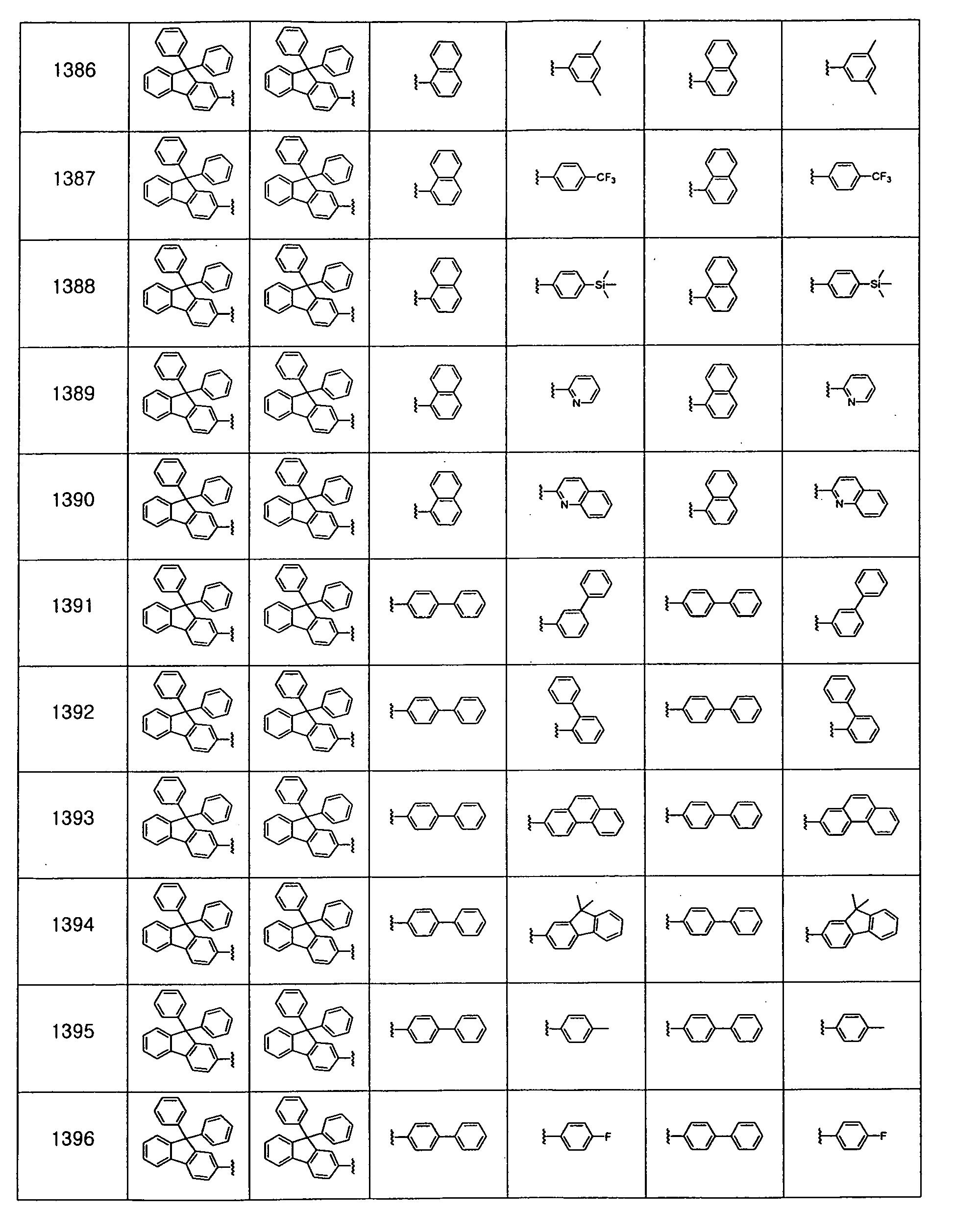

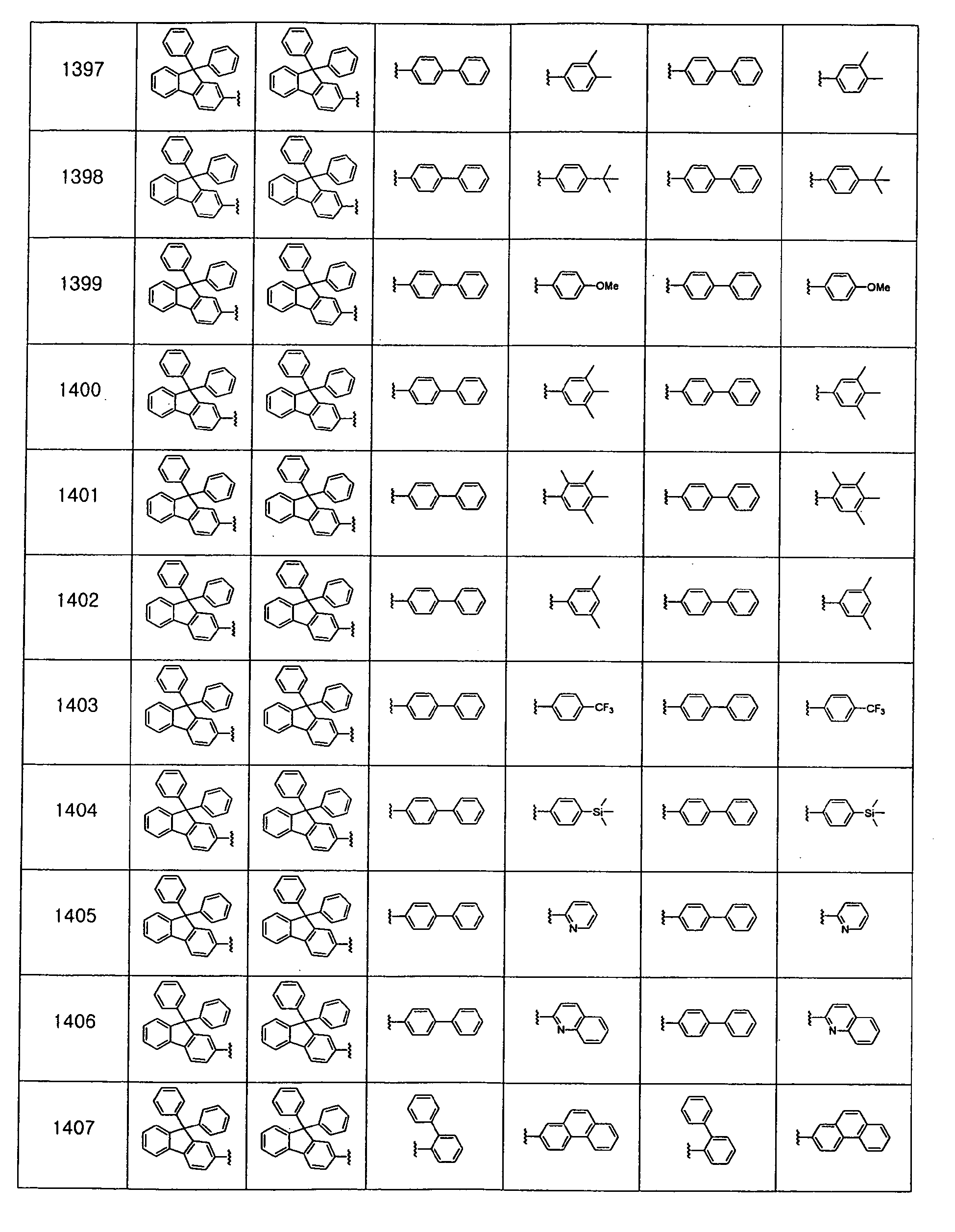

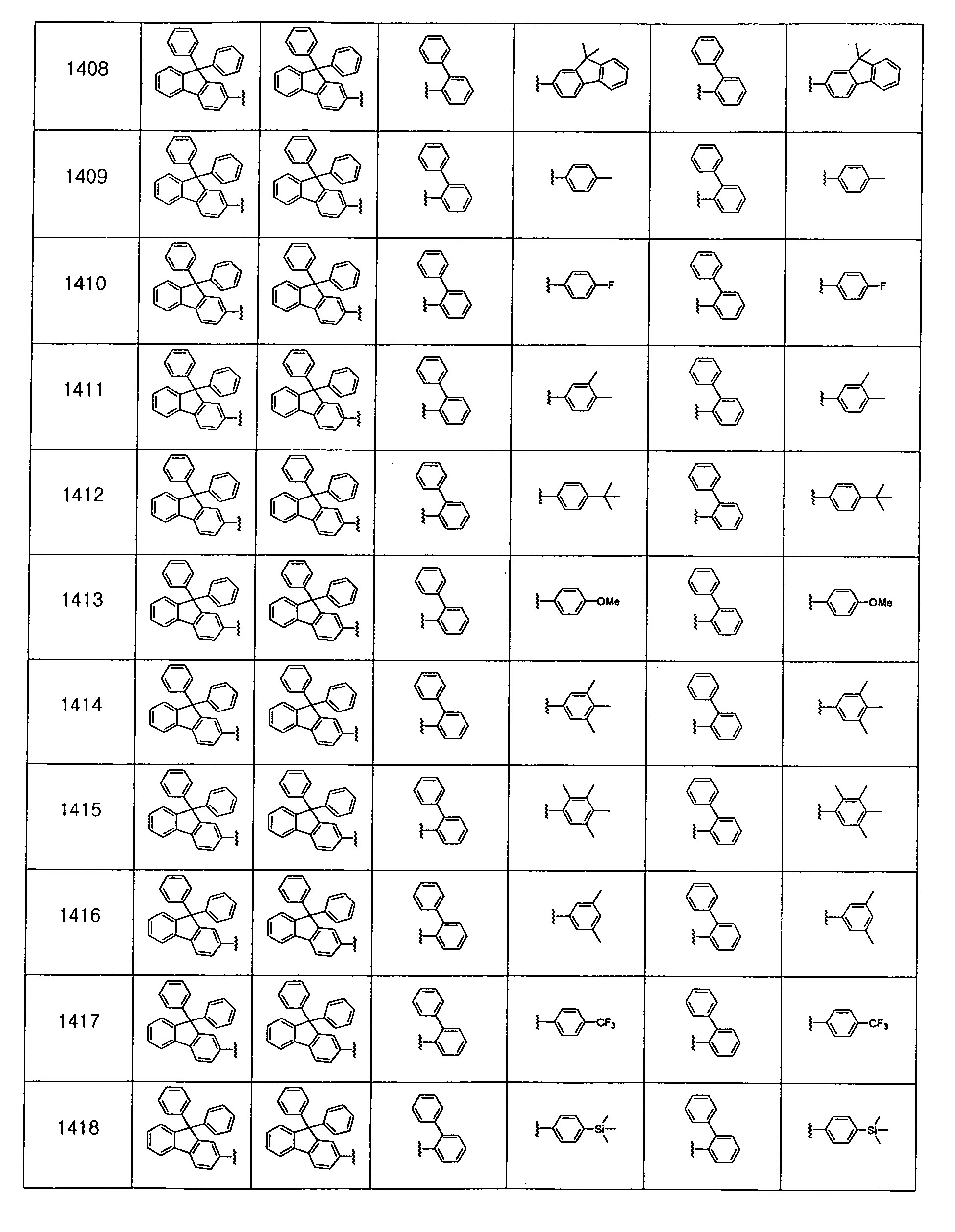

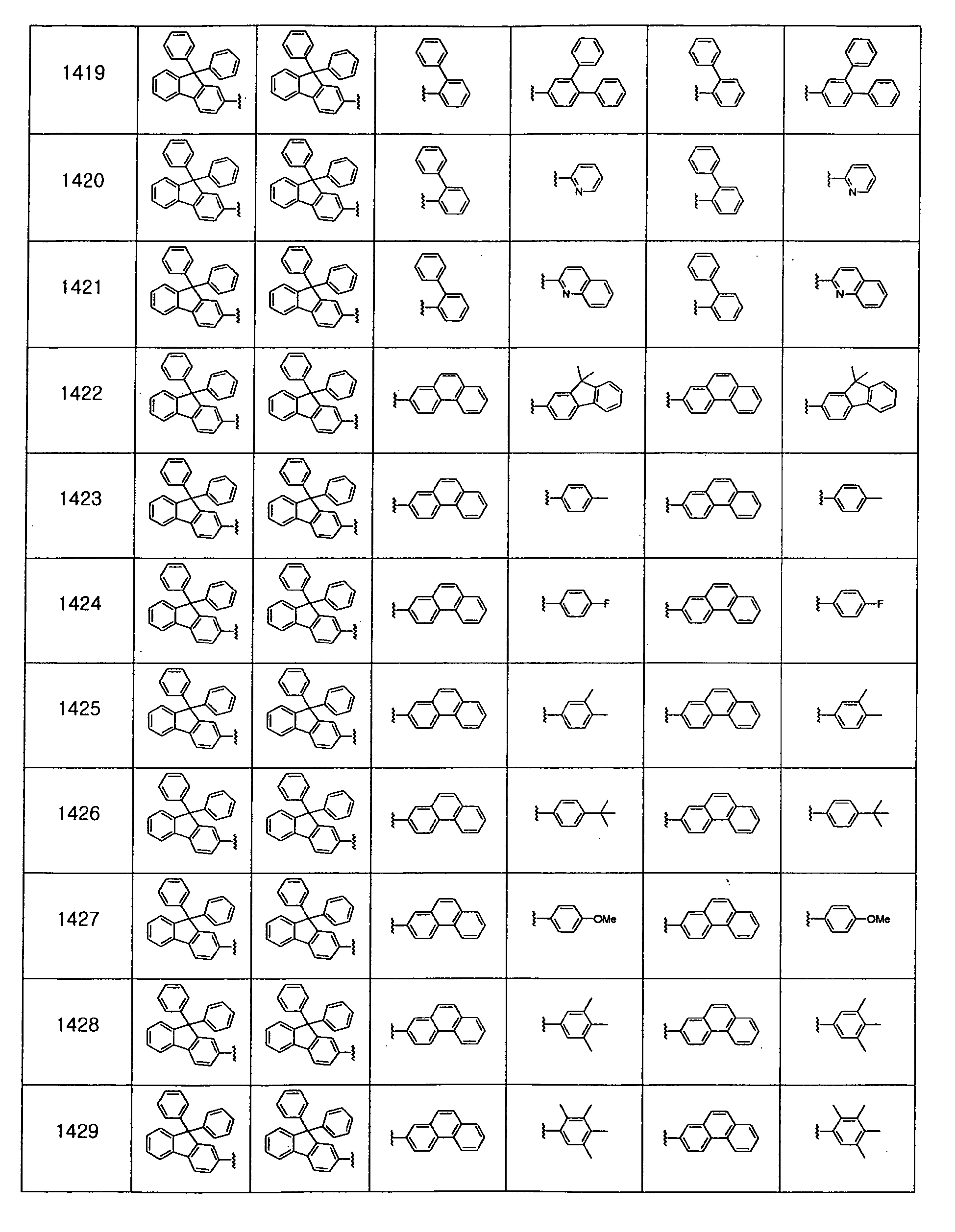

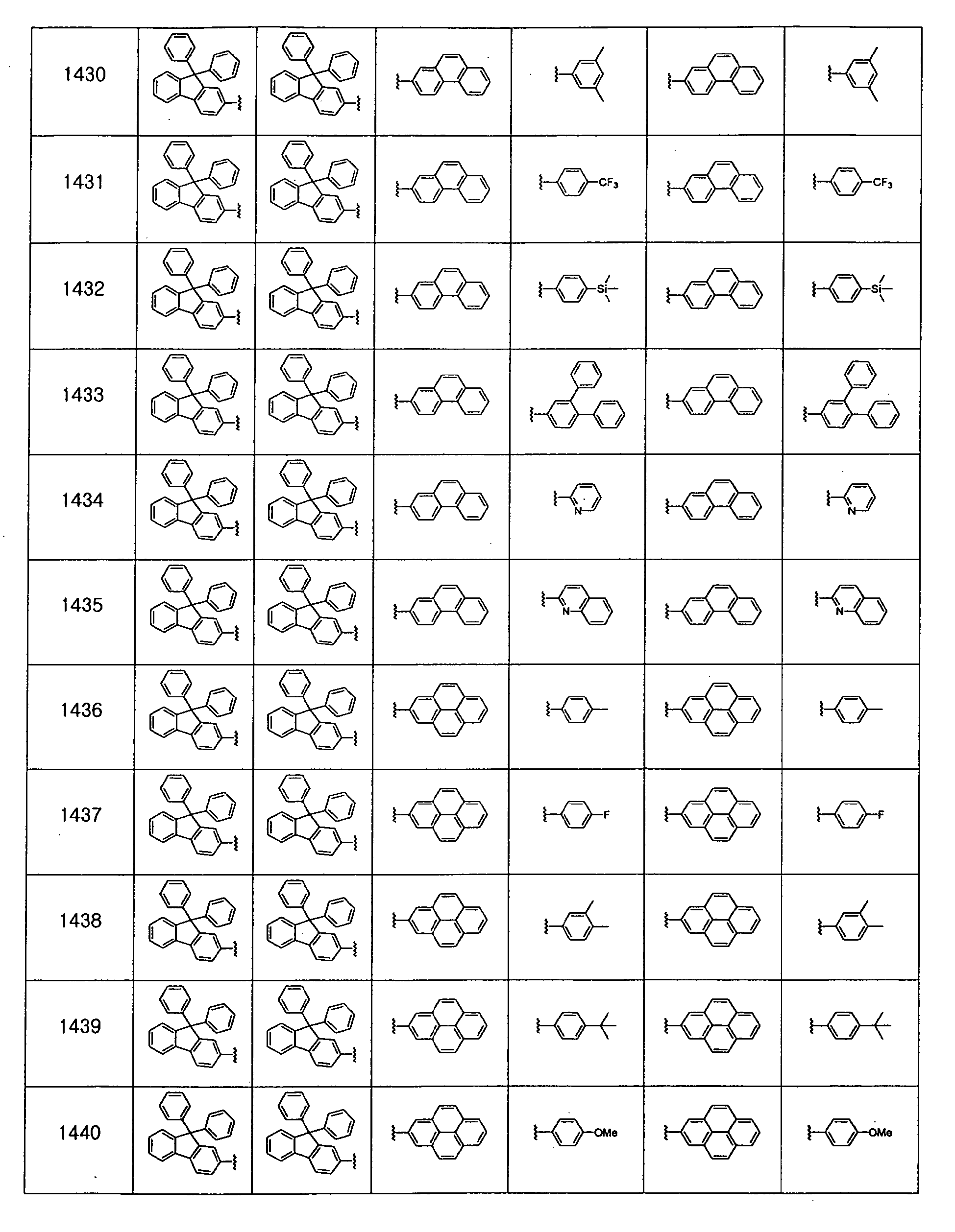

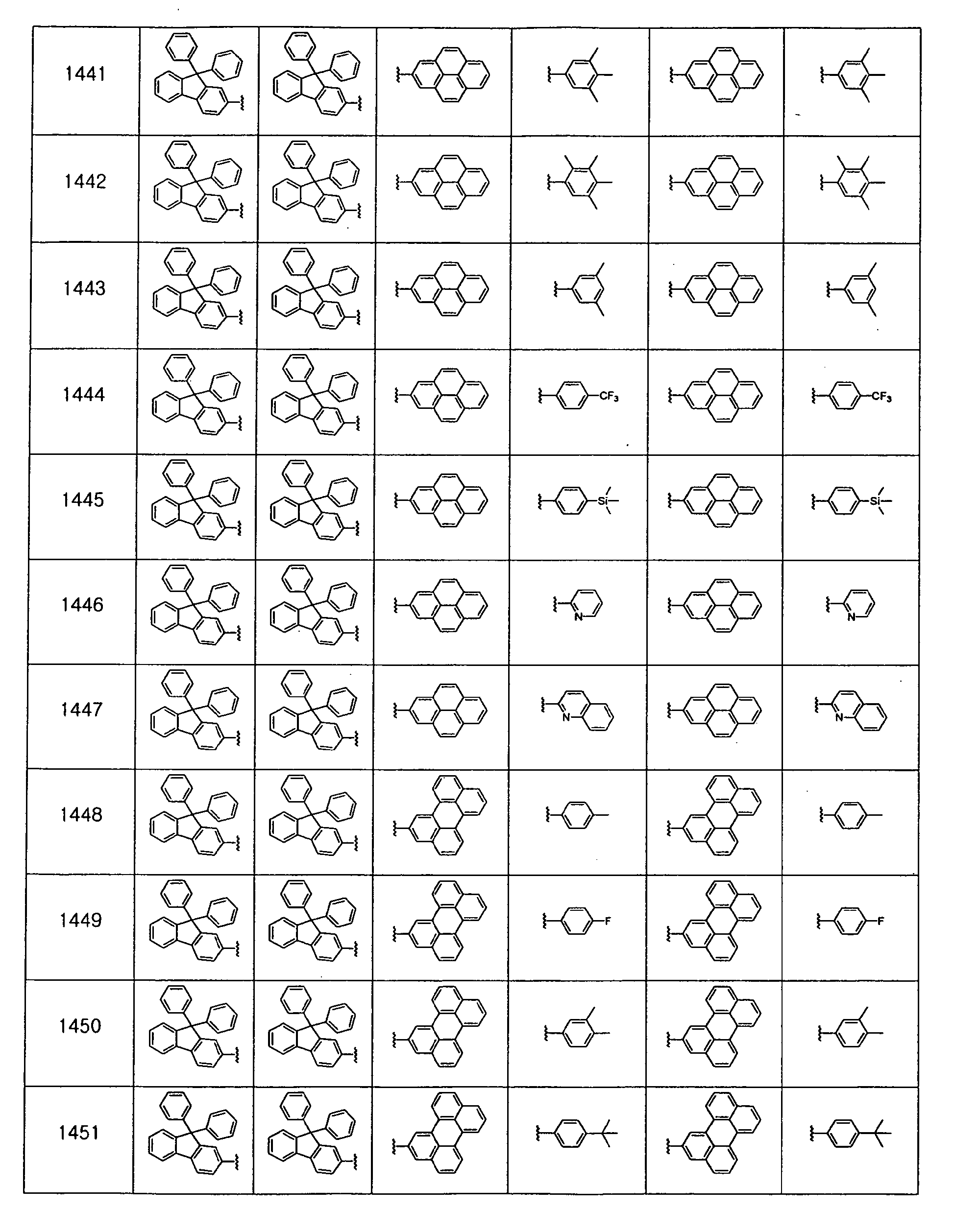

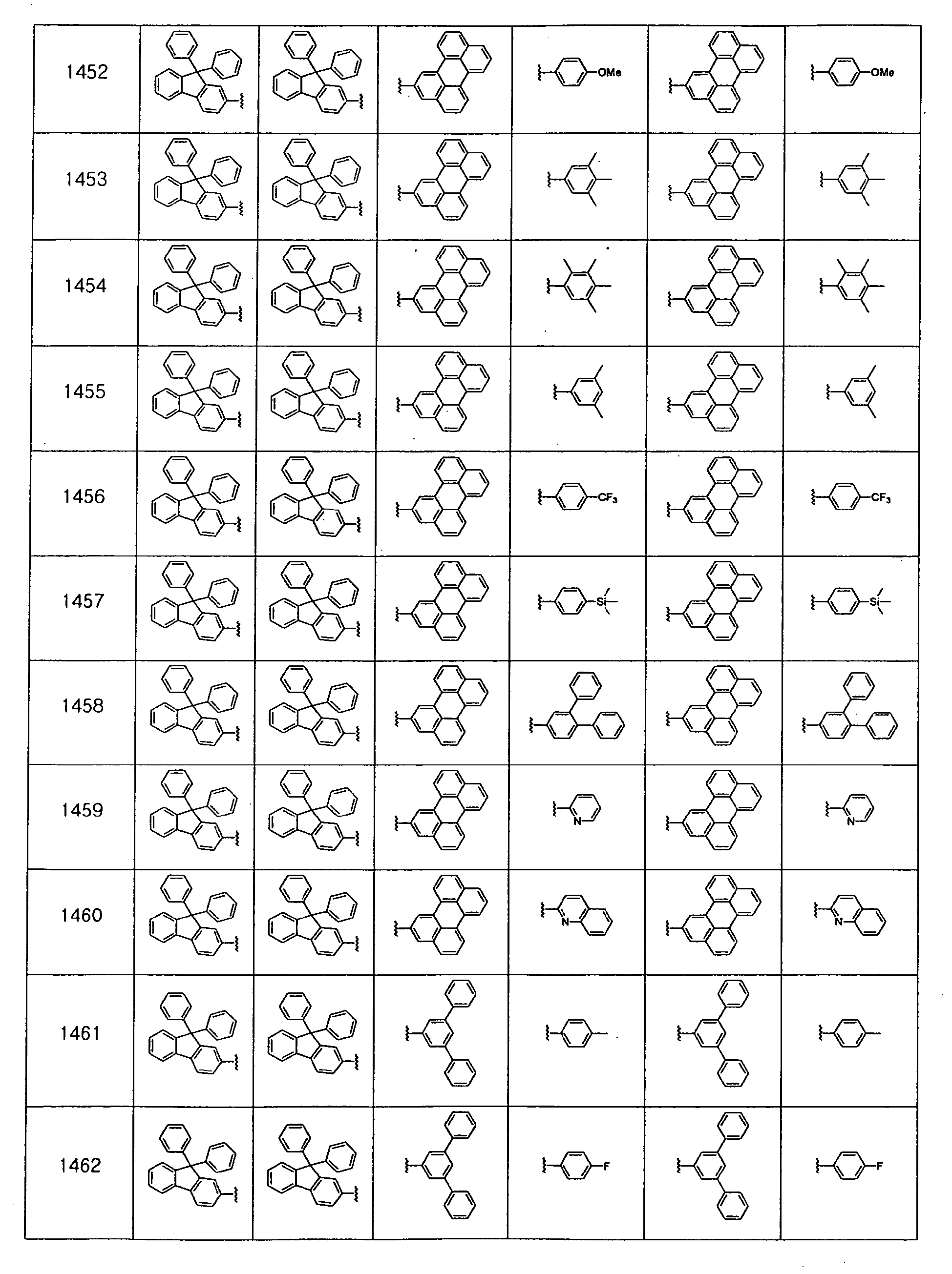

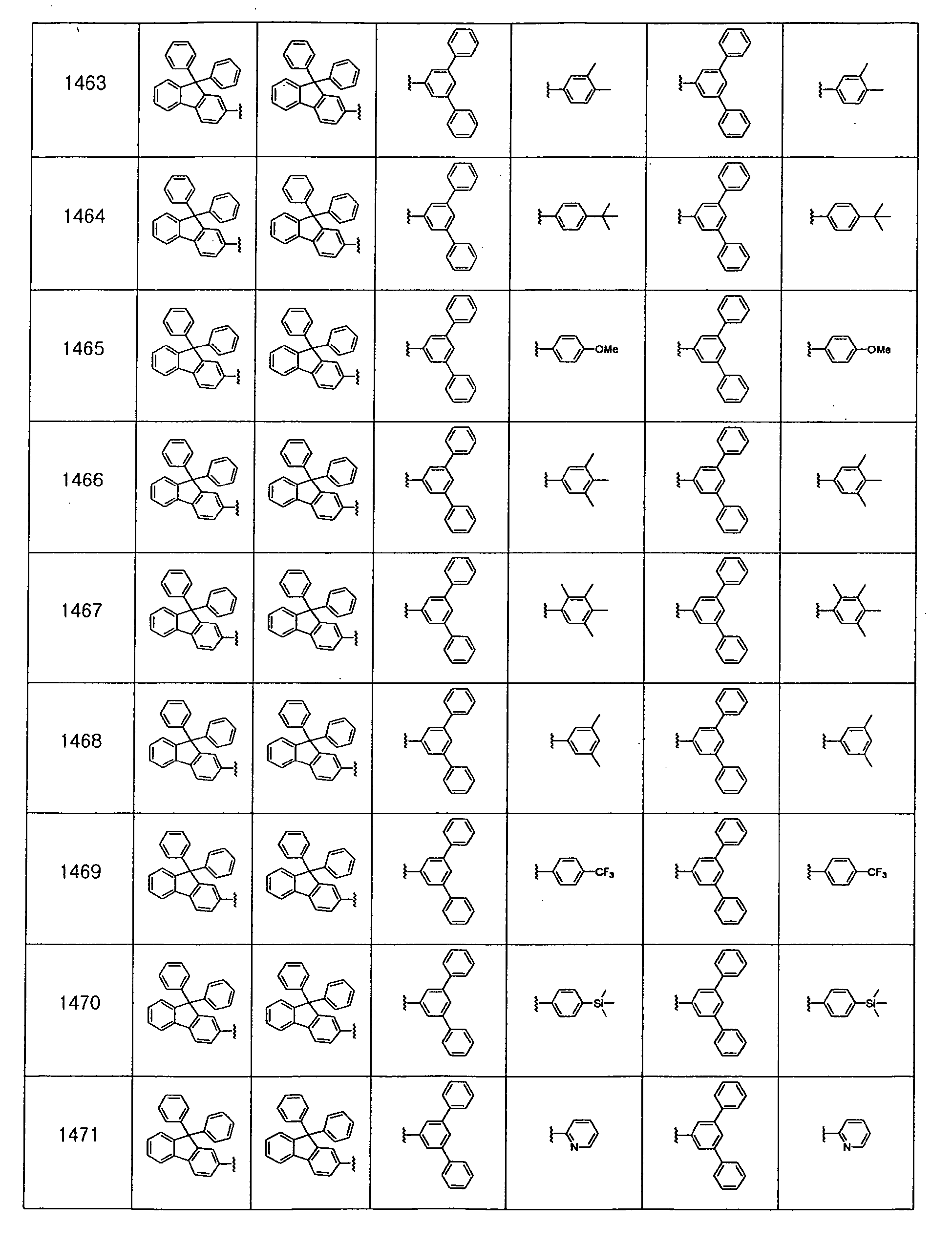

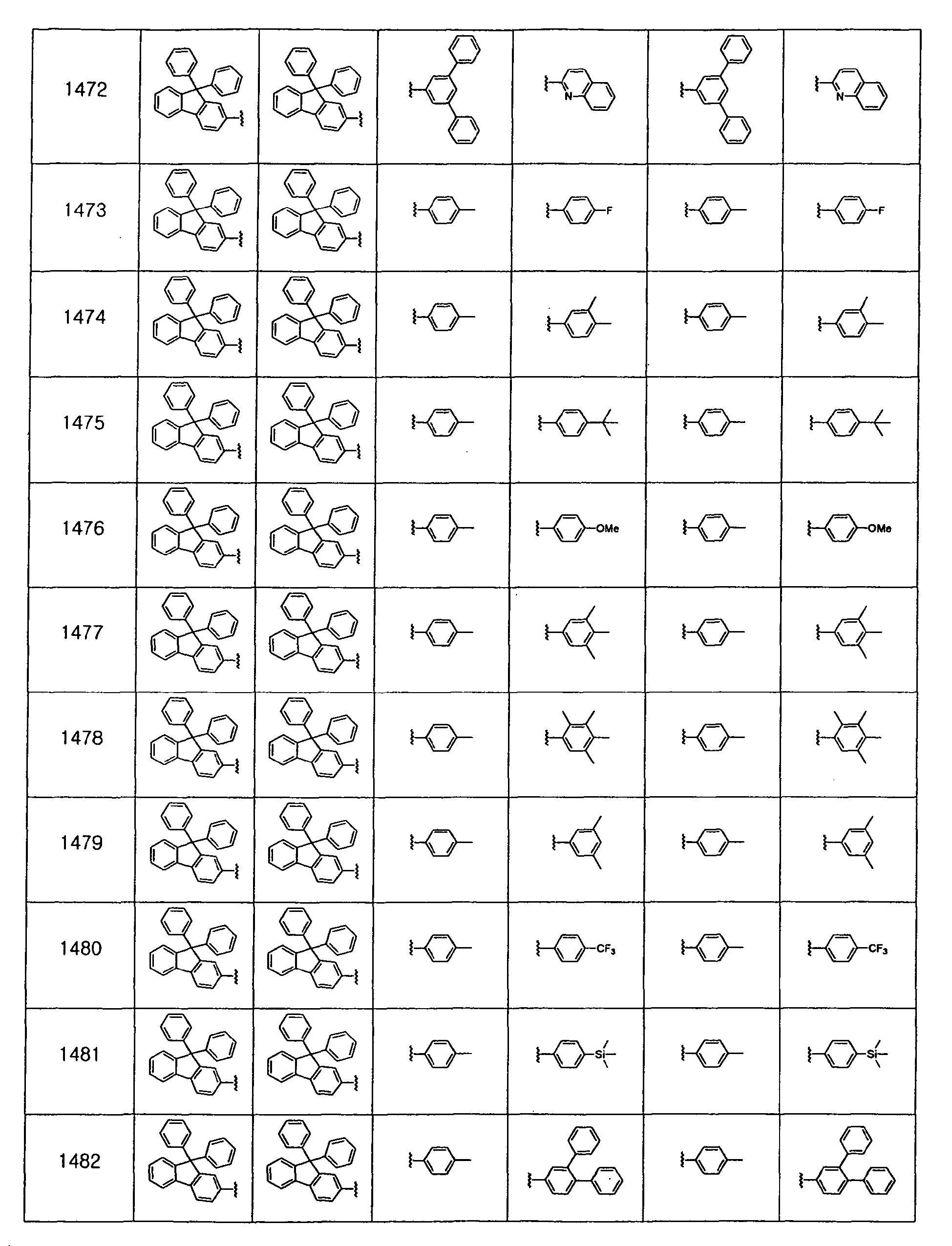

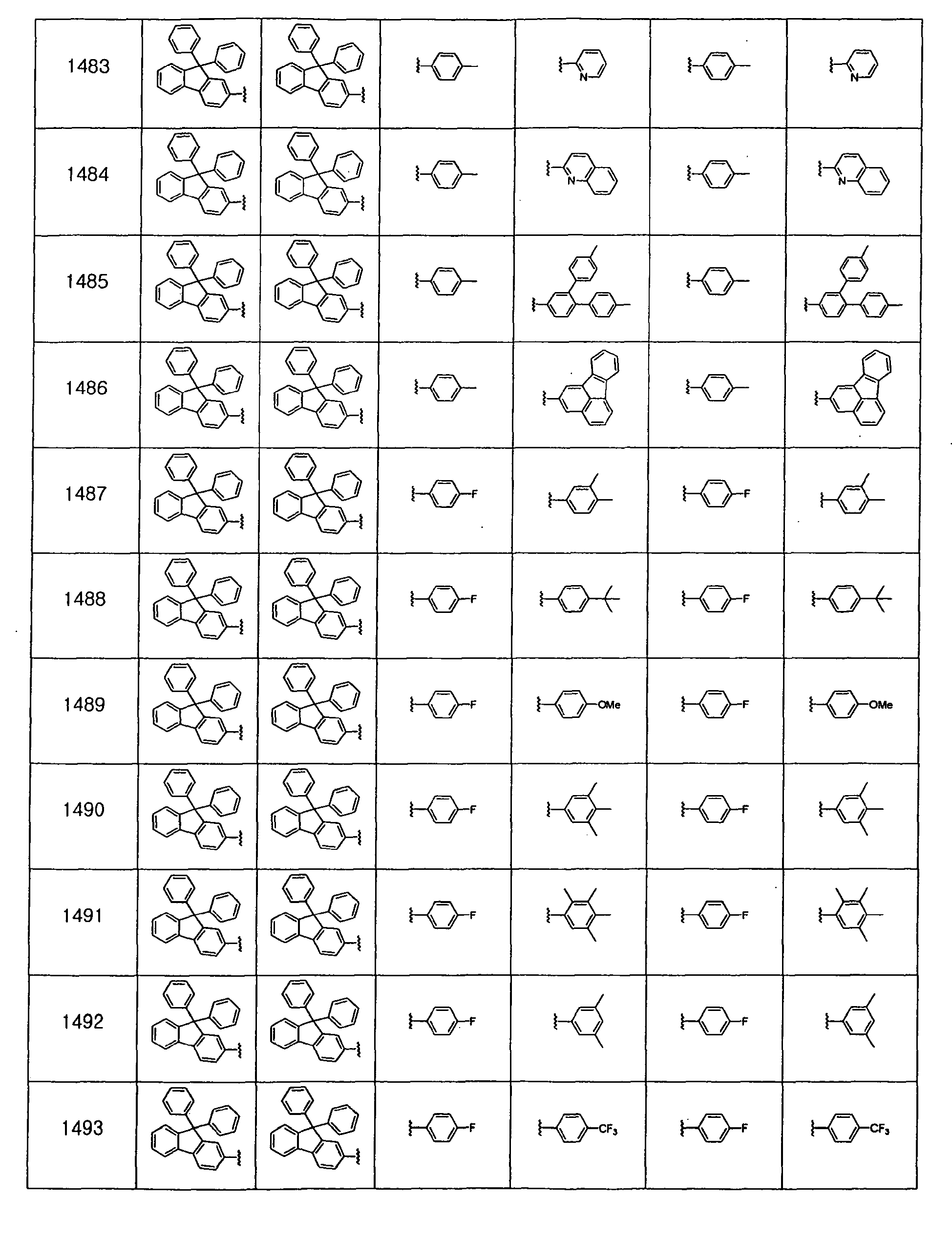

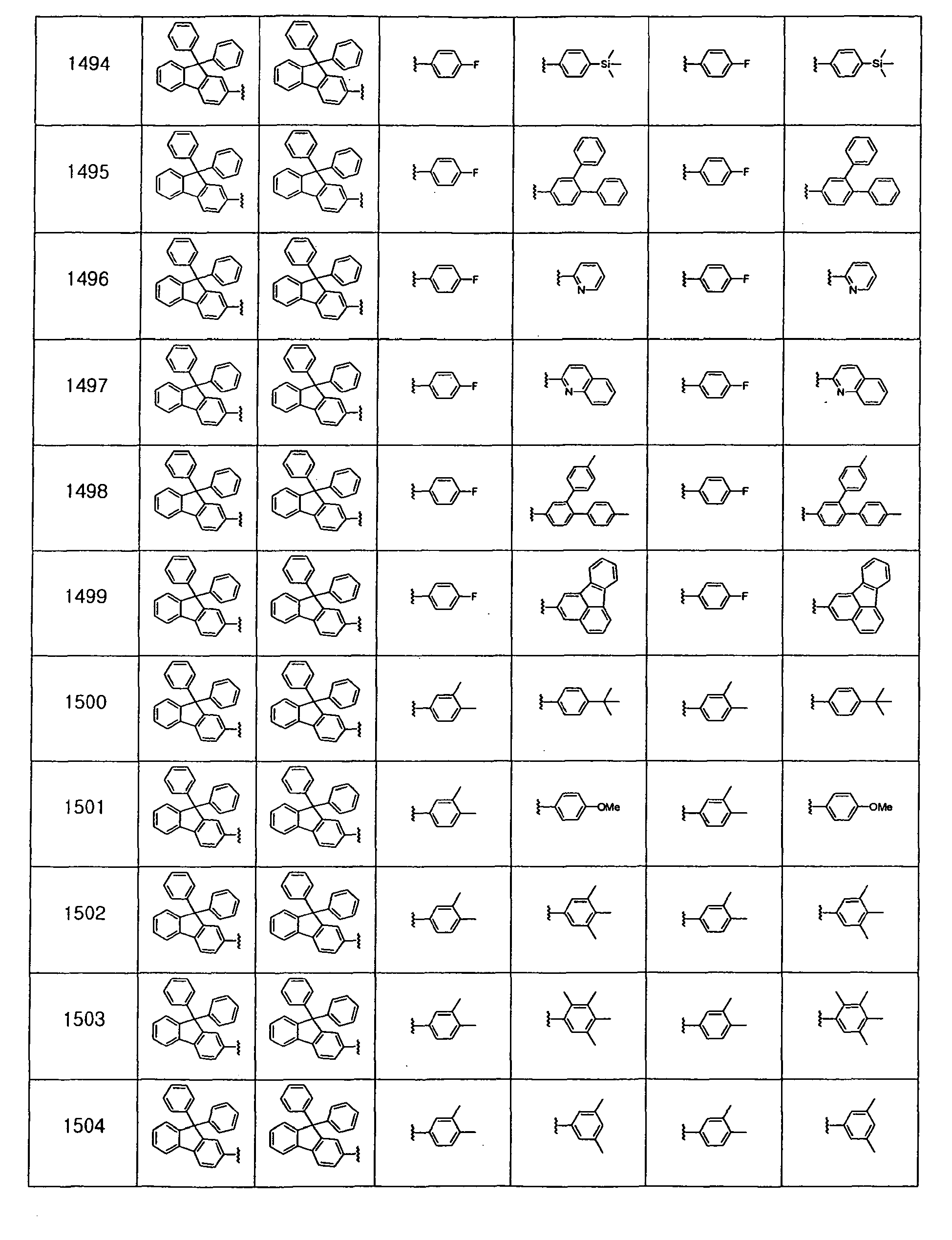

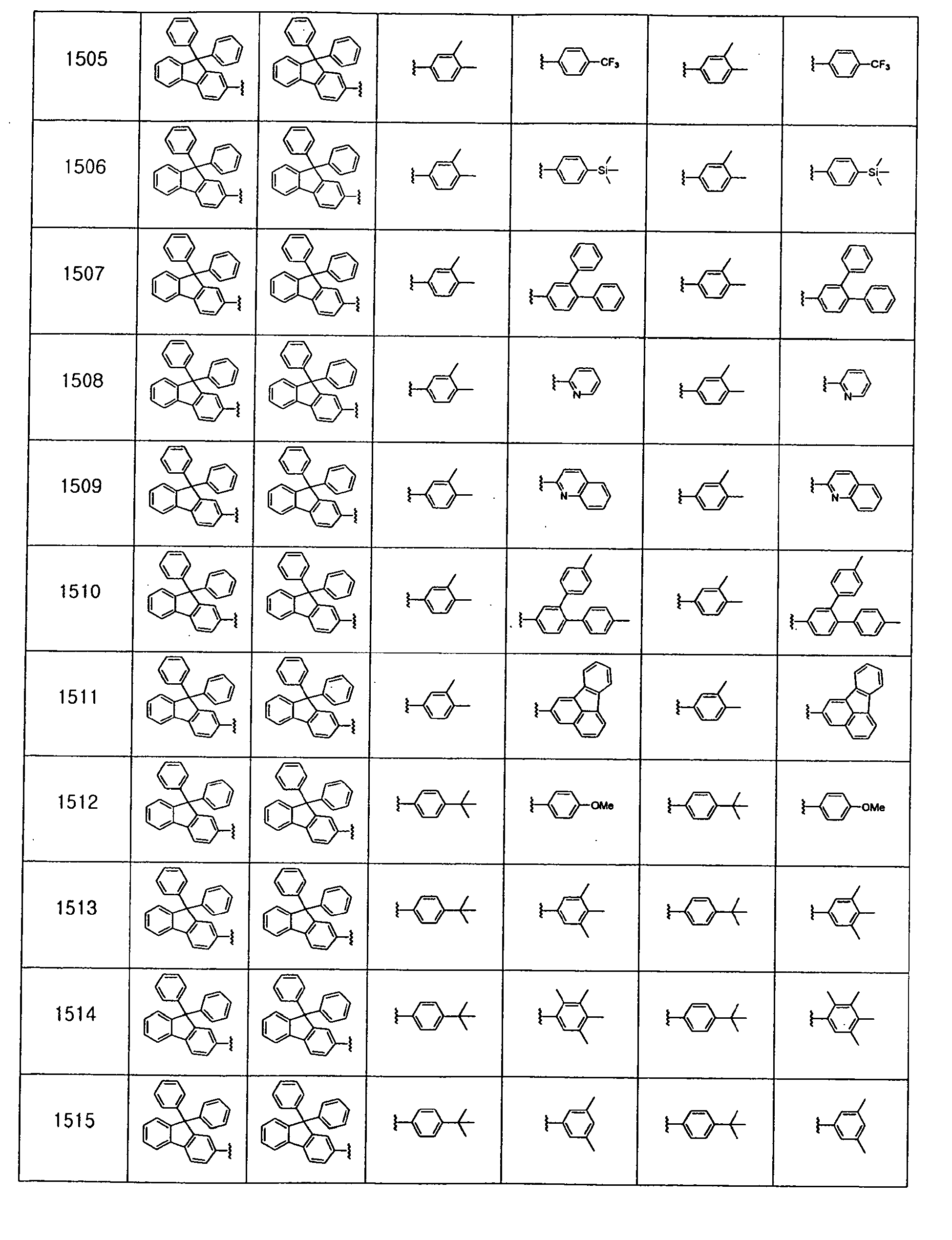

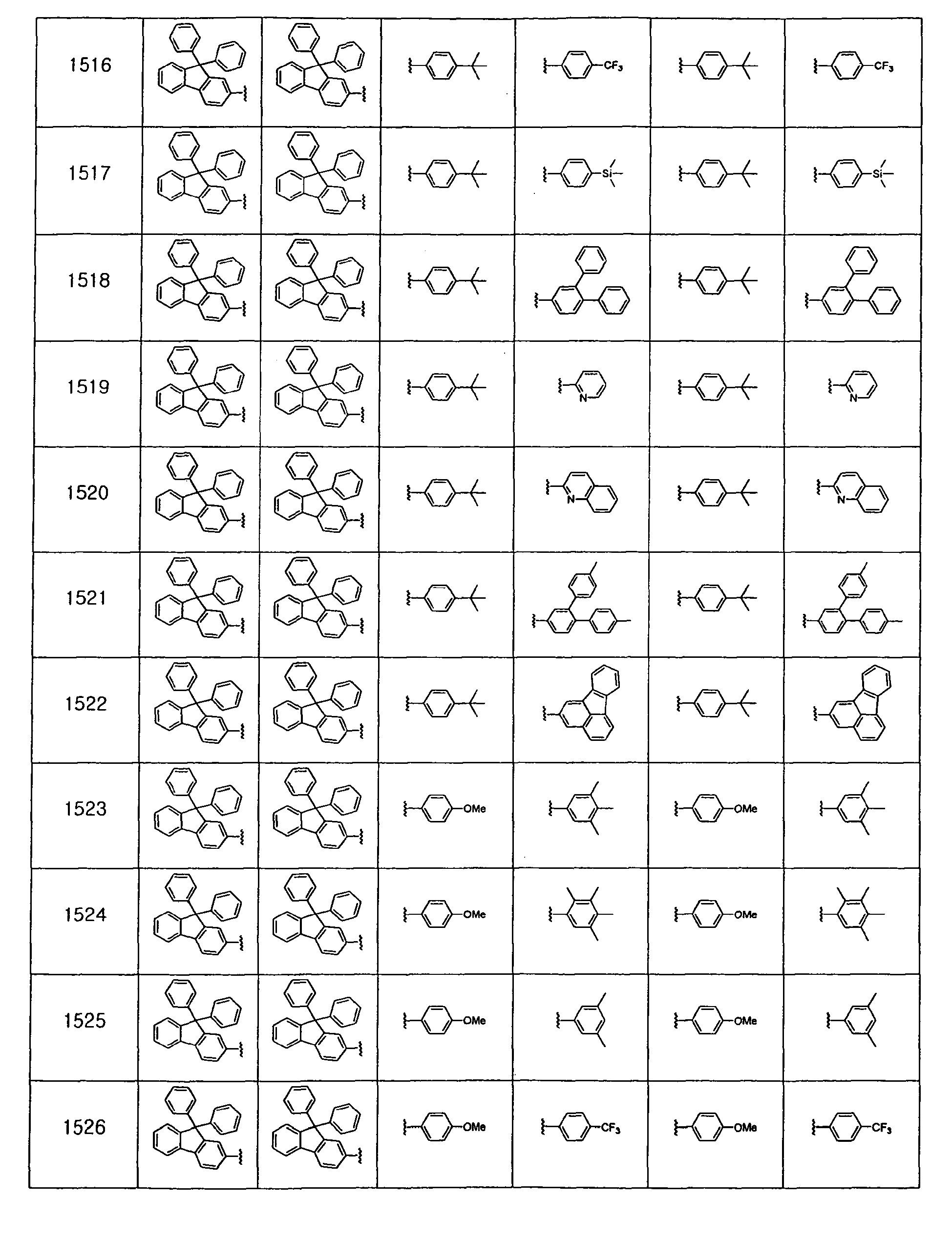

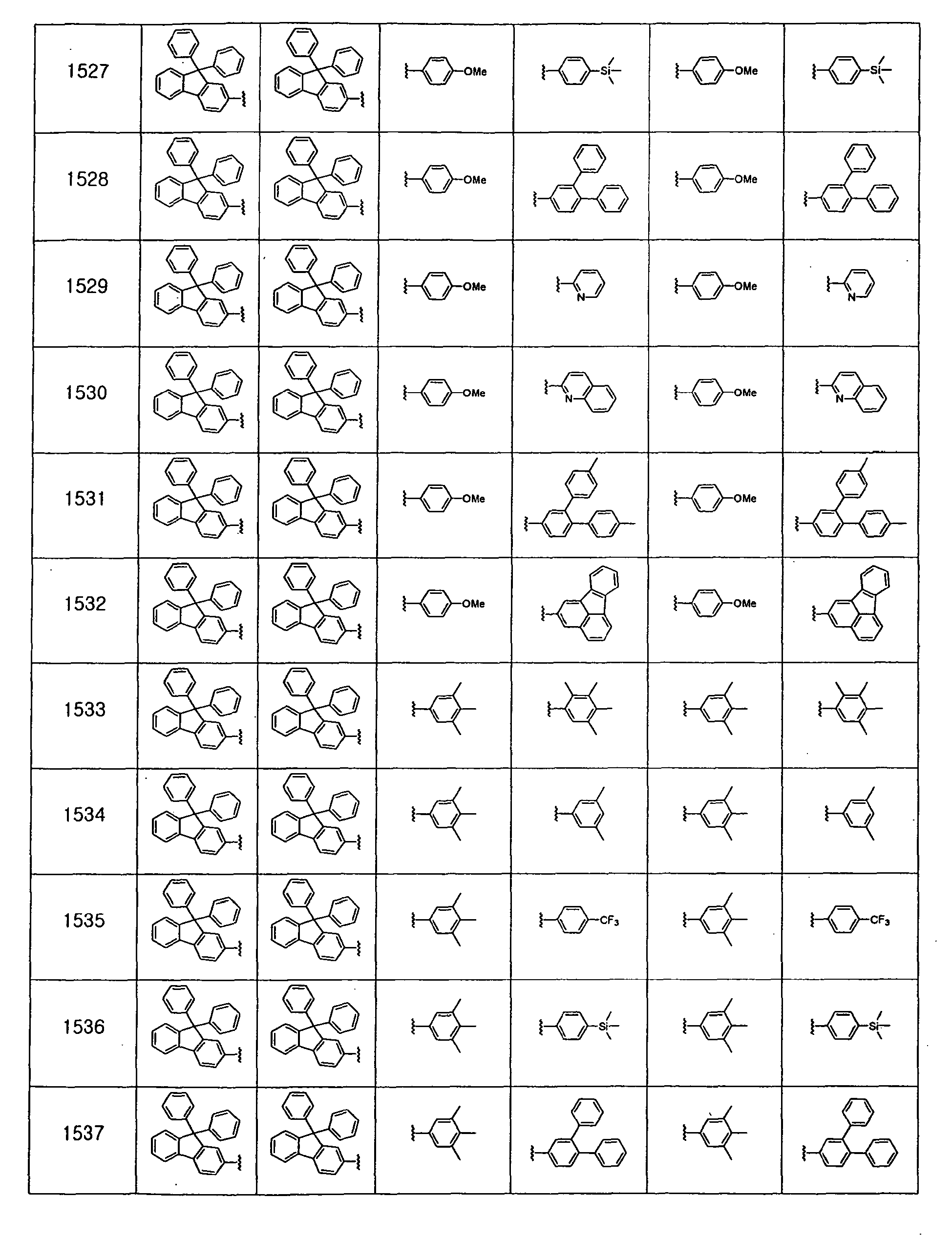

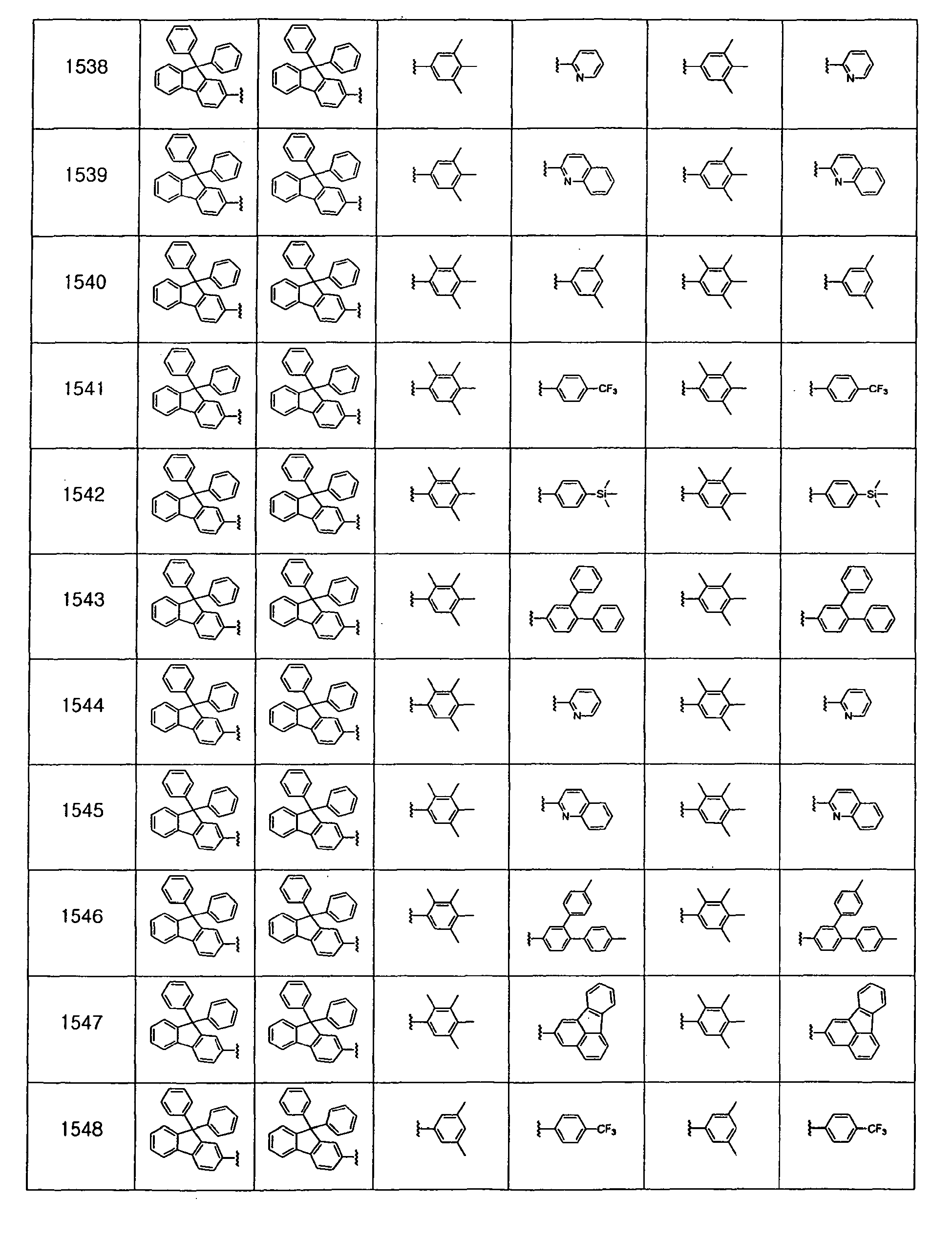

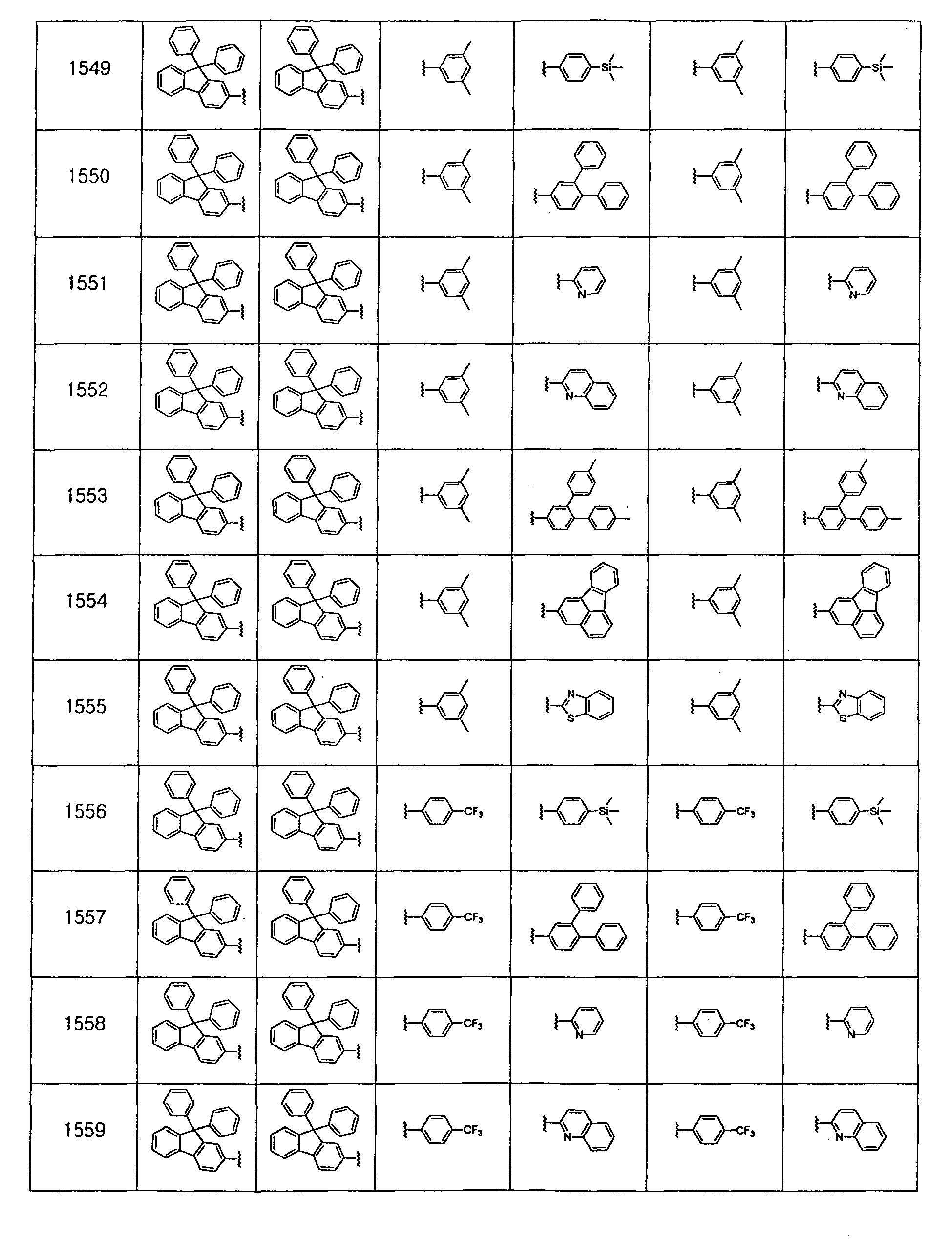

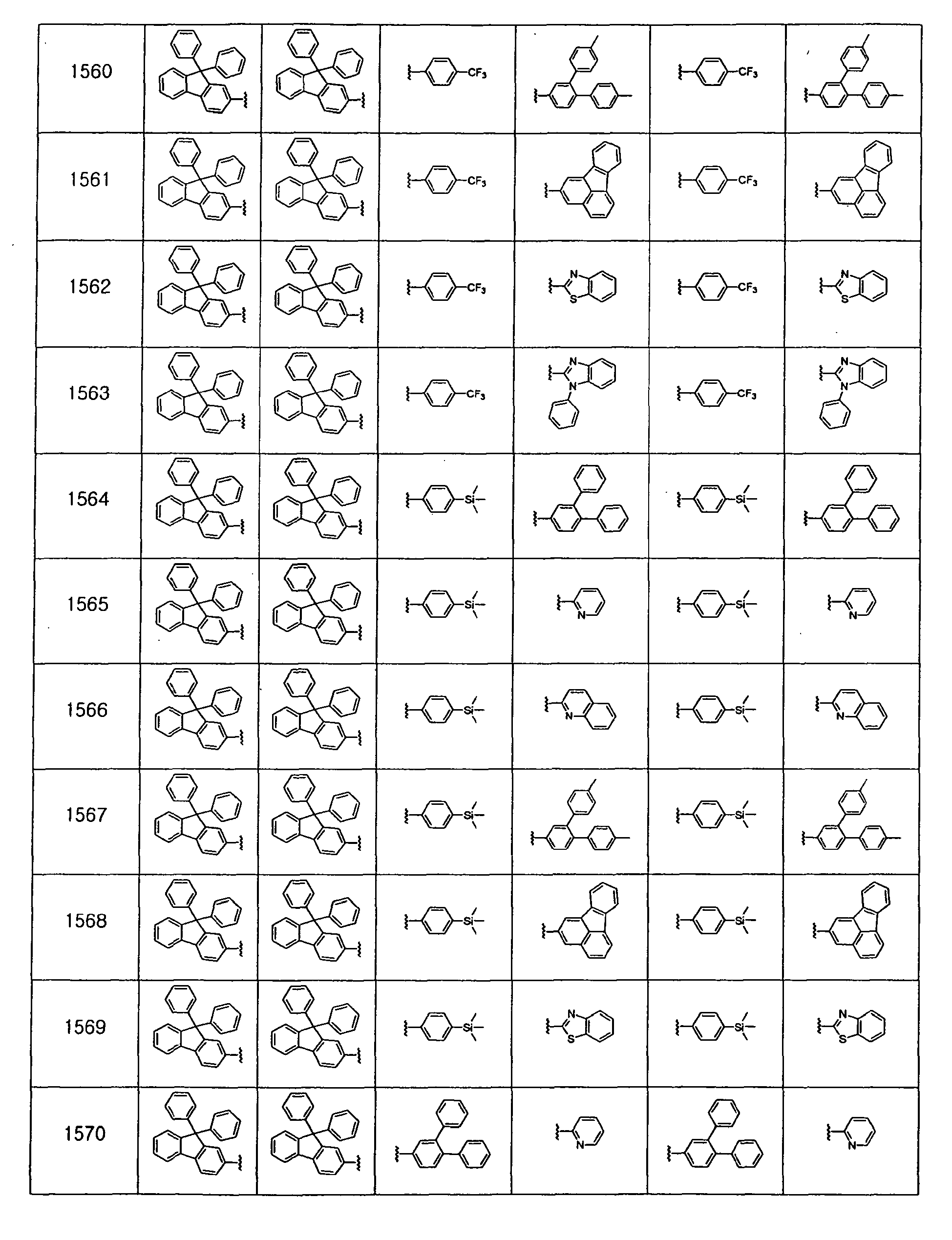

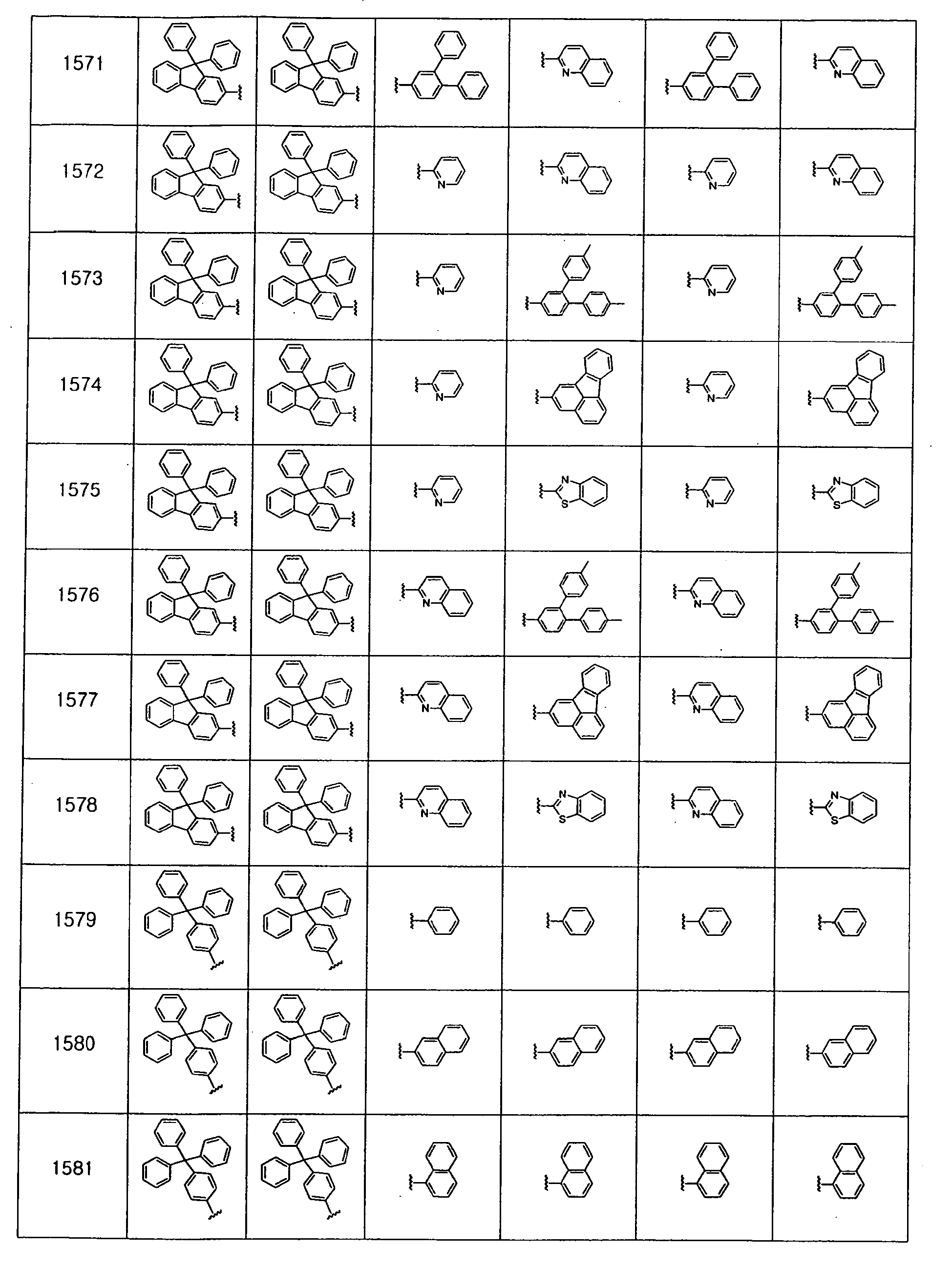

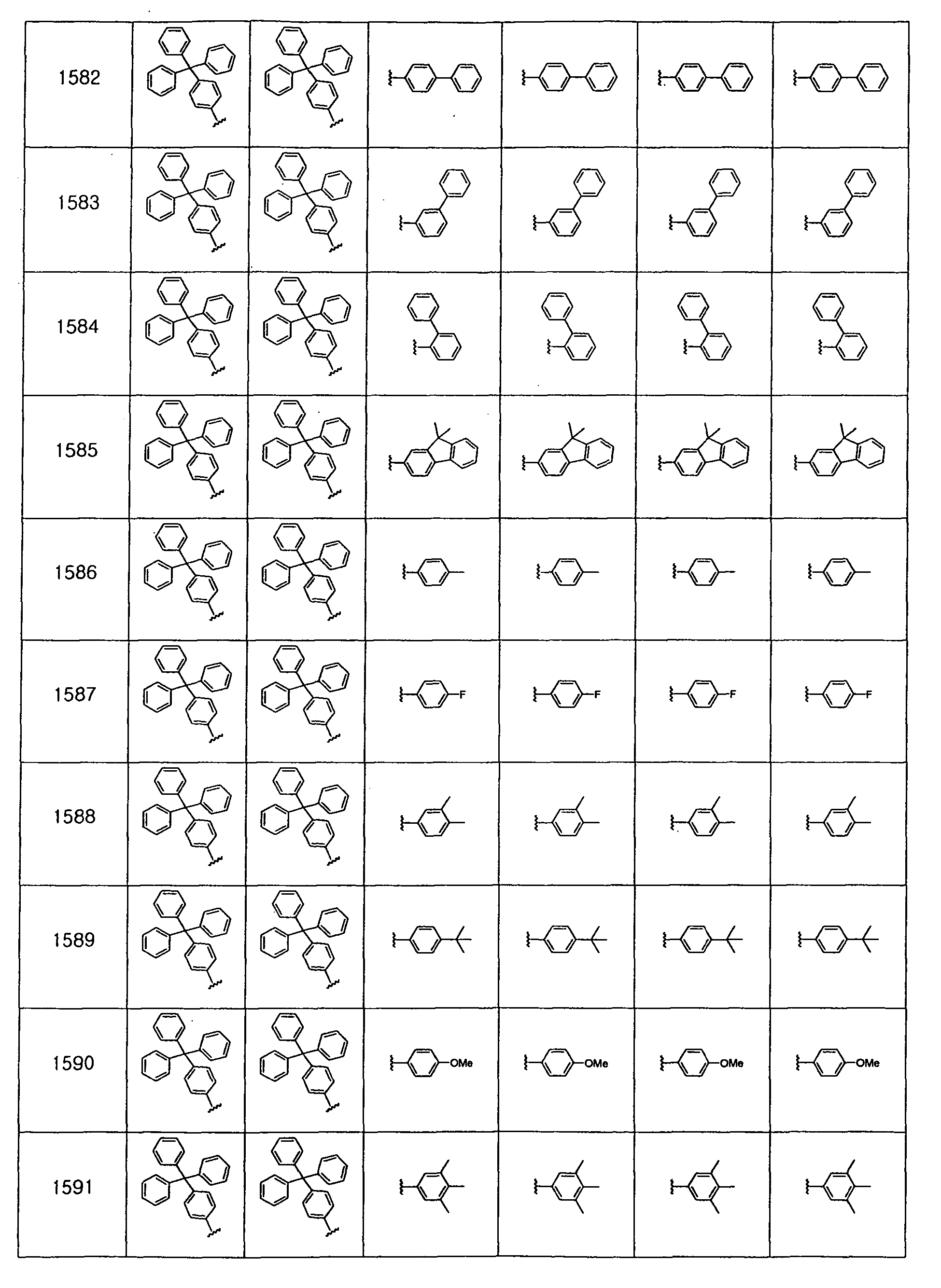

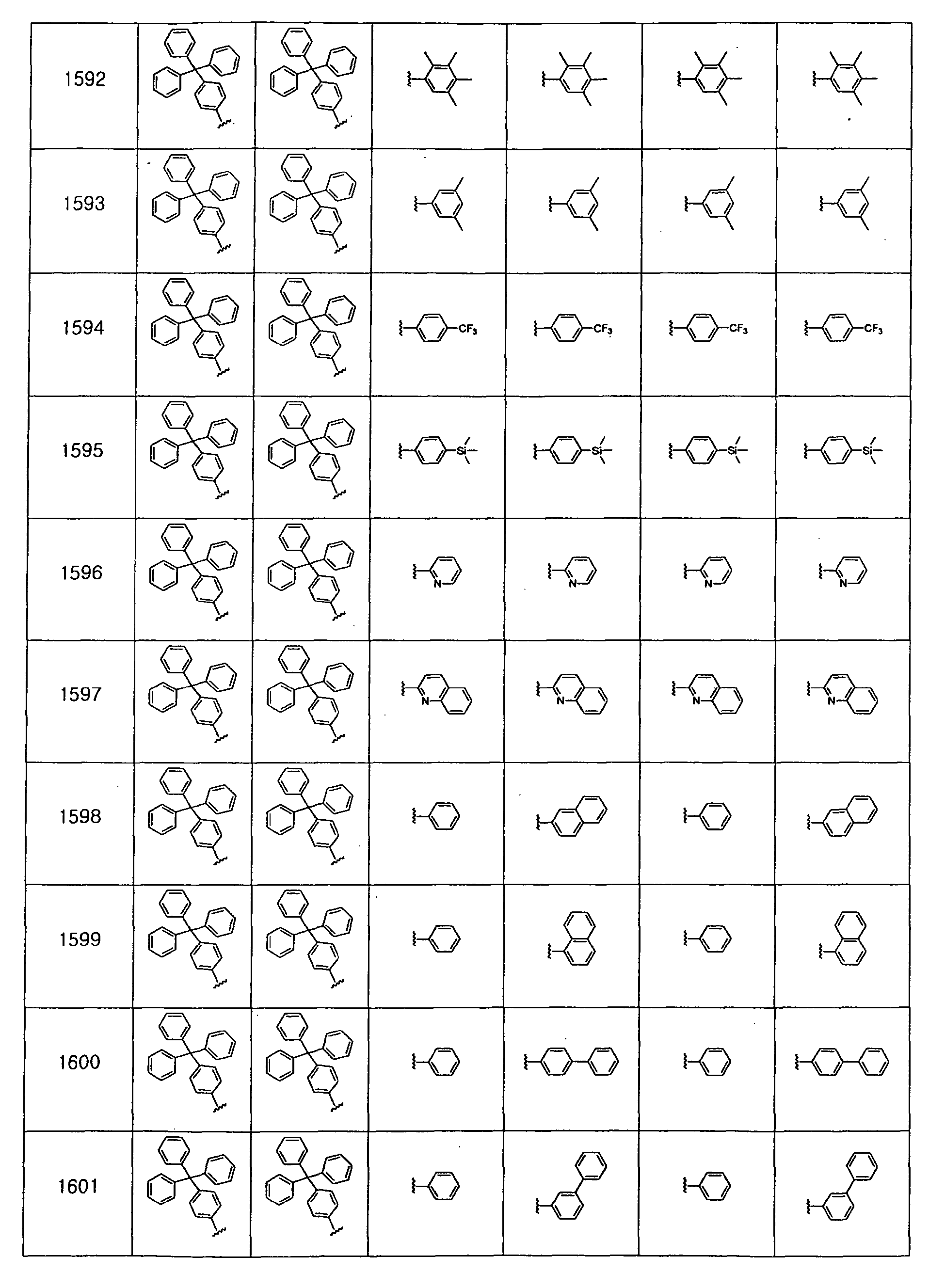

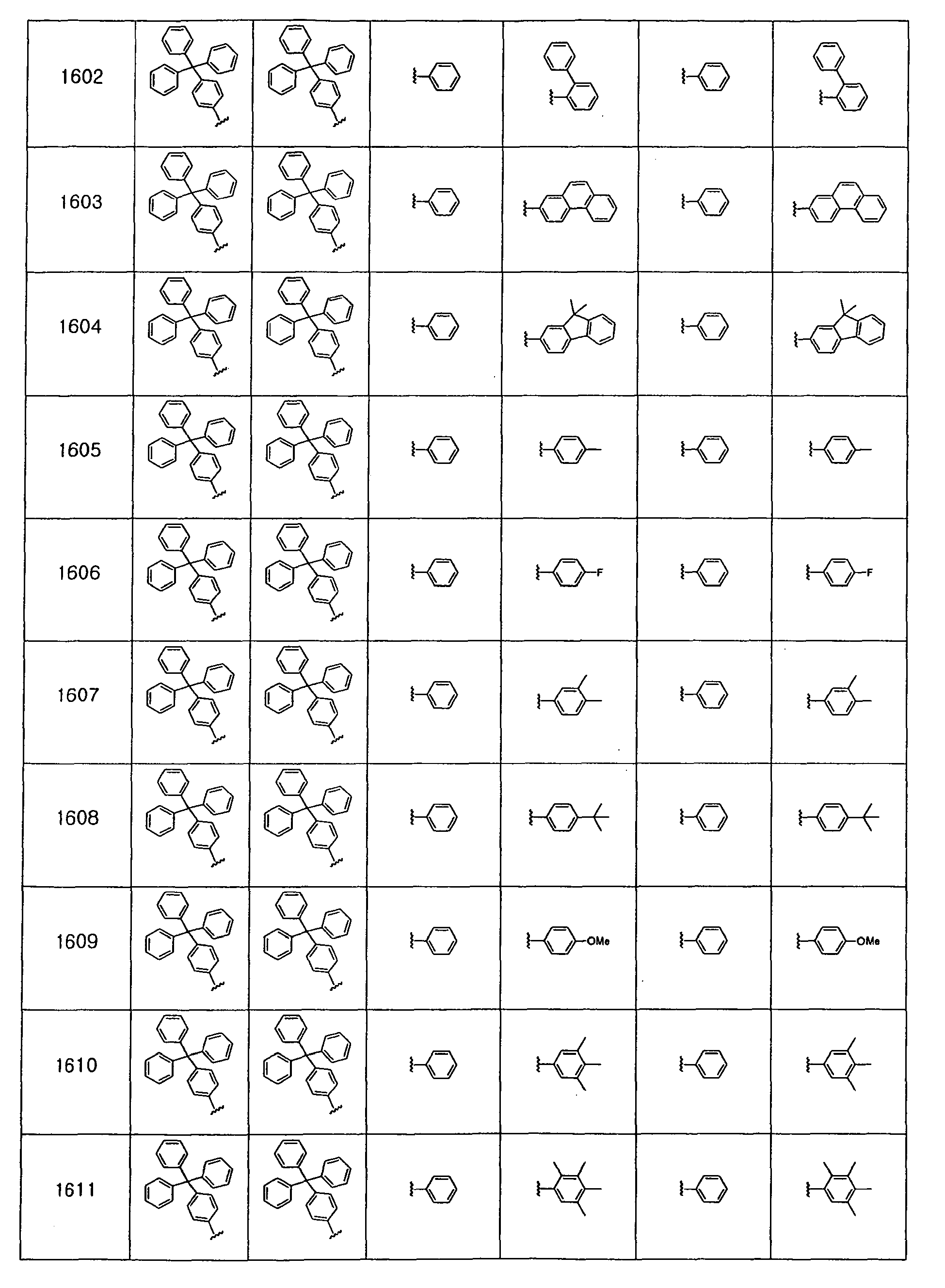

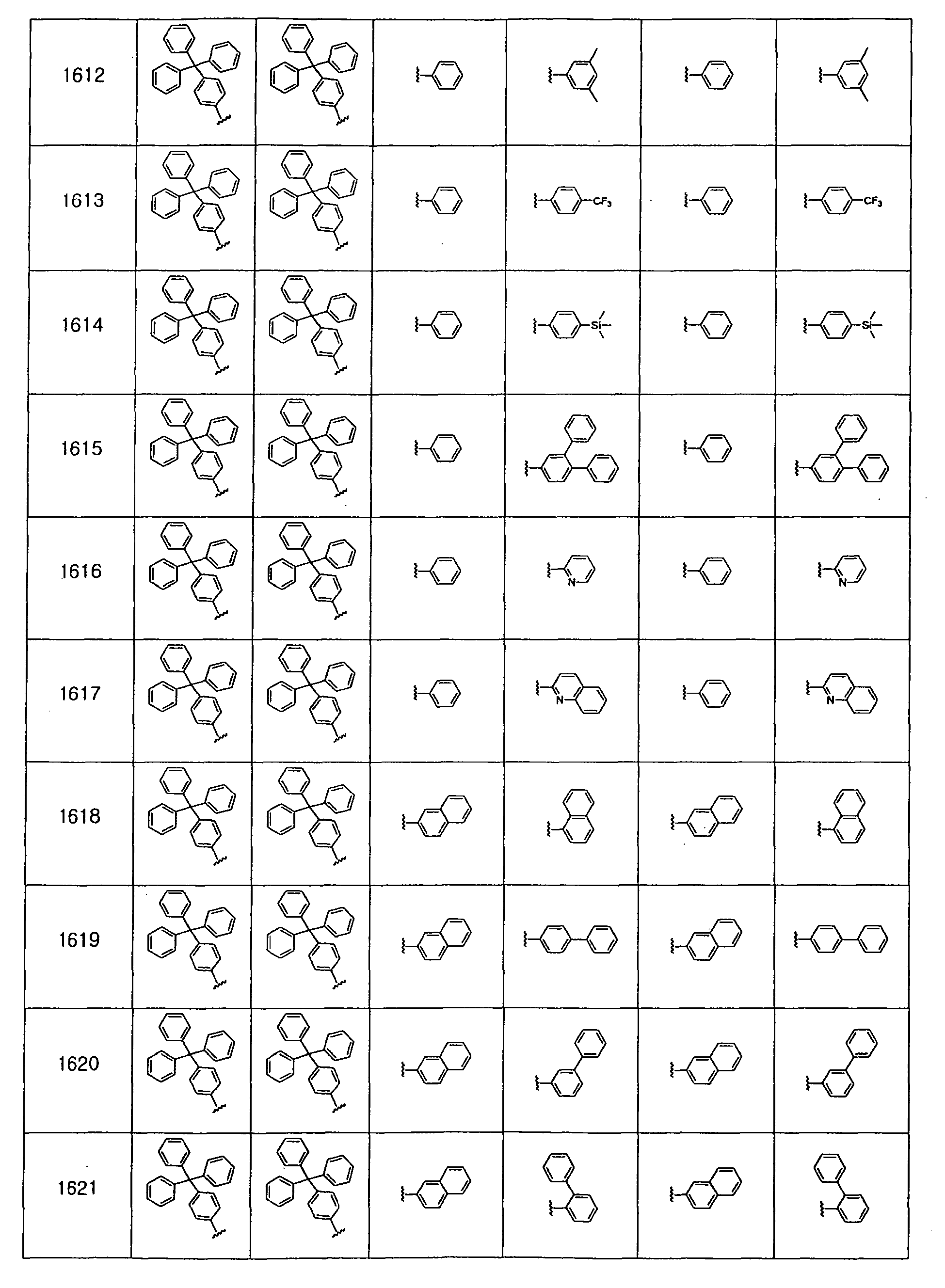

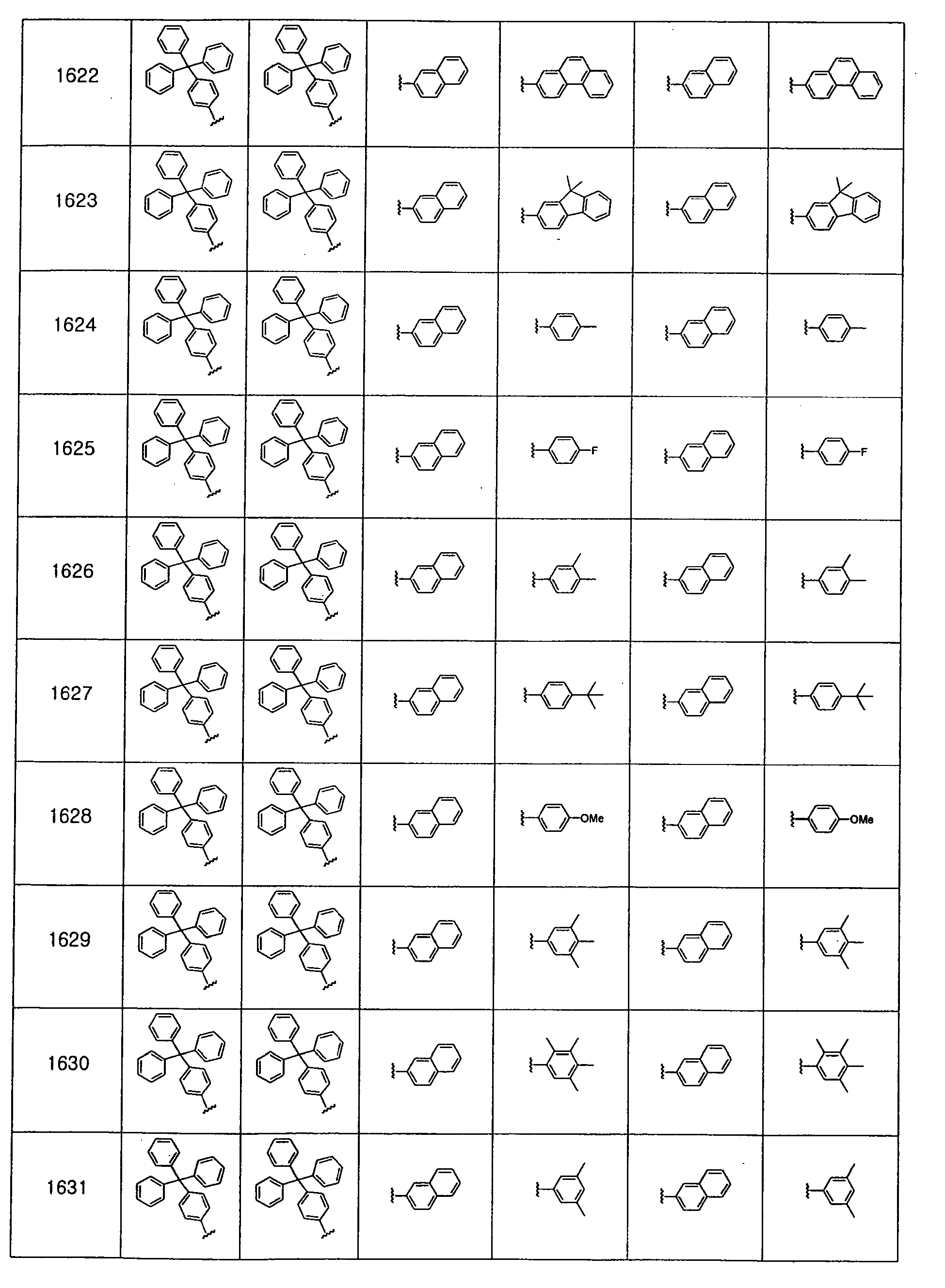

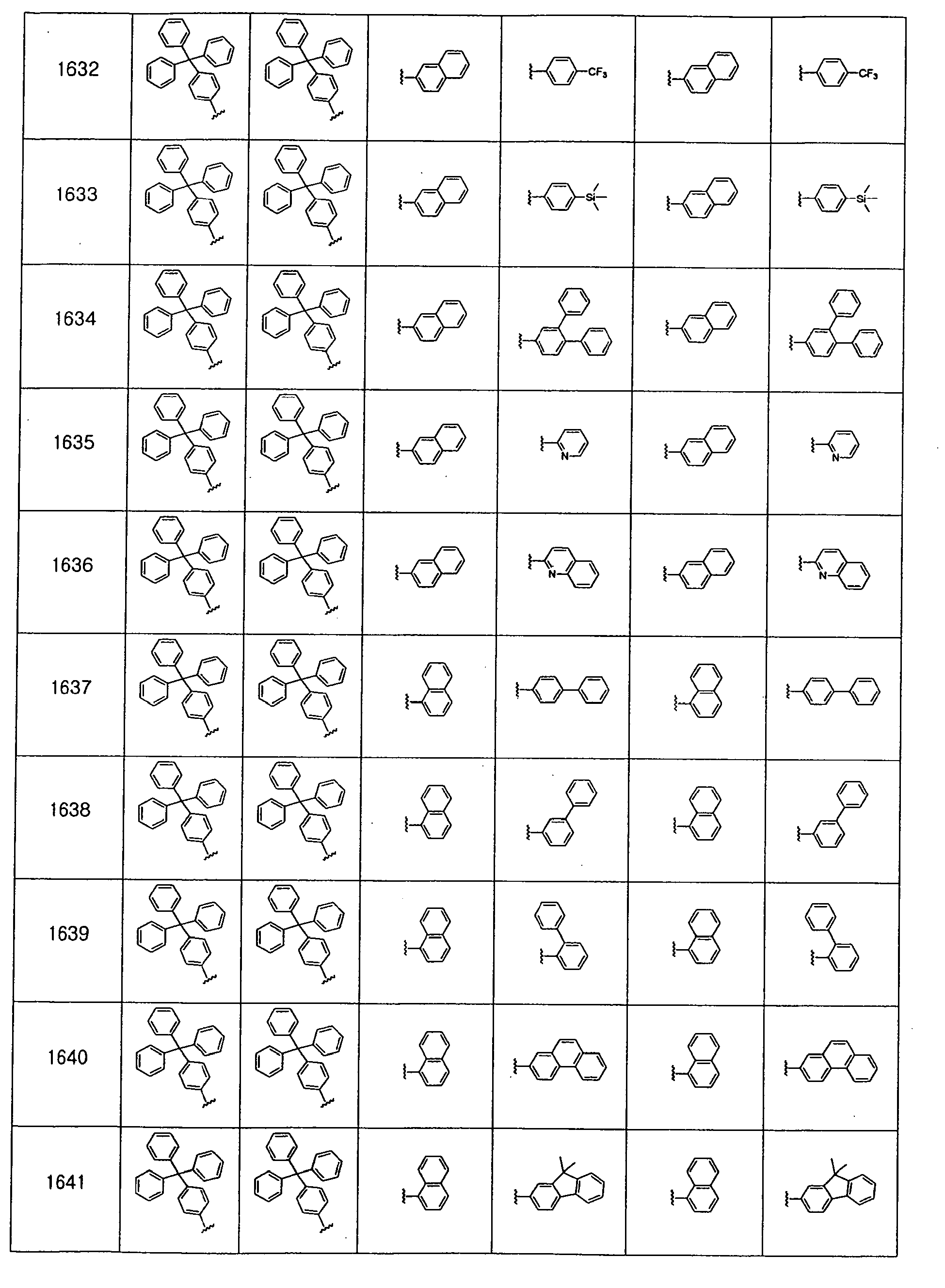

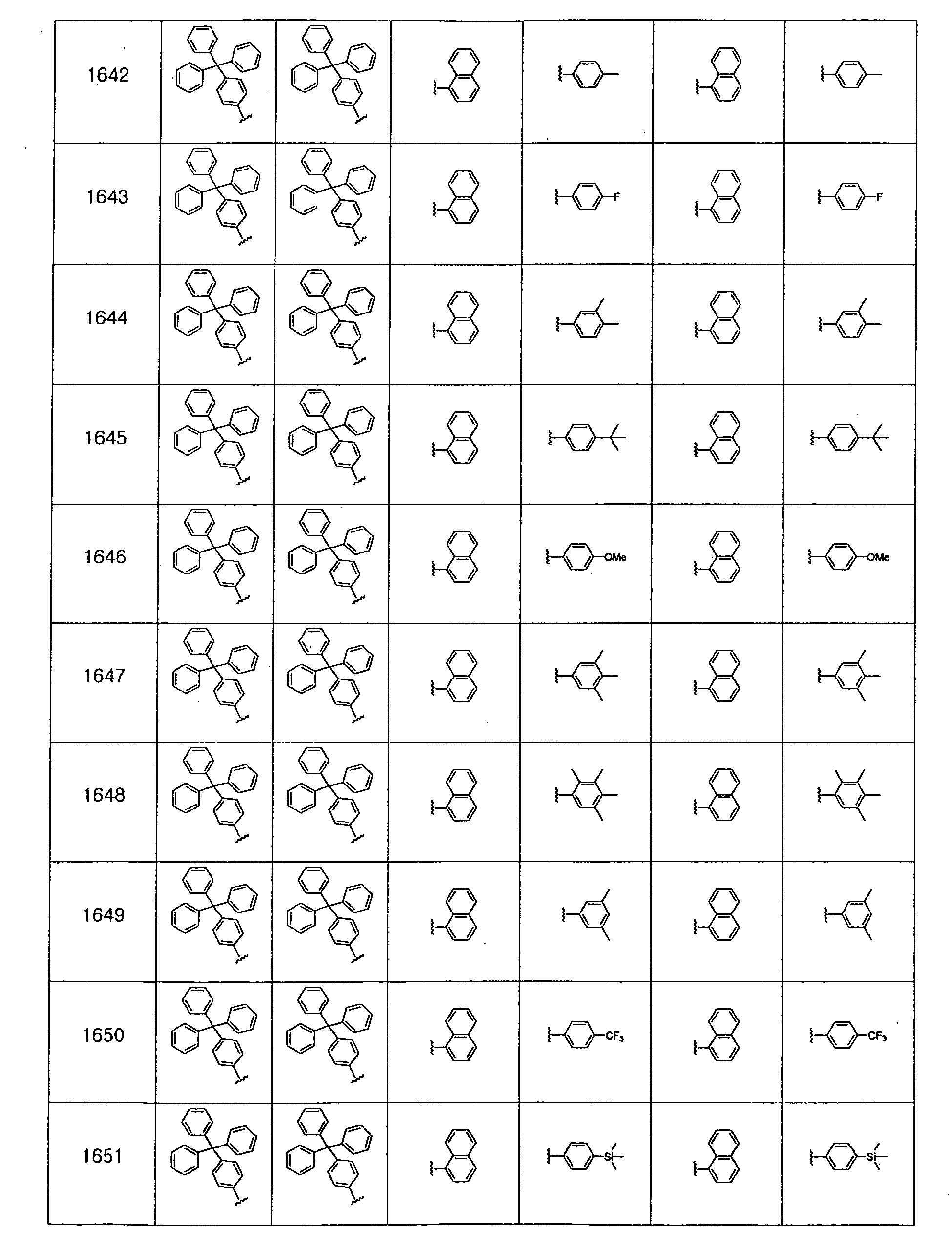

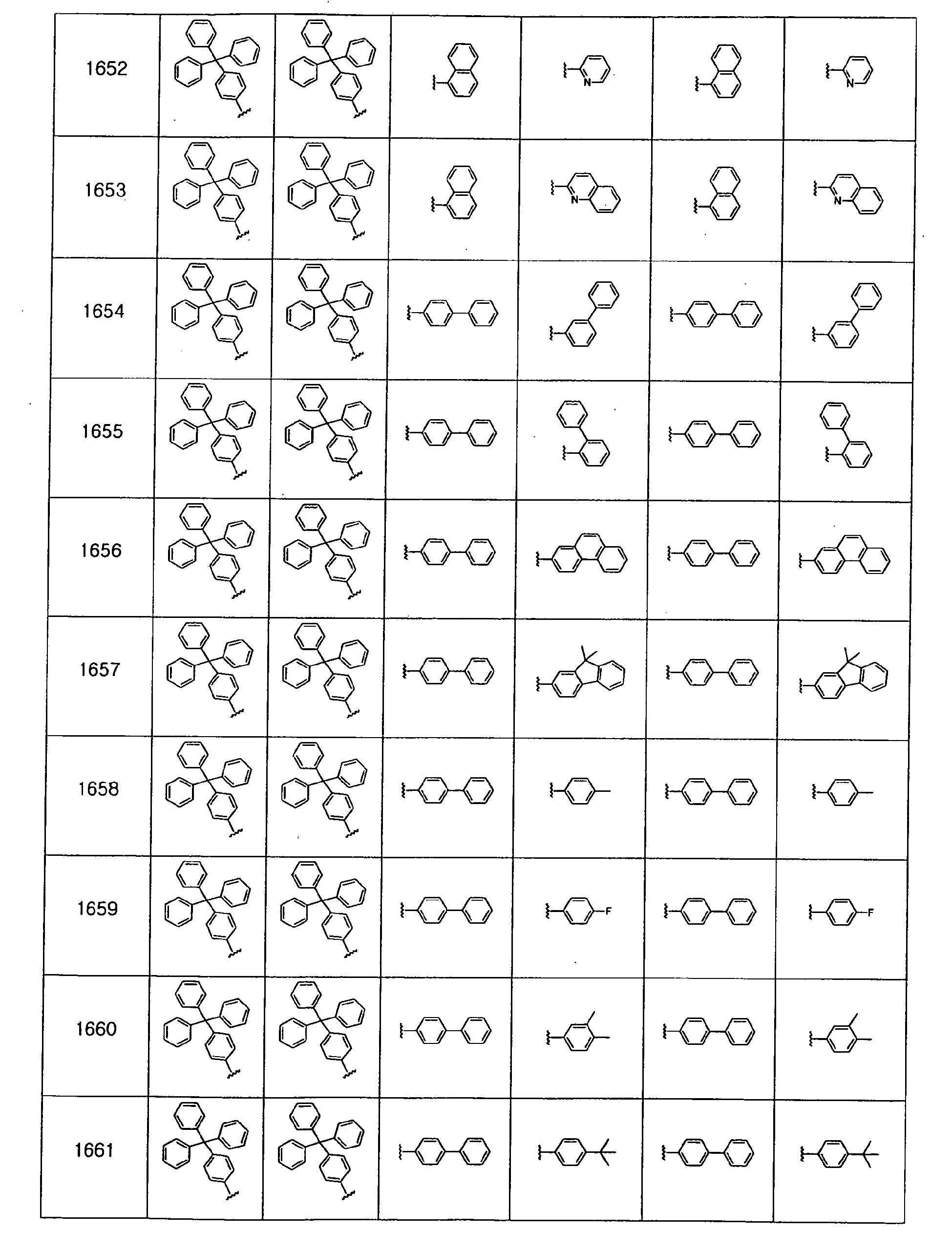

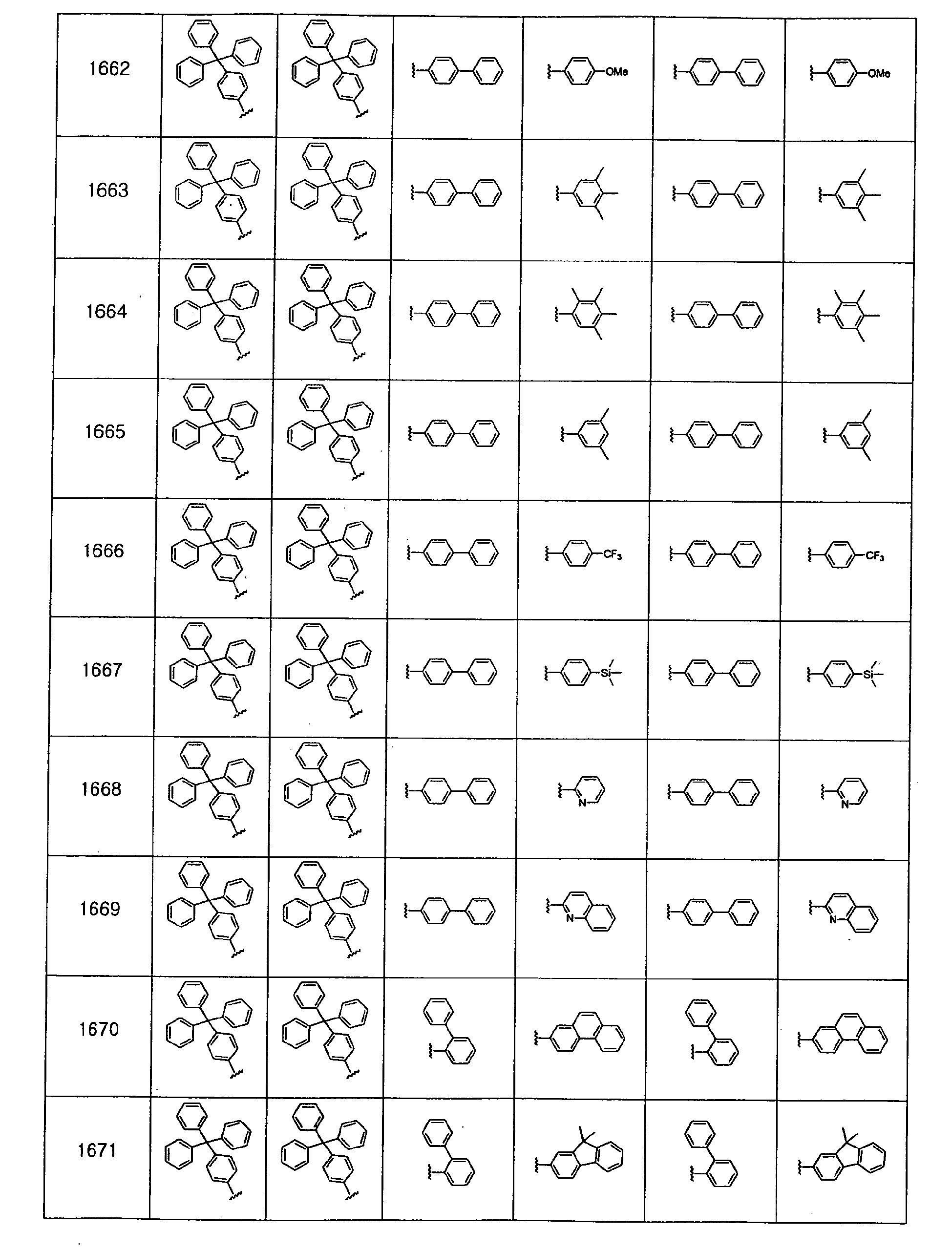

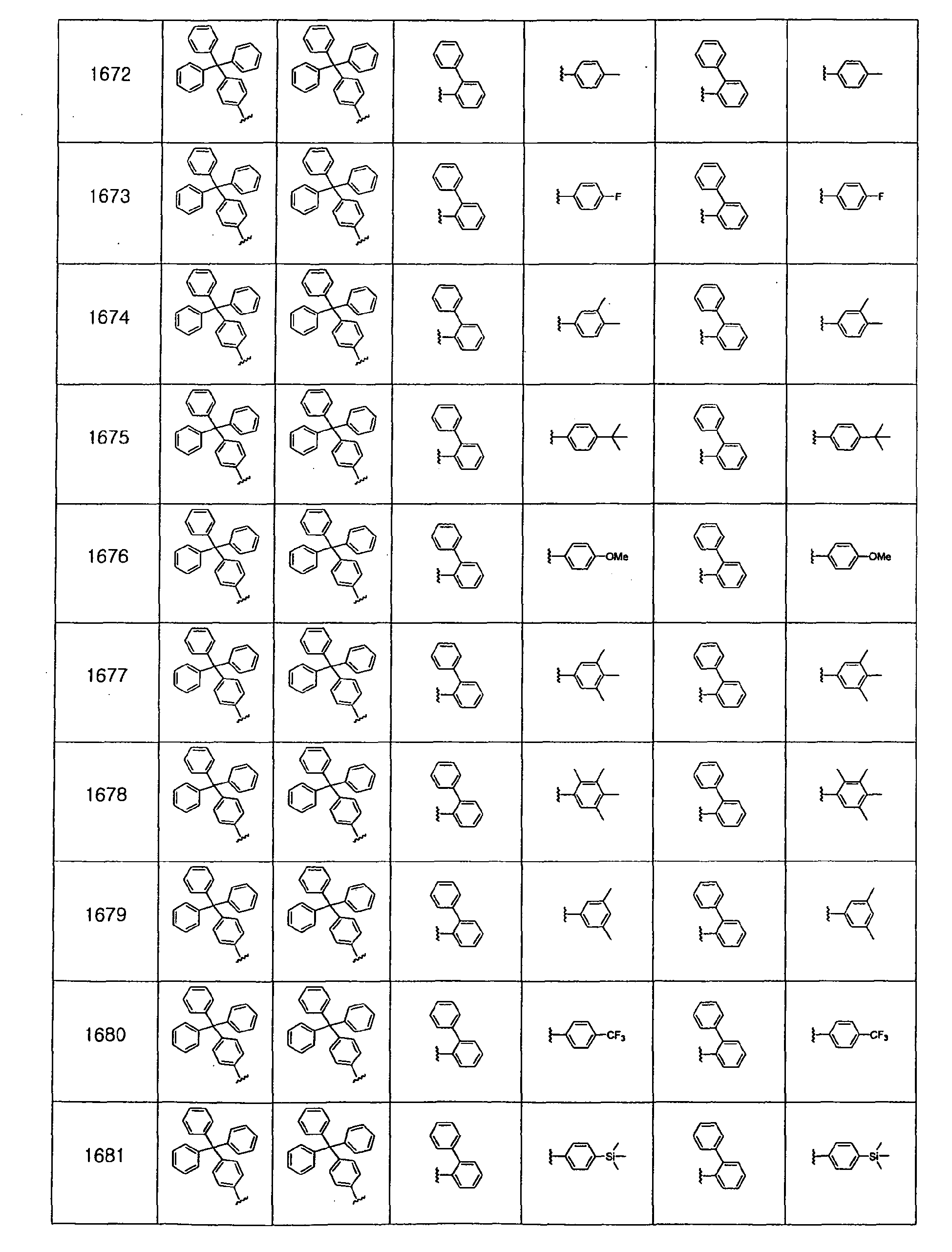

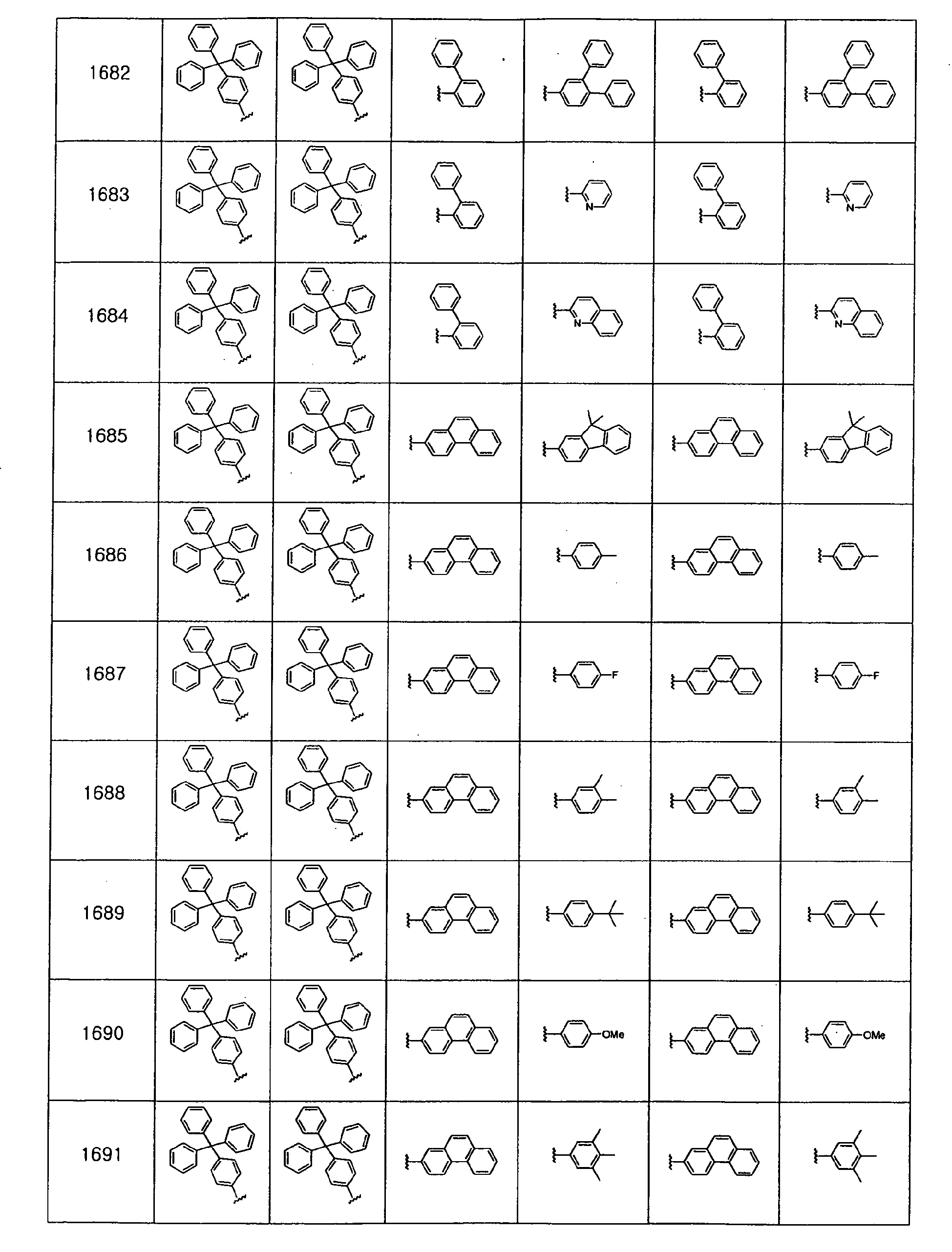

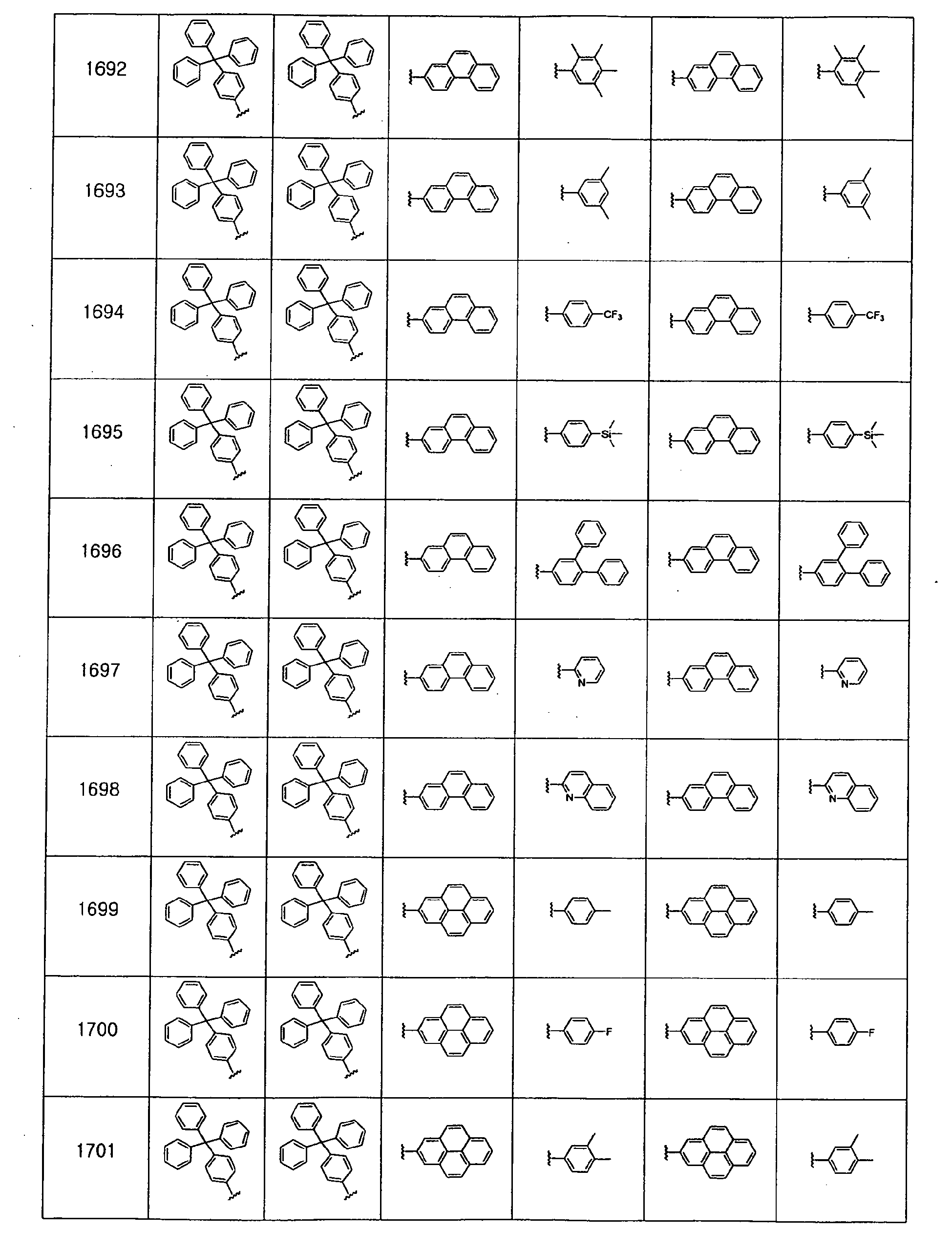

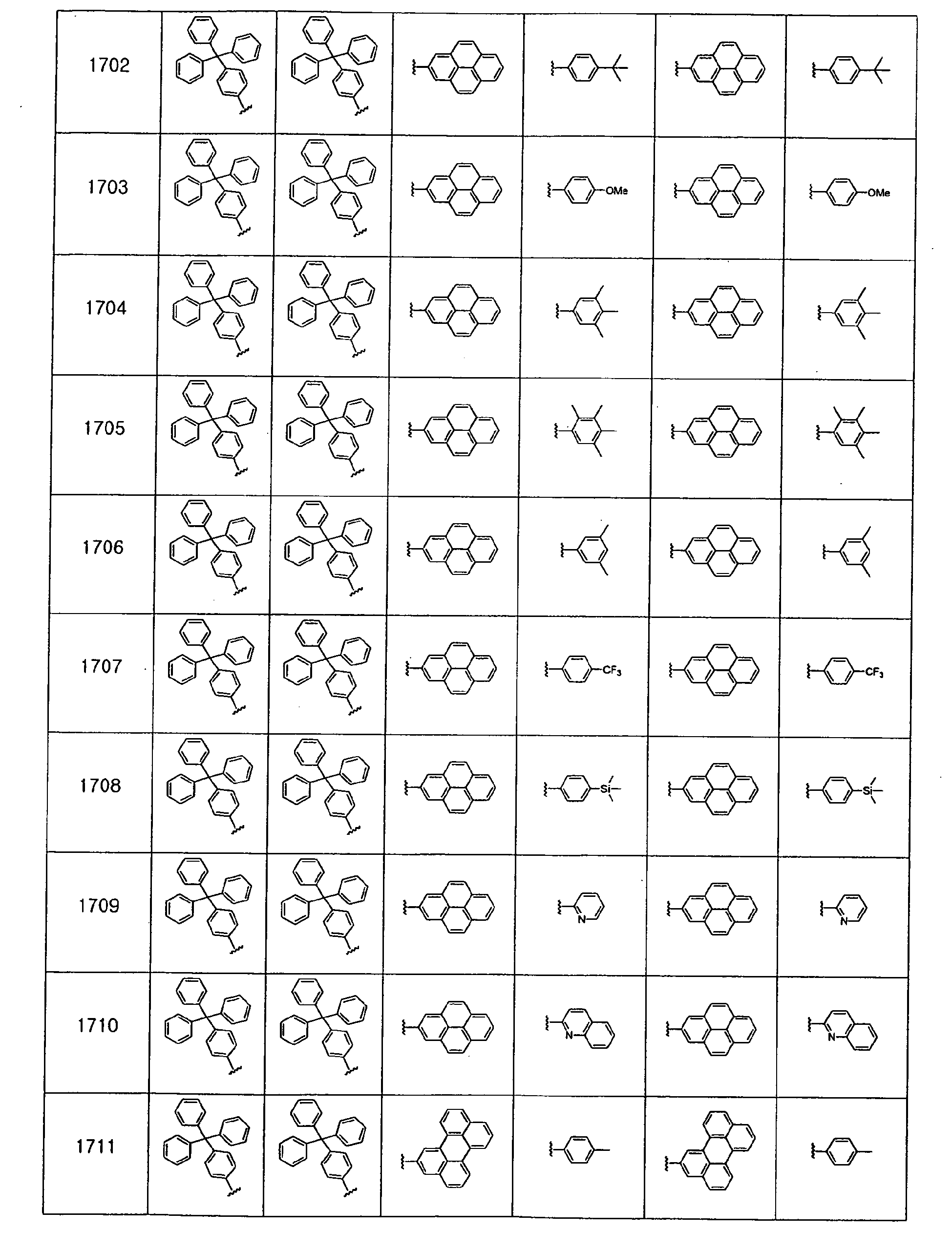

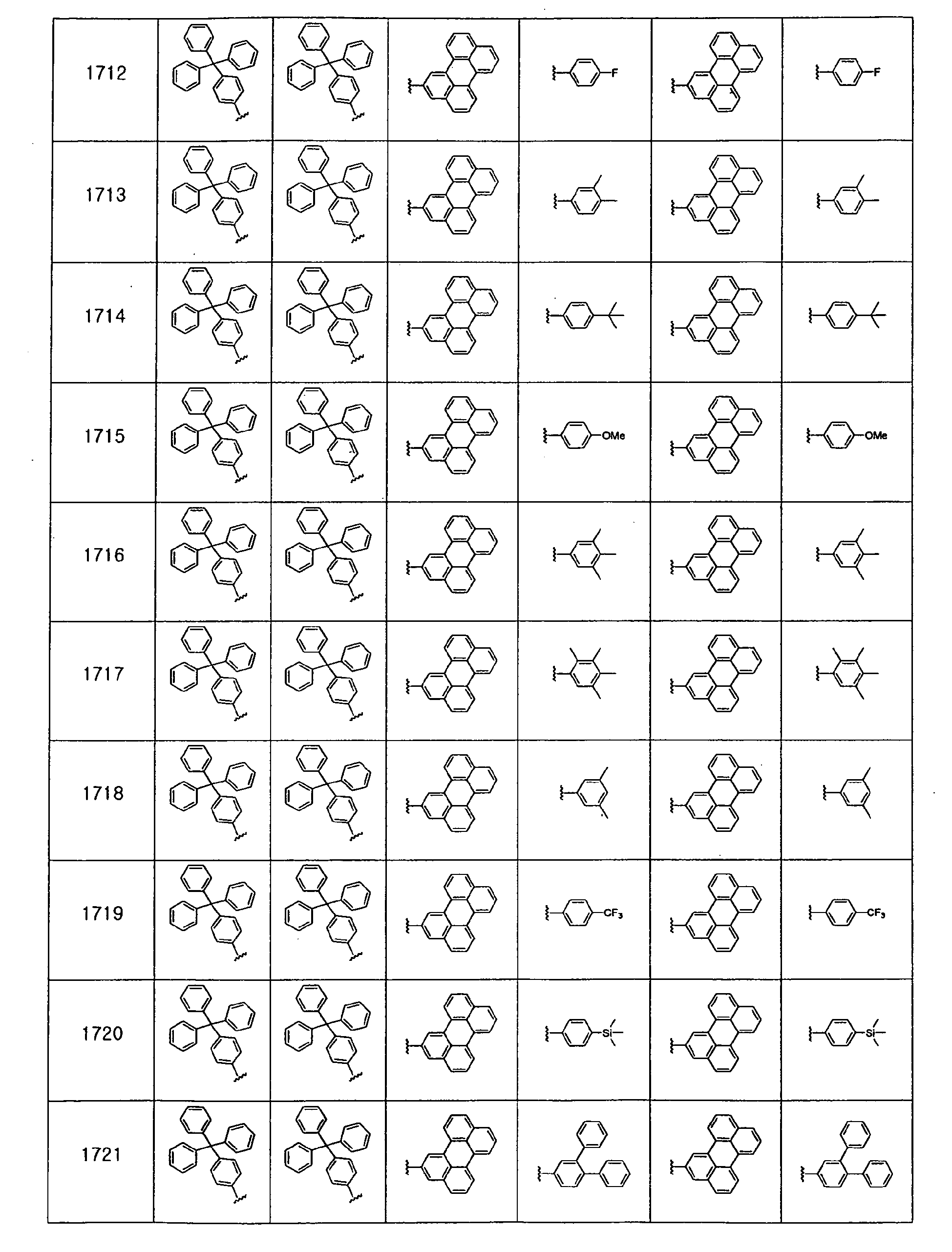

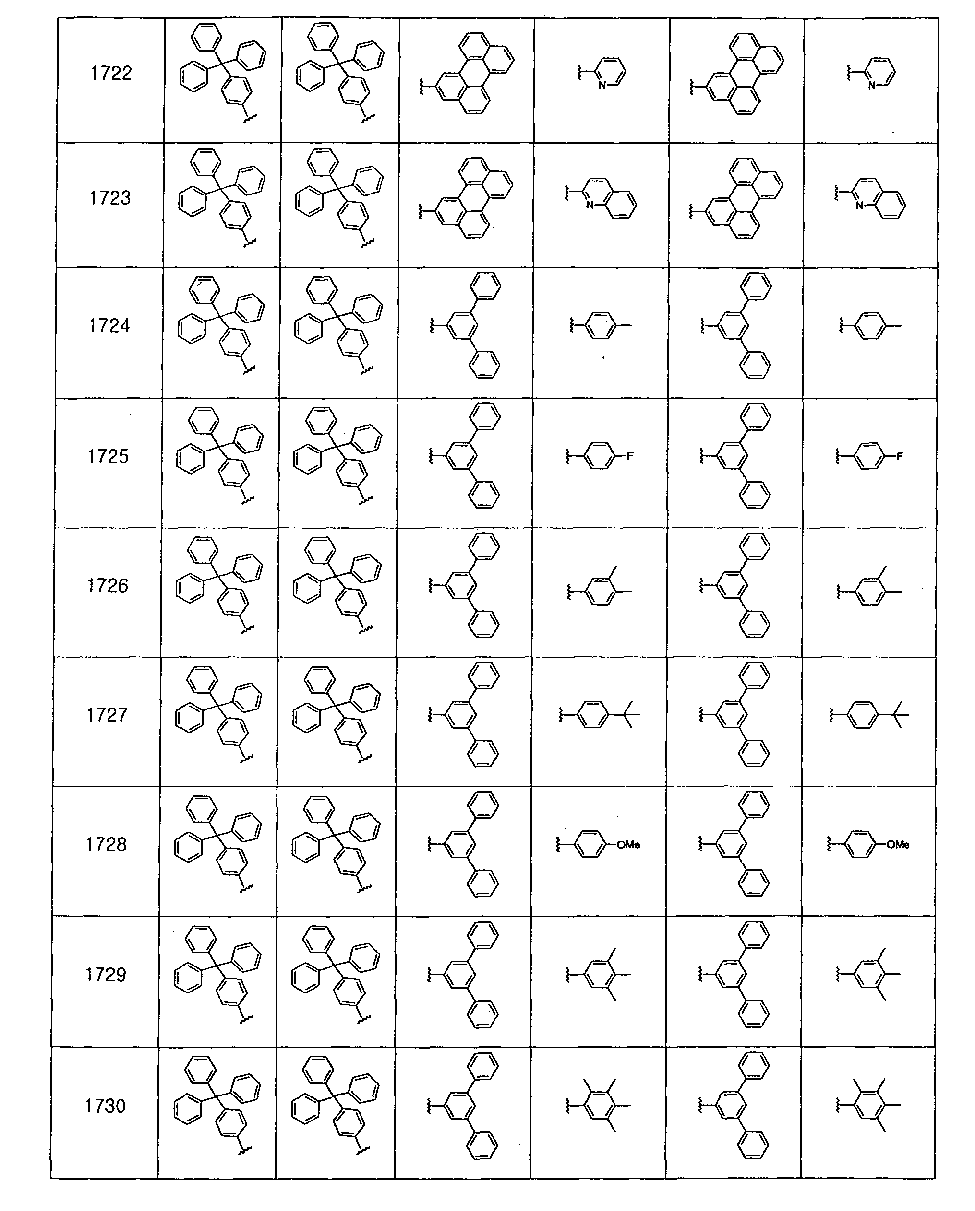

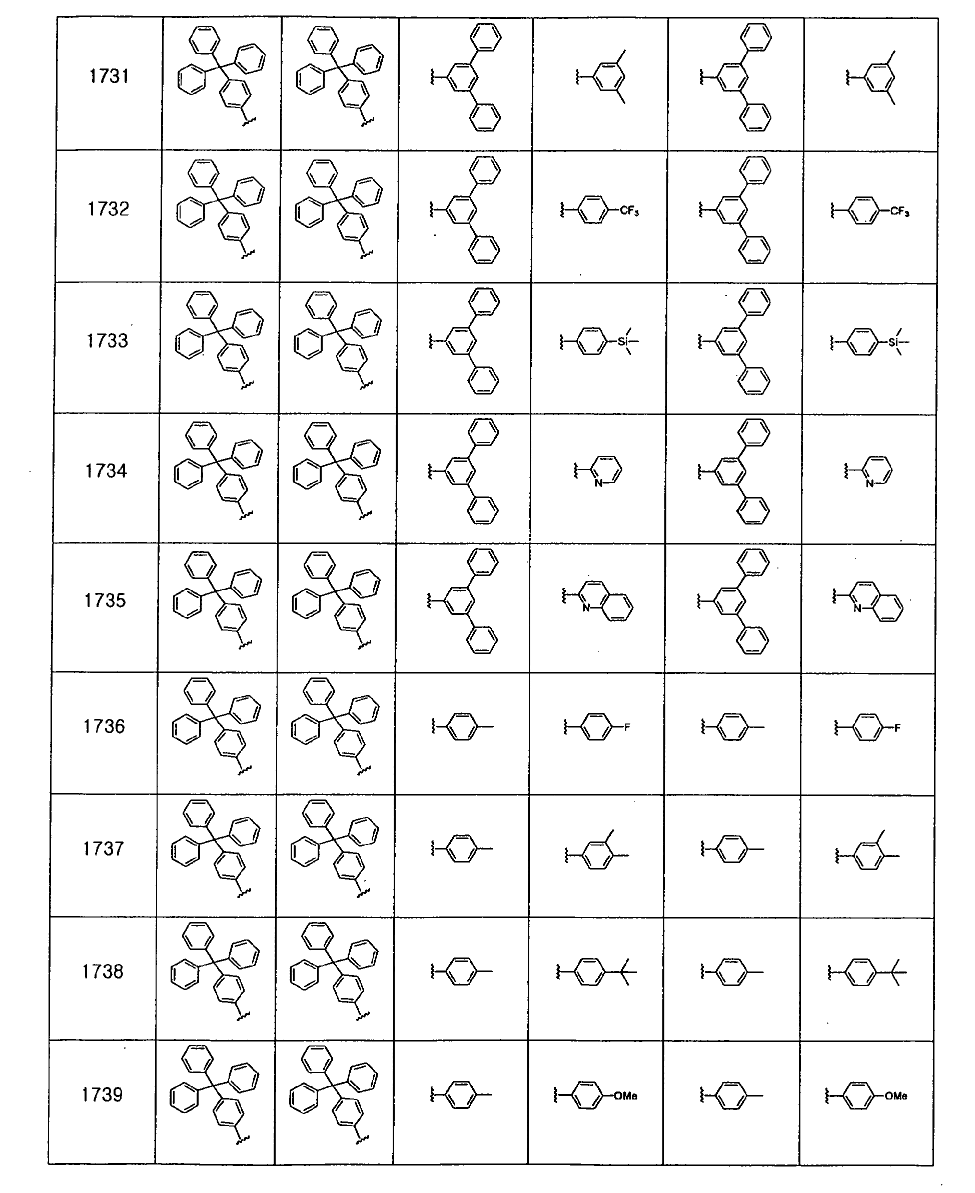

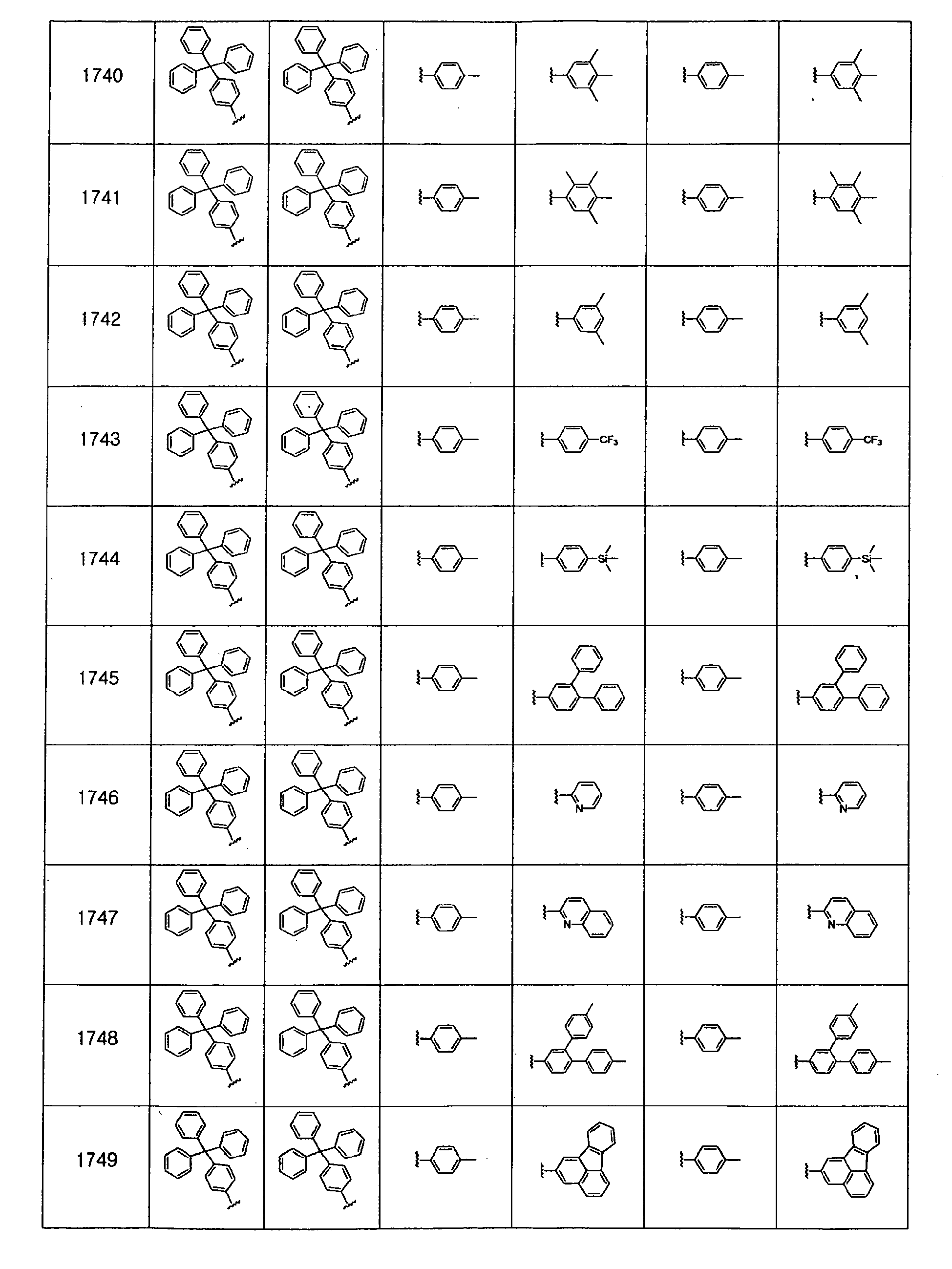

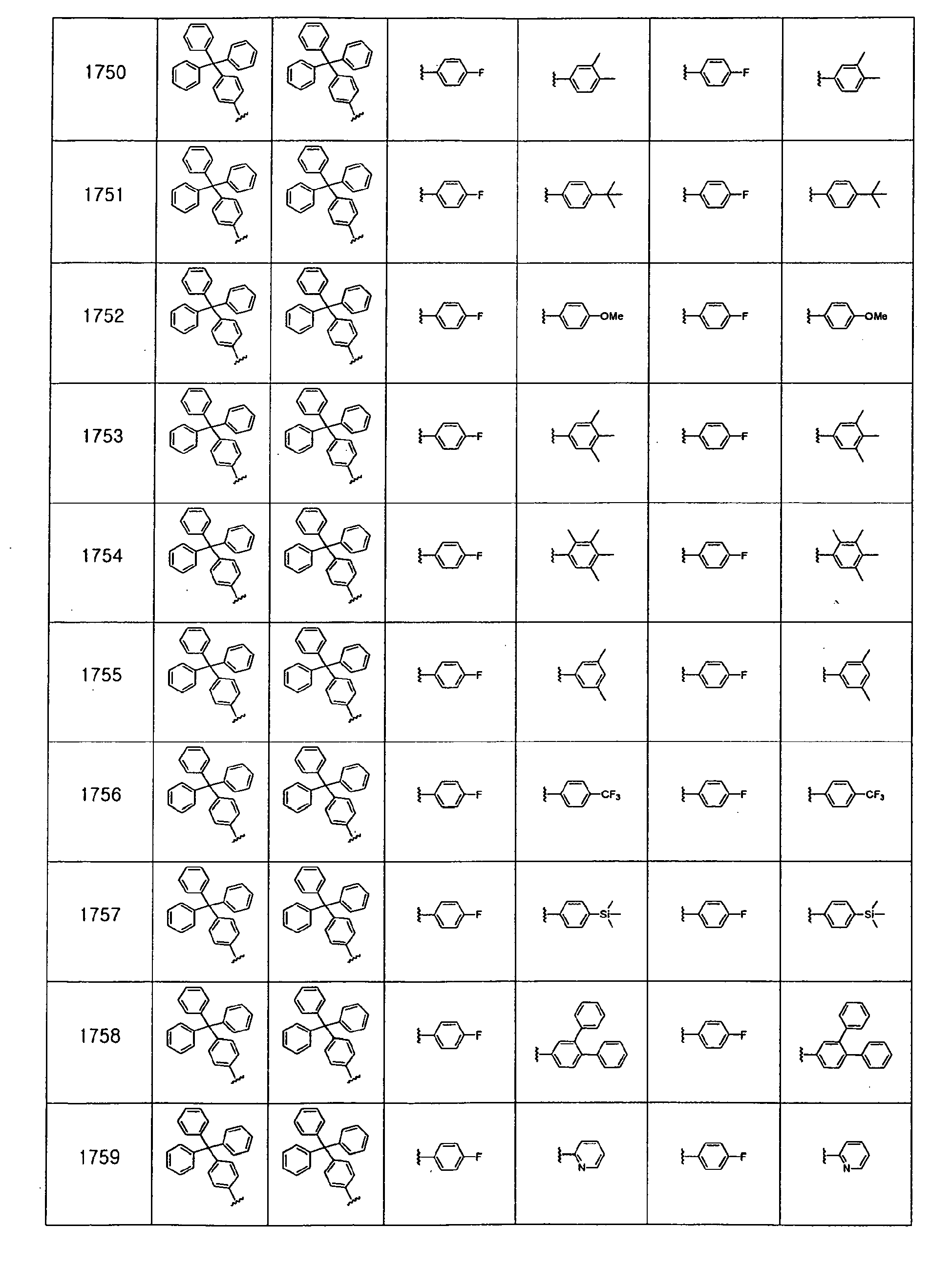

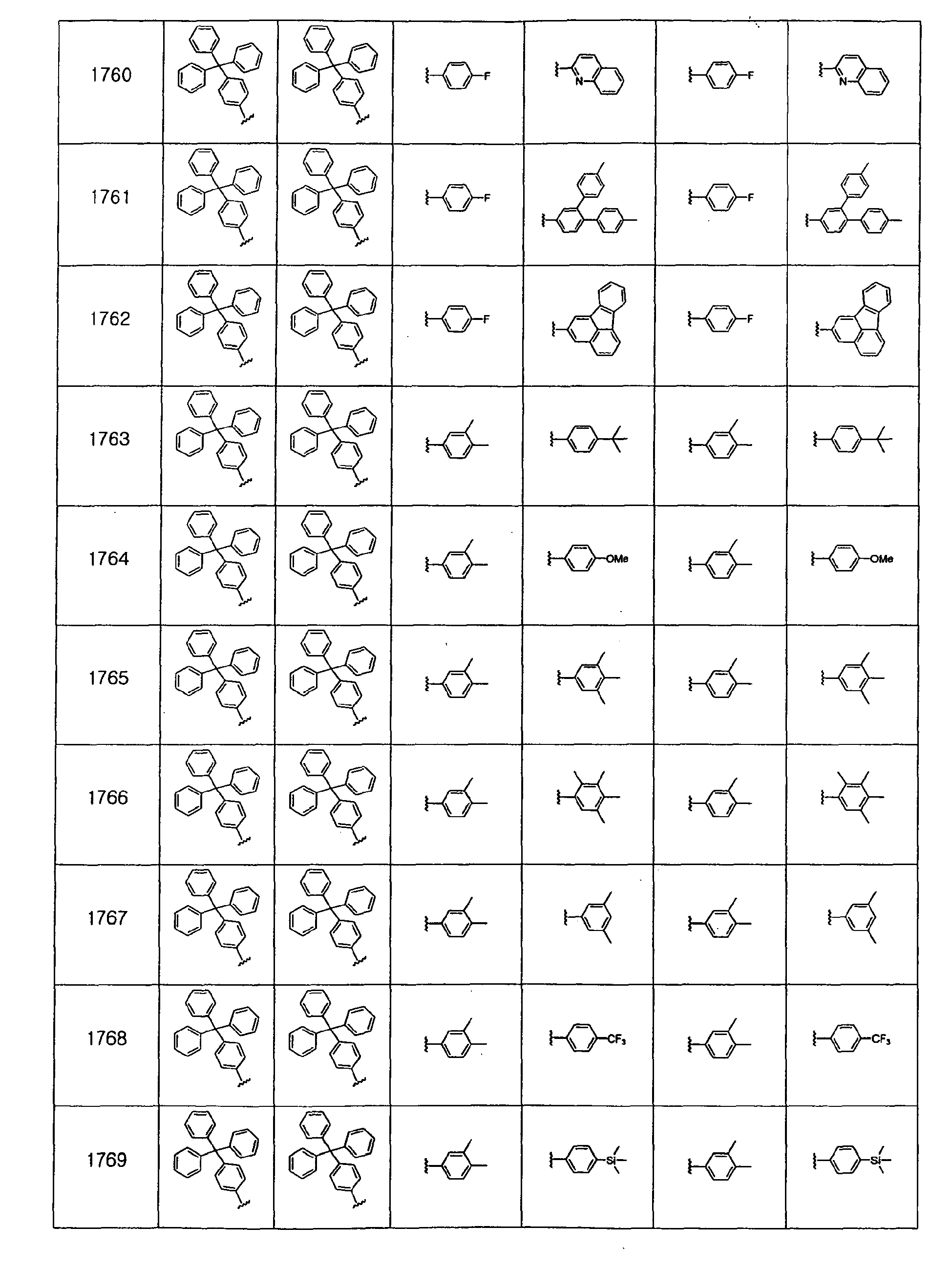

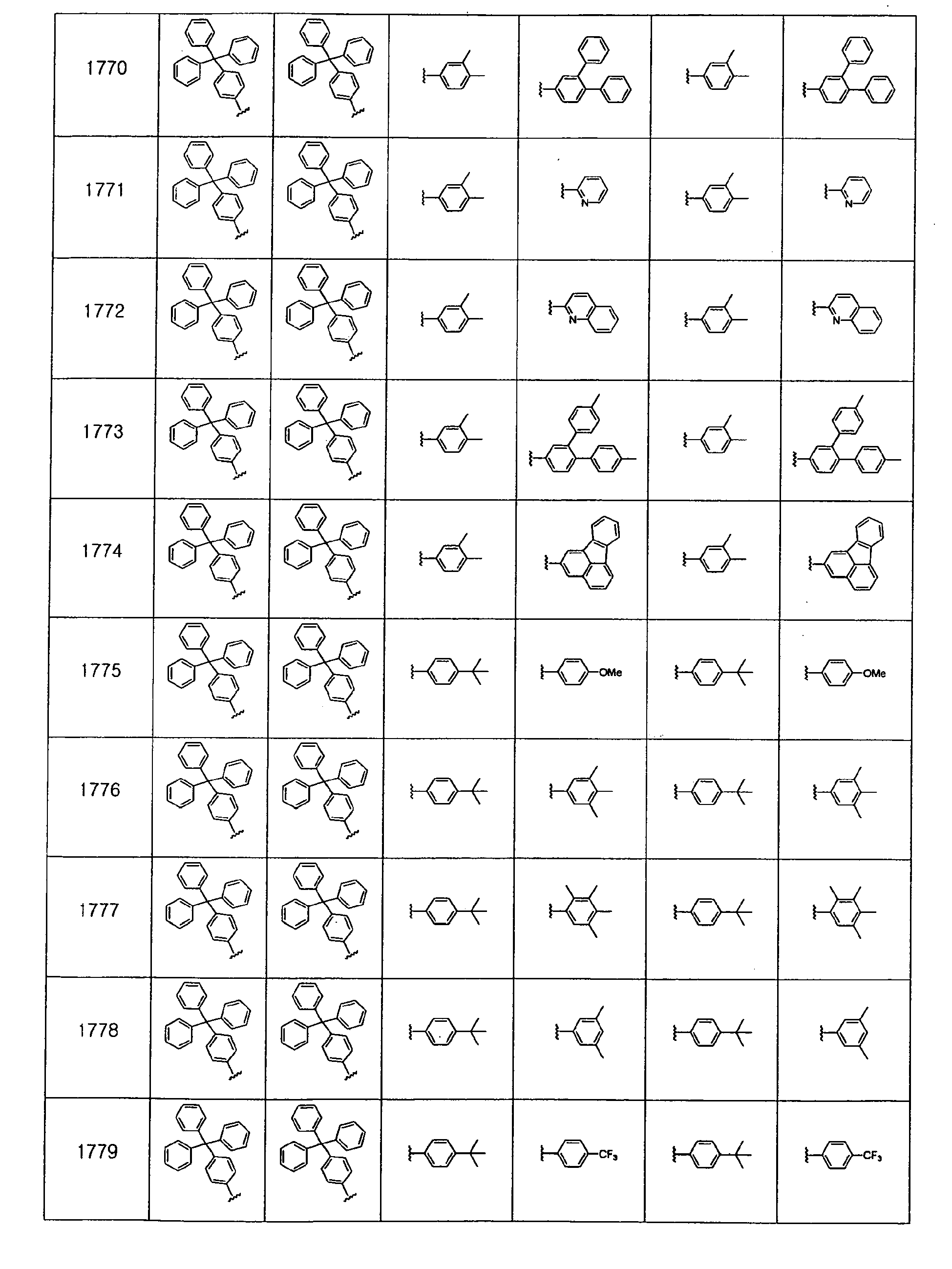

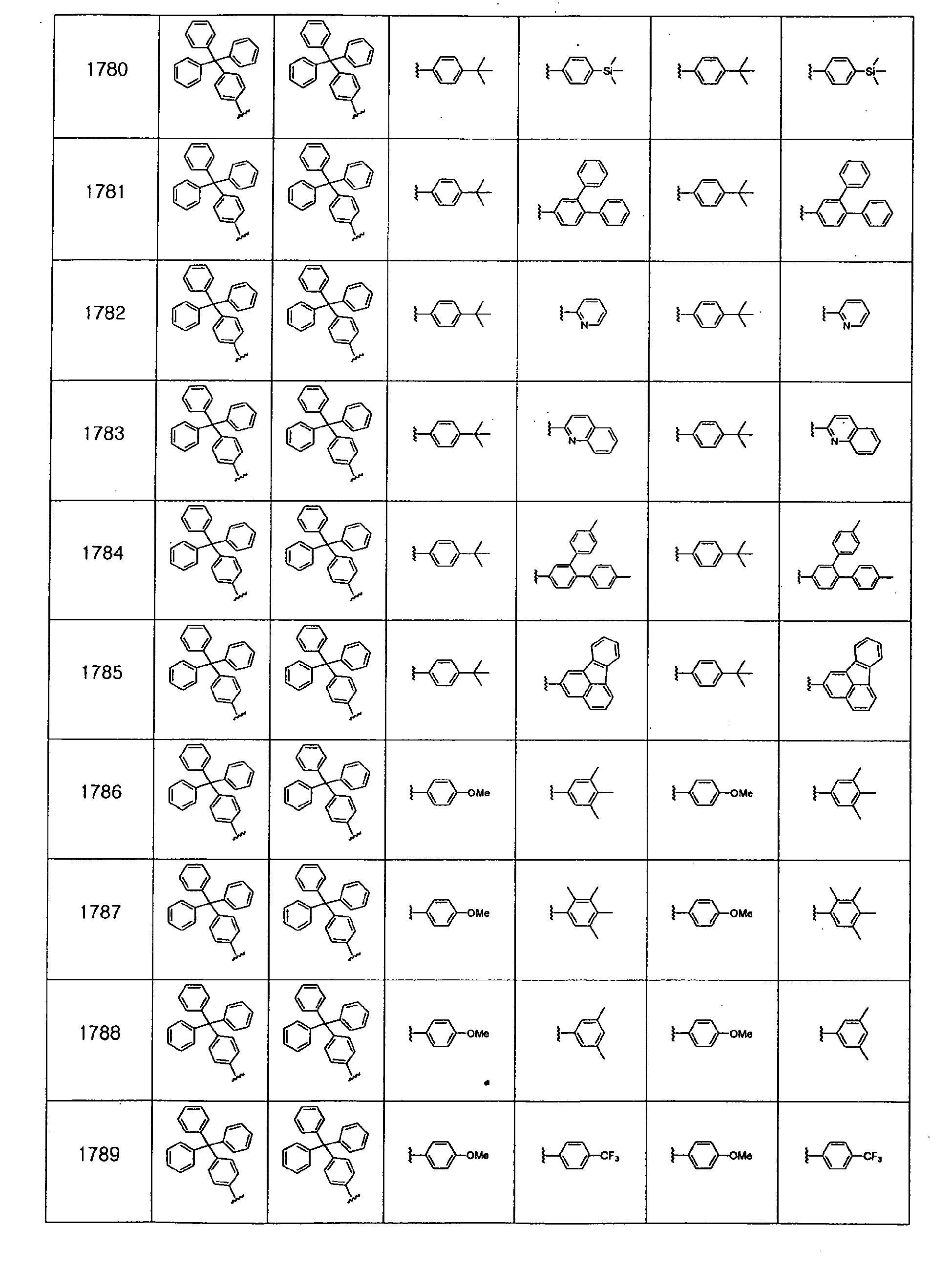

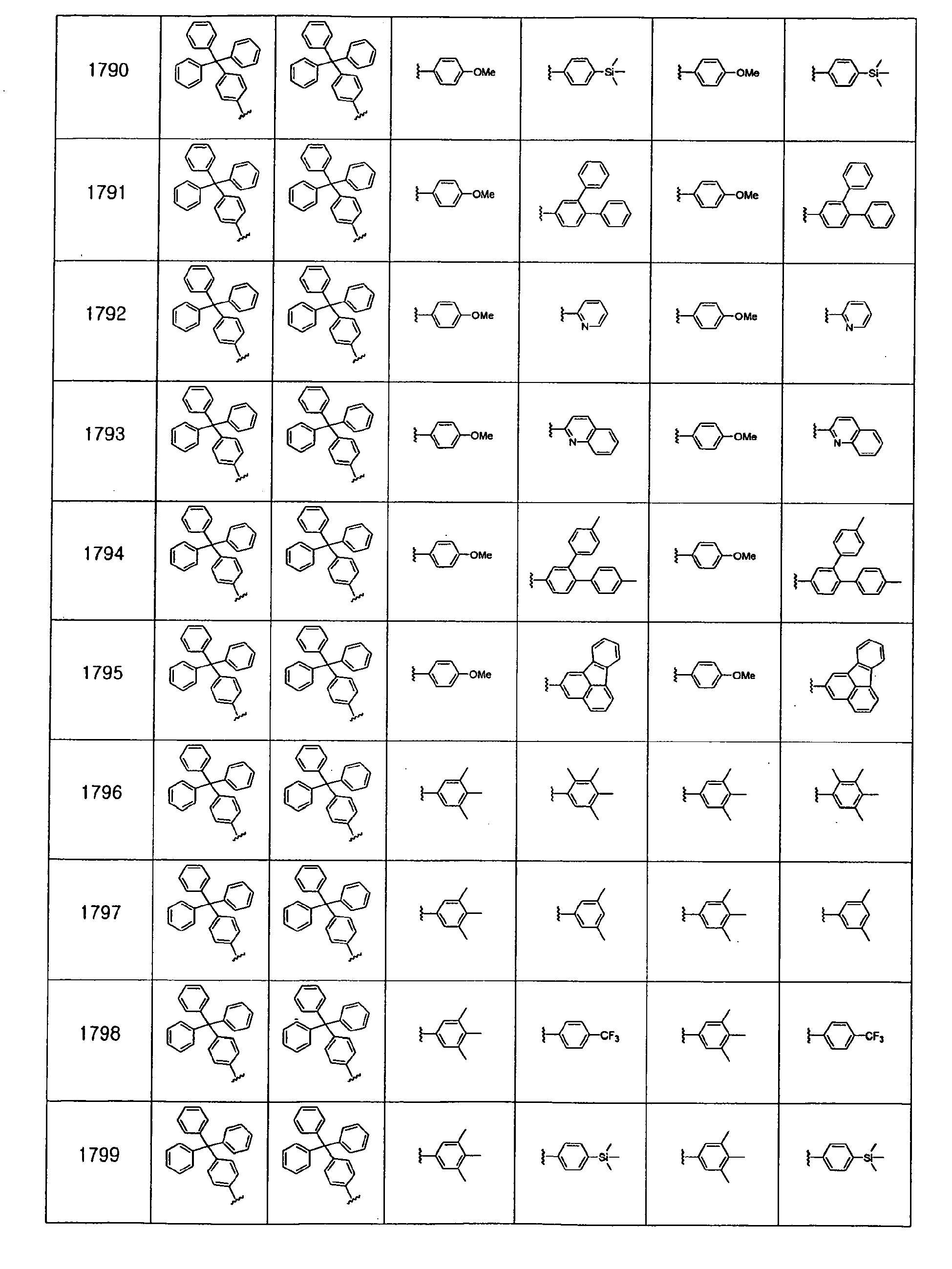

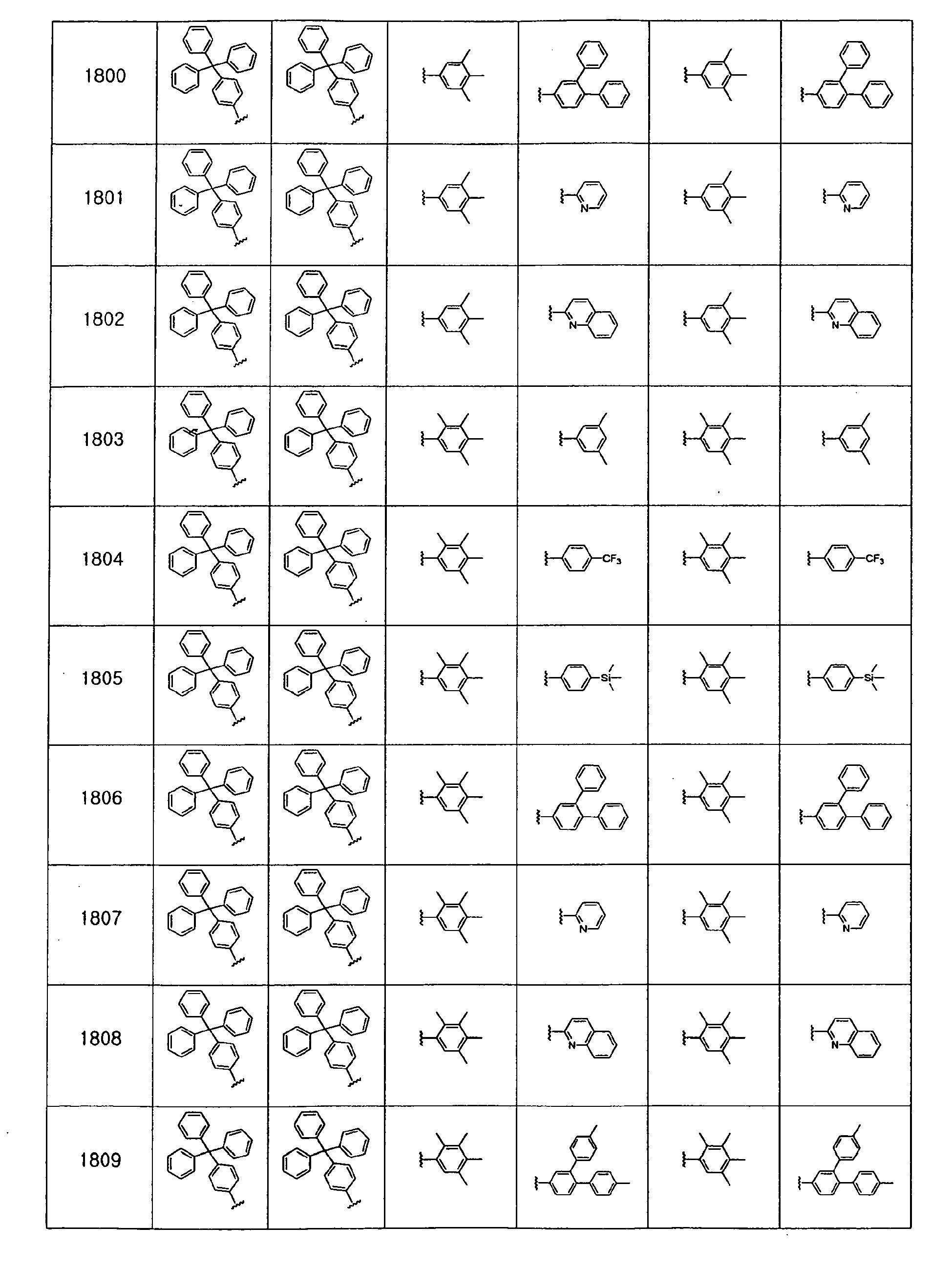

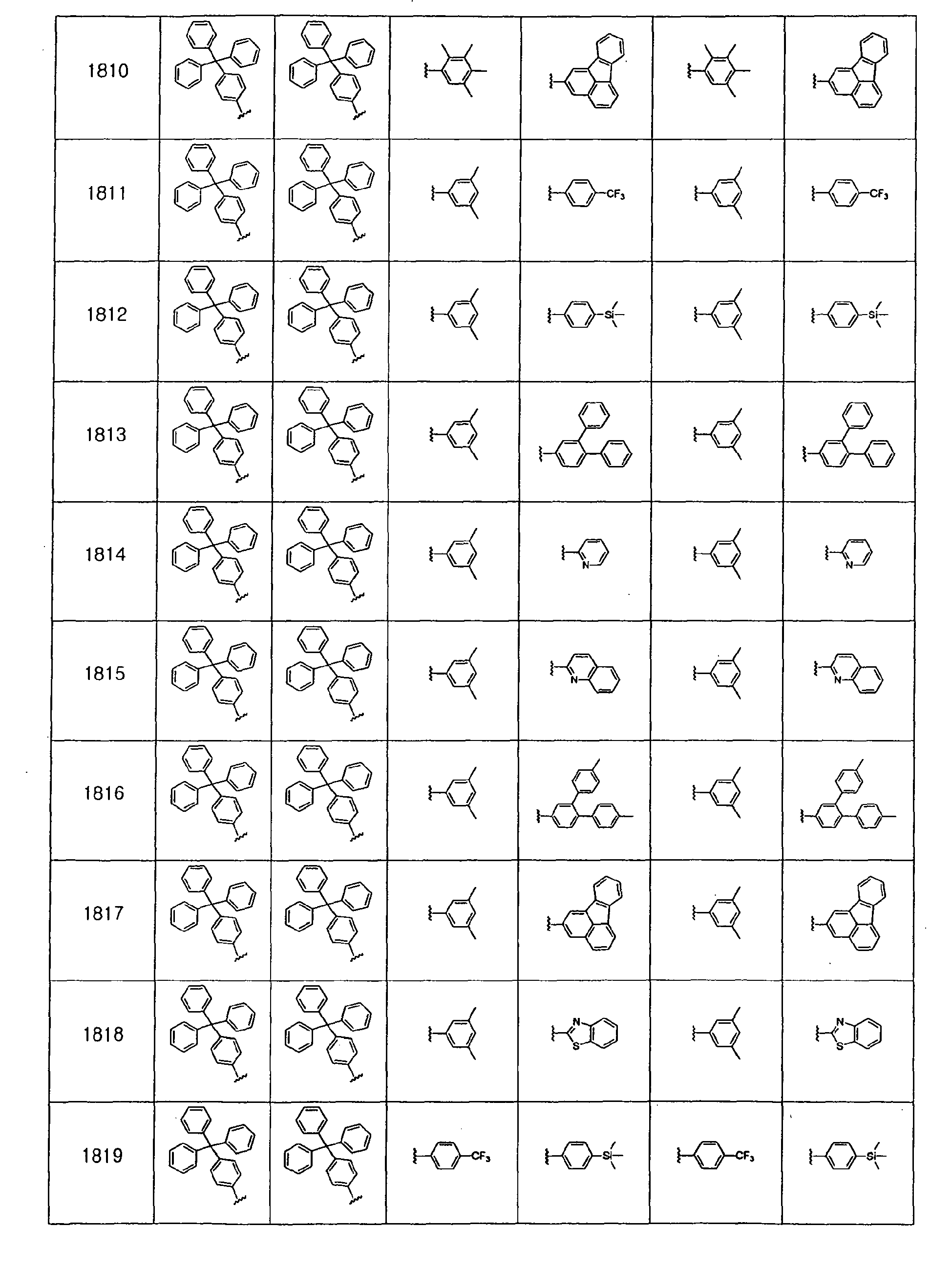

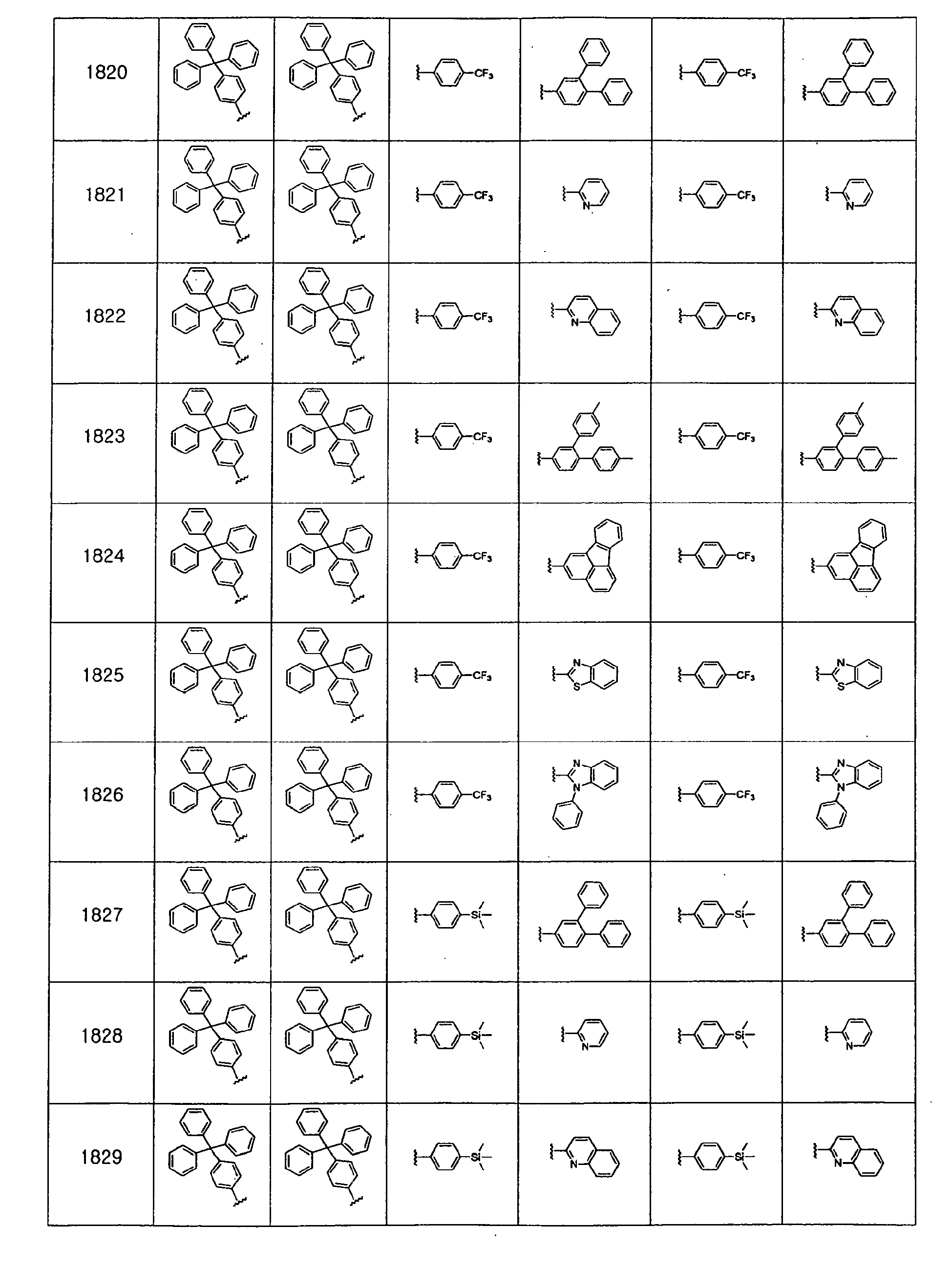

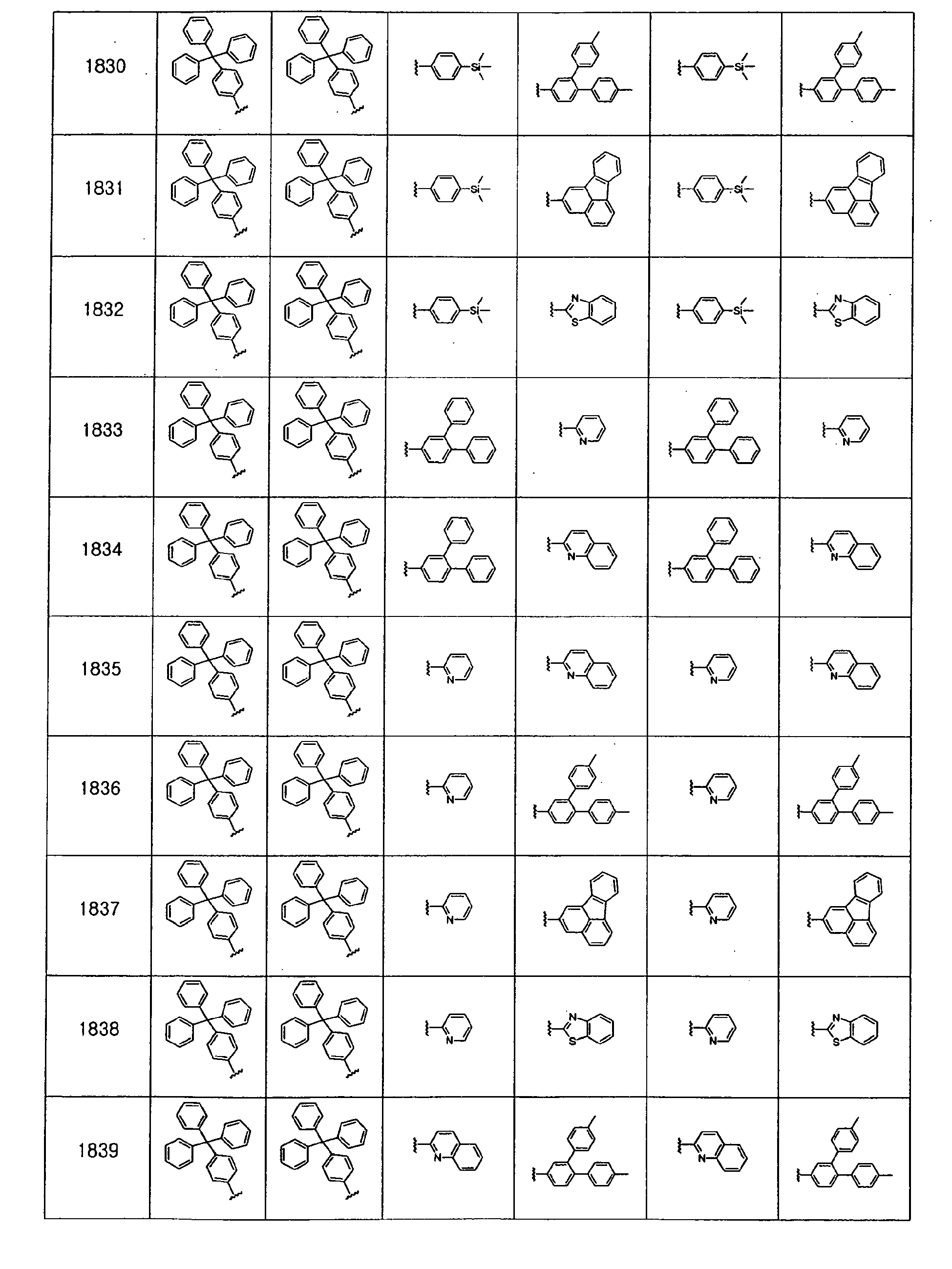

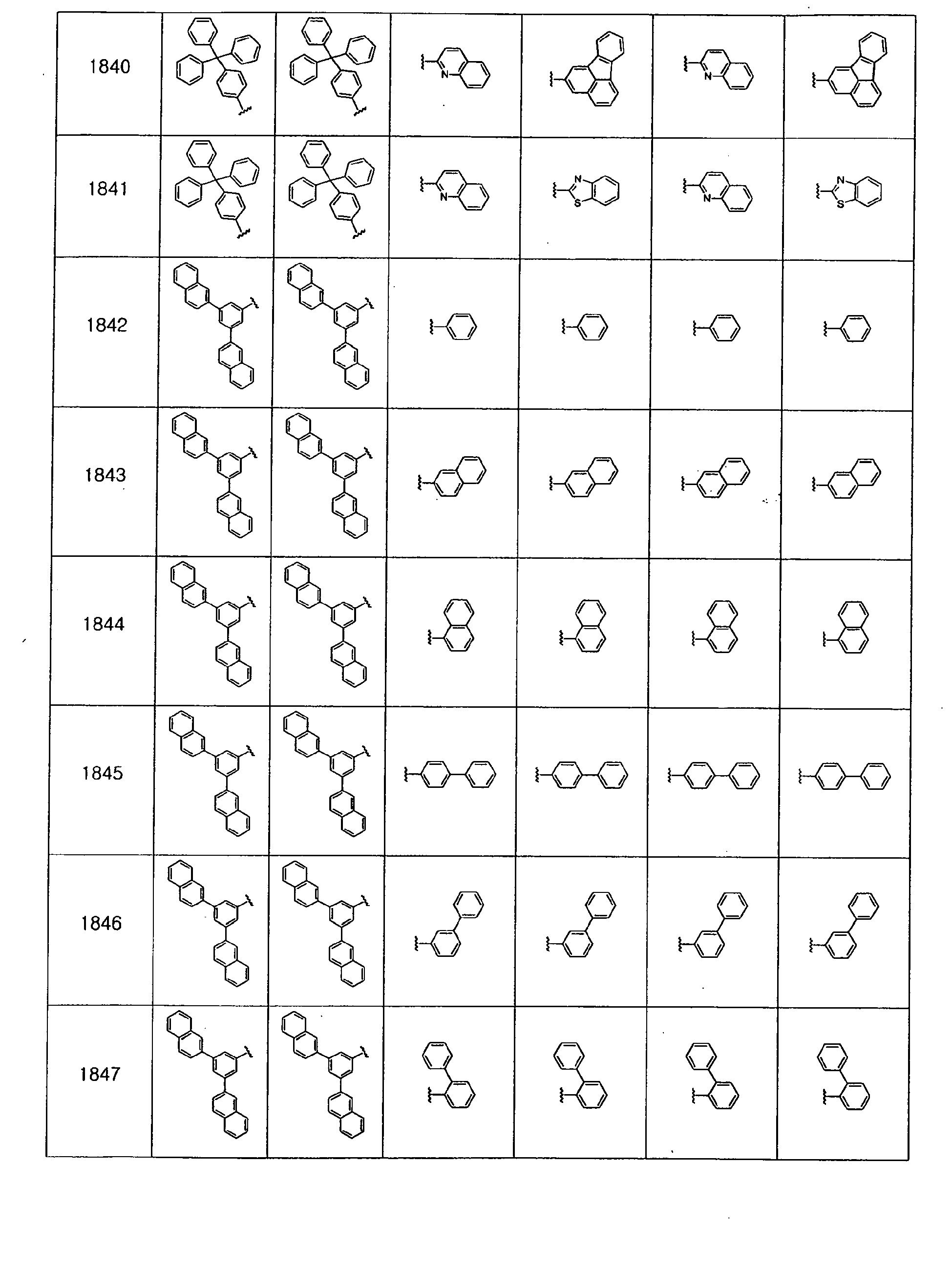

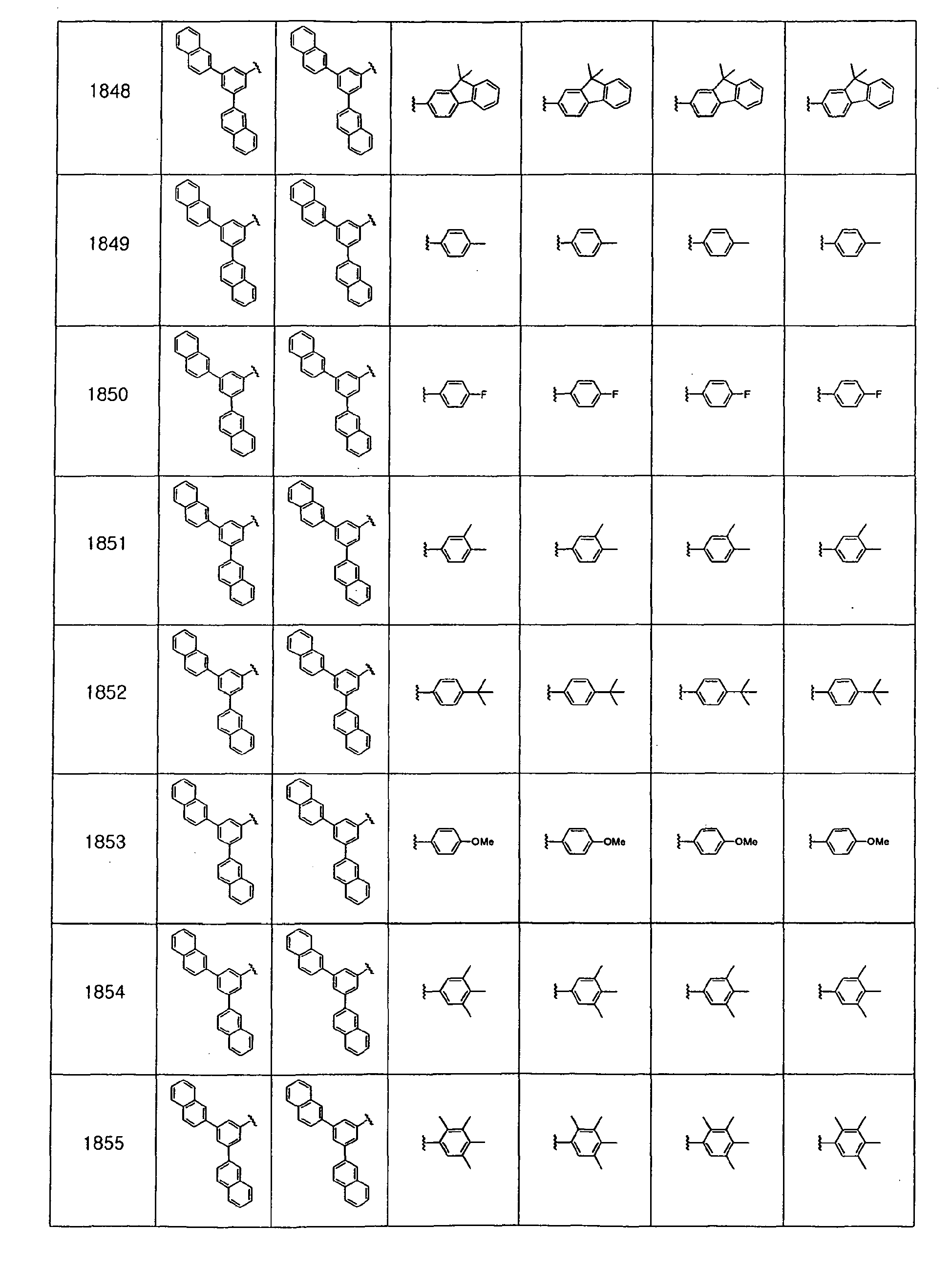

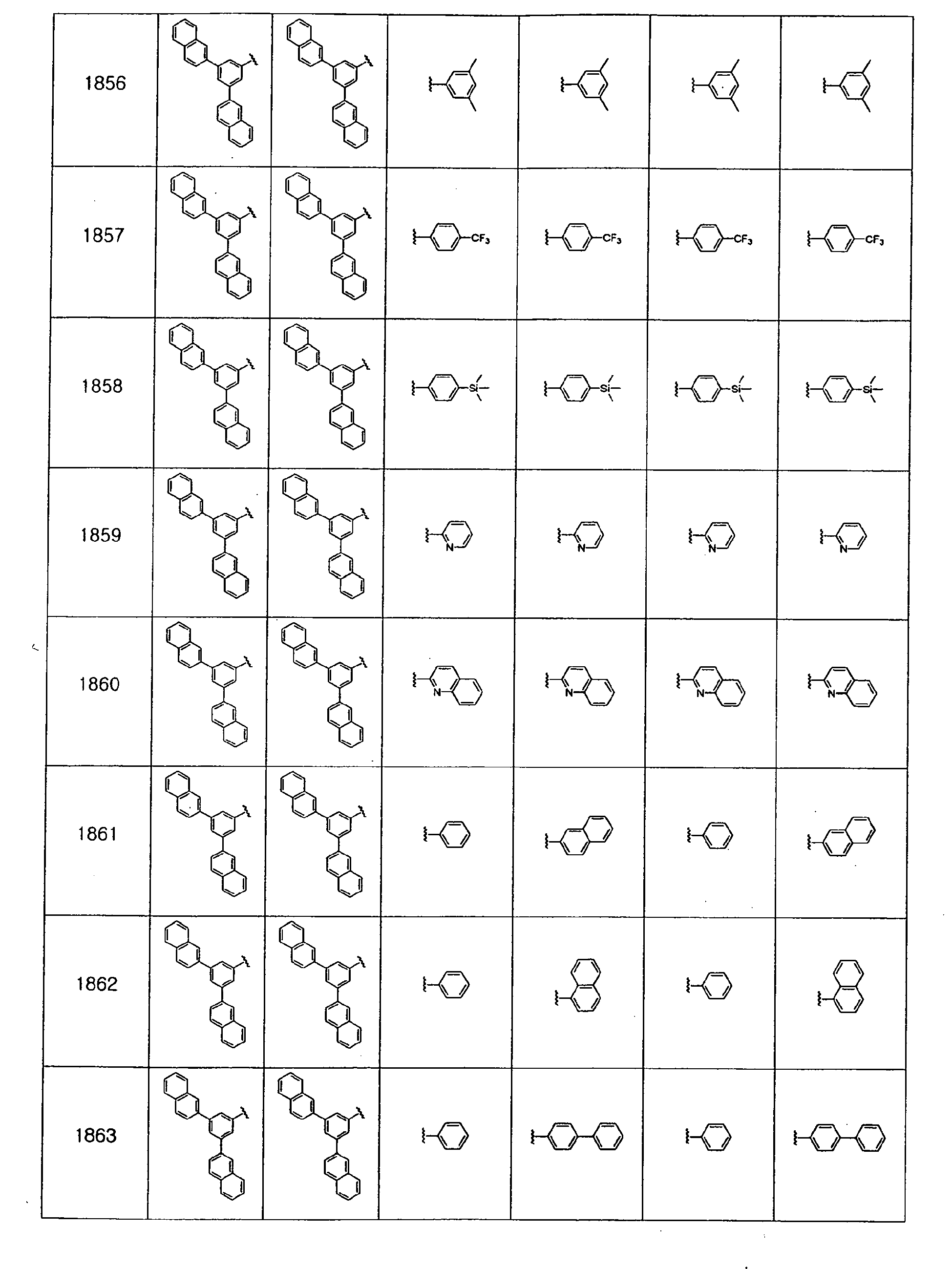

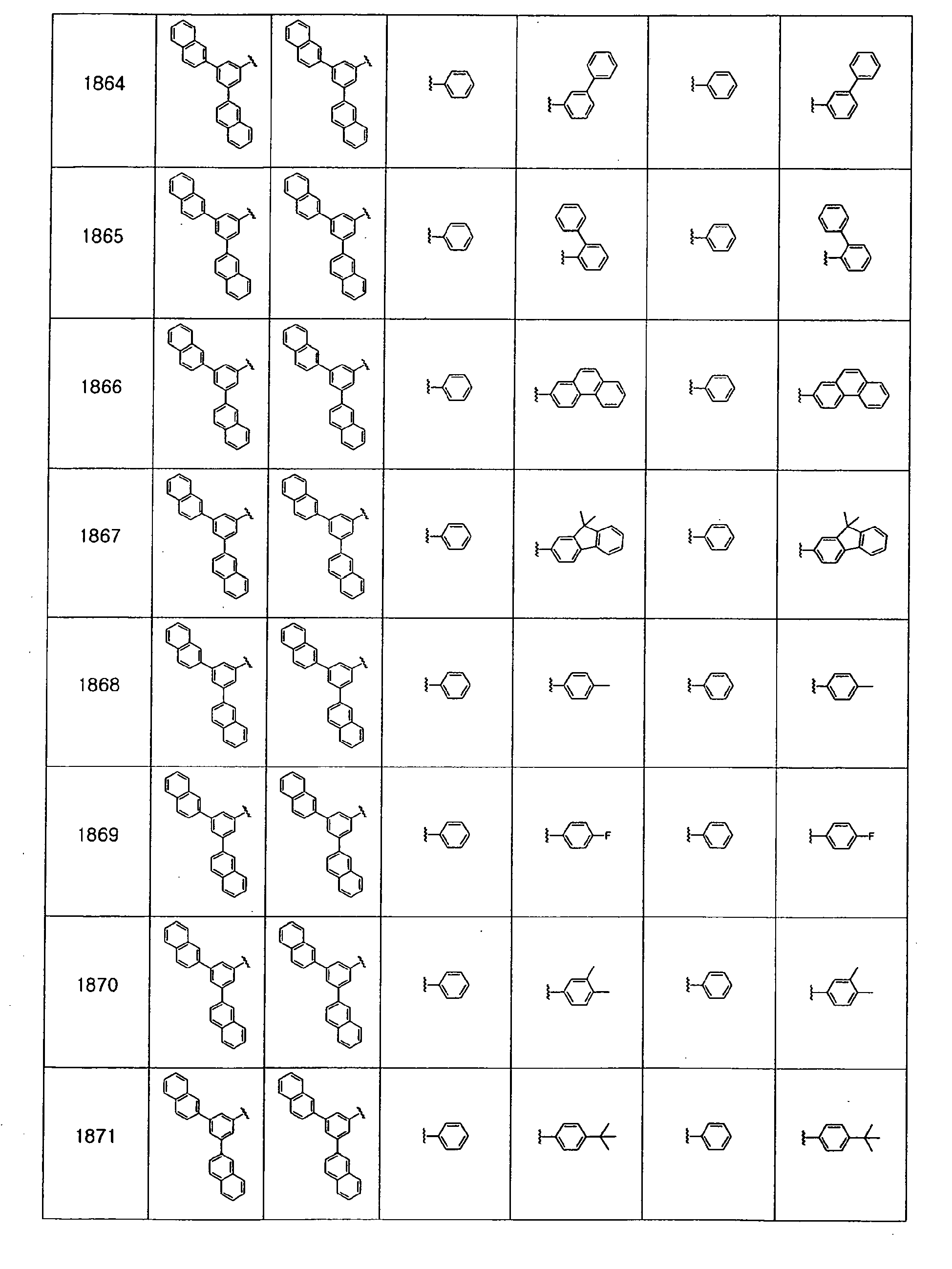

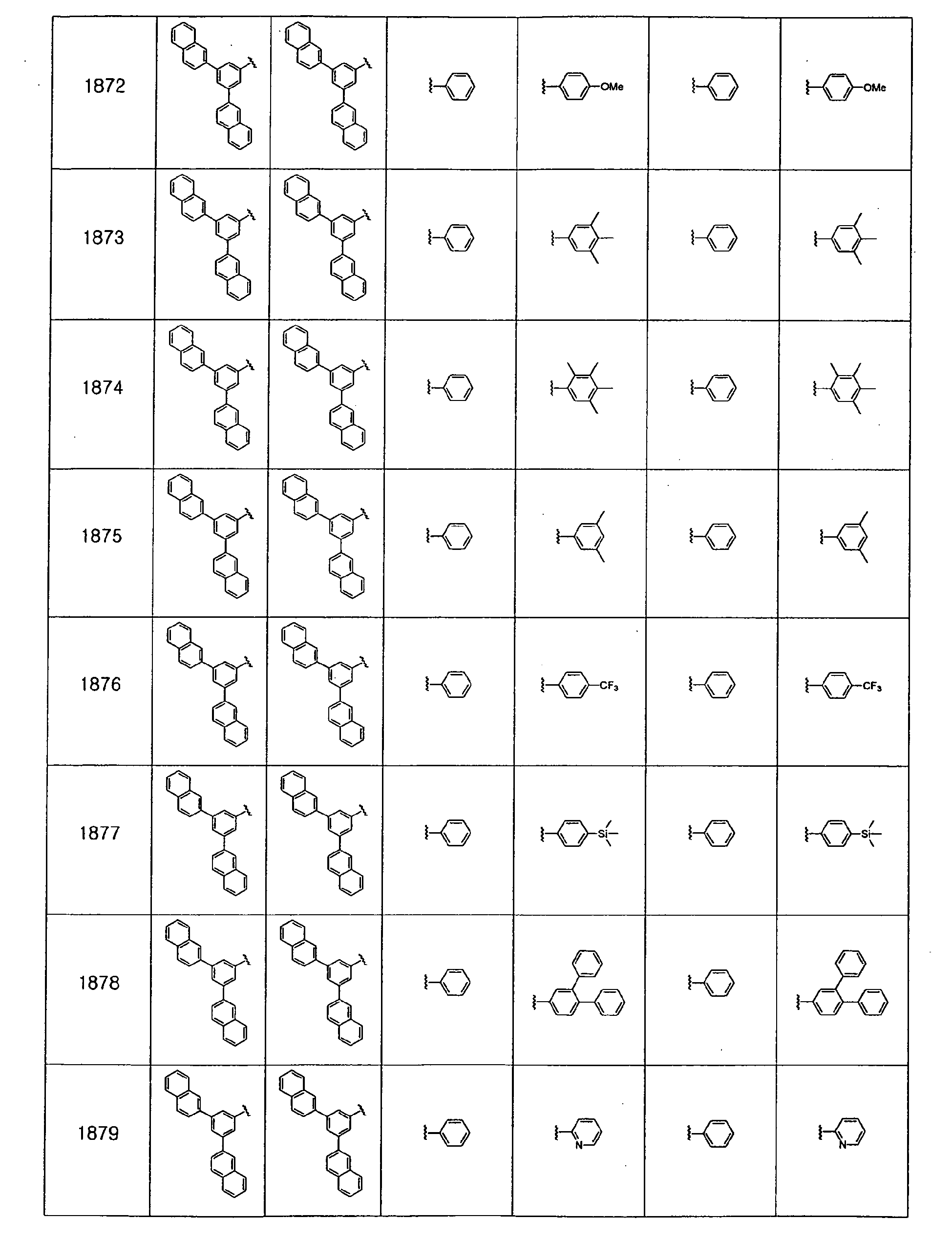

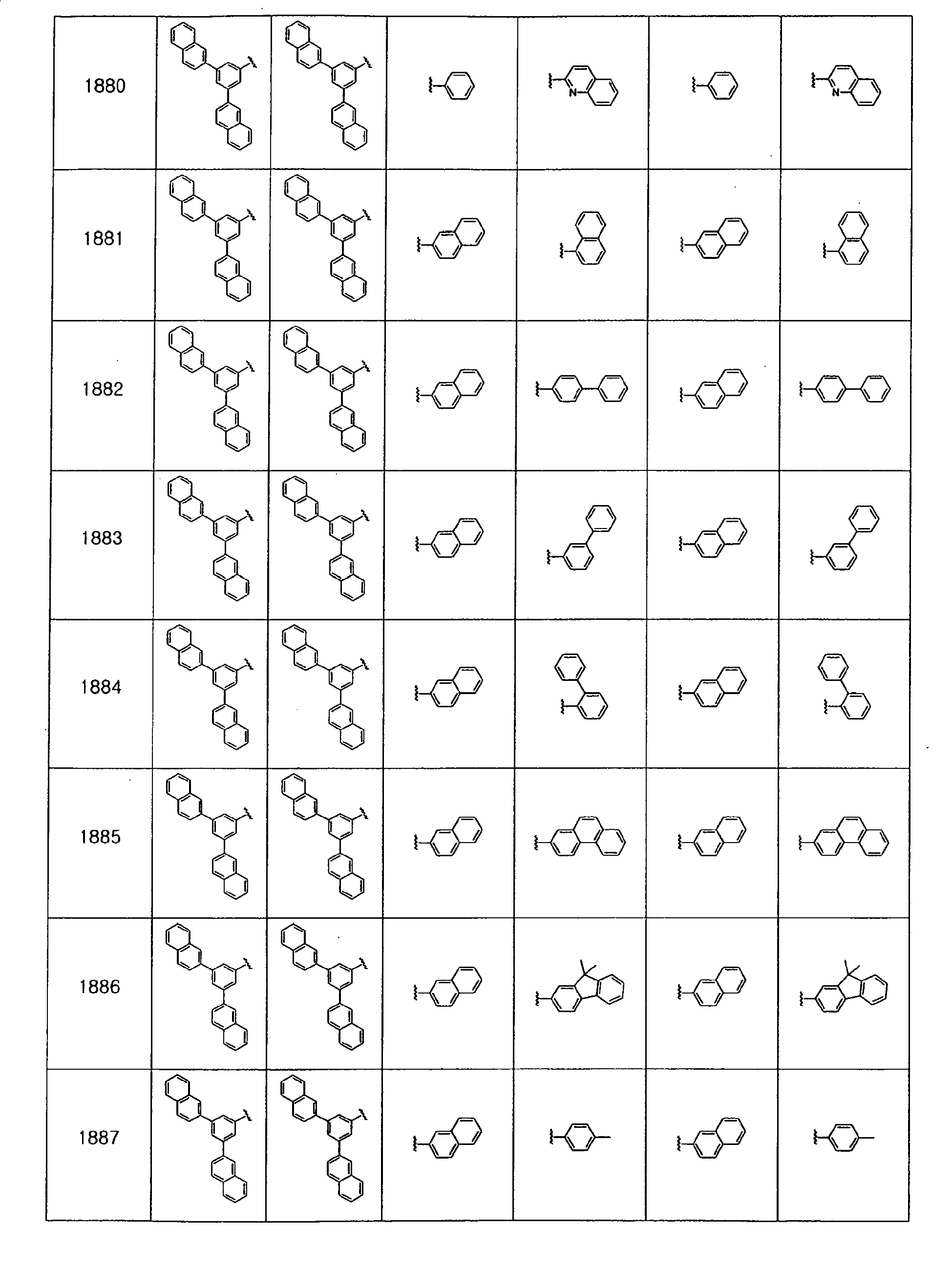

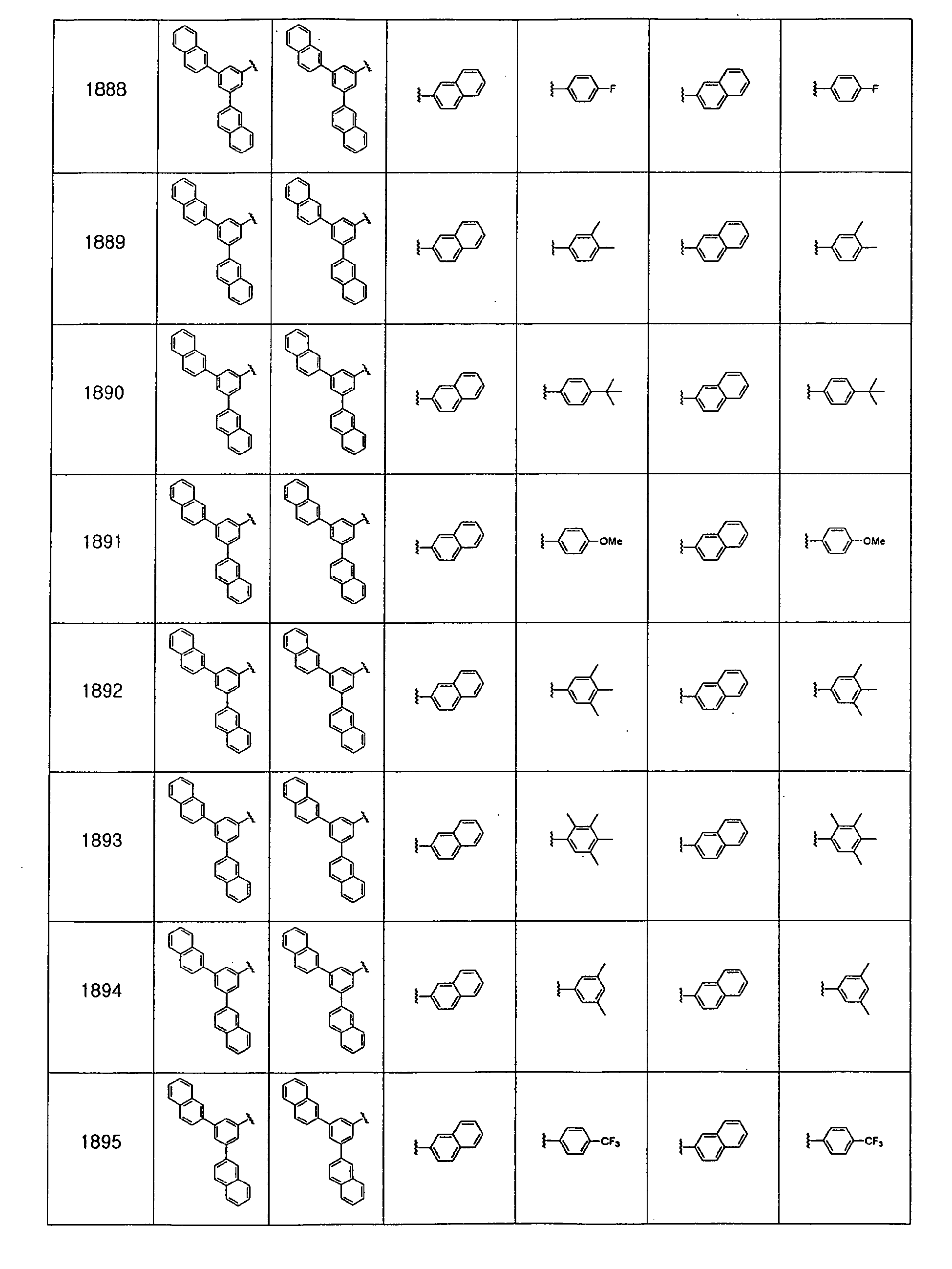

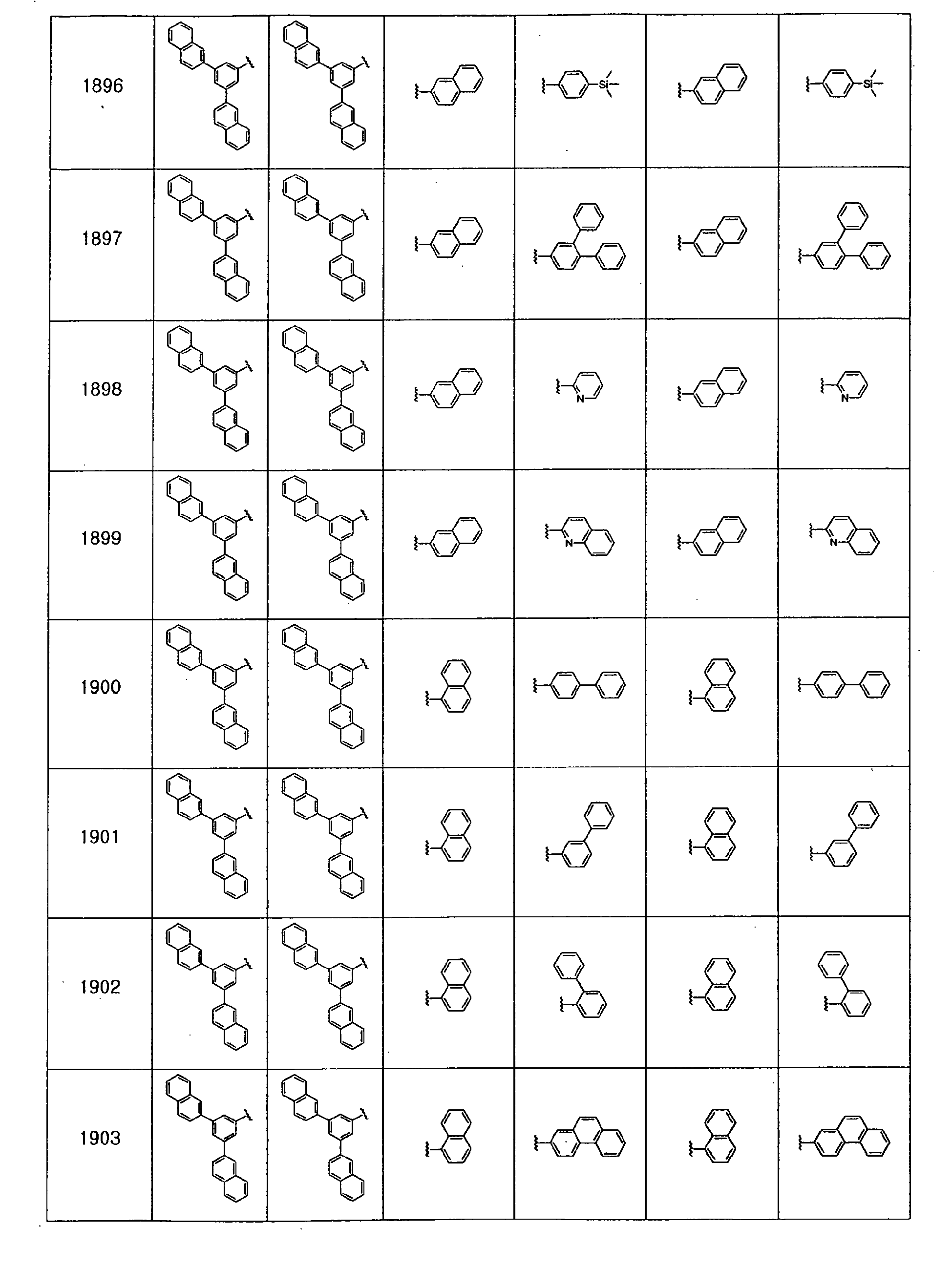

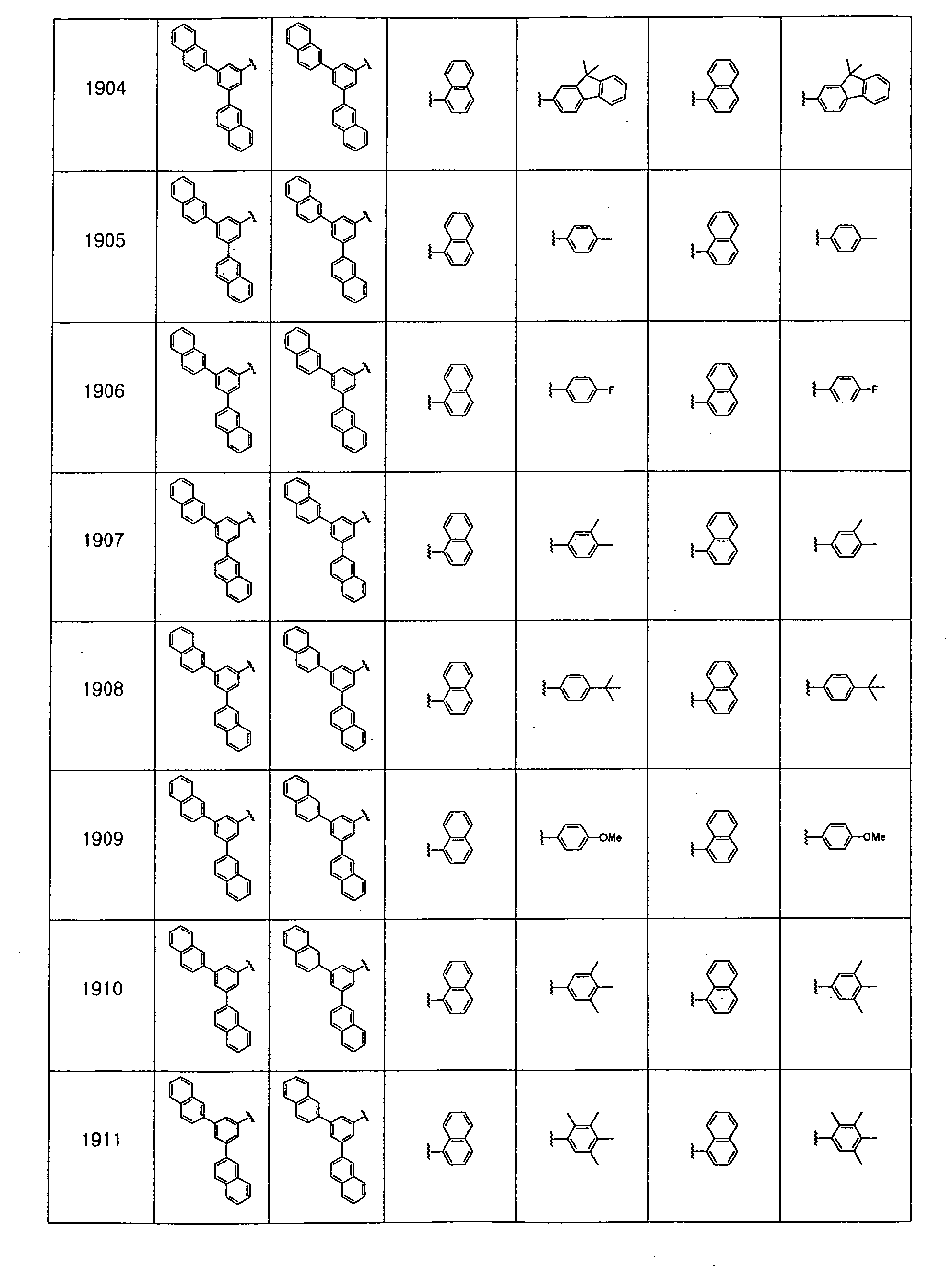

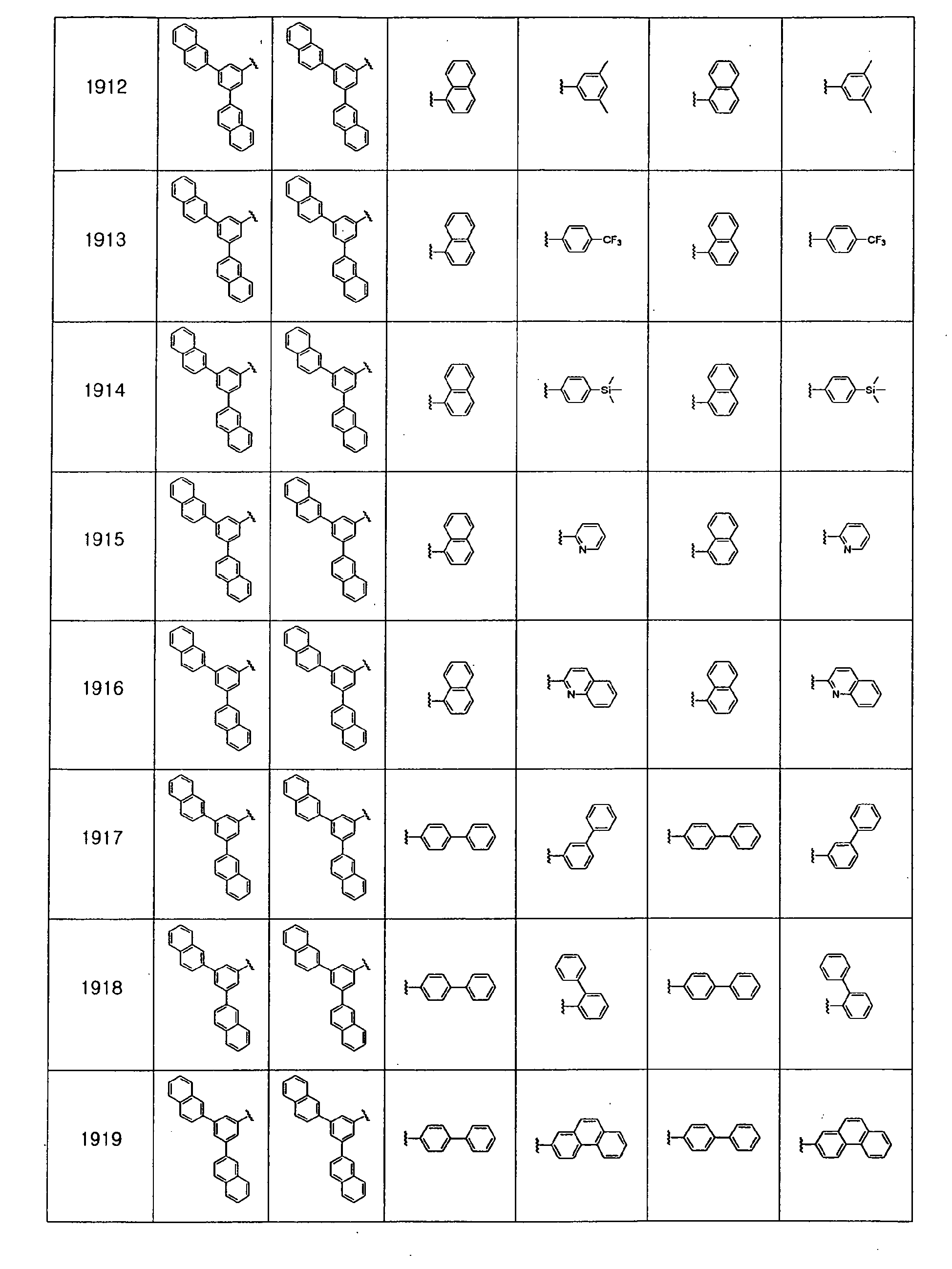

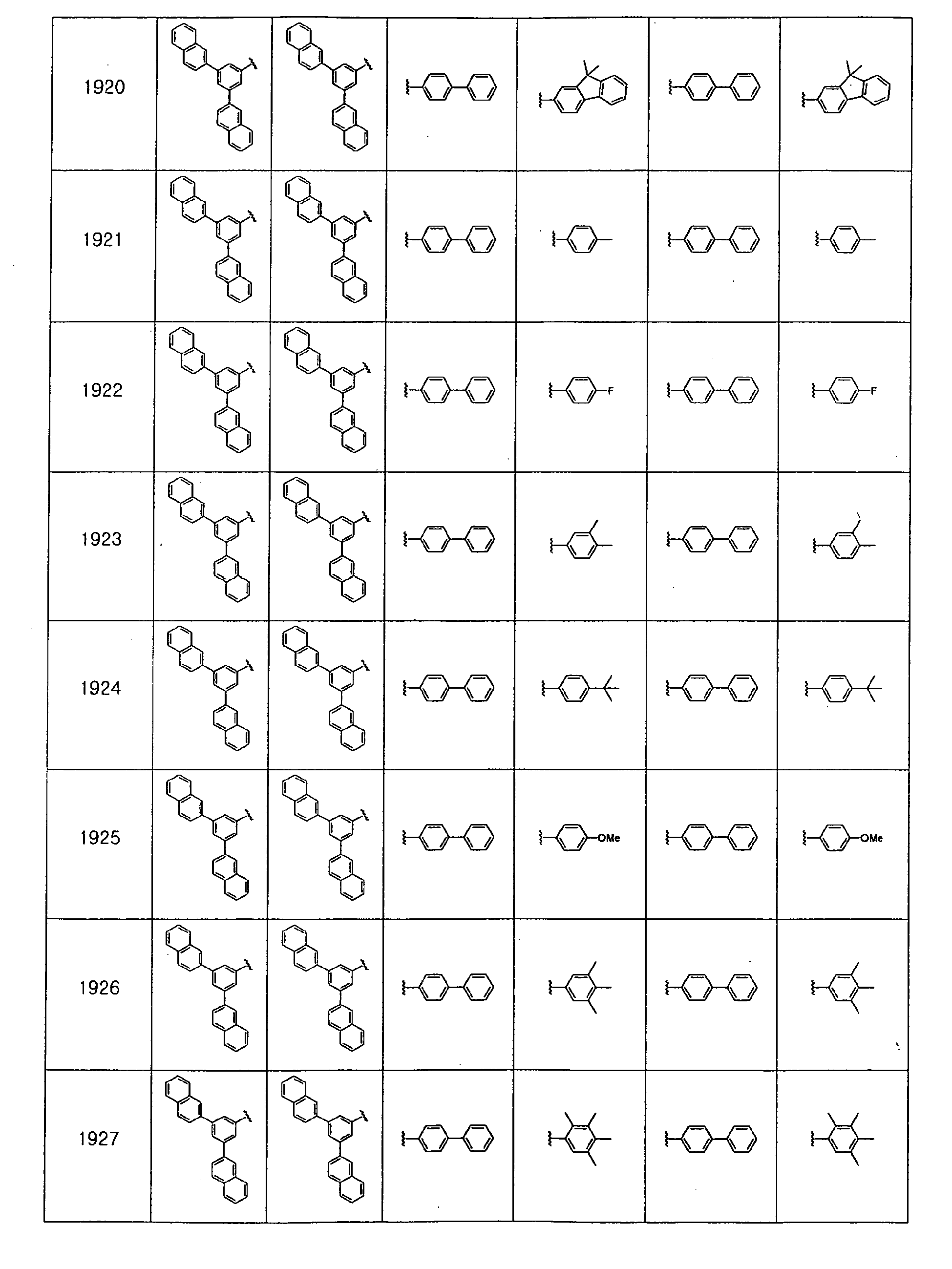

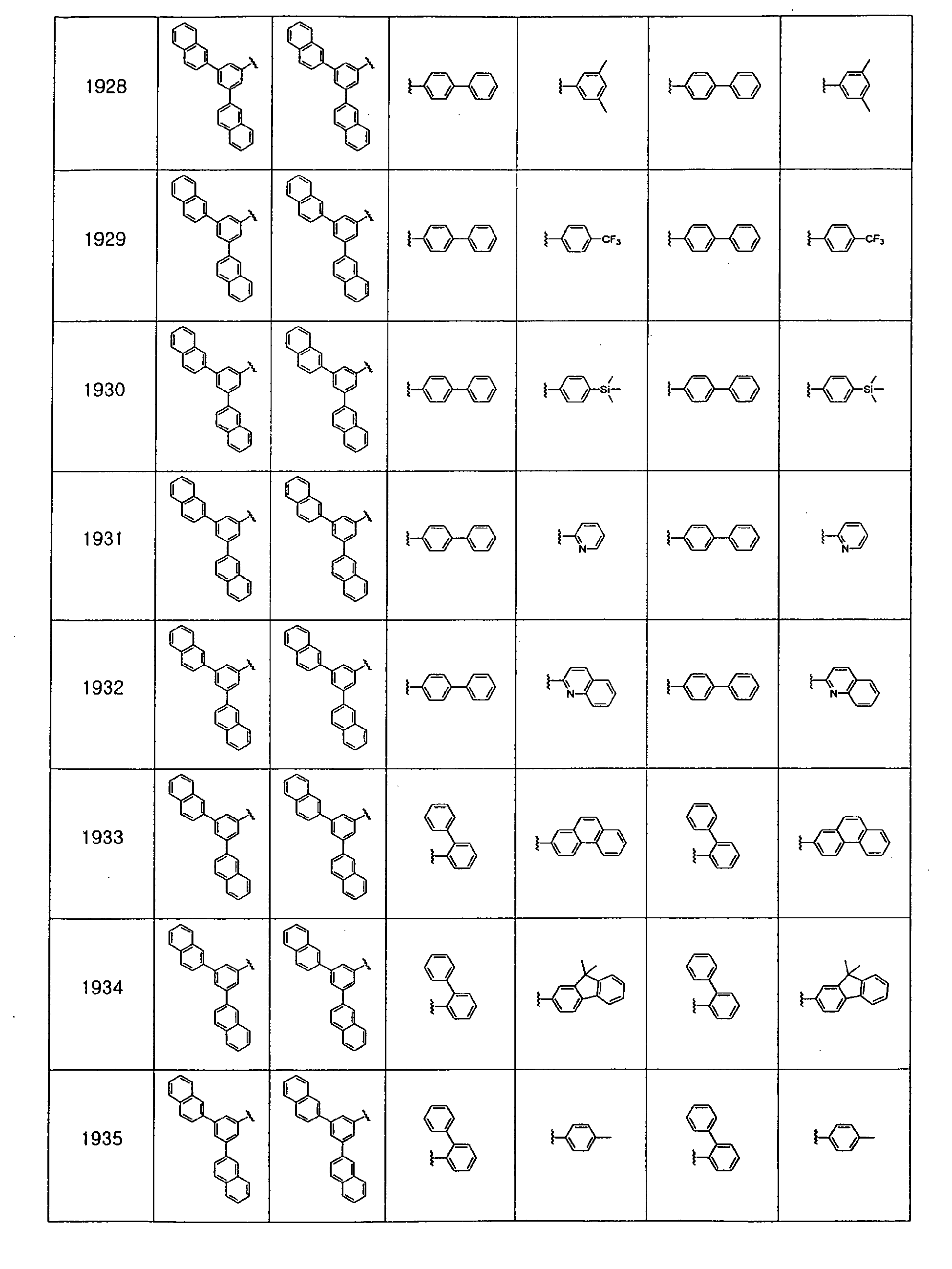

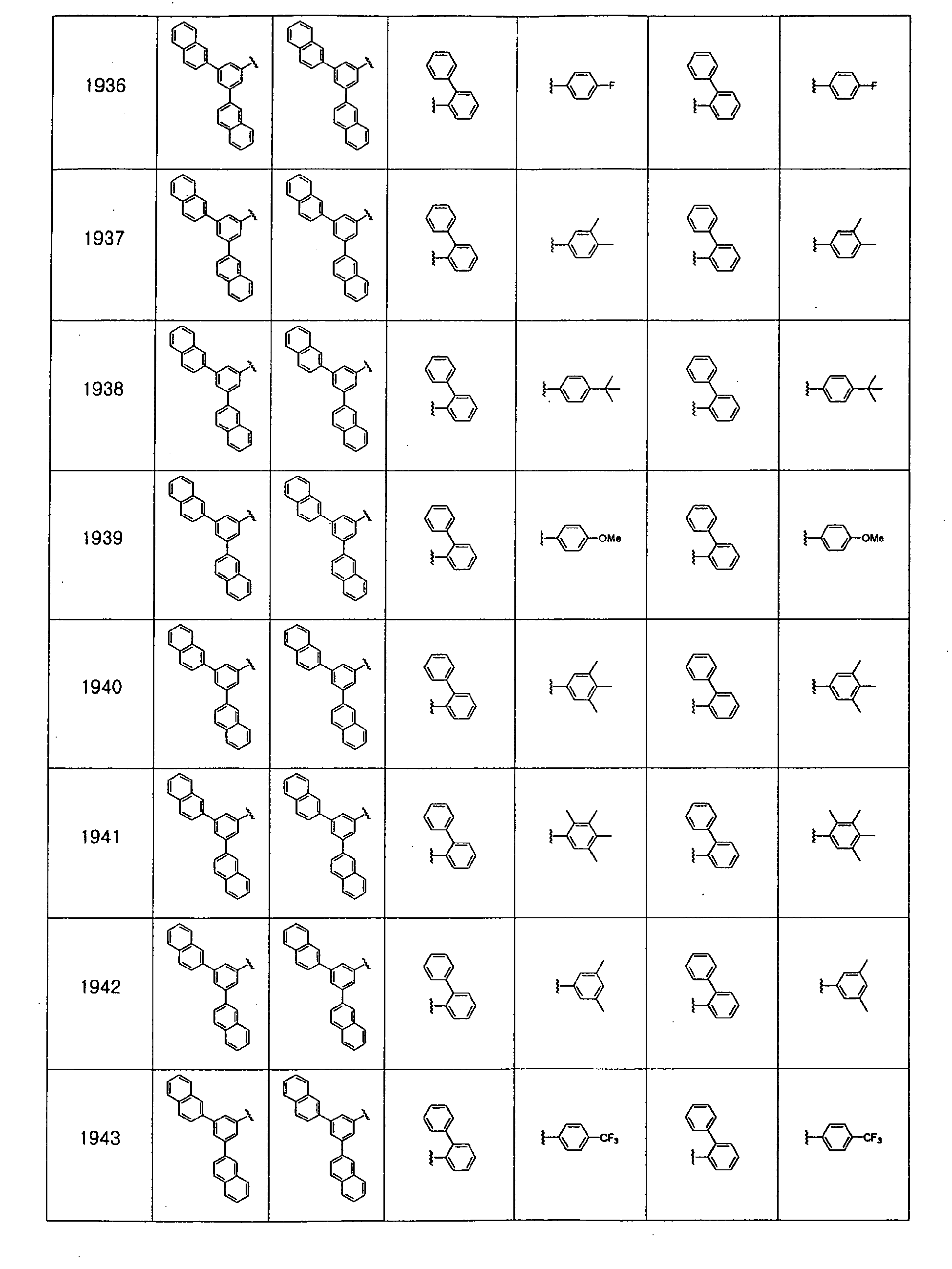

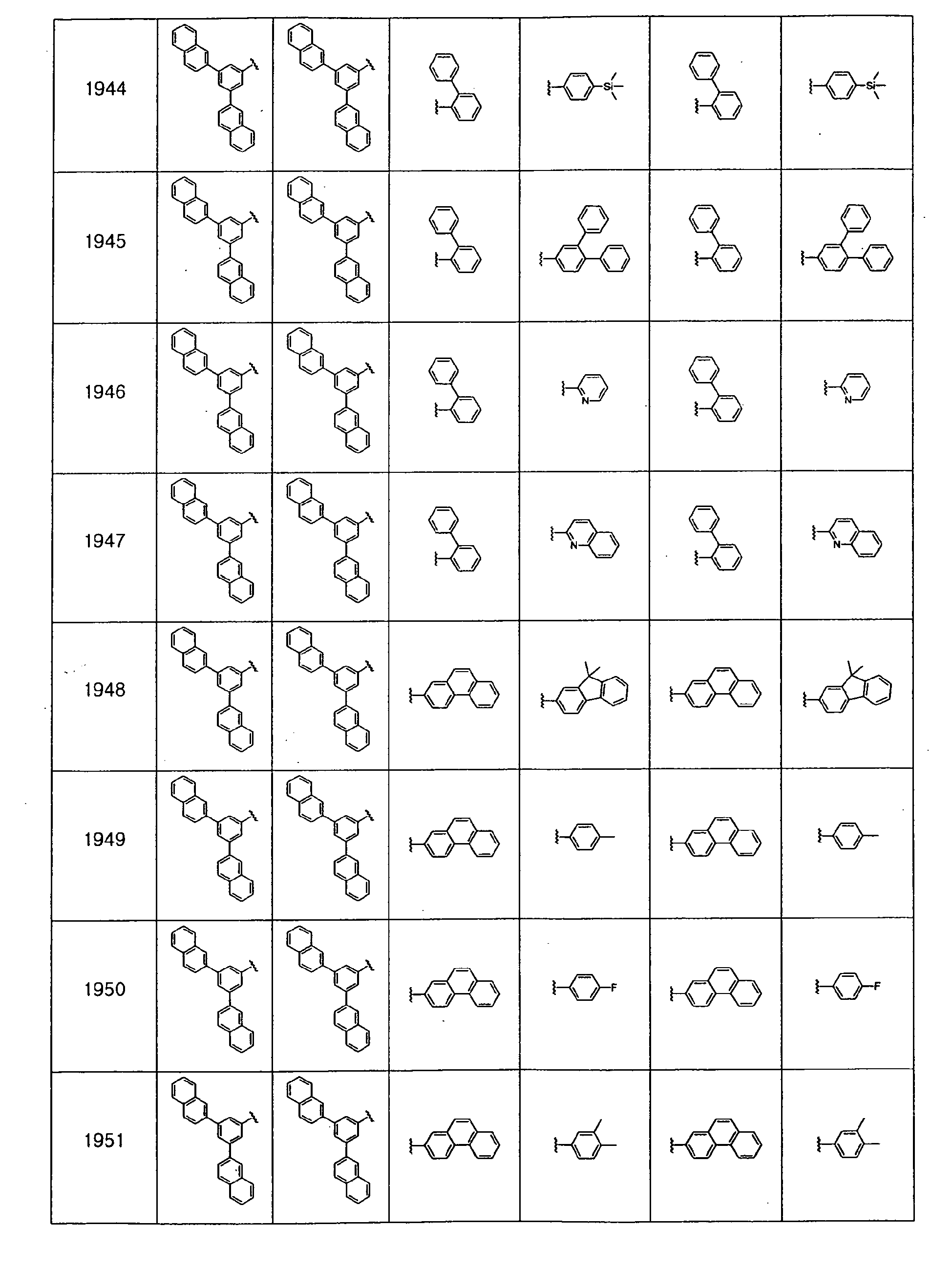

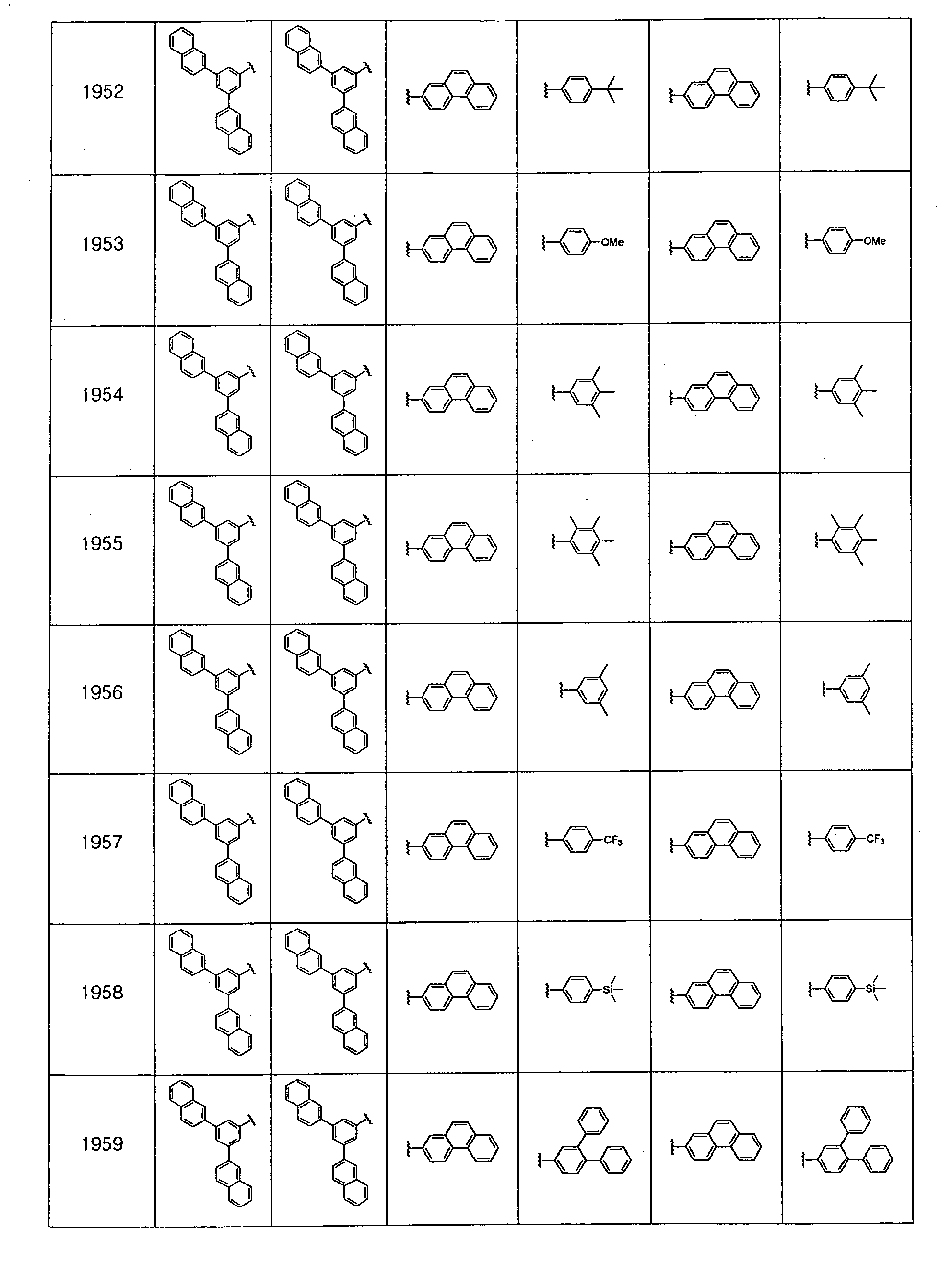

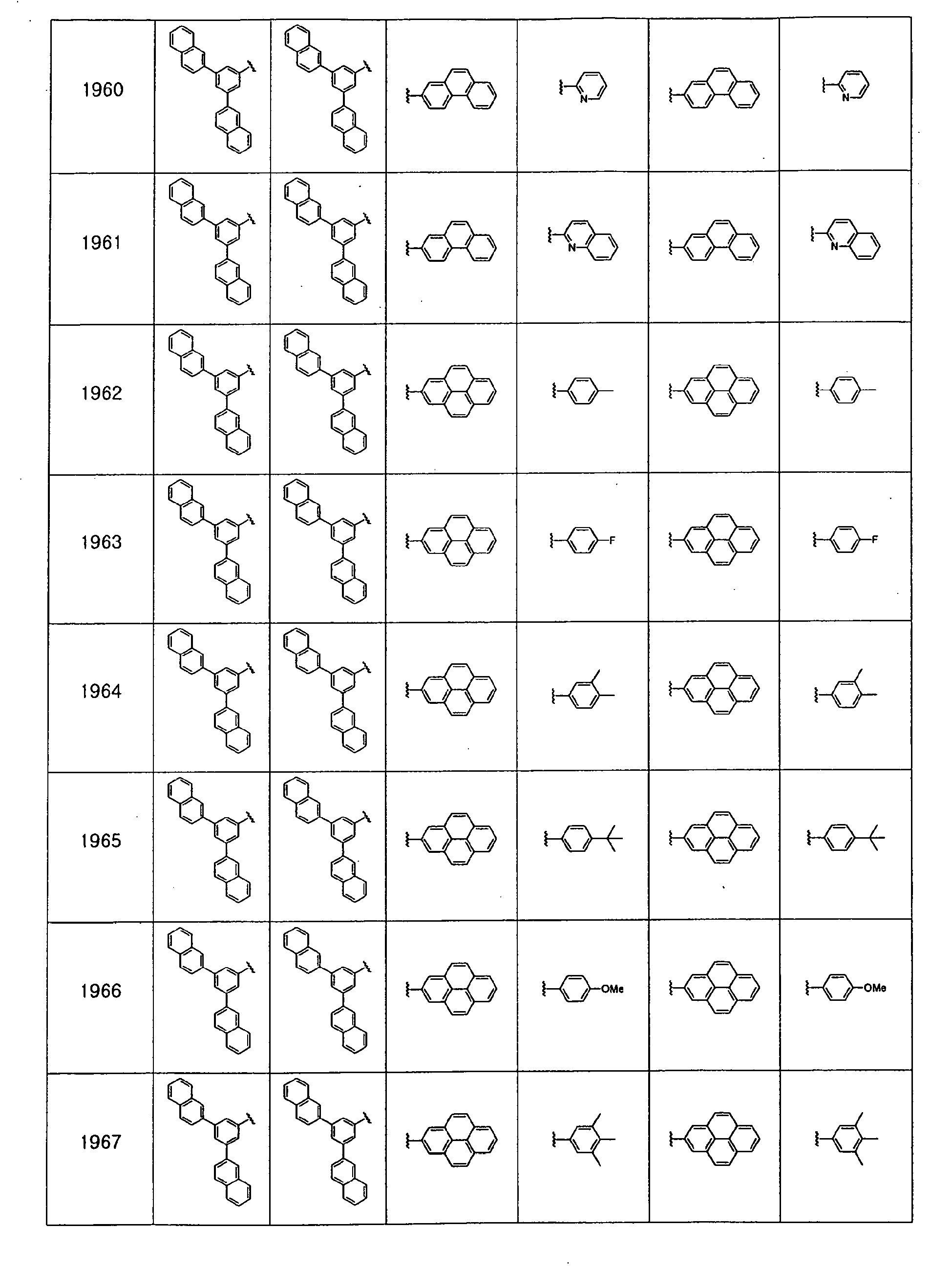

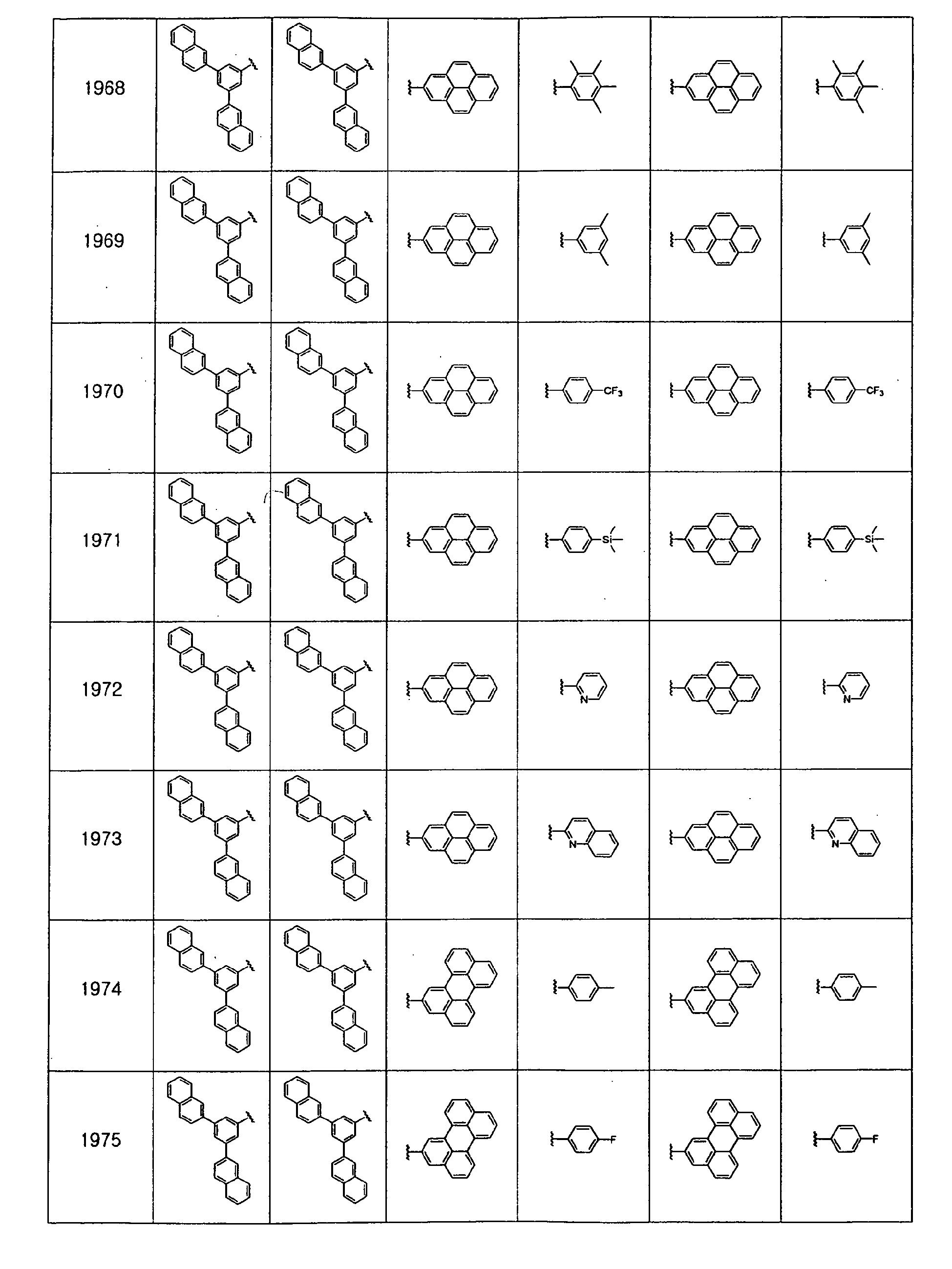

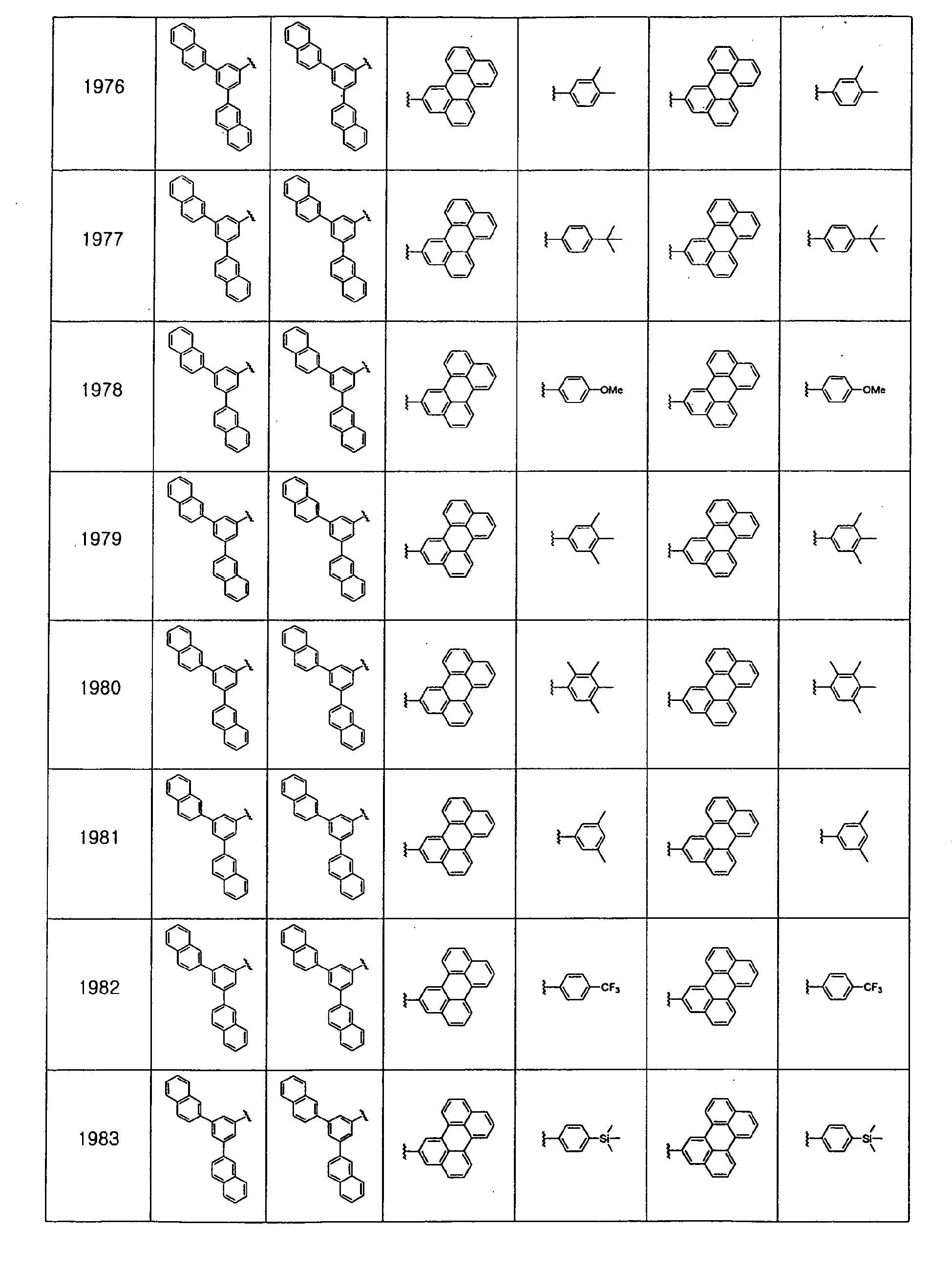

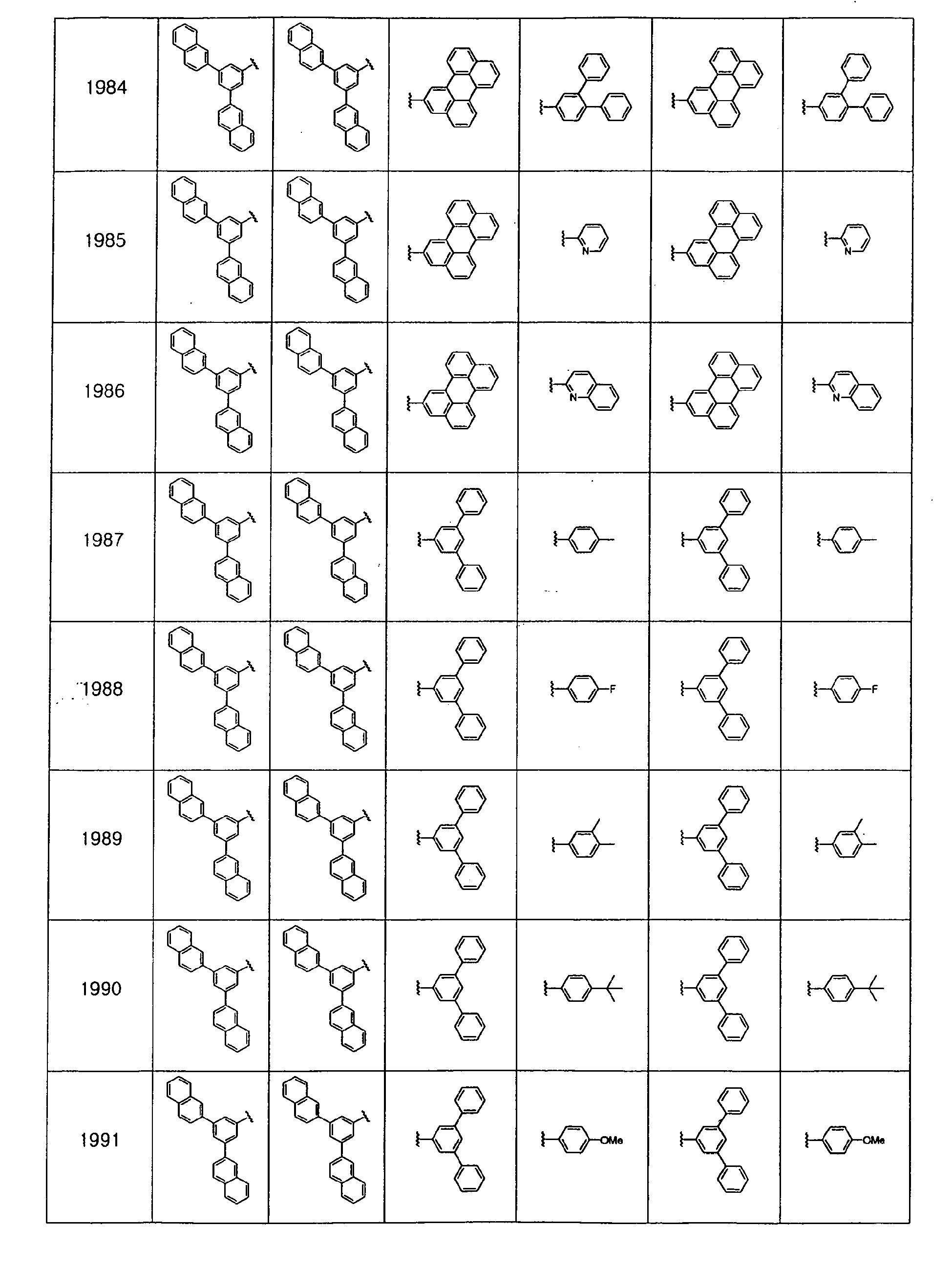

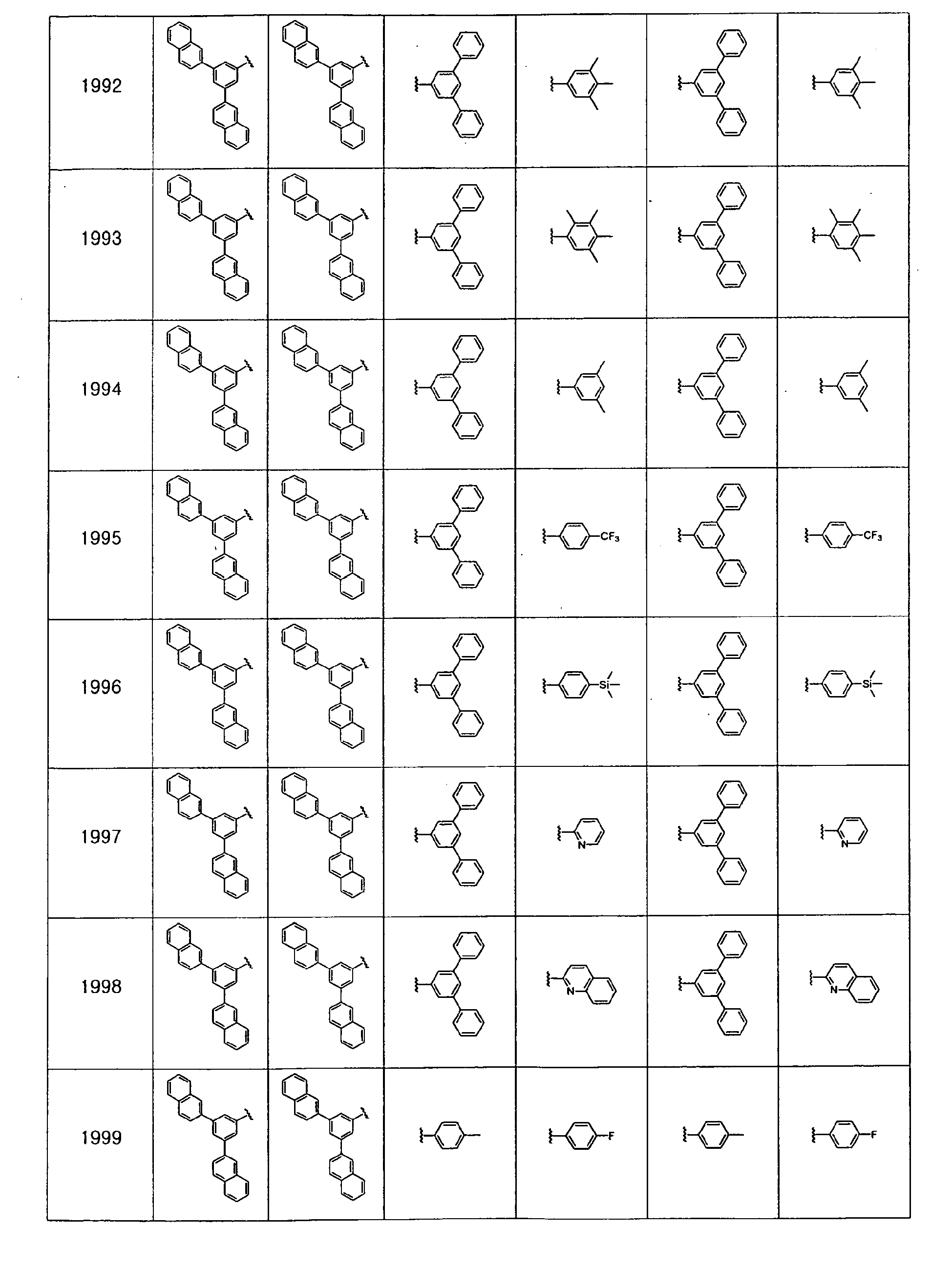

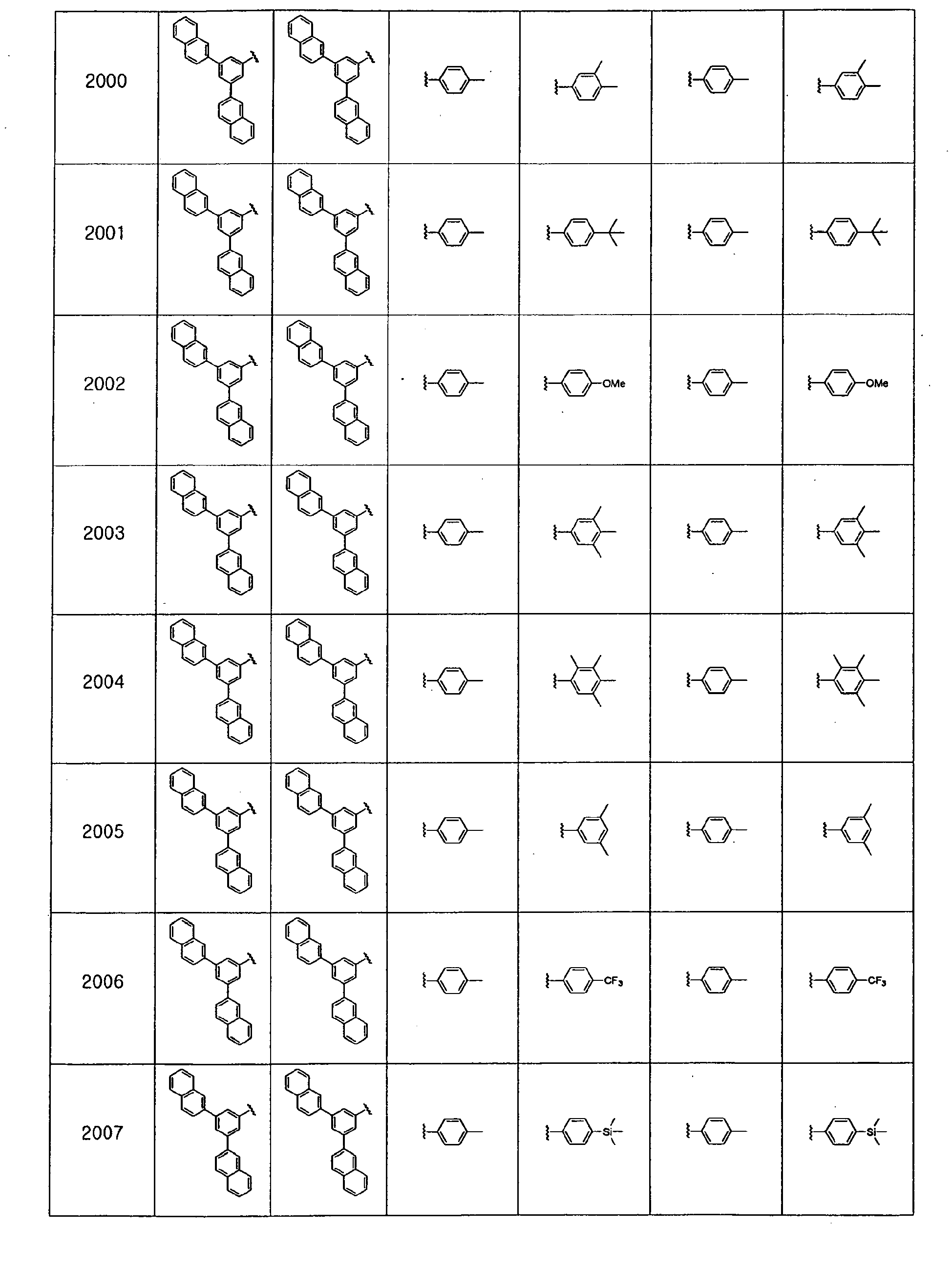

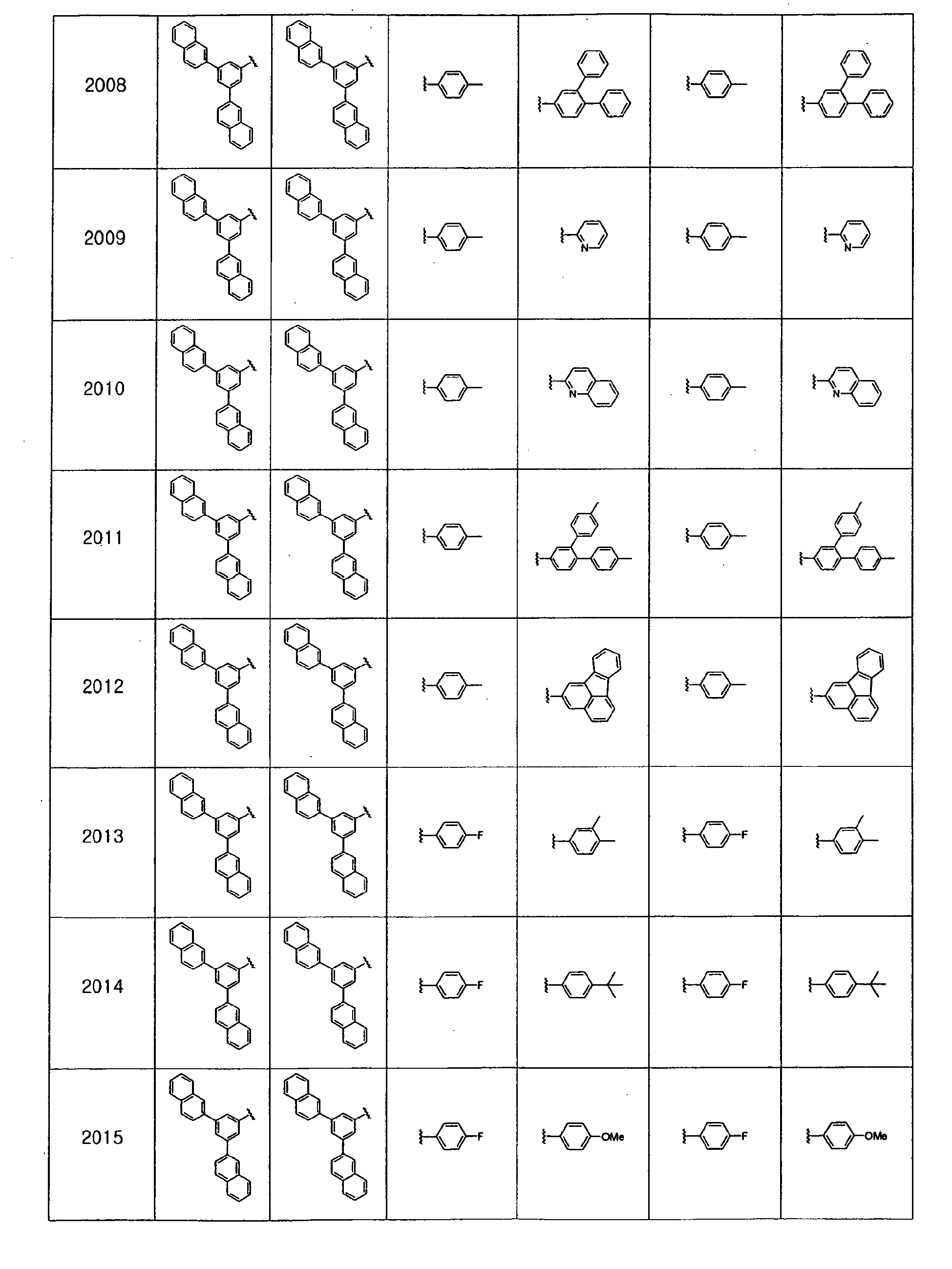

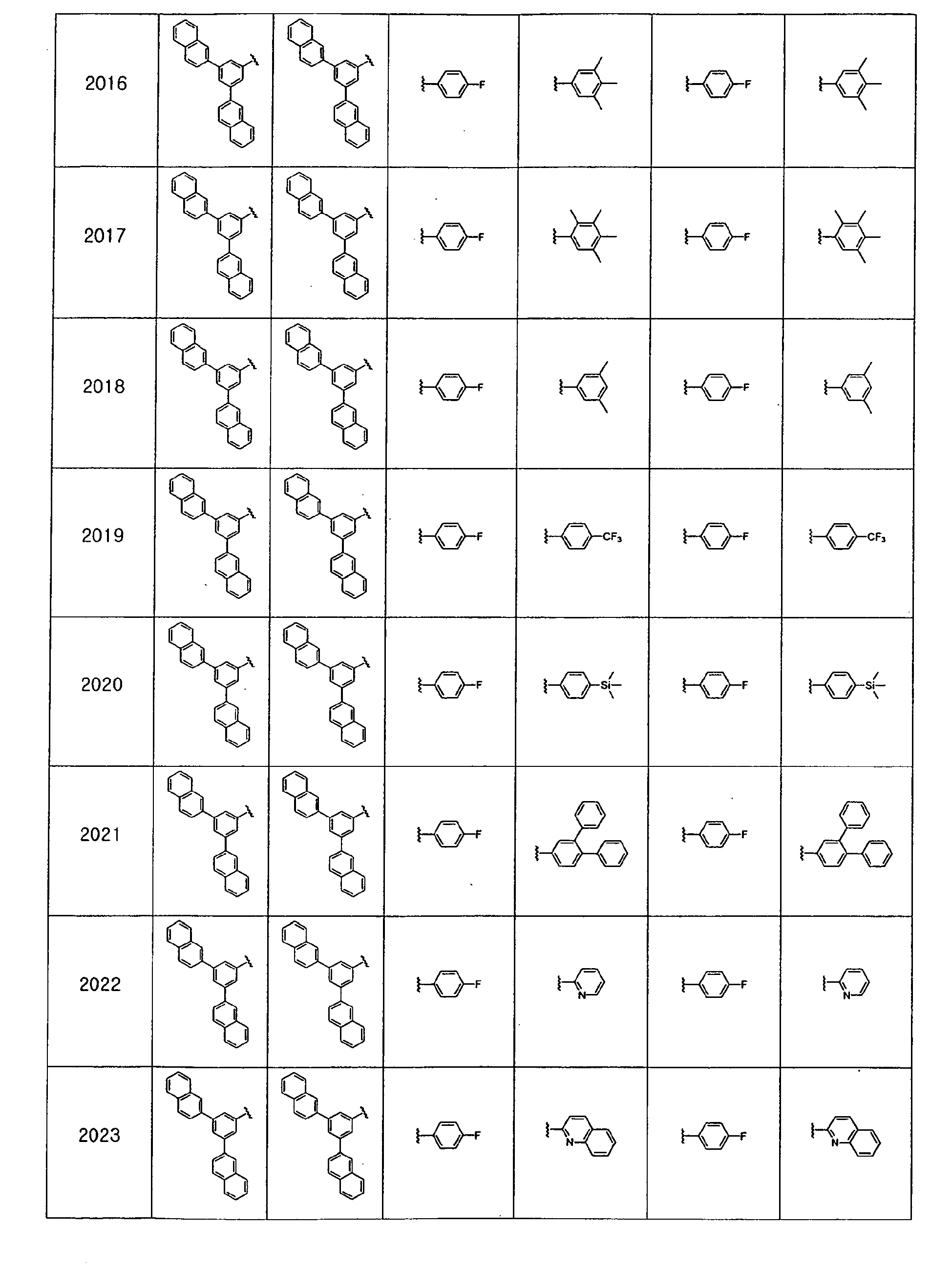

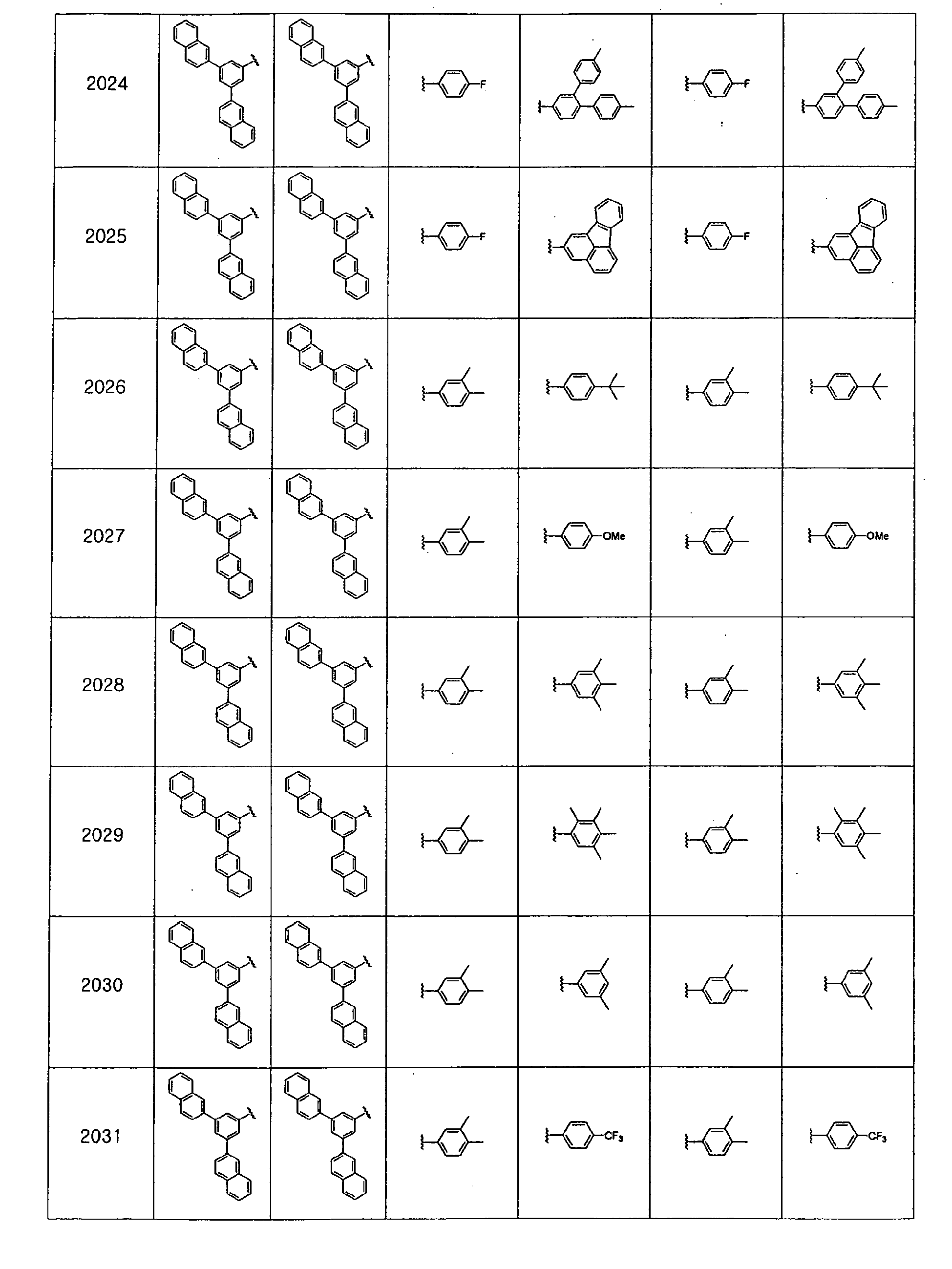

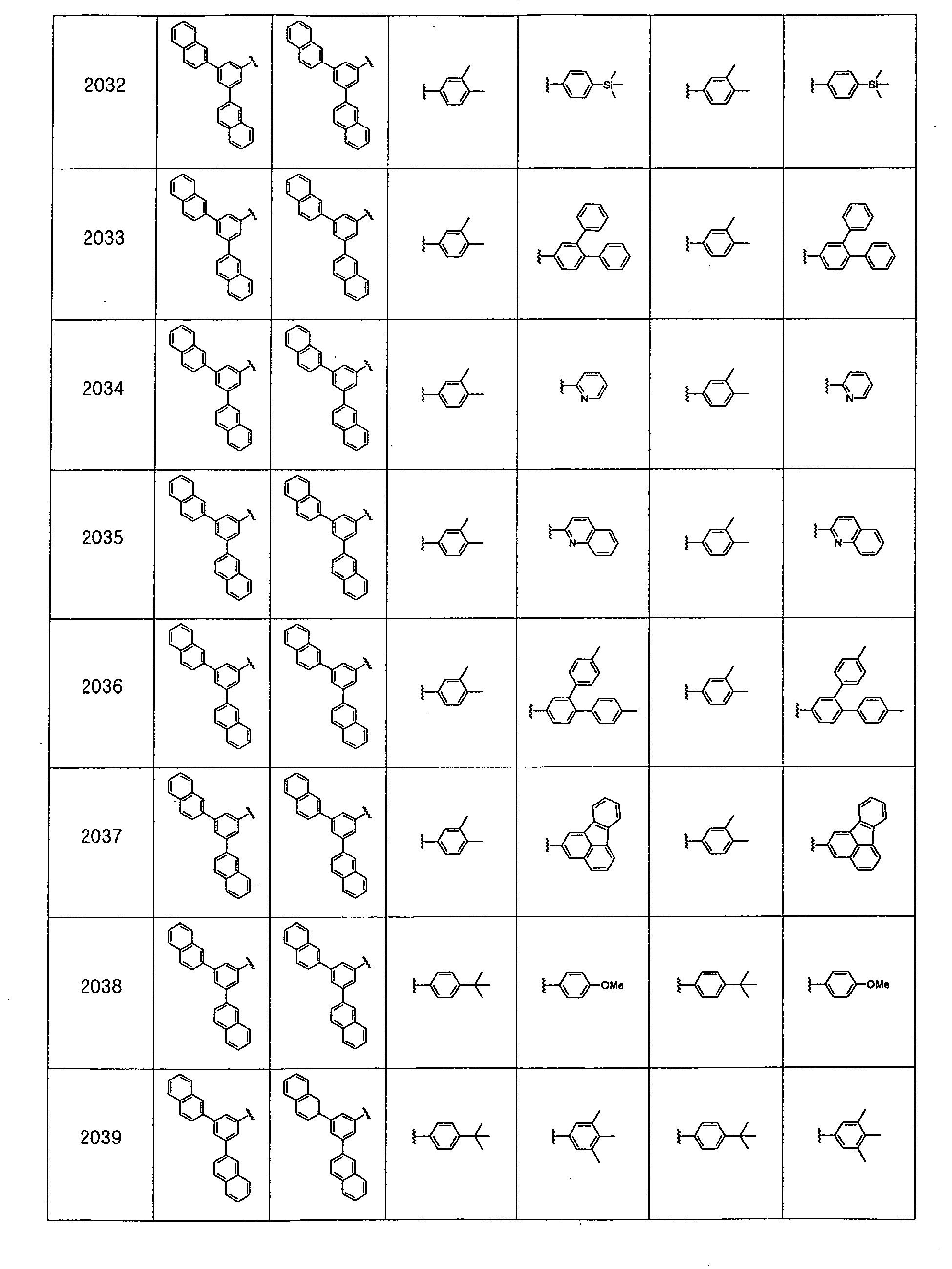

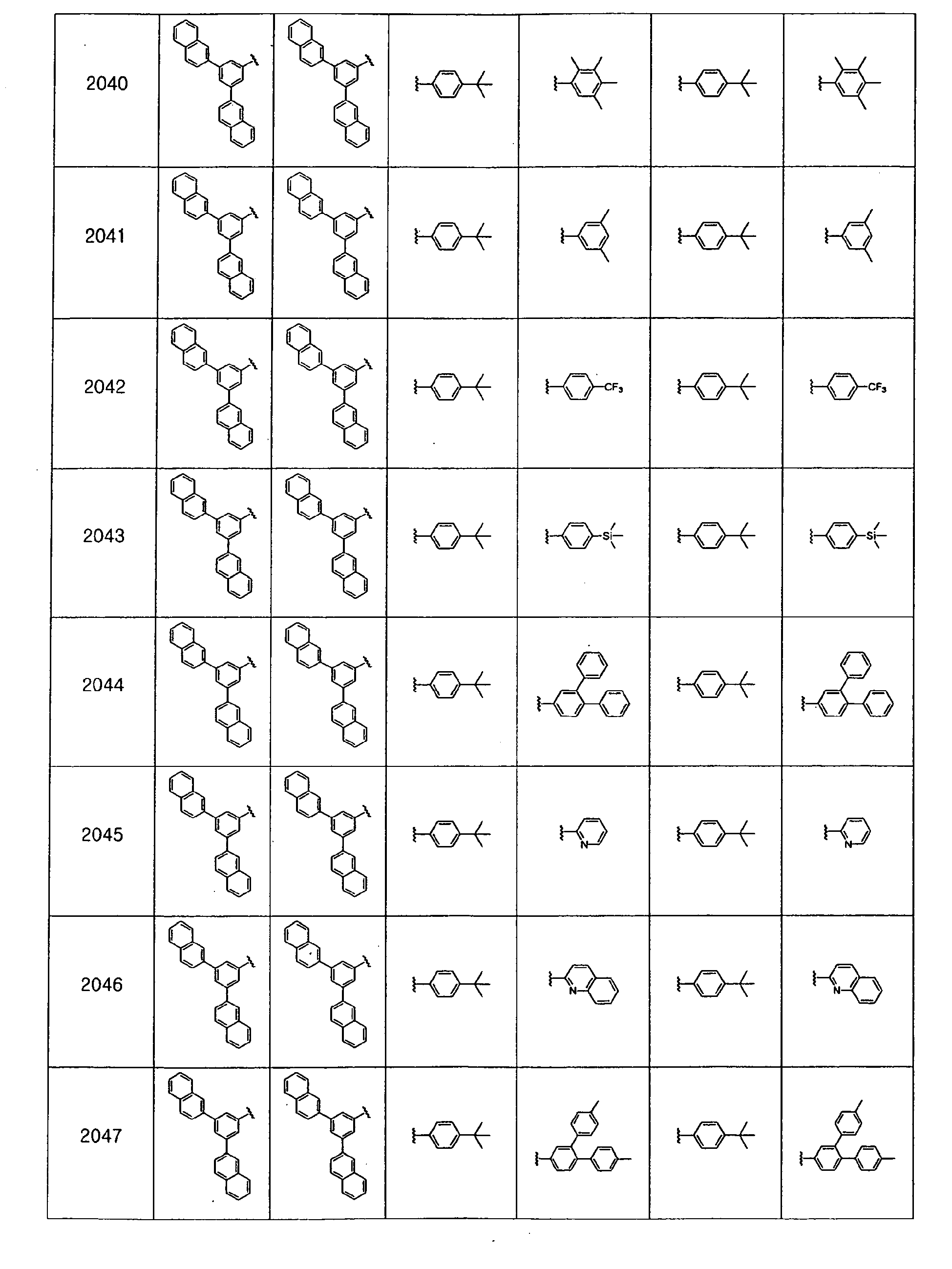

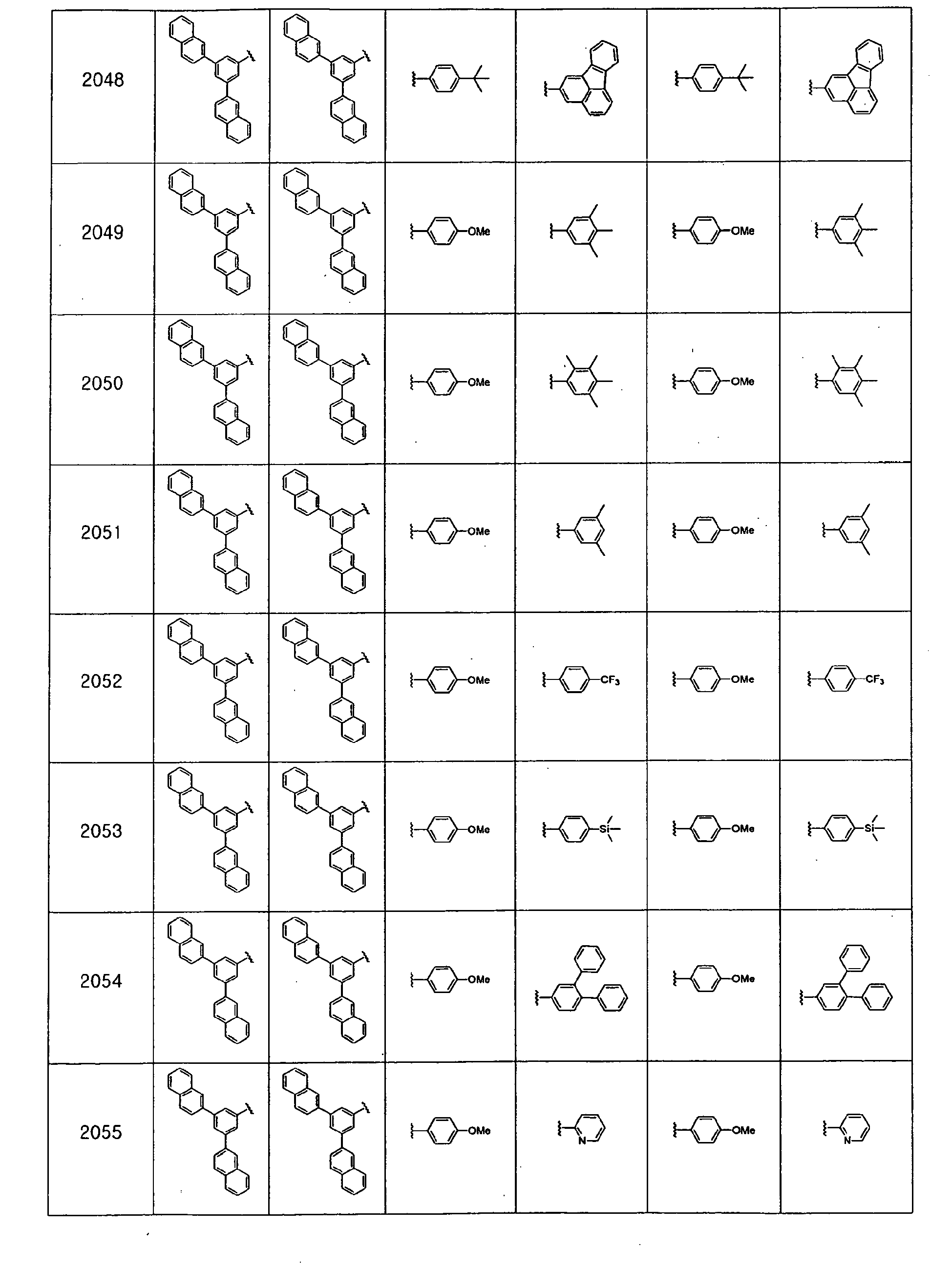

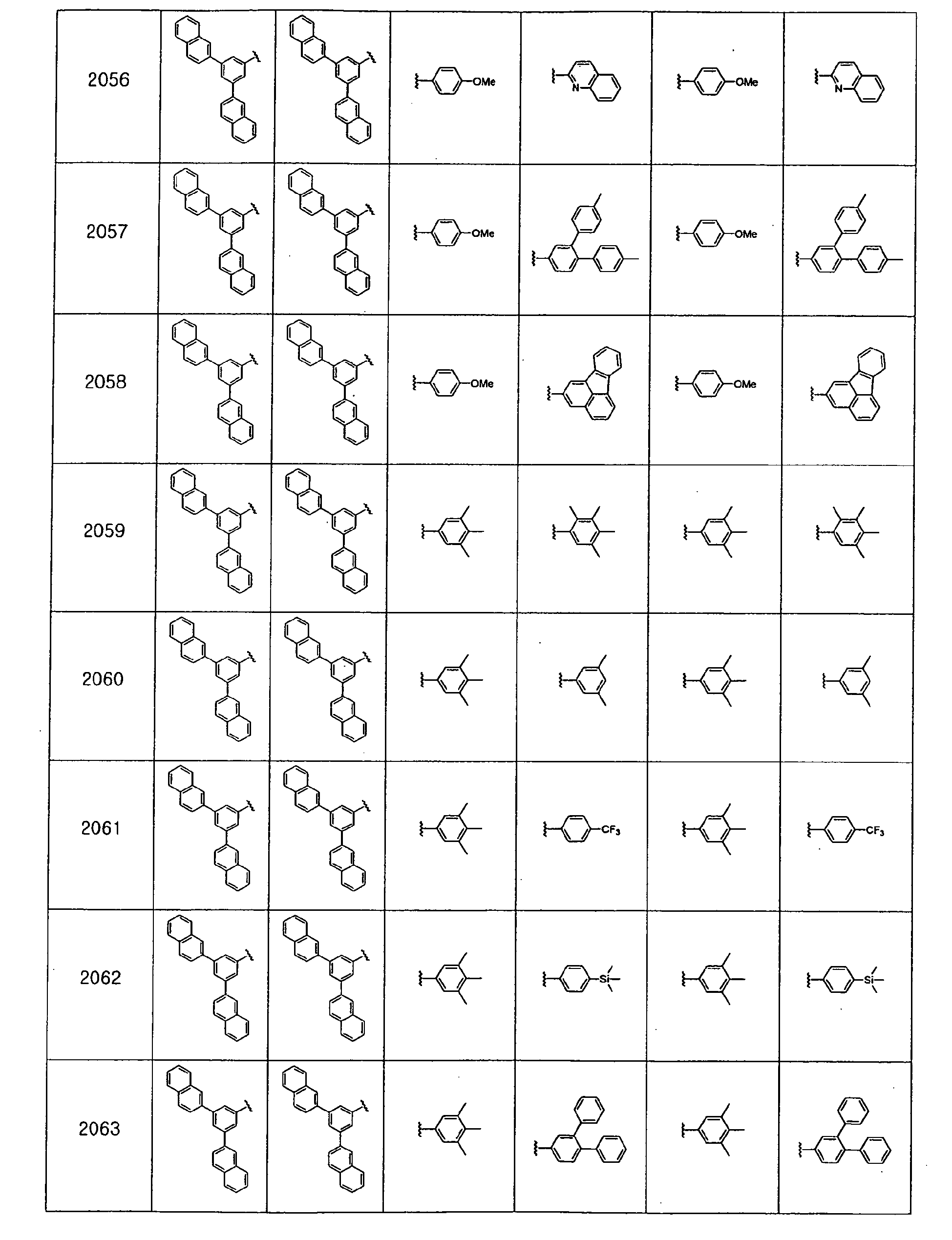

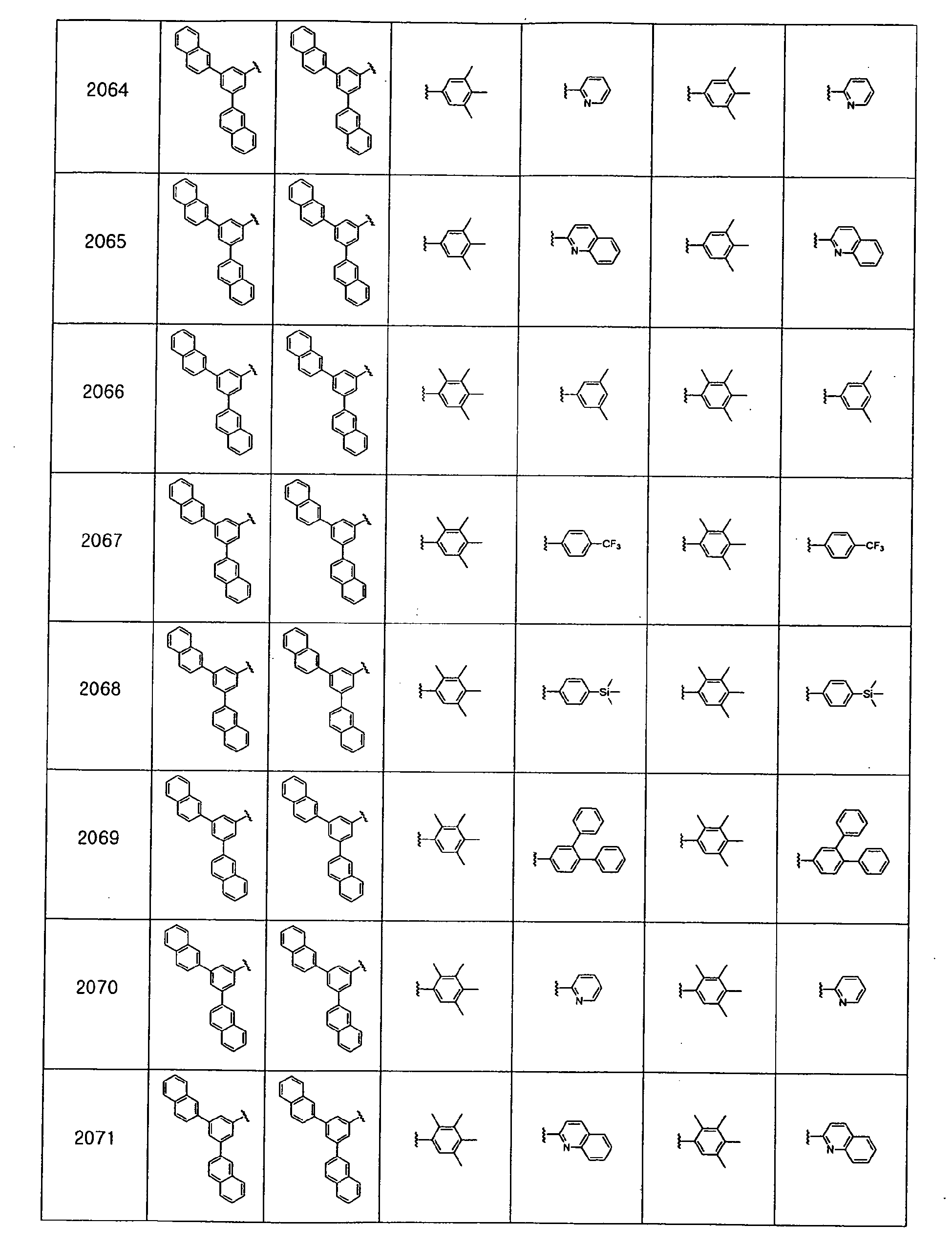

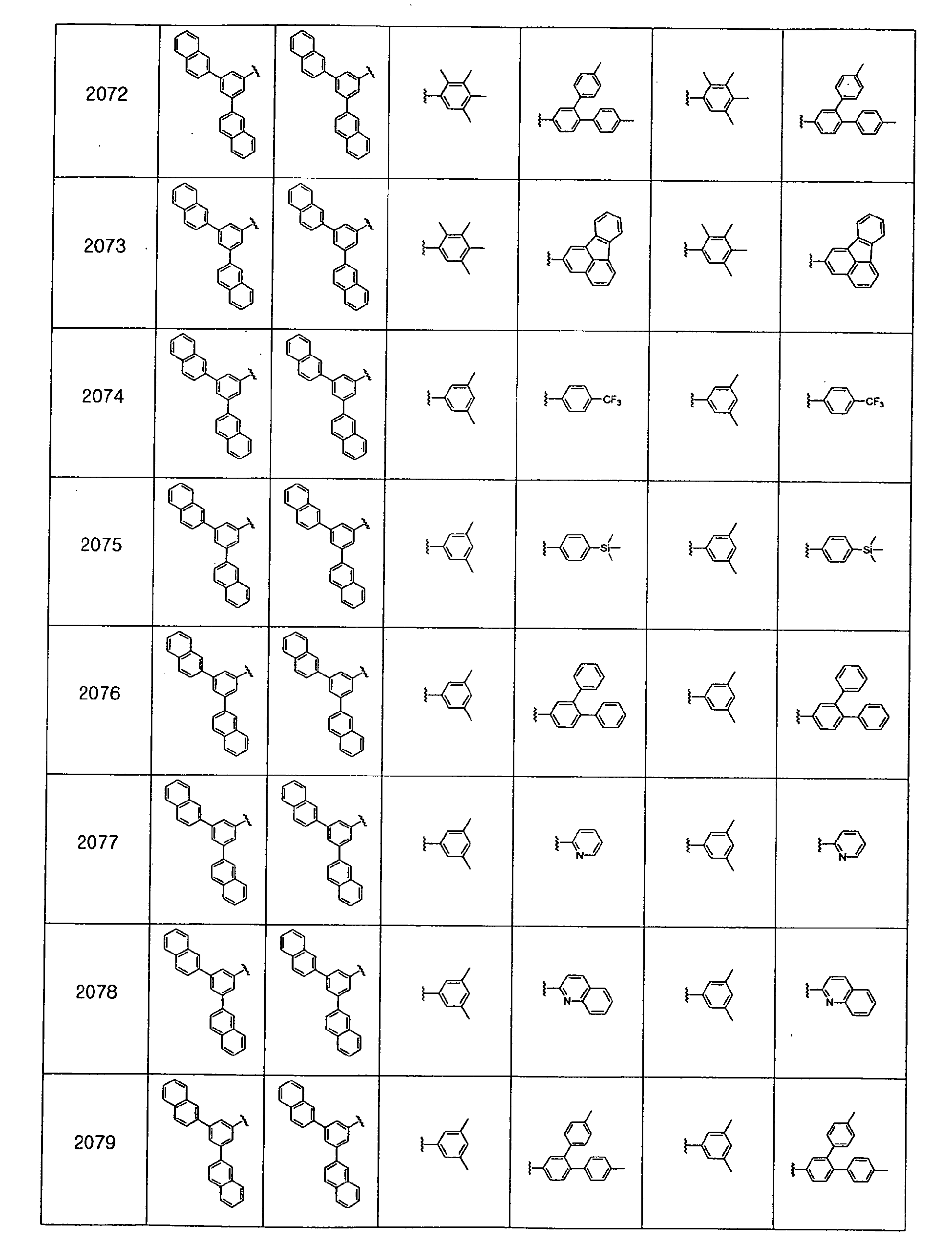

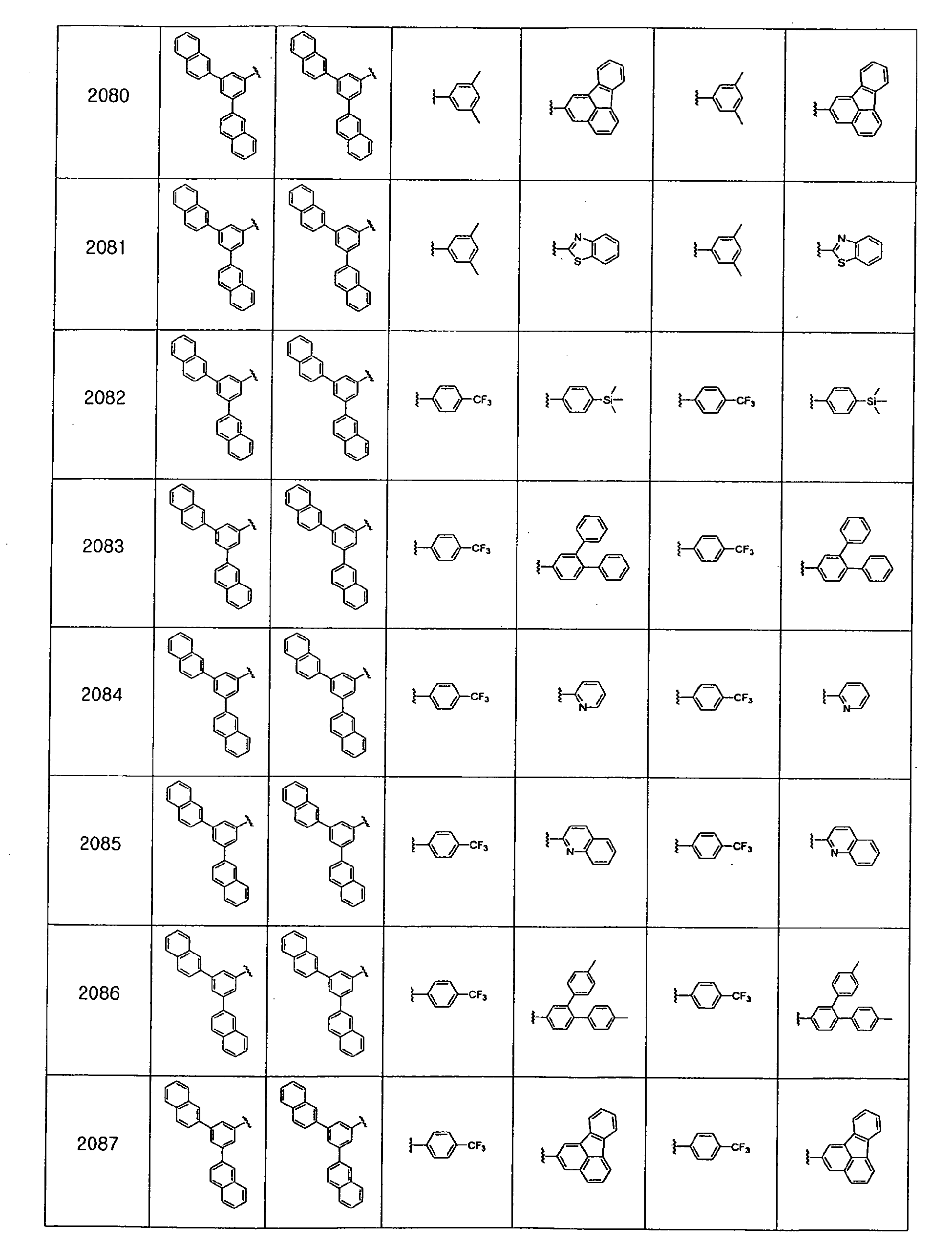

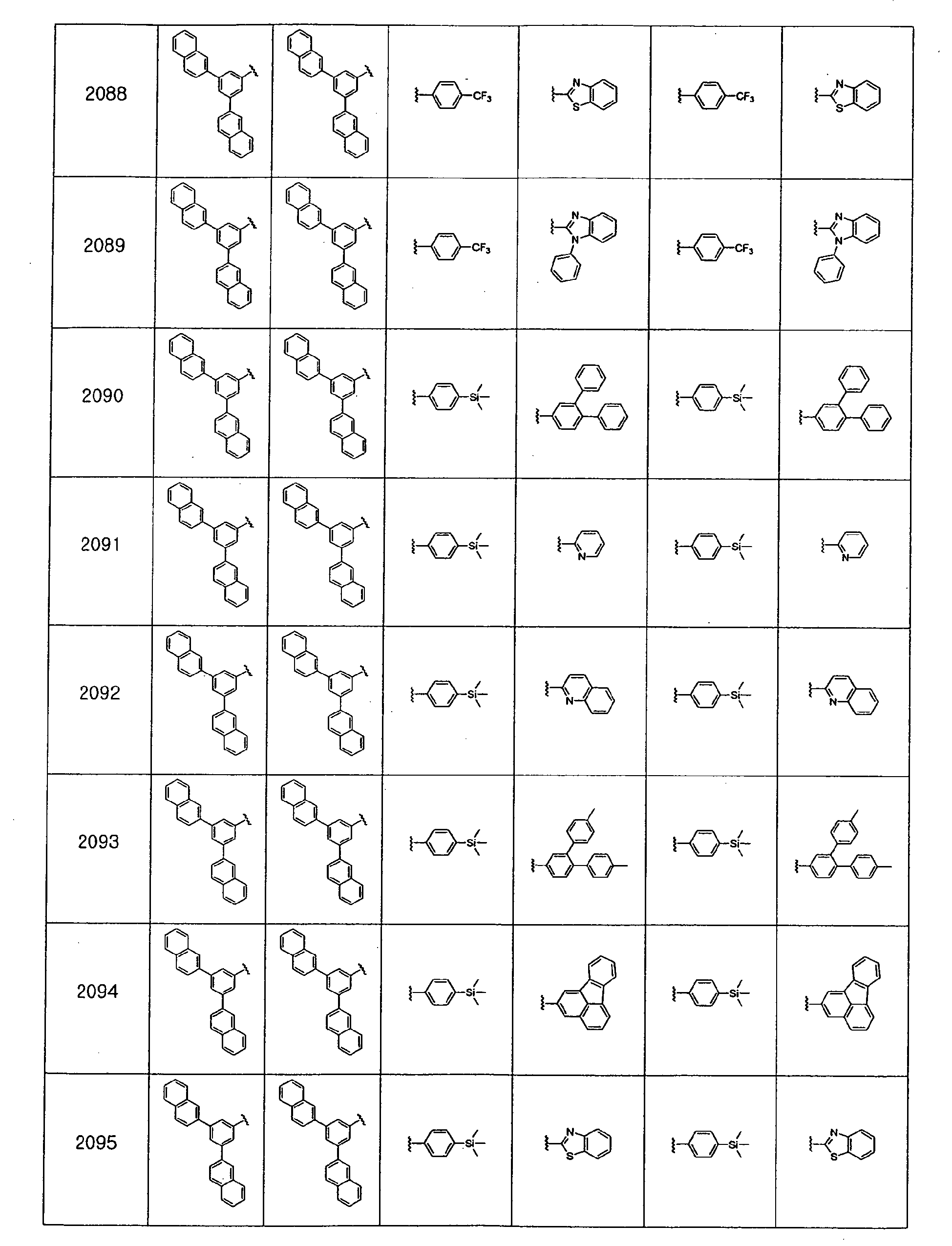

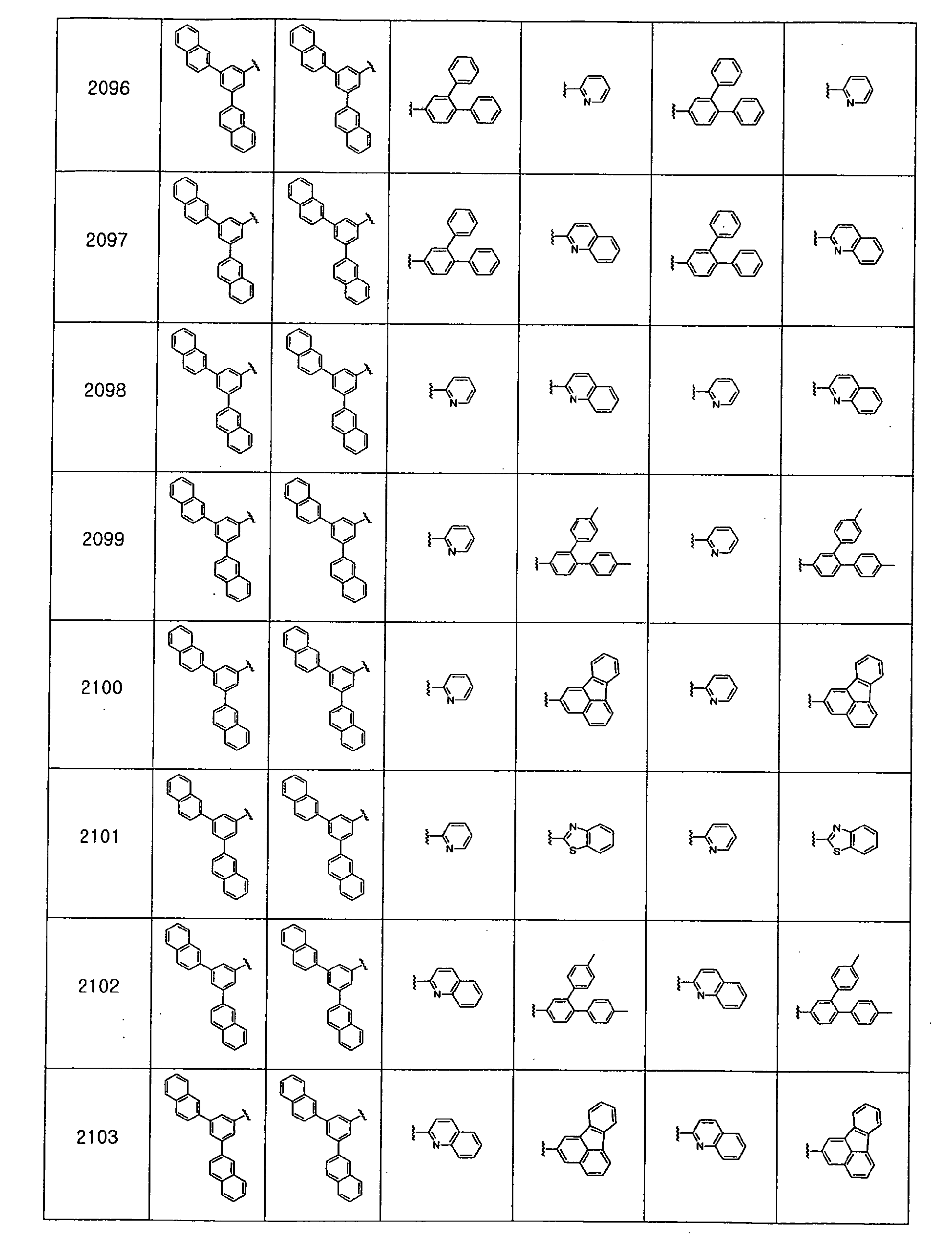

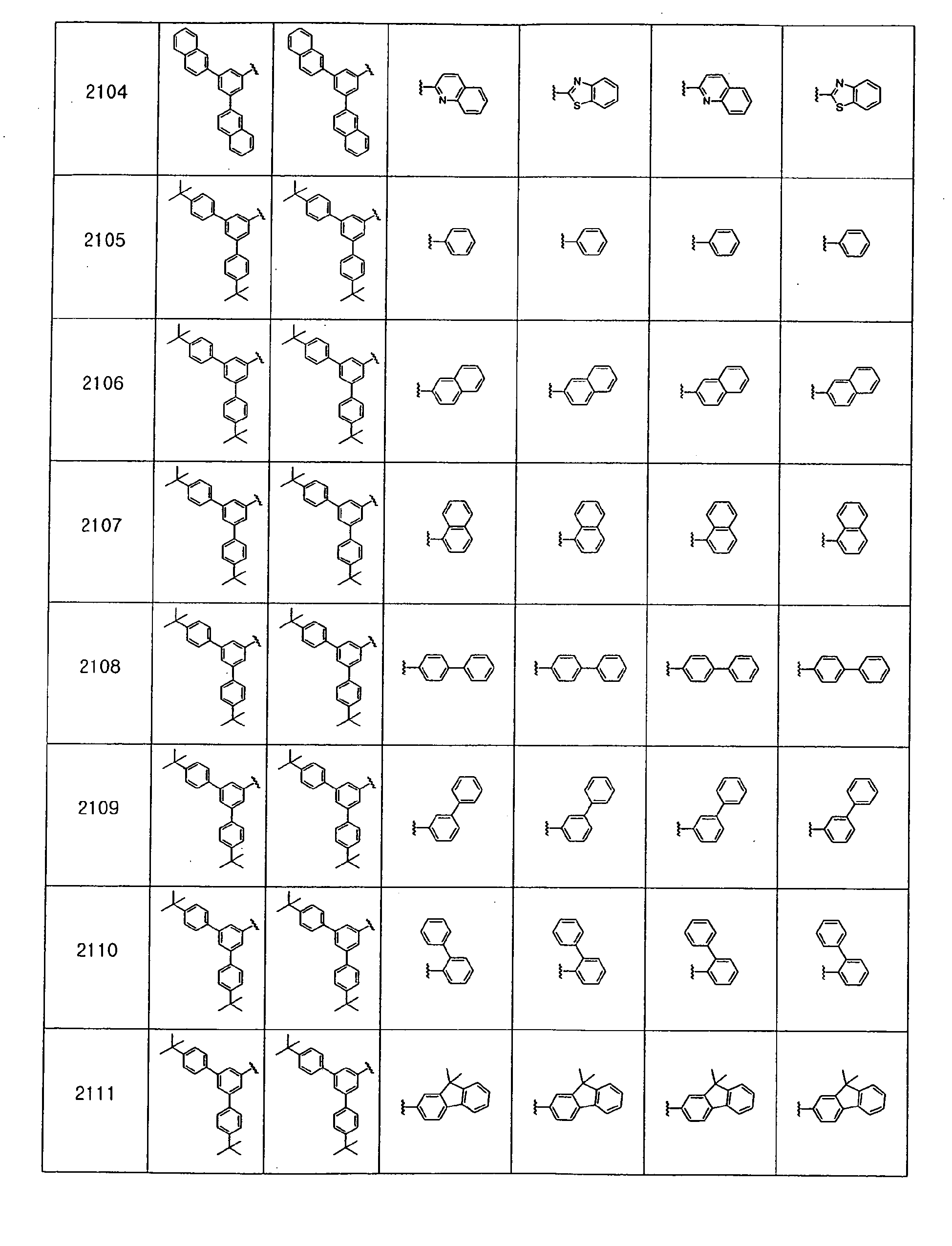

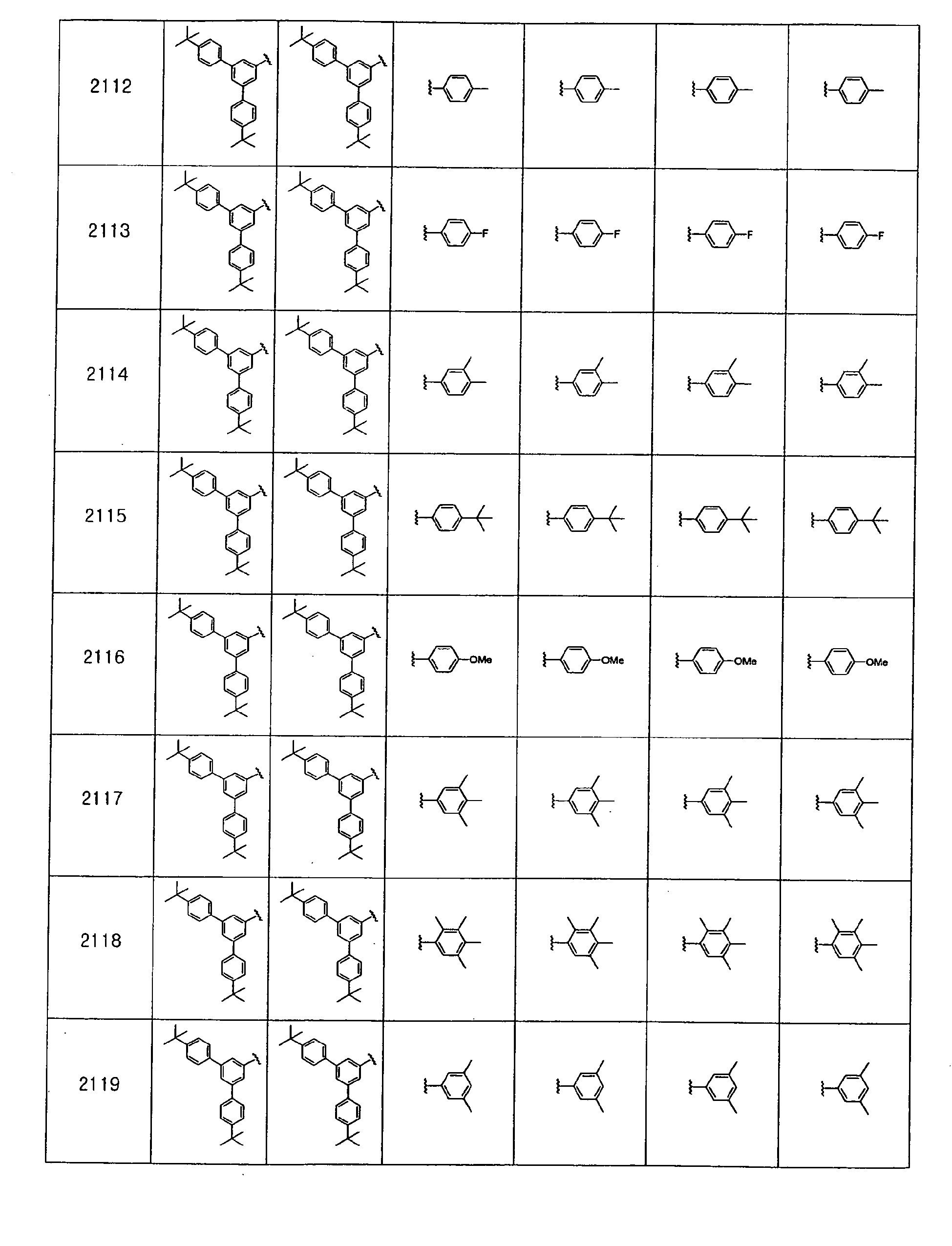

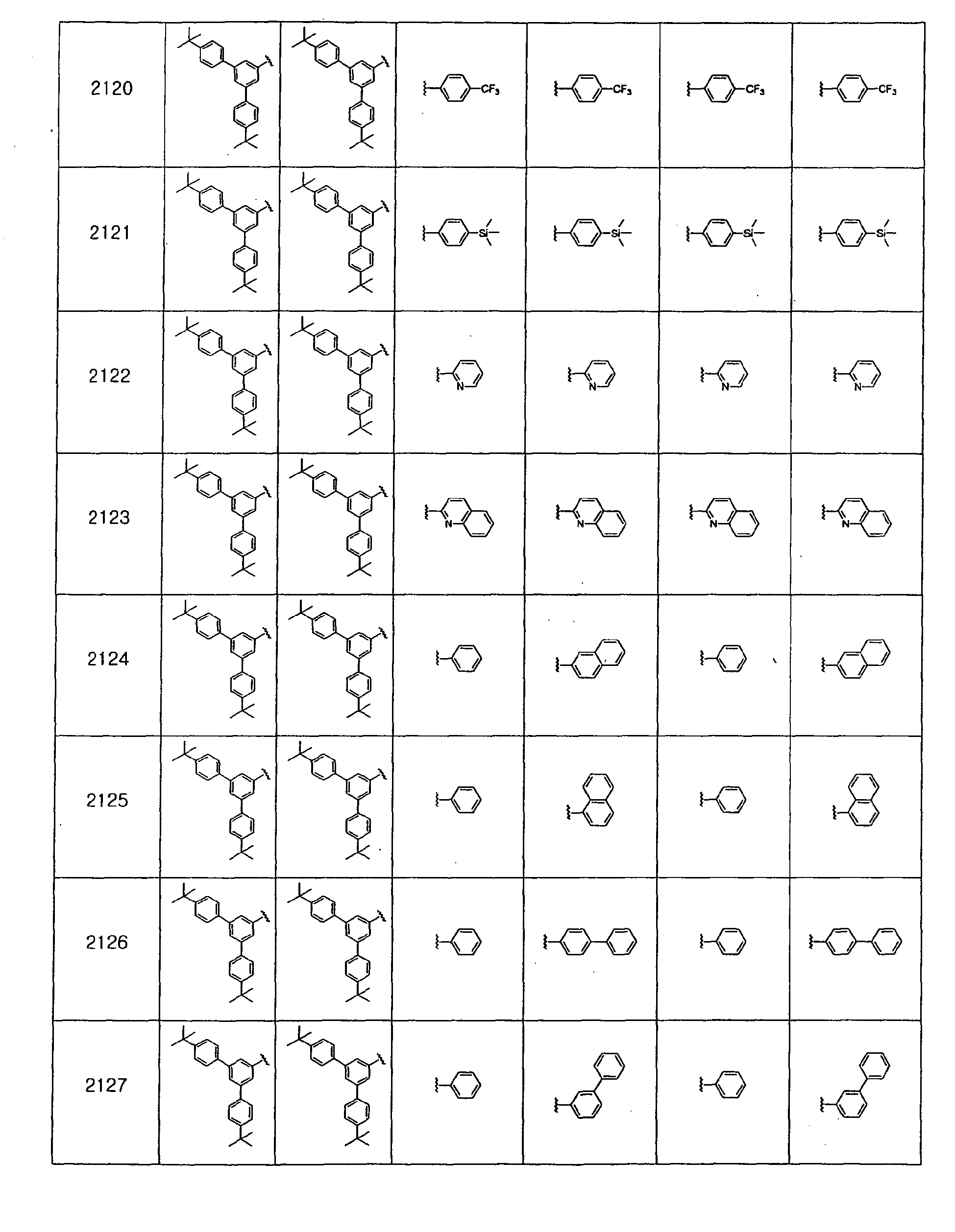

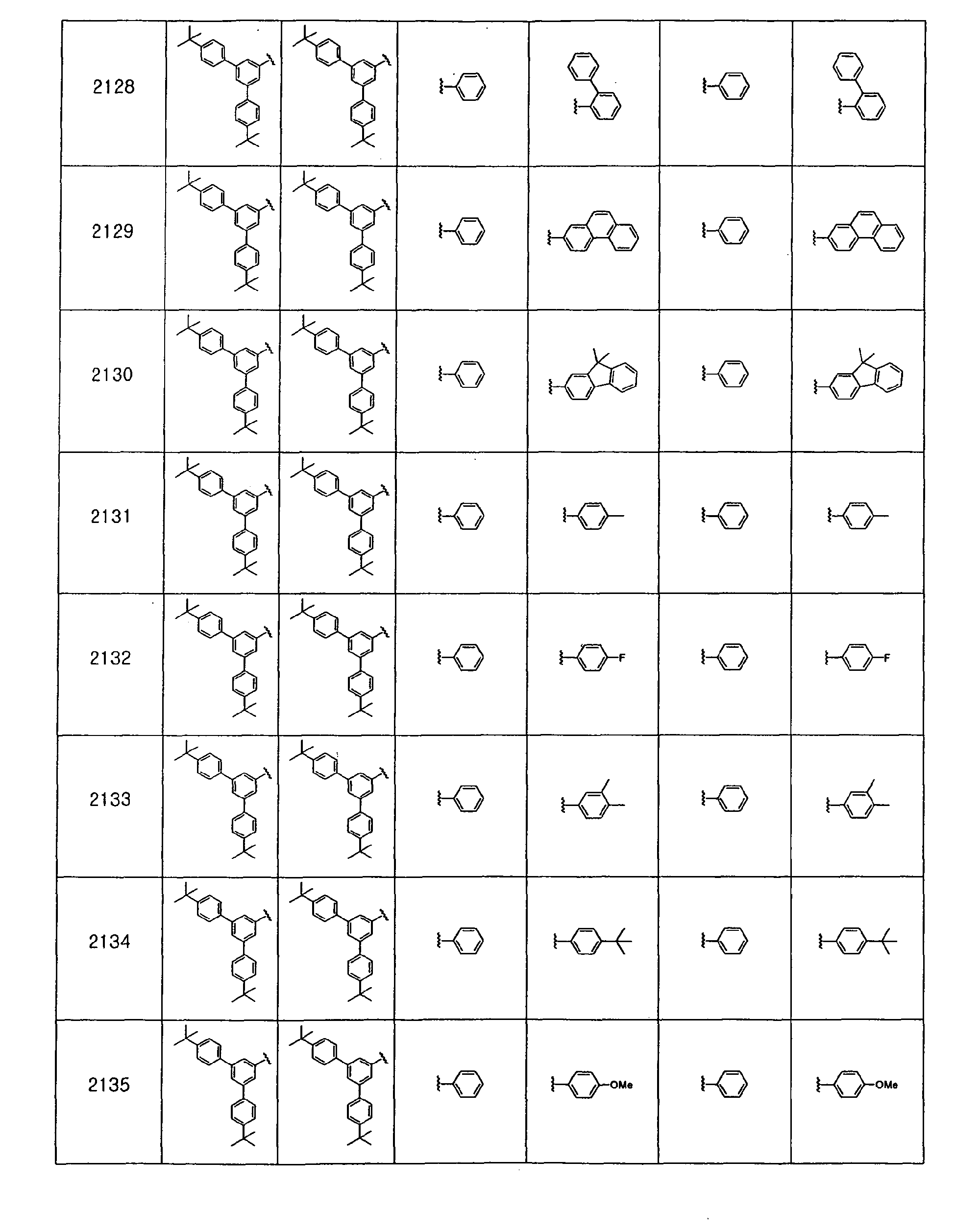

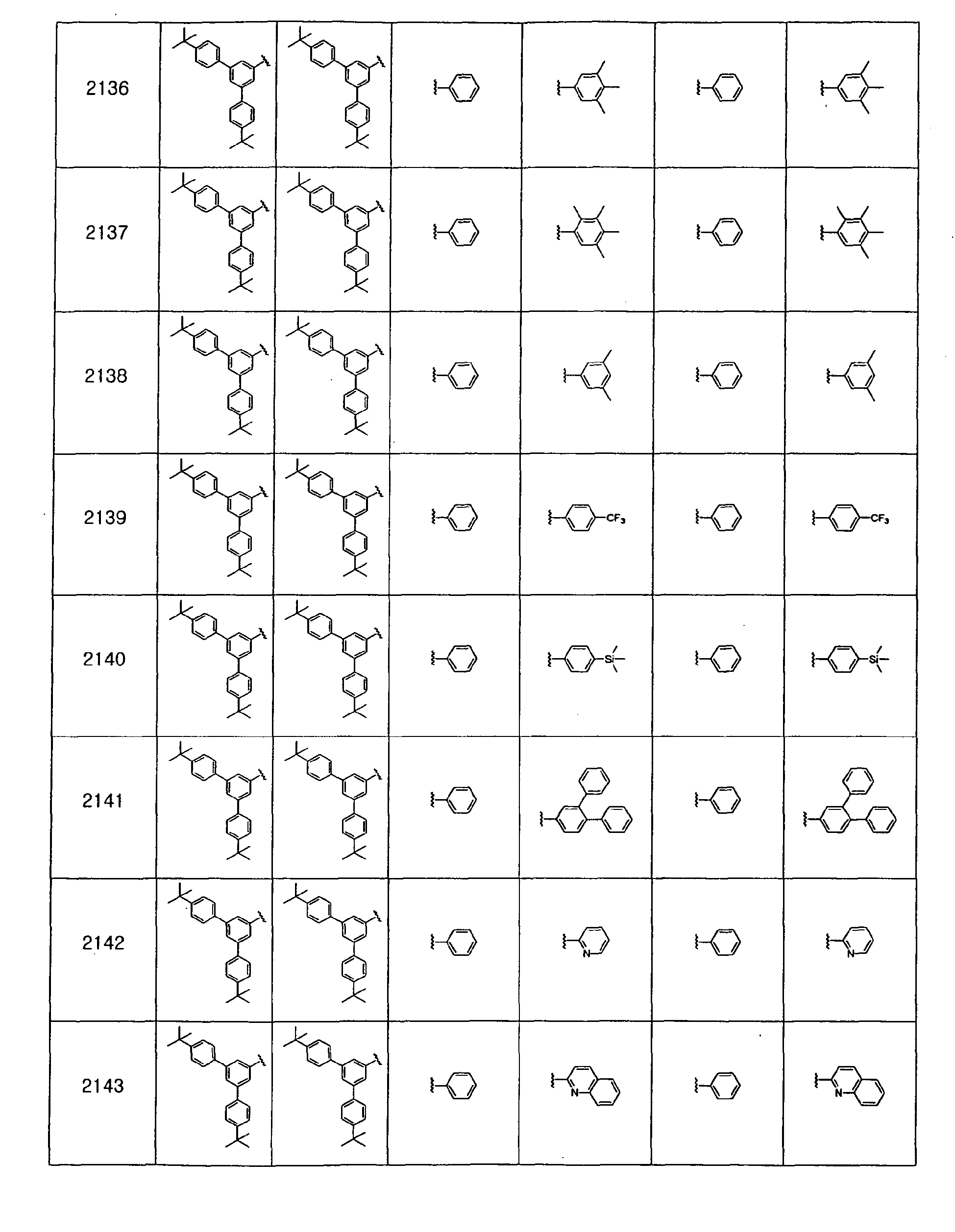

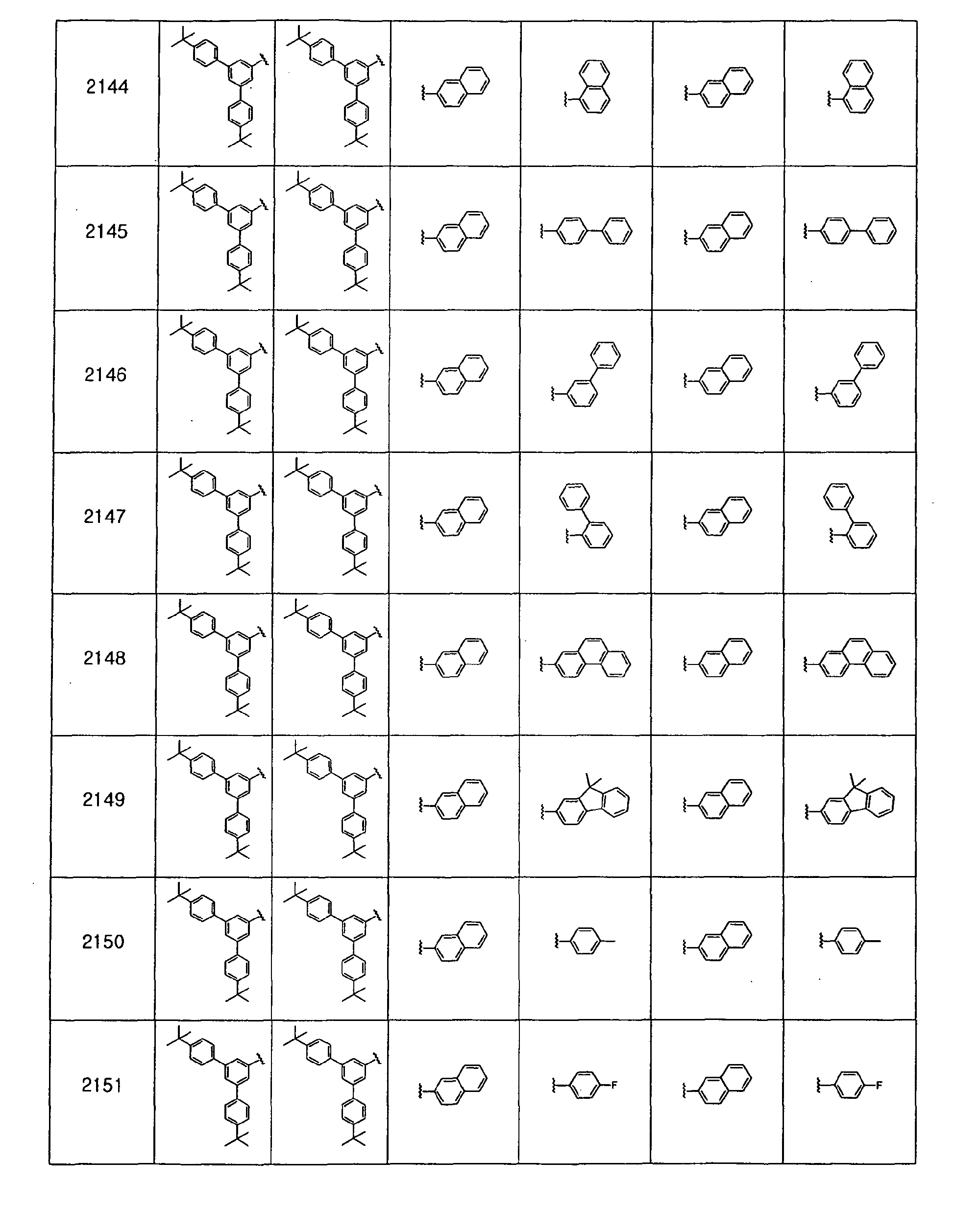

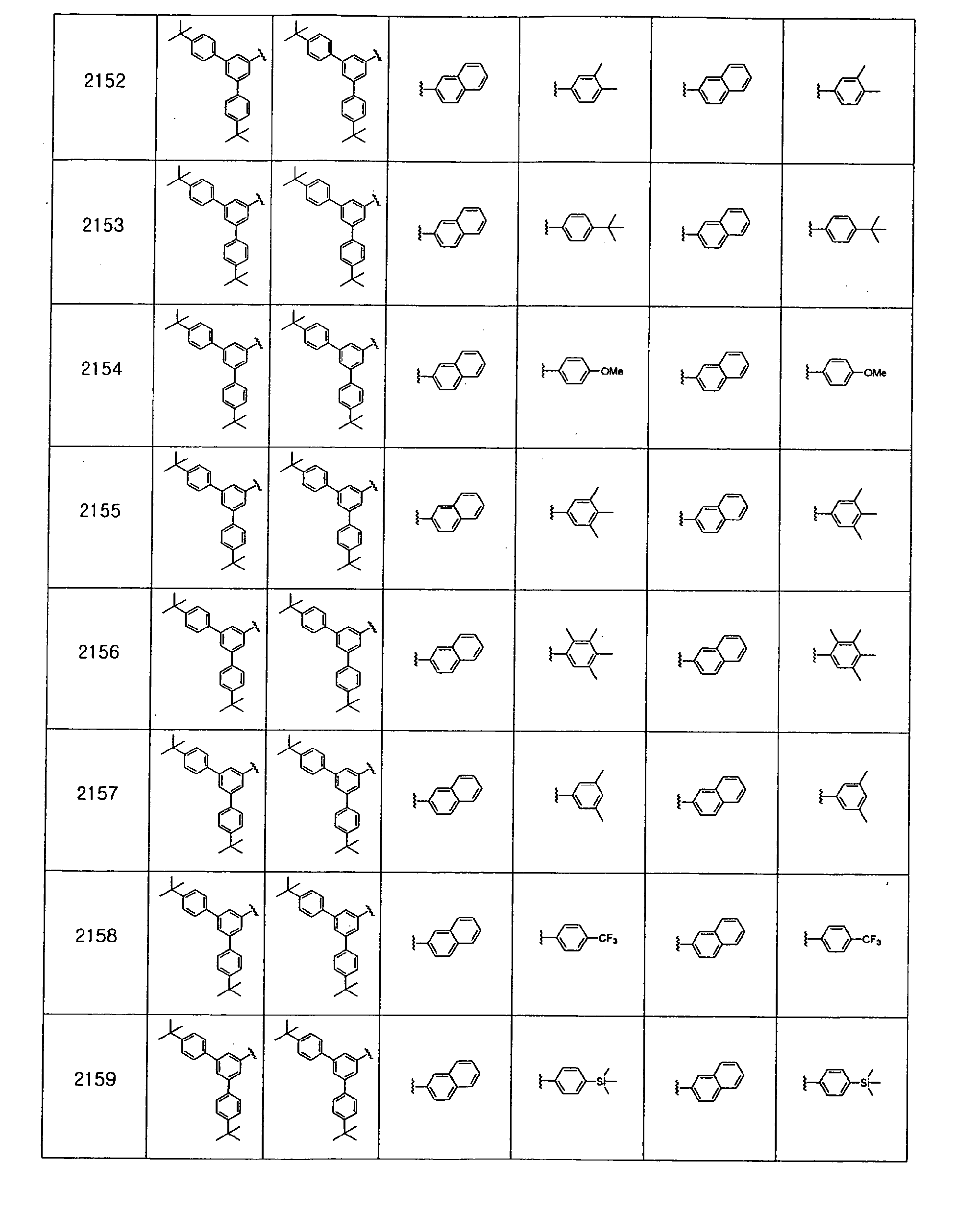

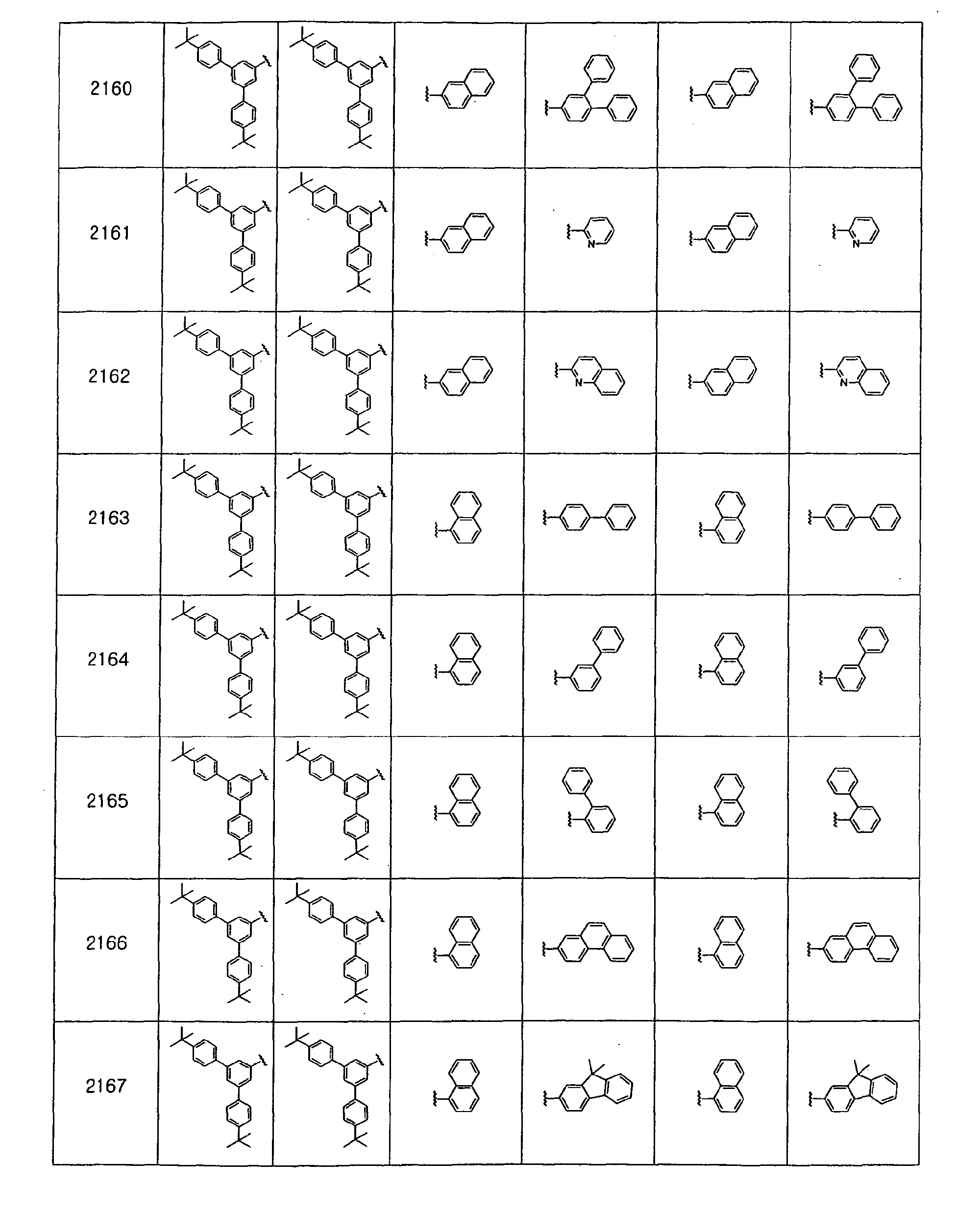

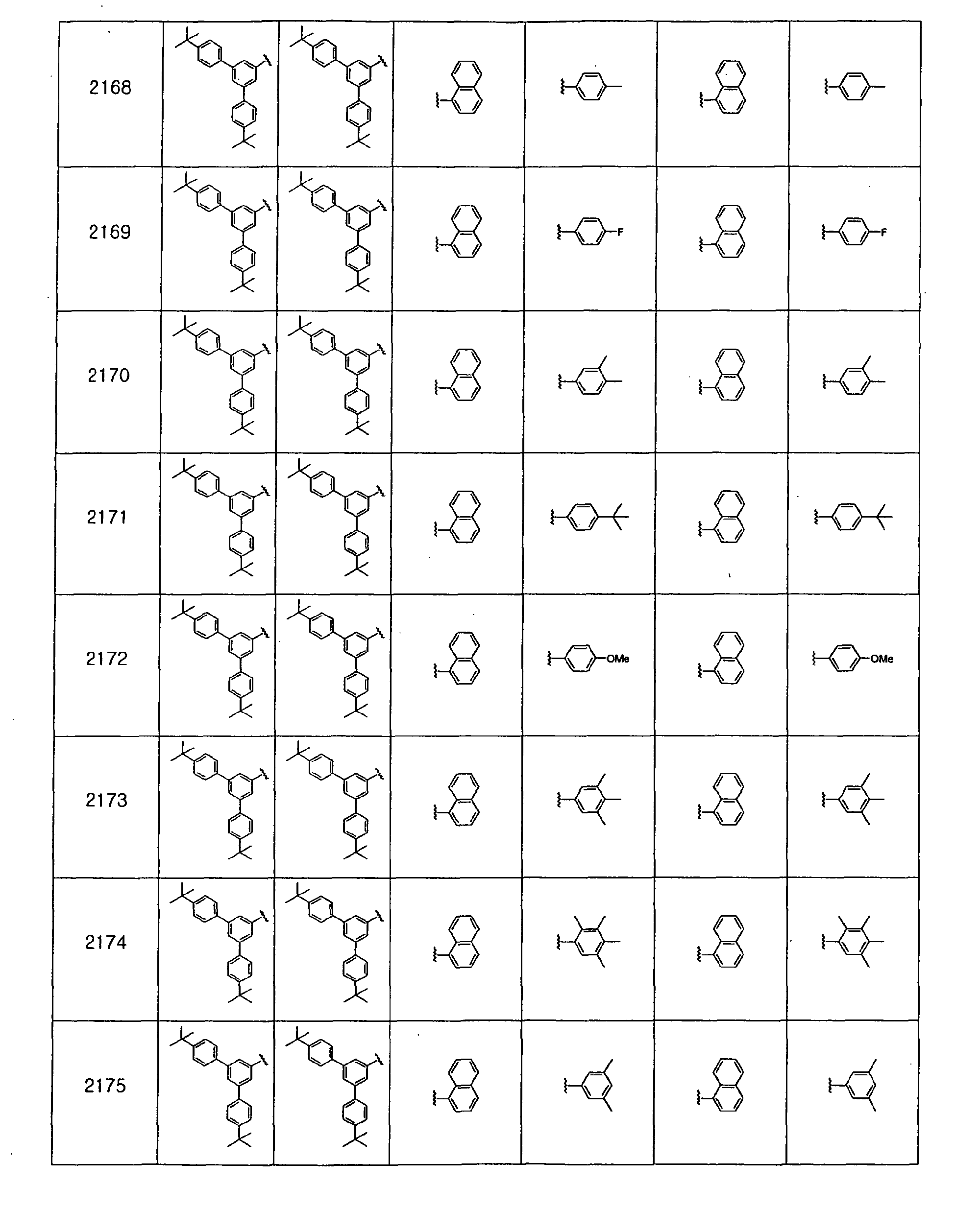

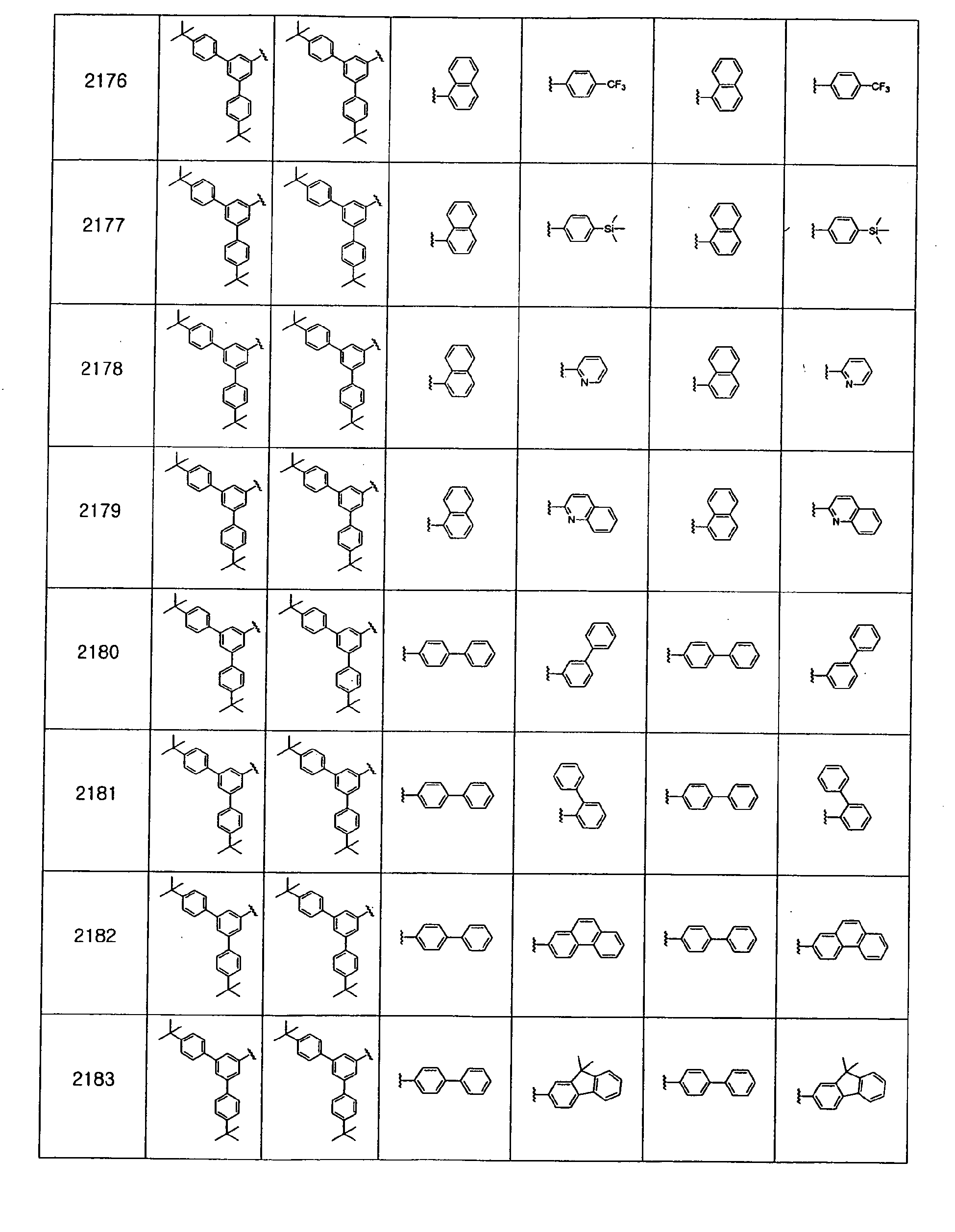

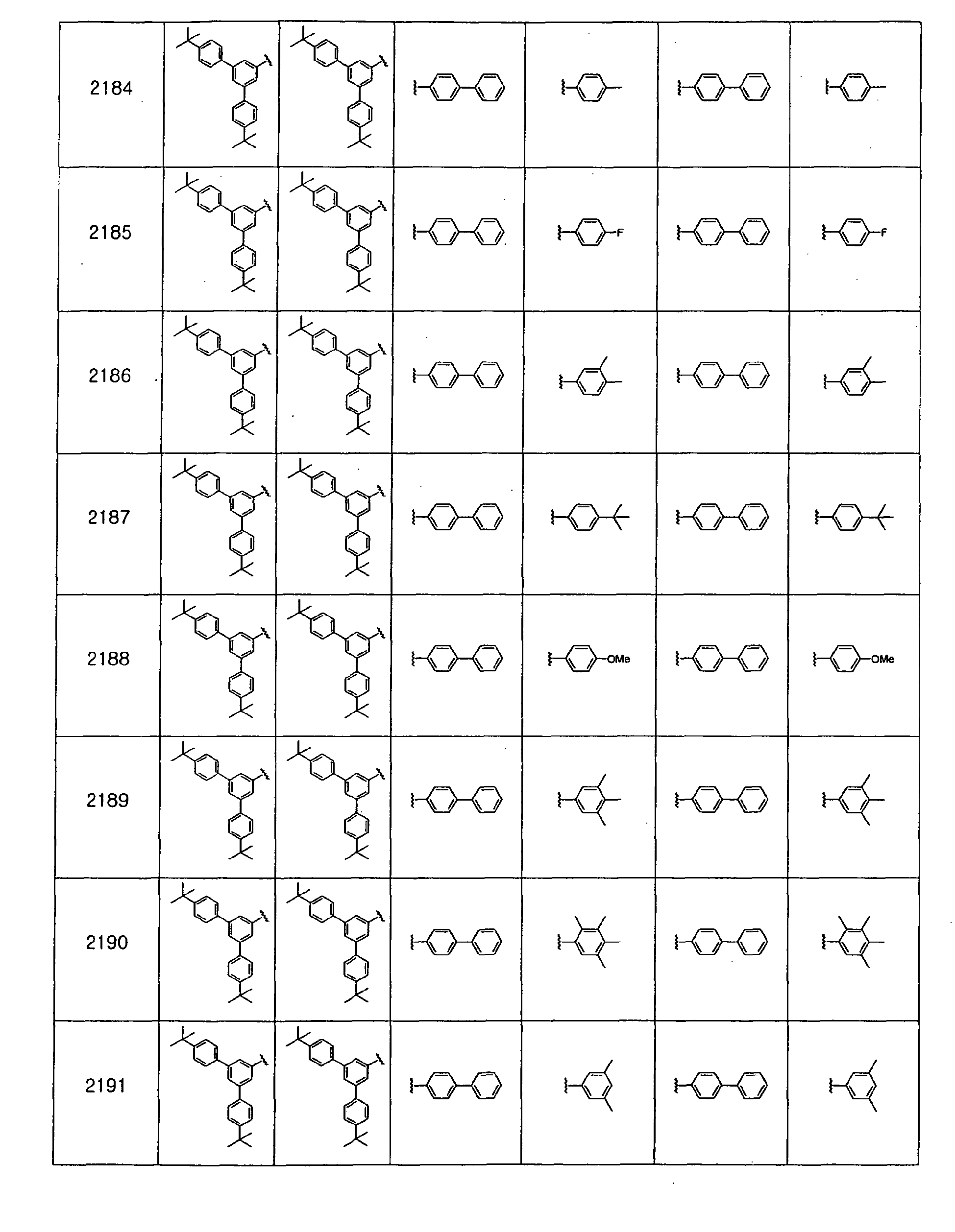

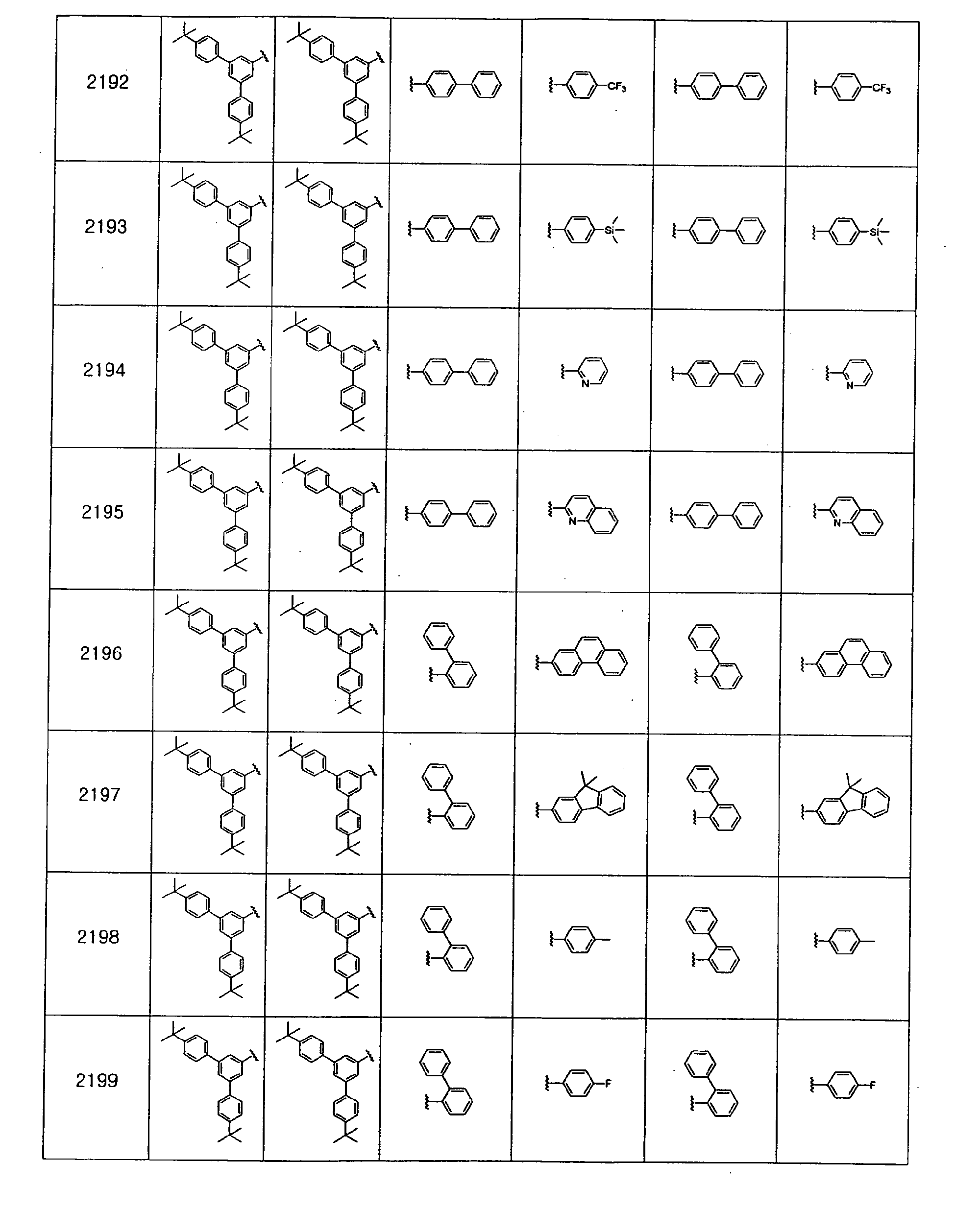

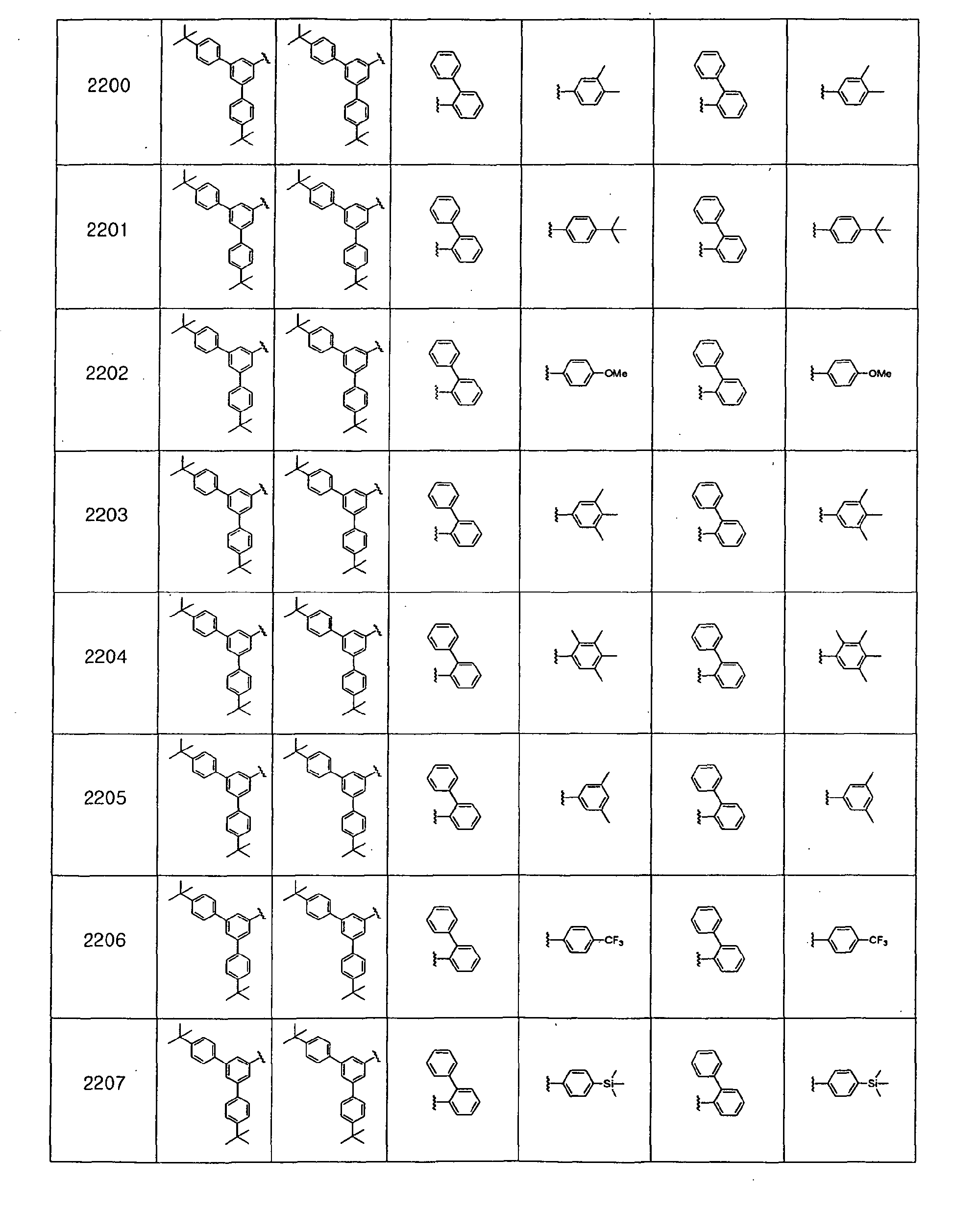

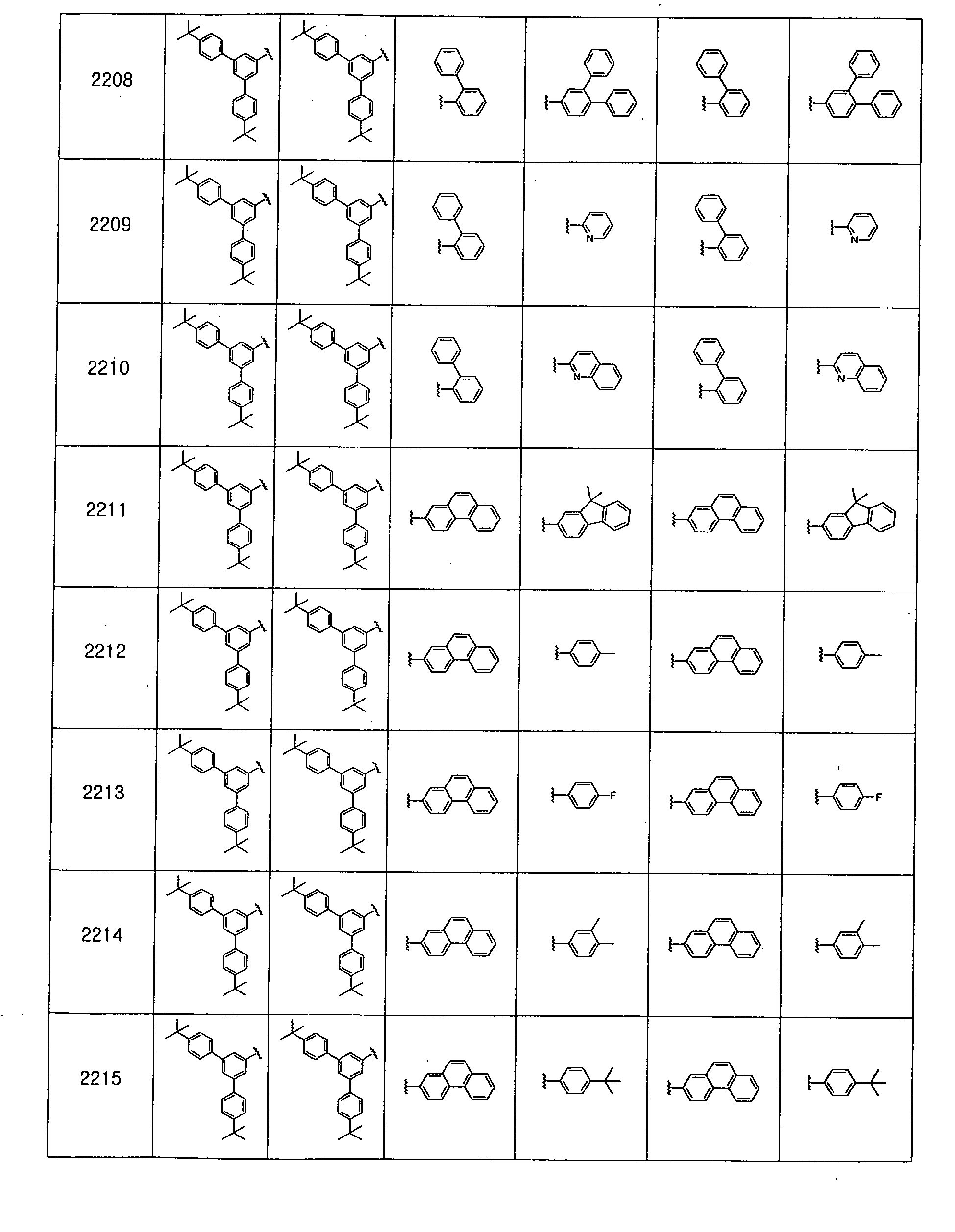

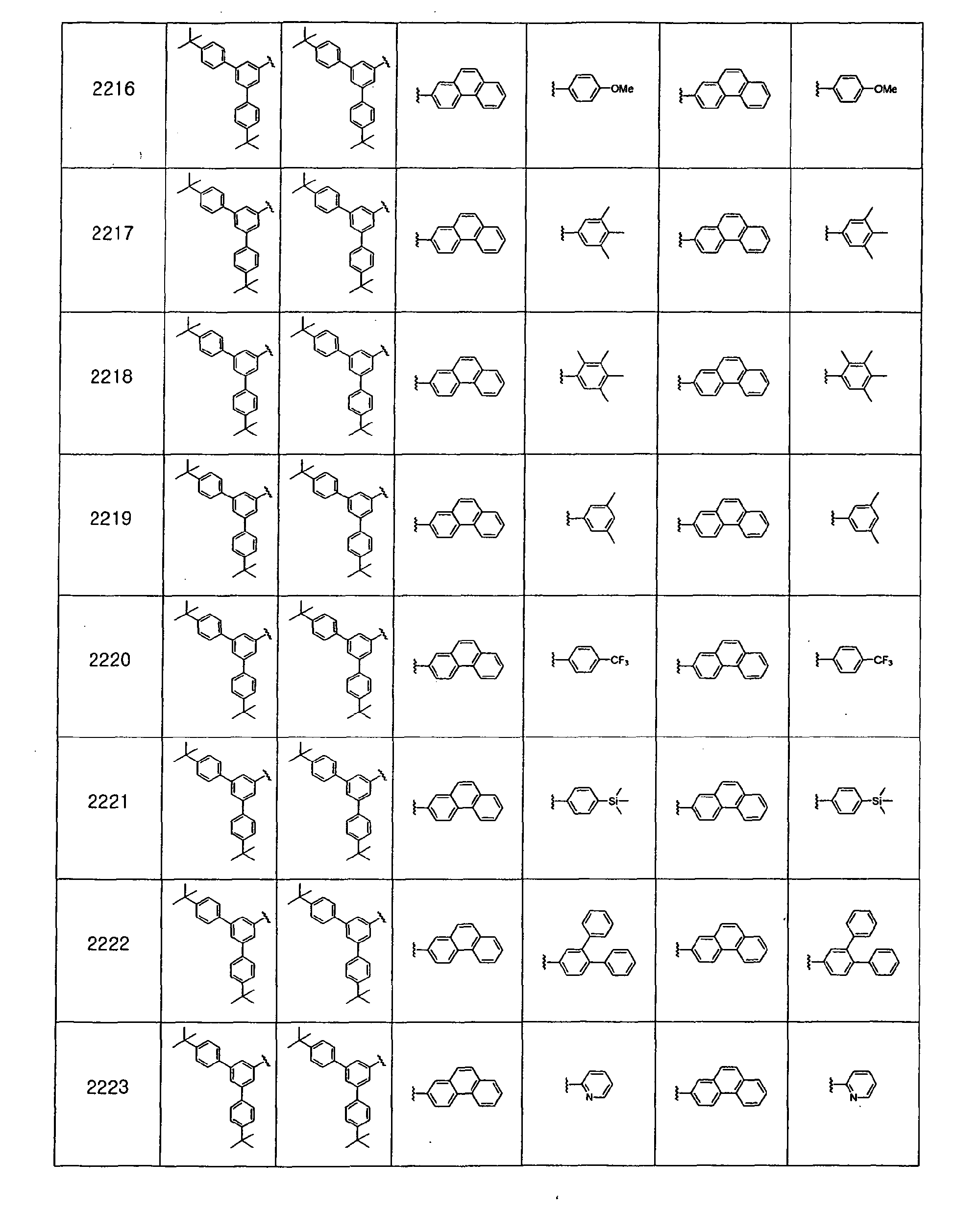

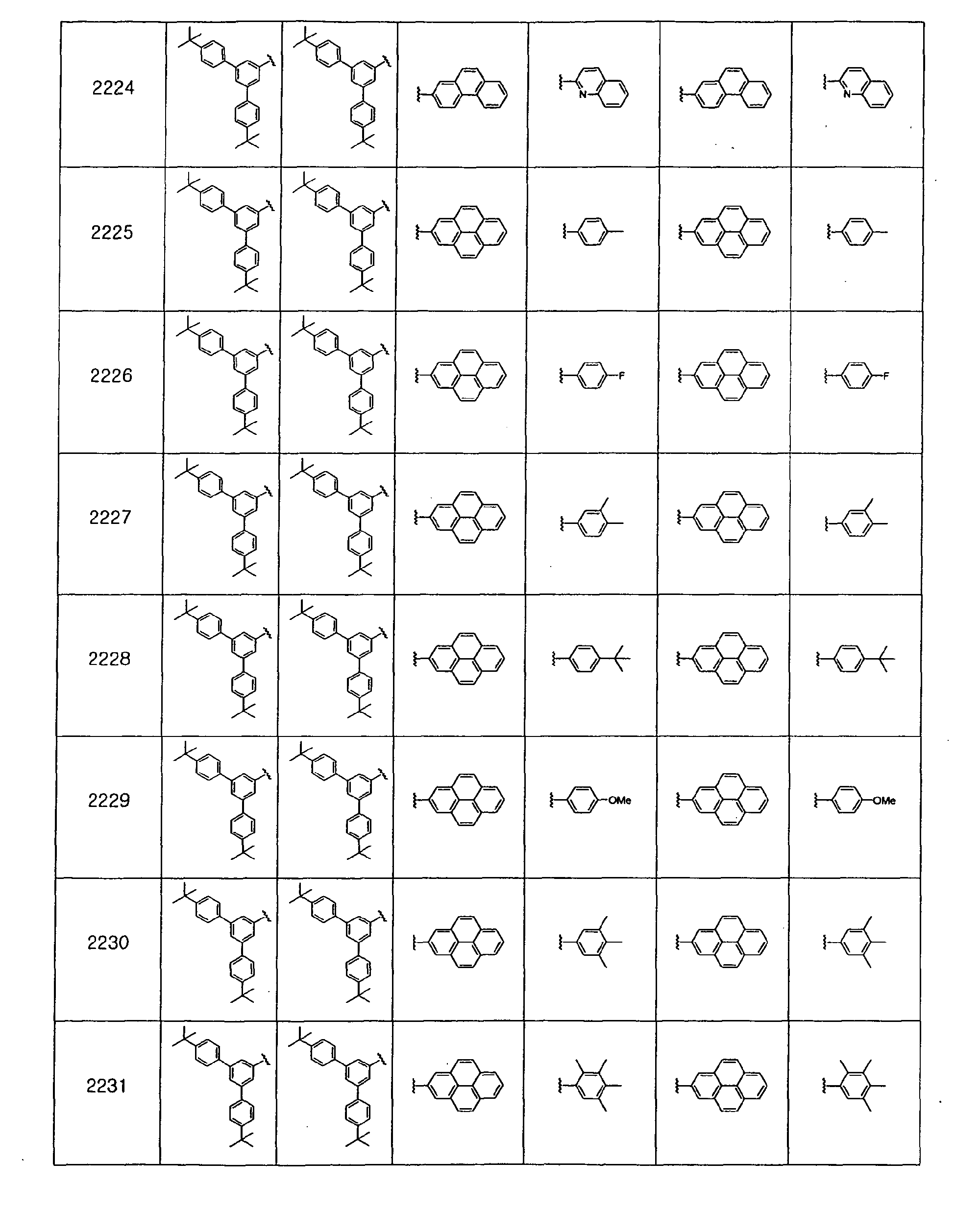

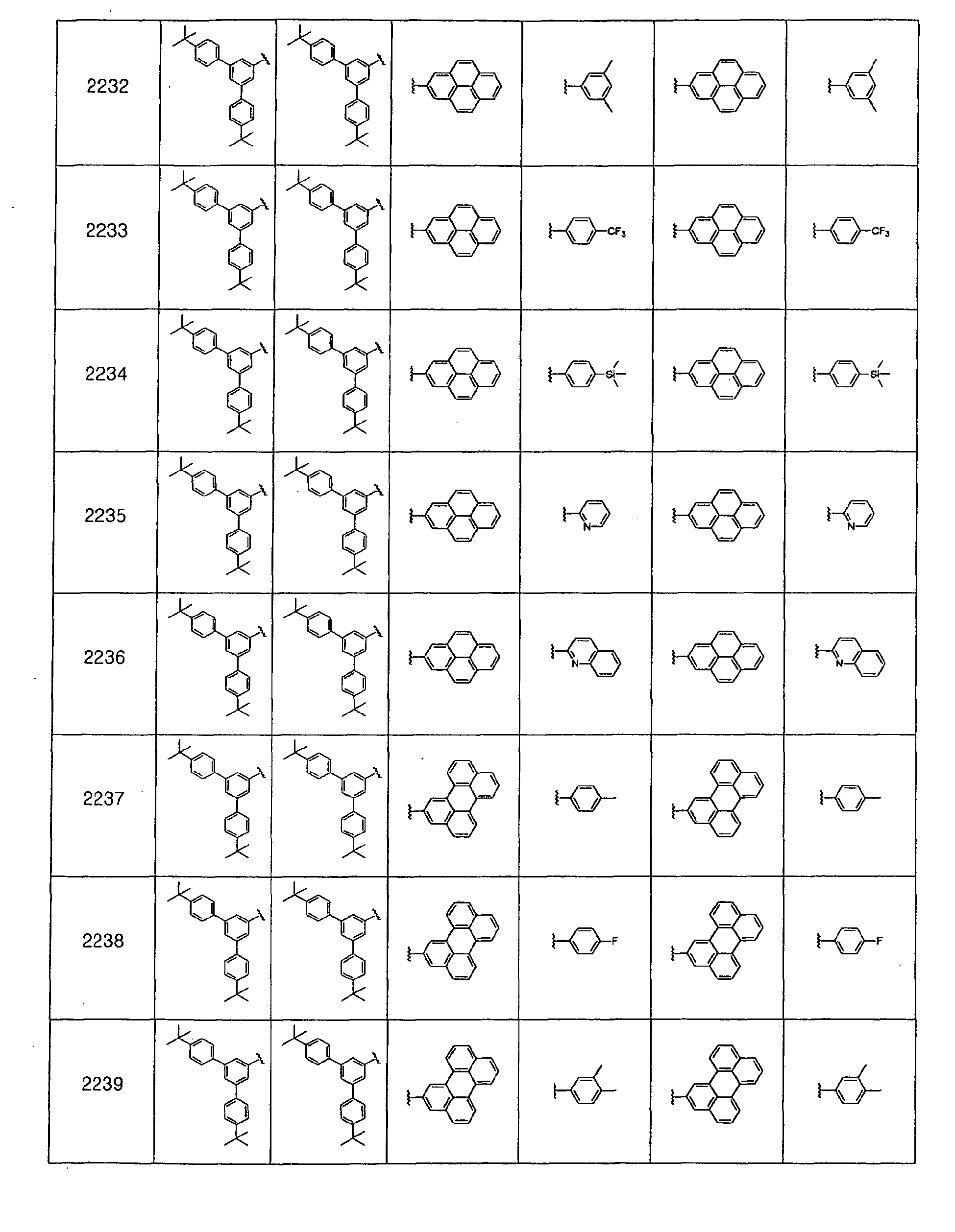

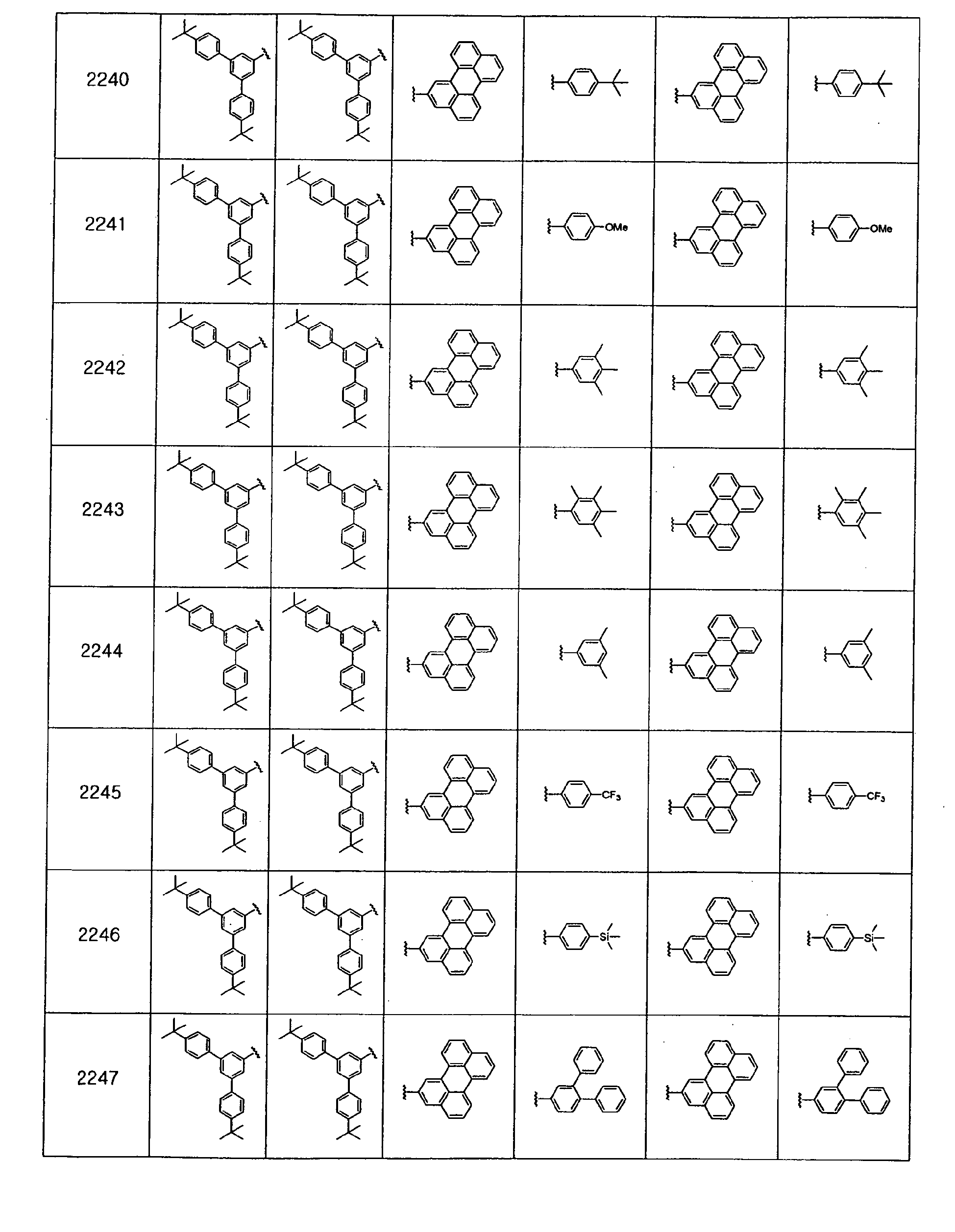

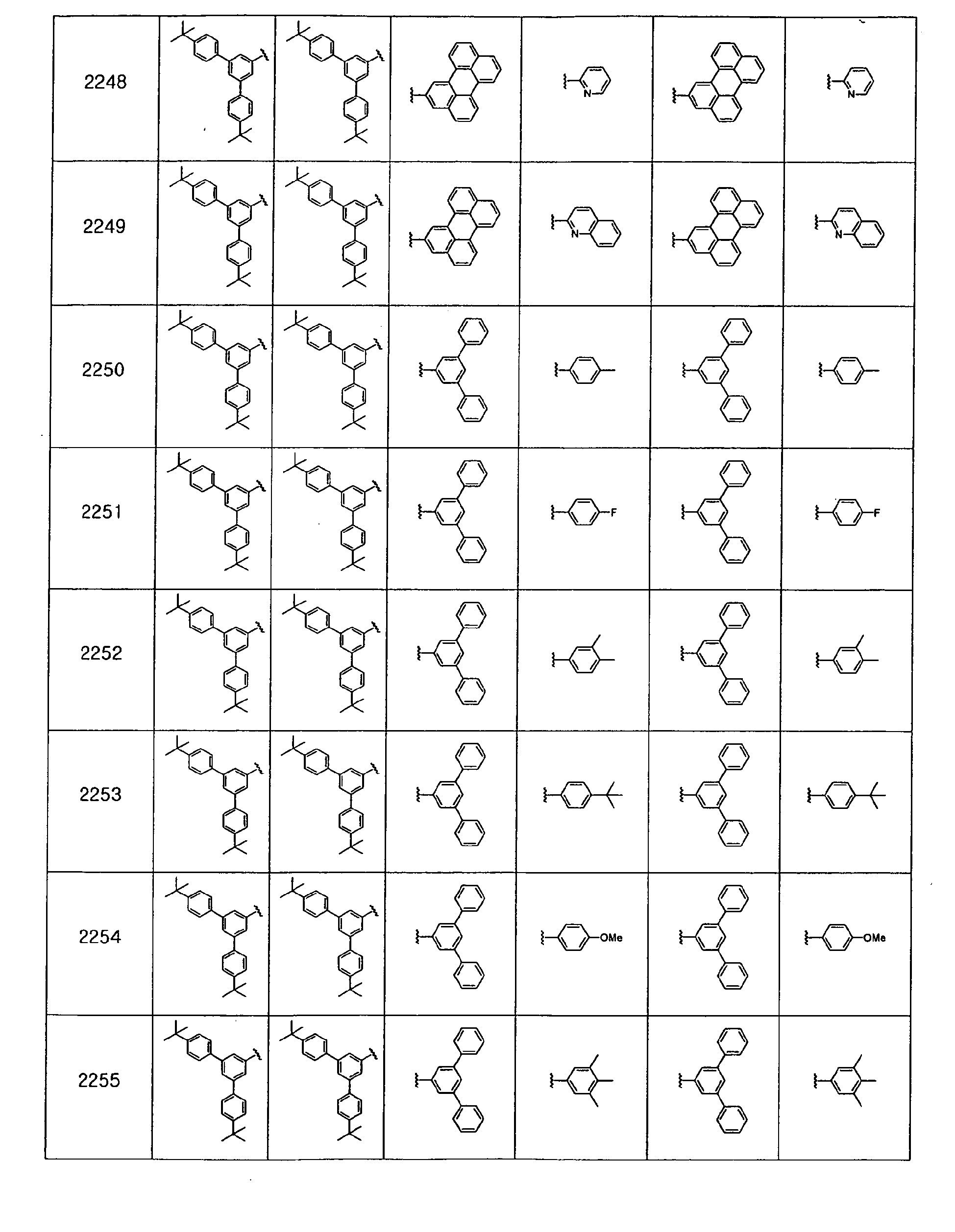

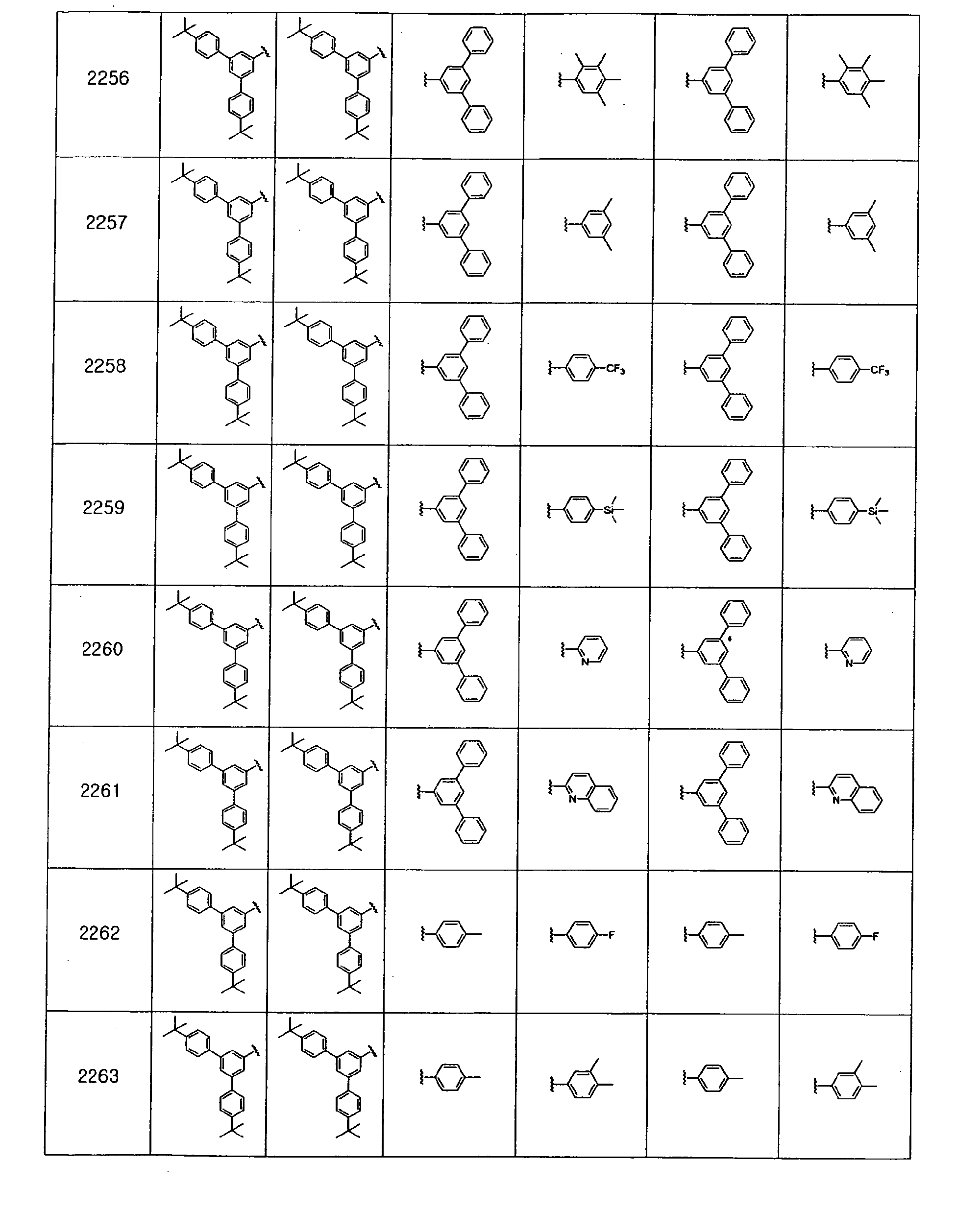

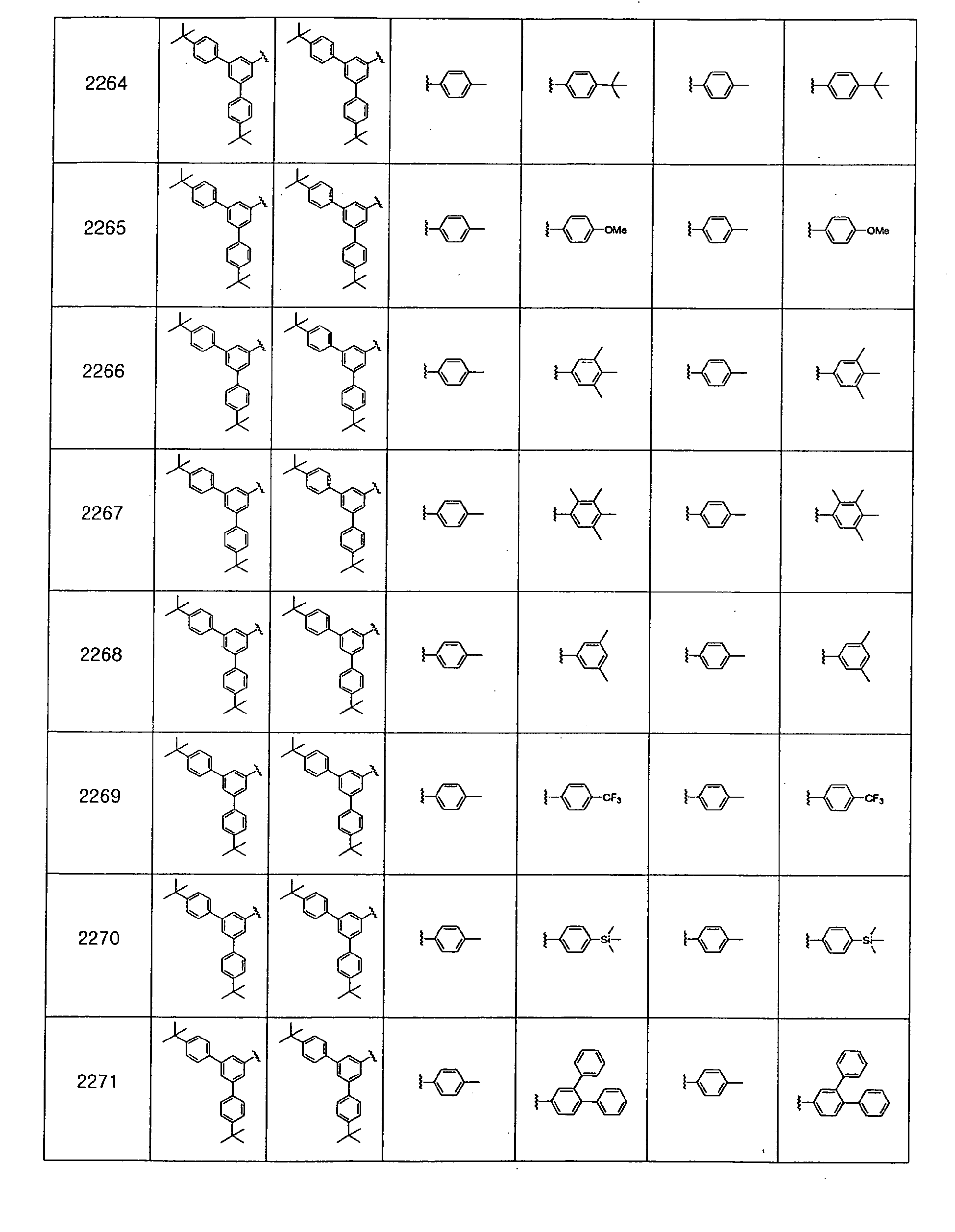

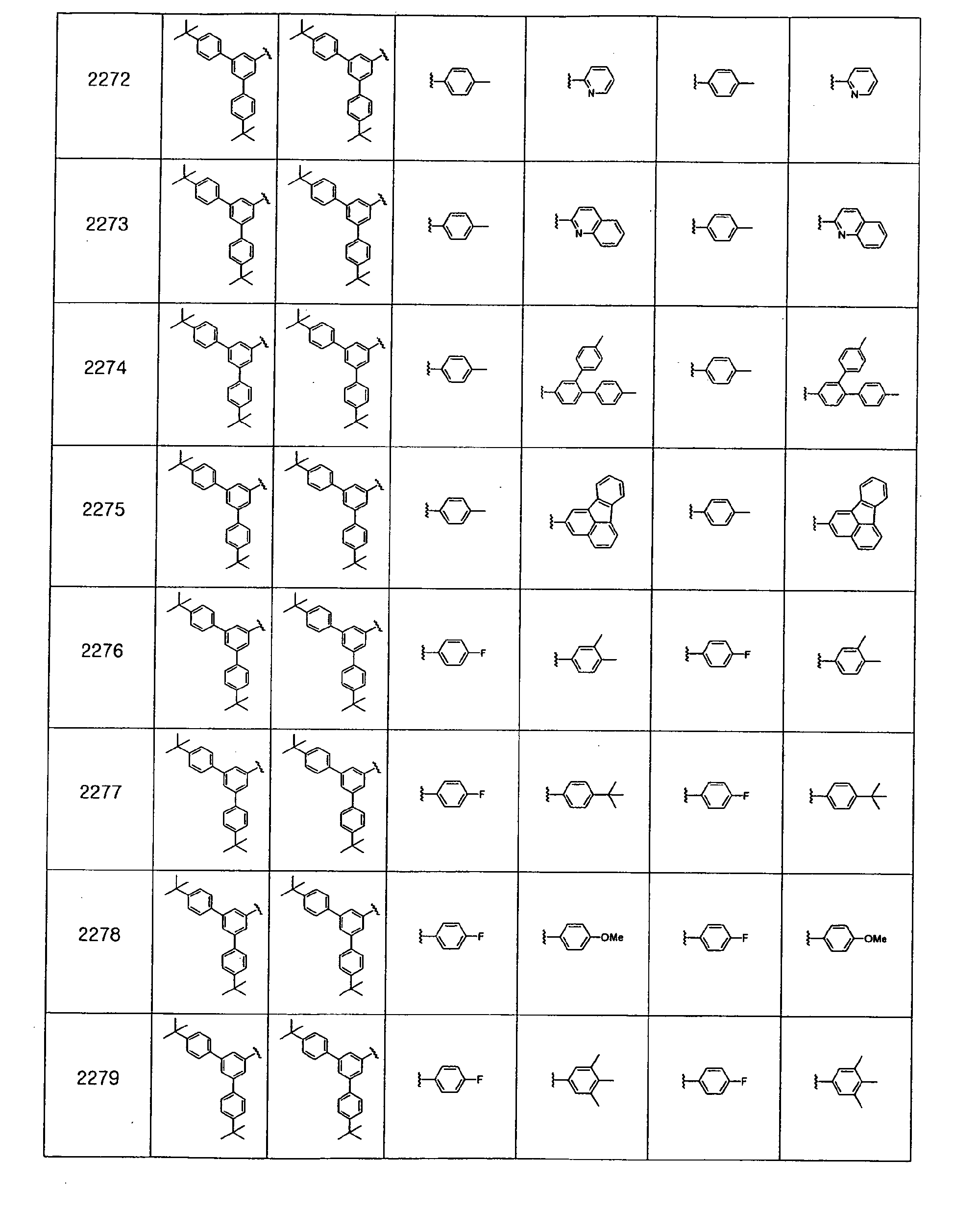

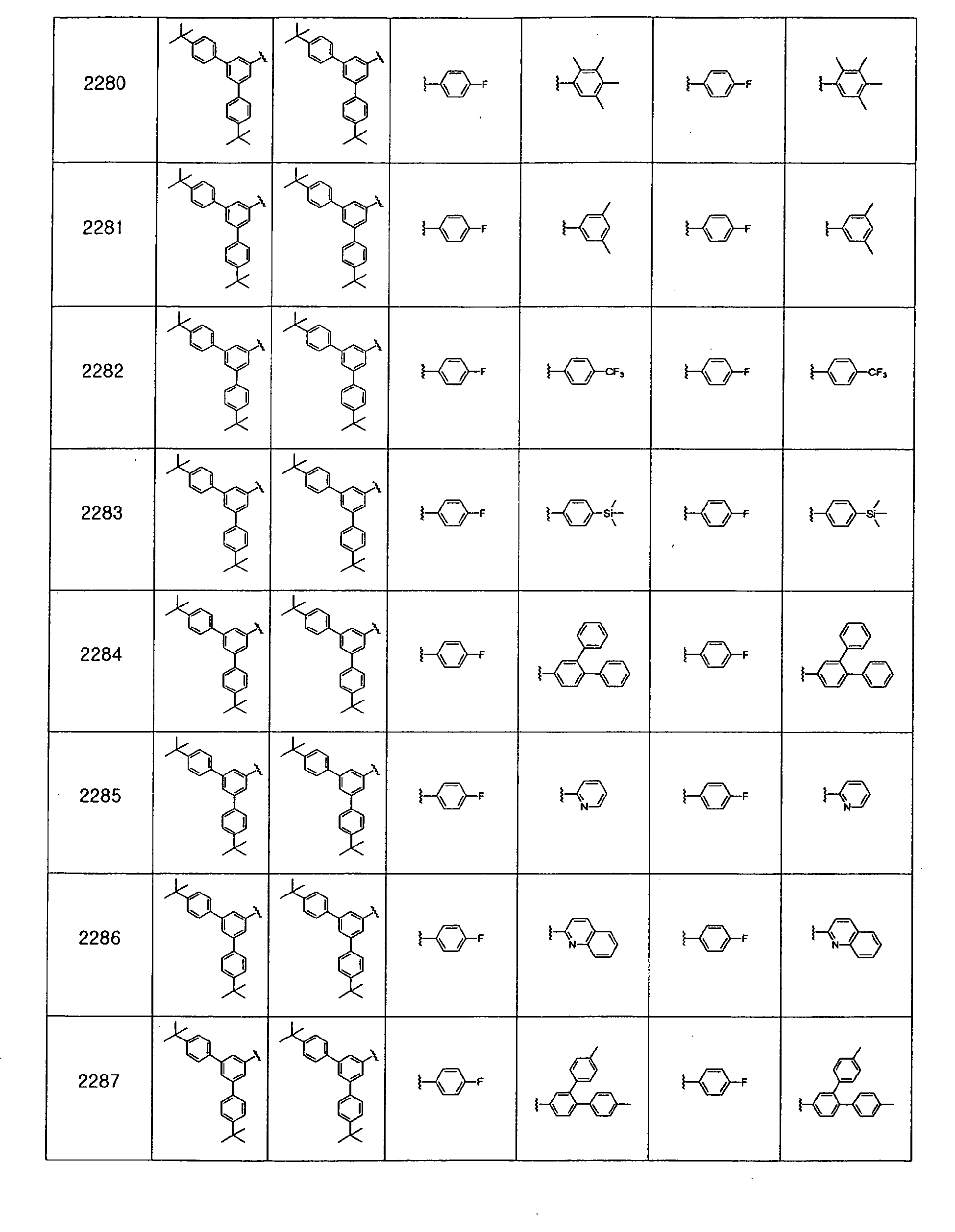

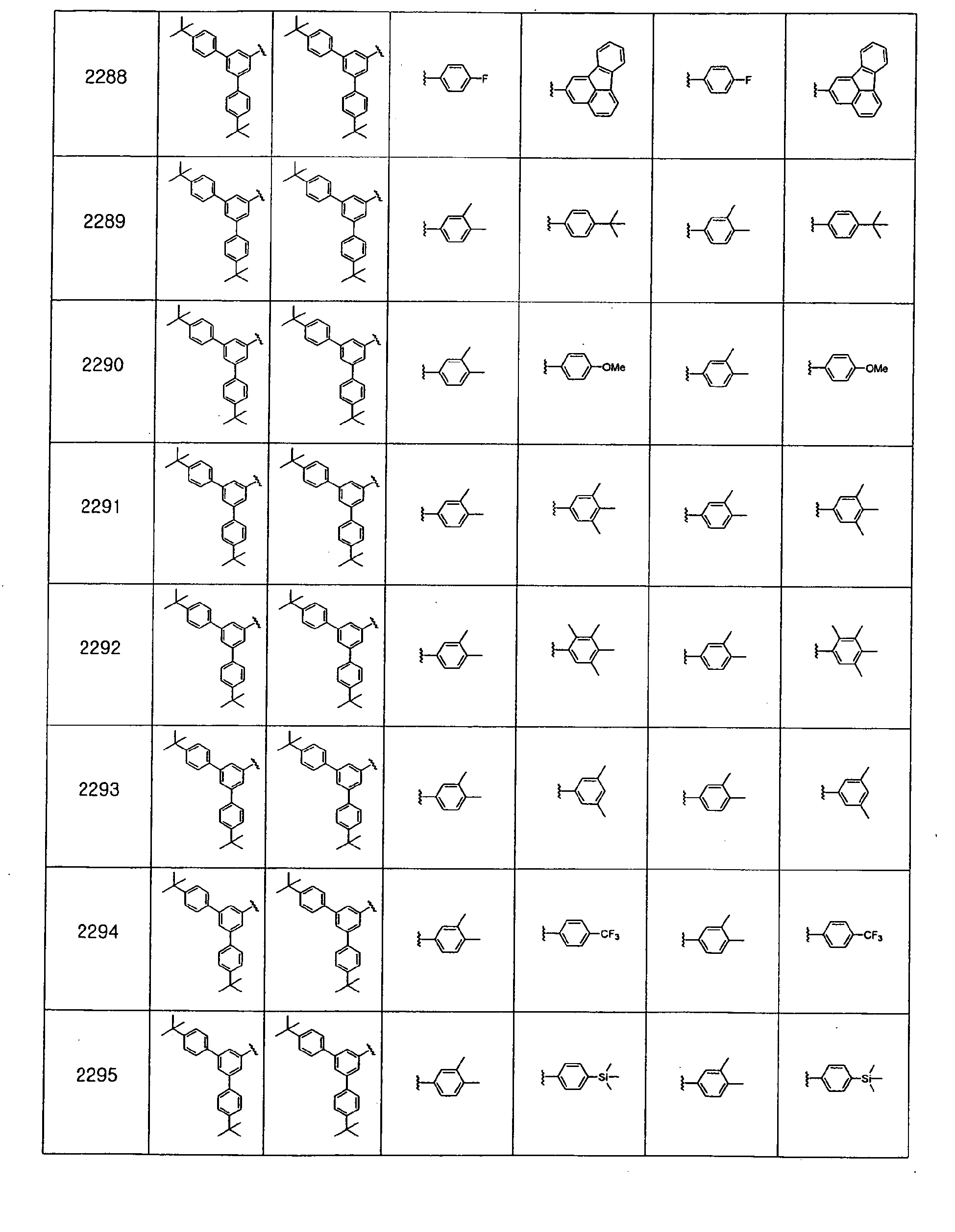

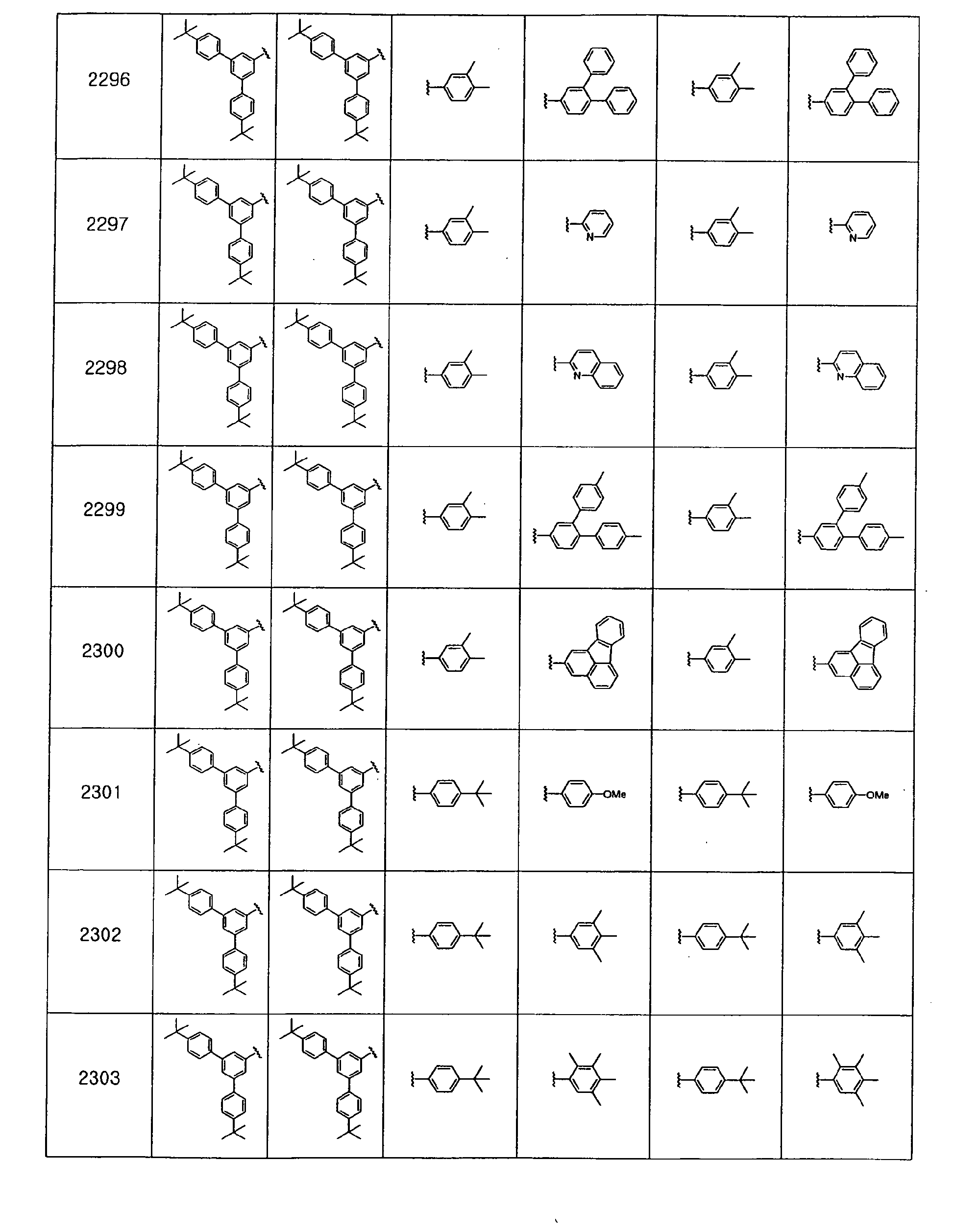

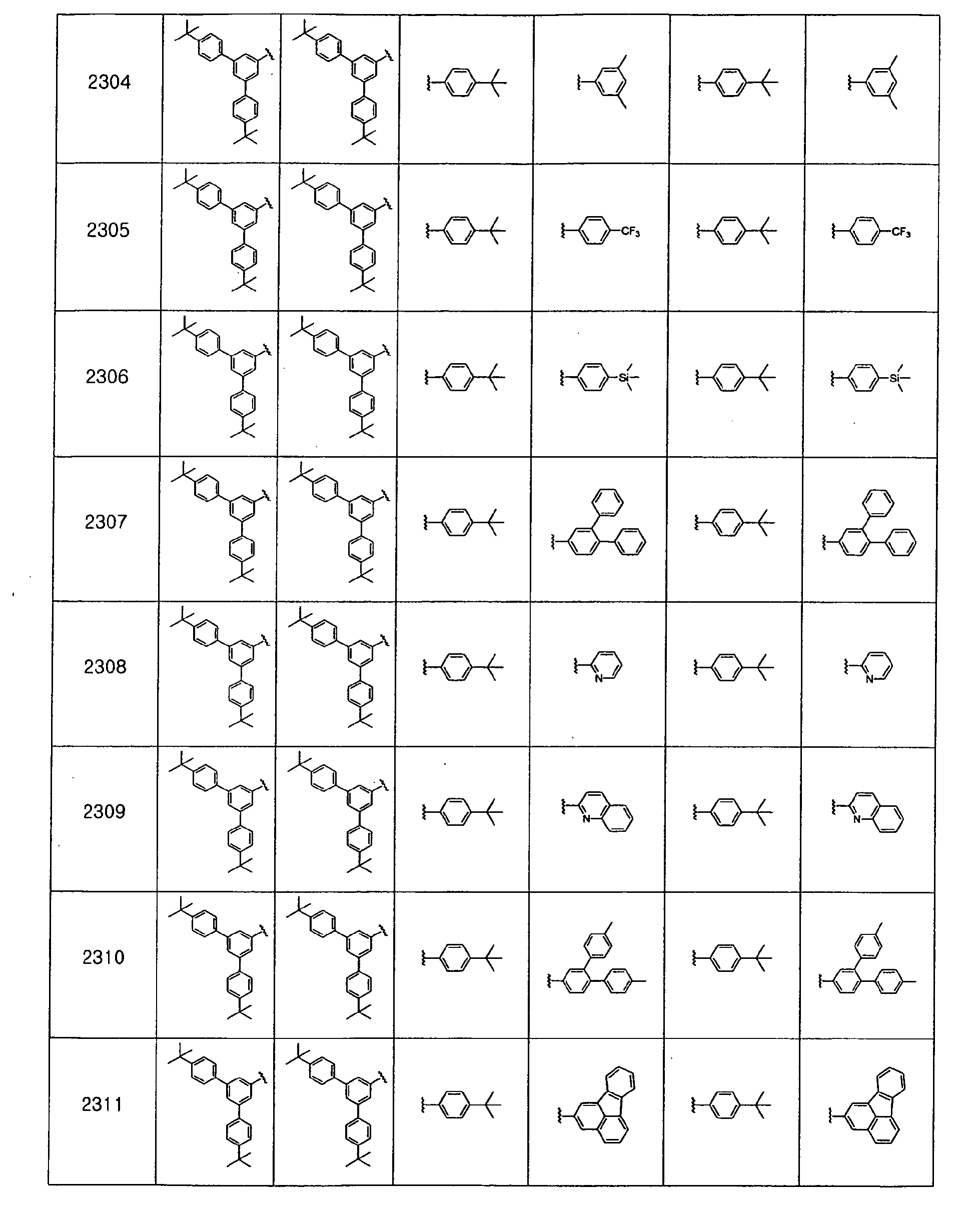

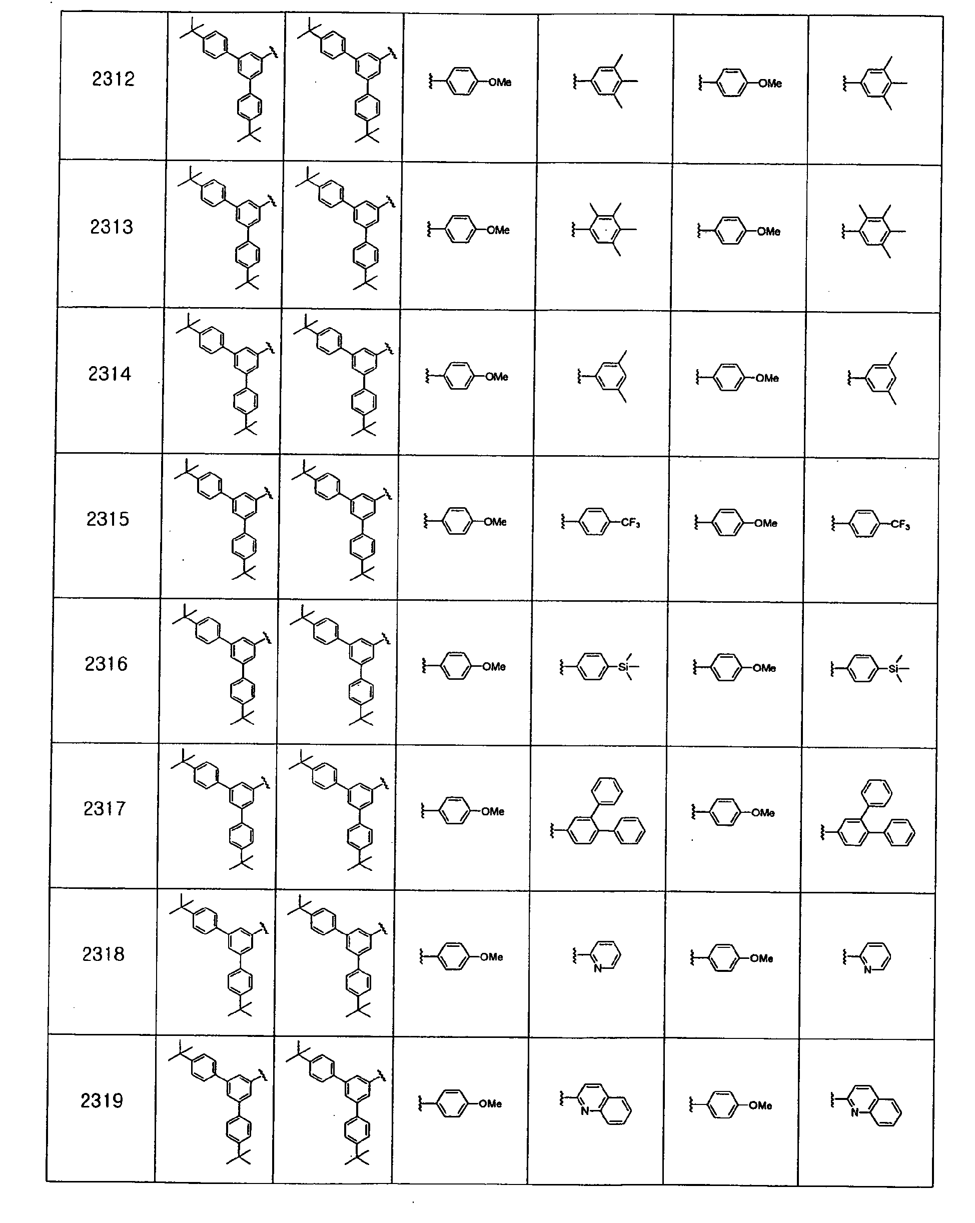

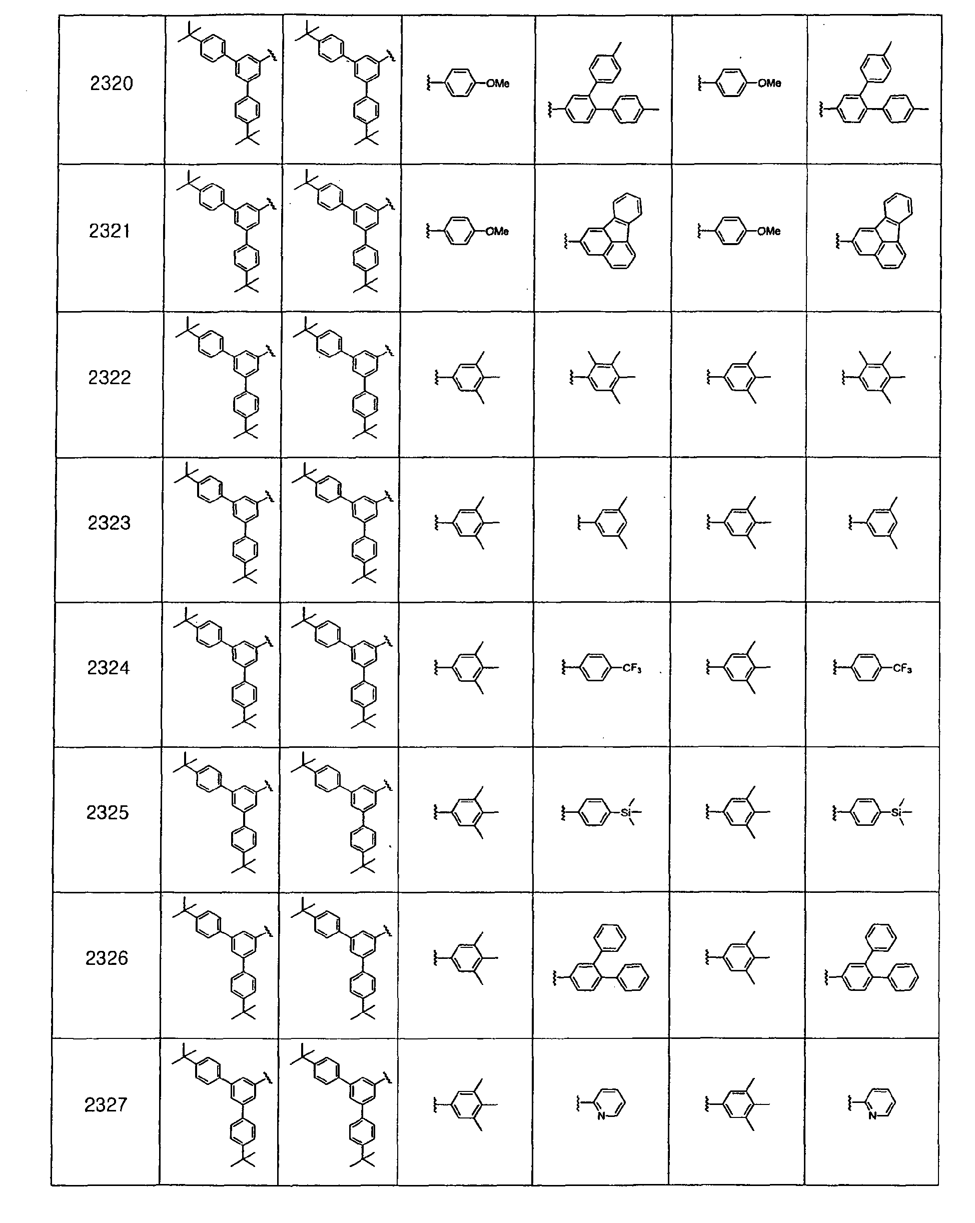

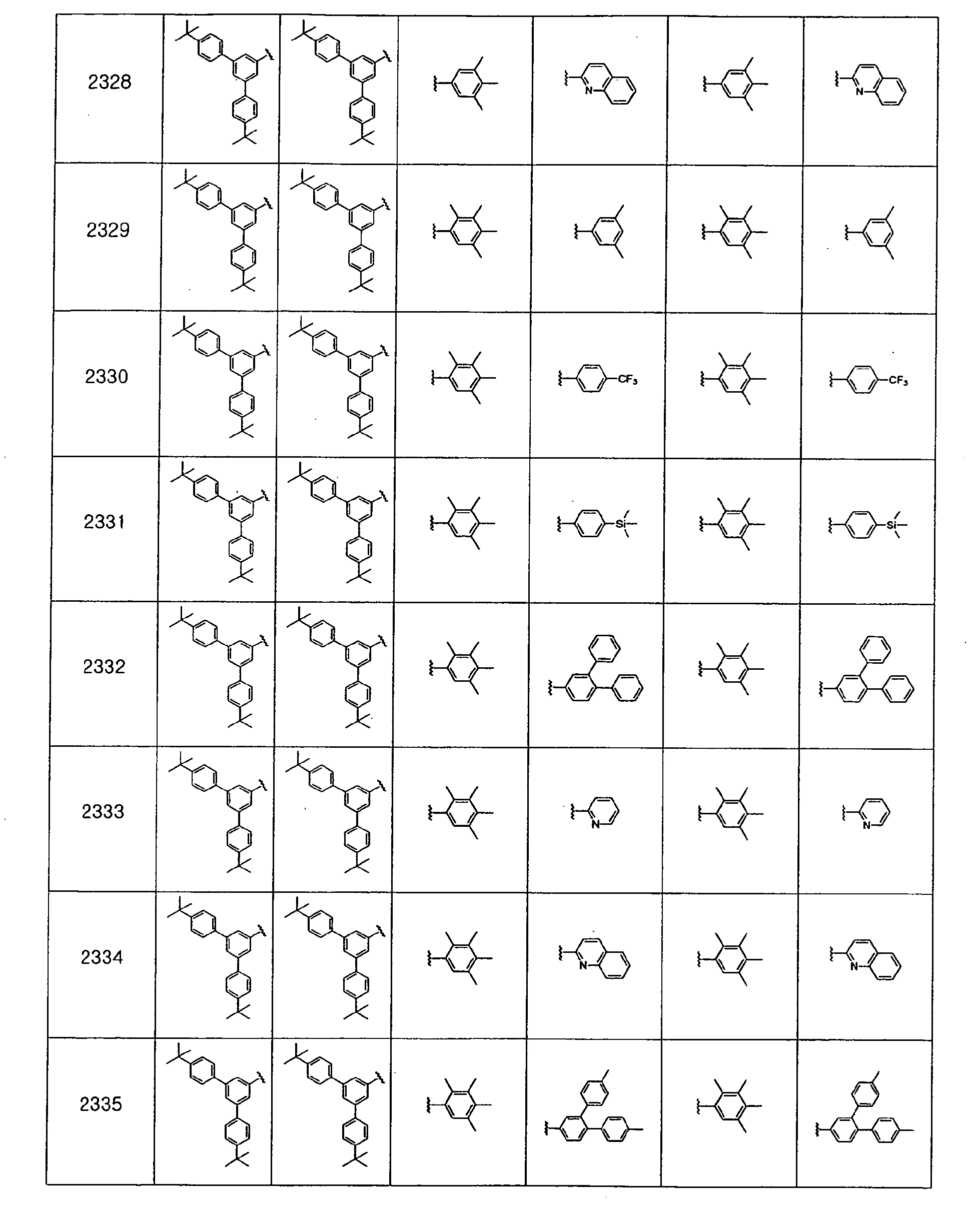

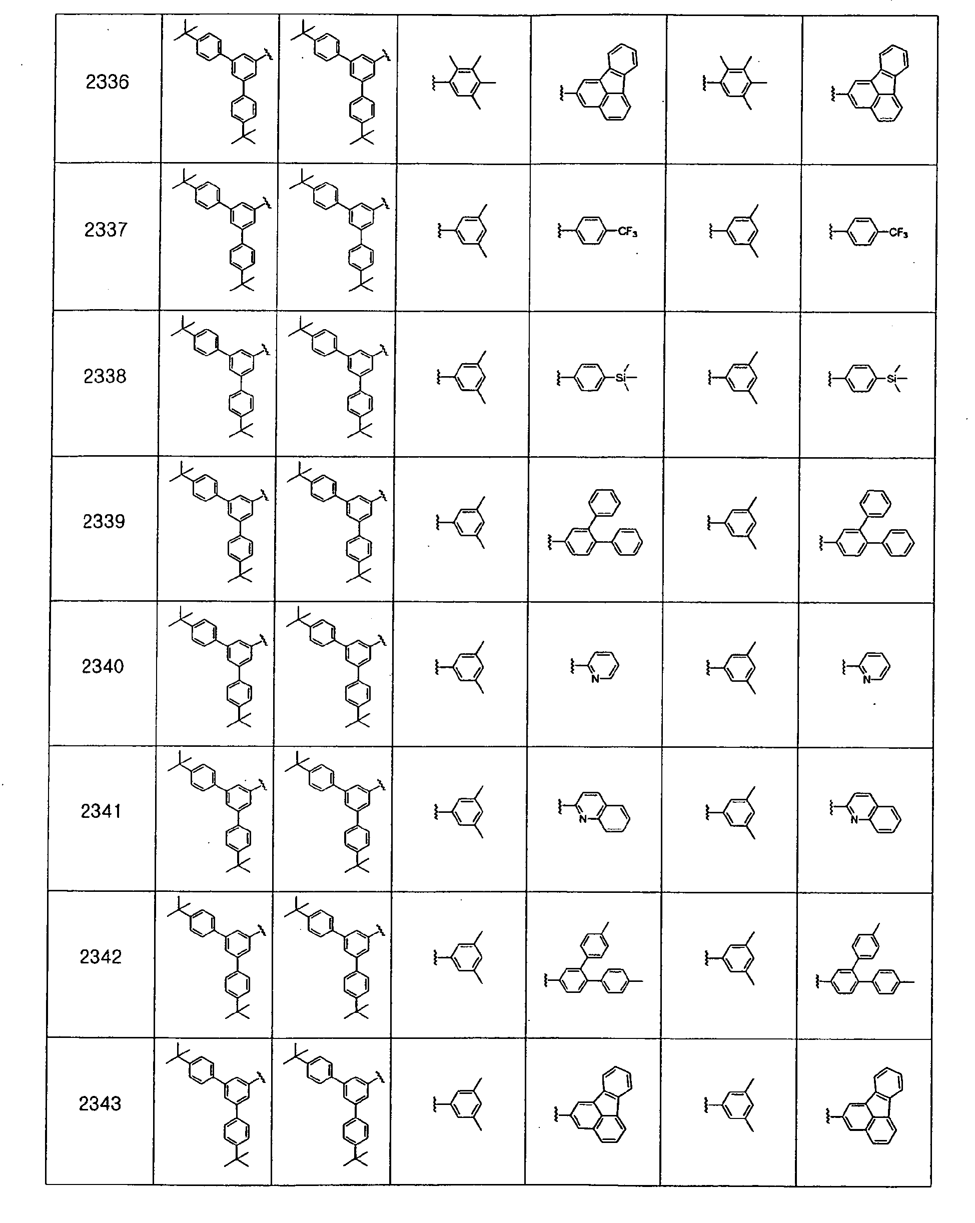

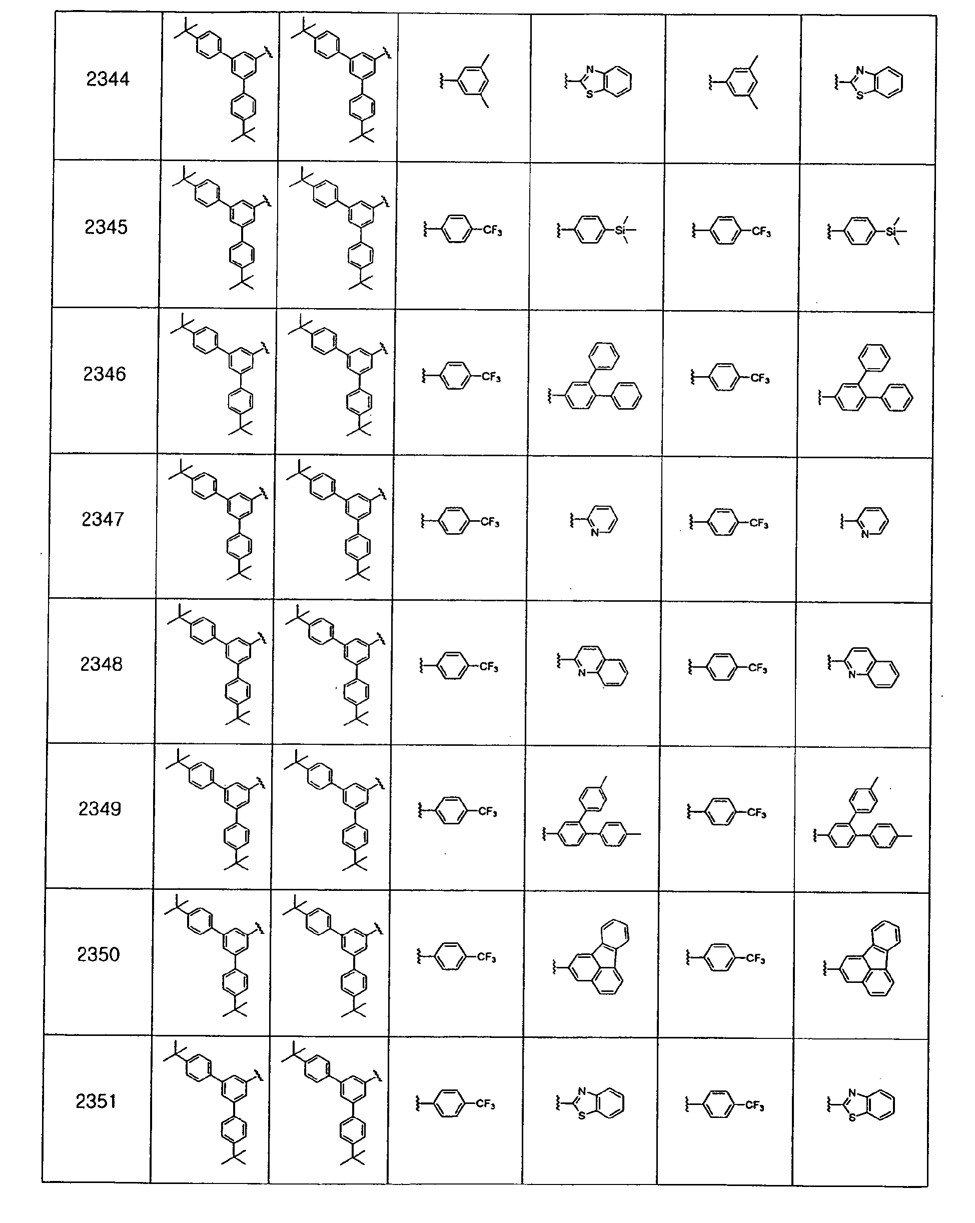

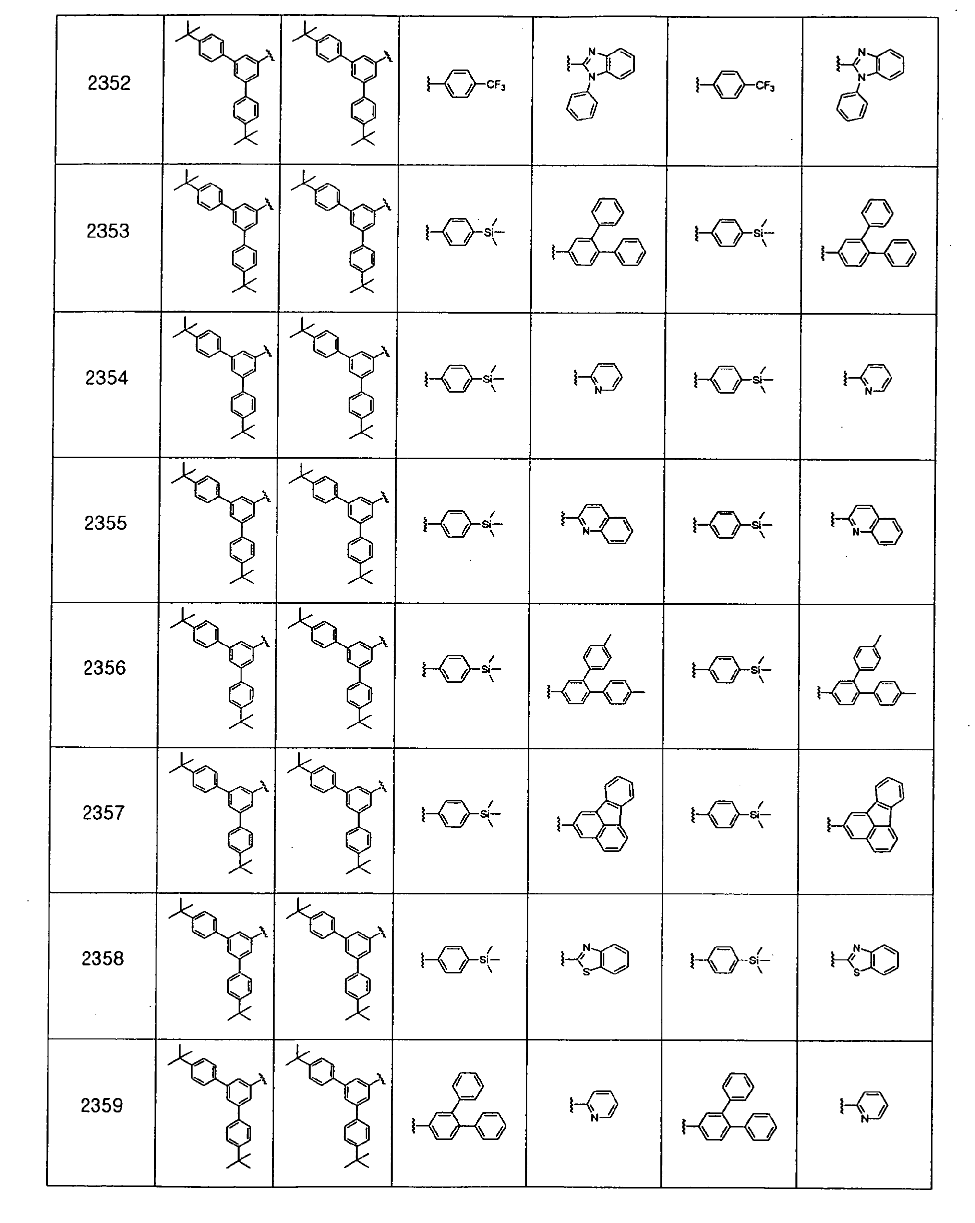

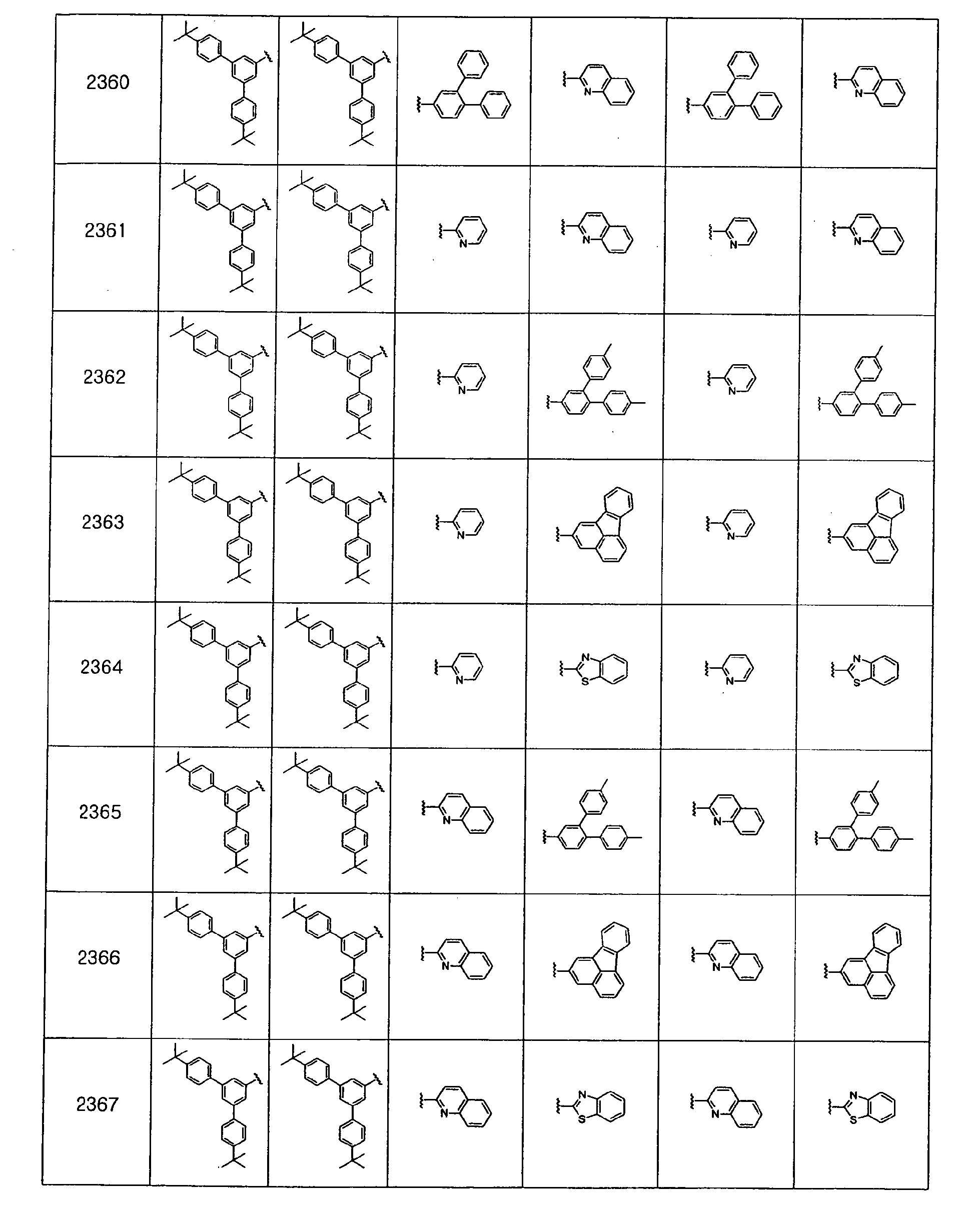

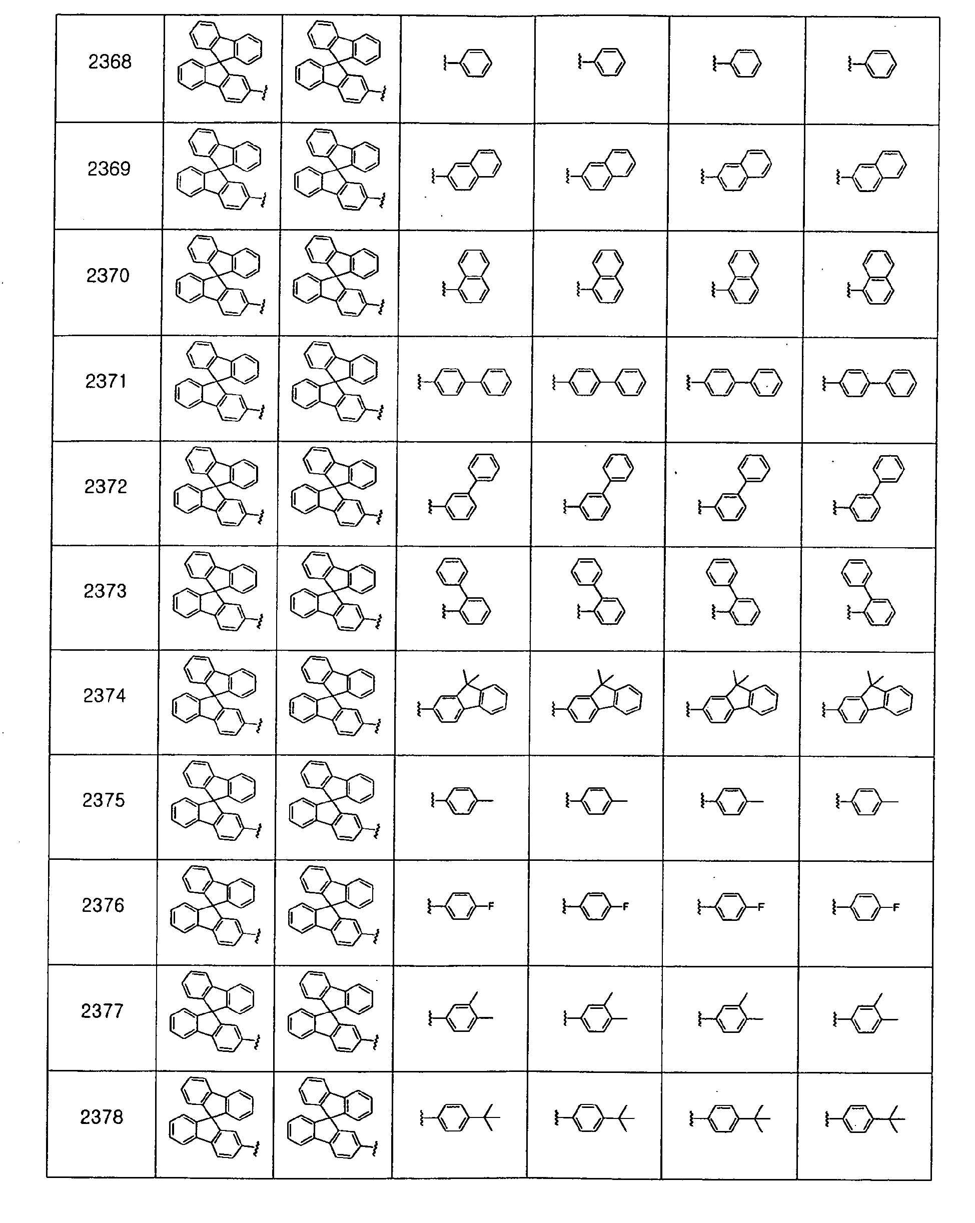

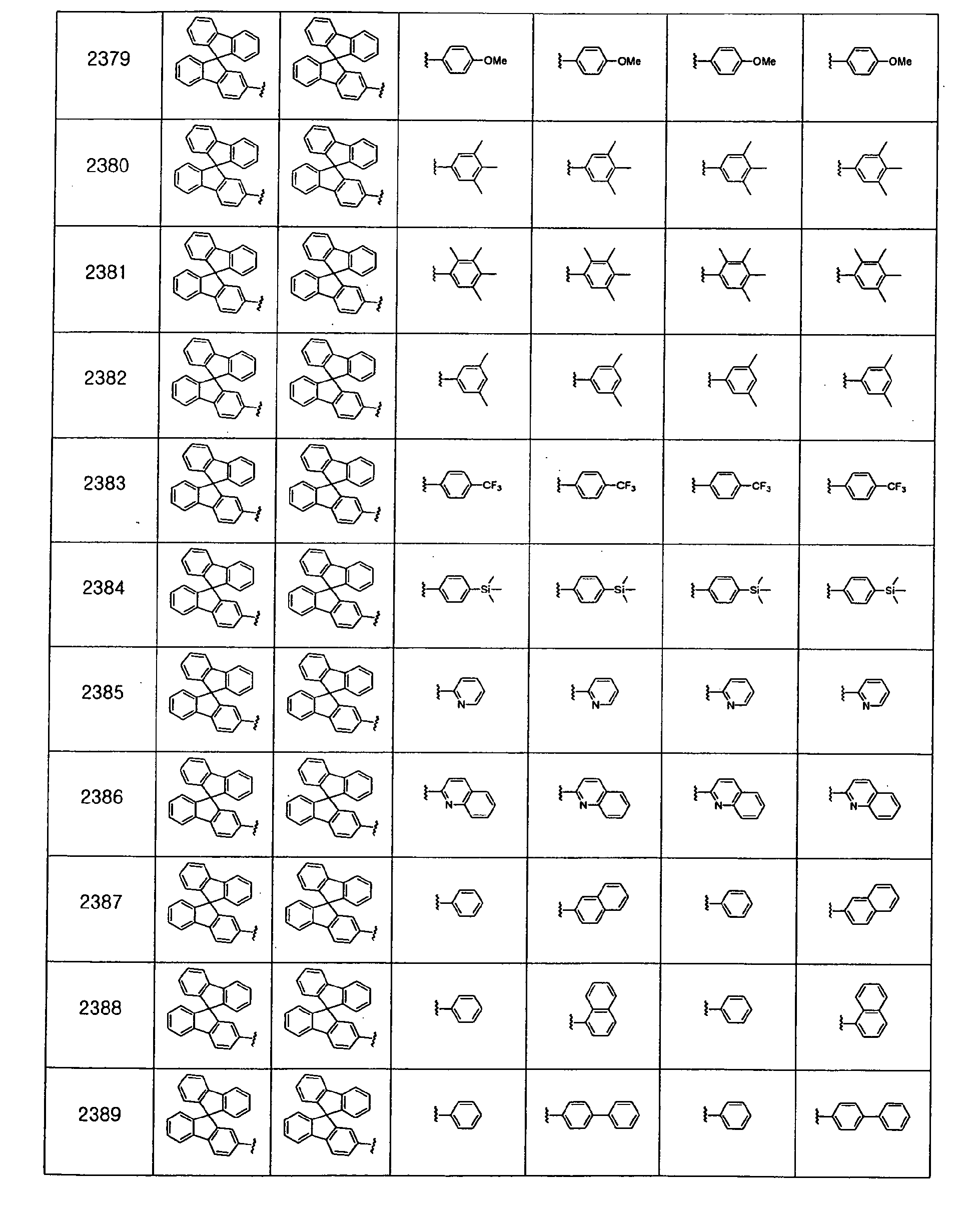

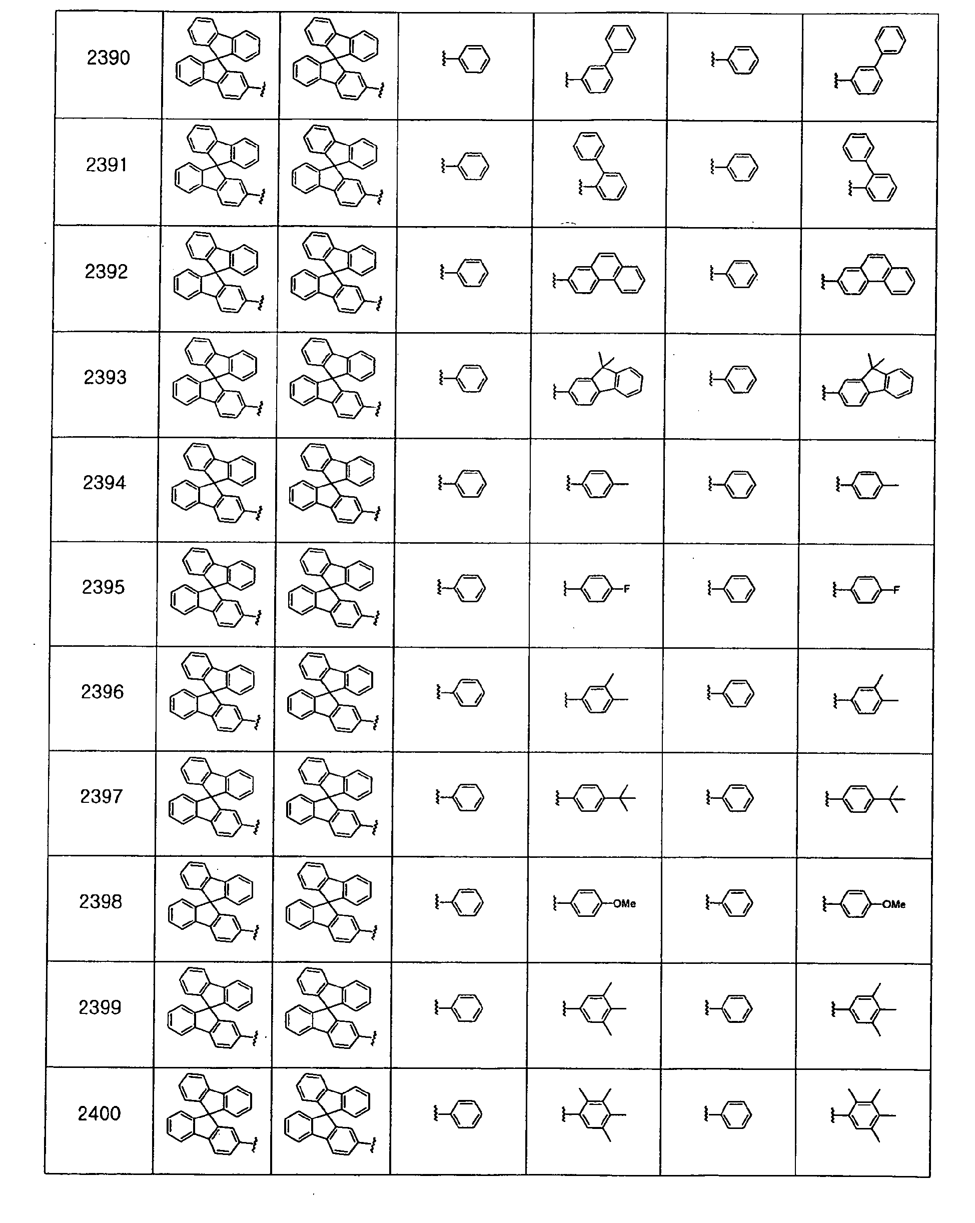

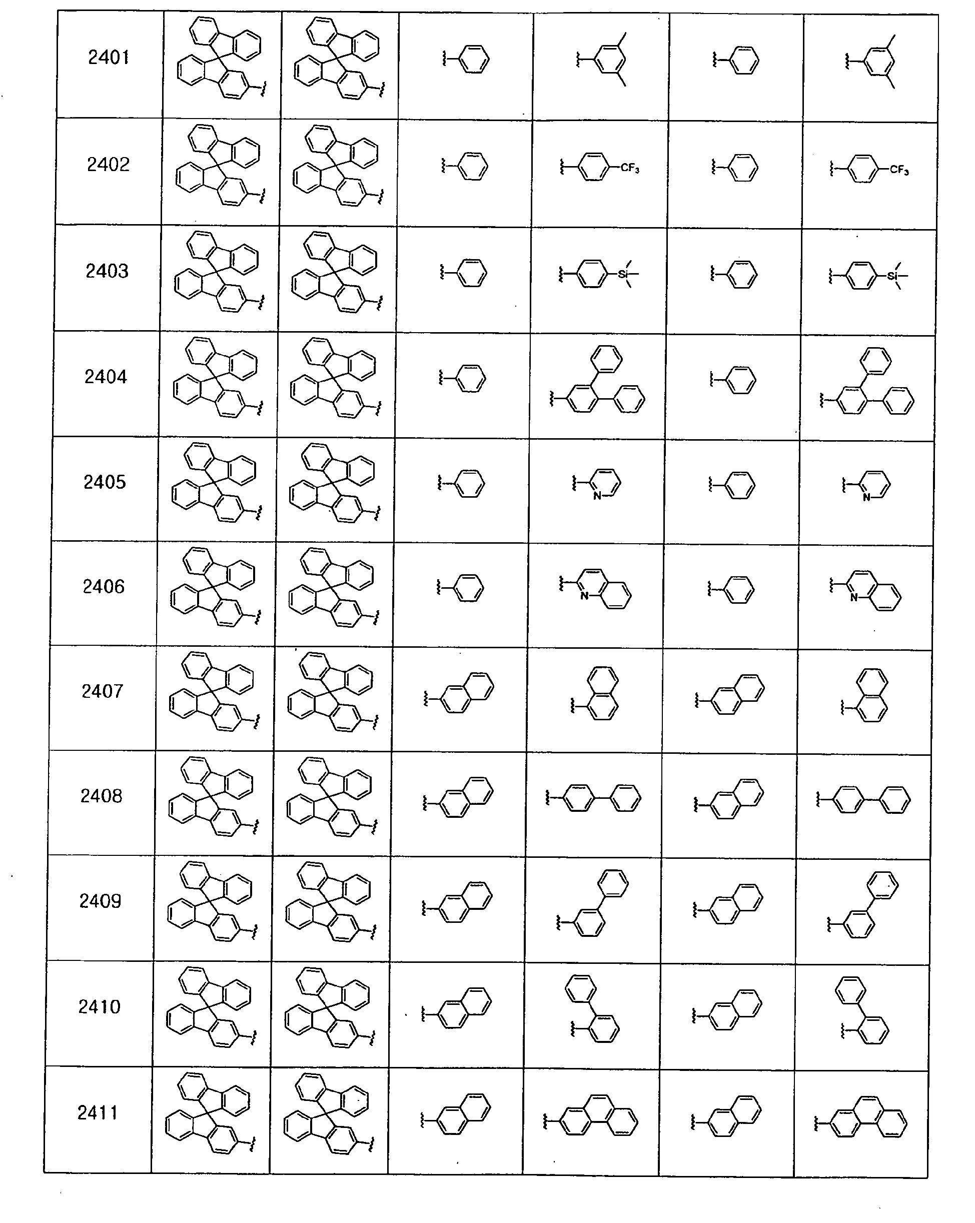

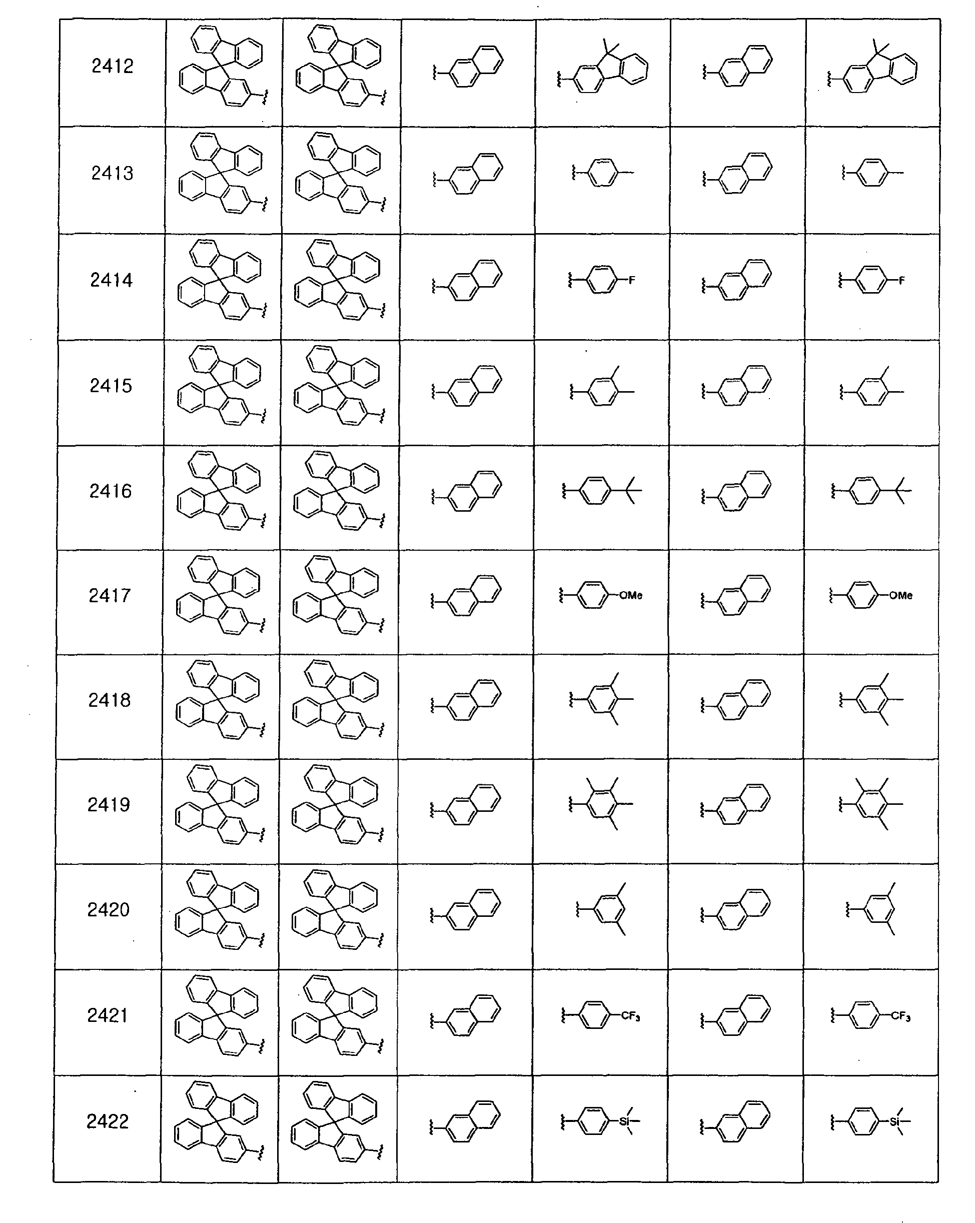

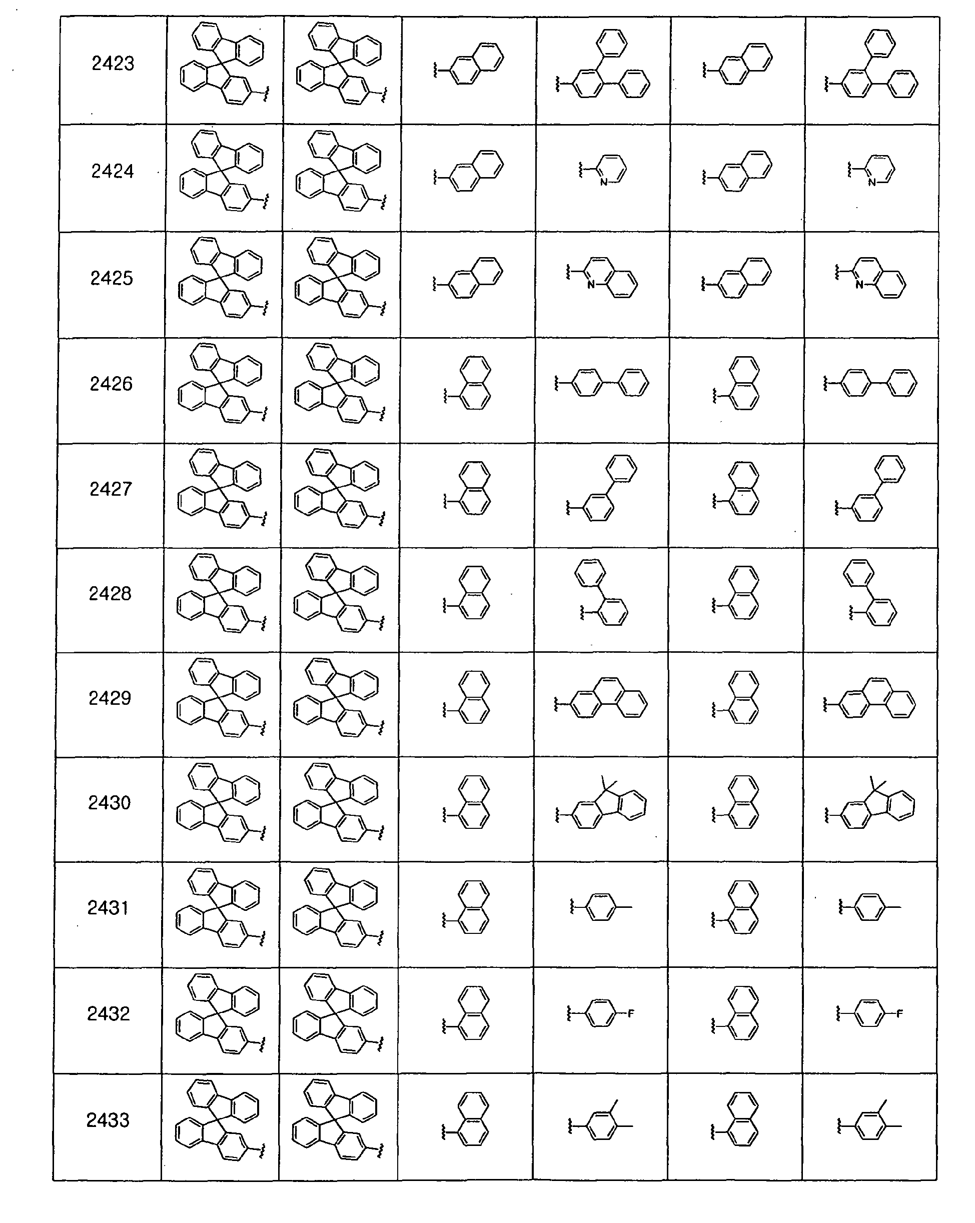

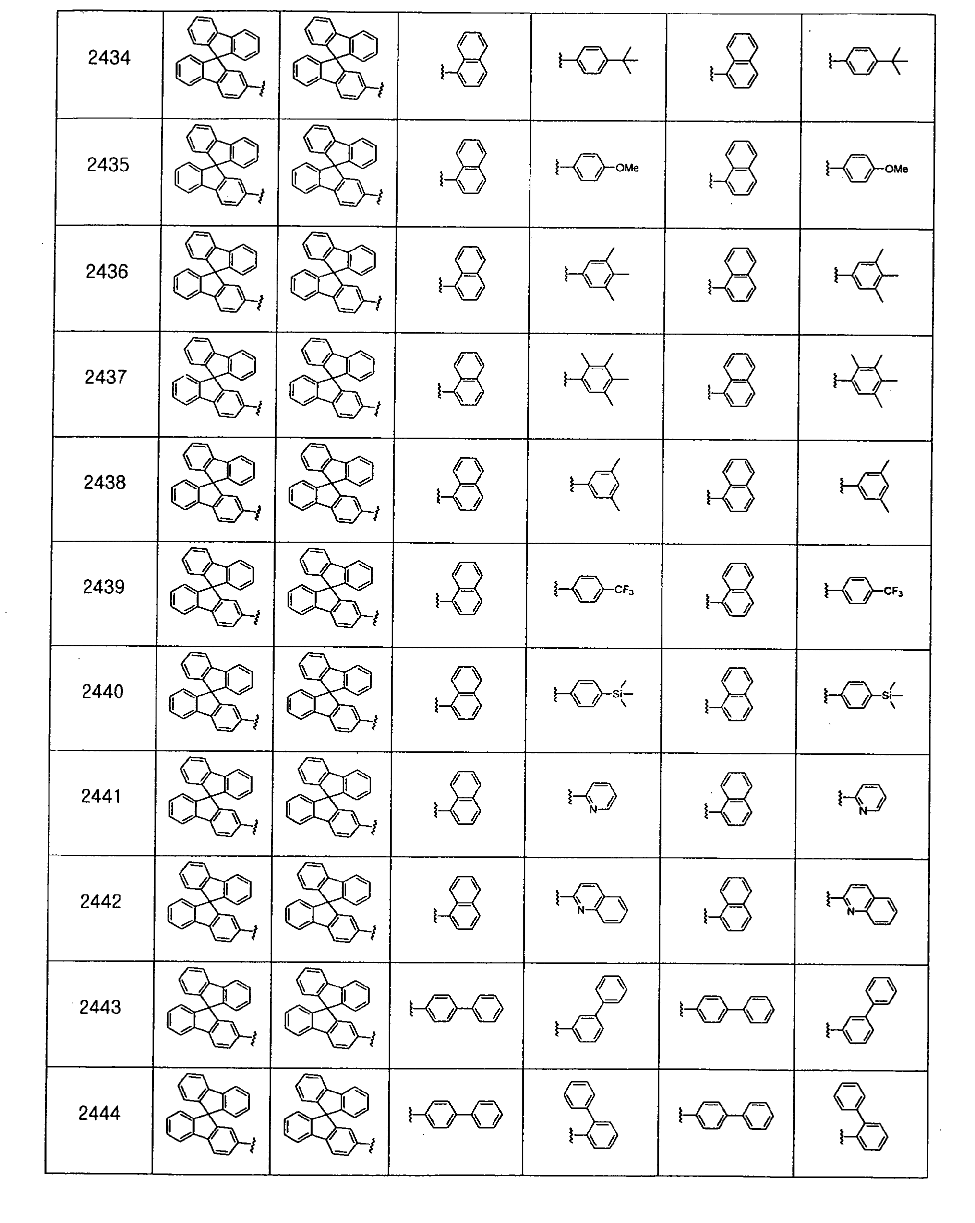

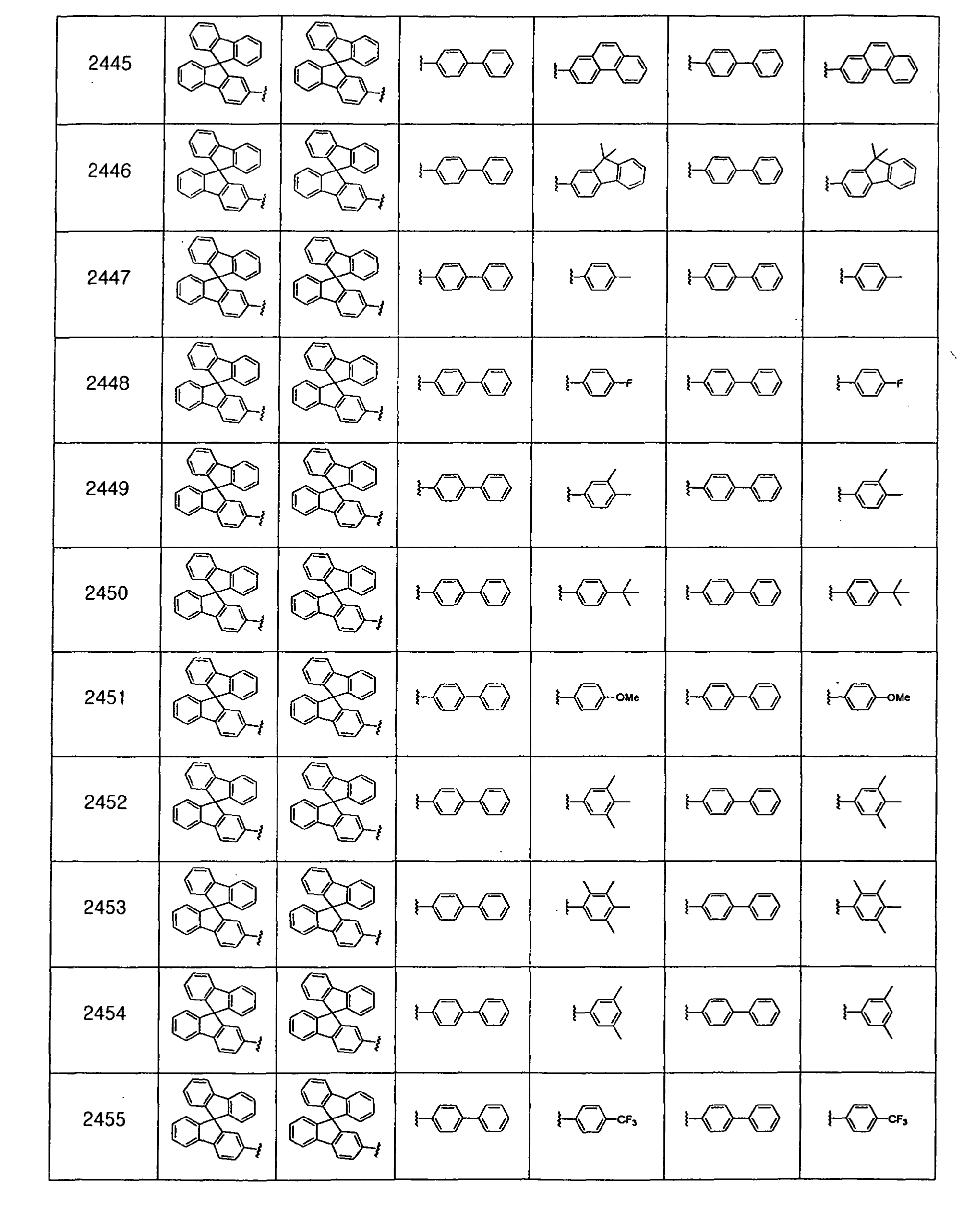

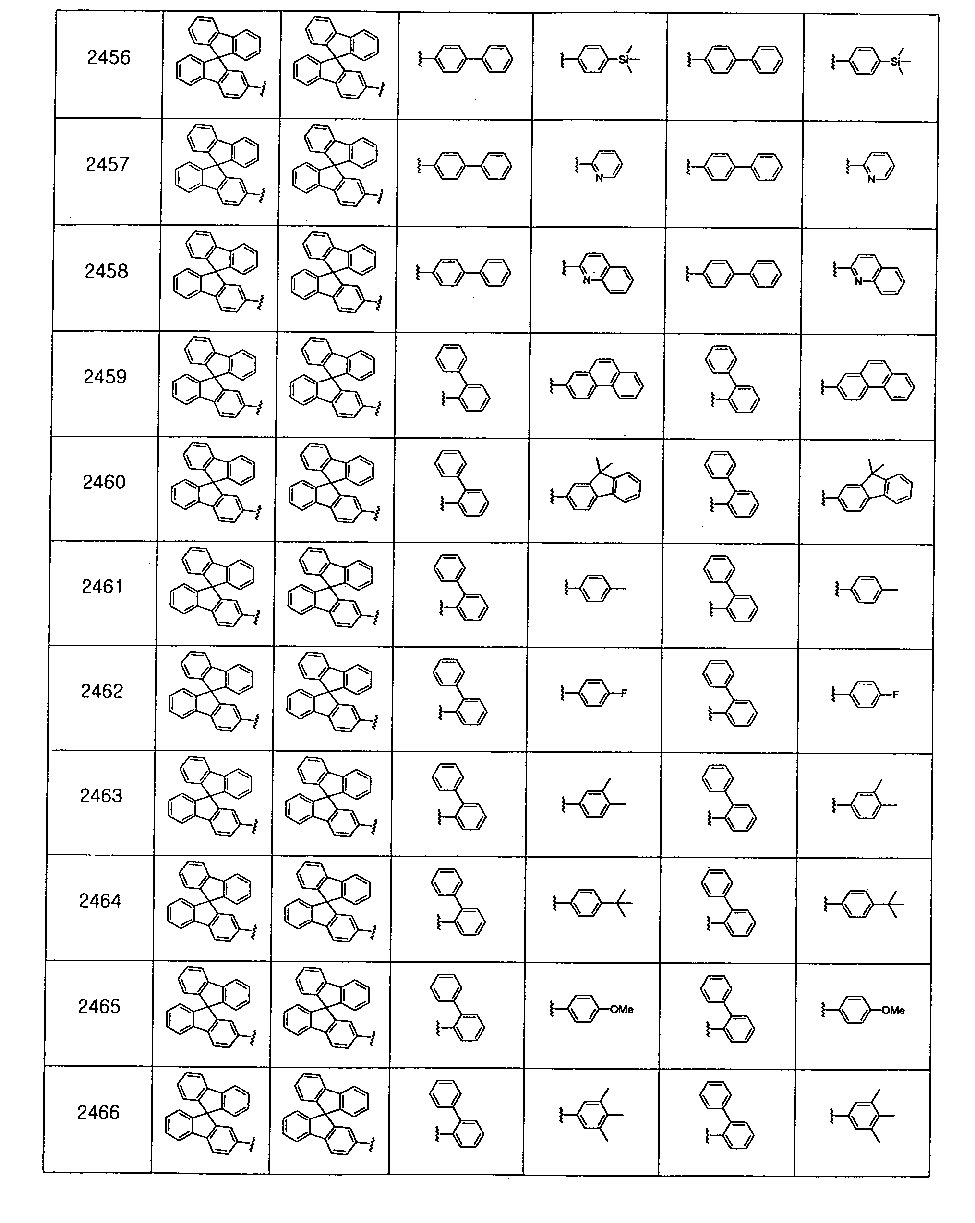

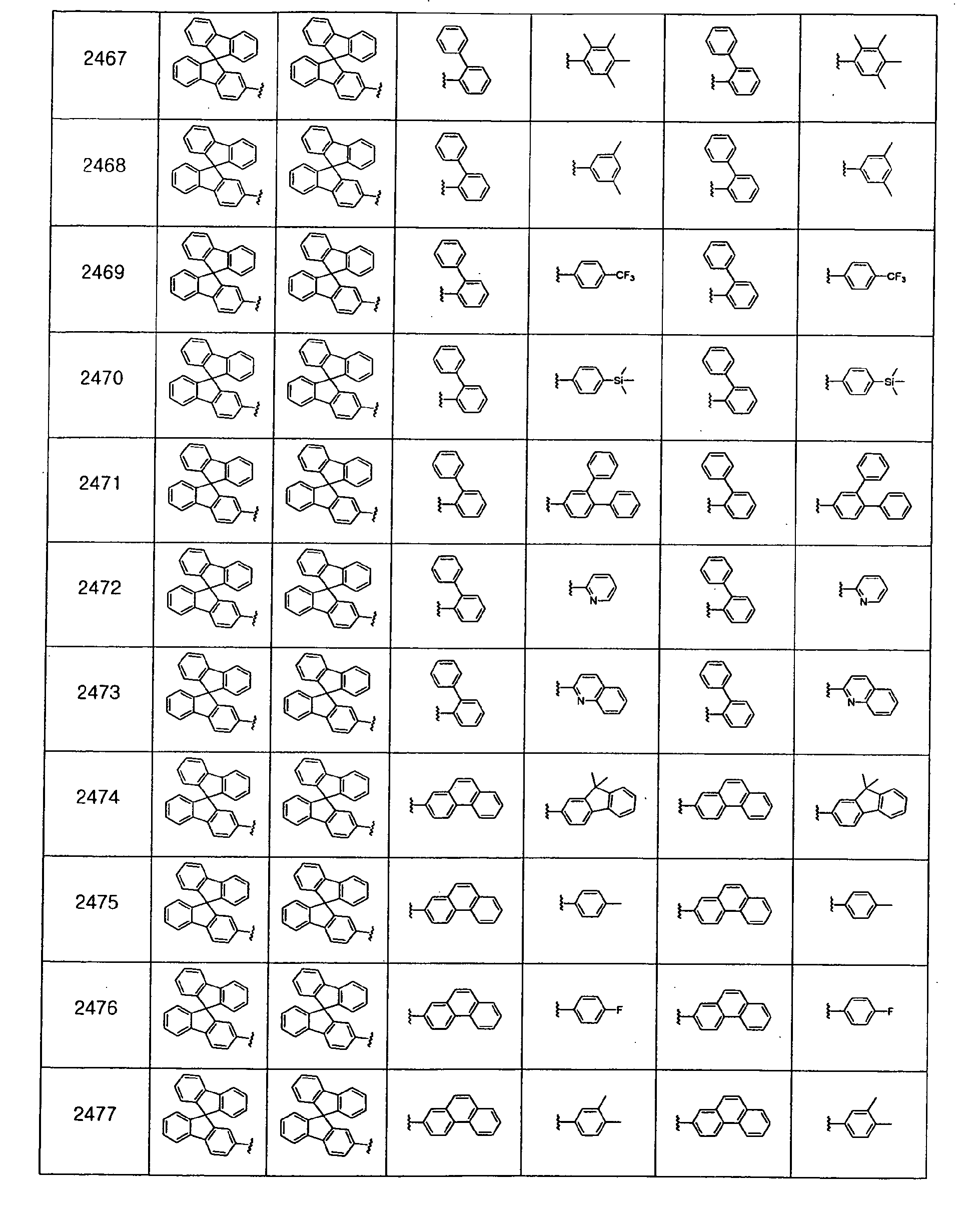

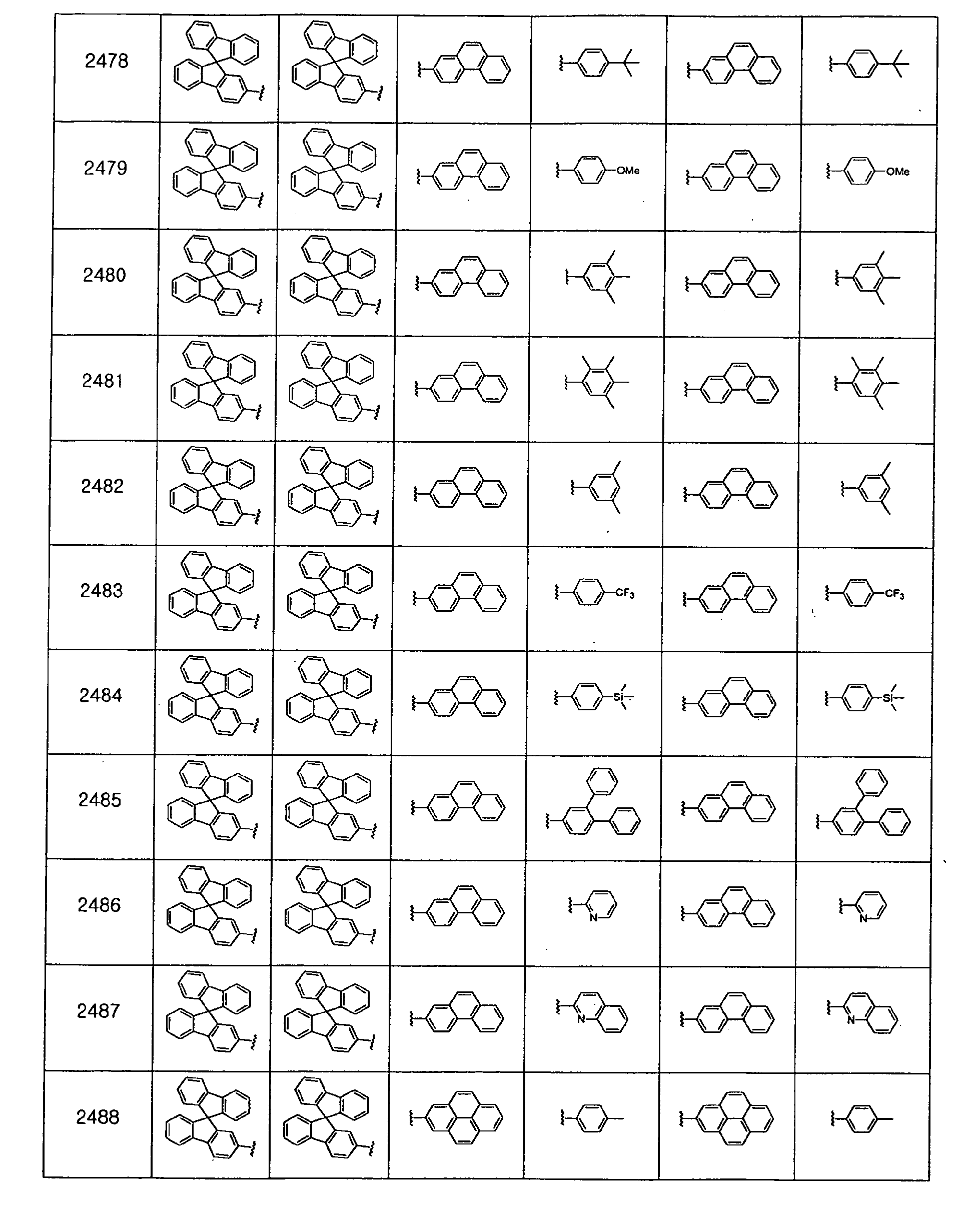

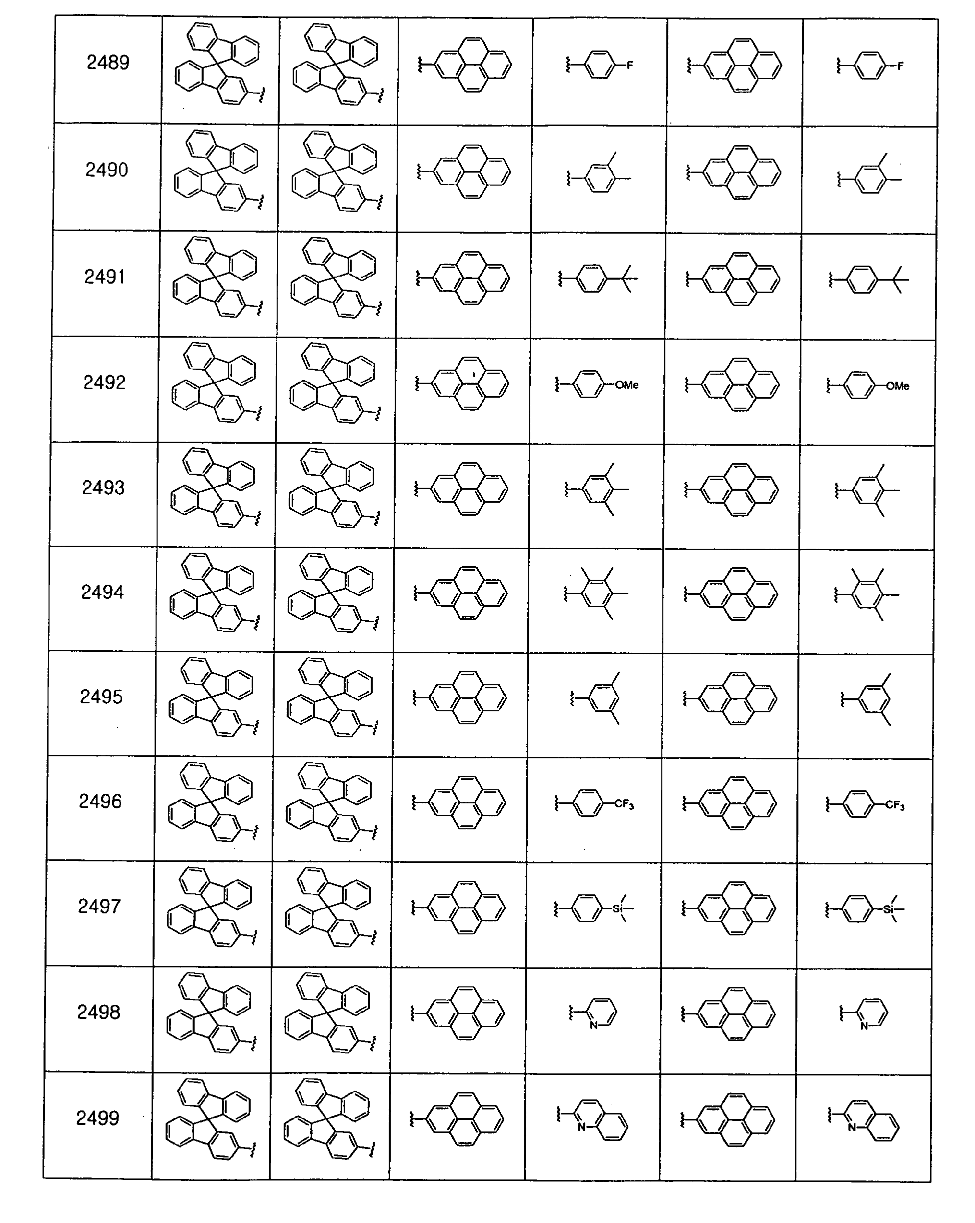

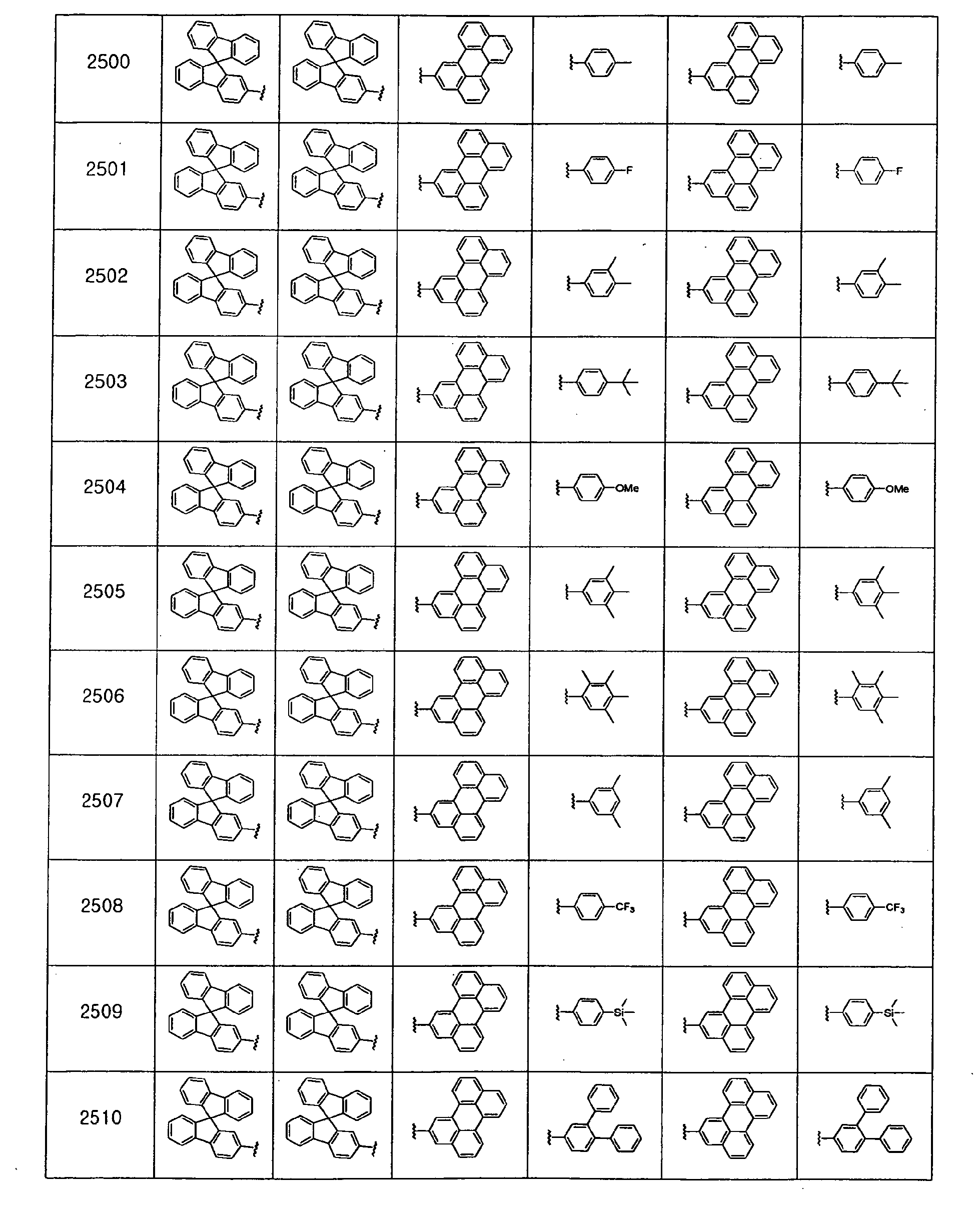

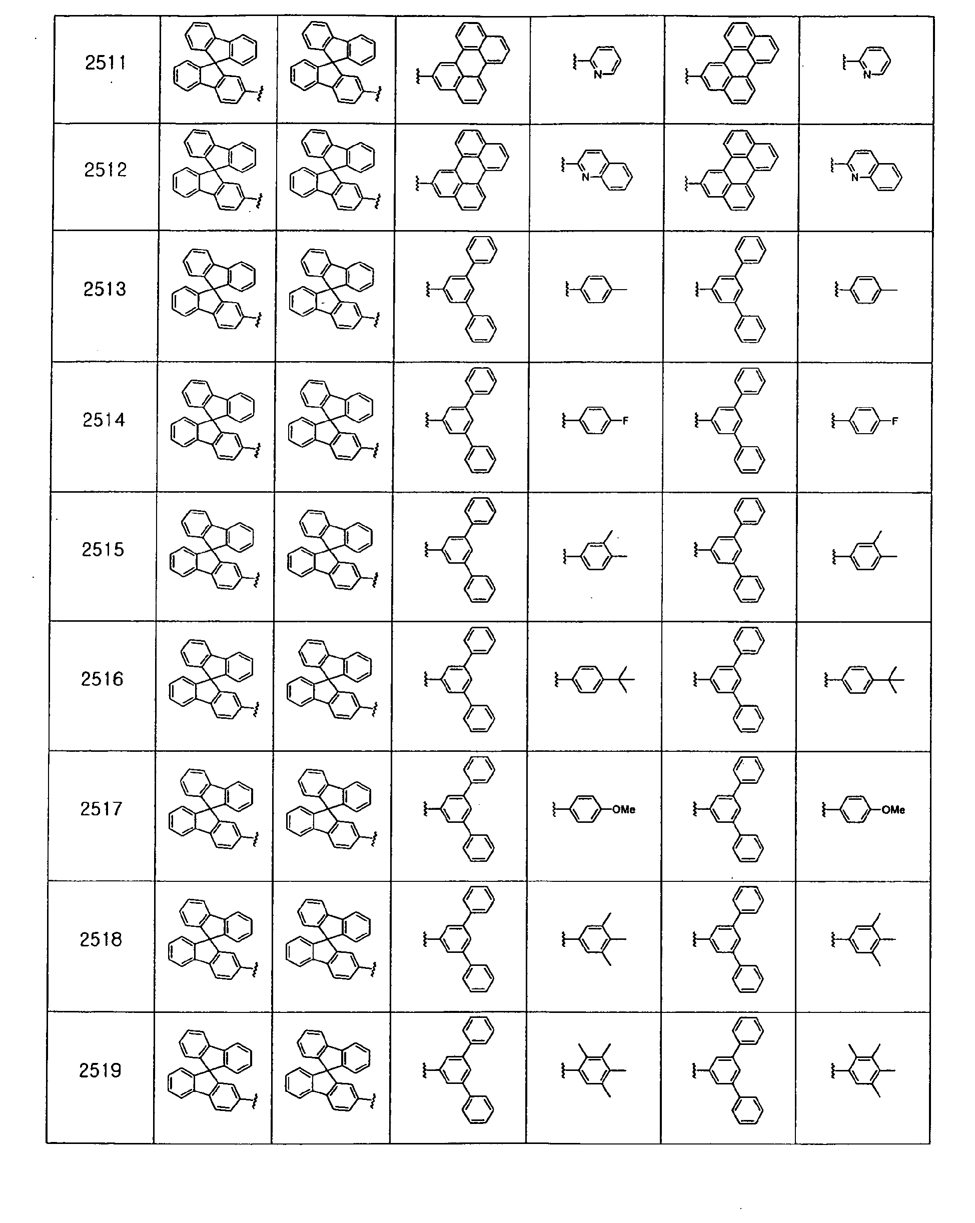

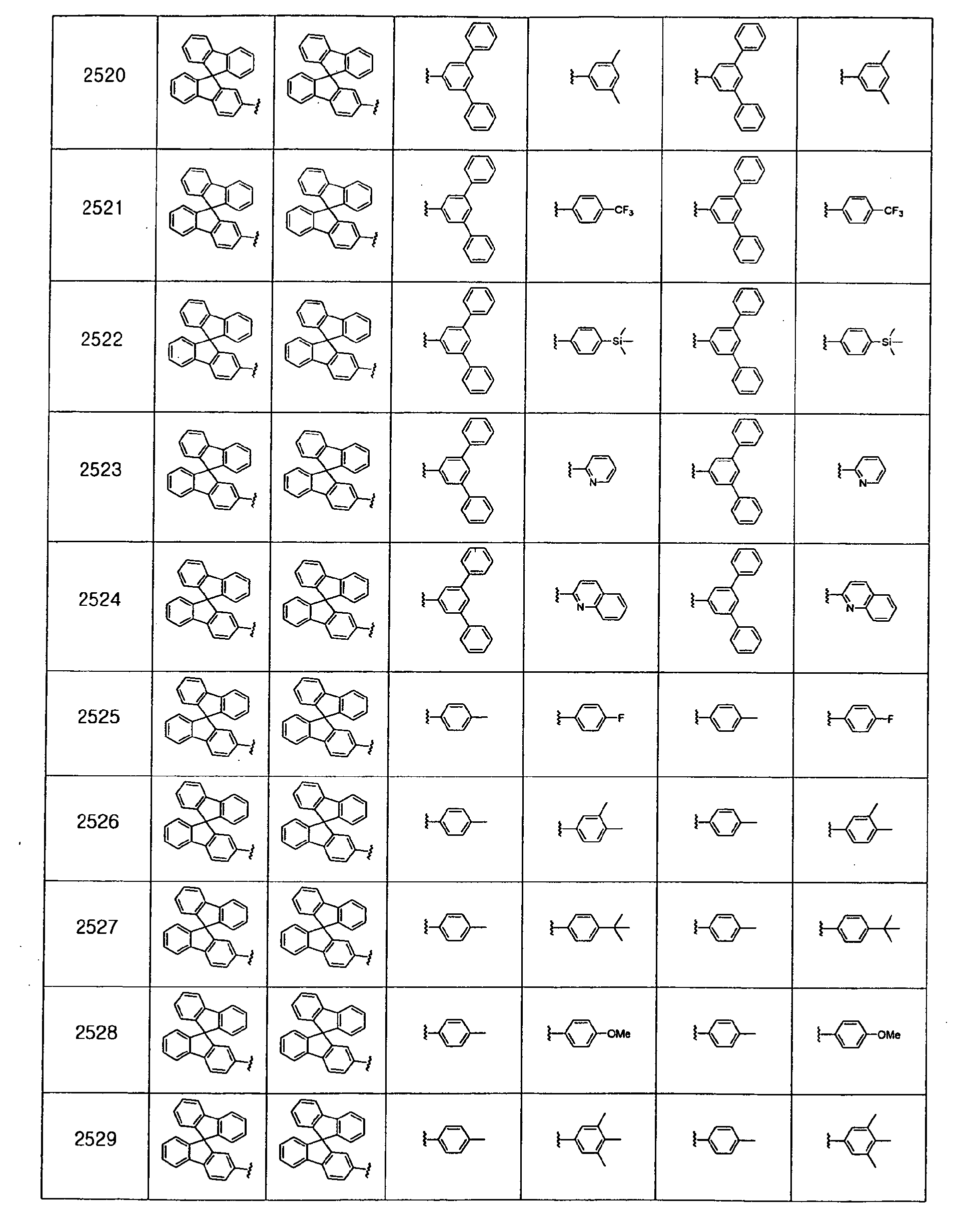

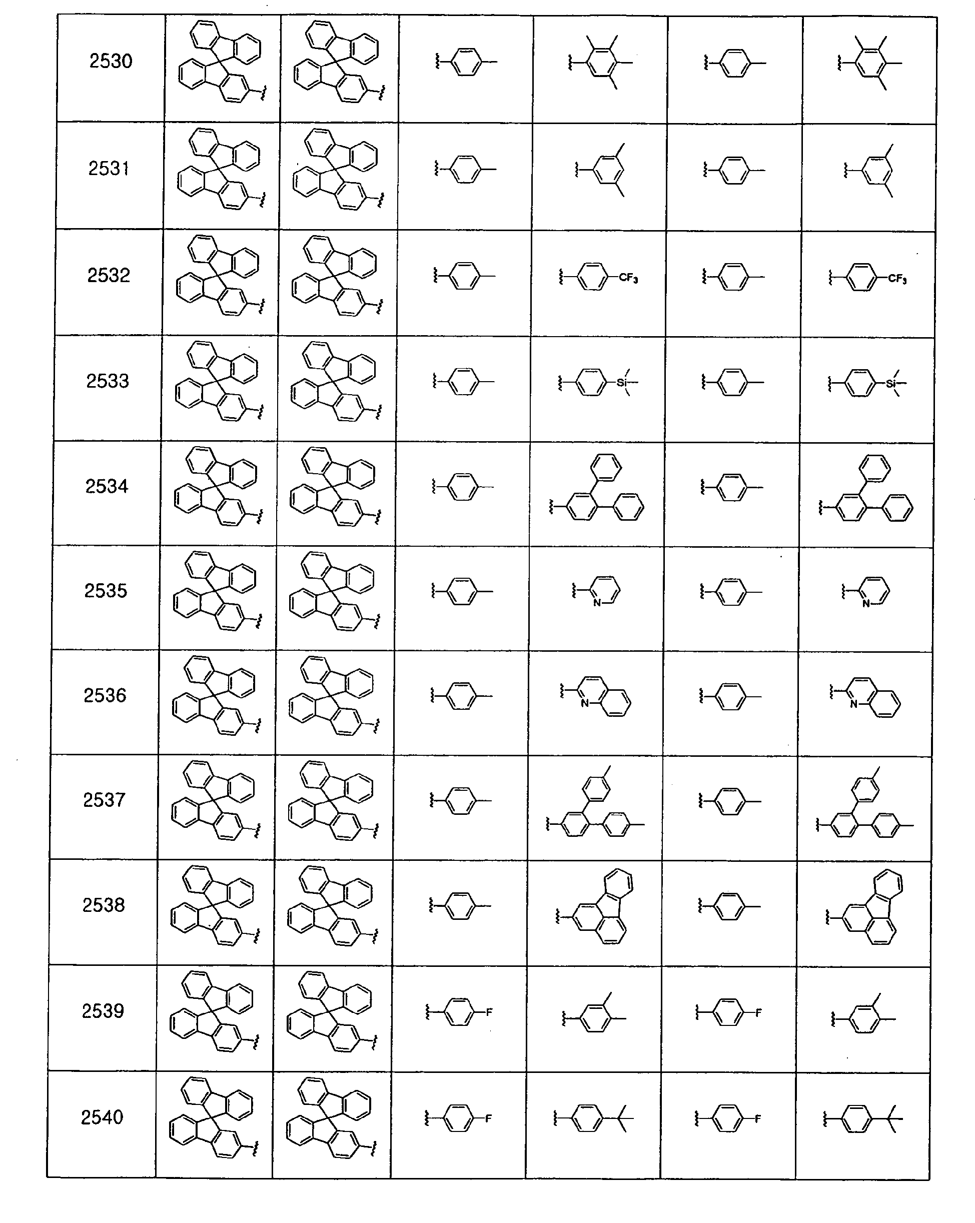

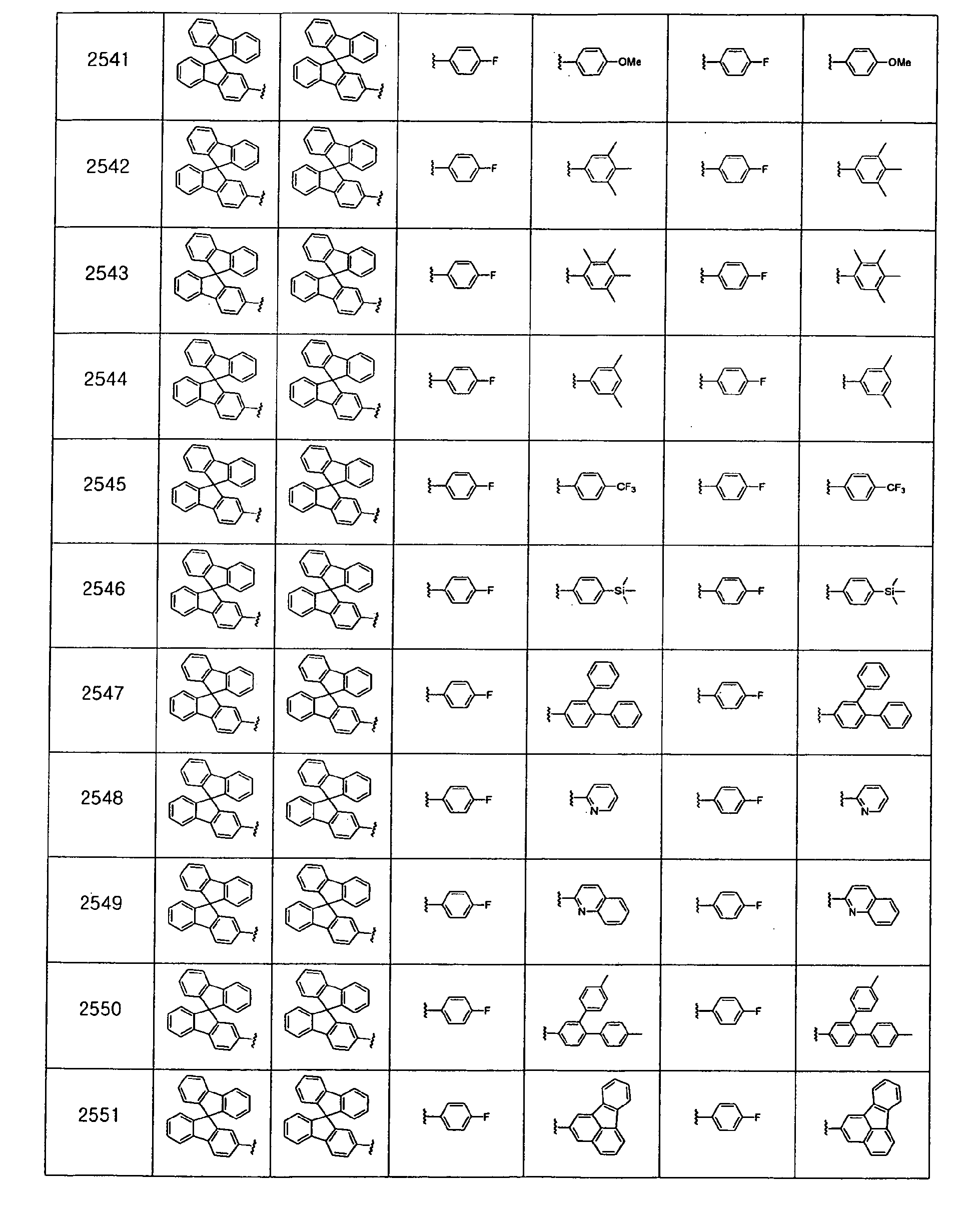

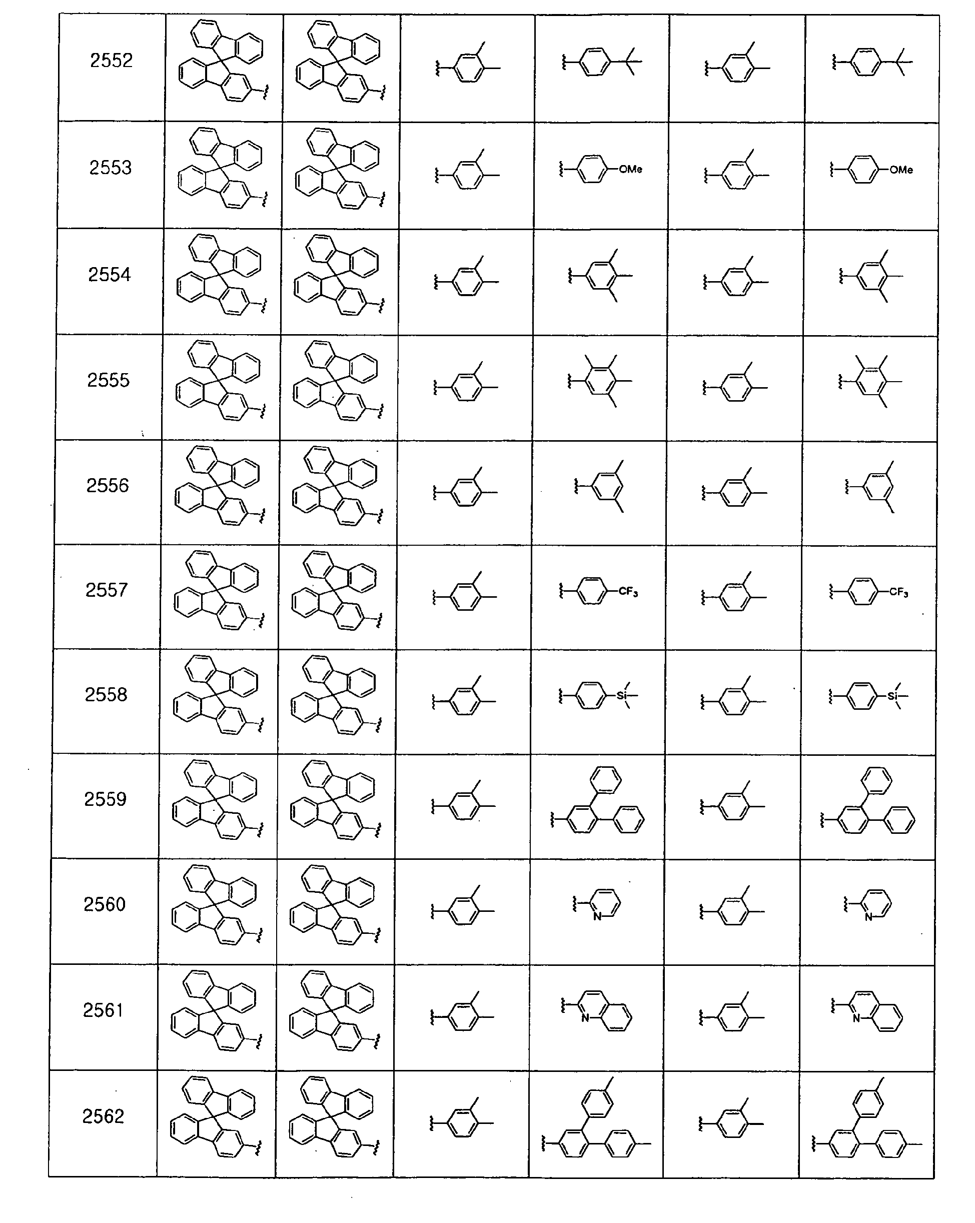

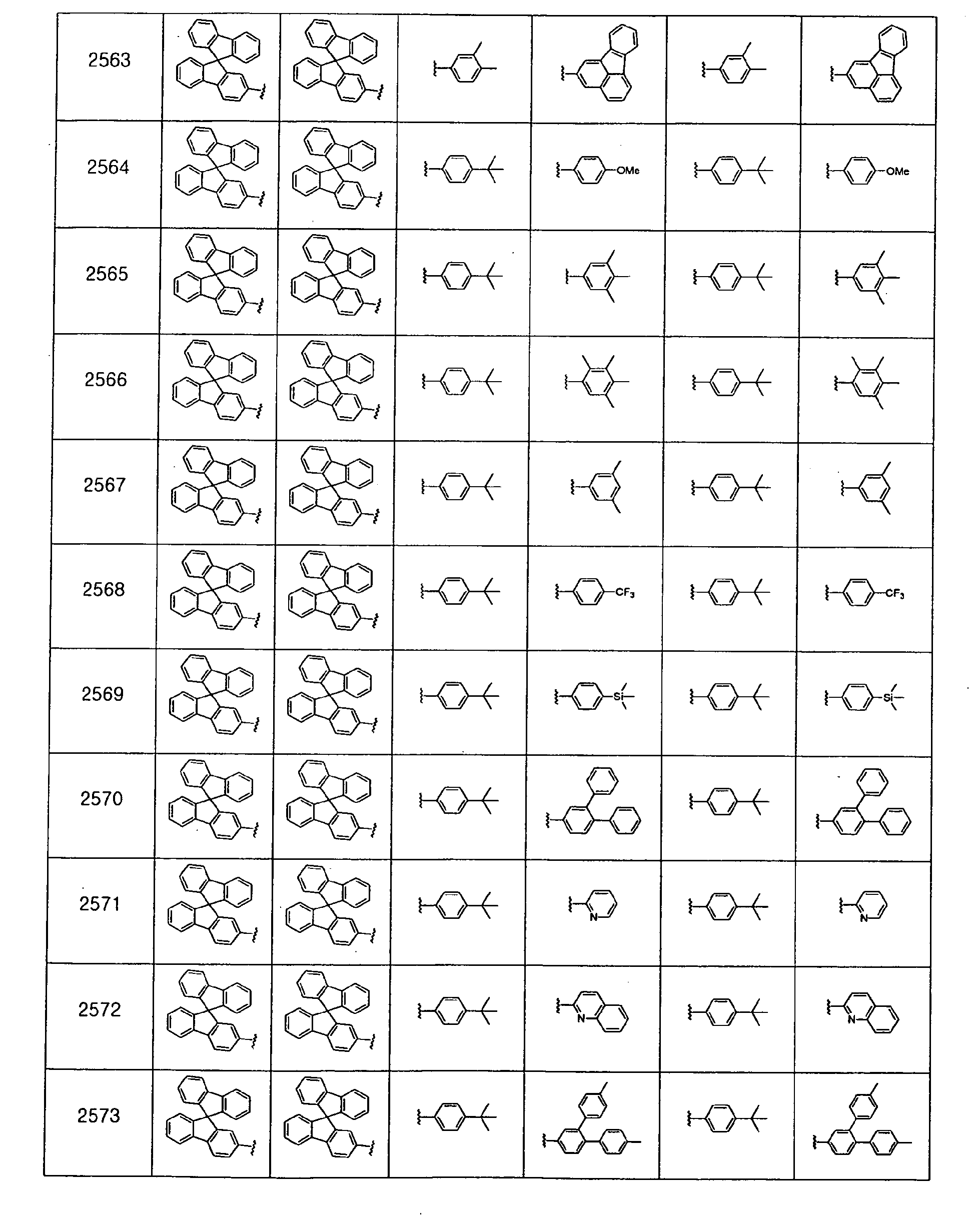

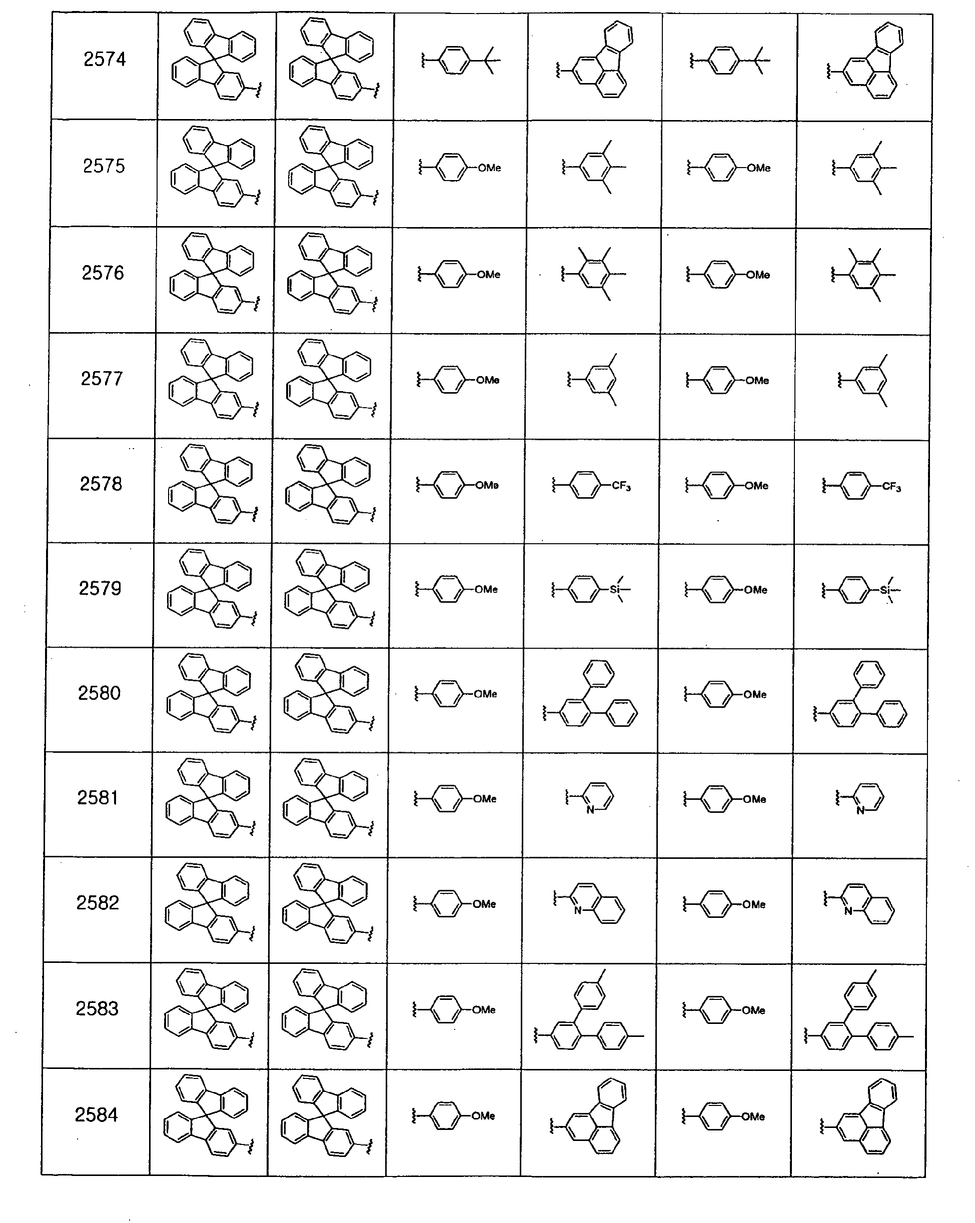

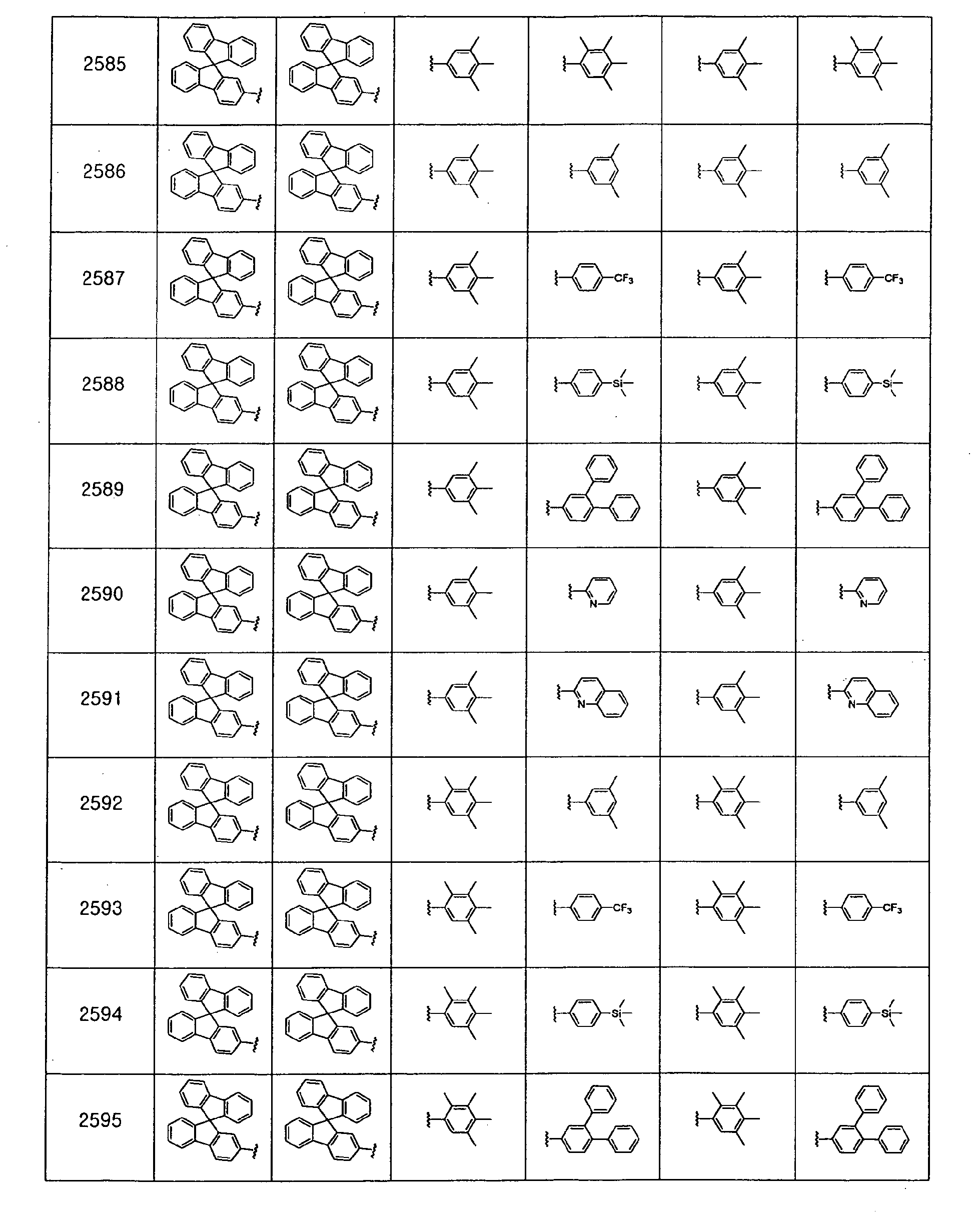

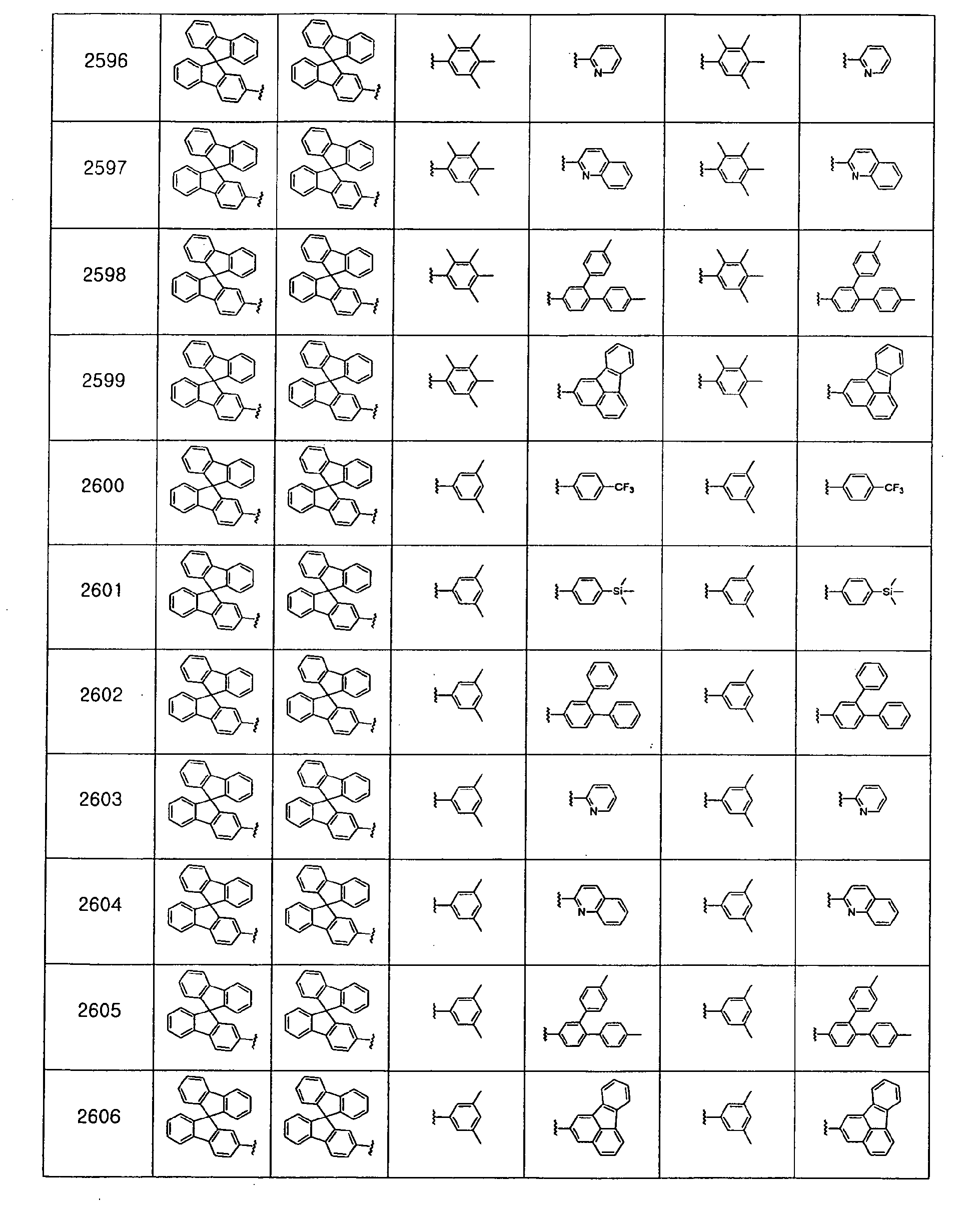

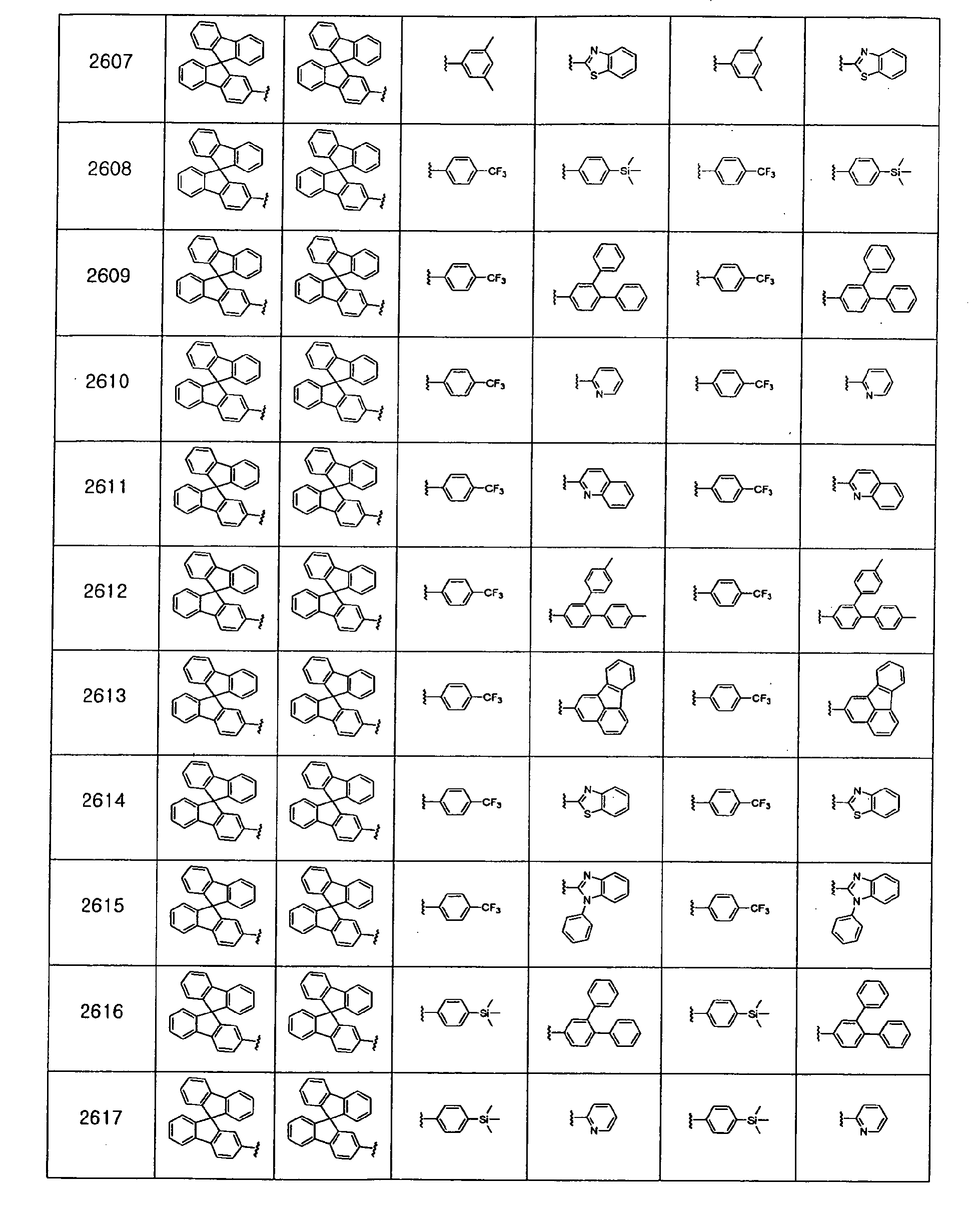

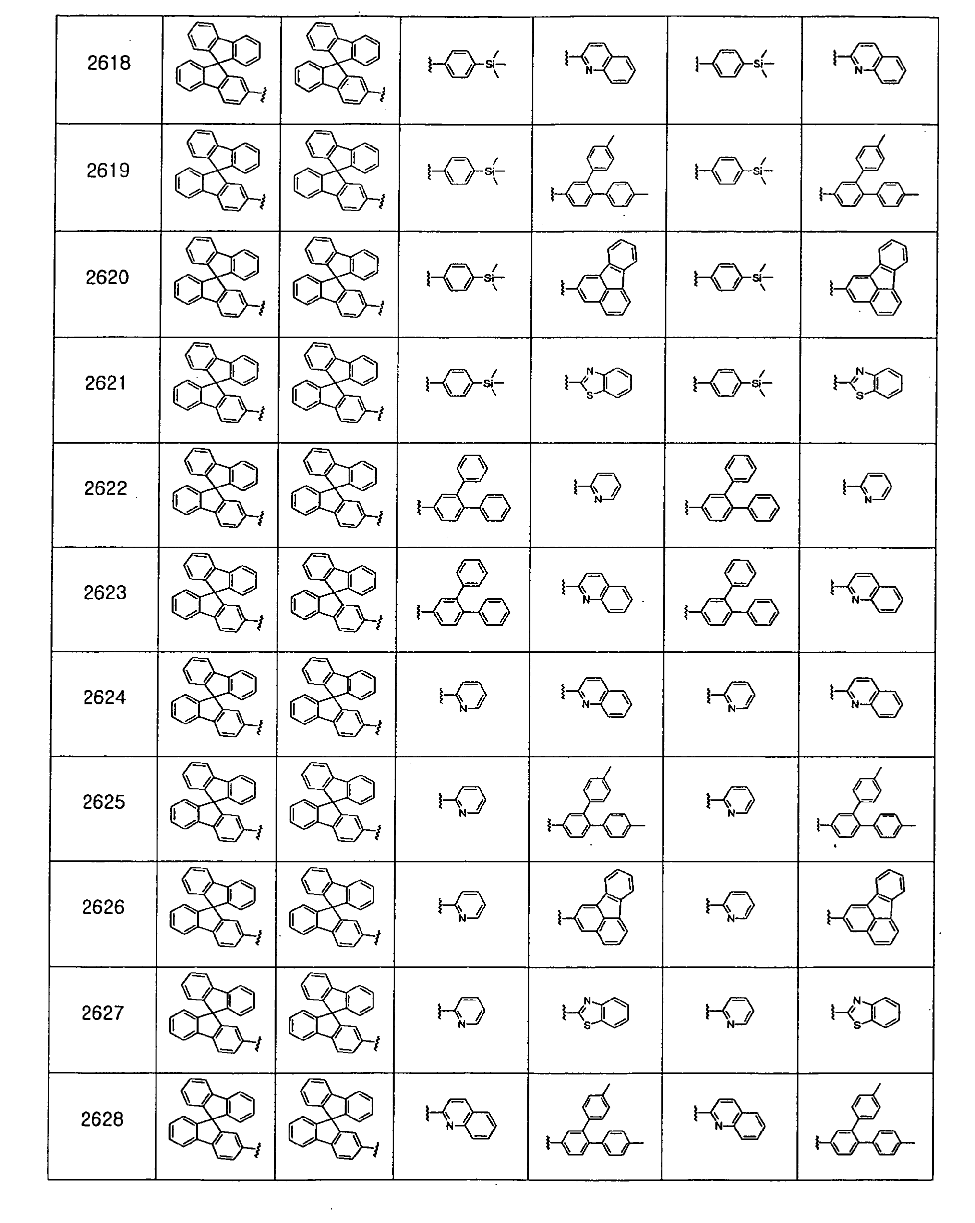

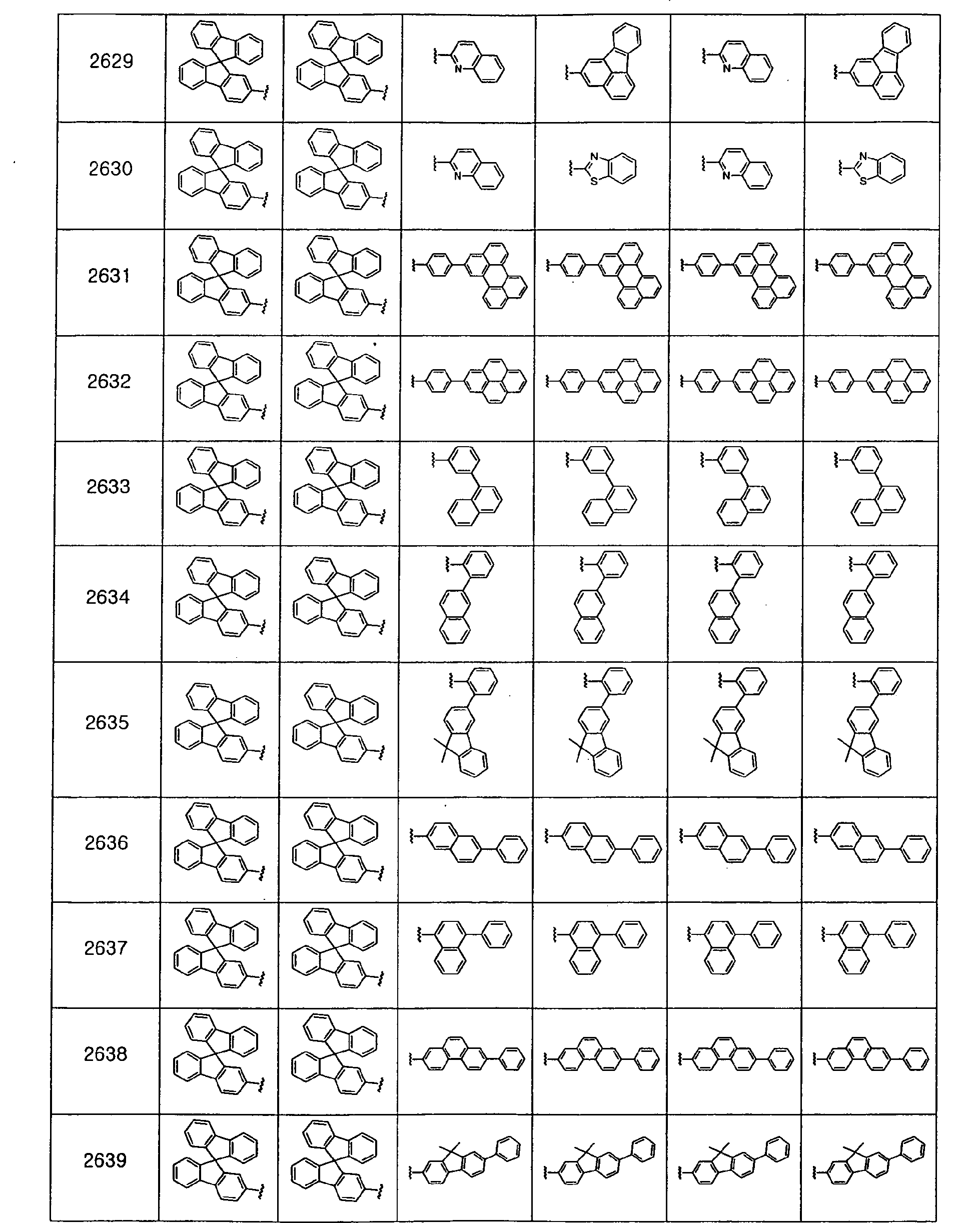

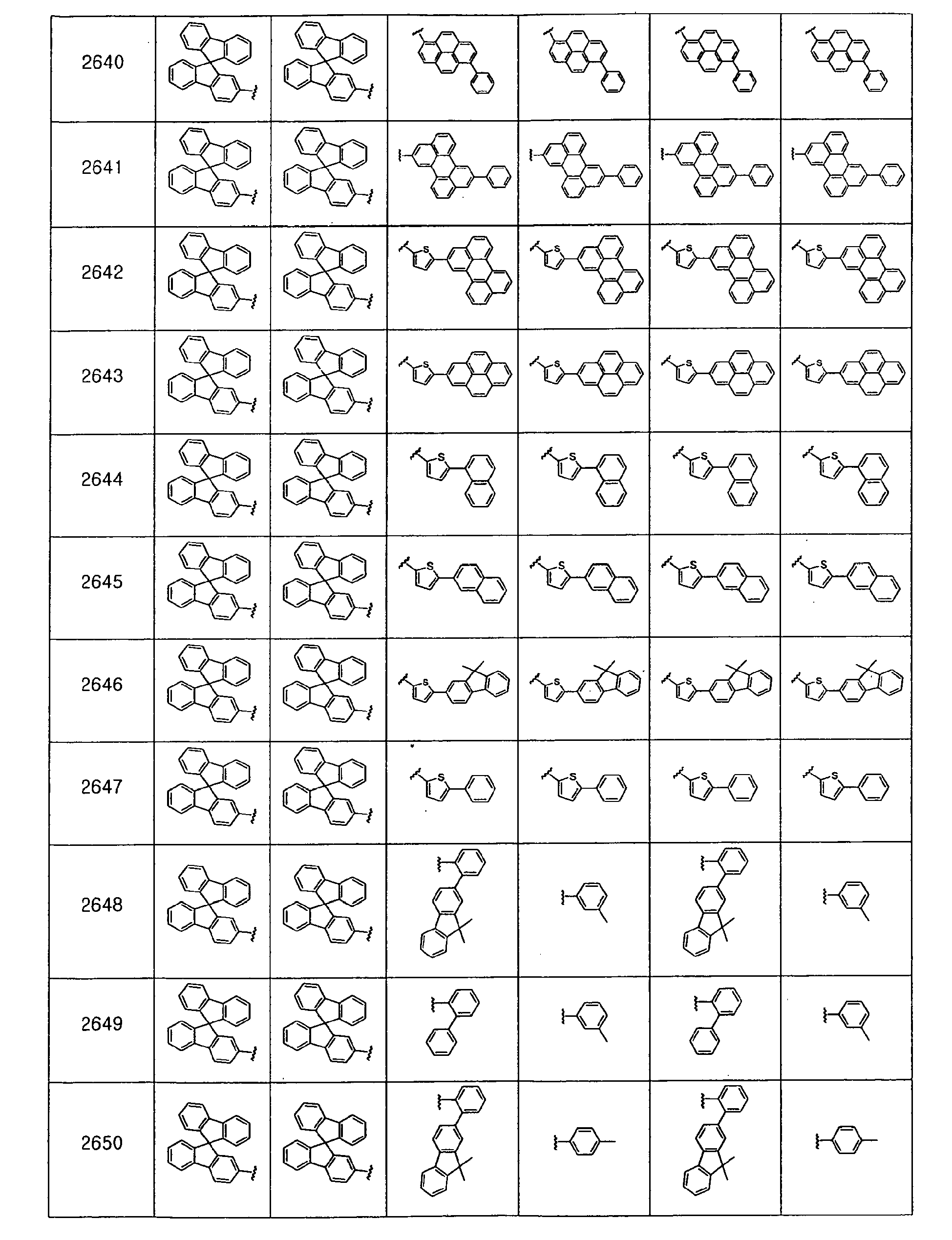

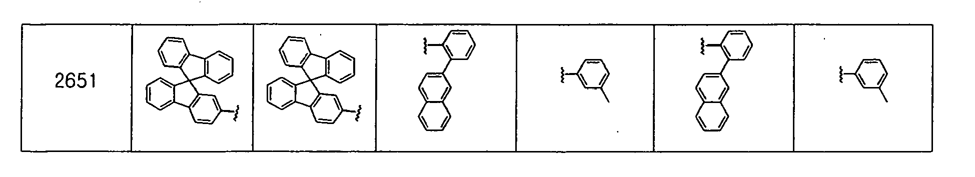

**[Table 3]**

| compound | ¹H NMR(CDCl₃, 200 MHz) | MS/FAB | |
|---|---|---|---|
| | | found | calculated |
| 3 | δ = 6.83(2H, m), 6.98∼7.03(6H, m), 7.11(8H, m), 7.26∼7.38(20H, m), 7.53∼7.63(16H, m), 7.75∼7.77(4H, m), 7.87∼7.93(4H, m), 8.02∼8.07(8H, m) | 1345.7 | 1344.5 |
| 56 | δ = 6.65(2H, m), 6.83(2H, m), 6.95(2H, m), 7.03(2H, m), 7.11(8H, m), 7.26∼7.41(22H, m), 7.49∼7.63(18H, m), 7.74∼7.79(24H, m) | 1549.9 | 1548.6 |
| 76 | δ = 6.59(2H, m), 6.69(4H, m), 6.83∼6.89(6H, m), 7.03(2H, m), 7.11(8H, m), 7.26∼7.44(22H, m), 7.51∼7.55(22H, m), 7.63(2H, m), 7.75∼7.77(4H, m), 7.87∼7.93(4H, m) | 1449.8 | 1448.6 |
| 105 | δ = 6.62(2H, m), 6.69∼6.7(4H, m), 6.83∼6.87(4H, m), 7.03∼7.16(16H, m), 7.26~7.41(18H, m), 7.51~7.55(10H, m), 7.63(2H, m), 7.75∼7.77(4H, m), 7.87~7.93(4H, m), 8.07(2H, m) | 1299.6 | 1298.5 |
| 181 | δ = 6.61∼6.62(6H, m), 6.7(2H, m), 6.83(2H, m), 6.99∼7.03(6H, m), 7.11(8H, m), 7.26∼7.38(16H, m), 7.55(4H, m), 7.63(2H, m), 7.75∼7.77(4H, m), 7.87∼7.93(4H, m), 8.07(2H, m) | 1183.4 | 1182.4 |
| 229 | δ = 2.12(6H, s), 2.18(12H, s), 2.34(6H, s), 6.12(2H, s), 6.62(2H, m), 6.7(2H, m), 6.83(2H, m), 7.03(2H, m), 7.11(8H, m), 7.26∼7.38(16H, m), 7.55(4H, m), 7.63(2H, m), 7.75∼7.77(4H, m), 7.87∼7.93(4H, m), 8.07(2H, m) | 1259.6 | 1258.6 |
| 264 | δ = 6.63(8H, m), 6.81∼6.83(6H, m), 7.03(2H, m), 7.11(12H, m), 7.2∼7.33(34H, m), 7.75(2H, m) | 1149.5 | 1148.5 |
| 284 | δ = 6.63(4H, m), 6.81∼6.83(4H, m), 6.98∼7.03(4H, m), 7.11(12H, m), 7.2∼7.38(32H, m), 7.53∼7.57(6H, m), 7.75(2H, m), 8.02∼8.07(4H, m) | 1249.6 | 1248.5 |
| 303 | δ = 6.83(2H, m), 6.98∼7.03(4H, m), 7.11(12H, m), 7.26∼7.38(30H, m), 7.49∼7.57(10H, m), 7.74∼7.77(6H, m), 7.84∼7.88(4H, m), 8.02∼8.07(4H, m) | 1349.7 | 1348.6 |
| 341 | δ = 6.69(4H, m), 6.83(2H, m), 7.03∼7.11(16H, m), 7.26∼7.33(28H, m), 7.41(2H, m), 7.51∼7.54(12H, m), 7.71~7.75(6H, m), 7.82∼7.88(4H, m), 8.12(2H, m), 8.68(2H, m), 8.93(2H, m) | 1501.9 | 1500.6 |
| 364 | δ = 2.34(12H, s), 6.36(4H, m), 6.69∼6.71(4H, m), 6.83∼6.87(4H, m), 7.03∼7.16(20H, m), 7.26∼7.33(26H, m), 7.41(2H, m), 7.51~7.54(6H, m), 7.75(2H, m) | 1357.8 | 1356.6 |
| 427 | δ = 2.34(18H, s), 6.36(4H, m), 6.51(4H, m), 6.71(2H, m), 6.83(2H, m), 6.98∼7.03(6H, m), 7.11(12H, m), 7.26∼7.33(26H, m), 7.75(2H, m) | 1233.6 | 1232.6 |
| 452 | δ = 2.34(24H, s), 6.32∼6.36(6H, m), 6.43(2H, m), 6.71(2H, m), 6.83∼6.86(4H, m), 7.03(2H, m), 7.11(12H, m), 7.26∼7.33(26H, m), 7.75(2H, m) | 1261.7 | 1260.6 |
| 491 | δ = 2.12(6H, s), 2.18(12H, s), 2.34(6H, s), 6.12(2H, s), 6.65(2H, m), 6.83(2H, m), 6.95(2H, m), 7.03(2H, m), 7.11(12H, m), 7.26∼7.33(26H, m), 7.41(4H, m), 7.51(8H, m), 7.6(2H, m), 7.75∼7.79(10H, m) | 1566.1 | 1564.8 |
| 514 | δ = 0.25(18H, s), 6.61(4H, m), 6.83(2H, m), 7.03(2H, m), 7.11∼7.15(16H, m), 7.26∼7.37(28H, m), 7.57(2H, m), 7.69∼7.75(6H, m), 7.94(2H, m), 8.22(2H, m) | 1395.9 | 1394.6 |
| 567 | δ = 6.69(4H, m), 6.83(2H, m), 7.03(2H, m), 7.36∼7.41(4H, m), 7.49∼7.59(28H, m), 7.66(6H, m), 7.73∼7.77(10H, m), 7.84∼7.92(8H, m), 8(8H, m) | 1421.8 | 1420.6 |
| 627 | δ = 2.34(12H, s), 6.36(4H, m), 6.69∼6.71(4H, m), 6.83∼6.87(4H, m), 7.03∼7.08(6H, m), 7.16(2H, m), 7.41(2H, m), 7.51∼7.59(18H, m), 7.66(6H, m), 7.73∼7.75(6H, m), 7.92(4H, m), 8(8H, m) | 1377.8 | 1376.6 |
| 645 | δ = 6.62(2H, m), 6.7(2H, m), 6.83(2H, m), 7.02∼7.08(4H, m), 7.32(1H, m), 7.55∼7.59(14H, m), 7.66∼7.75(16H, m), 7.82∼7.92(9H, m), 8(8H, m), 8.07∼8.13(5H, m), 8.68(1H, m), 8.93(2H, m) | 1371.7 | 1370.5 |
| 659 | δ = 2.34(6H, s), 6.51(4H, m), 6.83(4H, m), 6.98∼7.03(8H, m), 7.39(12H, m), 7.58∼7.59(12H, m), 7.66(6H, m), 7.73∼7.75(6H, m), 7.91~7.92(10H, m), 8(8H, m) | 1545.9 | 1544.6 |
| 674 | δ = 2.34(12H, s), 6.32(2H, m), 6.43(2H, m), 6.83∼6.86(8H, m), 7.03∼7.06(4H, m), 7.41(4H, m), 7.51∼7.52(16H, m), 7.58∼7.59(12H, m), 7.66(6H, m), 7.73∼7.75(6H, m), 7.92(4H, m), 8(8H, m) | 1529.9 | 1528.7 |
| 694 | δ = 2.34(6H, s), 6.51(4H, m), 6.62(2H, m), 6.7(2H, m), 6.83(2H, m), 6.98∼7.03(6H, m), 7.55∼7.59(14H, m), 7.66(6H, m), 7.73∼7.75(6H, m), 7.92(4H, m), 8(8H, m), 8.07(2H, m) | 1199.5 | 1198.5 |
| 715 | δ = 2.34(24H, s), 6.32∼6.36(6H, m), 6.43(2H, m), 6.71(2H, m), 6.83∼6.86(4H, m), 7.03(2H, m), 7.58∼7.59(12H, m), 7.66(6H, m), 7.73∼7.75(6H, m), 7.92(4H, m), 8(8H, m) | 1281.7 | 1280.6 |
| 733 | δ = 1.35(18H, s), 6.55(4H, m), 6.83(2H, m), 6.91(2H, m), 7.01∼7.03(6H, m), 7.34(2H, m), 7.49(2H, m), 7.56∼7.59(14H, m), 7.66∼7.67(8H, m), 7.73∼7.75(6H, m), 7.92(4H, m), 8(8H, m), 8.1 (4H, m), 8.42(4H, m) | 1529.9 | 1528.7 |
| 814 | δ = 1.35(36H, s), 6.63(4H, m), 6.81∼6.83(4H, m), 7.03∼7.08(4H, m), 7.2(4H, m), 7.32∼7.38(18H, m), 7.66∼7.75(12H, m), 7.82-7.88(4H, m), 8.12(2H, m), 8.68(2H, m), 8.93(2H, m) | 1393.9 | 1392.7 |
| 829 | δ = 1.35(36H, s), 6.83(2H, m), 6.98∼7.03(4H, m), 7.36∼7.38(20H, m), 7.49∼7.57(10H, m), 7.66(6H, m), 7.74∼7.77(6H, m), 7.84∼7.88(4H, m), 8.02∼8.07(4H, m) | 1393.9 | 1392.7 |
| 848 | δ = 1.35(36H, s), 6.69(4H, m), 6.83(2H, m), 6.98∼7.03(4H, m), 7.37∼7.41(20H, m), 7.51∼7.57(18H, m), 7.66(6H, m), 7.75(2H, m), 8.02~8.07(4H, m) | 1446.0 | 1444.8 |
| 863 | δ = 1.35(36H, s), 6.62(2H, m), 6.7(2H, m), 6.83(2H, m), 6.98∼7.03(4H, m), 7.37∼7.38(18H, m), 7.53∼7.57(8H, m), 7.66(6H, m), 7.75(2H, m), 8.02∼8.07(6H, m) | 1295.7 | 1294.7 |
| 878 | δ = 0.25(18H, s), 1.35(36H, s), 6.61(4H, m), 6.69(4H, m), 6.83(2H, m), 7.03(2H, m), 7.15(4H, m), 7.37∼7.41(18H, m), 7.51∼7.54(12H, m), 7.66(6H, m), 7.75(2H, m) | 1490.2 | 1488.8 |
| 923 | δ = 1.35(36H, s), 6.61(4H, m), 6.83(4H, m), 6.99∼7.03(8H, m), 7.37∼7.39(28H, m), 7.66(6H, m), 7.75(2H, m), 7.91(6H, m) | 1578.0 | 1576.7 |
| 940 | δ = 1.35(36H, s), 2.18(6H, s), 2.34(12H, s), 6.24(4H, m), 6.83∼6.85(6H, m), 7.03∼7.06(4H, m), 7.37∼7.41 (20H, m), 7.51∼7.52(16H, m), 7.66(6H, m), 7.75(2H, m) | 1582.2 | 1580.9 |
| 979 | δ = 1.35(36H, s), 2.34(12H, s), 6.32(2H, m), 6.43(2H, m), 6.56(4H, m), 6.83∼6.86(4H, m), 7.03(2H, m), 7.37∼7.38(20H, m), 7.66(6H, m), 7.75(2H, m) | 1385.7 | 1384.7 |
| 1032 | δ = 1.35(36H, s), 6.56(4H, m), 6.62(2H, m), 6.7(2H, m), 6.83(2H, m), 7.03(2H, m), 7.37∼7.38(20H, m), 7.55(2H, m), 7.66(6H, m), 7.75(2H, m), 8.07(2H, m) | 1331.6 | 1330.6 |
| 1092 | δ = 6.83(2H, m), 6.98∼7.03(4H, m), 7.16∼7.19(4H, m), 7.28(4H, m), 7.35∼7.38(10H, m), 7.44∼7.57(18H, m), 7.74∼7.88(18H, m), 8.02∼8.07(4H, m) | 1341.6 | 1340.5 |
| 1114 | δ = 6.83(2H, m), 6.98∼7.08(6H, m), 7.16∼7.19(4H, m), 7.28∼7.38(14H, m), 7.44(2H, m), 7.53∼7.57(12H, m), 7.71∼7.88(18H, m), 8.02∼8.12(6H, m), 8.68(2H, m), 8.93(2H, m) | 1441.8 | 1440.5 |
| 1142 | δ = 6.62(2H, m), 6.69∼6.7(6H, m), 6.83(2H, m), 7.03(2H, m), 7.16∼7.19(4H, m), 7.28(4H, m), 7.35∼7.44(10H, m), 7.51∼7.57(20H, m), 7.75∼7.81(6H, m), 7.87(4H, m), 8.07(2H, m) | 1295.6 | 1294.5 |
| 1171 | δ = 6.62(2H, m), 6.7(2H, m), 6.83(2H, m), 7.03∼7.08(4H, m), 7.16∼7.19(4H, m), 7.28∼7.38(12H, m), 7.44(2H, m), 7.55∼7.57(8H, m), 7.71∼7.88(18H, m), 8.07∼8.12(4H, m), 8.68(2H, m), 8.93(2H, m) | 1343.6 | 1342.5 |
| 1187 | δ = 2.34(12H, s), 6.32(2H, m), 6.43(2H, m), 6.83∼6.86(6H, m), 7.03(4H, m), 7.16∼7.19(4H, m), 7.28(4H, m), 7.35∼7.44(20H, m), 7.55∼7.57(6H, m), 7.75∼7.81(6H, m), 7.87∼7.91(10H, m) | 1545.9 | 1544.6 |
| 1237 | δ = 1.35(18H, s), 2.34(12H, s), 6.32(2H, m), 6.43(2H, m), 6.55(4H, m), 6.83∼6.86(4H, m), 7.01∼7.03(6H, m), 7.16∼7.19(4H, m), 7.28(4H, m), 7.35∼7.38(6H, m), 7.44(2H, m), 7.55∼7.57(6H, m), 7.75∼7.81(6H, m), 7.87(4H, m) | 1309.7 | 1308.6 |
| 1266 | δ = 3.83(6H, s), 6.52(4H, m), 6.62(2H, m), 6.7∼6.74(6H, m), 6.83(2H, m), 7.03(2H, m), 7.16∼7.19(4H, m), 7.28(4H, m), 7.35∼7.38(6H, m), 7.44(2H, m), 7.55∼7.57(8H, m), 7.75∼7.81(6H, m), 7.87(4H, m), 8.07(2H, m) | 1203.4 | 1202.5 |
| 1289 | δ = 2.34(12H, s), 6.36(4H, m), 6.71(2H, m), 6.83(2H, m), 7.03(2H, m), 7.16∼7.19(4H, m), 7.28(4H, m), 7.35∼7.38(8H, m), 7.44(2H, m), 7.55∼7.57(8H, m), 7.69∼7.81(10H, m), 7.87(4H, m), 7.94(2H, m), 8.22(2H, m) | 1299.6 | 1298.5 |
| 1318 | δ = 6.83(2H, m), 6.98∼7.03(6H, m), 7.11(8H, m), 7.26∼7.38(20H, m), 7.53∼7.63(16H, m), 7.75∼7.77(4H, m), 7.87∼7.93(4H, m), 8.02∼8.07(8H, m) | 1345.7 | 1344.5 |
| 1371 | δ = 6.65(2H, m), 6.83(2H, m), 6.95(2H, m), 7.03(2H, m). 7.11(8H, m), 7.26∼7.41(22H, m), 7.49∼7.63(18H, m), 7.74∼7.79(24H, m) | 1549.9 | 1548.6 |
| 1391 | δ = 6.59(2H, m), 6.69(4H, m), 6.83∼6.89(6H, m), 7.03(2H, m), 7.11(8H, m), 7.26∼7.44(22H, m), 7.51∼7.55(22H, m), 7.63(2H, m), 7.75∼7.77(4H, m), 7.87∼7.93(4H, m) | 1449.8 | 1448.6 |
| 1420 | δ = 6.62(2H, m), 6.69∼6.7(4H, m), 6.83∼6.87(4H, m), 7.03∼7.16(16H, m), 7.26∼7.41(18H, m), 7.51∼7.55(10H, m), 7.63(2H, m), 7.75∼7.77(4H, m), 7.87∼7.93(4H, m), 8.07(2H, m) | 1299.6 | 1298.5 |
| 1496 | δ = 6.61∼6.62(6H, m), 6.7(2H, m), 6.83(2H, m), 6.99∼7.03(6H, m), 7.11(8H, m), 7.26~7.38(16H, m), 7.55(4H, m), 7.63(2H, m), 7.75∼7.77(4H, m), 7.87∼7.93(4H, m), 8.07(2H, m) | 1183.4 | 1182.4 |
| 1544 | δ = 2.12(6H, s), 2.18(12H, s), 2.34(6H, s), 6.12(2H, s), 6.62(2H, m), 6.7(2H, m), 6.83(2H, m), 7.03(2H, m), 7.11(8H, m), 7.26∼7.38(16H, m), 7.55(4H, m), 7.63(2H, m), 7.75∼7.77(4H, m), 7.87∼7.93(4H, m), 8.07(2H, m) | 1259.6 | 1258.6 |
| 1579 | δ = 6.63(8H, m), 6.81∼6.83(6H, m), 7.03(2H, m), 7.11(12H, m), 7.2∼7.33(34H, m), 7.75(2H, m) | 1149.5 | 1148.5 |
| 1599 | δ = 6.63(4H, m), 6.81∼6.83(4H, m), 6.98∼7.03(4H, m), 7.11(12H, m), 7.2∼7.38(32H, m), 7.53∼7.57(6H, m), 7.75(2H, m), 8.02∼8.07(4H, m) | 1249.6 | 1248.5 |
| 1618 | δ = 6.83(2H, m), 6.98∼7.03(4H, m), 7.11(12H, m), 7.26~7.38(30H, m), 7.49~7.57(10H, m), 7.74~7.77(6H, m), 7.84∼7.88(4H, m), 8.02∼8.07(4H, m) | 1349.7 | 1348.6 |
| 1656 | δ = 6.69(4H, m), 6.83(2H, m), 7.03∼7.11(16H, m), 7.26∼7.33(28H, m), 7.41(2H, m), 7.51∼7.54(12H, m), 7.71~7.75(6H, m), 7.82∼7.88(4H, m), 8.12(2H, m), 8.68(2H, m), 8.93(2H, m) | 1501.9 | 1500.6 |
| 1679 | δ = 2.34(12H, s), 6.36(4H, m), 6.69∼6.71(4H, m), 6.83∼6.87(4H, m), 7.03∼7.16(20H, m), 7.26∼7.33(26H, m), 7.41(2H, m), 7.51∼7.54(6H, m), 7.75(2H, m) | 1357.8 | 1356.6 |
| 1742 | δ = 2.34(18H, s), 6.36(4H, m), 6.51(4H, m), 6.71(2H, m), 6.83(2H, m), 6.98∼7.03(6H, m), 7.11(12H, m), 7.26∼7.33(26H, m), 7.75(2H, m) | 1233.6 | 1232.6 |
| 1767 | δ = 2.34(24H, s), 6.32∼6.36(6H, m), 6.43(2H, m), 6.71(2H, m), 6.83∼6.86(4H, m), 7.03(2H, m), 7.11(12H, m), 7.26∼7.33(26H, m), 7.75(2H, m) | 1261.7 | 1260.6 |
| 1806 | δ = 2.12(6H, s), 2.18(12H, s), 2.34(6H, s), 6.12(2H, s), 6.65(2H, m), 6.83(2H, m), 6.95(2H, m), 7.03(2H, m), 7.11(12H, m), 7.26∼7.33(26H, m), 7.41(4H, m), 7.51(8H, m), 7.6(2H, m), 7.75∼7.79(10H, m) | 1566.1 | 1564.8 |
| 1829 | δ = 0.25(18H, s), 6.61(4H, m), 6.83(2H, m), 7.03(2H, m), 7.11∼7.15(16H, m), 7.26∼7.37(28H, m), 7.57(2H, m), 7.69∼7.75(6H, m), 7.94(2H, m), 8.22(2H, m) | 1395.9 | 1394.6 |
| 1861 | δ = 6.63(4H, m), 6.81∼6.83(4H, m), 7.03(2H, m), 7.2(4H, m), 7.36(2H, m), 7.49∼7.5(4H, m), 7.58∼7.59(12H, m), 7.66(6H, m), 7.73∼7.77(10H, m), 7.84∼7.92(8H, m), 8(8H, m) | 1269.6 | 1268.5 |
| 1882 | δ = 6.69(4H, m), 6.83(2H, m), 7.03(2H, m), 7.36∼7.41(4H, m), 7.49∼7.59(28H, m), 7.66(6H, m), 7.73∼7.77(10H, m), 7.84∼7.92(8H, m), 8(8H, m) | 1421.8 | 1420.6 |
| 1942 | δ = 2.34(12H, s), 6.36(4H, m), 6.69∼6.71(4H, m), 6.83∼6.87(4H, m), 7.03∼7.08(6H, m), 7.16(2H, m), 7.41(2H, m), 7.51∼7.59(18H, m), 7.66(6H, m), 7.73∼7.75(6H, m), 7.92(4H, m), 8(8H, m) | 1377.8 | 1376.6 |
| 1960 | δ = 6.62(2H, m), 6.7(2H, m), 6.83(2H, m), 7.02∼7.08(4H, m), 7.32(1H, m), 7.55∼7.59(14H, m), 7.66∼7.75(16H, m), 7.82∼7.92(9H, m), 8(8H, m), 8.07∼8.13(5H, m), 8.68(1H, m), 8.93(2H, m) | 1371.7 | 1370.5 |
| 1974 | δ = 2.34(6H, s), 6.51(4H, m), 6.83(4H, m), 6.98∼7.03(8H, m), 7.39(12H, m), 7.58∼7.59(12H, m), 7.66(6H, m), 7.73∼7.75(6H, m), 7.91∼7.92(10H, m), 8(8H, m) | 1545.9 | 1544.6 |
| 1989 | δ = 2.34(12H, s), 6.32(2H, m), 6.43(2H, m), 6.83∼6.86(8H, m), 7.03∼7.06(4H, m), 7.41(4H, m), 7.51∼7.52(16H, m), 7.58∼7.59(12H, m), 7.66(6H, m), 7.73∼7.75(6H, m), 7.92(4H, m), 8(8H, m) | 1529.9 | 1528.7 |
| 2009 | δ = 2.34(6H, s), 6.51(4H, m), 6.62(2H, m), 6.7(2H, m), 6.83(2H, m), 6.98∼7.03(6H, m), 7.55∼7.59(14H, m), 7.66(6H, m), 7.73∼7.75(6H, m), 7.92(4H, m), 8(8H, m), 8.07(2H, m) | 1199.5 | 1198.5 |
| 2030 | δ = 2.34(24H, s), 6.32∼6.36(6H, m), 6.43(2H, m), 6.71(2H, m), 6.83∼6.86(4H, m), 7.03(2H, m), 7.58∼7.59(12H, m), 7.66(6H, m), 7.73∼7.75(6H, m), 7.92(4H, m), 8(8H, m) | 1281.7 | 1280.6 |
| 2079 | δ = 2.34(24H, s), 6.36(4H, m), 6.65∼6.71(4H, m), 6.83(2H, m), 6.95(2H, m), 7.03(2H, m), 7.29∼7.33(16H, m), 7.58∼7.6(14H, m), 7.66(6H, m), 7.73∼7.75(6H, m), 7.92(4H, m), 8(8H, m) | 1586.0 | 1584.7 |
| 2129 | δ = 1.35(36H, s), 6.63(4H, m), 6.81∼6.83(4H, m), 7.03∼7.08(4H, m), 7.2(4H, m), 7.32∼7.38(18H, m), 7.66∼7.75(12H, m), 7.82∼7.88(4H, m), 8.12(2H, m), 8.68(2H, m), 8.93(2H, m) | 1393.9 | 1392.7 |
| 2144 | δ = 1.35(36H, s), 6.83(2H, m), 6.98∼7.03(4H, m), 7.36∼7.38(20H, m), 7.49∼7.57(10H, m), 7.66(6H, m), 7.74∼7.77(6H, m), 7.84∼7.88(4H, m), 8.02∼8.07(4H, m) | 1393.9 | 1392.7 |
| 2163 | δ = 1.35(36H, s), 6.69(4H, m), 6.83(2H, m), 6.98∼7.03(4H, m), 7.37∼7.41(20H, m), 7.51∼7.57(18H, m), 7.66(6H, m), 7.75(2H, m), 8.02∼8.07(4H, m) | 1446.0 | 1444.8 |
| 2178 | δ = 1.35(36H, s), 6.62(2H, m), 6.7(2H, m), 6.83(2H, m), 6.98∼7.03(4H, m), 7.37∼7.38(18H, m), 7.53∼7.57(8H, m), 7.66(6H, m), 7.75(2H, m), 8.02∼8.07(6H, m) | 1295.7 | 1294.7 |
| 2193 | δ = 0.25(18H, s), 1.35(36H, s), 6.61(4H, m), 6.69(4H, m), 6.83(2H, m), 7.03(2H, m), 7.15(4H, m), 7.37∼7.41(18H, m), 7.51∼7.54(12H, m), 7.66(6H, m), 7.75(2H, m) | 1490.2 | 1488.8 |
| 2238 | δ = 1.35(36H, s), 6.61(4H, m), 6.83(4H, m), 6.99∼7.03(8H, m), 7.37∼7.39(28H, m), 7.66(6H, m), 7.75(2H, m), 7.91(6H, m) | 1578.0 | 1576.7 |
| 2255 | δ = 1.35(36H, s), 2.18(6H, s), 2.34(12H, s), 6.24(4H, m), 6.83∼6.85(6H, m), 7.03∼7.06(4H, m), 7.37∼7.41(20H, m), 7.51∼7.52(16H, m), 7.66(6H, m), 7.75(2H, m) | 1582.2 | 1580.9 |
| 2294 | δ = 1.35(36H, s), 2.34(12H, s), 6.32(2H, m), 6.43(2H, m), 6.56(4H, m), 6.83∼6.86(4H, m), 7.03(2H, m), 7.37∼7.38(20H, m), 7.66(6H, m), 7.75(2H, m) | 1385.7 | 1384.7 |
| 2347 | δ = 1.35(36H, s), 6.56(4H, m), 6.62(2H, m), 6.7(2H, m), 6.83(2H, m), 7.03(2H, m), 7.37∼7.38(20H, m), 7.55(2H, m), 7.66(6H, m), 7.75(2H, m), 8.07(2H, m) | 1331.6 | 1330.6 |
| 2407 | δ = 6.83(2H, m), 6.98∼7.03(4H, m), 7.16∼7.19(4H, m), 7.28(4H, m), 7.35∼7.38(10H, m), 7.44∼7.57(18H, m), 7.74∼7.88(18H, m), 8.02∼8.07(4H, m) | 1341.6 | 1340.5 |
| 2429 | δ = 6.83(2H, m), 6.98∼7.08(6H, m), 7.16∼7.19(4H, m), 7.28∼7.38(14H, m), 7.44(2H, m), 7.53∼7.57(12H, m), 7.71∼7.88(18H, m), 8.02∼8.12(6H, m), 8.68(2H, m), 8.93(2H, m) | 1441.8 | 1440.5 |
| 2457 | δ = 6.62(2H, m), 6.69∼6.7(6H, m), 6.83(2H, m), 7.03(2H, m), 7.16∼7.19(4H, m), 7.28(4H, m), 7.35∼7.44(10H, m), 7.51∼7.57(20H, m), 7.75∼7.81(6H, m), 7.87(4H, m), 8.07(2H, m) | 1295.6 | 1294.5 |
| 2486 | δ = 6.62(2H, m), 6.7(2H, m), 6.83(2H, m), 7.03∼7.08(4H, m), 7.16∼7.19(4H, m), 7.28∼7.38(12H, m), 7.44(2H, m), 7.55∼7.57(8H, m), 7.71∼7.88(18H, m), 8.07∼8.12(4H, m), 8.68(2H, m), 8.93(2H, m) | 1343.6 | 1342.5 |
| 2502 | δ = 2.34(12H, s), 6.32(2H, m), 6.43(2H, m), 6.83∼6.86(6H, m), 7.03(4H, m), 7.16∼7.19(4H, m), 7.28(4H, m), 7.35∼7.44(20H, m), 7.55∼7.57(6H, m), 7.75∼7.81(6H, m), 7.87∼7.91(10H, m) | 1545.9 | 1544.6 |
| 2552 | δ = 1.35(18H, s), 2.34(12H, s), 6.32(2H, m), 6.43(2H, m), 6.55(4H, m), 6.83∼6.86(4H, m), 7.01∼7.03(6H, m), 7.16∼7.19(4H, m), 7.28(4H, m), 7.35∼7.38(6H, m), 7.44(2H, m), 7.55∼7.57(6H, m), 7.75∼7.81(6H, m), 7.87(4H, m) | 1309.7 | 1308.6 |
| 2581 | δ = 3.83(6H, s), 6.52(4H, m), 6.62(2H, m), 6.7∼6.74(6H, m), 6.83(2H, m), 7.03(2H, m), 7.16∼7.19(4H, m), 7.28(4H, m), 7.35∼7.38(6H, m), 7.44(2H, m), 7.55∼7.57(8H, m), 7.75∼7.81(6H, m), 7.87(4H, m), 8.07(2H, m) | 1203.4 | 1202.5 |
| 2604 | δ = 2.34(12H, s), 6.36(4H, m), 6.71(2H, m), 6.83(2H, m), 7.03(2H, m), 7.16∼7.19(4H, m), 7.28(4H, m), 7.35∼7.38(8H, m), 7.44(2H, m), 7.55∼7.57(8H, m), 7.69∼7.81(10H, m), 7.87(4H, m), 7.94(2H, m), 8.22(2H, m) | 1299.6 | 1298.5 |
| 2637 | δ = 6.83(2H, m), 7.03∼7.04(6H, m), 7.16∼7.19(4H, m), 7.28(4H, m), 7.35∼7.44(12H, m), 7.51∼7.57(22H, m), 7.75∼7.81(18H, m), 7.87(4H, m), 8.07(4H, m), 8.49(4H, m) | 1646.0 | 1644.6 |

### [Example 1] Manufacture of OLED's by using the organic electroluminescent compounds of the invention

An OLED device was manufactured by using the electroluminescent compound according to the invention.

First, a transparent electrode ITO thin film (15 Ω/□) (2) prepared from glass for OLED (1) (manufactured by Samsung-Corning) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm of thickness on the ITO substrate.

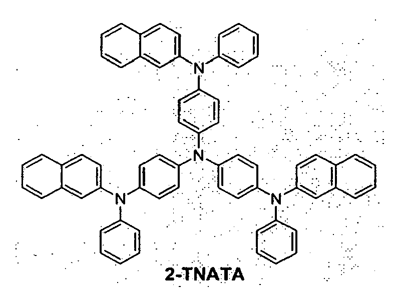

Then, to another cell of the vacuum vapor-deposit device, charged was N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer (4) having 20 nm of thickness on the hole injecting layer.

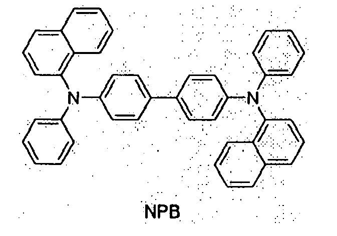

After forming the hole injecting layer and the hole transport layer, an electroluminescent layer was vapor-deposited as follows. To one cell of a vacuum vapor-deposit device, charged was H-5 (of which the structure is shown below) as a host, and a compound according to the invention (Compound 3) was charged to another cell as a dopant. Two substances were evaporated at different rates to give doping at 2 to 5mol% by weight on the basis of the host, to vapor-deposit an electroluminescent layer (5) with a thickness of 30 nm on the hole transport layer.

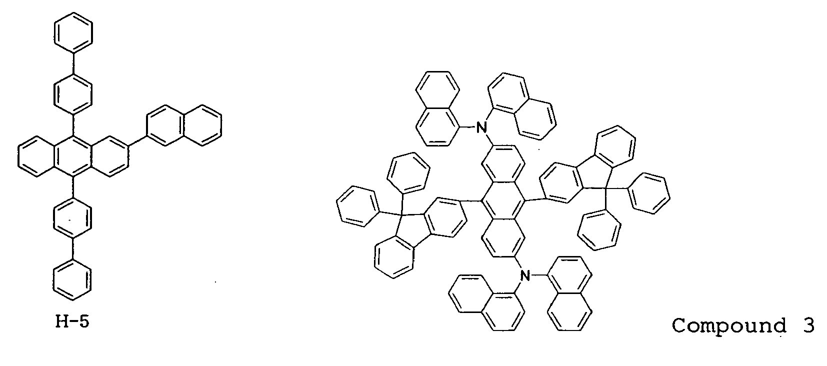

Then, tris(8-hydroxyquinoline)aluminum (III) (Alq) (of which the structure is shown below) was vapor-deposited as an electron transport layer (6) with a thickness of 20 nm, and lithium quinolate (Liq) (of which the structure shown below) was vapor-deposited as an electron injecting layer (7) with a thickness of 1 to 2 nm. Thereafter, an Al cathode (8) was vapor-deposited with a thickness of 150 nm by using another vacuum vapor-deposit device to manufacture an OLED.

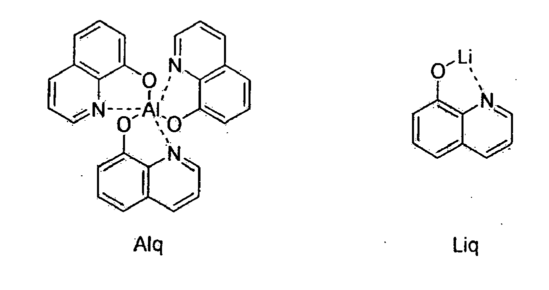

Each material employed for manufacturing an OLED was used as the electroluminescent material after being purified via vacuum sublimation at 10⁻⁶ torr.

### [Comparative Example 1] Manufacture of an OLED by using conventional electroluminescent material

After forming a hole injecting layer and hole transport layer according to the same procedure as described in Example 1, tris(8-hydroxyquinoline)-aluminum (III) (Alq) was charged to another cell of said vacuum vapor-deposit device as electroluminescent host material, while Coumarin 545T (C545T) (of which the structure is shown below) was charged to still another cell. The two substances were evaporated at different rates to carry out doping, thereby vapor-depositing an electroluminescent layer with a thickness of 30 nm on the hole transport layer. The doping concentration preferably is from 1 to 3mol% by weight on the basis of Alq.

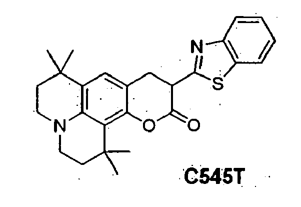

Then, an electron transport layer and an electron injecting layer were vapor-deposited according to the same procedure as in Example 1, and Al cathode was vapor-deposited by using another vacuum vapor-deposit device with a thickness of 150 nm, to manufacture an OLED.

### [Comparative Example 2] Manufacture of an OLED by using conventional electroluminescent material

After forming a hole injecting layer and a hole transport layer according to the same procedure as described in Example 1, H-5 was charged to another cell of said vacuum vapor-deposit device as electroluminescent host material, while Compound (G) was charged to still another cell. The two substances were evaporated at different rates to carry out doping at a concentration of 2 to 5mol% by weight on the basis of the host, thereby vapor-depositing an electroluminescent layer with a thickness of 30 nm on the hole transport layer.

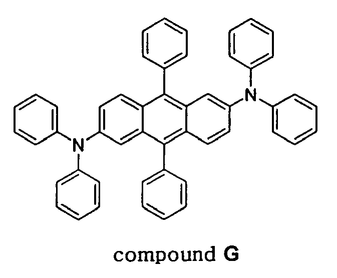

Then, an electron transport layer and an electron injecting layer were vapor-deposited according to the same procedure as in Example 1, and Al cathode was vapor-deposited by using another vacuum vapor-deposit device with a thickness of 150 nm, to manufacture an OLED.

### [Example 2] Electroluminescent properties of OLED's manufactured

The luminous efficiencies of the OLED's comprising the organic electroluminescent compound according to the present invention (Examples 1) or conventional EL compounds (Comparative Examples 1 and 2) were measured at 5,000 cd/m² and 20,000 cd/m², respectively, and the results are shown in Table 4. Since the electroluminescent properties in high luminance region are very important, particularly in case of green electroluminescent materials, the data at high luminance (about 20,000 cd/m²) are attached in order to reflect the properties.

**[Table 4]**

| No. | Host | Dopant | Doping Conc. (mol%) | Efficiency(cd/A) | | Color |
|---|---|---|---|---|---|---|
| | | | | @5,000 cd/m² | @20,000 cd/m² | |
| 1 | H-5 | 3 | 3 | 21.9 | 21.0 | Green |
| 2 | H-5 | 27 | 3 | 18.1 | 17.3 | Green |
| 3 | H-5 | 540 | 3 | 16.6 | 15.8 | Green |
| 4 | H-5 | 687 | 3 | 19.7 | 19.1 | Green |
| 5 | H-5 | 1291 | 3 | 19.0 | 18.1 | Green |
| 6 | H-5 | 1469 | 3 | 19.0 | 18.5 | Green |
| 7 | H-5 | 1803 | 3 | 20.0 | 18.4 | Green |
| 8 | H-5 | 2769 | 3 | 21.0 | 20.8 | Green |
| 9 | H-27 | 2871 | 3 | 20.4 | 19.5 | Green |
| 10 | H-27 | 4467 | 3 | 16.7 | 16.1 | Green |
| 11 | H-27 | 5066 | 3 | 16.4 | 16.0 | Green |
| 12 | H-27 | 6162 | 3 | 19.9 | 19.1 | Green |
| Comp. 1 | Alq | Compound C545T | 1.0 | 10.3 | 9.1 | Green |
| Comp. 2 | H-5 | Compound G | 3.0 | 16.3 | 14.1 | Green |

As can be seen from Table 4, it is found that Compound (H-5) with 3.0% doping of Compound (3) exhibited highest luminous efficiency, which is more than twice of that of conventional Alq:C545T (Comparative Example 1), being corresponding to 20∼30% increase of luminous efficiency as compared to Compound (3) (Comparative Example 2).

The high performance electroluminescent materials according to the invention showing the decrease of the efficiency within 1 ∼ 2 cd/A at high luminance of about 20,000 cd/m², suggests that they have excellent material properties to maintain good feature even at high luminance. Thus the materials can exhibit advantageous properties for both passive and active organic electroluminescent devices.

## Claims

1. An organic electroluminescent compound represented by Chemical Formula (1) or Chemical Formula (2): 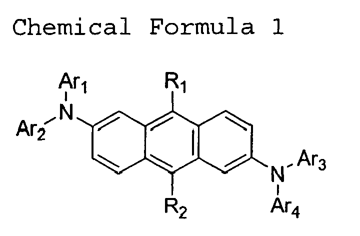 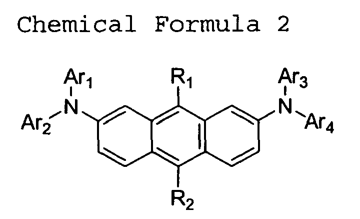
wherein, R₁ and R₂ independently represent (C6-C60)aryl or (C5-C60)heteroaryl, and the aryl and heteroaryl may be further substituted by one or more substituent(s) selected from halogen, (C1-C60)alkyl, (C6-C60)aryl, (C4-C60)heteroaryl, halo(C1-C30)alkyl, halo(C1-C30)alkoxy, (C1-C30)alkyl(C6-C30)aryl, (C6-C30)ar(C1-C30)alkyl, (C6-C30)ar(C1-C30)alkoxy, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, provided that the total number of carbons in R₁ or R₂ is from 21 to 60;
Ar₁ through Ar₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60) alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and the alkyl, cycloalkyl, heterocycloalkyl, aryl, heteroaryl, arylsilyl, alkylsilyl, alkylamino and arylamino of Ar₁ through Ar₄ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

2. The organic electroluminescent compound according to claim 1, which is represented by Chemical Formula (3) or Chemical Formula (4): 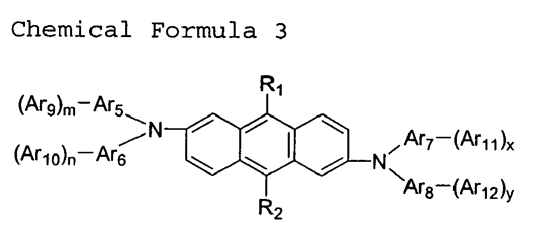 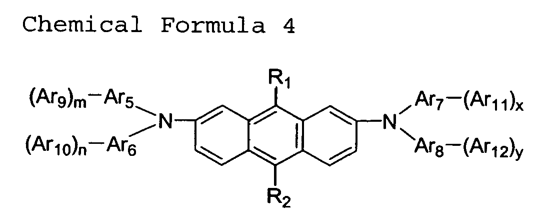
wherein, R₁ and R₂ are defined as in claim 1;
Ar₅ through Ar₈ independently represent (C6-C60)aryl, (C4-C60)heteroaryl, (C3-C60)cycloalkyl, 5- or 6-membered heterocycloalkyl containing one or more heteroatom(s) selected from N, O and S, (C6-C60)arylene or (C4-C60)heteroarylene; the aryl, heteroaryl, arylene or heteroarylene of Ar₅ through Ar₈ may be further substituted by one or more substituent(s) selected from a group consisting of (C1-C60)alkyl, halo(C1-C30)alkyl, (C1-C30)alkoxy, halo(C1-C30)alkoxy, (C3-C60)cycloalkyl, halogen, cyano, (C1-C30)alkyl(C6-C30)aryl, (C6-C30)ar(C1-C30)alkyl, (C6-C30)ar(C1-C30)alkoxy, tri(C1-C30)alkylsilyl, di(C1-C30)alkyl(C6-C30)arylsilyl and tri(C6-C30)arylsilyl;
Ar₉ through Ar₁₂ independently represent (C6-C60)aryl or (C4-C60)heteroaryl; the aryl or heteroaryl of Ar₉ through Ar₁₂ may be further substituted by one or more substituent(s) selected from a group consisting of (C1-C60)alkyl, halo(C1-C30)alkyl, (C1-C30)alkoxy, halo(C1-C30)alkoxy, (C3-C60)cycloalkyl, halogen, cyano, (C1-C30)alkyl(C6-C30)aryl, (C6-C30)ar(C1-C30)alkyl, (C6-C30)ar(C1-C30)alkoxy, tri(C1-C30)alkylsilyl, di(C1-C30)alkyl(C6-C30)arylsilyl and tri(C6-C30)arylsilyl;
provided that m is 0 when Ar₅ represents (C6-C60)aryl, (C4-C60)heteroaryl, (C3-C60)cycloalkyl or 5- or 6-membered heterocycloalkyl containing one or more heteroatom(s) selected from N, O and S, while m is an integer from 1 to 4 when Ar₅ represents (C6-C60)arylene or (C4-C60)heteroarylene;
n is 0 when Ar₆ represents (C6-C60)aryl, (C4-C60)heteroaryl, (C3-C60)cycloalkyl or 5- or 6-membered heterocycloalkyl containing one or more heteroatom(s) selected from N, O and S, while n is an integer from 1 to 4 when Ar₆ represents (C6-C60)arylene or (C4-C60)heteroarylene;
x is 0 when Ar₁₁ represents (C6-C60)aryl, (C4-C60)heteroaryl, (C3-C60)cycloalkyl or 5- or 6-membered heterocycloalkyl containing one or more heteroatom(s) selected from N, O and S, while x is an integer from 1 to 4 when Ar₁₁ represents (C6-C60)arylene or (C4-C60)heteroarylene; and
y is 0 when Ar₁₂ represents (C6-C60)aryl, (C4-C60)heteroaryl, (C3-C60)cycloalkyl or 5- or 6-membered heterocycloalkyl containing one or more heteroatom(s) selected from N, O and S, while y is an integer from 1 to 4 when Ar₁₂ represents (C6-C60)arylene or (C4-C60)heteroarylene.

3. The organic electroluminescent compound according to claim 2, wherein R₁ and R₂ are independently selected from the following structures: 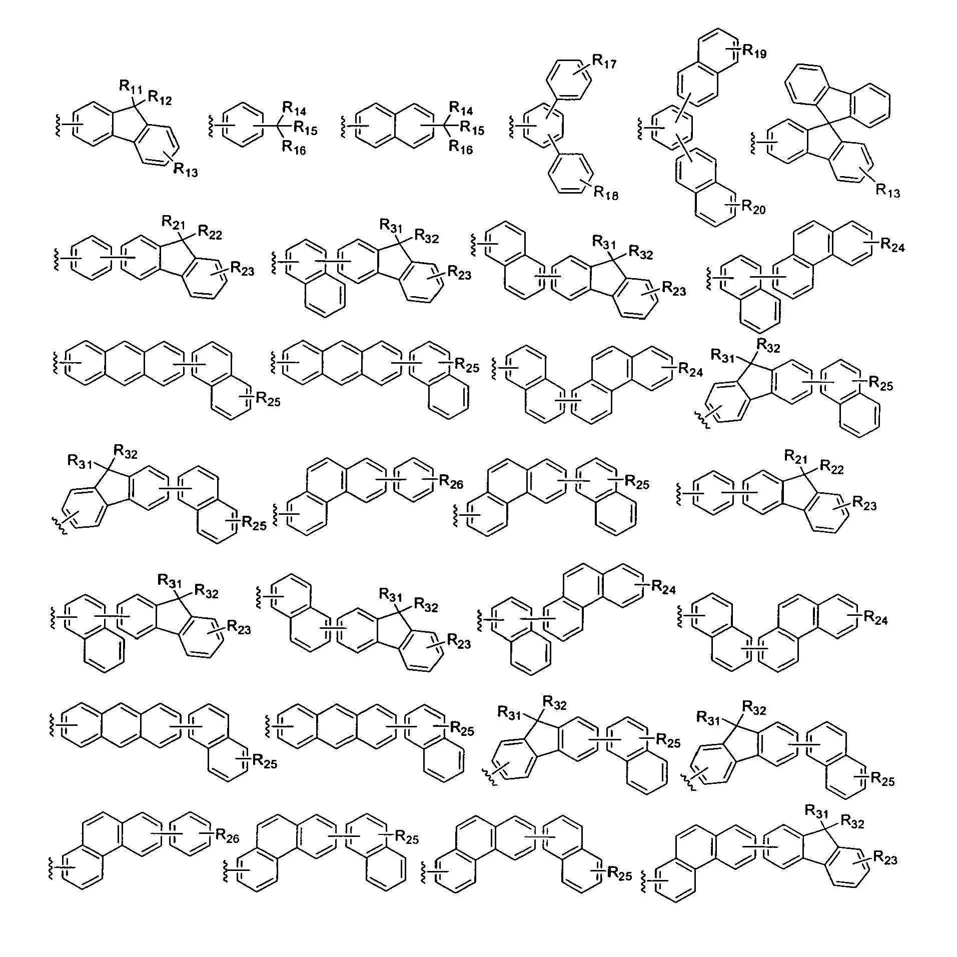 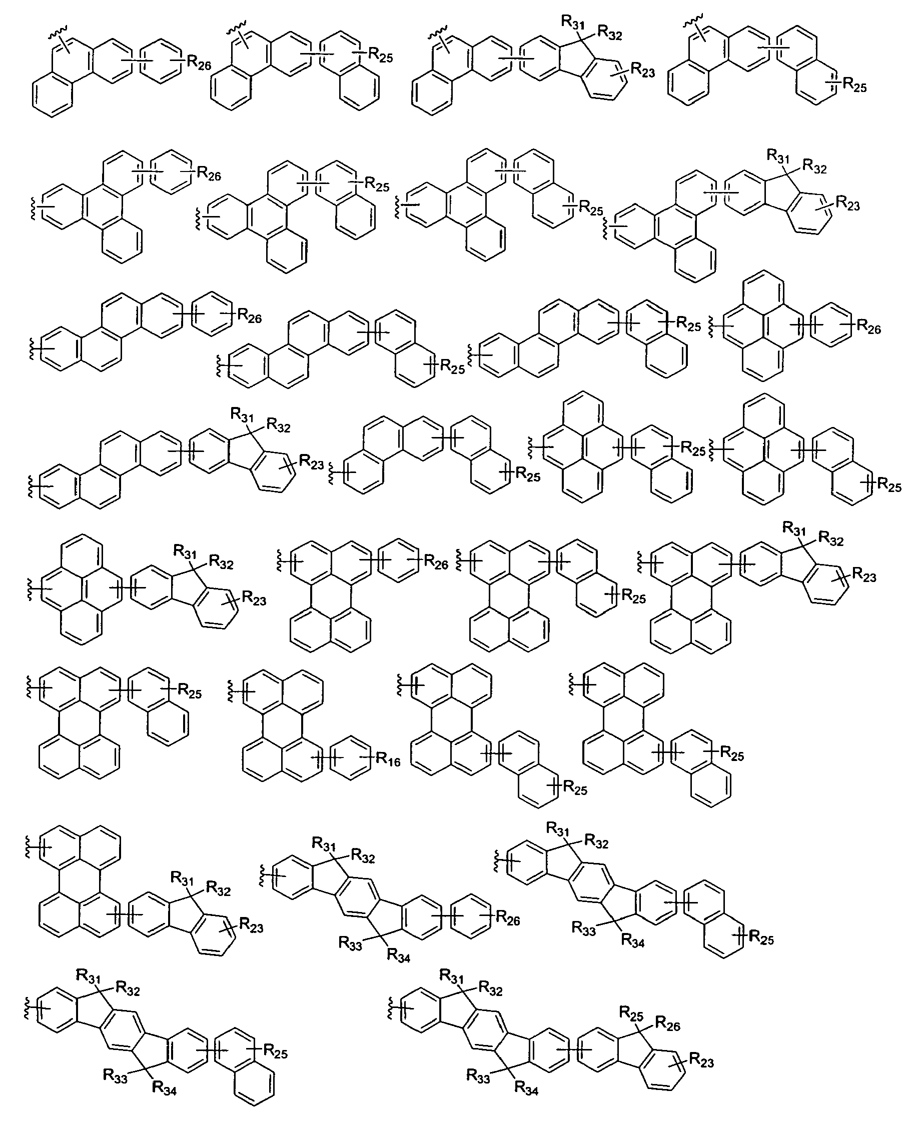
wherein, R₁₁ and R₁₂ independently represent (C6-C60)aryl, (C1-C30)alkyl(C6-C30)aryl or (C6-C30)ar(C1-C30)alkyl; R₁₃ represents hydrogen, (C1-C60)alkyl, (C6-C60)aryl, (C1-C30)alkyl(C6-C30)aryl or (C6-C30)ar(C1-C30)alkyl; R₁₄ through R₁₆ independently represent (C6-C60)aryl, (C1-C30)alkyl(C6-C30)aryl or (C6-C30)ar(C1-C30)alkyl; R₁₇ and R₁₈ independently represent (C1-C60)alkyl; R₁₉ and R₂₀ independently represent hydrogen or (C1-C60)alkyl; R₂₁ through R₂₆ independently represent (C1-C60)alkyl; and R₃₁ through R₃₄ independently represent hydrogen or (C1-C60)alkyl.

4. An organic electroluminescent device which is comprised of a first electrode; a second electrode; and at least one organic layer(s) interposed between the first electrode and the second electrode; wherein the organic layer comprises an electroluminescent layer comprising an organic electroluminescent compound represented by Chemical Formula (1) or Chemical Formula (2): 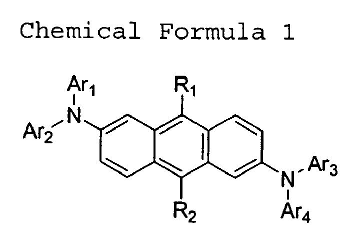 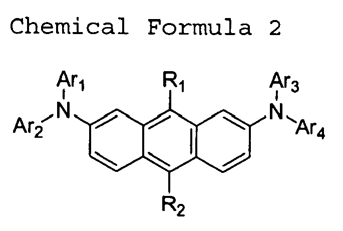
wherein, R₁ and R₂ independently represent (C6-C60)aryl or (C5-C60)heteroaryl, and the aryl and heteroaryl may be further substituted by one or more substituent(s) selected from halogen, (C1-C60)alkyl, (C6-C60)aryl, (C4-C60)heteroaryl, halo(C1-C30)alkyl, halo(C1-C30)alkoxy, (C1-C30)alkyl(C6-C30)aryl, (C6-C30)ar(C1-C30)alkyl, (C6-C30)ar(C1-C30)alkoxy, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, provided that the total number of carbons in R₁ or R₂ is from 21 to 60;
Ar1 through Ar4 independently represent hydrogen, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and the alkyl, cycloalkyl, heterocycloalkyl, aryl, heteroaryl, arylsilyl, alkylsilyl, alkylamino and arylamino of Ar1 through Ar4 may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl and one or more host(s) selected from the compounds represented by Chemical Formula (5) to (7): 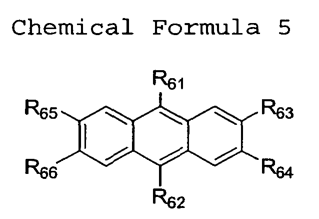 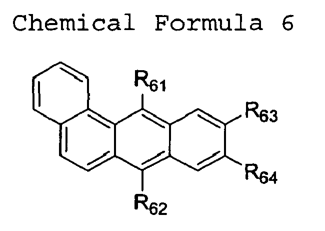 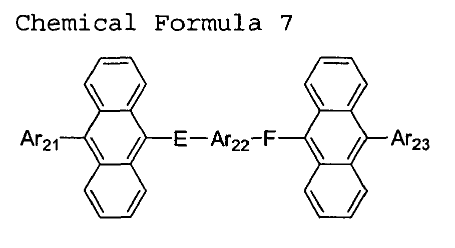
wherein, R₆₁ and R₆₂ independently represent (C6-C60)aryl, (C4-C60)heteroaryl, 5- or 6-membered heterocycloalkyl containing one or more heteroatom(s) selected from N, O and S, or (C3-C60)cycloalkyl; and the aryl or heteroaryl of R₆₁ and R₆₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₆₃ through R₆₆ represent hydrogen, (C1-C60)alkyl, (C1-C60)alkoxy, halogen, (C4-C60)heteroaryl, (C5-C60)cycloalkyl or (C6-C60)aryl; and the heteroaryl, cycloalkyl or aryl of R₆₃ through R₆₆ may be further substituted by one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
E and F independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₂₁ and Ar₂₃ represent aryl selected from the following structures, or (C4-C60)heteroaryl: 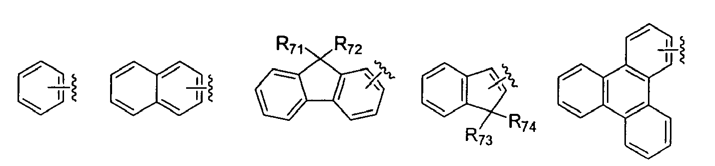 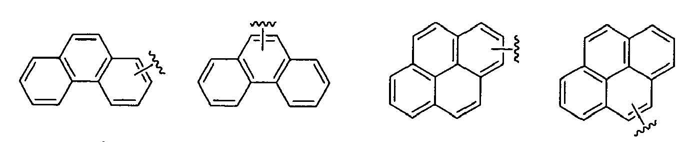 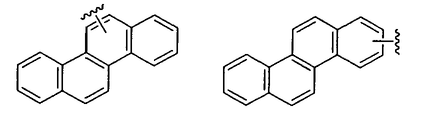
the aryl or heteroaryl of Ar₂₁ and Ar₂₃ may be substituted by one or more substituent(s) selected from (C1-C60)alkyl, (C1-C60)alkoxy, (C6-C60)aryl and (C4-C60)heteroaryl;
Ar₂₂ represents (C6-C60)arylene, (C4-C60)heteroarylene, or a compound represented by the following structural formula: 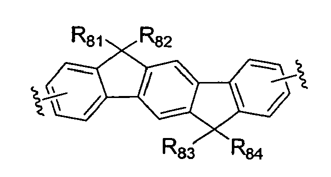
the arylene or heteroarylene of Ar₂₂ may be substituted by one or more substituent(s) selected from (C1-C60)alkyl, (C1-C60)alkoxy, (C6-C60)aryl, (C4-C60)heteroaryl and halogen;
R₇₁ through R₇₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₈₁ through R₈₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

5. The organic electroluminescent device according to claim 4, wherein the organic layer comprises one or more compound(s) selected from a group consisting of arylamine compounds and styrylarylamine compounds.

6. The organic electroluminescent device according to claim 4, wherein the organic layer comprises one or more metal(s) selected from a group consisting of organic metals of Group 1, Group 2, 4^{th} period and 5^{th} period transition metals, lanthanide metals and d-transition elements from the Periodic Table of Elements.

7. The organic electroluminescent device according to claim 4, wherein the organic layer comprises a charge generating layer as well as the electroluminescent layer.

8. The white electroluminescent device which comprises an organic electroluminescent compound represented by Chemical Formula (1) or Chemical Formula (2): 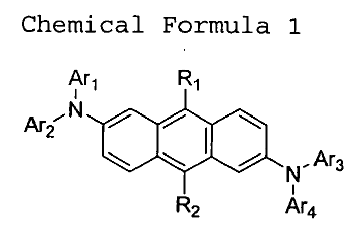 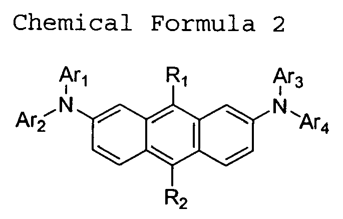
wherein, R₁ and R₂ independently represent (C6-C60)aryl or (C5-C60)heteroaryl, and the aryl and heteroaryl may be further substituted by one or more substituent(s) selected from halogen, (C1-C60)alkyl, (C6-C60)aryl, (C4-C60)heteroaryl, halo(C1-C30)alkyl, halo(C1-C30)alkoxy, (C1-C30)alkyl(C6-C30)aryl, (C6-C30)ar(C1-C30)alkyl, (C6-C30)ar(C1-C30)alkoxy, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, provided that the total number of carbons in R₁ or R₂ is from 21 to 60;
Ar₁ through Ar₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and the alkyl, cycloalkyl, heterocycloalkyl, aryl, heteroaryl, arylsilyl, alkylsilyl, alkylamino and arylamino of Ar₁ through Ar₄ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

9. An organic solar cell which comprises an organic electroluminescent compound represented by Chemical Formula (1) or Chemical Formula (2): 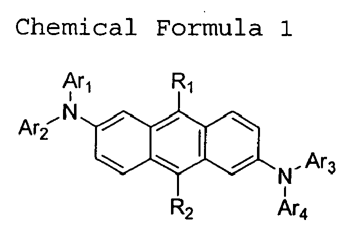 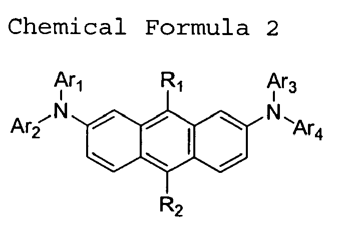
wherein, R₁ and R₂ independently represent (C6-C60)aryl or (C5-C60)heteroaryl, and the aryl and heteroaryl may be further substituted by one or more substituent(s) selected from halogen, (C1-C60)alkyl, (C6-C60)aryl, (C4-C60)heteroaryl, halo(C1-C30)alkyl, halo(C1-C30)alkoxy, (C1-C30)alkyl(C6-C30)aryl, (C6-C30)ar(C1-C30)alkyl, (C6-C30)ar(C1-C30)alkoxy, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl, provided that the total number of carbons in R₁ or R₂ is from 21 to 60;
Ar₁ through Ar₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and the alkyl, cycloalkyl, heterocycloalkyl, aryl, heteroaryl, arylsilyl, alkylsilyl, alkylamino and arylamino of Ar₁ through Ar₄ may be further substituted by one or more substituent(s) selected from halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.
